# EUROPEAN PATENT APPLICATION

(11) **EP 2 067 767 A1**
(43) Date of publication of application: **10.06.2009**
(21) Application number: 08253858.8
(22) Date of filing: 02.12.2008
(51) Int. Cl.: C07C 211/61, H01L 51/00, C09K 11/06

(54) **Novel organic electroluminescent compounds and organic electroluminescent device using the same**

(30) Priority: 04.12.2007 KR 20070124677
(71) Applicant: Gracel Display Inc., Seoul 133-833 (KR)
(72) Inventor: Lee, Mi Ae, Mapo-gu Seoul 121-190 (KR); Kim, Chi Sik, Dongjak-gu Seoul 156-825 (KR); Cho, Young Jun, Seongbuk-gu Seoul 136-060 (KR); Kwon, Hyuck Joo, Dongdaemun-gu Seoul 130-100 (KR); Kim, Bong Ok, Gangnam-gu Seoul 135-090 (KR); Kim, Sung Min, Kangseo-gu Seoul-city 157-886 (KR); Yoon, Seung Soo, Gangnam-gu, Seoul 135-884 (KR)
(74) Representative: Kent, Venetia Katherine

(57) **Abstract**

The present invention relates to novel organic electroluminescent compounds, and organic electroluminescent devices comprising the same. Specifically, the organic electroluminescent compounds according to the invention are represented by Chemical Formula (1): 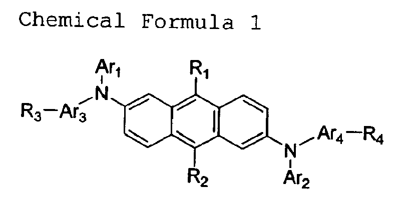 wherein, total number of carbons in R₃-Ar₃- 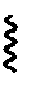 and R₄-Ar₄- 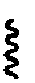 is from 21 to 60.

The electroluminescent compounds according to the present invention are green electroluminescent compounds, of which the luminous efficiency and device lifetime have been maximized.

## Description

### FIELD OF THE INVENTION

The present invention relates to novel organic electroluminescent compounds, and organic electroluminescent devices employing the same in the electroluminescent layer. Specifically, the organic electroluminescent compounds according to the present invention are characterized in that they are compounds represented by Chemical Formula (1): 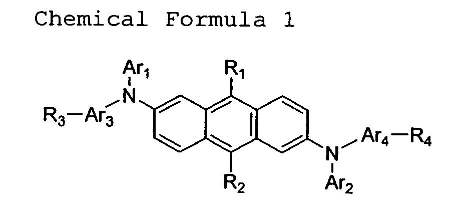 wherein, total number of carbons in 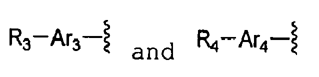 is from 21 to 60.

### BACKGROUND OF THE INVENTION

The most important matter in developing an OLED having high efficiency and long life is development of electroluminescent material of high performance. In view of current development of electroluminescent material, green electroluminescent materials show superior electroluminescent property to red or blue electroluminescent materials. However, conventional green electroluminescent materials still have many problems to achieve manufacturing panels of large size with low power consumption. In view of practical efficiency and life, various kinds of materials for green have been reported up to now. Though they show from 2 to 5 times of electroluminescent property as compared to red or blue electroluminescent materials, development of green electroluminescent material is getting challenged by the improvement of properties of red or blue electroluminescent material. In the meanwhile, enhancement of device life of the green material is still insufficient, so that a green electroluminescent material providing long life is seriously required.

As green fluorescent material, a coumarin derivative (Compound D), a quinacrydone derivative (Compound E), OPT (Compound F) and the like have been known. Compound D is the structure of C545T that is the most widely used coumarin derivative up to the present. In general, those materials are doped by using Alq as the host, at a concentration of several % to about several ten %, to form an electroluminescent device.

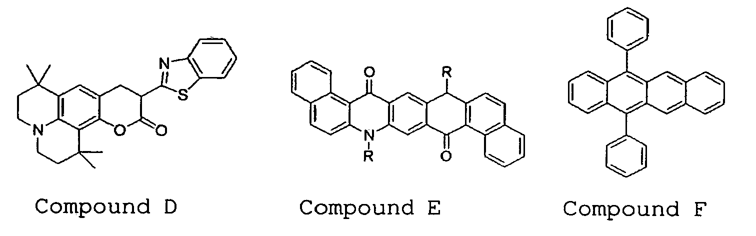

Japanese Patent Laid-Open No. 2001-131541 discloses bis(2,6-diarylamino)-9,10-diphenylanthracene derivatives represented by Compound G shown below, wherein diarylamino groups are directly substituted at 2- and 6-position of anthracene, respectively.

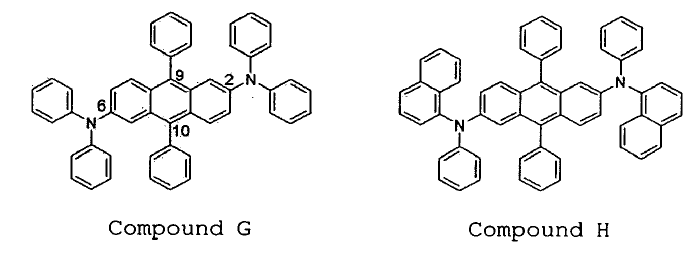

Japanese Patent Laid-Open No. 2003-146951 (which discloses compounds for a hole transport layer) does not mention the compounds wherein diarylamino groups are directly substituted at 2- and 6-position, respectively, only describing the compounds having phenyl substituents at 9- and 10-position of anthracene. As considering that Japanese Patent Laid-Open No. 2003-146951 indicated the problem of Compound (H) (wherein diarylamino groups are directly substituted at 2- and 6-position of the anthracene ring, respectively) having lowered luminous efficiency, the invention of Japanese Patent Laid-Open No. 2003-146951 did not recognize the compounds other than those having phenyl substituents at 9- and 10-position of anthracene.

In the meanwhile, Japanese Patent Laid-Open No. 2004-91334 suggested the organic electroluminescent compounds represented by Compound (J), which overcomes the poor luminous efficiency of conventional compounds but exhibits low ionization potential and excellent hole transportation, by further substituting the aryl group of the diarylamino group with diarylamino group, even though the diarylamino groups are directly substituted on the anthracene group.

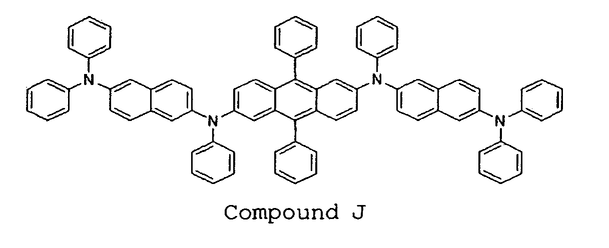

The compounds suggested by Japanese Patent Laid-Open No. 2004-91334 (applied as a hole transport layer), however, show the problem of shortened operation life as a hole transport layer because of too many amine functional groups, even though they show lowered ionization potential due to many amine functional groups and overcame the problem of increase in hole transporting property.

### SUMMARY OF THE INVENTION

According to the present invention, alkyl, alkenyl, alkynyl, cycloalkyl, alkylsilyl, arylsilyl, adamantyl, bicycloalkyl, heteroaryl or aryl (such as hydrogen, methyl, t-butyl, phenylethenyl, phenylethynyl, cyclohexyl, trimethylsilyl, triphenylsilyl, adamantyl, 4-pentylbicyclo[2.2.2]octyl, benzothiazolyl, phenyl, naphthyl, fluorenyl, phenanthryl and biphenyl) are incorporated to 9- and 10-position of anthracene, with direct substitution of diarylamino groups at 2- and 6-position, at the same time. One of the two aryl substituted to the amino groups consists of arylene and substituent to the arylene, and the total number of carbons is from 21 to 60. The inventors confirmed that the compounds as described above noticeably enhance the luminous properties, and completed the present invention.

The present inventors surprisingly found that the compounds wherein alkyl, alkenyl, alkynyl, cycloalkyl, alkylsilyl, arylsilyl, adamantyl, bicycloalkyl, heteroaryl or aryl (such as hydrogen, methyl, t-butyl, phenylethenyl, phenylethynyl, cyclohexyl, trimethylsilyl, triphenylsilyl, adamantyl, 4-pentylbicyclo[2.2.2]octyl, benzothiazolyl, phenyl, naphthyl, fluorenyl, phenanthryl and biphenyl) are incorporated to 9- and 10-position of anthracene, with direct substitution of diarylamino groups at 2- and 6-position, at the same time, and one of the two aryl substituted to the amino groups consists of arylene and substituent to the arylene, and the total number of carbons is from 21 to 60, can overcome the problems of conventional hole transport material including poor luminous efficiency, short operation life, and high ionization potential. Thus, they incorporated the structure to be applied as EL material, and completed the invention. The inventors also found that the color reproducibility is enhanced due to improved color purity, and the luminous efficiency and device life are noticeably enhanced, when one or more compound(s) selected from certain anthracene derivatives and benz[a]anthracene derivatives is (are) employed as the luminous host in the EL region, together with one or more organic electroluminescent compounds according to the invention.

The object of the present invention is to provide novel organic electroluminescent compounds wherein hydrogen, alkyl, alkenyl, alkynyl, cycloalkyl, alkylsilyl, arylsilyl, adamantyl, bicycloalkyl, heteroaryl or aryl are incorporated to 9- and 10-position of anthracene, with direct substitution of diarylamino groups at 2- and 6-position, at the same time, and one of the two aryl substituted to the amino groups consists of arylene and substituent to the arylene, and the total number of carbons is from 21 to 60.

Another object of the present invention is to provide organic electroluminescent devices which comprise an electroluminescent region employing one or more organic electroluminescent compound(s) as described above, and one or more compound(s) selected from certain anthracene derivatives and benz[a]anthracene derivatives as an electroluminescent host. Still another object of the present invention is to provide organic electroluminescent compounds exhibiting excellent color purity, high luminous efficiency and much improved device lifetime, and to provide organic electroluminescent devices comprising the novel organic electroluminescent compound as described above.

The present invention relates to novel organic electroluminescent compounds and organic electroluminescent devices comprising the same. Specifically, the organic electroluminescent compounds according to the invention are characterized in that they are represented by Chemical Formula (1): 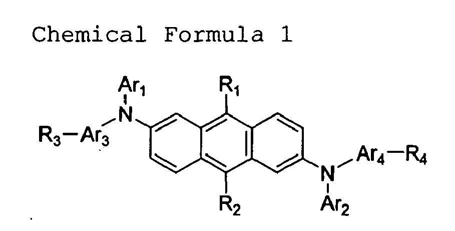 wherein, R₁ and R₂ independently represent hydrogen, deuterium, (C1-C60)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, (C7-C15)tricycloalkyl, (C4-C15)bicycloalkyl, (C6-C60)aryl or (C3-C60)heteroaryl, and the alkyl, alkenyl, alkynyl, cycloalkyl, trialkylsilyl, dialkylarylsilyl, triarylsilyl, tricycloalkyl, bicycloalkyl, aryl or heteroaryl of R₁ and R₂ may be further substituted by one or more substituent(s) selected from deuterium, (C1-C60)alkyl, (C1-C20)alkenyl, (C1-C20)alkynyl, halogen, cyano, phenyl, biphenyl, fluorenyl, naphthyl and anthryl; and

Ar₁ and Ar₂ independently represent (C6-C60)aryl, (C3-C60)heteroaryl, morpholino or thiomorpholino, and the aryl or heteroaryl of Ar₁ and Ar₂ may be further substituted by one or more substituent(s) selected from a group consisting of deuterium, (C1-C60)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl, tri(C6-C20)arylsilyl, phenyl, biphenyl, fluorenyl, naphthyl and anthryl;

Ar₃ and Ar₄ independently represent (C6-C20)arylene with or without (C1-C20)alkyl substituent;

R₃ and R₄ independently represent (C1-C20)alkyl or (C6-C20)aryl, and the aryl of R₃ and R₄ may be further substituted by deuterium or (C1-C20)alkyl;
provided that total number of carbons in 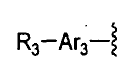 and 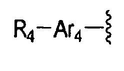 is from 21 to 60.

### BRIEF DESCRIPTION OF THE DRAWINGS

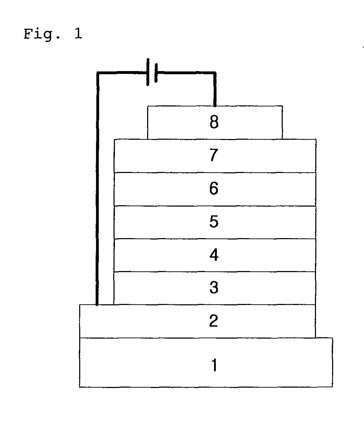

Fig. 1 is a cross-sectional view of an OLED.

### DETAILED DESCRIPTION OF THE INVENTION

Referring now to the Drawings, Fig. 1 illustrates a cross-sectional view of an OLED comprising a Glass 1, a Transparent electrode 2, a Hole injection layer 3, a Hole transport layer 4, an Electroluminescent layer 5, an Electron Transport layer 6, an Electron injection layer 7 and an Al cathode layer 8.

The term "alkyl", "alkoxy" described herein and any substituents comprising "alkyl" moiety include both linear and branched species.

The term "aryl" described herein means an organic radical derived from aromatic hydrocarbon via elimination of one hydrogen atom. Each ring comprises a monocyclic or fused ring system containing from 4 to 7, preferably from 5 to 6 cyclic atoms. Specific examples include phenyl, naphthyl, biphenyl, anthryl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S as the aromatic cyclic backbone atom(s), and carbon atom(s) for remaining aromatic cyclic backbone atoms. The heteroaryl may be a 5- or 6-membered monocyclic heteroaryl or a polycyclic heteroaryl which is fused with one or more benzene ring(s), and may be partially saturated. The heteroaryl group may comprise a bivalent aryl group, of which the heteroatoms may be oxidized or quaternized to form N-oxide and quaternary salt. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The term "saturated 5- or 6-membered heterocyclic amino" described herein refers a compound containing nitrogen as a cyclic atom of a 5- or 6-membered ring comprised of saturated bonding, which may further comprise one or more heteroatom(s) selected from N, O and S.

The organic electroluminescent compounds of Chemical Formula (1) are characterized by their structure of novel concept which maximizes luminous efficiency of green electroluminescent devices resulted from those compounds and their device life, being unexpected by conventional inventions.

The organic electroluminescent compounds of Chemical Formula (1) according to the invention adopted a structure showing an efficient energy transfer mechanism between the host and the dopant, which can realize electroluminescent property with a reliably high efficiency on the basis of improvement in electron density distribution. The structure of the novel compounds according to the present invention can provide a skeletal which can also tune an electroluminescent property with high efficiency in the range from blue to red, not only for green electroluminescence. Beyond the concept of using a host material with high electron conductivity such as Alq, the invention applies a host having appropriate balance of hole conductivity and electron conductivity, thereby overcoming the problems of conventional materials including low initial efficiency and short lifetime, and ensures electroluminescent properties with high performance having high efficiency and long life for each color.

The naphthyl of Chemical Formula (1) may be 1-naphthyl or 2-naphthyl; the anthryl may be 1-anthryl, 2-anthryl or 9-anthryl; and the fluorenyl may be 1-fluorenyl, 2-fluorenyl, 3-fluorenyl, 4-fluorenyl or 9-fluorenyl.

The substituents comprising "(C1-C60)alkyl" moiety described herein may contain 1 to 60 carbon atoms, 1 to 20 carbon atoms, or 1 to 10 carbon atoms. The substituents comprising "(C6-C60)aryl" moiety may contain 6 to 60 carbon atoms, 6 to 20 carbon atoms, or 6 to 12 carbon atoms. The substituents comprising "(C3-C60)heteroaryl" moiety may contain 3 to 60 carbon atoms, 4 to 20 carbon atoms, or 4 to 12 carbon atoms.

In Chemical Formula (1), R₁ and R₂ are independently selected from hydrogen, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, benzyl, trityl, ethenyl, phenylethenyl, ethynyl, phenylethynyl, cyclopentyl, cyclohexyl, cycloheptyl, cyclooctyl, trimethylsilyl, triethylsilyl, tripropylsilyl, tri(t-butyl)silyl, t-butyldimethylsilyl, dimethylphenylsilyl, triphenylsilyl, bicyclo[1.1.0]butyl, bicyclo[1.1.1]pentyl, bicyclo[2.1.0]pentyl, bicyclo[2.1.1]hexyl, bicyclo[3.2.0]hexyl, bicyclo[3.2.0]heptyl, bicyclo[3.1.1]heptyl, bicyclo[4.1.0]heptyl, bicyclo[2.2.1]heptyl, octahydropentalenyl, bicyclo[2.2.2]octyl, bicyclo[4.2.0]octyl, bicyclo[4.1.1]octyl, bicyclo[3.2.1]octyl, octahydro-1H-indenyl, bicyclo[5.2.0]nonyl, bicyclo[4.2.1]nonyl, bicyclo[3.3.1]nonyl, bicyclo[3.3.2]decyl, bicyclo[4.3.1]decyl, bicyclo[4.2.2]decyl, decahydronaphthalenyl, bicyclo[3.3.3]undecyl, bicyclo[4.3.2]undecyl, bicyclo[4.3.3]dodecyl, 4-pentylbicyclo[2.2.2]octyl, tricyclo[2.2.1.0]heptyl, tricyclo[5.2.1.0^{2,6}]decyl, tricyclo[5.3.1.1]dodecyl, tricyclo[5.4.0.0^{2,9}]undecyl, adamantyl, tricyclo[5.3.2.0^{4,9}]dodecyl, tricyclo[4.4.1.1^{1,5}]dodecyl, tricyclo[5.5.1.0^{3,11}]tridecyl, phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl or benzoxazolyl; and
the phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl or benzoxazolyl may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl and triphenylsilyl.

In Chemical Formula (1), Ar₁ and Ar₂ independently represent phenyl, naphthyl, fluorenyl, anthryl, phenanthryl, pyrenyl, perylenyl, fluoranthenyl, pyridyl, pyrrolyl, furanyl, thiophenyl, imidazolyl, benzimidazolyl, pyrazinyl, pyrimidinyl, quinolyl, benzofuranyl, benzothiophenyl, pyrazolyl, indolyl, carbazolyl, thiazolyl, oxazolyl, benzothiazolyl, benzoxazolyl, morpholino or thiomorpholino; and
the phenyl, naphthyl, fluorenyl, anthryl, phenanthryl, pyrenyl, perylenyl, fluoranthenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl, benzoxazolyl, morpholino or thiomorpholino may be further substituted by one or more substituent(s) selected from a group consisting of methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopentyl, cyclohexyl, cycloheptyl, cyclooctyl, fluoro, cyano, trimethylsilyl, triethylsilyl, tripropylsilyl, tri(t-butyl)silyl, t-butyldimethylsilyl, dimethylphenylsilyl, triphenylsilyl, phenyl, biphenyl, fluorenyl, naphthyl and anthryl.

Group 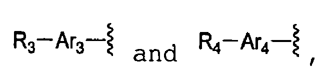 of which the total number of carbons is from 21 to 60, may be independently selected from the following structures, but are not restricted thereto: 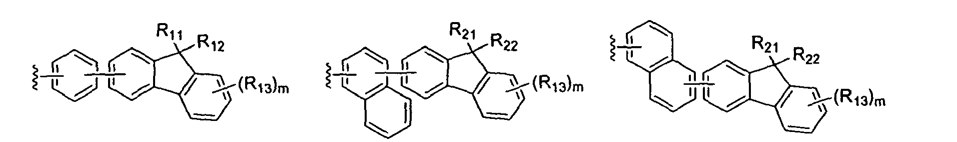 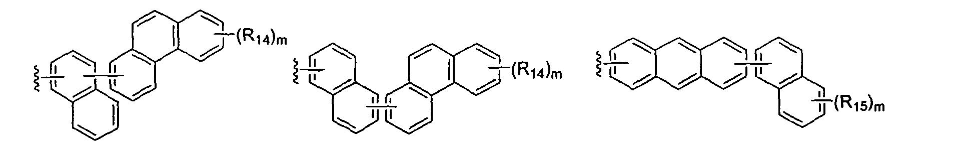 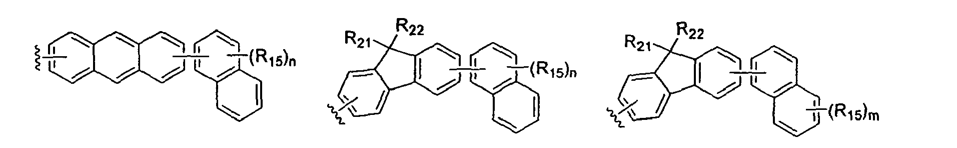 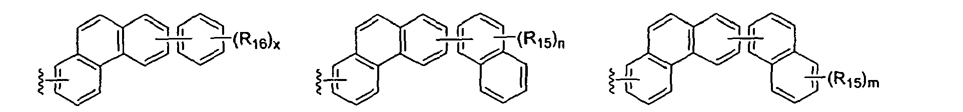 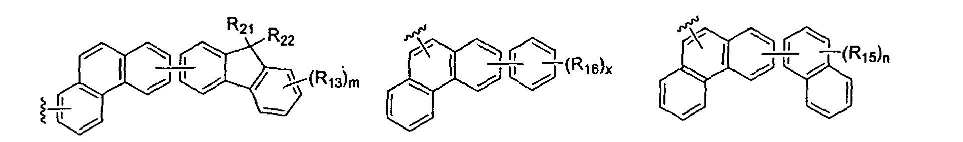 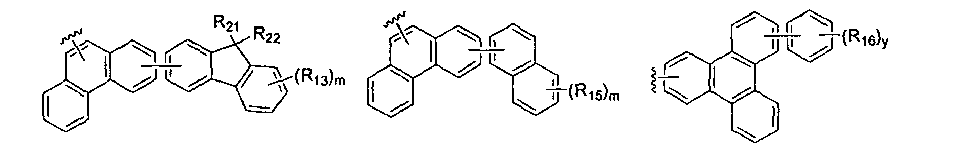 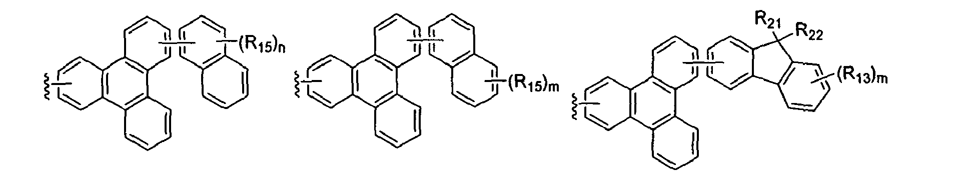 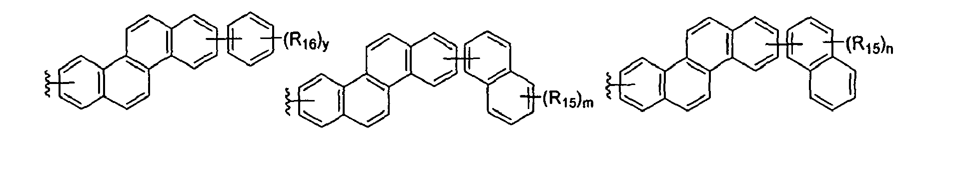 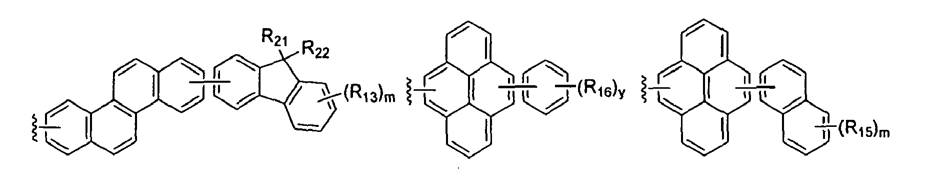 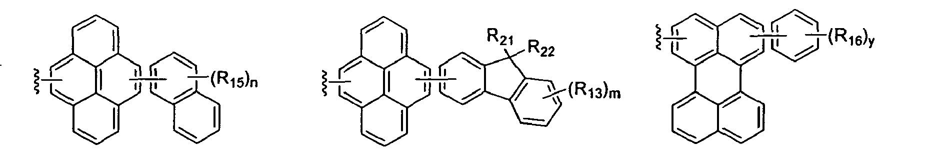 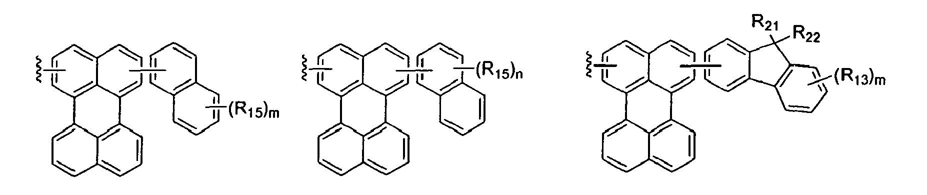 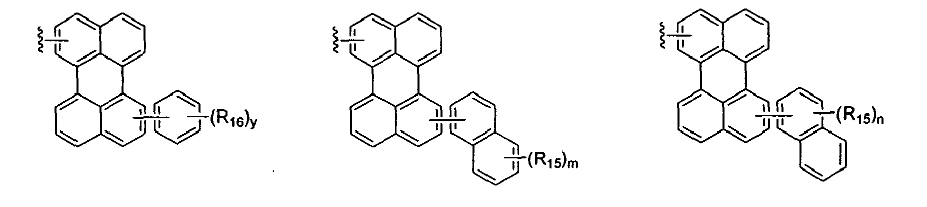 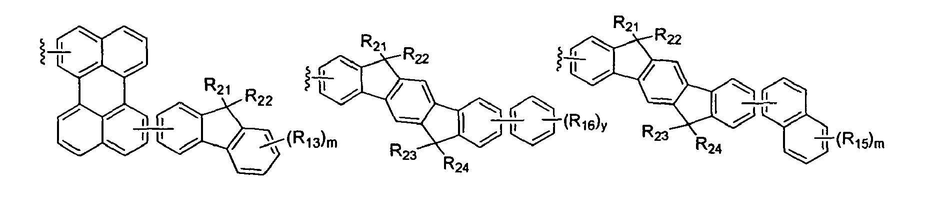 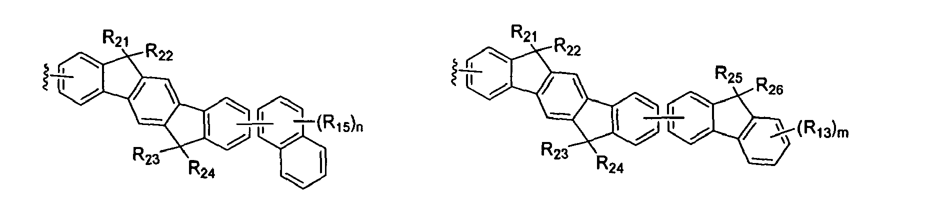 wherein, R₁₁ through R₁₆ independently represent (C1-C20)alkyl or (C6-C20)aryl;
R₂₁ through R₂₆ independently represent hydrogen, (C1-C20)alkyl or (C6-C20)aryl; the aryl of R₁₁ through R₁₆ and R₂₁ through R₂₆ may be further substituted by deuterium or (C1-C20)alkyl;
m is an integer from 0 to 4;
n is an integer from 0 to 3;
x is an integer from 1 to 5; and
y is an integer from 0 to 5.

The organic electroluminescent compounds according to the present invention can be specifically exemplified by the following compounds, but they are not restricted thereto: 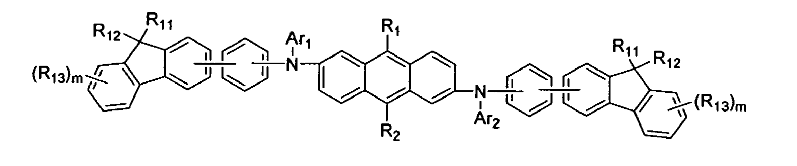 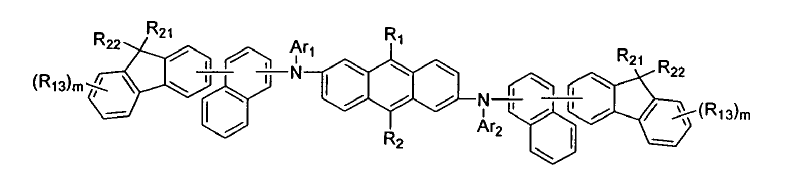 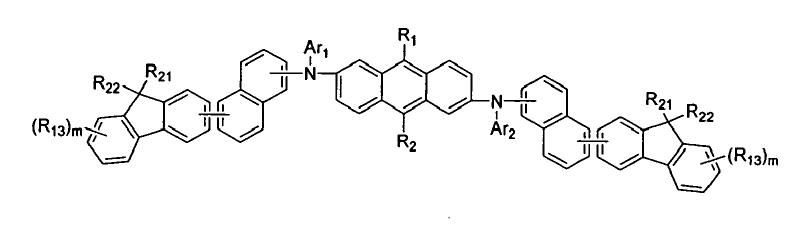 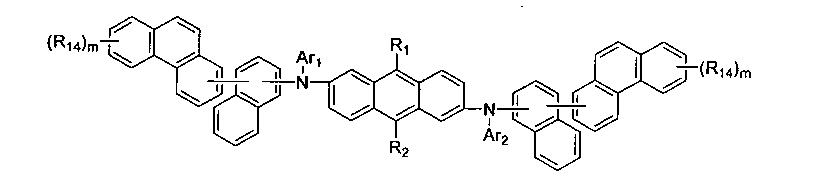 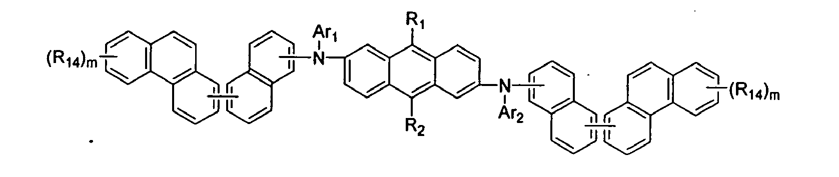 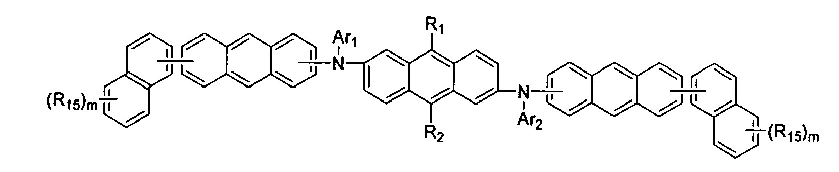 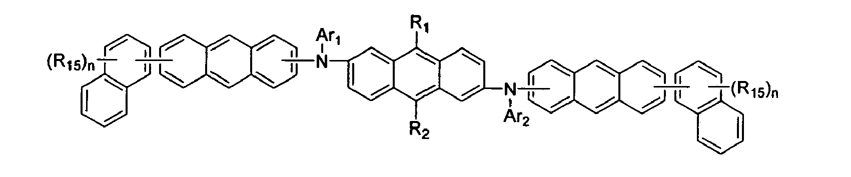 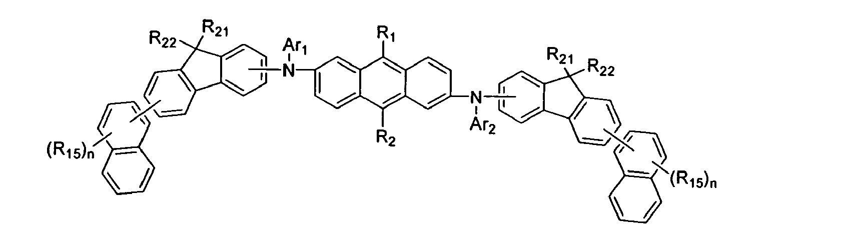 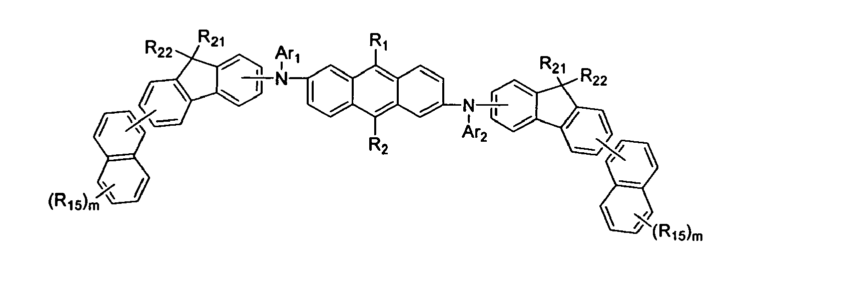 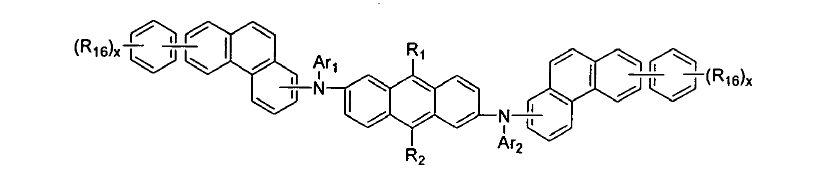 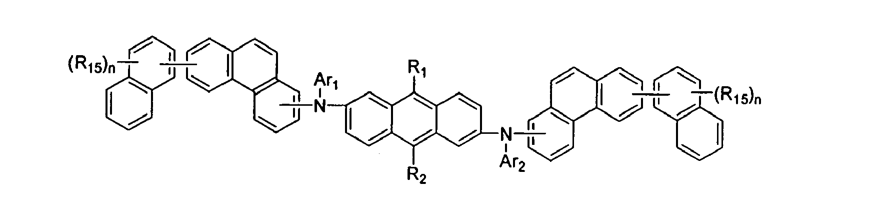 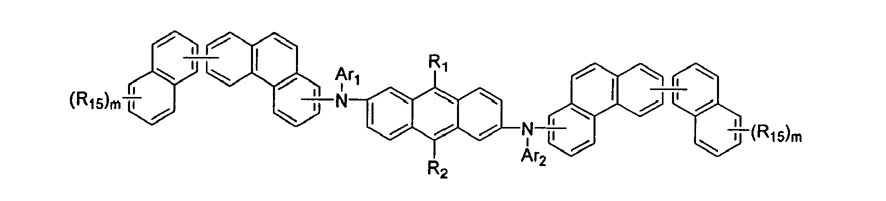 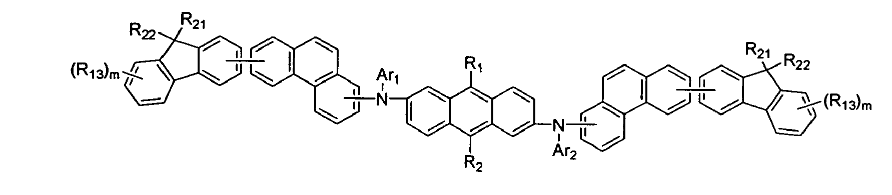 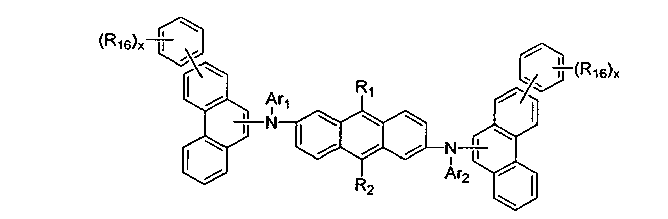 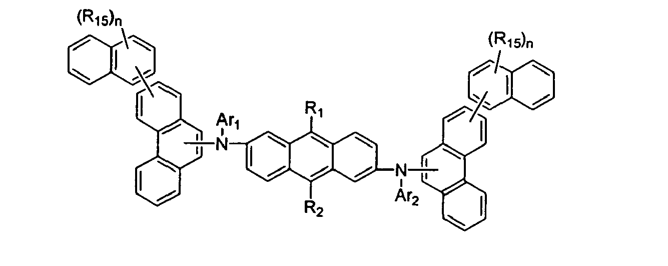 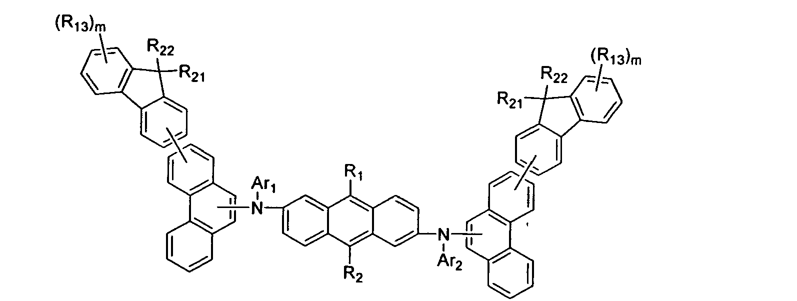 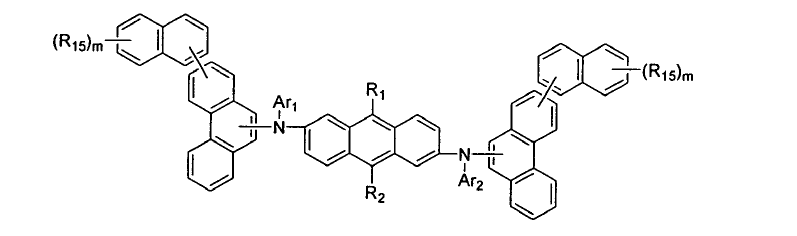 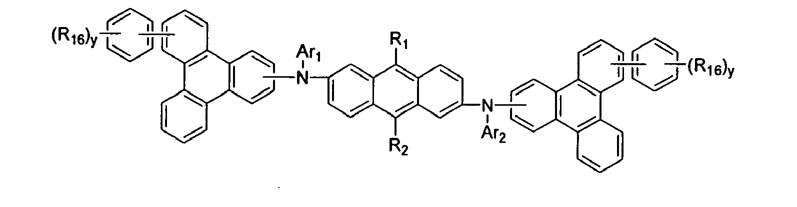 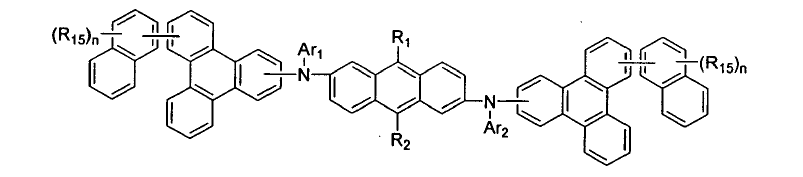 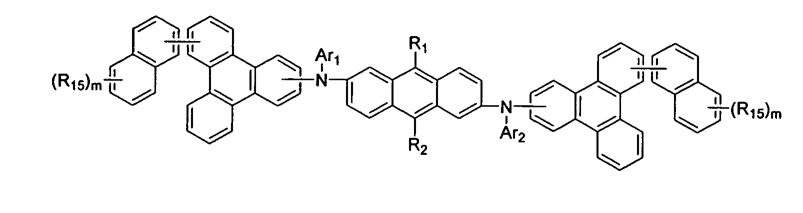 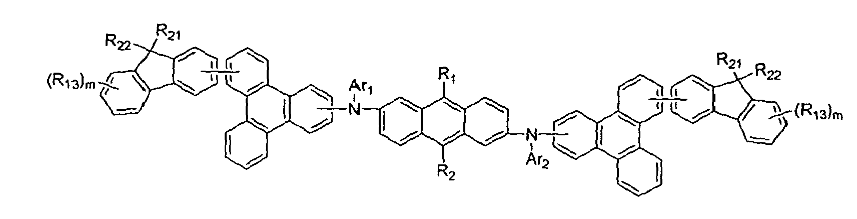 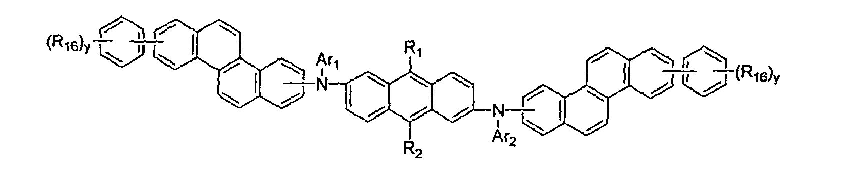 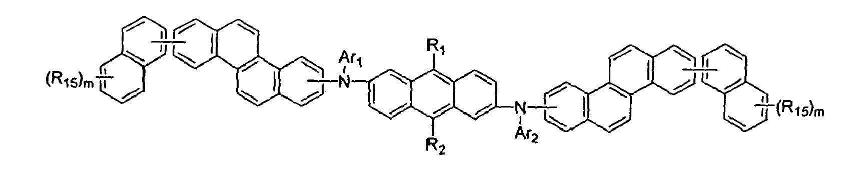 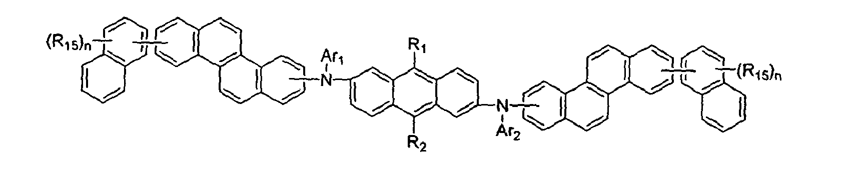 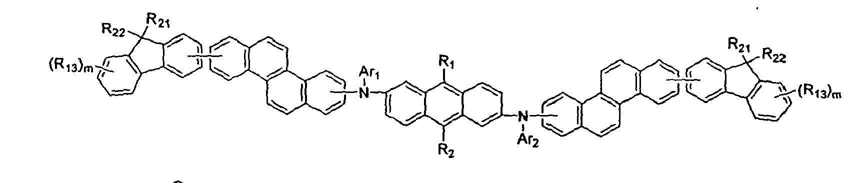 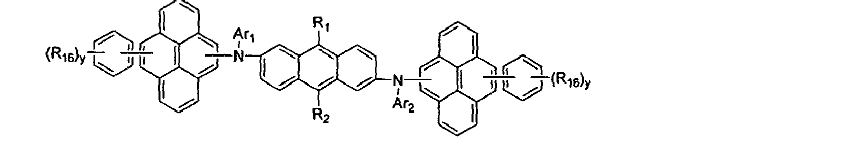 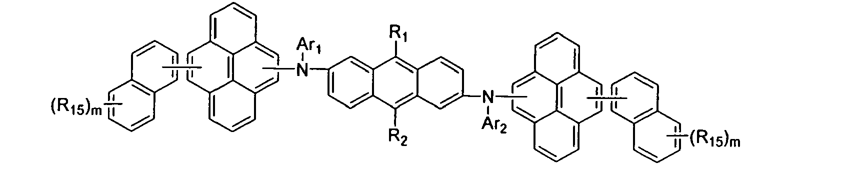 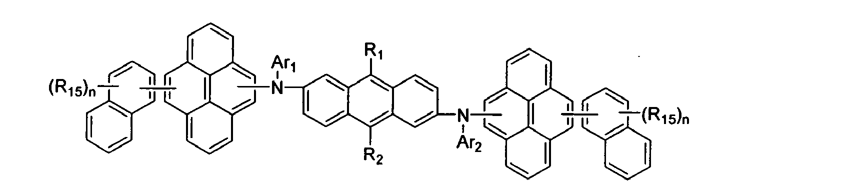 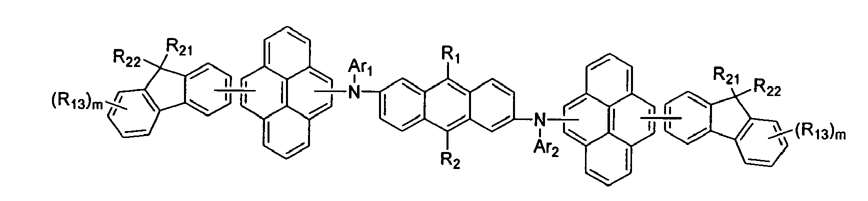 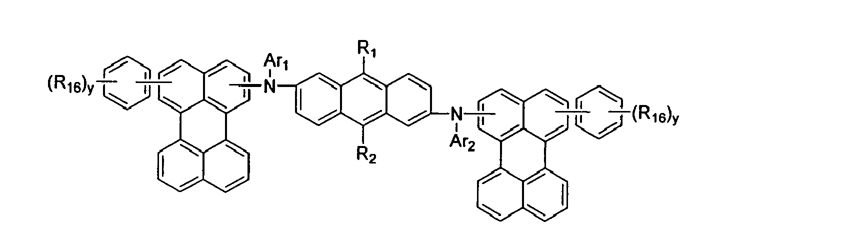 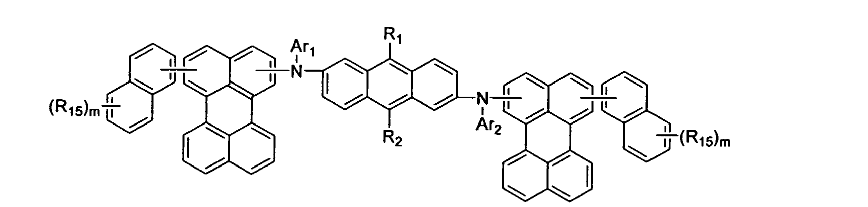 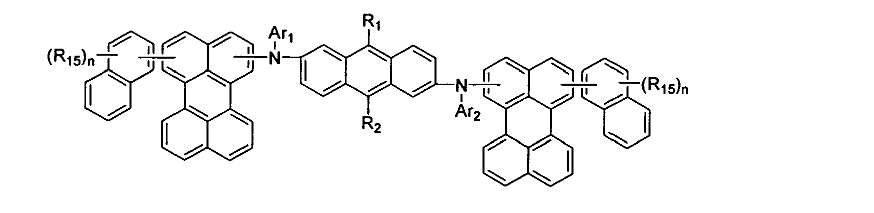 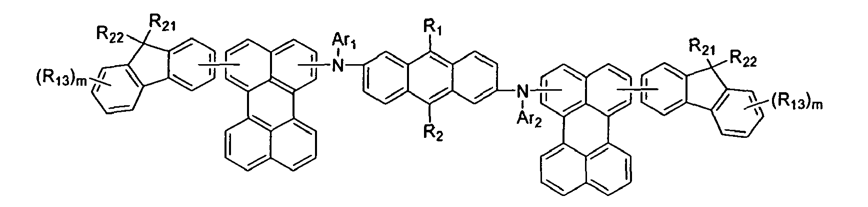 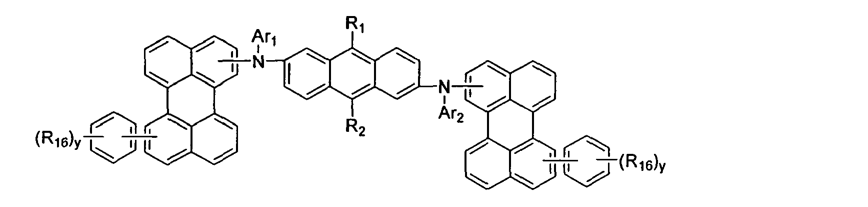 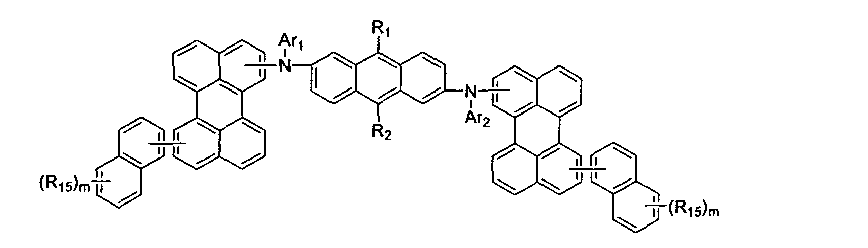 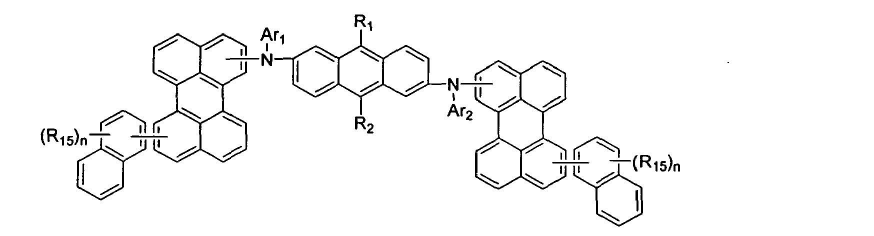 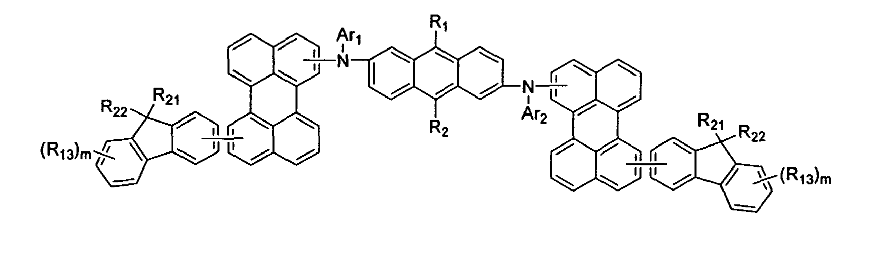 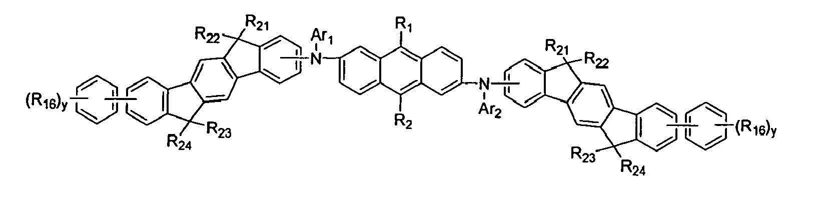 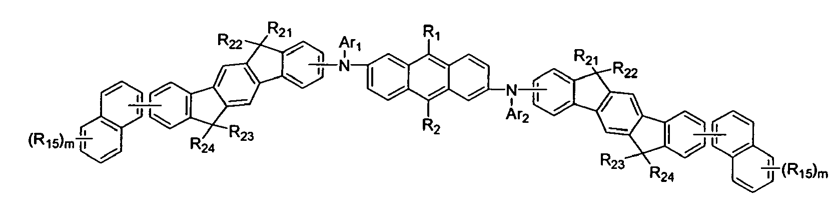 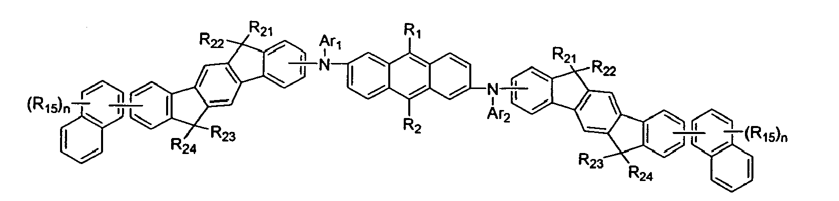 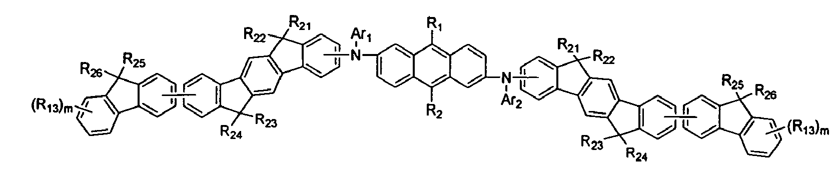 wherein, R₁ and R₂ independently represent hydrogen, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, benzyl, trityl, ethenyl, phenylethenyl, ethynyl, phenylethynyl, cyclopentyl, cyclohexyl, cycloheptyl, cyclooctyl, trimethylsilyl, triethylsilyl, tripropylsilyl, tri(t-butyl)silyl, t-butyldimethylsilyl, dimethylphenylsilyl, triphenylsilyl, bicyclo[1.1.0]butyl, bicyclo[1.1.1]pentyl, bicyclo[2.1.0]pentyl, bicyclo[2.1.1]hexyl, bicyclo[3.2.0]hexyl, bicyclo[3.2.0]heptyl, bicyclo[3.1.1]heptyl, bicyclo[4.1.0]heptyl, bicyclo[2.2.1]heptyl, octahydropentalenyl, bicyclo[2.2.2]octyl, bicyclo[4.2.0]octyl, bicyclo[4.1.1]octyl, bicyclo[3.2.1]octyl, octahydro-1*H*-indenyl, bicyclo[5.2.0]nonyl, bicyclo[4.2.1]nonyl, bicyclo[3.3.1]nonyl, bicyclo[3.3.2]decyl, bicyclo[4.3.1]decyl, bicyclo[4.2.2]decyl, decahydronaphthalenyl, bicyclo[3.3.3]undecyl, bicyclo[4.3.2]undecyl, bicyclo[4.3.3]dodecyl, 4-pentylbicyclo[2.2.2]octyl, tricyclo[2.2.1.0]heptyl, tricyclo[5.2.1.0^{2.6}]decyl, tricyclo[5.3.1.1]dodecyl, tricyclo[5.4.0.0^{2.9}]undecyl, adamantyl, tricyclo[5.3.2.0^{4.9}]dodecyl, tricyclo[4.4.1.1^{1.5}]dodecyl, tricyclo[5.5.1.0^{3.11}]tridecyl, phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl or benzoxazolyl; and
the phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl or benzoxazolyl of R₁ and R₂ may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl and triphenylsilyl;
Ar₁ and Ar₂ independently represent phenyl, naphthyl, fluorenyl, anthryl, phenanthryl, pyrenyl, perylenyl, fluoranthenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl, benzoxazolyl, morpholino or thiomorpholino; and
the phenyl, naphthyl, fluorenyl, anthryl, phenanthryl, pyrenyl, perylenyl, fluoranthenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl, benzoxazolyl, morpholino or thiomorpholino of Ar₁ through Ar₃ may be further substituted by one or more substituent(s) selected from a group consisting of methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopentyl, cyclohexyl, cycloheptyl, cyclooctyl, fluoro, cyano, trimethylsilyl, triethylsilyl, tripropylsilyl, tri(t-butyl)silyl, t-butyldimethylsilyl, dimethylphenylsilyl, triphenylsilyl, phenyl, biphenyl, fluorenyl, naphthyl and anthryl;
R₁₁ through R₁₆ independently represent methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, phenyl, naphthyl, biphenyl, fluorenyl, phenanthryl, anthryl, fluoranthenyl, triphenylenyl, pyrenyl, chrysenyl, naphthacenyl or perylenyl;
R₂₁ through R₂₄ independently represent hydrogen, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, phenyl, naphthyl, biphenyl, fluorenyl, phenanthryl, anthryl, fluoranthenyl, triphenylenyl, pyrenyl, chrysenyl, naphthacenyl or perylenyl;
the phenyl, naphthyl, biphenyl, fluorenyl, phenanthryl, anthryl, fluoranthenyl, triphenylenyl, pyrenyl, chrysenyl, naphthacenyl or perylenyl of R₁₁ through R₁₆ and R₂₁ through R₂₄ may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl and hexadecyl;
m is an integer from 0 to 4;
n is an integer from 0 to 3;
x is an integer from 1 to 5; and
y is an integer from 0 to 5.

The organic electroluminescent compounds according to the present invention can be prepared according to the process illustrated by Reaction Schemes (1) shown below: 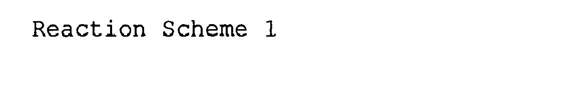 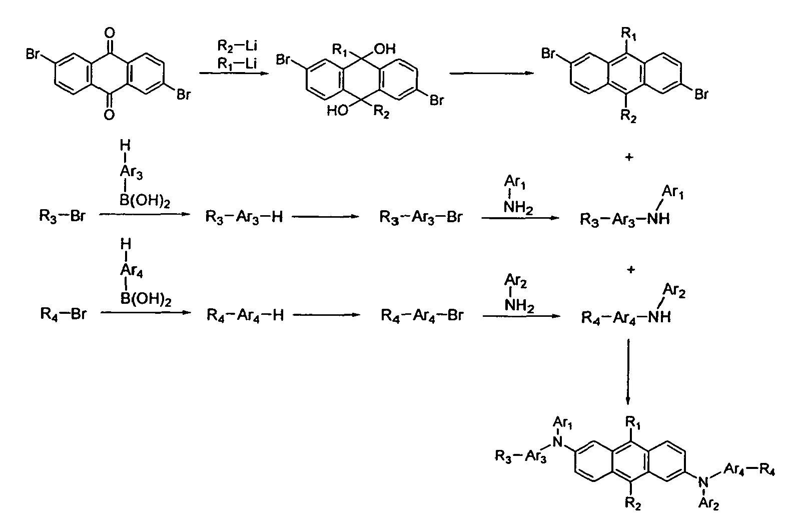 wherein, R₁, R₂, R₃, R₄, Ar₁, Ar₂, Ar₃ and Ar₄ are defined as in Chemical Formula (1).

The present invention also provides organic solar cells, which comprises one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more electroluminescent compound(s) represented by Chemical Formula (1).

The organic electroluminescent device according to the present invention is characterized in that the organic layer comprises an electroluminescent layer, which comprises one or more organic electroluminescent compound(s) represented by Chemical Formula (1) as electroluminescent dopant, and one or more host(s). The host applied to the organic electroluminescent device according to the invention is not particularly restricted, but is preferably selected from the compounds by one of Chemical Formulas (2) and (3):
Chemical Formula 2 (Ar₁₁)_{b}-L₁-(Ar₁₂)_{c}
Chemical Formula 3 (Ar₁₃)_{d}-L₂-(Ar₁₄)e
wherein, L₁ represents (C6-C60)arylene or (C4-C60)heteroarylene;
L₂ represents anthracenylene;
Ar₁₁ through Ar₁₄ are independently selected from hydrogen, (C1-C60)alkyl, (C1-C60)alkoxy, halogen, (C4-C60)heteroaryl, (C5-C60)cycloalkyl and (C6-C60)aryl; and the cycloalkyl, aryl or heteroaryl of Ar₁₁ through Ar₁₄ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
b, c, d and e independently represent an integer from 0 to 4.

The hosts represented by Chemical Formula (2) or (3) can be exemplified by anthracene derivatives or benz[a]anthracene derivatives represented by one of Chemical Formulas (4) to (7).

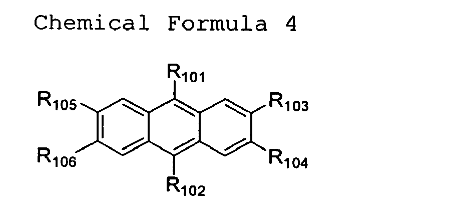

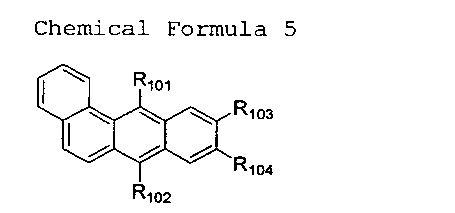

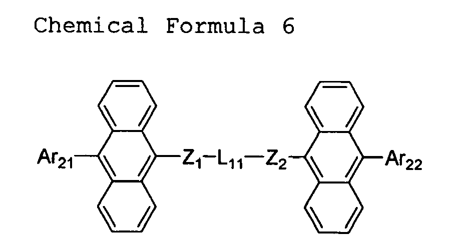

In Chemical Formulas (4) to (6),
R₁₀₁ and R₁₀₂ independently represent hydrogen, deuterium, (C1-C60)alkyl, halogen, (C6-C60)aryl, (C4-C60)heteroaryl, a 5- or 6-membered heterocycloalkyl containing one or more heteroatom(s) selected from N, O and S, or (C3-C60)cycloalkyl, and the aryl or heteroaryl of R₁₀₁ and R₁₀₂ may be further substituted by one or more substituent(s) selected from a group consisting of deuterium, (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₁₀₃ through R₁₀₆ independently represent hydrogen, deuterium, (C1-C60)alkyl, (C1-C60)alkoxy, halogen, (C4-C60)heteroaryl, (C5-C60)cycloalkyl or (C6-C60)aryl, and the heteroayl, cycloalkyl or aryl of R₁₀₃ through R₁₀₆ may be further substituted by one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
Z₁ and Z₂ independently represent a chemical bond, or (C6-C60)arylene with or without one or more substituent(s) selected from (C1-C60)alkyl, (C1-C60)alkoxy, (C6-C60)aryl, (C4-C60)heteroaryl and halogen;
Ar₂₁ and Ar₂₂ represent aryl selected from the following structures, or (C4-C60)heteroaryl: 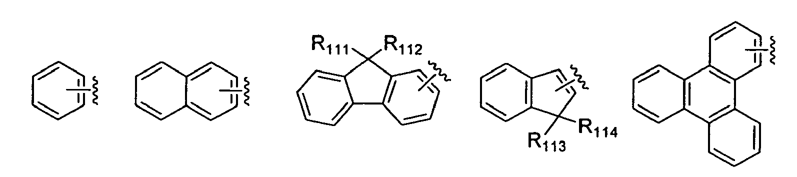 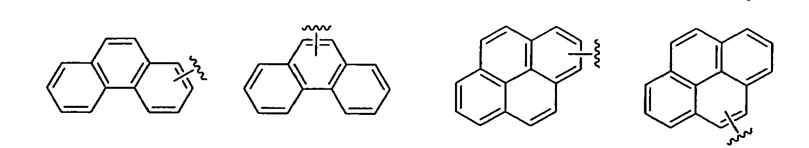 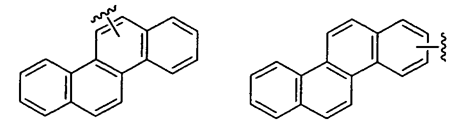 the aryl or heteroaryl of Ar₂₁ and Ar₂₂ may be substituted by one or more substituent(s) selected from deuterium, (C1-C60)alkyl, (C1-C60)alkoxy, (C6-C60)aryl and (C4-C60)heteroaryl;
L₁₁ represents (C6-C60)arylene, (C4-C60)heteroarylene or a compound represented by the following structure: 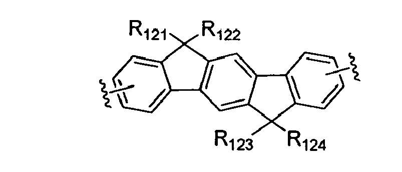 the arylene or heteroarylene of L₁₁ may be substituted by one or more substituent(s) selected from deuterium, (C1-C60)alkyl, (C1-C60)alkoxy, (C6-C60)aryl, (C4-C60)heteroaryl and halogen;
R₁₁₁, R₁₁₂, R₁₁₃ and R₁₁₄ independently represent hydrogen, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring;
R₁₂₁, R₁₂₂, R₁₂₃ and R₁₂₄ independently represent hydrogen, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

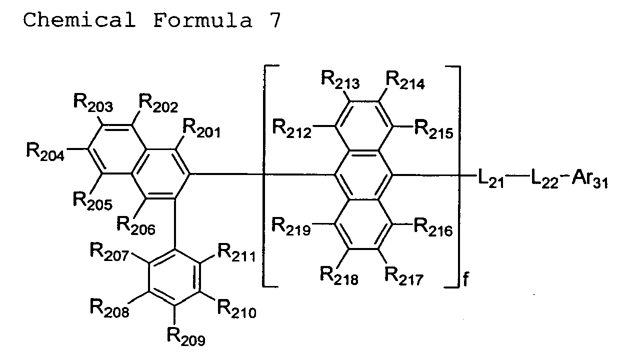

In Chemical Formula 7,
L₂₁ and L₂₂ independently represent a chemical bond, (C6-C60)arylene or (C3-C60)heteroarylene, and the arylene or heteroarylene of L₂₁ and L₂₂ may be further substituted by one or more substituent(s) selected from deuterium, (C1-C60)alkyl, halogen, cyano, (C1-C60)alkoxy, (C3-C60)cycloalkyl, (C6-C60)aryl, (C3-C60)heteroaryl, tri(C1-C30)alkylsilyl, di(C1-C30)alkyl(C6-C30)arylsilyl and tri(C6-C30)arylsilyl;
R₂₀₁ through R₂₁₉ independently represent hydrogen, deuterium, halogen, (C1-C60)alkyl, (C6-C60) aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀₁ through R₂₁₉ may be linked to an adjacent substituent via (C3-C60)alkylene or (C3-C60)alkenylene with or without a fused ring to form an alicyclic ring, or a monocyclic or polycyclic aromatic ring;
Ar₃₁ represents (C6-C60)aryl, (C4-C60)heteroaryl, a 5- or 6-membered heterocycloalkyl containing one or more heteroatom(s) selected from N, O and S, (C3-C60)cycloalkyl, adamantyl, (C7-C60)bicycloalkyl, or a substituent selected from the following structures: 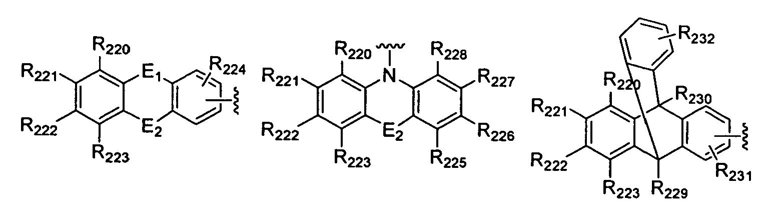 wherein, R₂₂₀ through R₂₃₂ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
E₁ and E₂ independently represent a chemical bond, - (CR₂₃₃R₂₃₄)_{g}-, -N(R₂₃₅)-, -S-, -O-, -Si(R₂₃₆)(R₂₃₇)-, -P(R₂₃₈)-, - C(=O)-, -B(R₂₃₉)-, -In(R₂₄₀)-, -Se-, -Ge(R₂₄₁) (R₂₄₂)-, - Sn(R₂₄₃) (R₂₄₄)-, -Ga(R₂₄₅)- or -(R₂₄₆)C=C(R₂₄₇)-;
R₂₃₃ through R₂₄₇ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60) alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₃₃ through R₂₄₇ may be linked to an adjacent substituent via (C3-C60)alkylene or (C3-C60)alkenylene with or without a fused ring to form an alicyclic ring, or a monocyclic or polycyclic aromatic ring;
the aryl, heteroaryl, heterocycloalkyl, adamantyl or bicycloalkyl of Ar₃₁, or the alkyl, alkenyl, alkynyl, cycloalkyl, heterocycloalkyl, aryl, heteroaryl, arylsilyl, alkylsilyl, alkylamino or arylamino of R₂₀₁ through R₂₃₂ may be further substituted by one or more substituent(s) selected from deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
f is an integer from 1 to 4; and
g is an integer from 0 to 4.

The electroluminescent layer means the layer where electroluminescence occurs, and it may be a single layer or a multi-layer consisting of two or more layers laminated. When a mixture of host-dopant is used according to the construction of the present invention, noticeable improvement in luminous efficiency could be confirmed. This can be achieved by the doping concentration of 0.5 to 10% by weight. The host according to the present invention exhibits higher hole and electron conductivity, and excellent stability of the material as compared to other conventional host materials, and provides improved device life as well as luminous efficiency.

Thus, it can be described that use of the compound represented by one of Chemical Formulas (4) to (7) as an electroluminescent host supplements electronic drawback of the organic electroluminescent compounds of Chemical Formula (1) according to the present invention.

The host compounds represented by one of Chemical Formulas (4) to (7) can be exemplified by the following compounds, but are not restricted thereto.

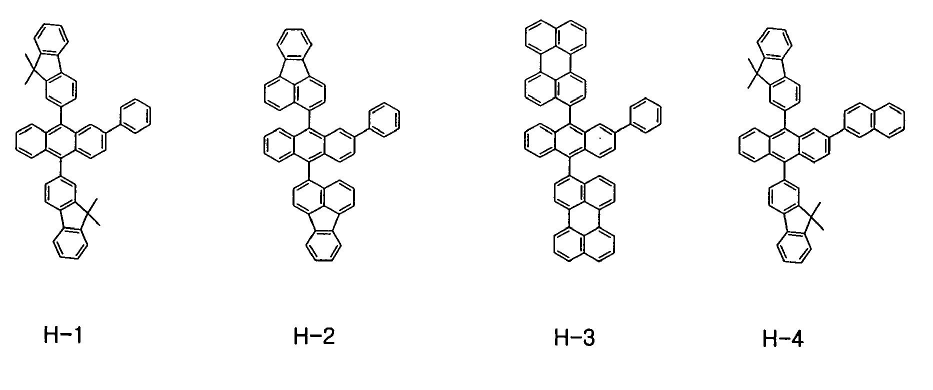

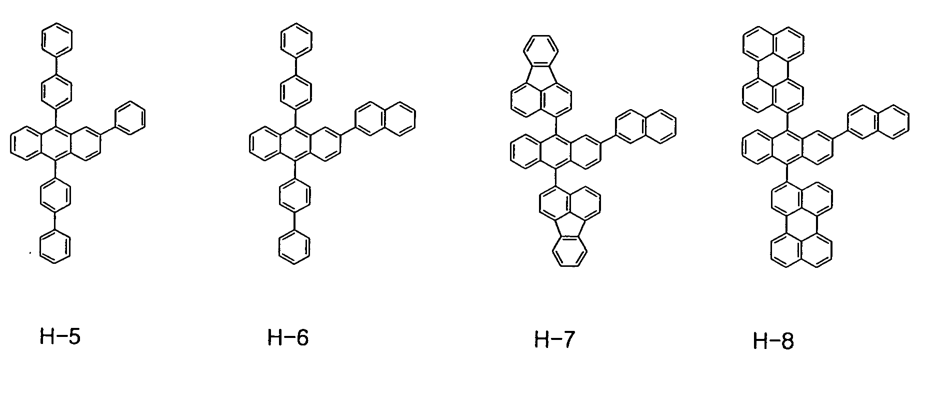

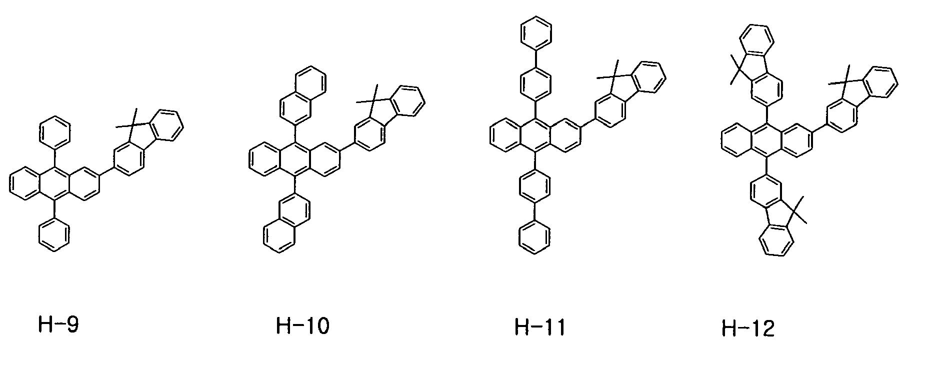

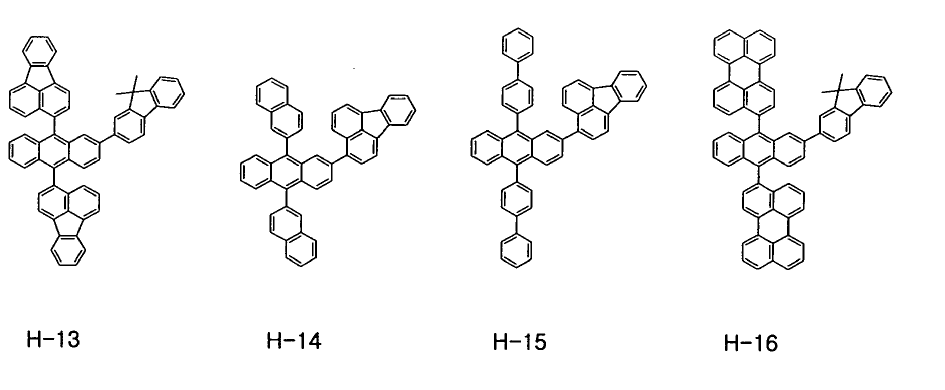

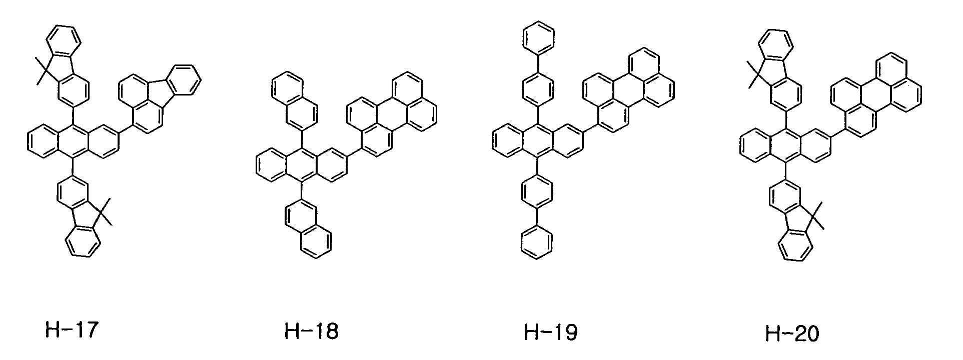

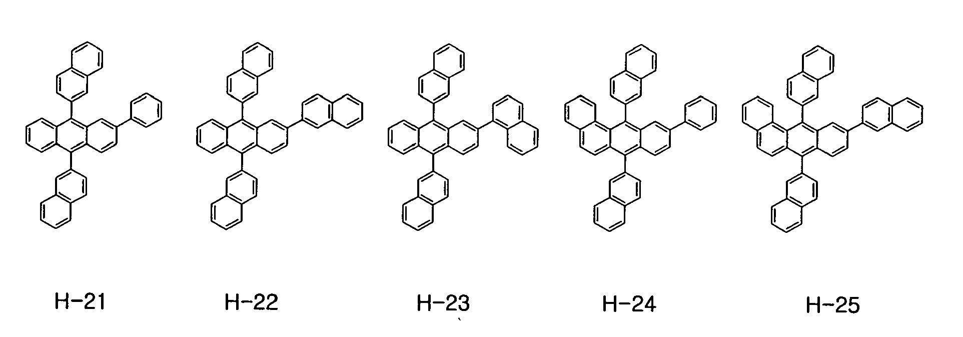

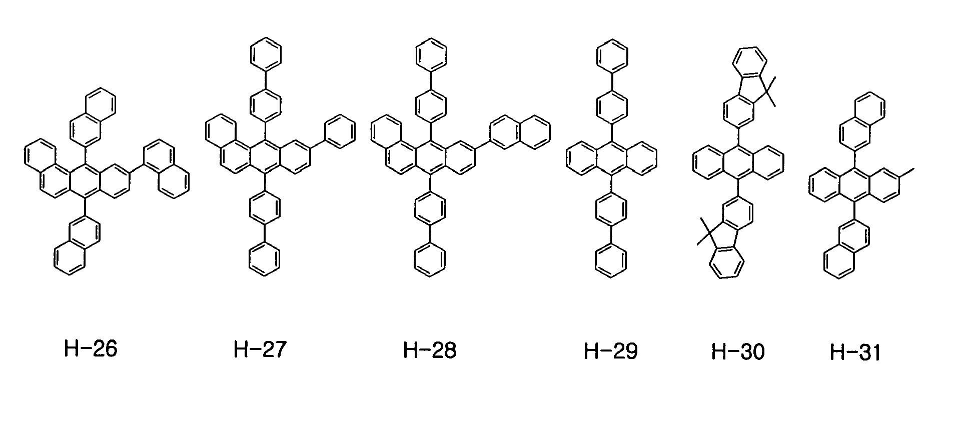

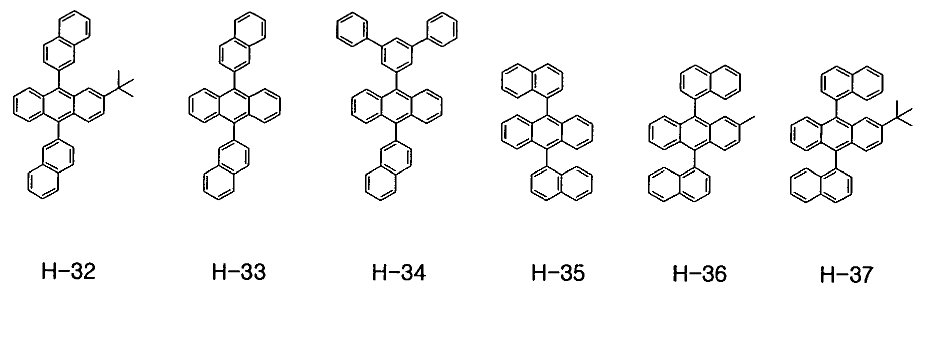

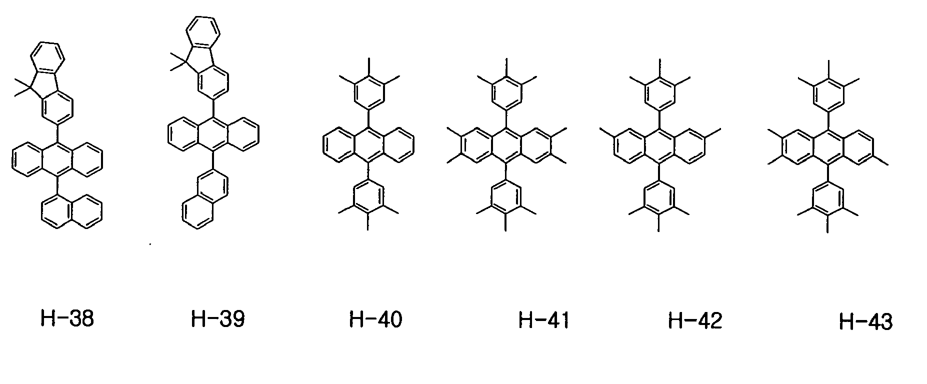

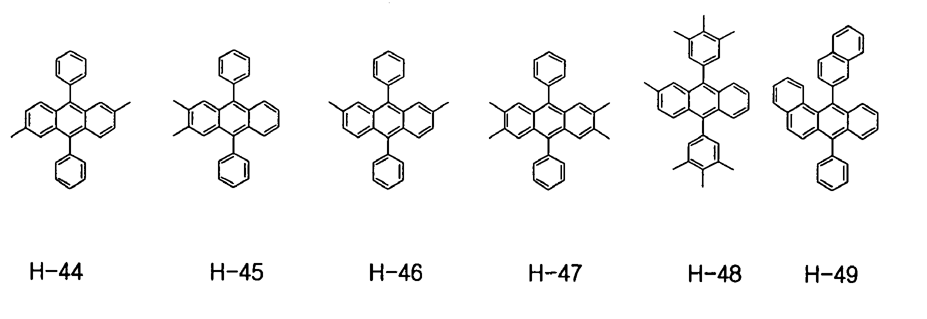

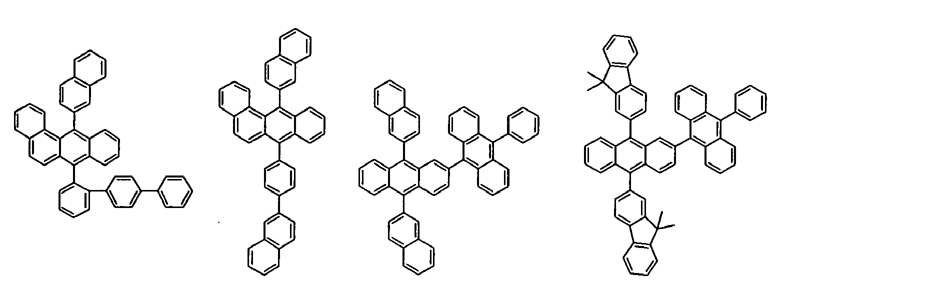

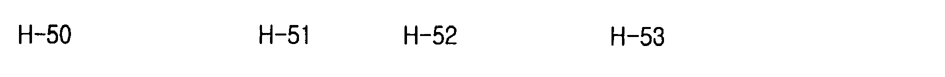

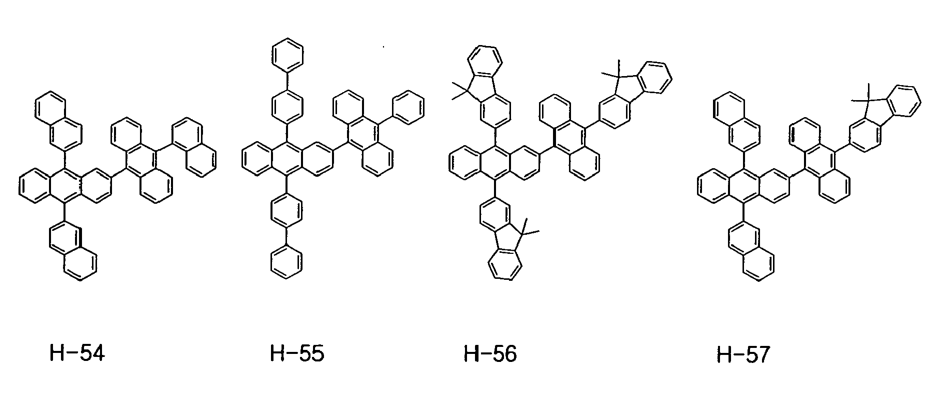

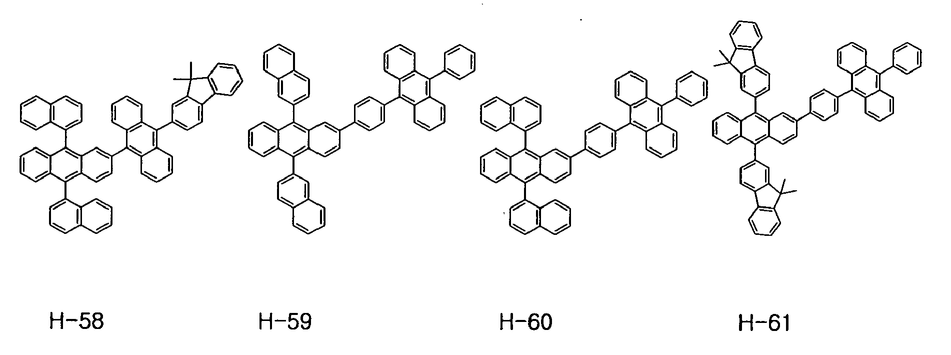

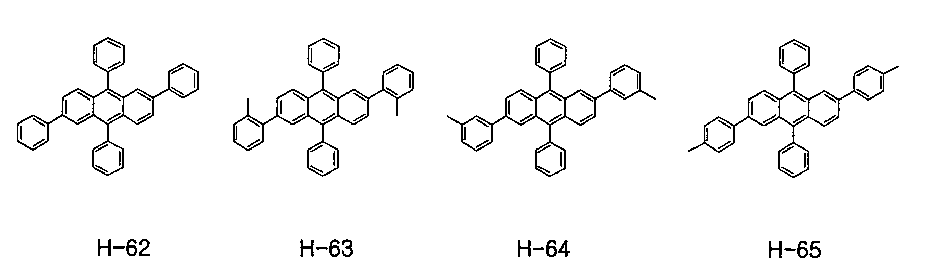

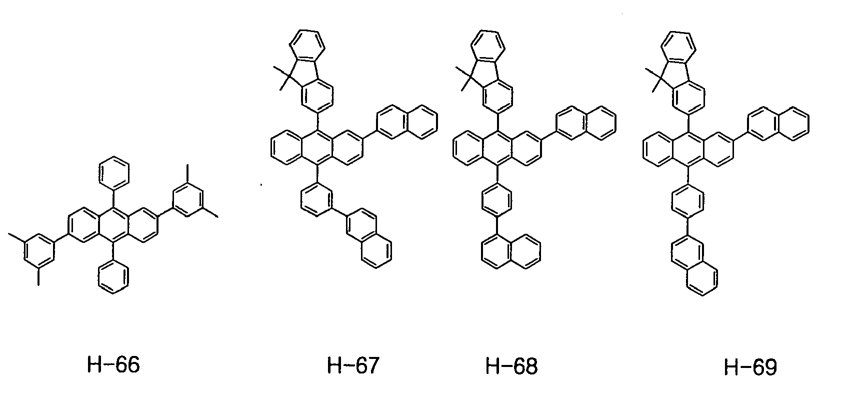

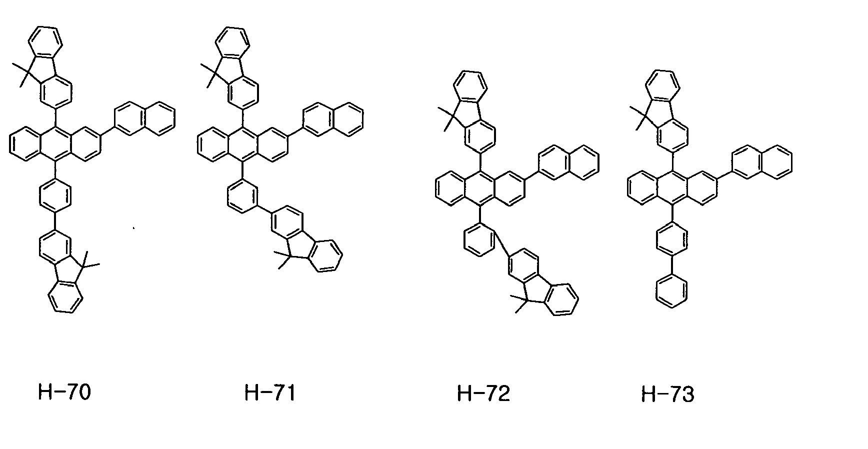

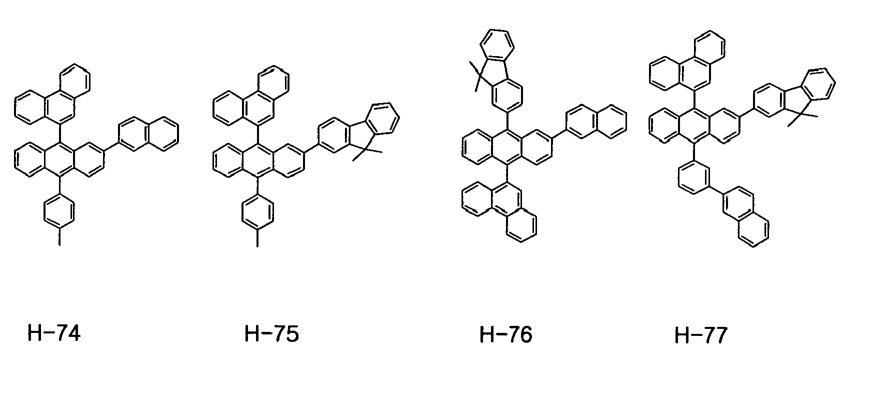

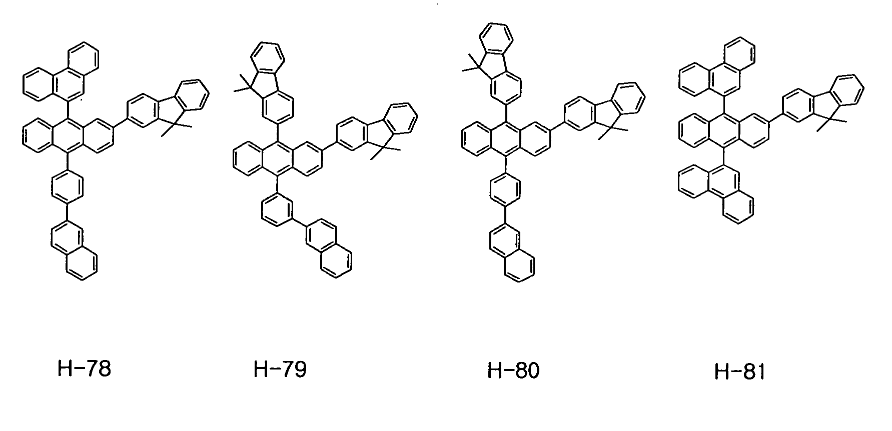

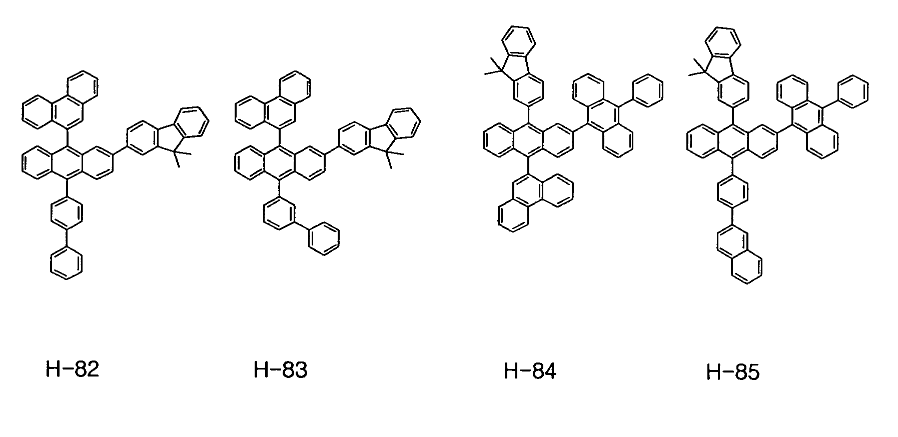

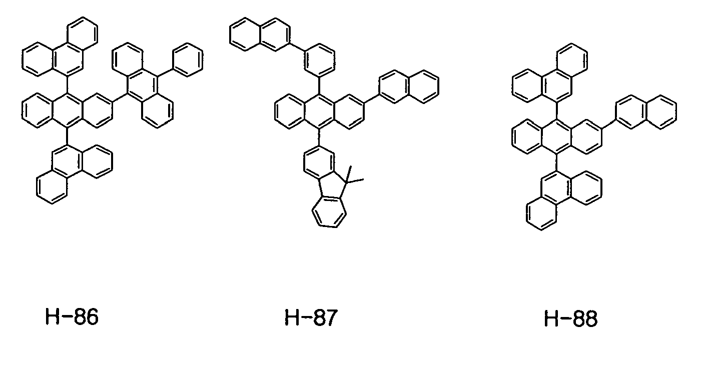

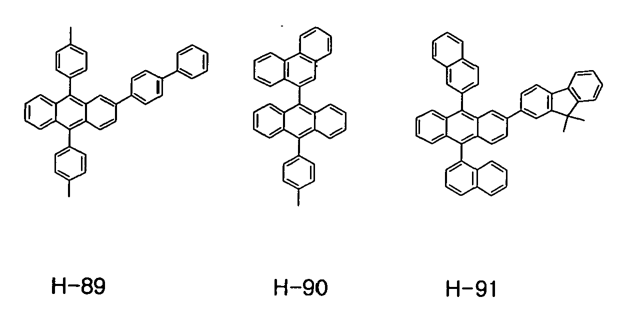

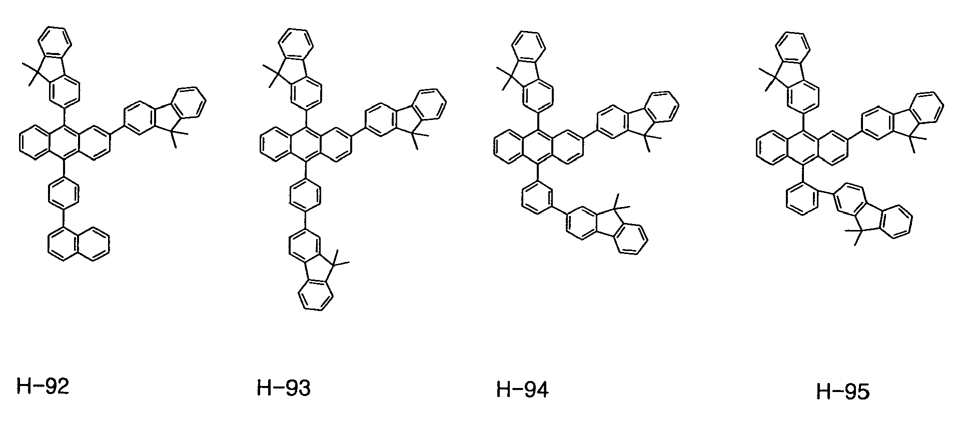

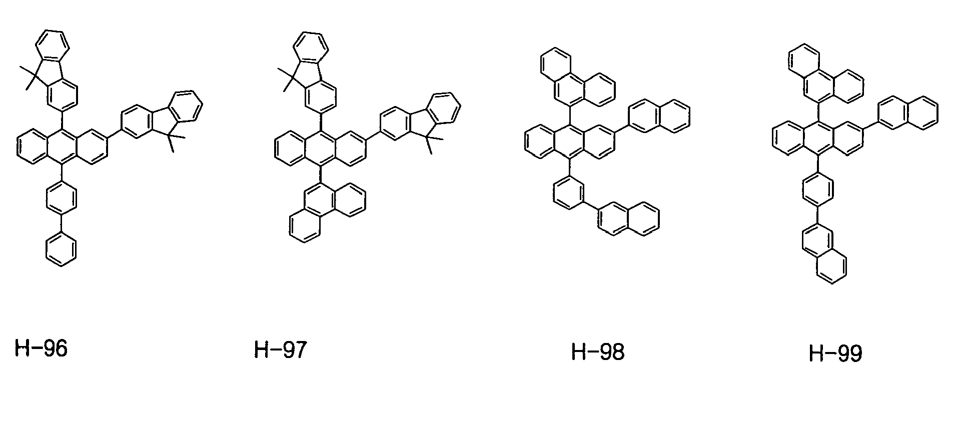

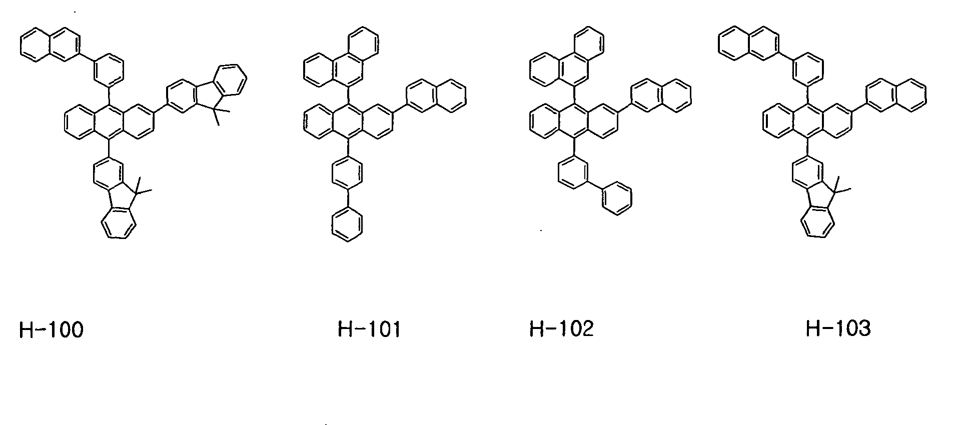

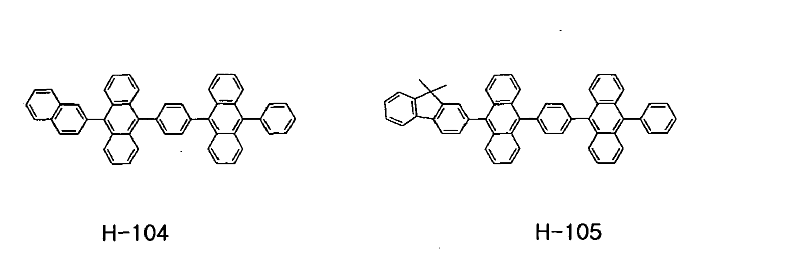

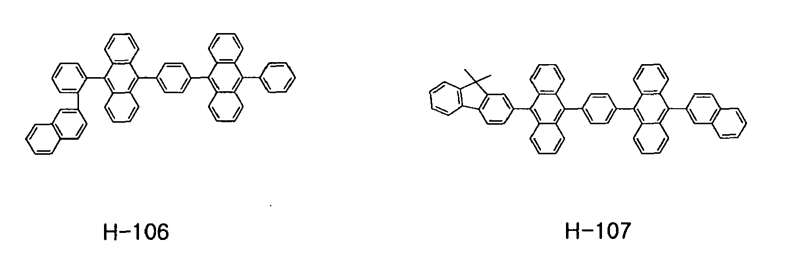

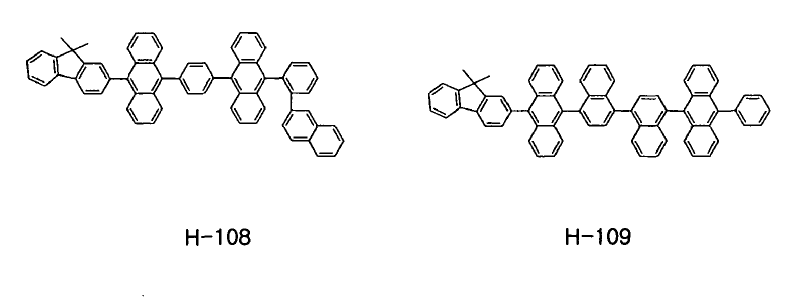

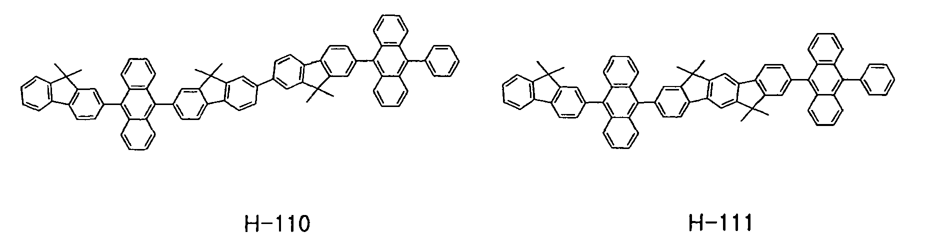

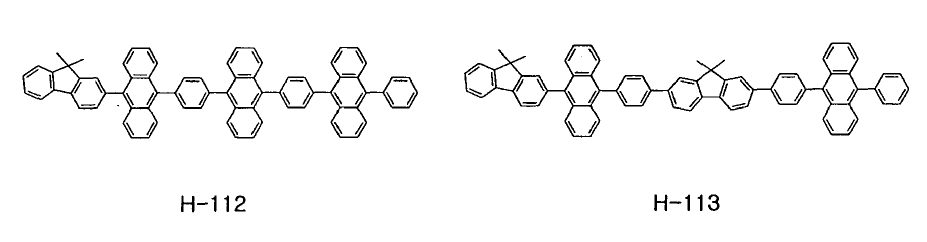

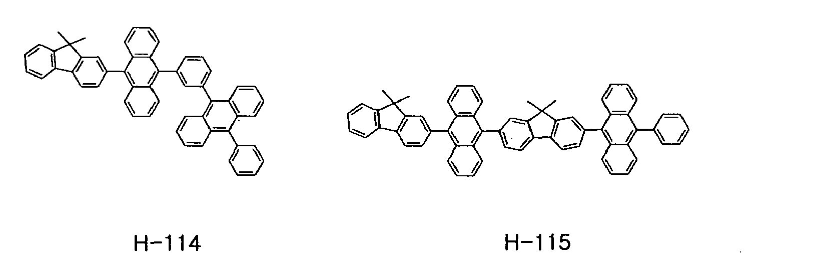

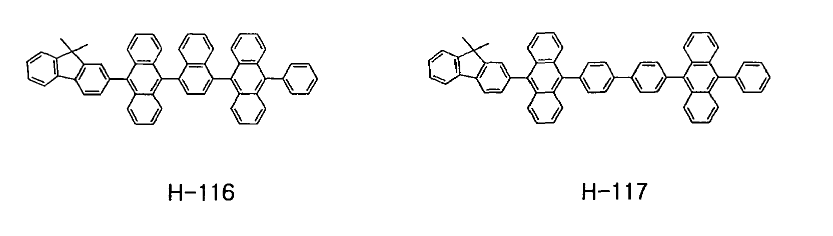

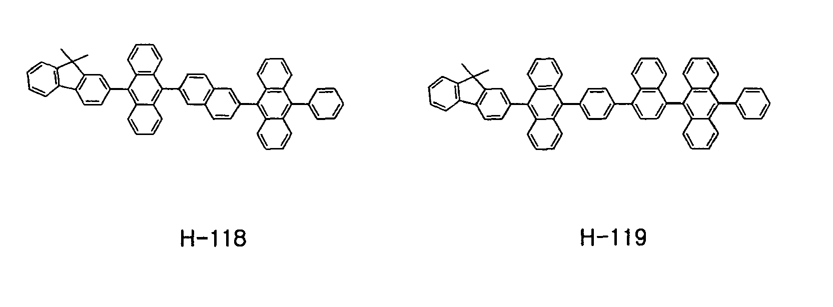

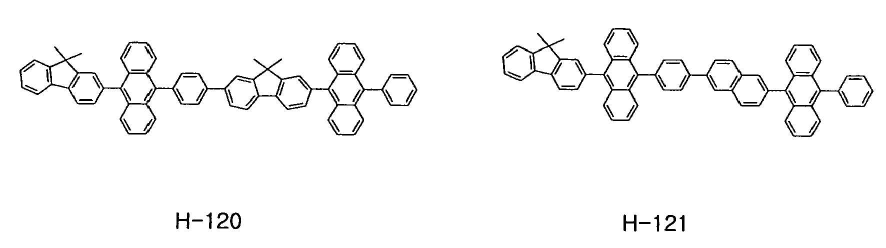

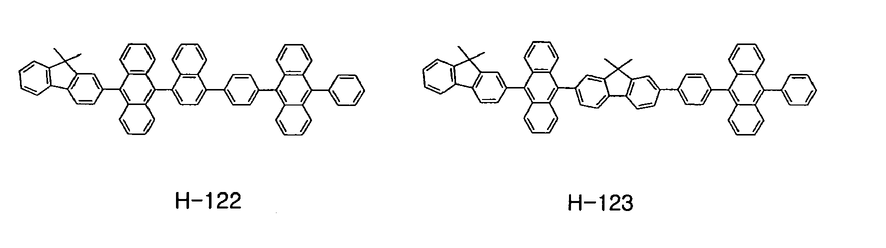

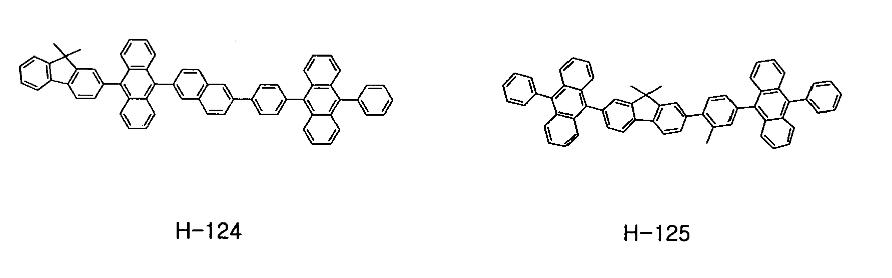

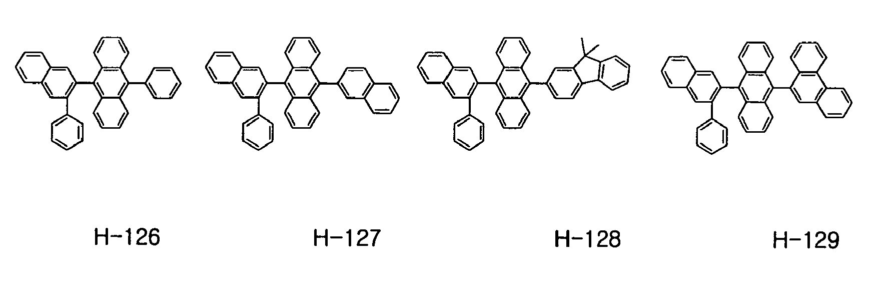

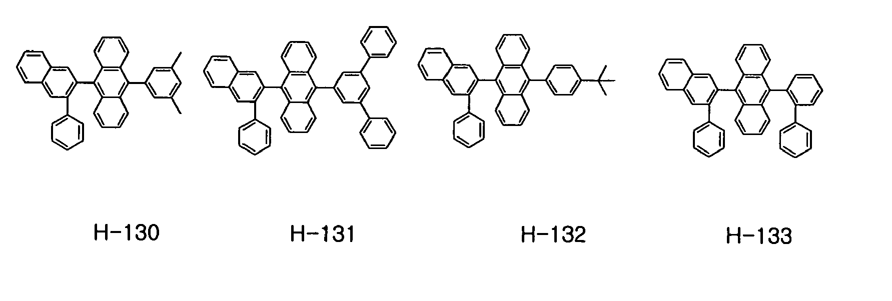

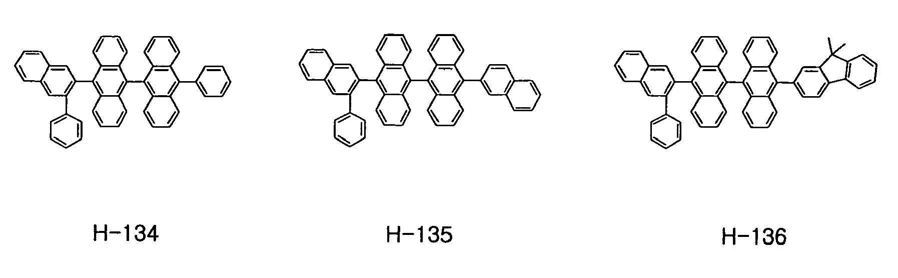

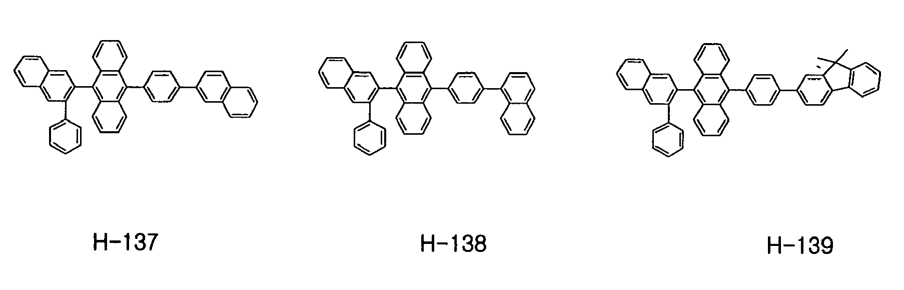

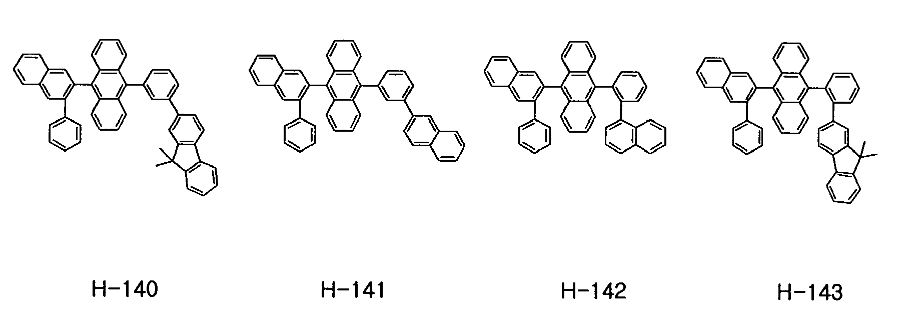

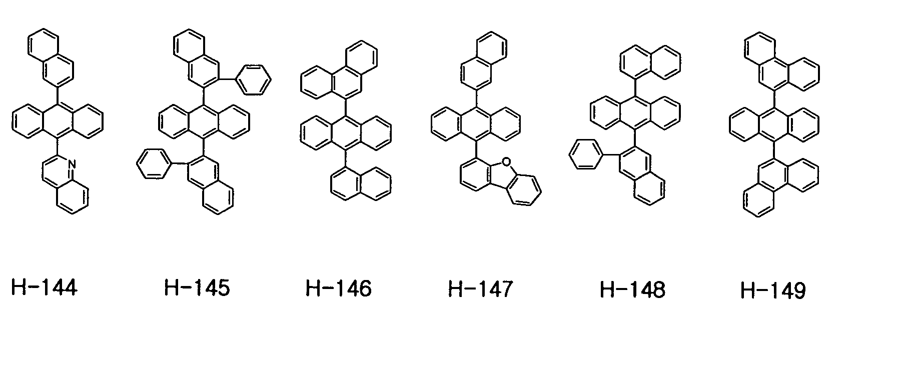

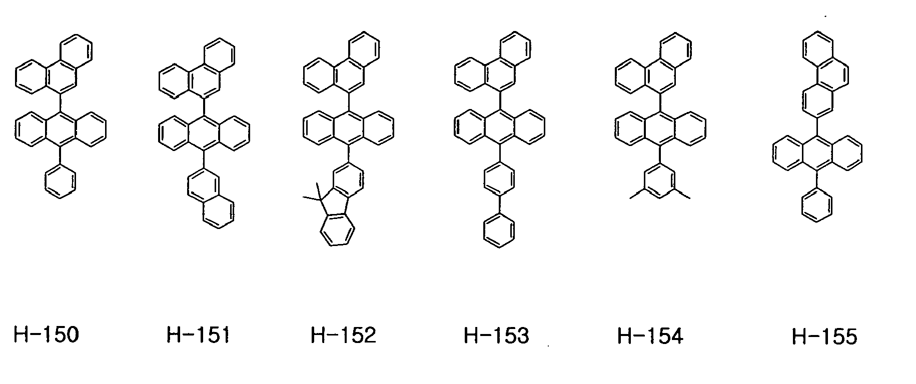

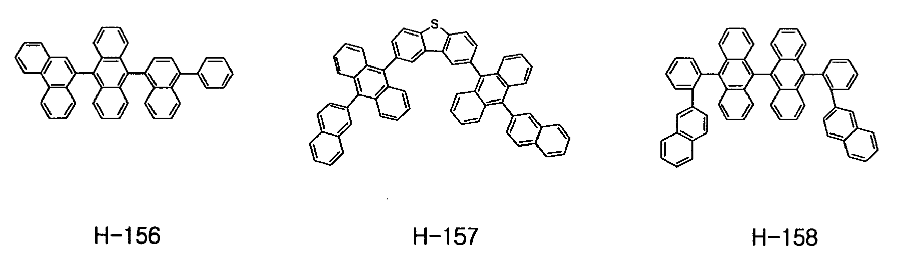

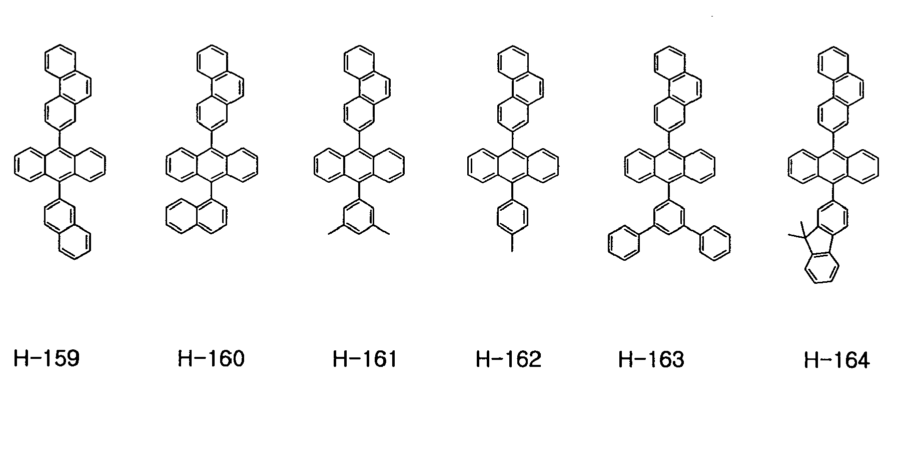

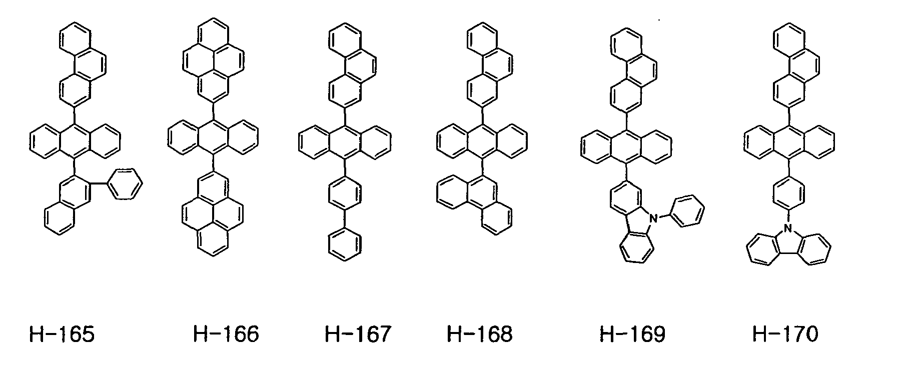

The organic electroluminescent device according to the invention may further comprise one or more compound(s) selected from arylamine compounds and styrylarylamine compounds, as well as the organic electroluminescent compound represented by Chemical Formula (1). Examples of arylamine or styrylarylamine compounds include the compounds represented by Chemical Formula (8), but they are not restricted thereto: 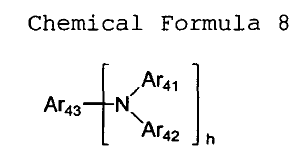 wherein, Ar₄₁ and Ar₄₂ independently represent (C1-C60)alkyl, (C6-C60)aryl, (C4-C60)heteroaryl, (C6-C60)arylamino, (C1-C60)alkylamino, a 5- or 6-membered heterocycloalkyl containing one or more heteroatom(s) selected from N, O and S, or (C3-C60)cycloalkyl, or Ar₄₁ and Ar₄₂ may be linked via (C3-C60)alkylene or (C3-C60)alkenylene with or without a fused ring to form an alicyclic ring, or a monocyclic or polycyclic aromatic ring;
when h is 1, Ar₄₃ represents (C6-C60)aryl, (C4-C60)heteroaryl, or a substituent represented by one of the following structural formulas: 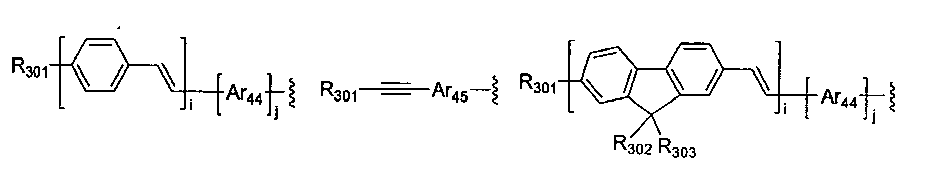 when h is 2, Ar₄₃ represents (C6-C60)arylene, (C4-C60)heteroarylene, or an arylene represented by one of the following structural formulas: 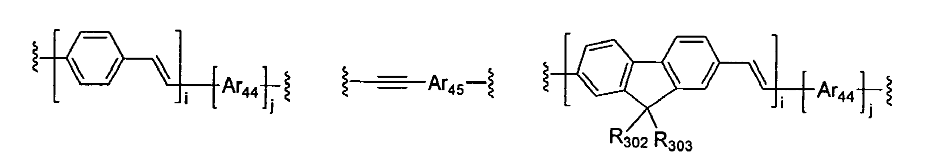 wherein Ar₄₄ and Ar₄₅ independently represent (C6-C60)arylene or (C4-C60)heteroarylene;
R₃₀₁, R₃₀₂ and R₃₀₃ independently represent hydrogen, (C1-C60)alkyl or (C6-C60)aryl;
i is an integer from 1 to 4, j is an integer of 0 or 1; and
the alkyl, aryl, heteroaryl, arylamino, alkylamino, cycloalkyl or heterocycloalkyl of Ar₄₁ and Ar₄₂, or the aryl, heteroaryl, arylene or heteroarylene of Ar₄₃, or the arylene or heteroarylene of Ar₄₄ and Ar₄₅, or the alkyl or aryl of R₃₀₁ through R₃₀₃ may be further substituted by one or more substituent(s) selected from a group consisting of deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

The arylamine compounds or styrylarylamine compounds may be more specifically exemplified by the following compounds, but are not restricted thereto.

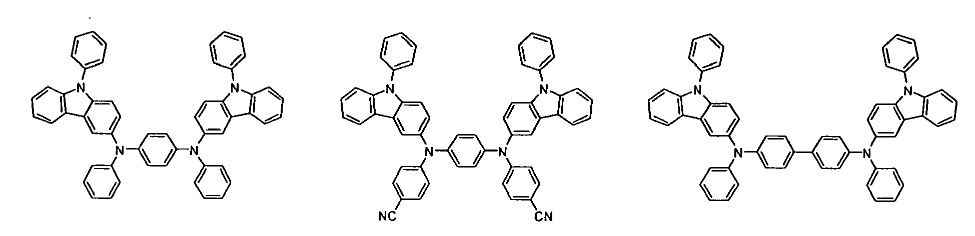

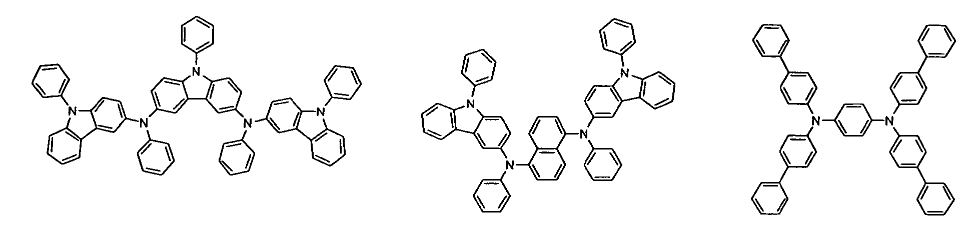

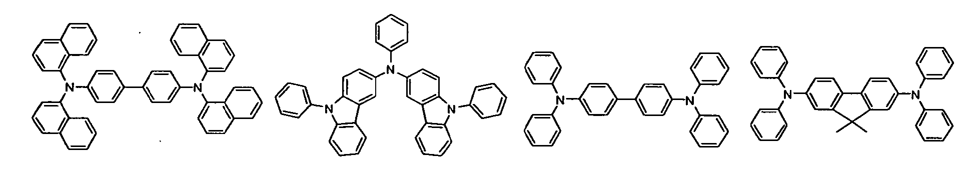

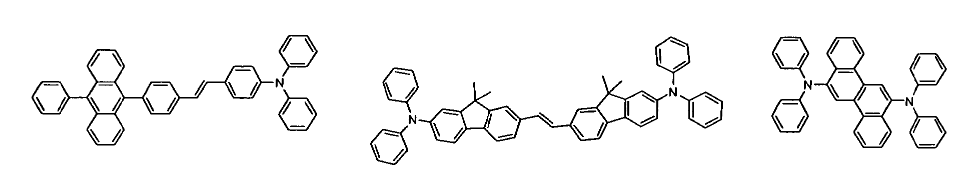

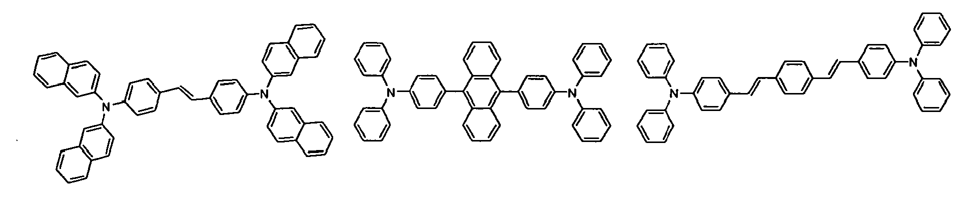

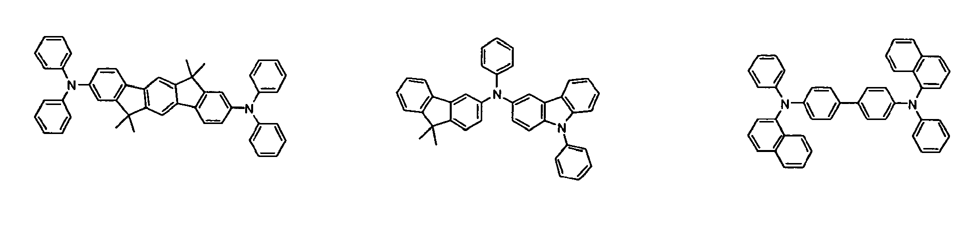

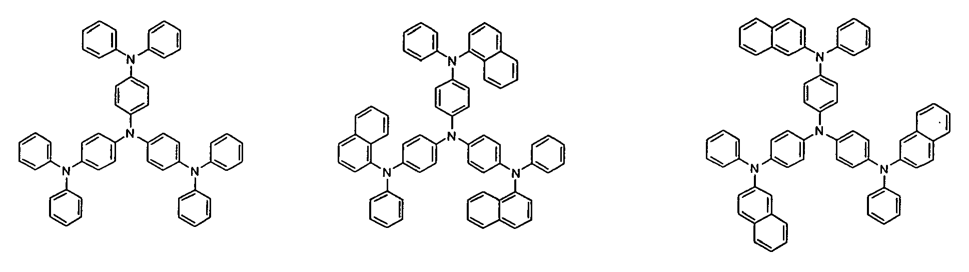

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements, as well as the organic electroluminescent compound represented by Chemical Formula (1). The organic layer may comprise a charge generating layer in addition to the electroluminescent layer.

The present invention can realize an electroluminescent device having a pixel structure of independent light-emitting mode, which comprises an organic electroluminescent device containing the compound of Chemical Formula (1) as a sub-pixel, and one or more sub-pixel(s) comprising one or more metal compound(s) selected from a group consisting of Ir, Pt, Pd, Rh, Re, Os, Tl, Pb, Bi, In, Sn, Sb, Te, Au and Ag, patterned in parallel at the same time.

Further, the organic electroluminescent device is an organic display wherein the organic layer comprises, in addition to the organic electroluminescent compound according to the invention, one or more compound(s) selected from compounds having electroluminescent peak of wavelength of not more than 500 nm or those having electroluminescent peak of wavelength of not less than 560 nm, at the same time. The compounds having electroluminescent peak of wavelength of not more than 500 nm or those having electroluminescent peak of wavelength of not less than 560 nm may be exemplified by the compounds represented by one of Chemical Formulas (9) to (15), but they are not restricted thereto.

Chemical Formula 9 M¹L¹⁰¹L¹⁰²L¹⁰³

In Chemical Formula (9), M¹ is selected from Group 7, 8, 9, 10, 11, 13, 14, 15 and 16 metals in the Periodic Table, and ligands L¹⁰¹, L¹⁰² and L¹⁰³ are independently selected from the following structures: 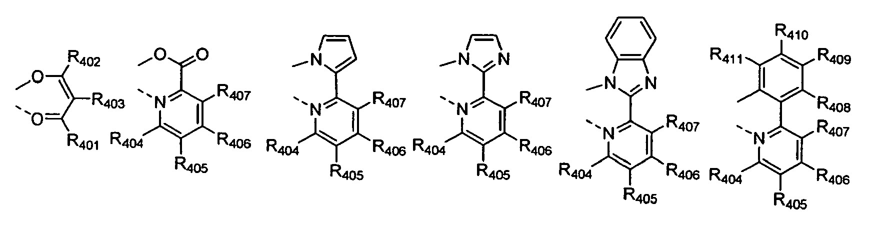 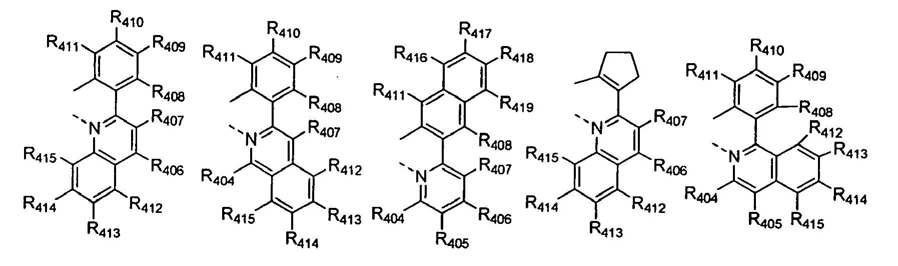 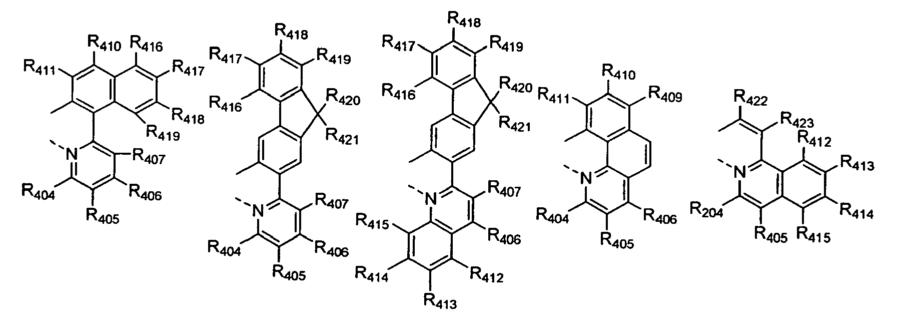 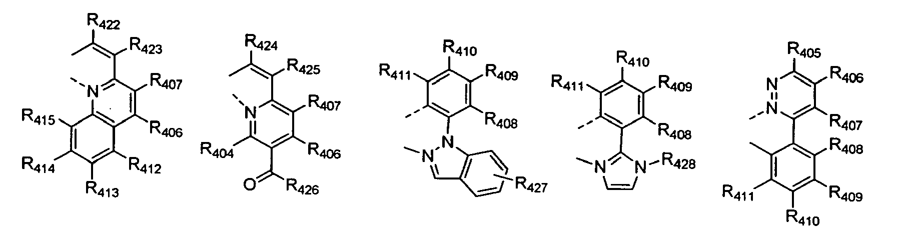 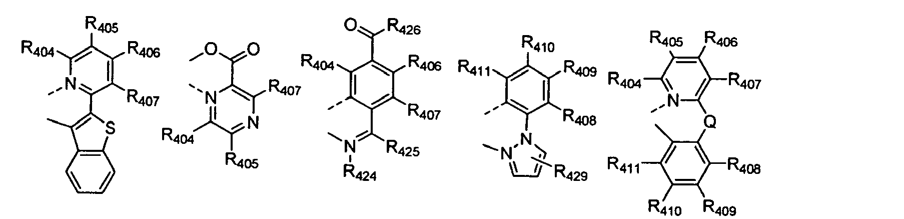 wherein, R₄₀₁ through R₄₀₃ independently represent hydrogen, (C1-C60)alkyl with or without halogen substituent(s), (C6-C60)aryl with or without (C1-C60)alkyl substituent(s), or halogen;
R₄₀₄ through R₄₁₉ independently represent hydrogen, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen, and the alkyl, cycloalkyl, alkenyl or aryl of R₄₀₄ through R₄₁₉ may be further substituted by one or more substituent(s) selected from (C1-C60)alkyl, (C6-C60)aryl and halogen;
R₄₂₀ through R₄₂₃ independently represent hydrogen, (C1-C60)alkyl with or without halogen substituent(s), (C6-C60)aryl with or without (C1-C60)alkyl substituent(s);
R₄₂₄ and R₄₂₅ independently represent hydrogen, (C1-C60)alkyl, (C6-C60)aryl or halogen, or R₄₂₄ and R₄₂₅ may be linked via (C3-C12)alkylene or (C3-C12)alkenylene with or without a fused ring to form an alicyclic ring, or a monocyclic or polycyclic aromatic ring; and the alkyl or aryl of R₄₂₄ and R₄₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from (C1-C60)alkyl with or without halogen substituent(s), (C1-C30)alkoxy, halogen, tri(C1-C30)alkylsilyl, tri(C6-C30)arylsilyl and (C6-C60)aryl;
R₄₂₆ represents (C1-C60)alkyl, (C6-C60) aryl, or (C5-C60)heteroaryl containing one or more heteroatom(s) selected from N, O and S, or halogen;
R₄₂₇ through R₄₂₉ independently represent hydrogen, (C1-C60)alkyl, (C6-C60)aryl or halogen, and the alkyl or aryl of R₄₂₇ through R₄₂₉ may be further substituted by halogen or (C1-C60)alkyl;
Q represents 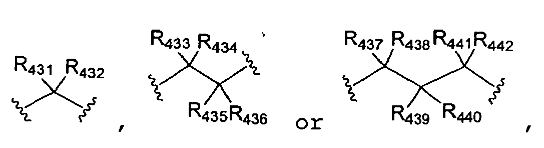 and R₄₃₁ through R₄₄₂ independently represent hydrogen, (C1-C60)alkyl with or without halogen substituent(s), (C1-C30)alkoxy, halogen, (C6-C60)aryl, cyano, (C5-C60)cycloalkyl, or each of R₄₃₁ through R₄₄₂ may be linked to an adjacent substituent via alkylene or alkenylene to form a (C5-C7) spiro-ring or (C5-C9) fused ring, or each of them may be linked to R₄₀₇ or R₄₀₈ to form a (C5-C7) fused ring.

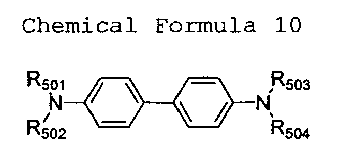

In Chemical Formula (10), R₅₀₁ through R₅₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₅₀₁ through R₅₀₄, or the alicyclic ring, or the monocyclic or polycyclic aromatic ring formed therefrom by linkage) via (C3-C60)alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

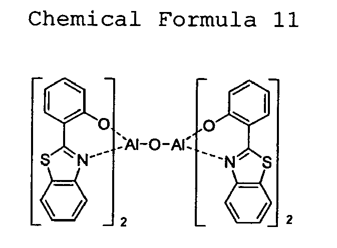

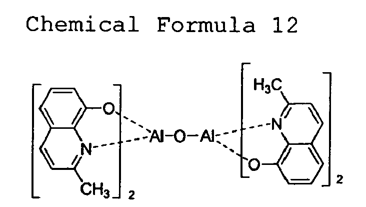

Chemical Formula 13 L²⁰¹L²⁰²M²(T)ₖ

In Chemical Formula (13), the ligands, L²⁰¹ and L²⁰² are independently selected from the following structures: 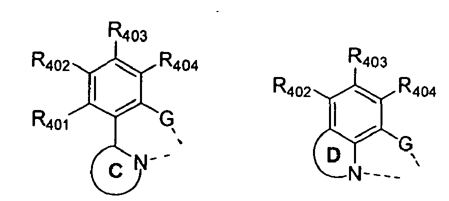 M² is a bivalent or trivalent metal;
k is 0 when M² is a bivalent metal, while k is 1 when M² is a trivalent metal;
T represents (C6-C60)aryloxy or tri(C6-C60)arylsilyl, and the aryloxy and triarylsilyl of T may be further substituted by (C1-C60)alkyl or (C6-C60)aryl;
G represents O, S or Se;
ring C represents oxazole, thiazole, imidazole, oxadiazole, thiadiazole, benzoxazole, benzothiazole, benzimidazole, pyridine or quinoline;
ring D represents pyridine or quinoline, and ring D may be further substituted by (C1-C60)alkyl, or phenyl or naphthyl with or without (C1-C60)alkyl substituent(s);
R₆₀₁ through R₆₀₄ independently represent hydrogen, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may form a chemical bond with R₆₀₁ to form a fused ring;
the aryl of ring C and R₆₀₁ through R₆₀₄ may be further substituted by (C1-C60)alkyl, halogen, (C1-C60)alkyl with halogen substituent(s), phenyl, naphthyl, tri(C1-C60)alkylsilyl, tri(C6-C60)arylsilyl or amino group.

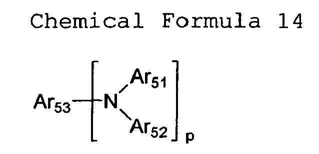

In Chemical Formula (14), Ar₅₁ and Ar₅₂ independently represent hydrogen, deuterium, halogen, (C1-C60)alkyl, (C6-C60)aryl, (C4-C60)heteroaryl, (C6-C60)arylamino, (C1-C60)alkylamino, a 5- or 6-membered heterocycloalkyl containing one or more heteroatom(s) selected from N, O and S, or (C3-C60)cycloalkyl, or Ar₅₁ and Ar₅₂ may be linked via (C3-C60)alkylene or (C3-C60)alkenylene with or without a fused ring to form an alicyclic ring, or a monocyclic or polycyclic aromatic ring;
when p is 1, Ar₅₃ represents (C6-C60)aryl, (C4-C60)heteroaryl, or a substituent represented by one of the following structural formulas: 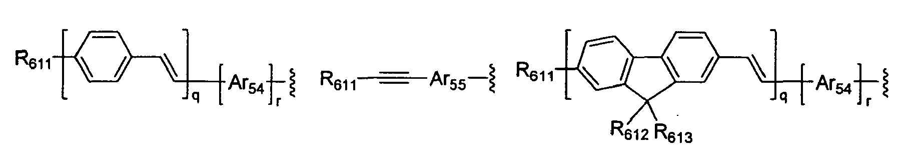 when p is 2, Ar₅₃ represents (C6-C60) arylene, (C4-C60)heteroarylene, or a substituent represented by one of the following structural formulas: 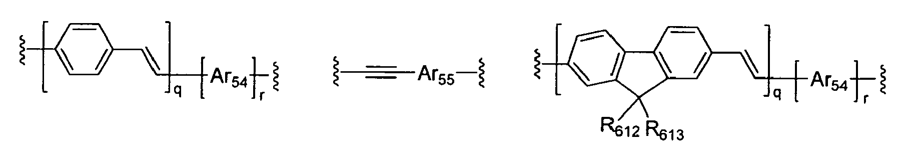 wherein Ar₅₄ and Ar₅₅ independently represent (C6-C60)arylene or (C4-C60)heteroarylene;
R₆₁₁ through R₆₁₃ independently represent hydrogen, deuterium, (C1-C60)alkyl or (C6-C60)aryl;
q is an integer from 1 to 4, r is an integer of 0 or 1; and
the alkyl, aryl, heteroaryl, arylamino, alkylamino, cycloalkyl or heterocycloalkyl of Ar₅₁ and Ar₅₂, or the aryl, heteroaryl, arylene or heteroarylene of Ar₅₃, or the arylene or heteroarylene of Ar₅₄ and Ar₅₅, or the alkyl or aryl of R₆₁₁ through R₆₁₃ may be further substituted by one or more substituent(s) selected from a group consisting of halogen, deuterium, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

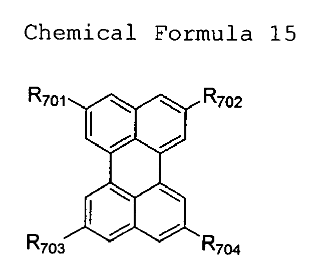

In Chemical Formula (15), R₇₀₁ through R₇₀₄ independently represent hydrogen, deuterium, halogen, (C1-C60)alkyl, (C6-C60)aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₇₀₁ through R₇₀₄ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino or arylamino of R₇₀₁ through R₇₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60) aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The compounds for an electroluminescent layer, having electroluminescent peak of wavelength of not more than 500 nm or those having electroluminescent peak of wavelength of not less than 560 nm, may be exemplified by the following compounds, but they are not restricted thereto.

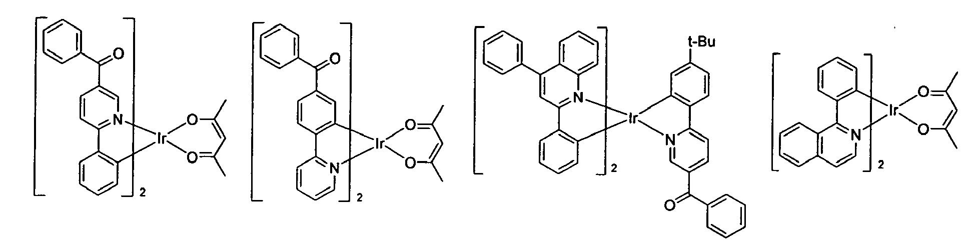

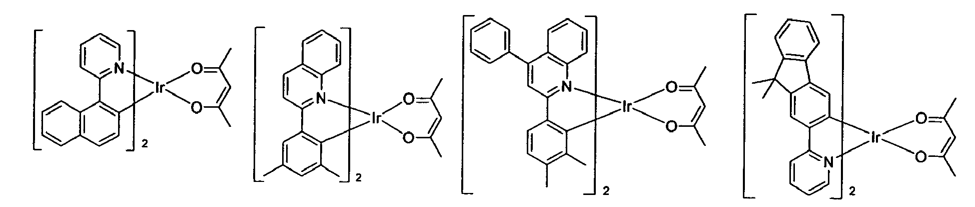

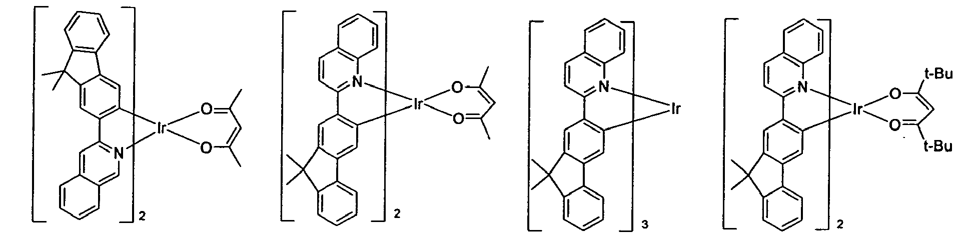

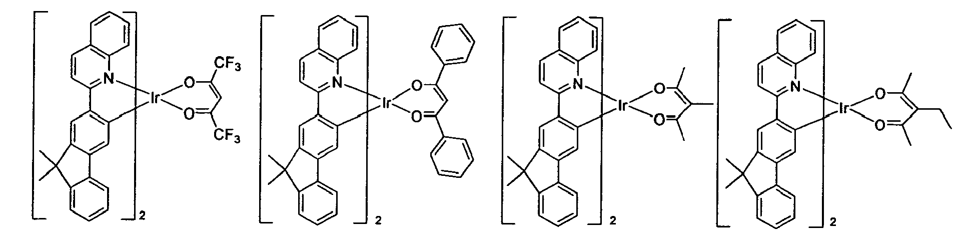

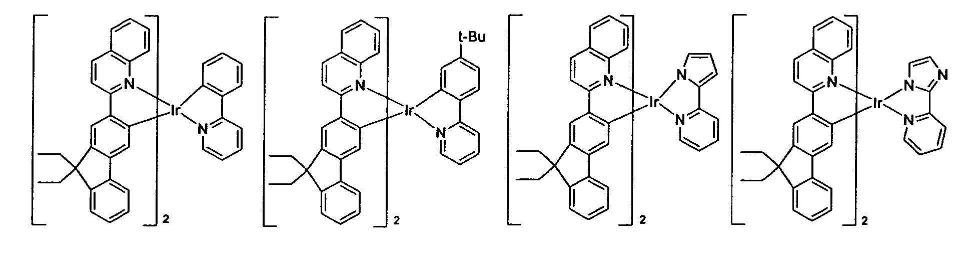

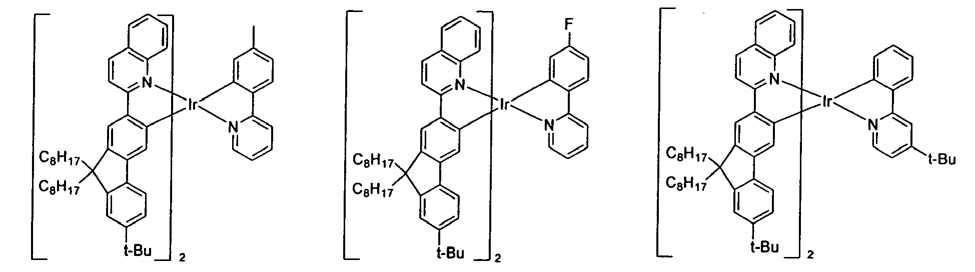

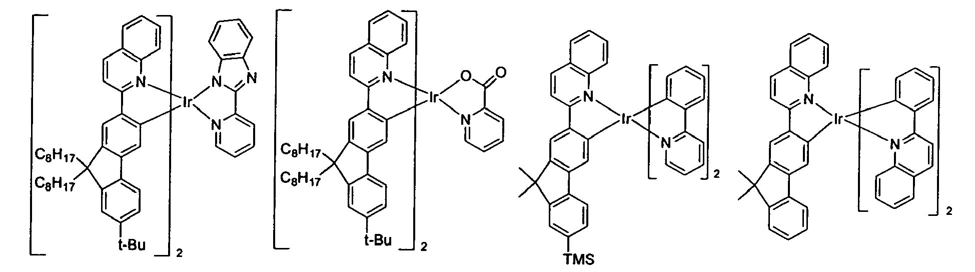

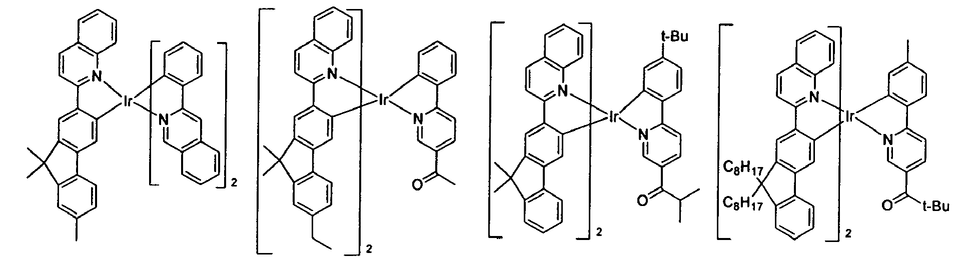

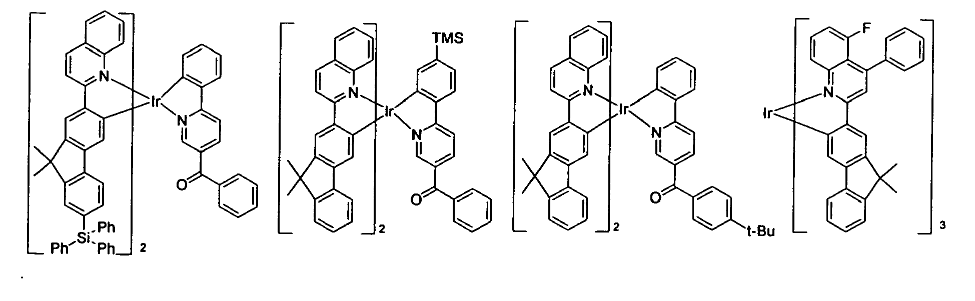

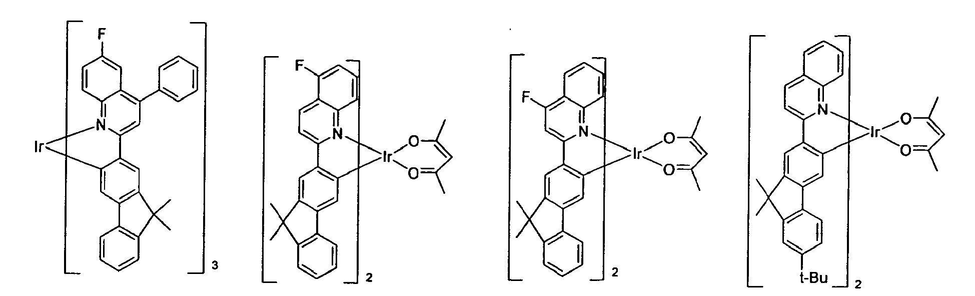

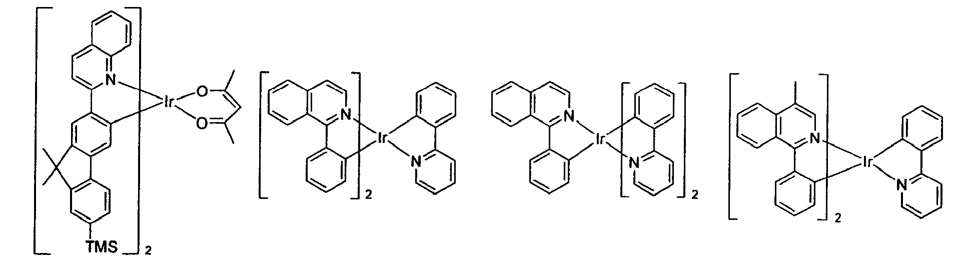

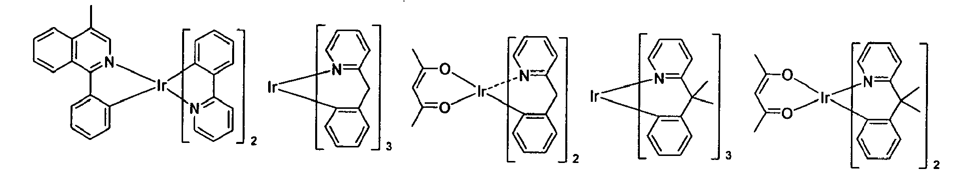

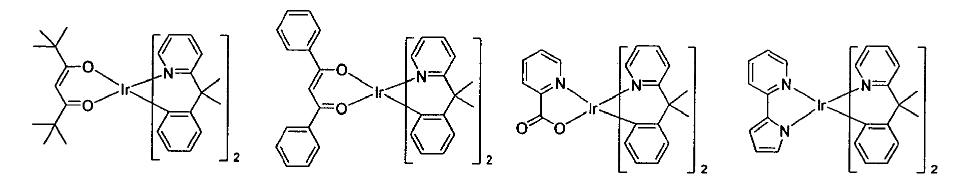

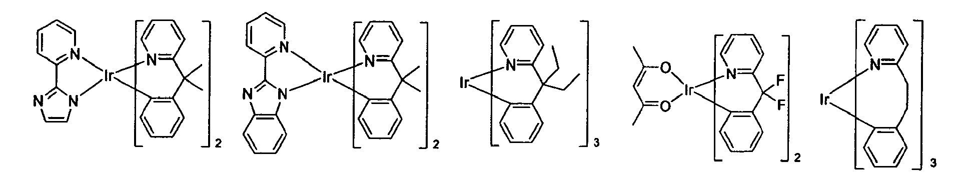

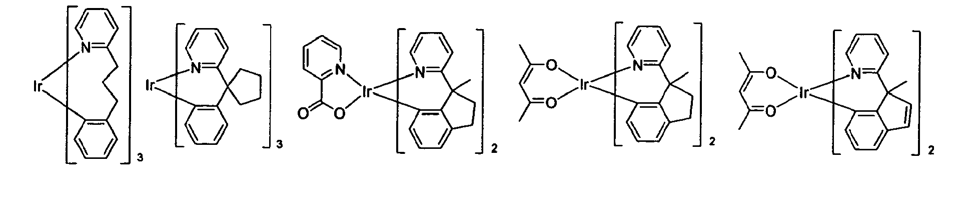

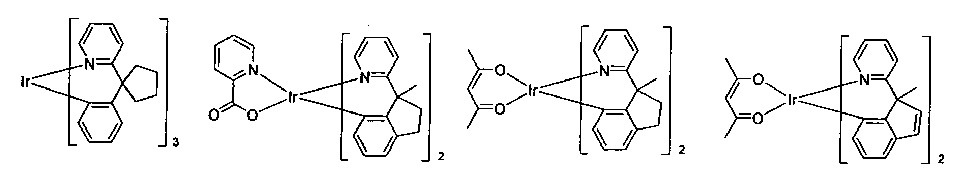

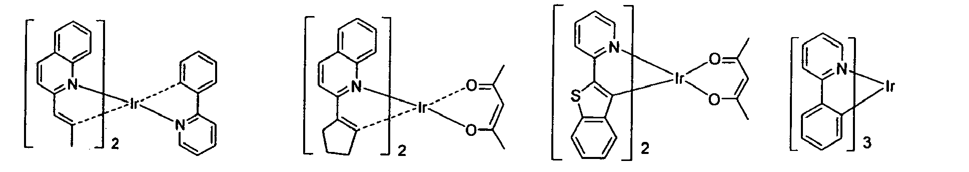

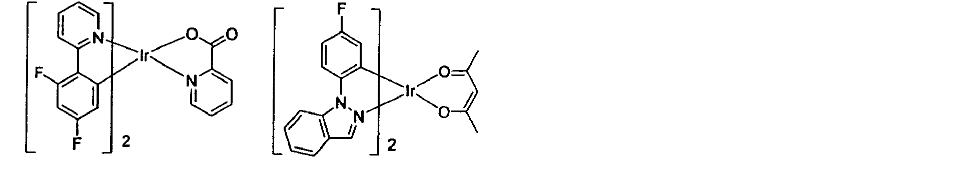

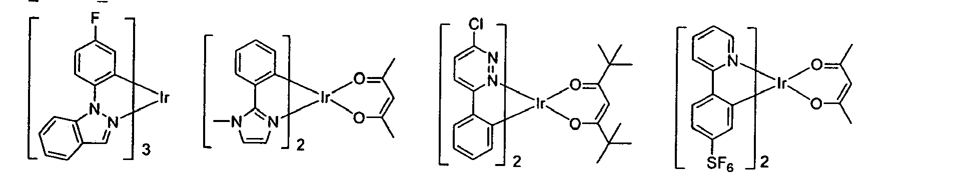

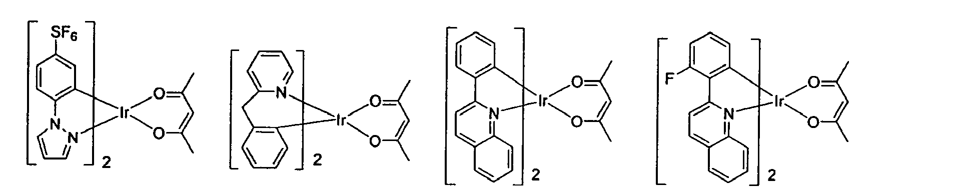

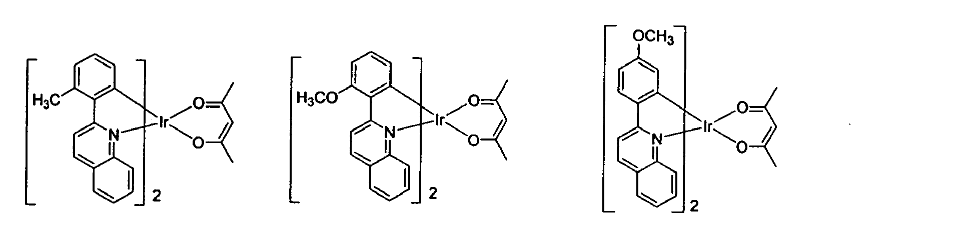

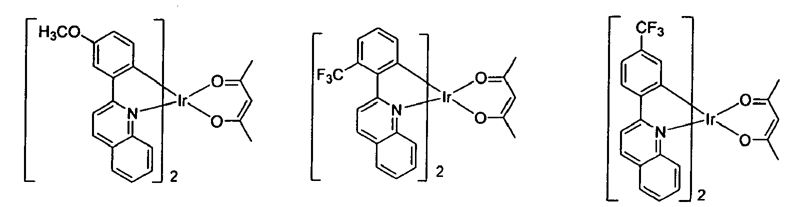

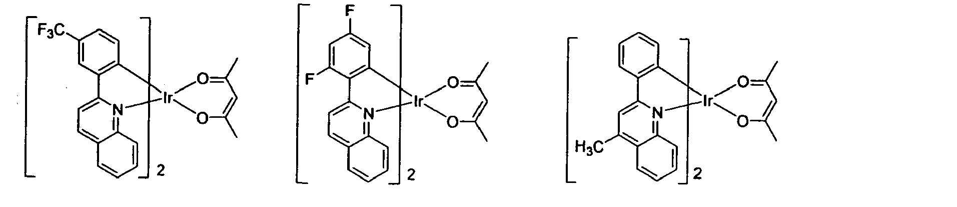

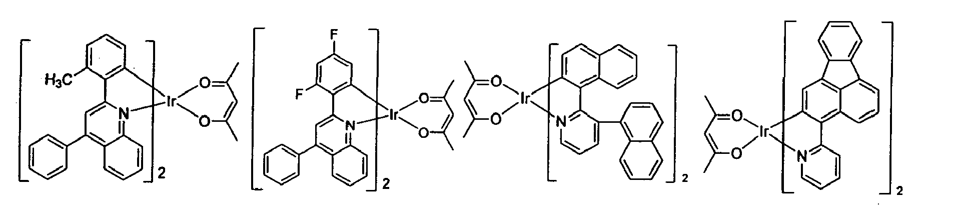

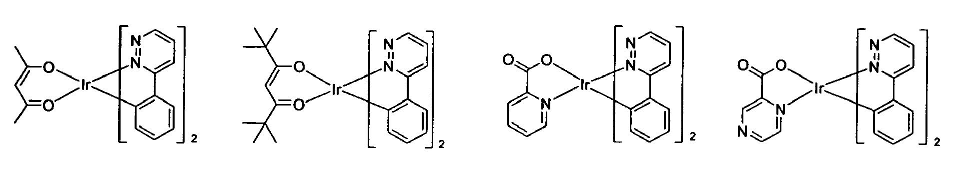

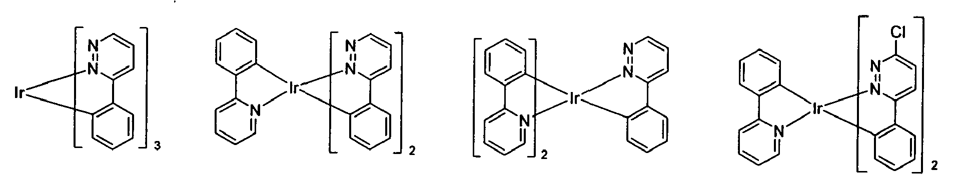

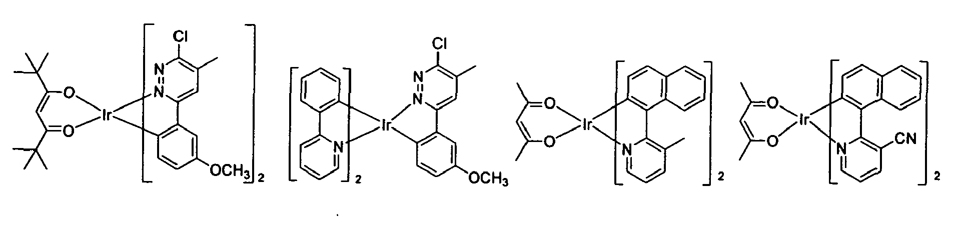

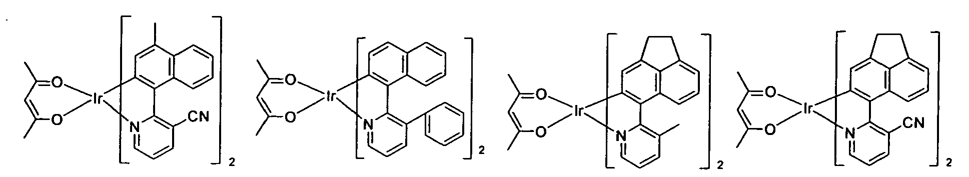

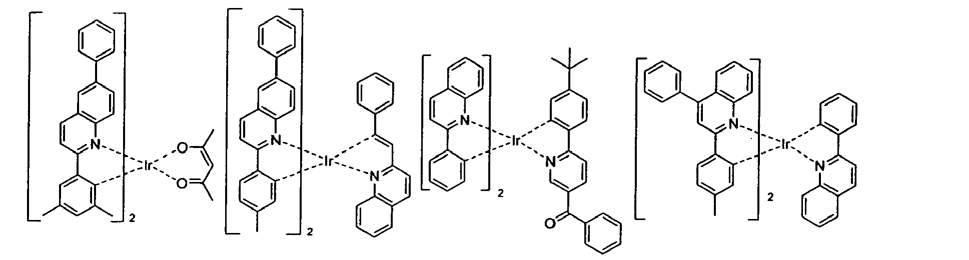

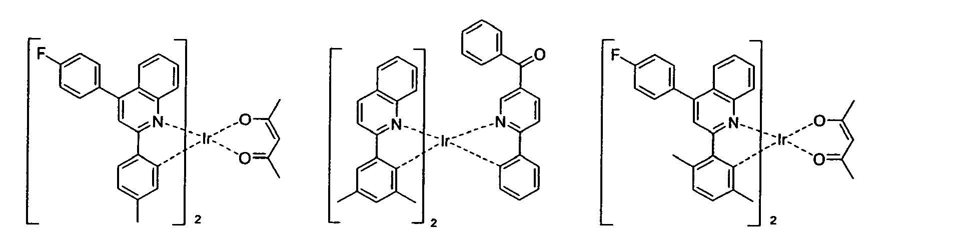

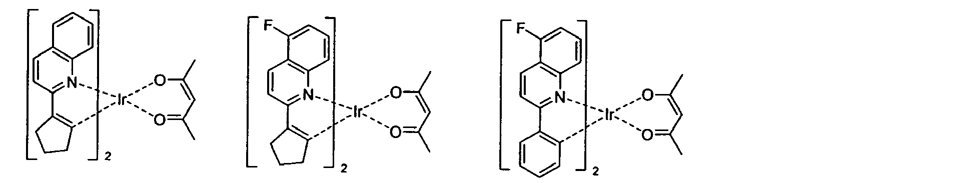

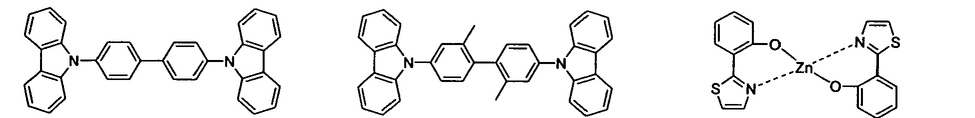

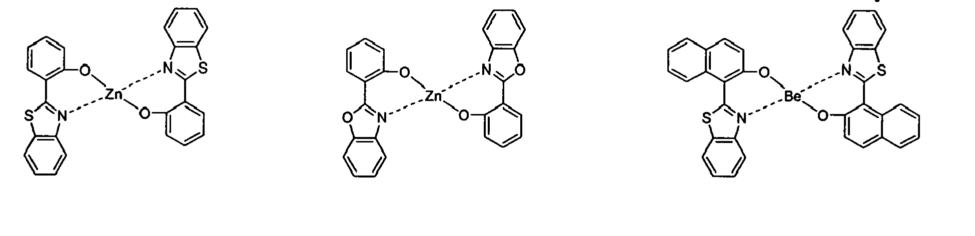

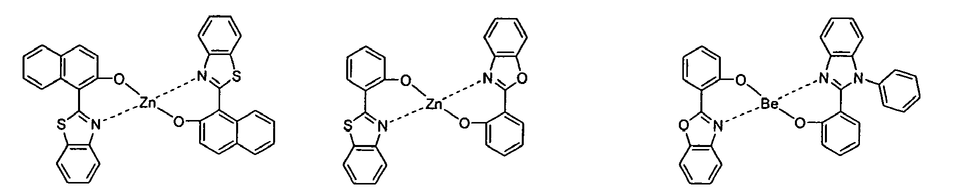

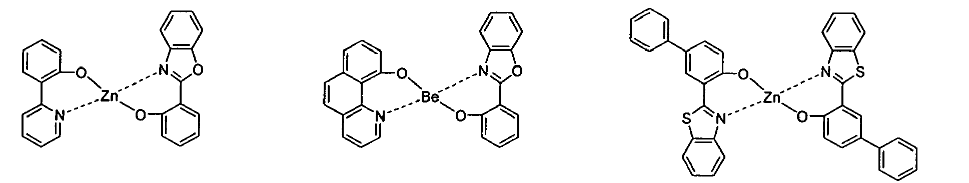

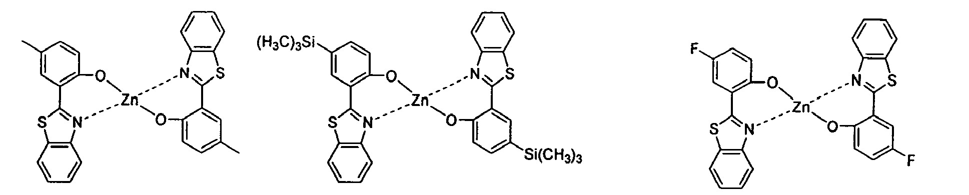

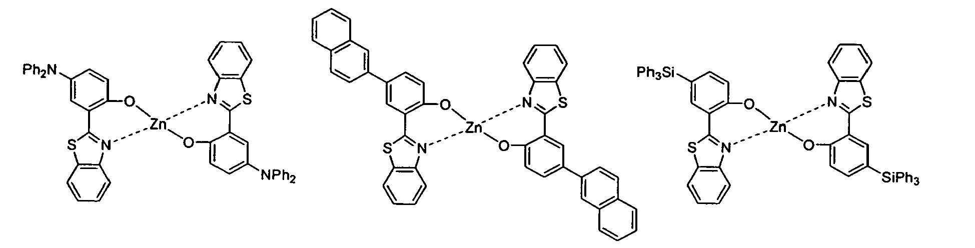

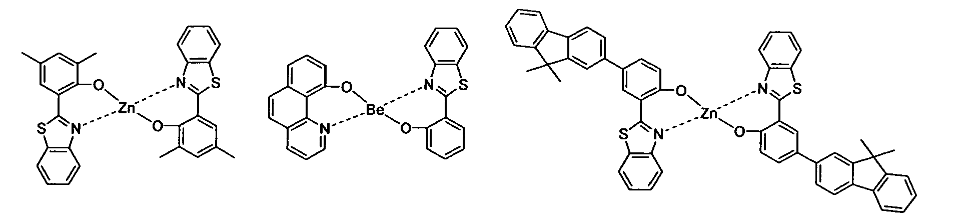

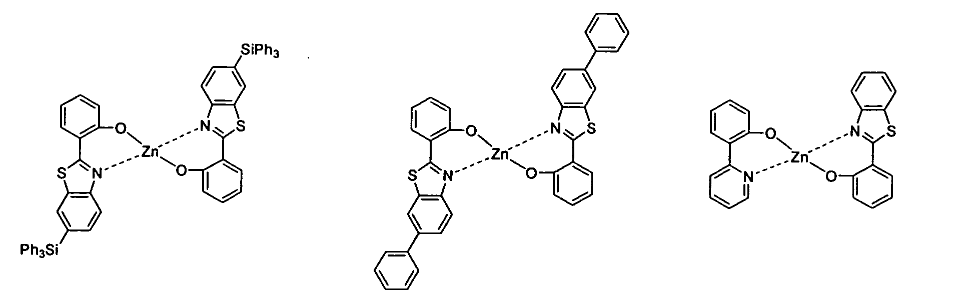

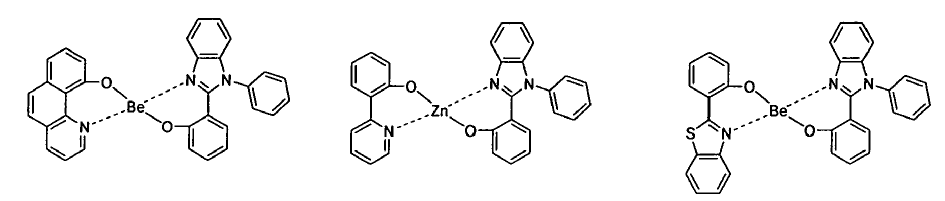

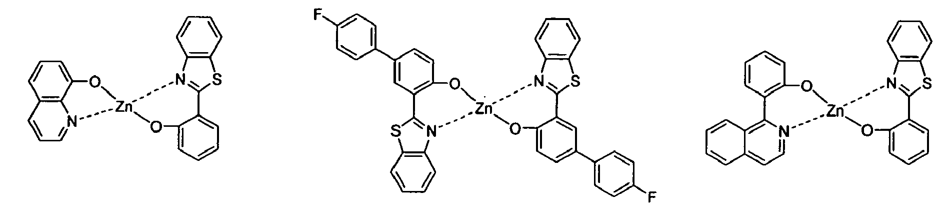

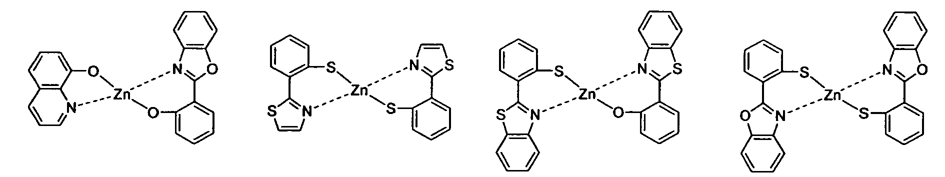

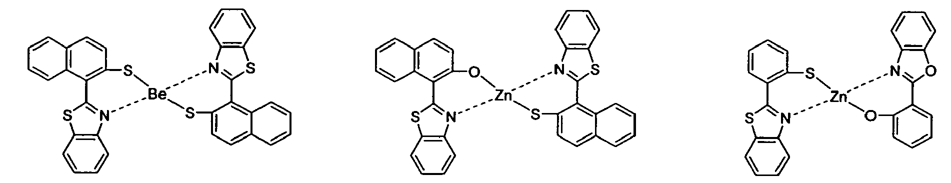

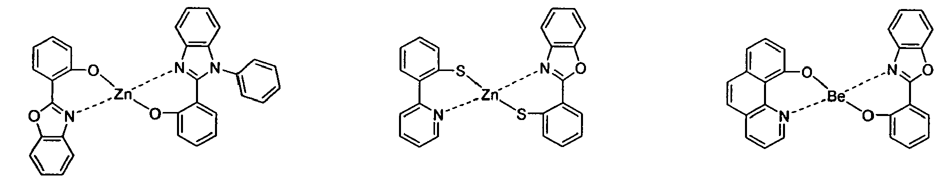

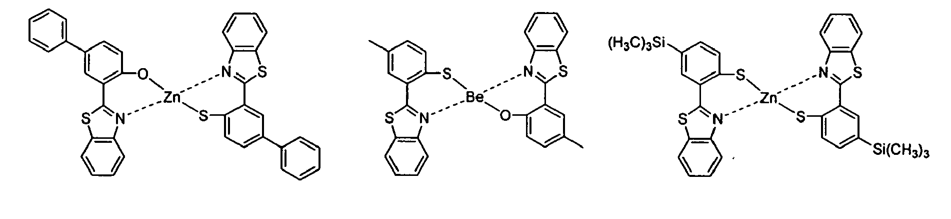

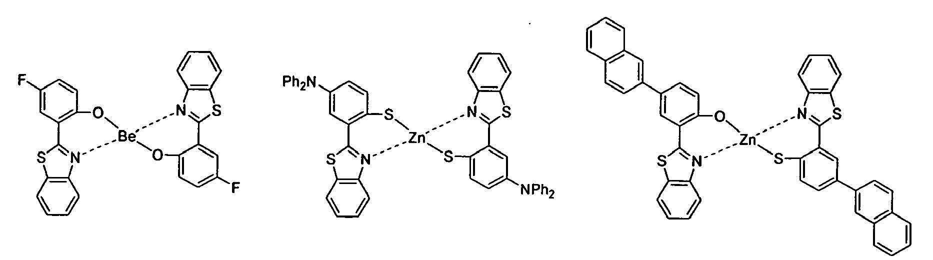

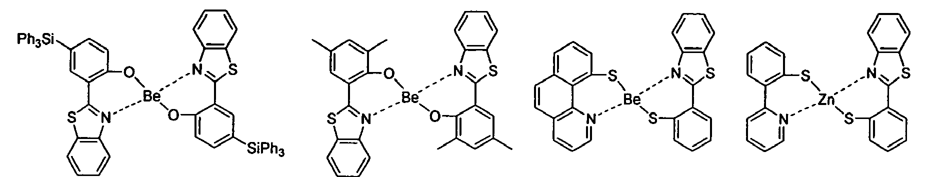

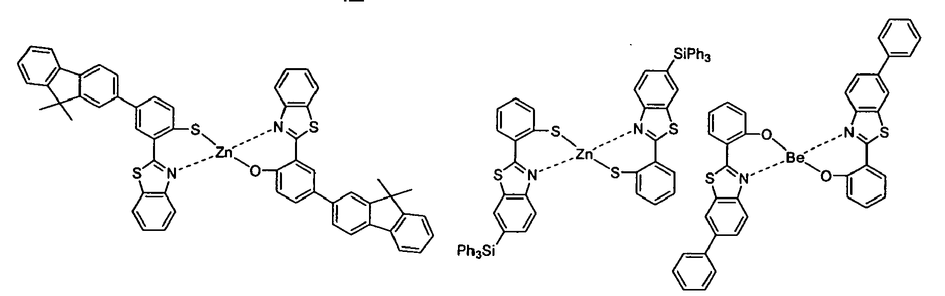

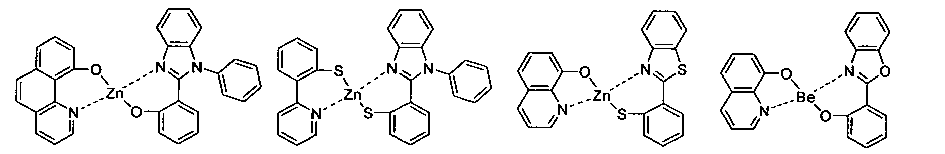

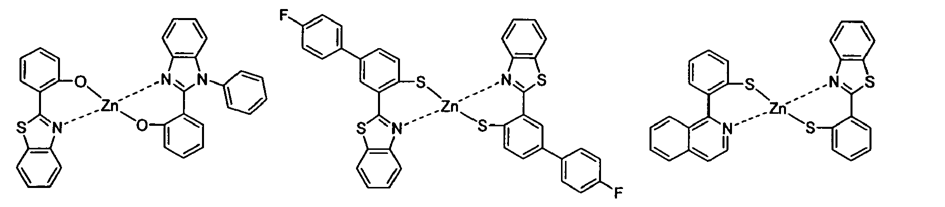

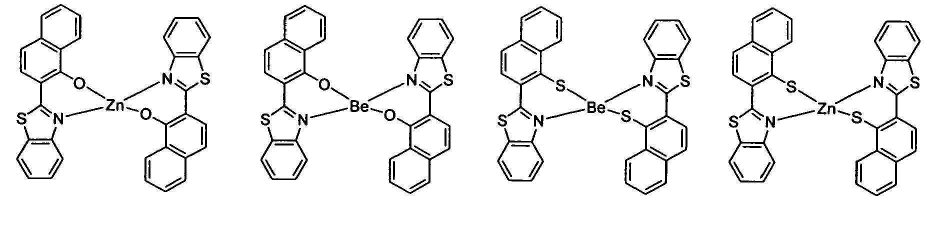

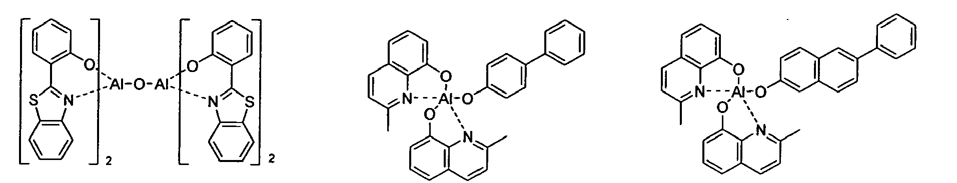

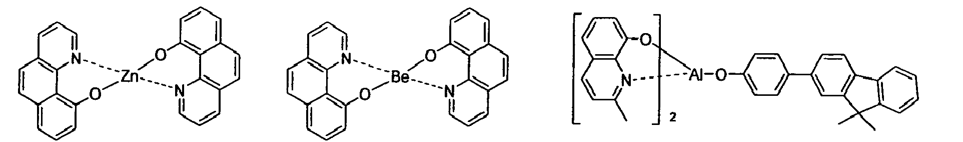

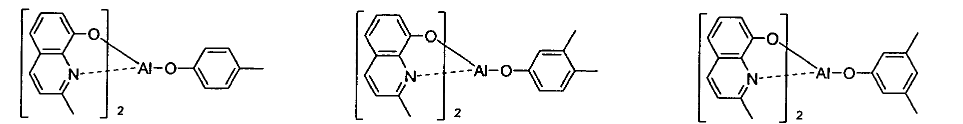

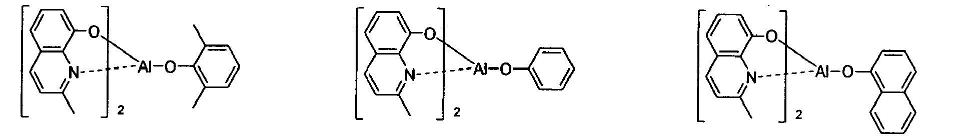

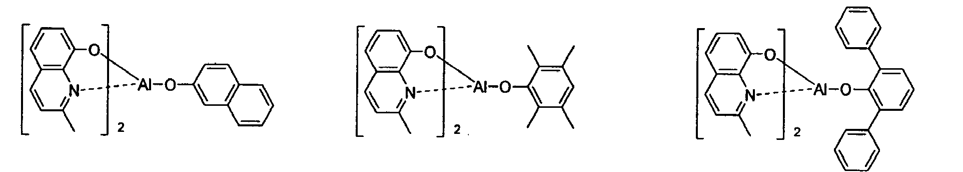

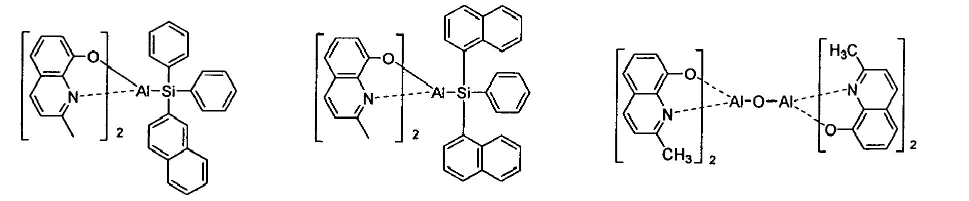

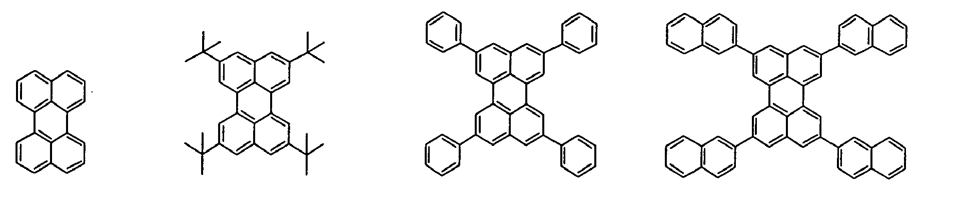

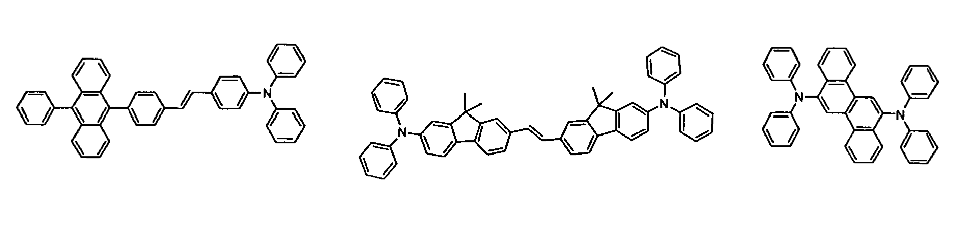

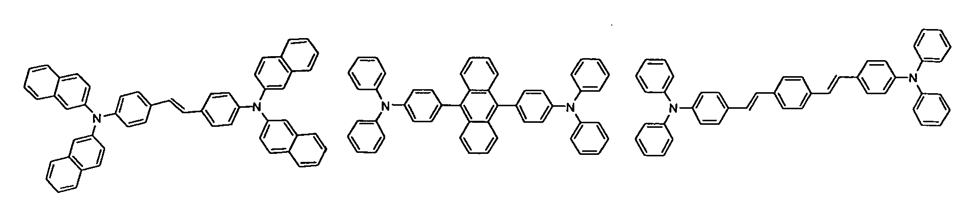

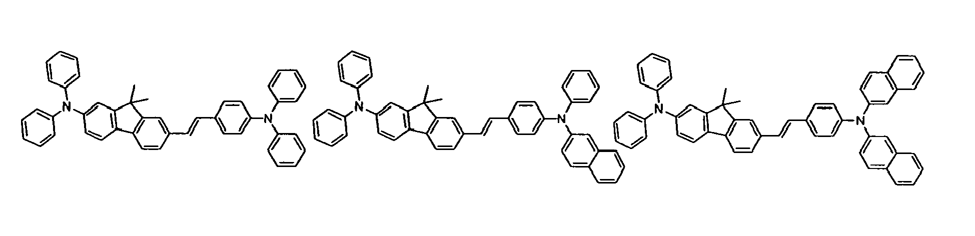

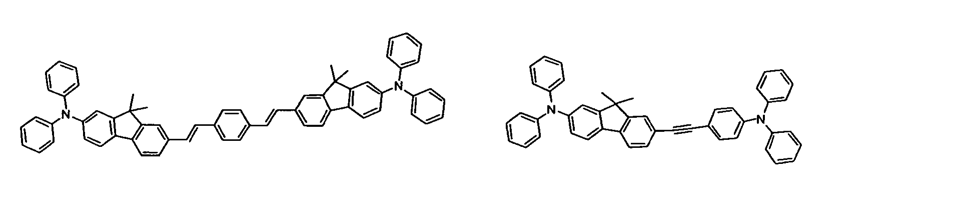

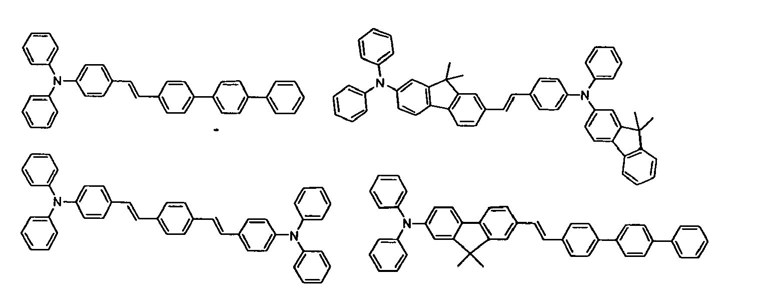

In an organic electroluminescent device according to the present invention, it is preferable to displace one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer side, and a metal halide layer or a metal oxide layer on the cathode surface of the electroluminescent (EL) medium layer side. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal or the like. Examples of metal oxides preferably include CS₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

Since the organic electroluminescent compounds according to the invention show high luminous efficiency and provide excellent life property of devices manufactured therefrom, OLED's with very good operation lifetime can be manufactured.

### Best Mode

The present invention is further described with respect to the compounds according to the invention, the processes for preparing the same, and electroluminescent properties of devices manufactured therefrom by referring to the representative compounds of the invention, which are provided for illustration of the embodiments only but are not intended to limit the scope of the invention by any means.

### Preparation Examples

[Preparation Example 1] Preparation of Compound (32)

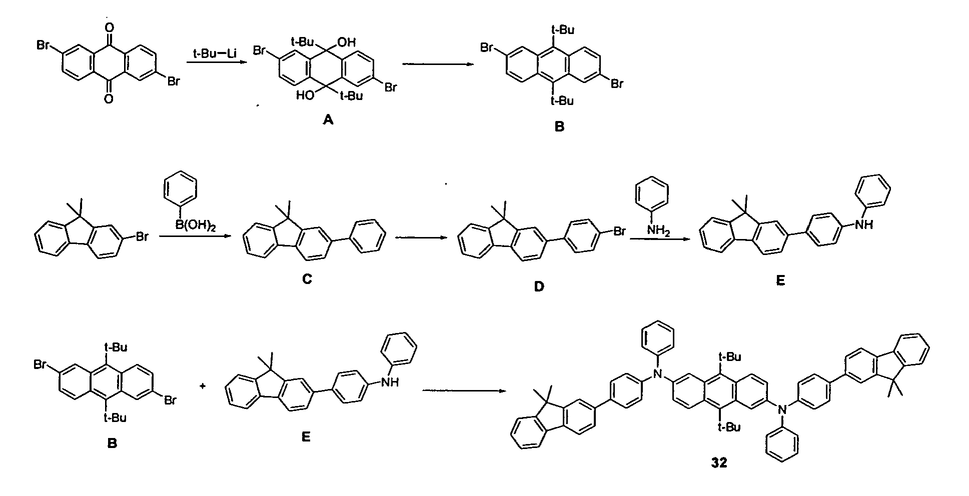

### Preparation of Compound (A)

In ethyl ether (300 mL) thoroughly purified under nitrogen atmosphere, dissolved was 2,6-dibromoanthracene-9,10-dione (10.0 g, 27.3 mmol). To the solution, t-butyllithium (1.7 M solution in pentane) (80.35 mL, 136.6 mmol) was slowly added dropwise at 0°C. The temperature was slowly raised to room temperature, and the reaction mixture was stirred for one day at the same temperature. The reaction was quenched by adding distilled water (200 mL), and the mixture was extracted with ethyl acetate (300 mL), and the extract was dried under reduced pressure. Recrystallization from ethyl acetate (50 mL) and n-hexane (150 mL) gave Compound (A) (7.0 g, 14.51 mmol).

### Preparation of Compound (B)

Compound A (7.0 g, 14.51 mmol), potassium iodide (KI) (9.64 g, 58.06 mmol) and sodium phosphate monohydrate (NaH₂PO₂· H₂O) (9.24 g, 87.12 mmol) were dissolved in acetic acid (150 mL), and the solution was stirred under reflux for 14 hours. Then, the reaction mixture was cooled to 25°C, neutralized by adding sodium hydroxide solution (200 mL), and washed with water (400 mL). The mixture was then extracted with dichloromethane solvent (300 mL), and the extract was dried over magnesium sulfate and filtered. After removing the solvent under reduced pressure, the compound obtained was purified via column chromatography (methylene chloride/hexane = 1/100) to obtain Compound (B) (4.49 g, 10.0 mmol).

### Preparation of Compound (C)

In toluene (2 L), ethanol (1 L) and Aliquat 336 (14 mL), dissolved were 2-bromo-9,9-dimethyl-9H-fluorene (100.0 g, 366.0 mmol), phenylboronic acid (49.0 g, 402.6 mmol), and dichlorobis(triphenylphosphine)palladium (II) (Pd(PPh₃)₂Cl₂) (15.0 g, 21.96 mmol) under nitrogen atmosphere. Aqueous 2M sodium carbonate solution (730 mL) was then added thereto, and the resultant mixture was stirred under reflux at 120°C for 5 hours. After cooling to 25°C, the reaction was quenched by adding distilled water (1 L). The reaction mixture was extracted with ethyl acetate (600 mL), and the extract was dried over magnesium sulfate, and filtered through a silica and celite filter. Drying under reduced pressure and recrystallization from ethyl acetate (300 mL) and methanol (600 mL) gave Compound (C) (70.0 g, 258.90 mmol).

### Preparation of Compound (D)

Compound (C) (70.0 g, 258.90 mmol) and N-bromosuccinimide (50.69 g, 284.79 mmol) were dissolved in dichloromethane (2 L) under nitrogen atmosphere, and the solution was stirred at 25°C for 10 hours. Then, the reaction mixture was thoroughly washed with distilled water (700 mL) to quench the reaction, and extracted with dichloromethane (500 mL). The extract was dried under reduced pressure, and the residue was recrystallized from tetrahydrofuran (200 mL) and methanol (400 mL) to obtain Compound (D) (67.8 g, 75%, 194.17 mmol).

### Preparation of Compound (E)

Aniline (20.0 g, 93.85 mmol), Compound (D) (42.83 g, 122.62 mmol), palladium acetate (Pd(OAc)₂) (1.05 g, 4.69 mmol), tricyclohexylphosphine (P(Cy)₃) (2.63 g, 9.39 mmol) and cesium carbonate (CS₂CO₃) (45.87 g, 140.78 mmol) were incorporated in a reaction vessel, and toluene (500 mL) was added thereto. The mixture was stirred under reflux at 110°C for 6 hours. When the reaction was completed, the reaction mixture was cooled to room temperature (25°C), washed with water (300 mL), and extracted with dichloromethane (600 mL). The extract was dried over magnesium sulfate and dried under reduced pressure. The solid compound obtained was recrystallized from ethyl acetate (200 mL) and methanol (500 mL) to obtain Compound (E) (22.73 g, 62.88 mmol).

### Preparation of Compound (32)

Compound (B) (5.0 g, 11.15 mmol), Compound (E) (12.09 g, 33.46 mmol), palladium acetate (Pd(OAc)) (0.25 g, 1.12 mmol), and cesium carbonate (Cs₂CO₃) (16.35 g, 33.45 mmol) were added to toluene solvent (100 mL) under nitrogen atmosphere, and the mixture was stirred. Immediately added was tri(t-butyl)phosphine (P(t-Bu)₃) (0.5 mL, 2.23 mmol), and the resultant mixture was stirred under reflux at 120°C for 6 hours. After cooling, methanol (200 mL) was added thereto, and the mixture was stirred for 1 hour. The solid produced in the reaction vessel was filtered. The solid obtained was dissolved in chloroform solvent (2 L), and filtered through silica gel and celite. The solvent was evaporated under reduced pressure, and the solid obtained was sequentially washed with methanol (300 mL), ethyl acetate (100 mL) and tetrahydrofuran (50 mL) to obtain the title compound (32) (5.18 g, 47%, 5.13 mmol) as a greenish solid.

According to the same procedure as Preparation Example 1, organic electroluminescent compounds (Compound 1 through Compound 4960) in Table 1 were prepared, of which the ¹H NMR and MS/FAB data are listed in Table 2.

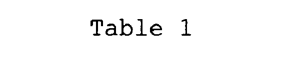

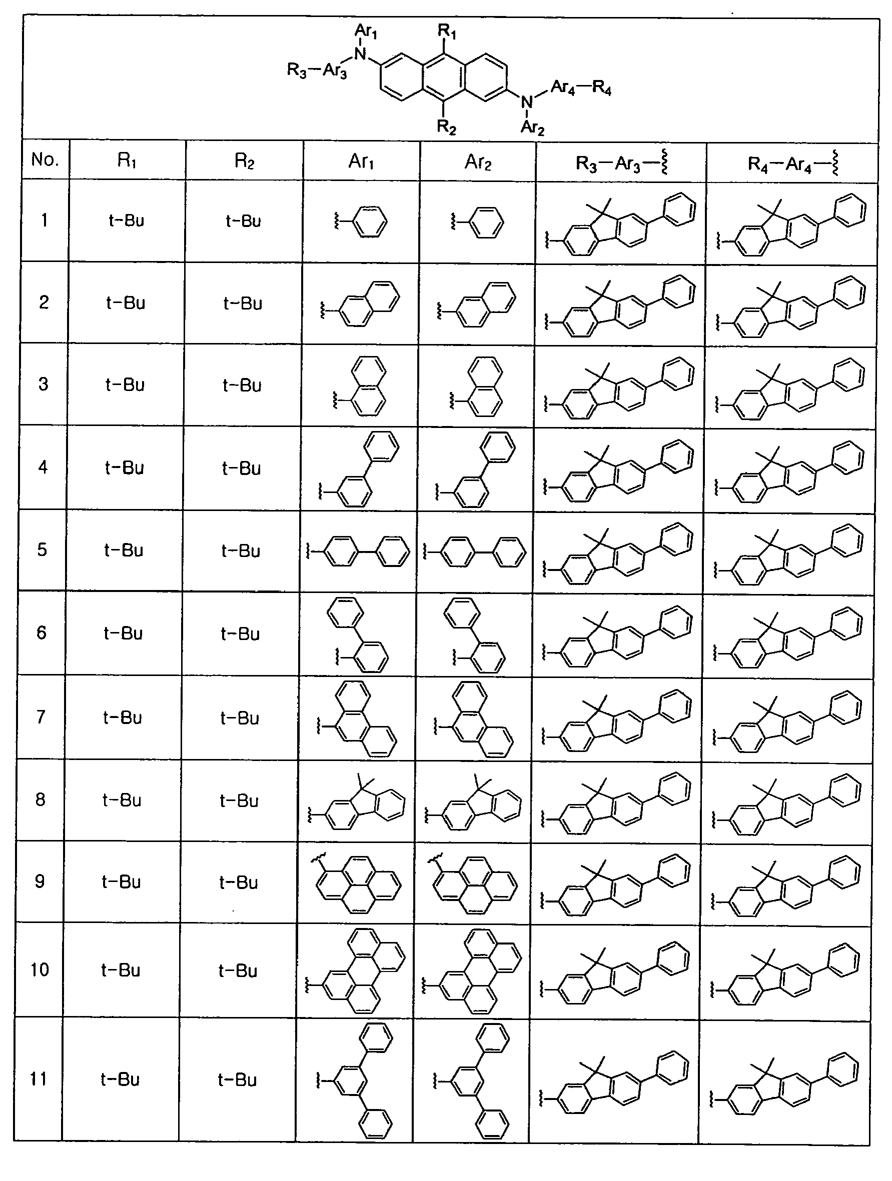

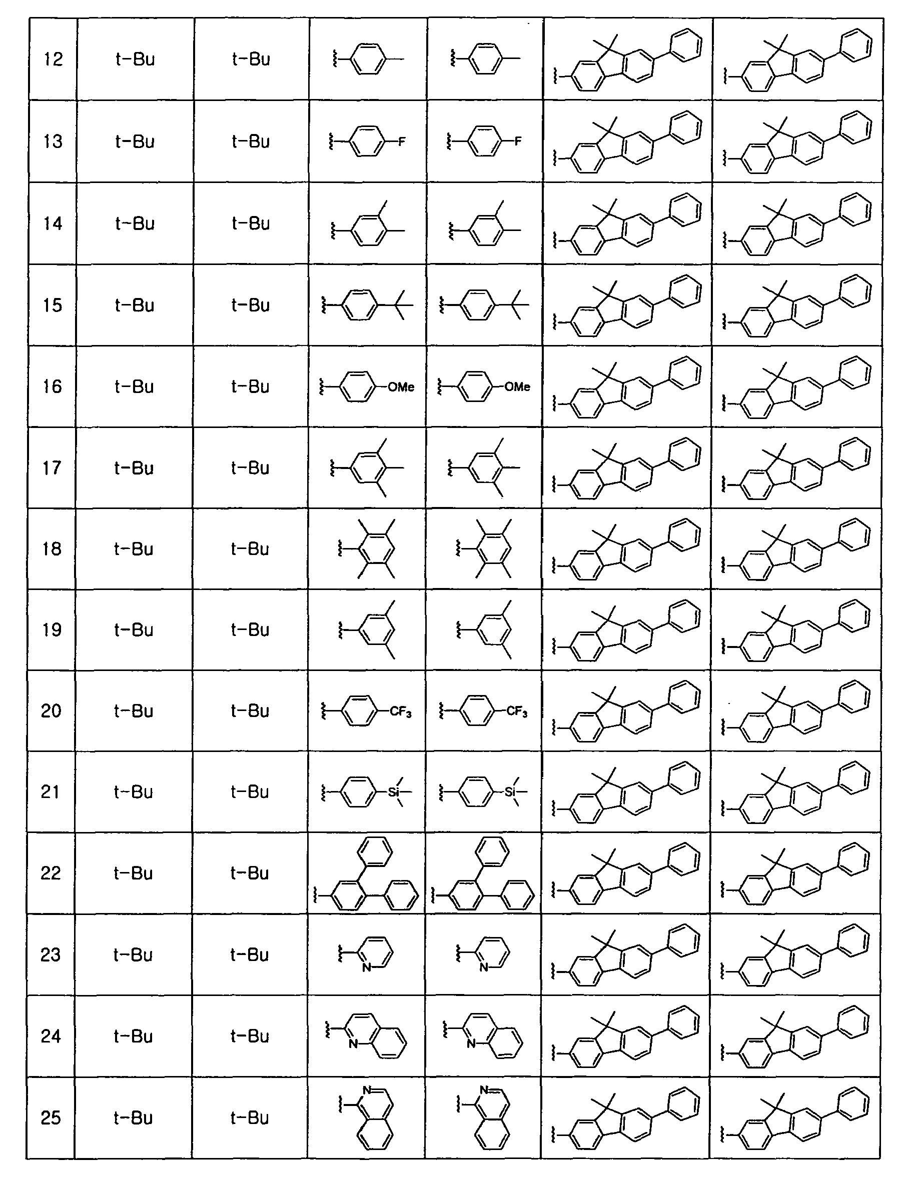

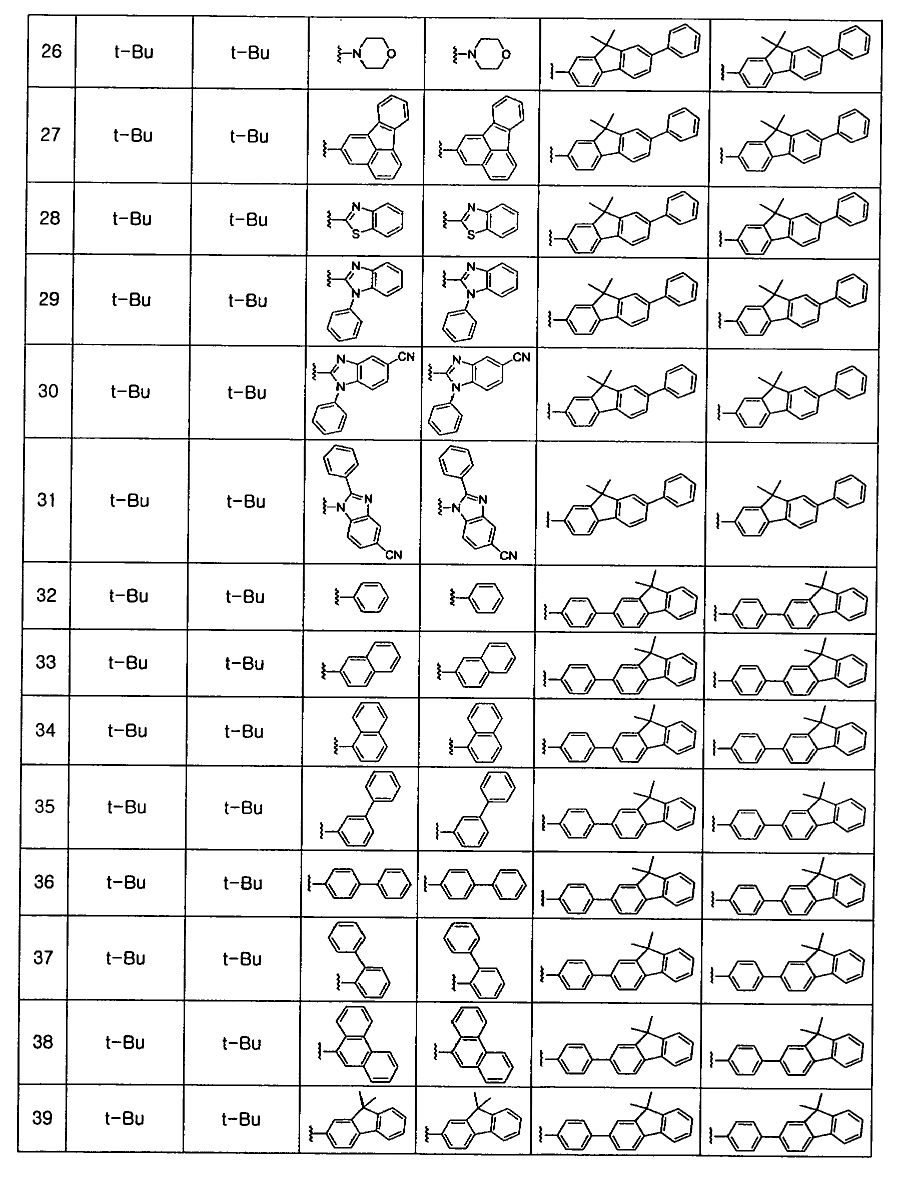

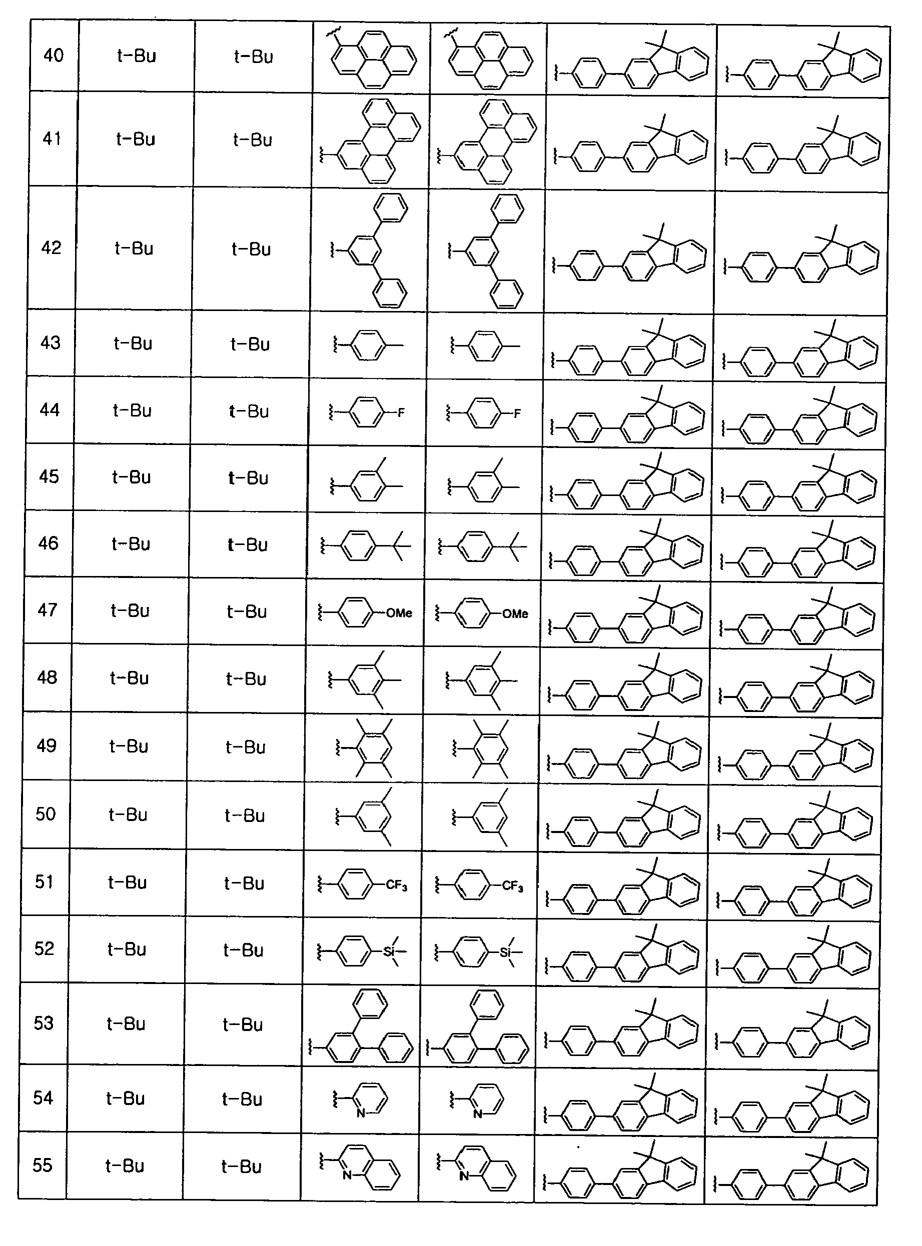

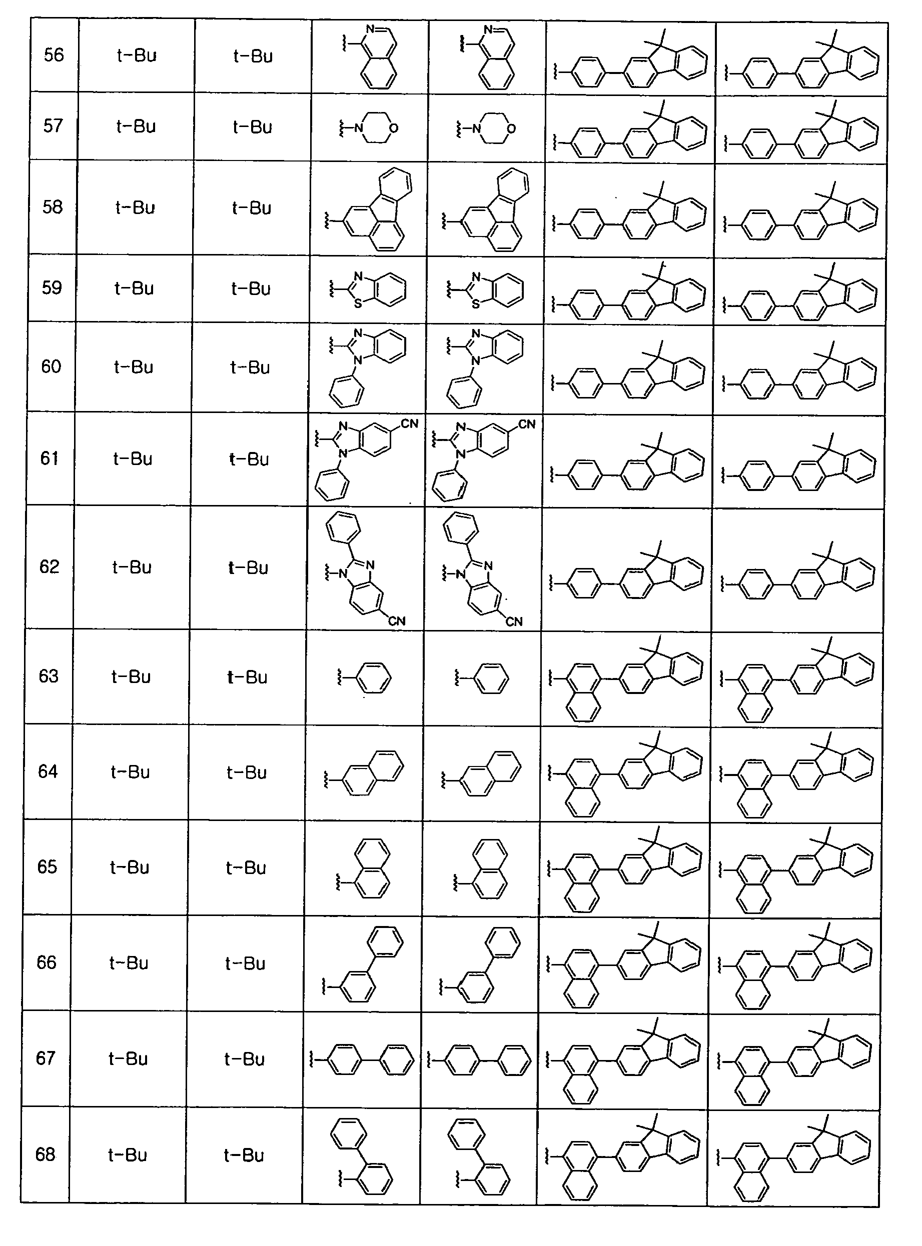

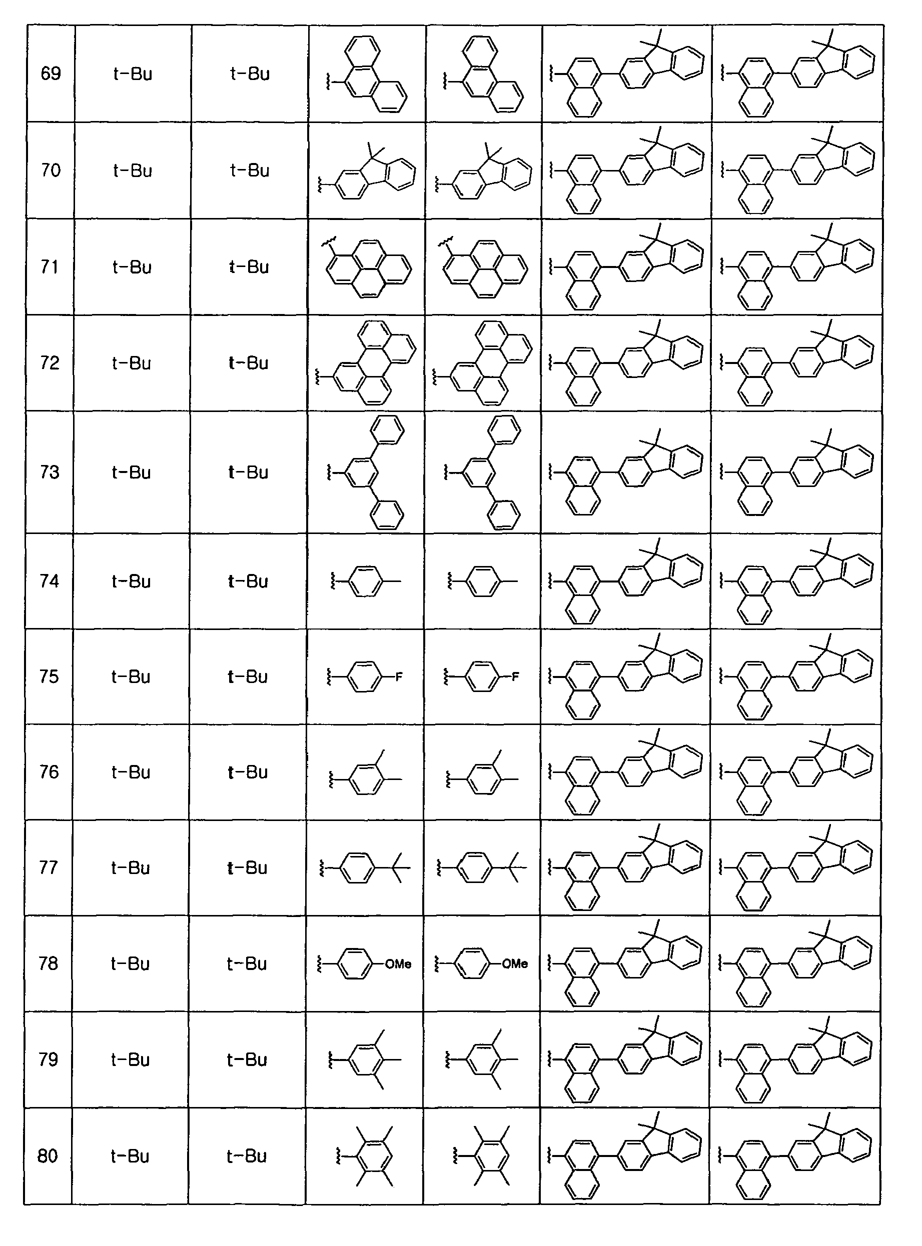

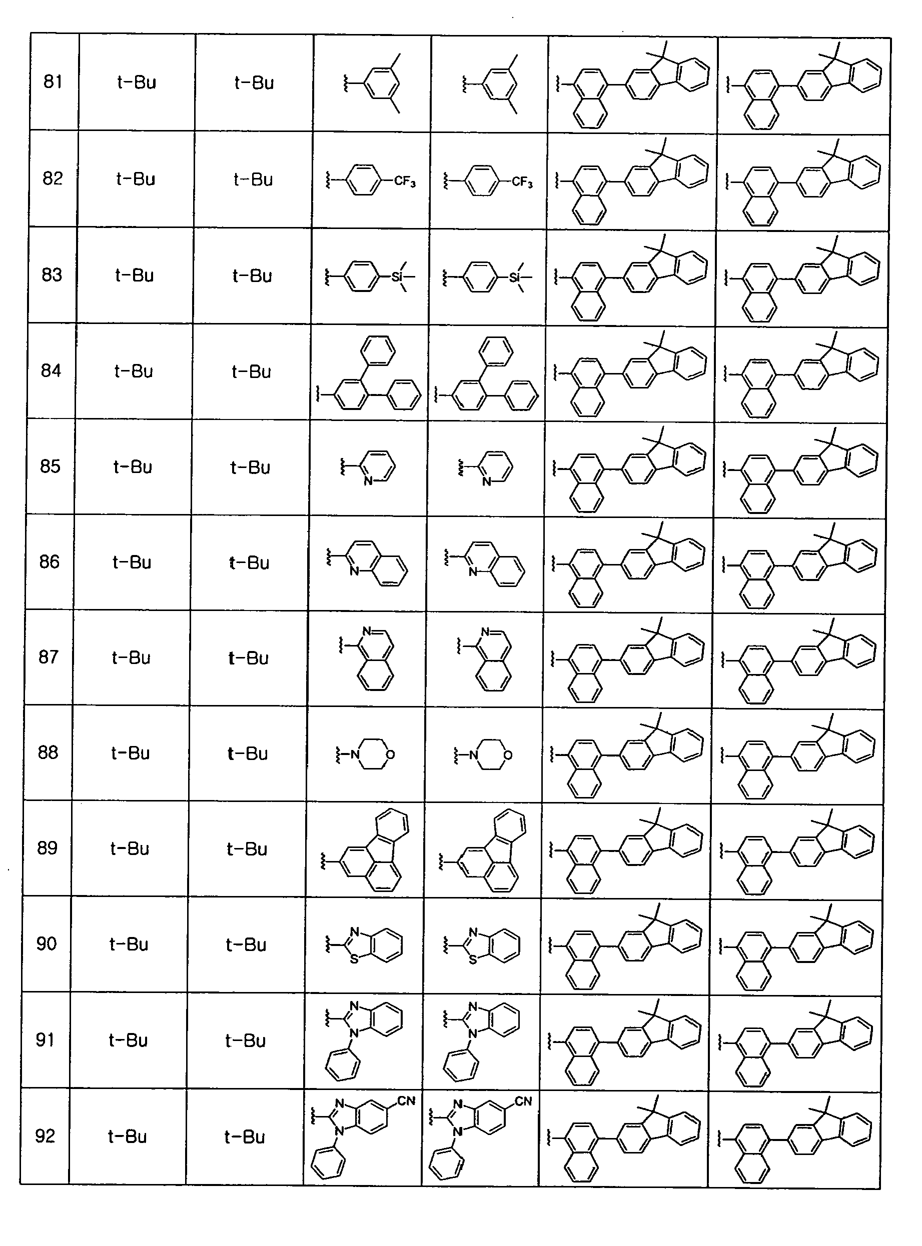

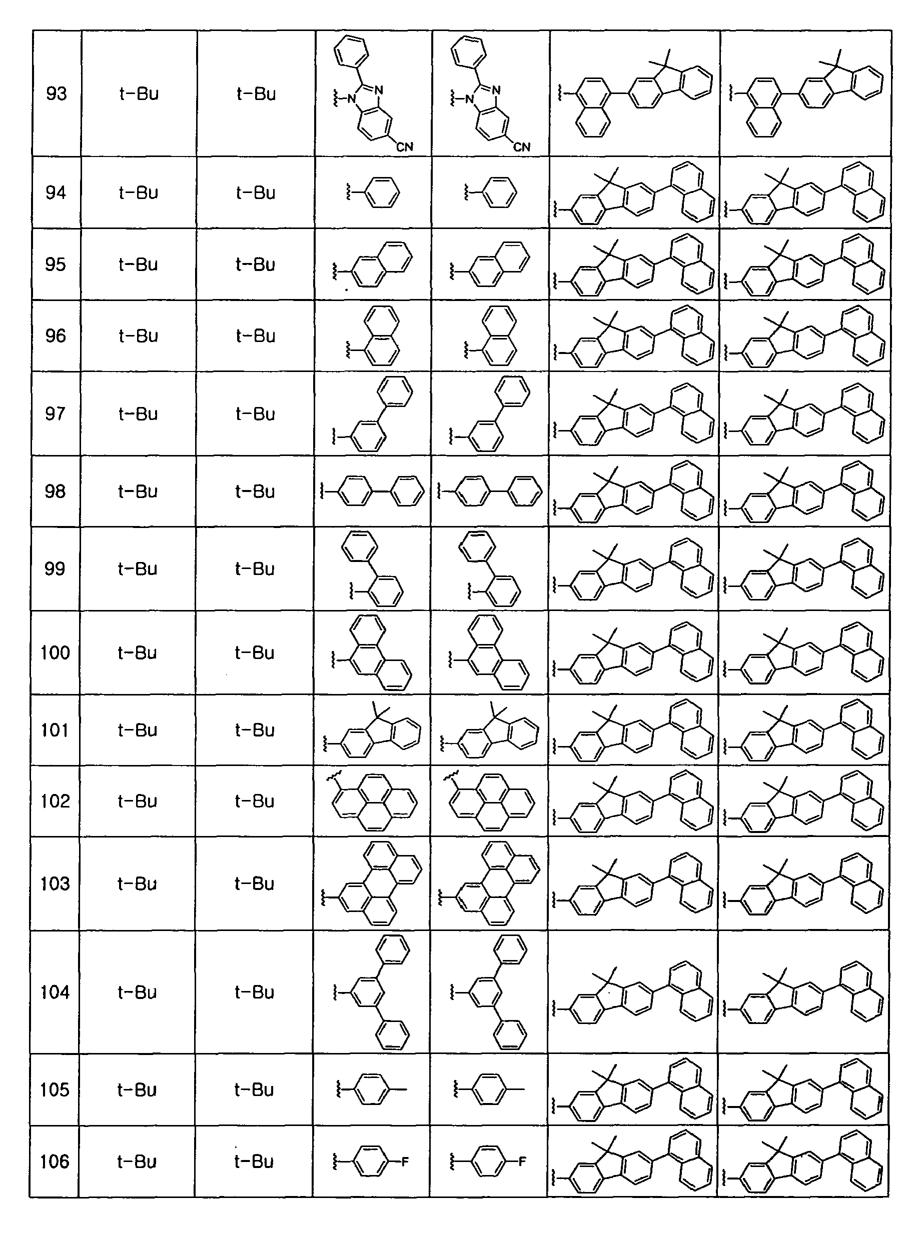

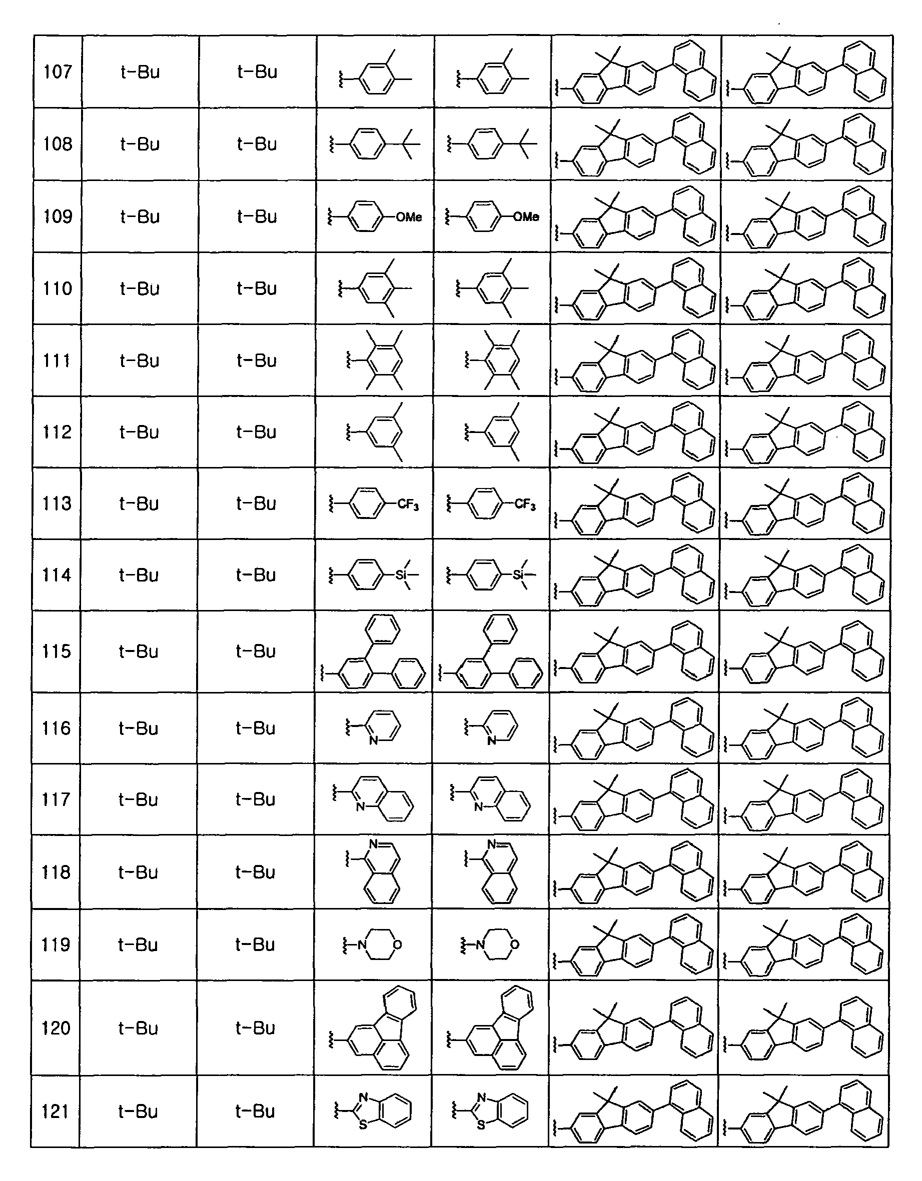

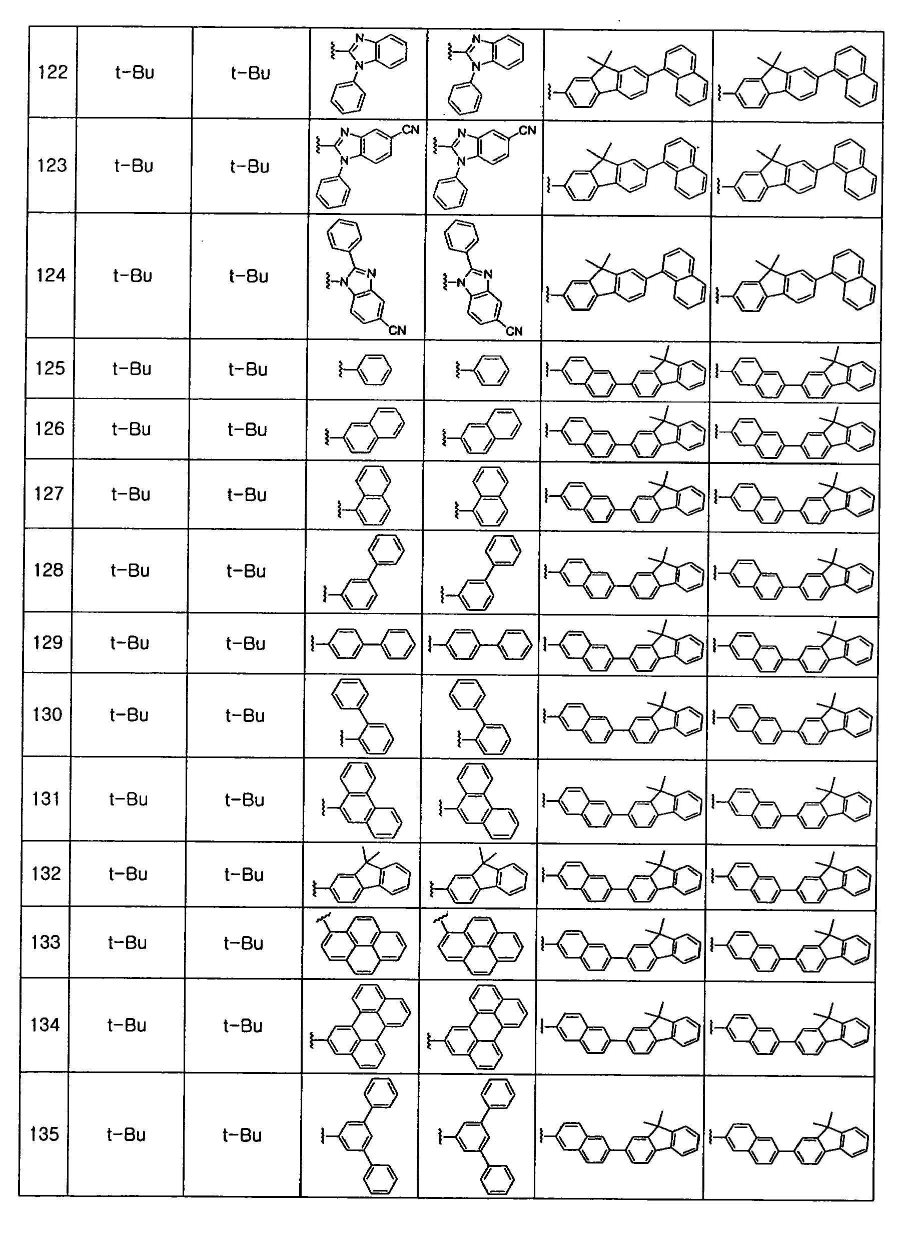

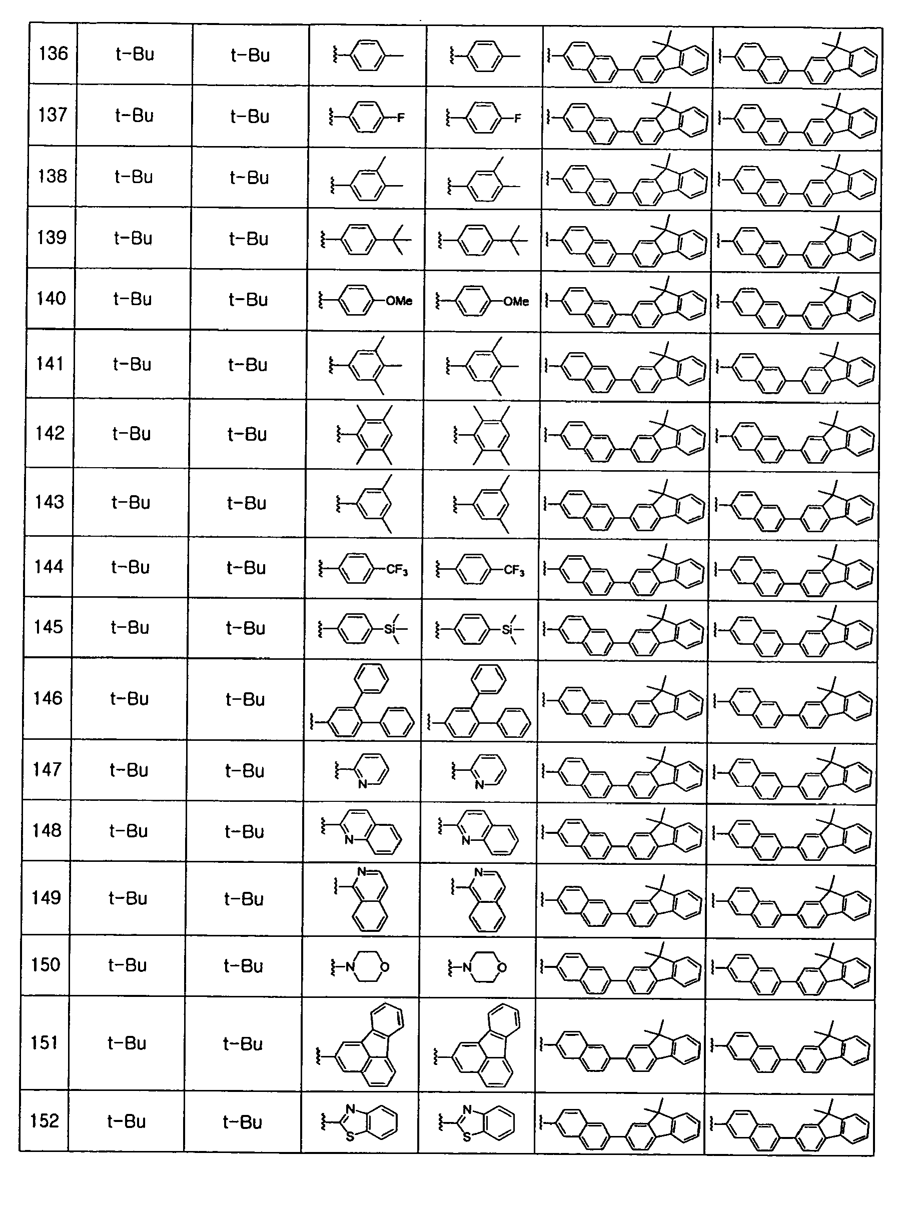

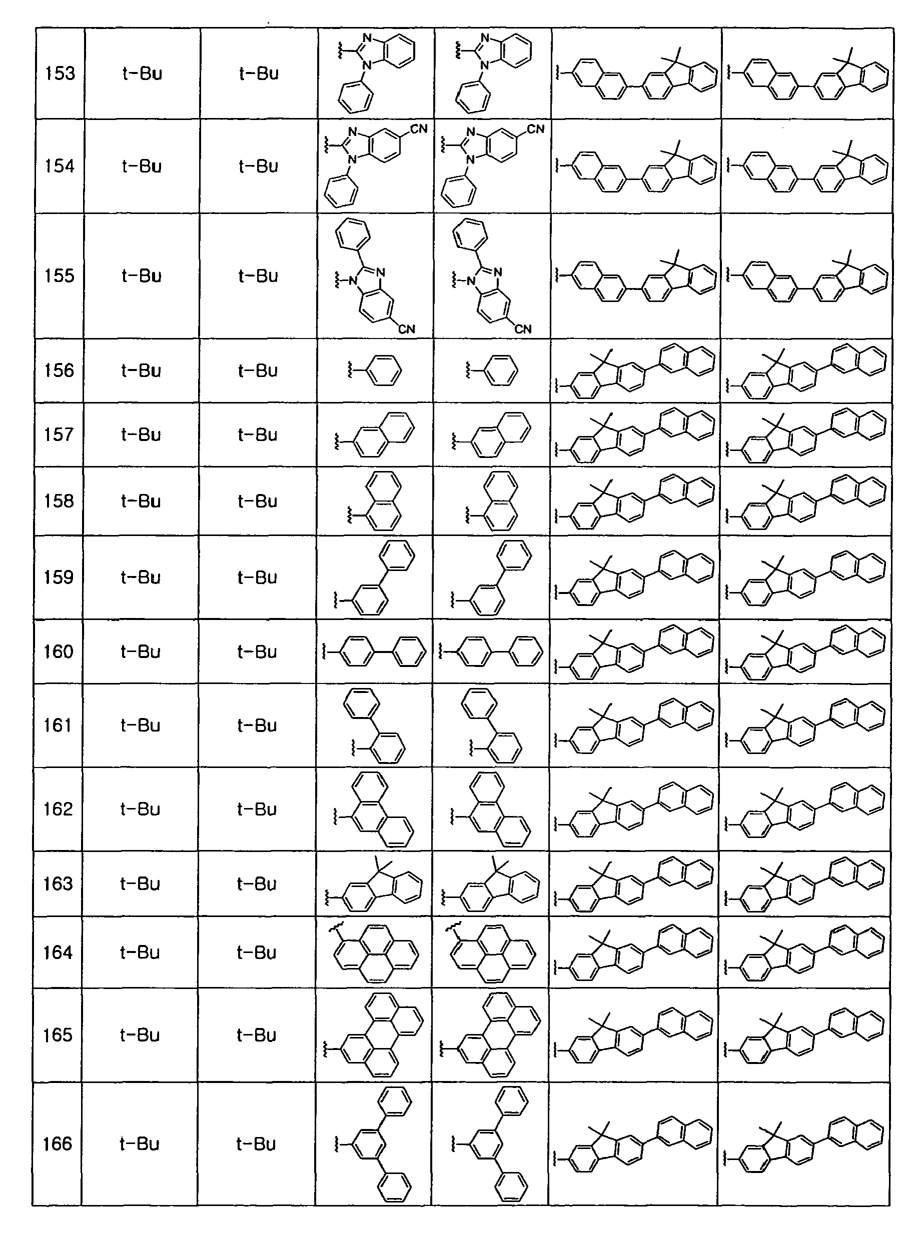

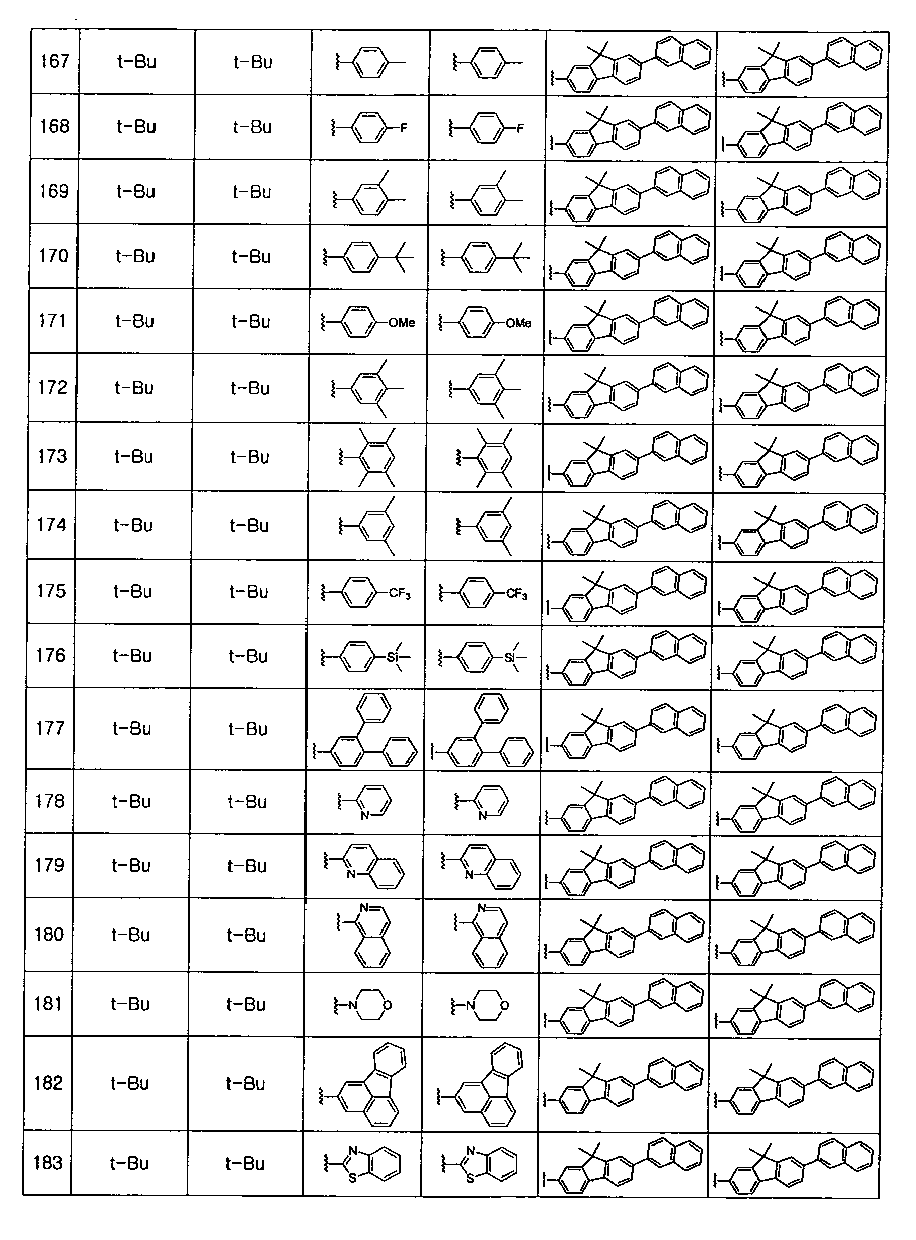

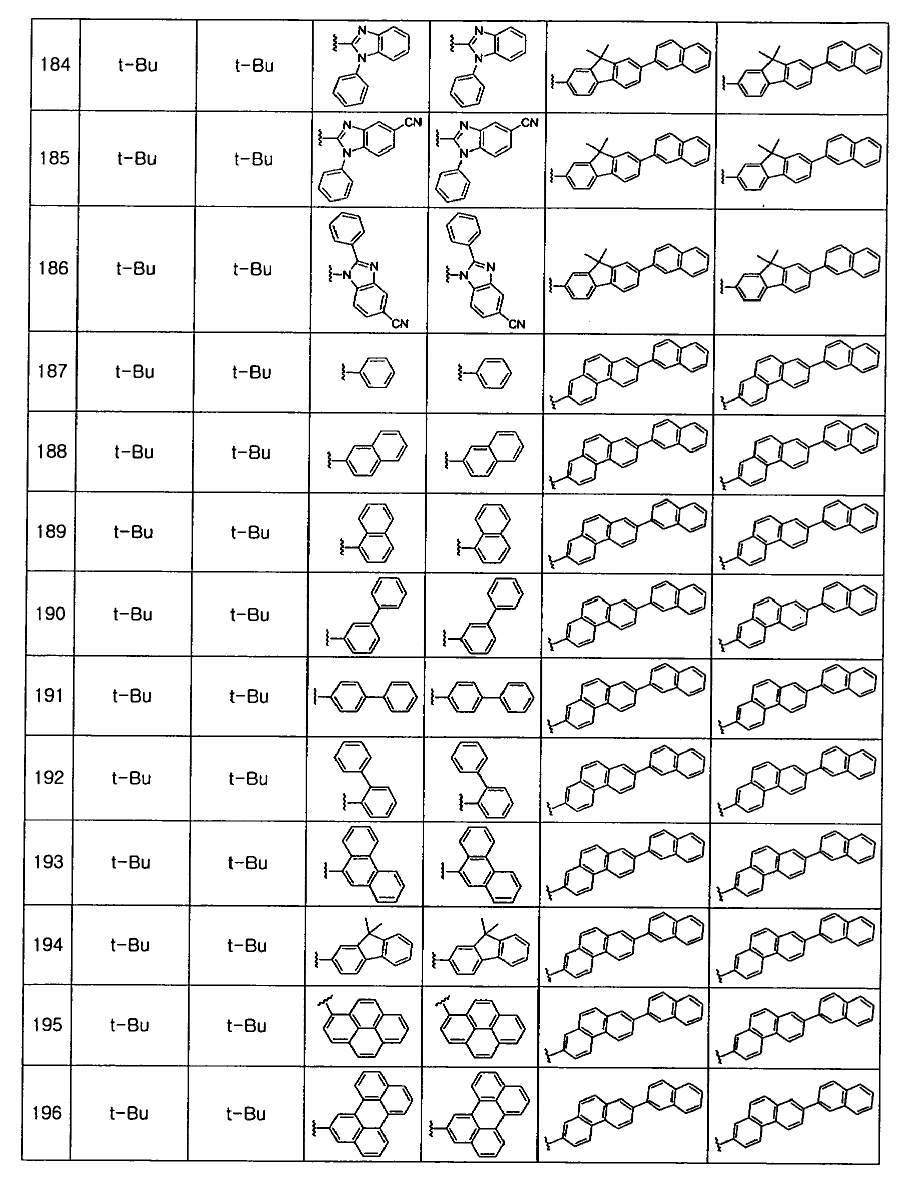

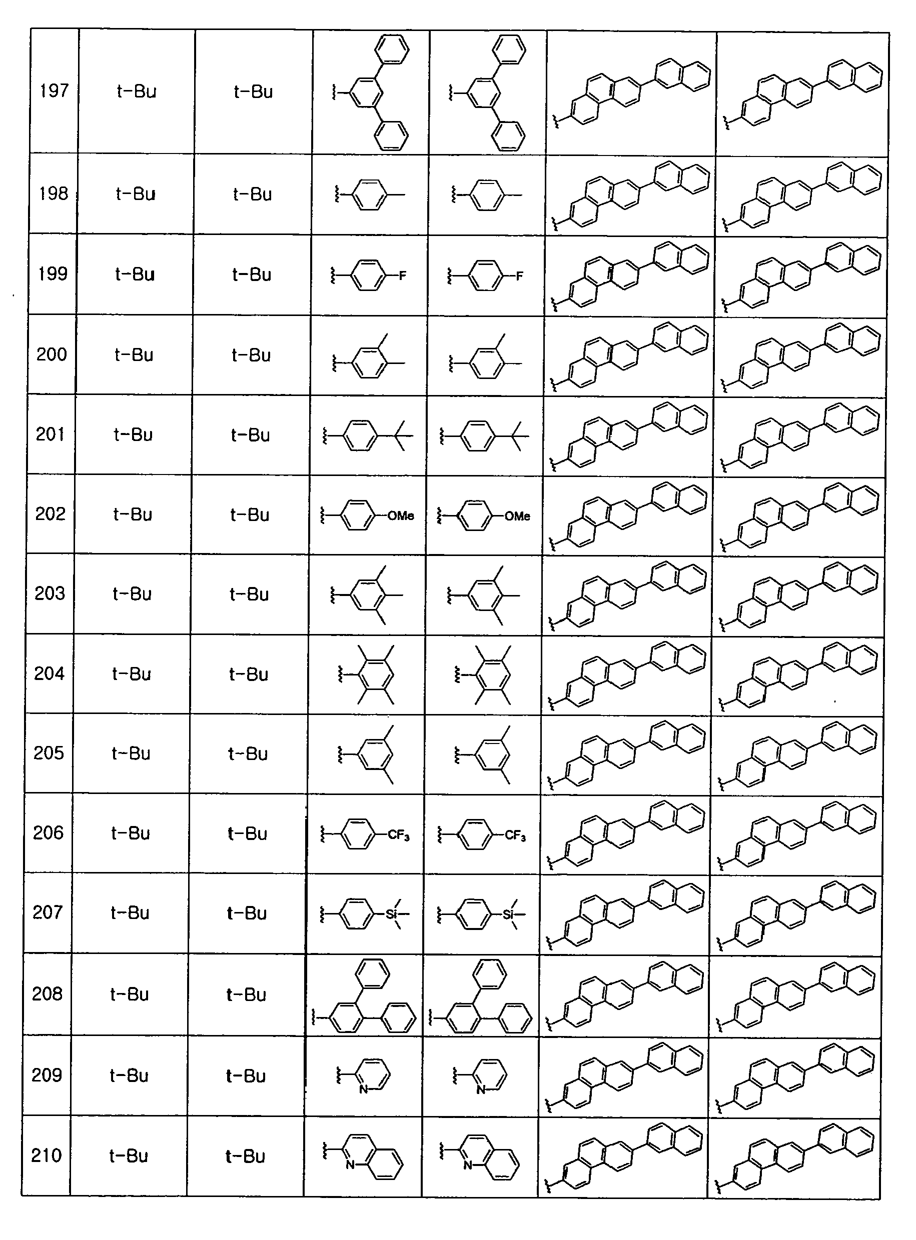

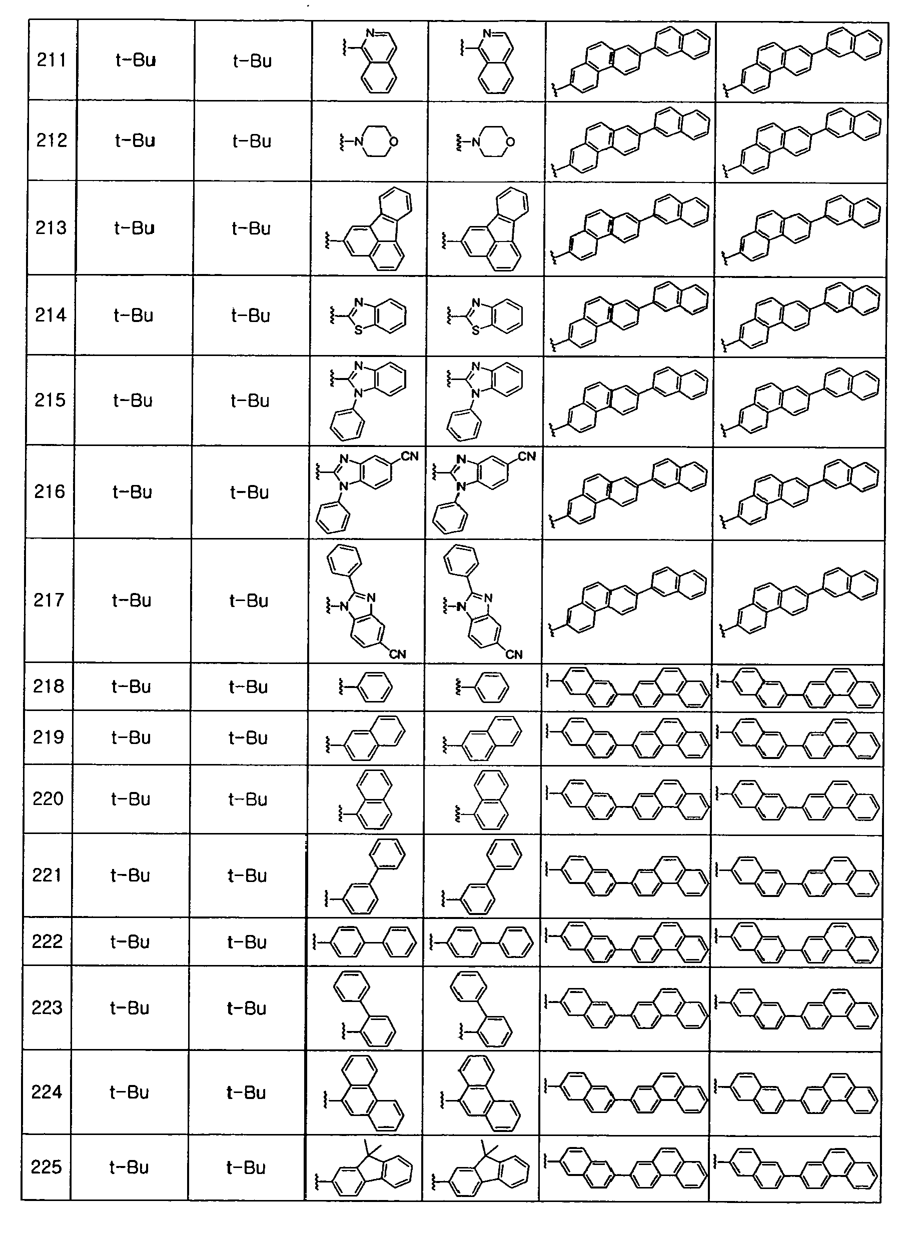

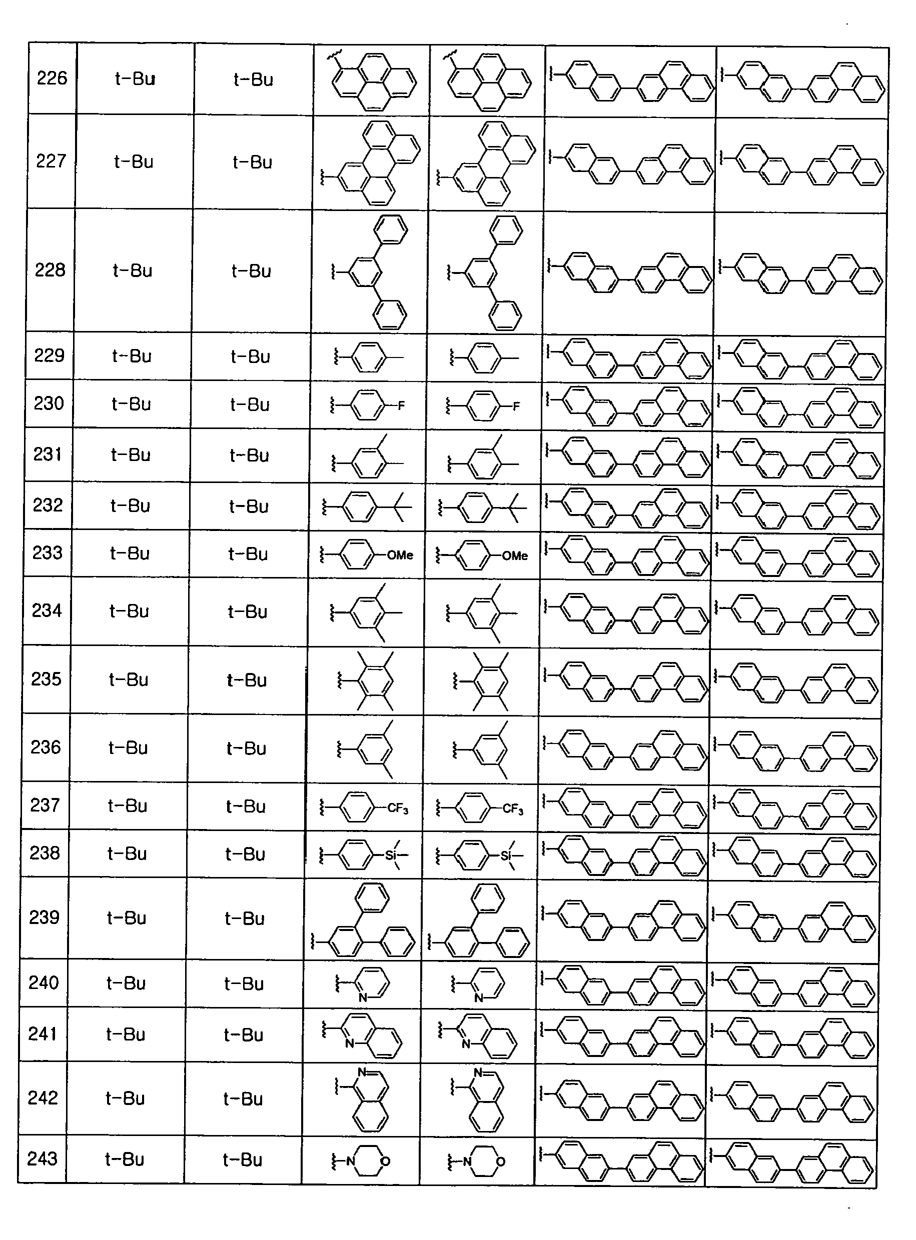

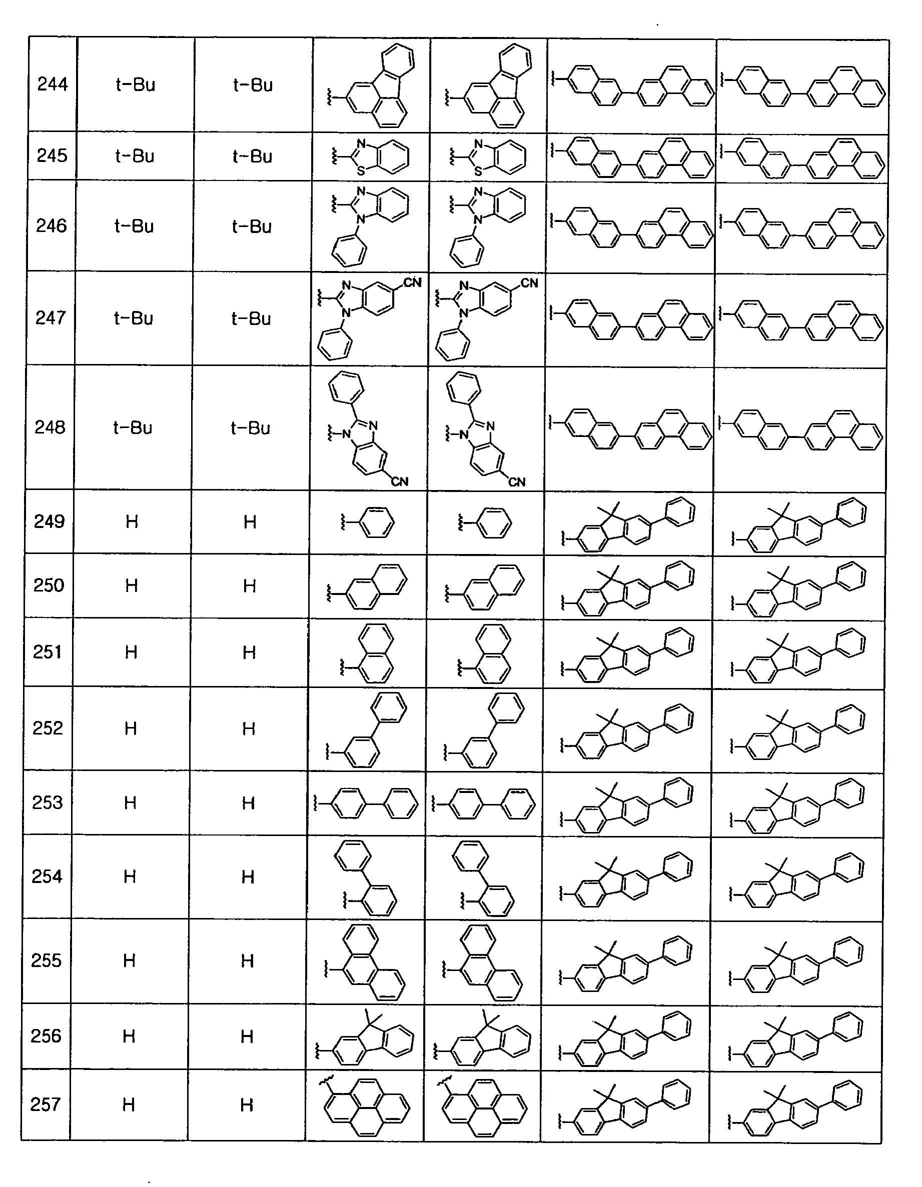

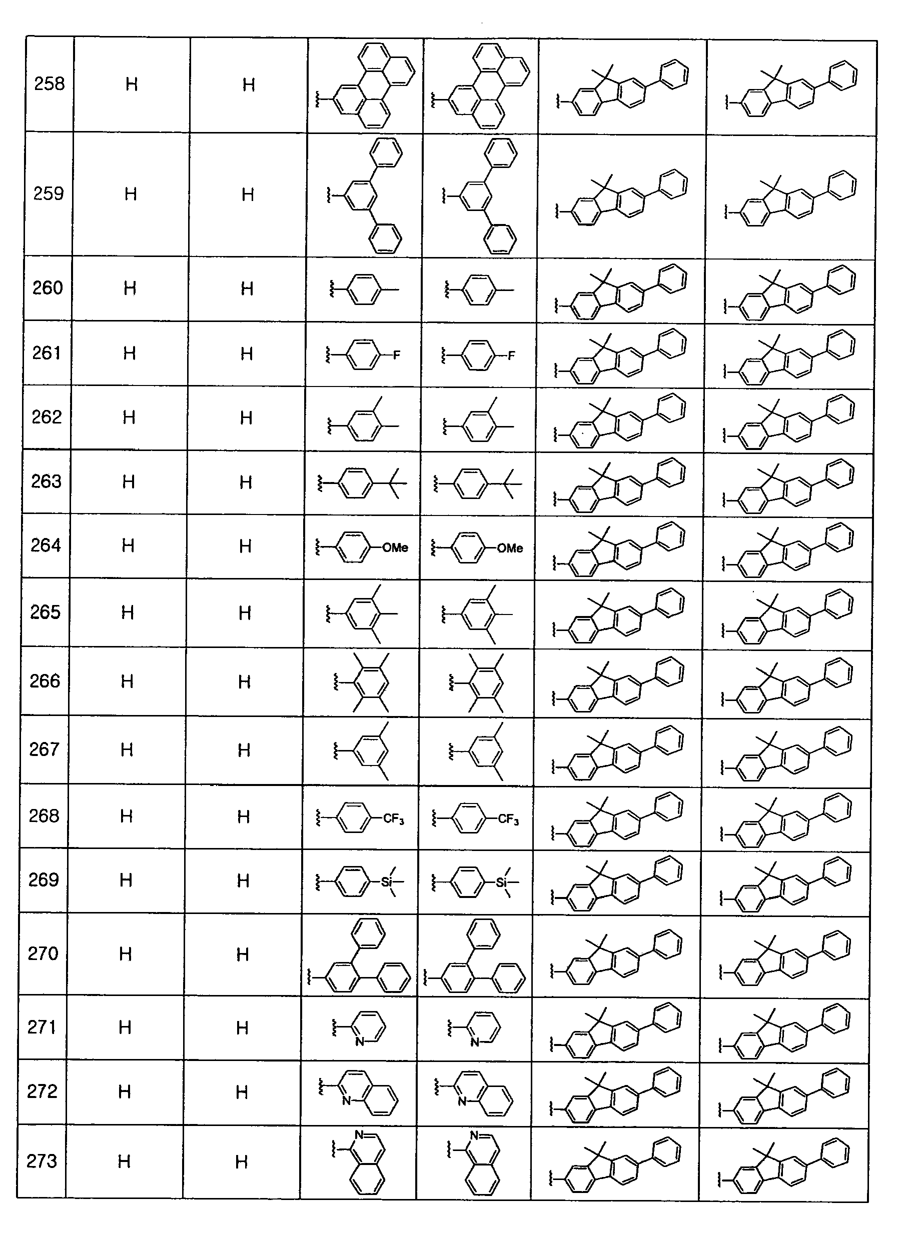

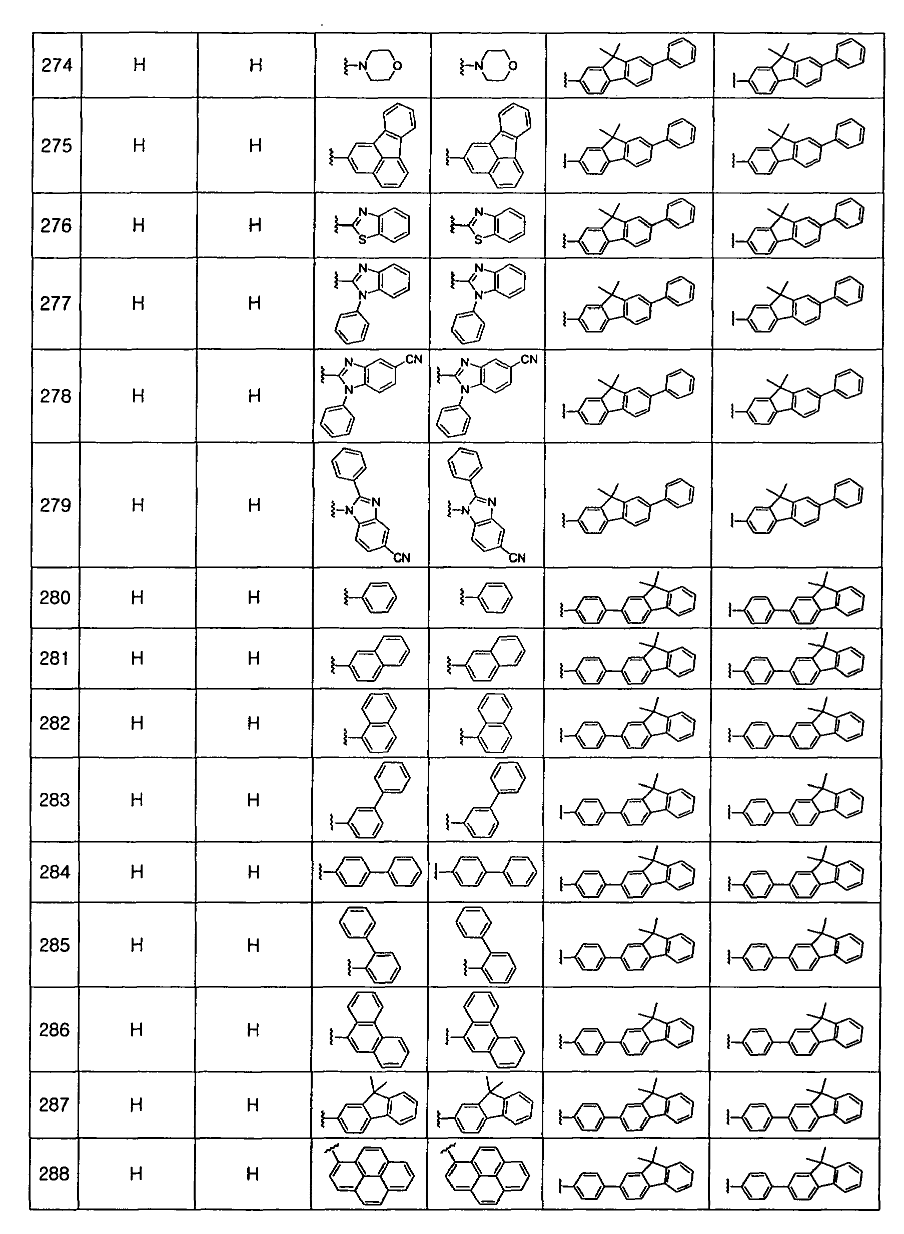

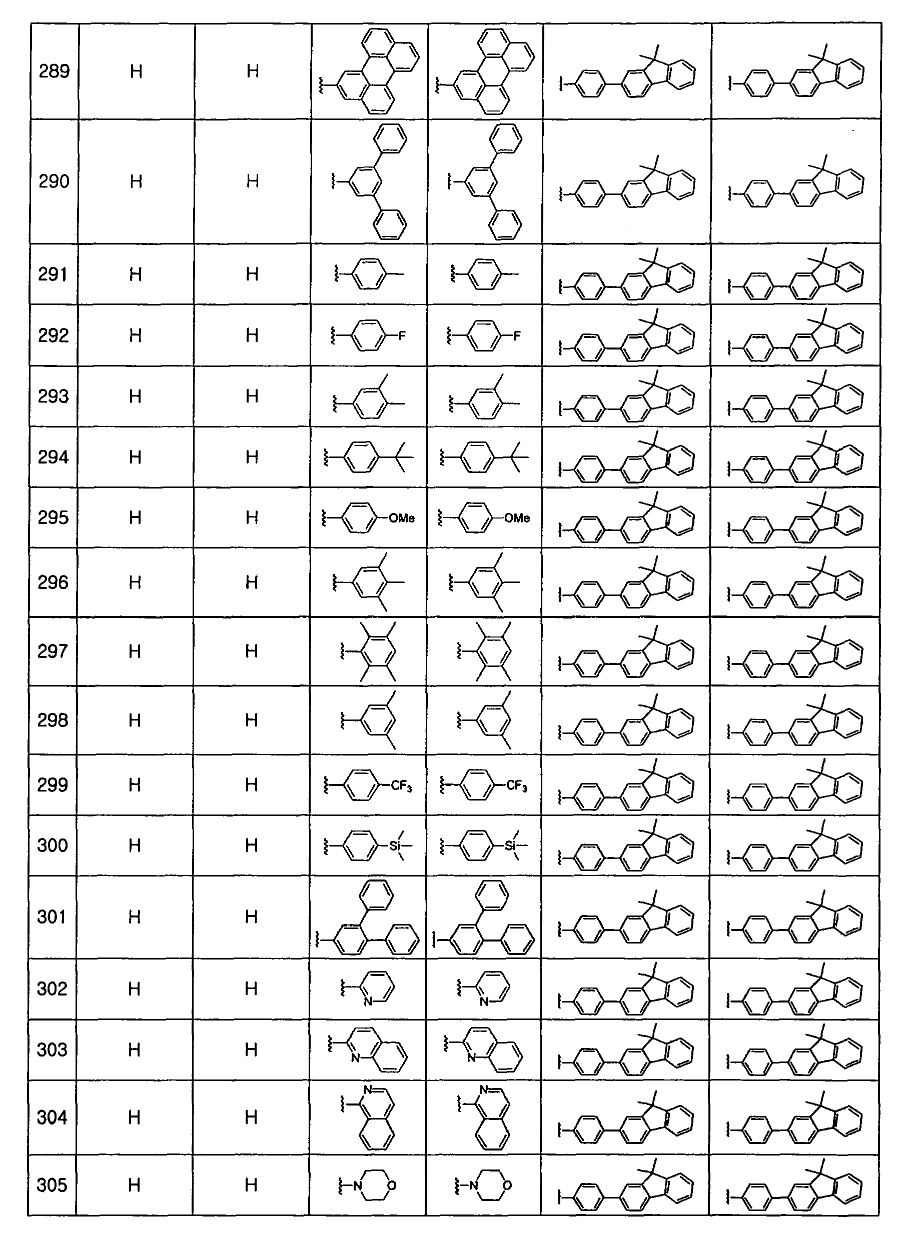

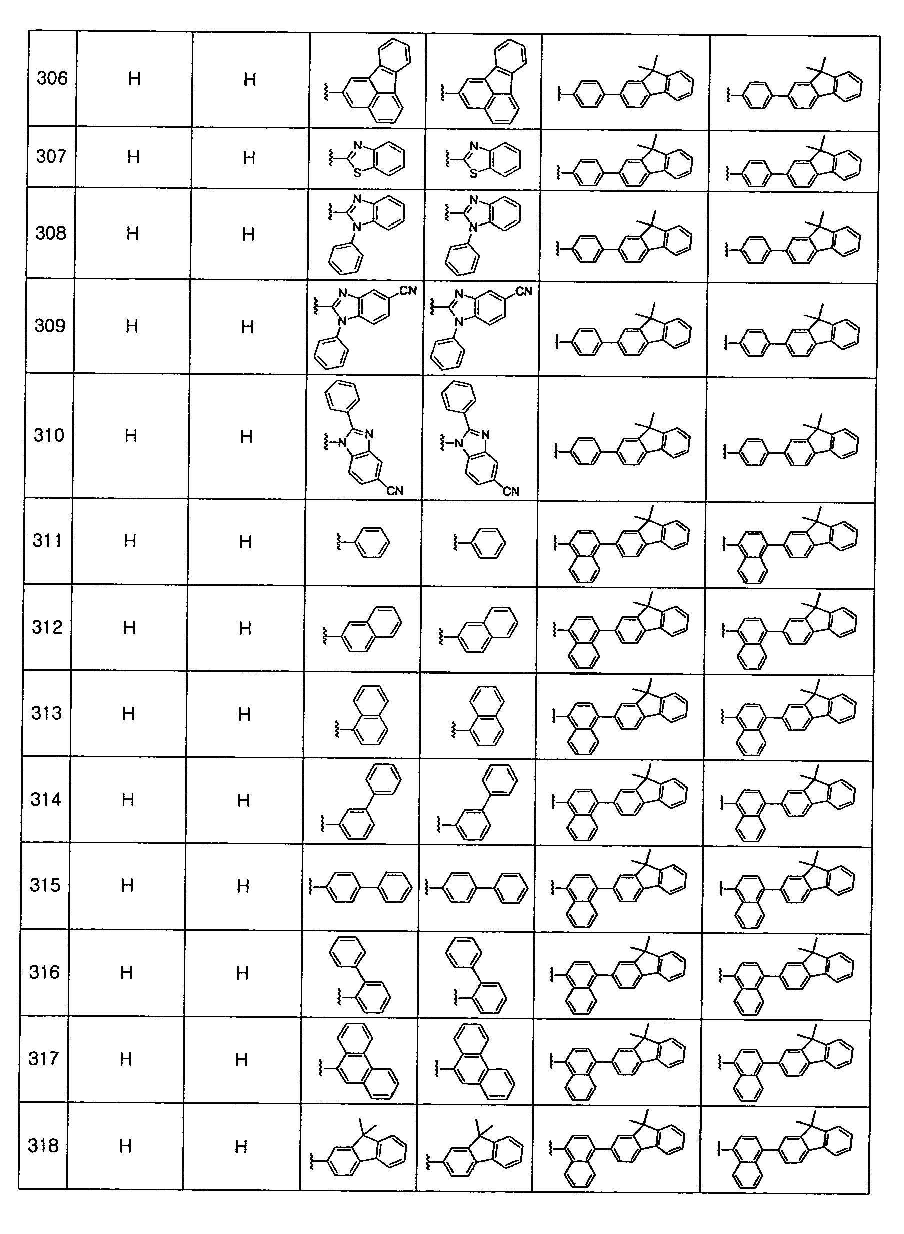

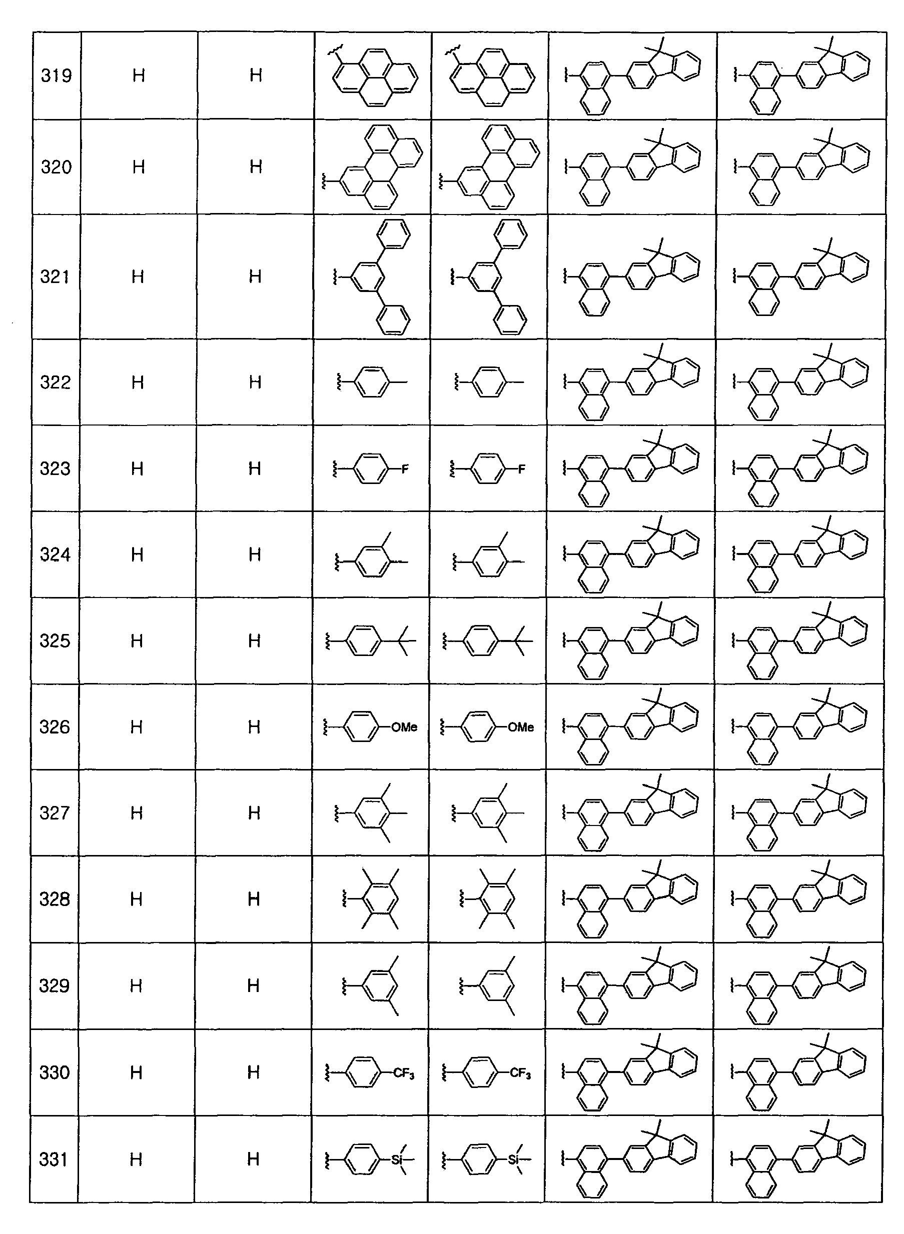

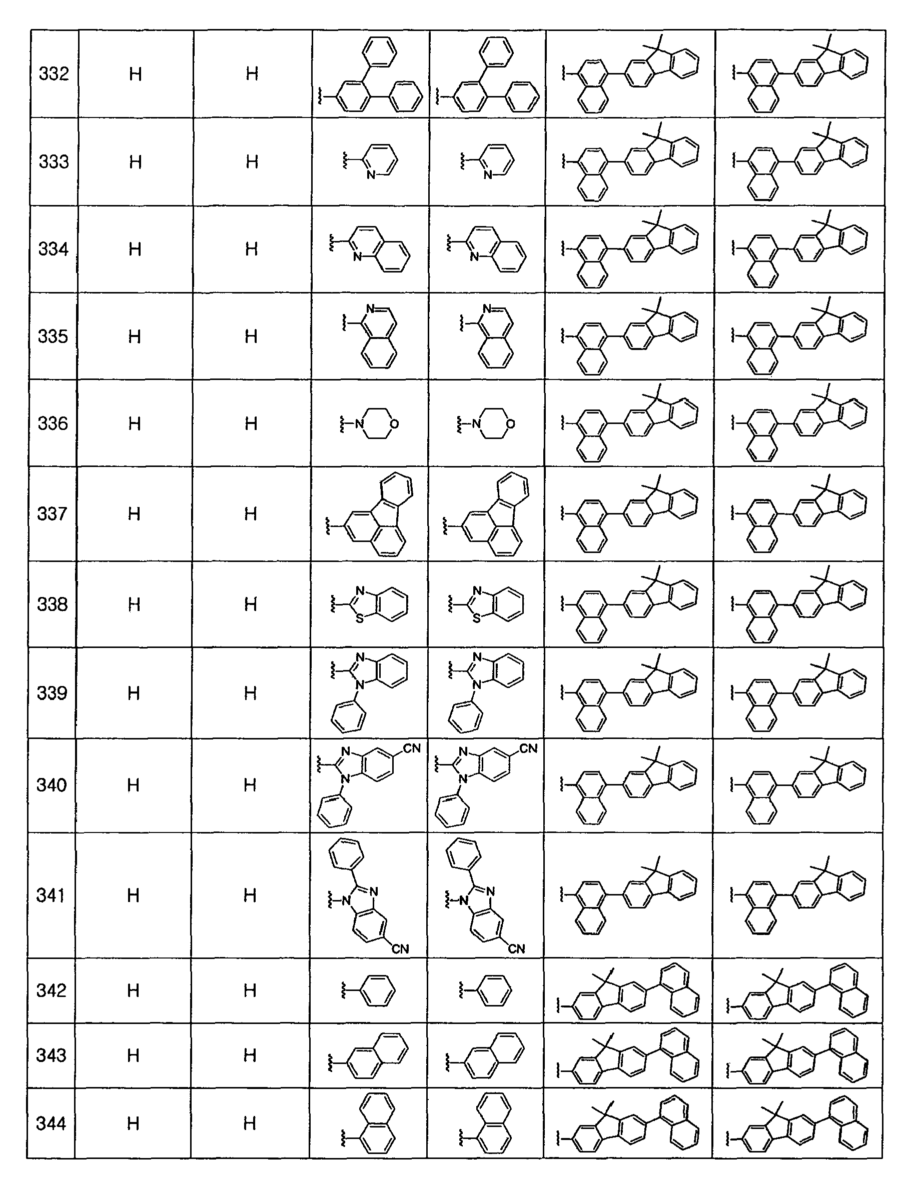

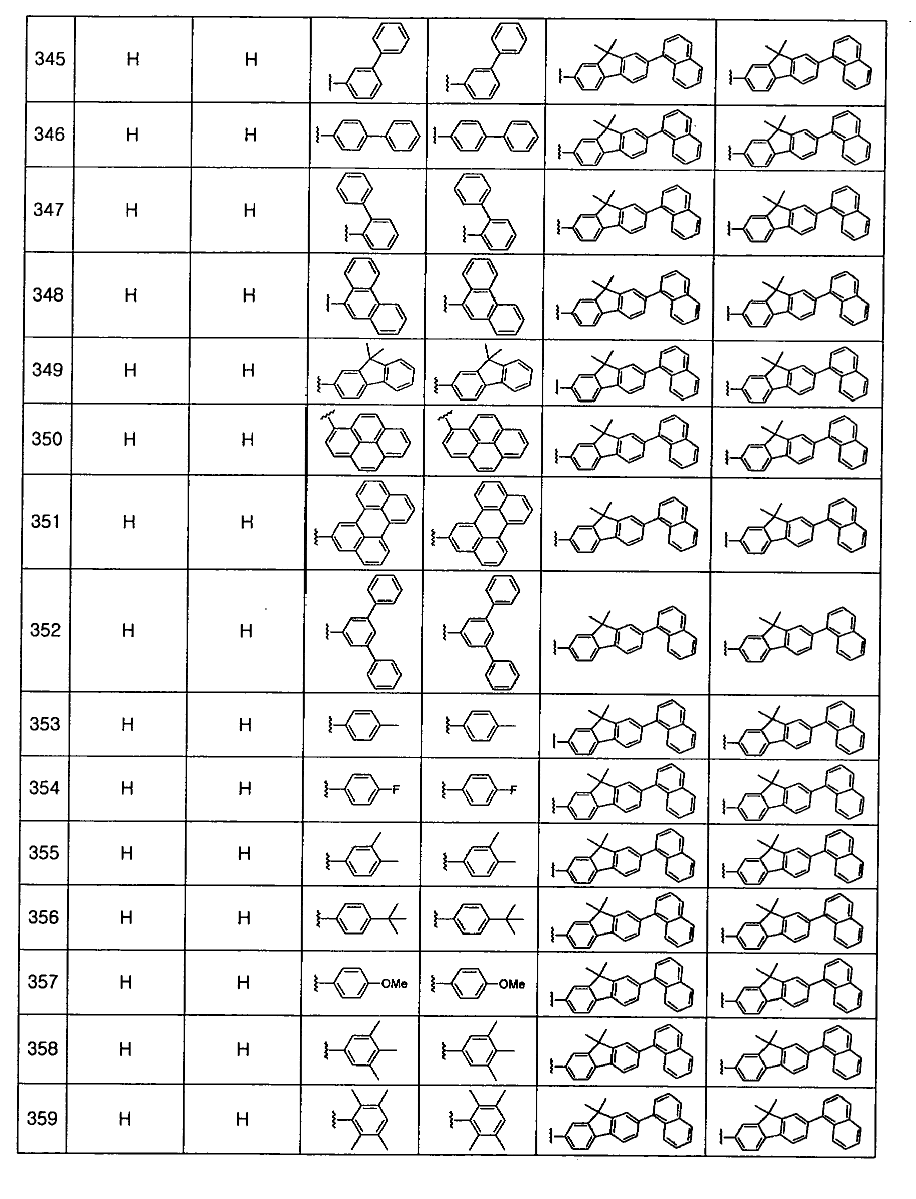

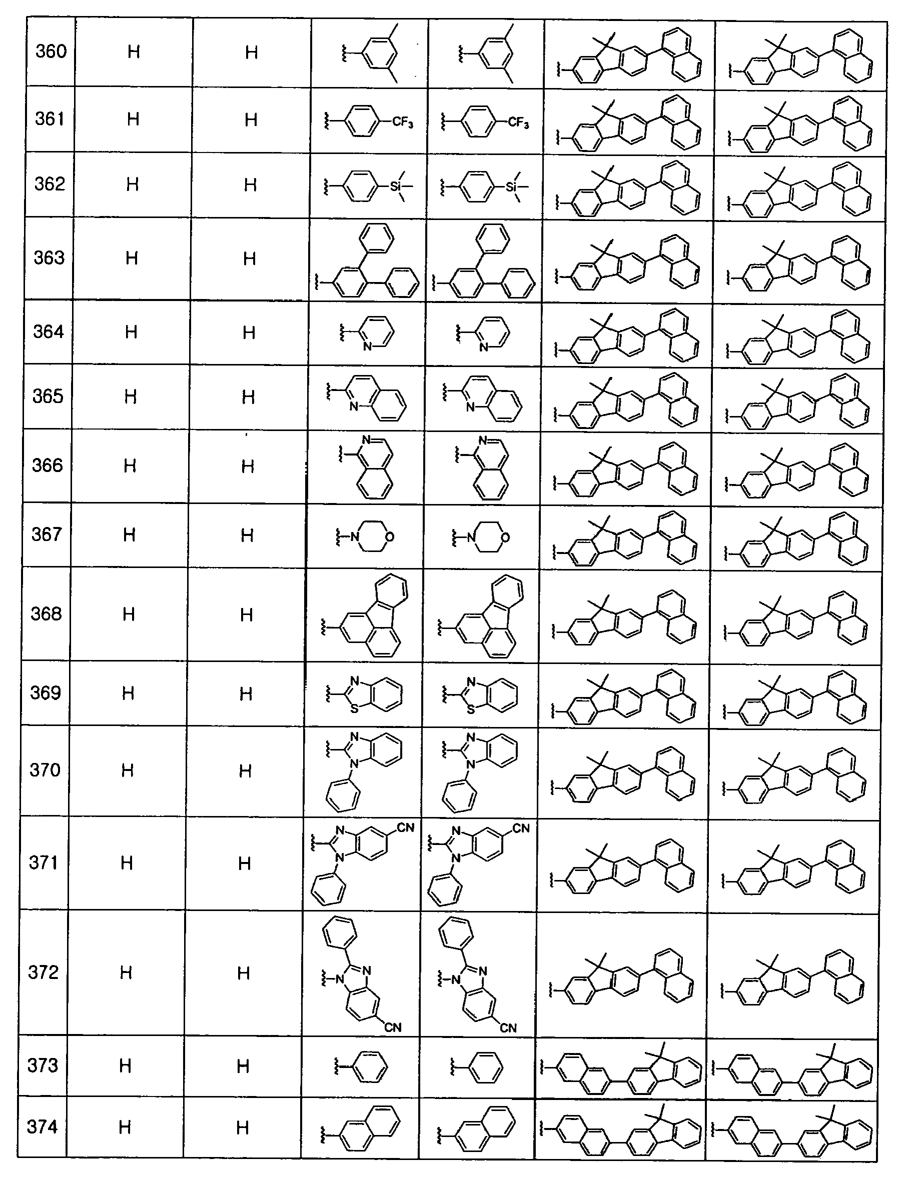

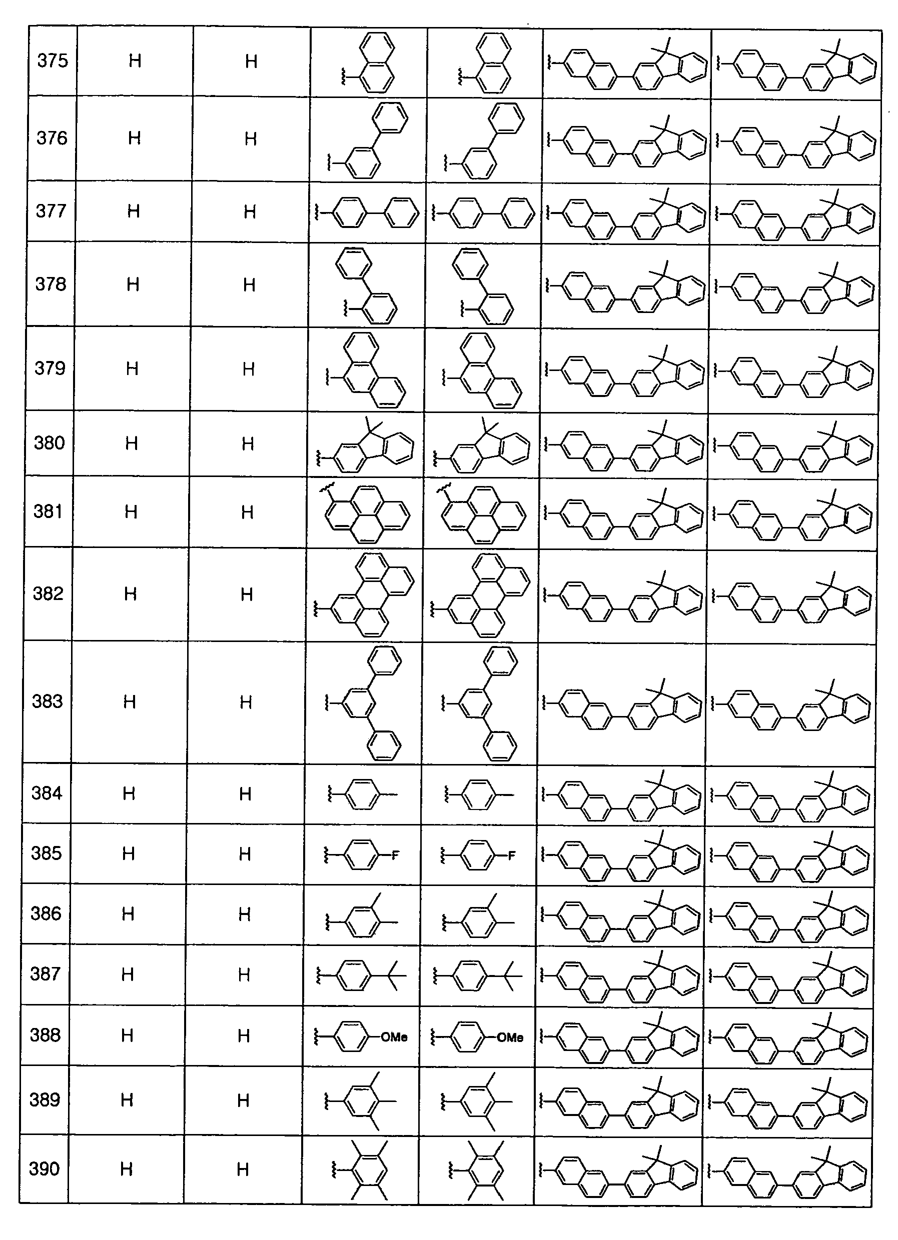

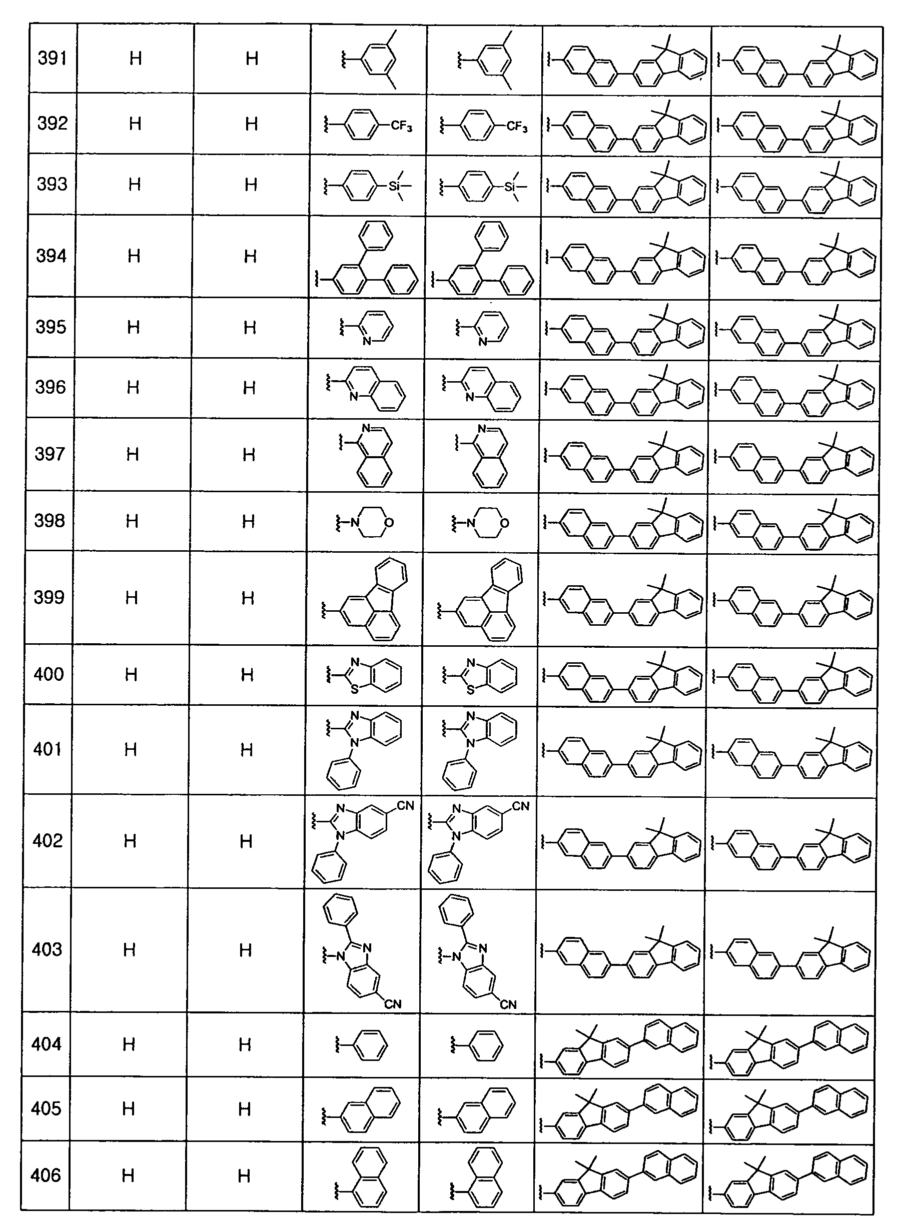

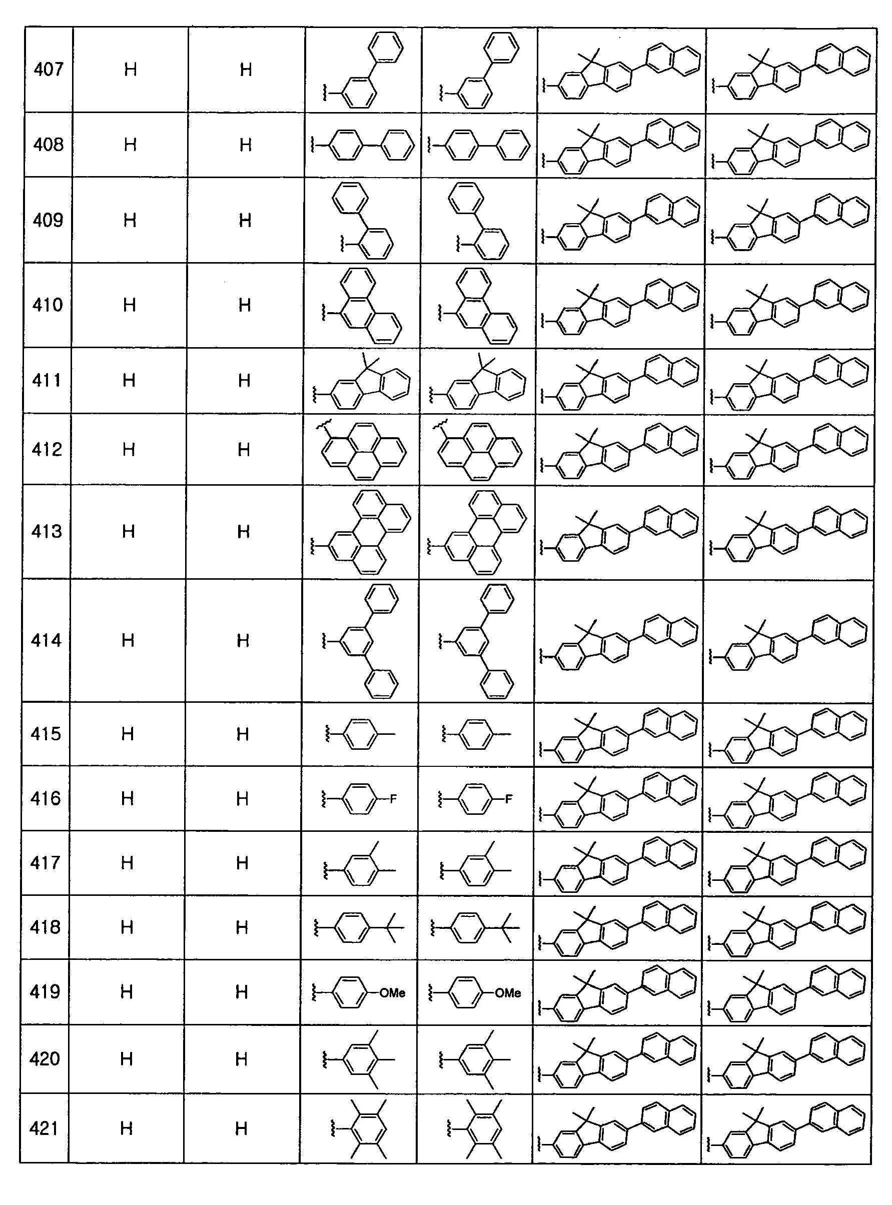

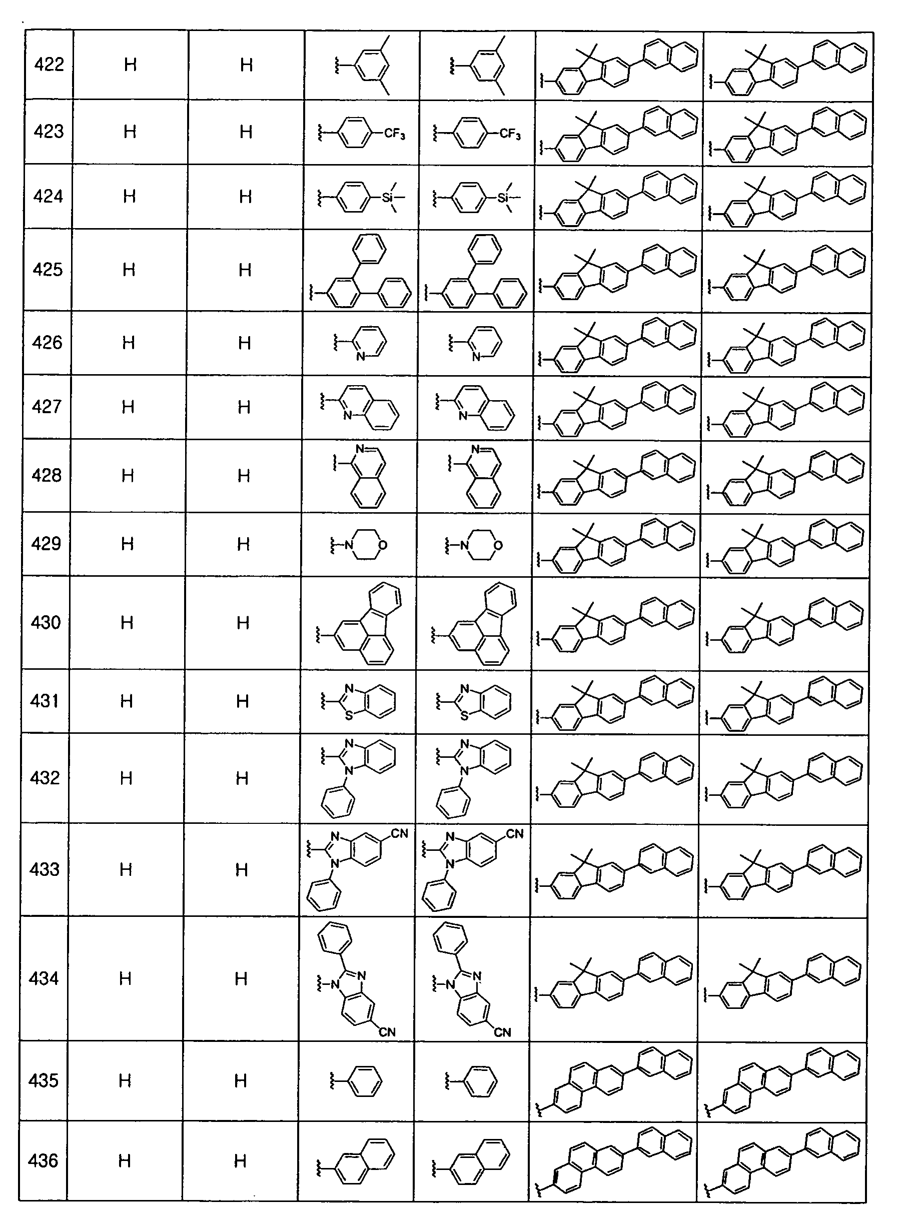

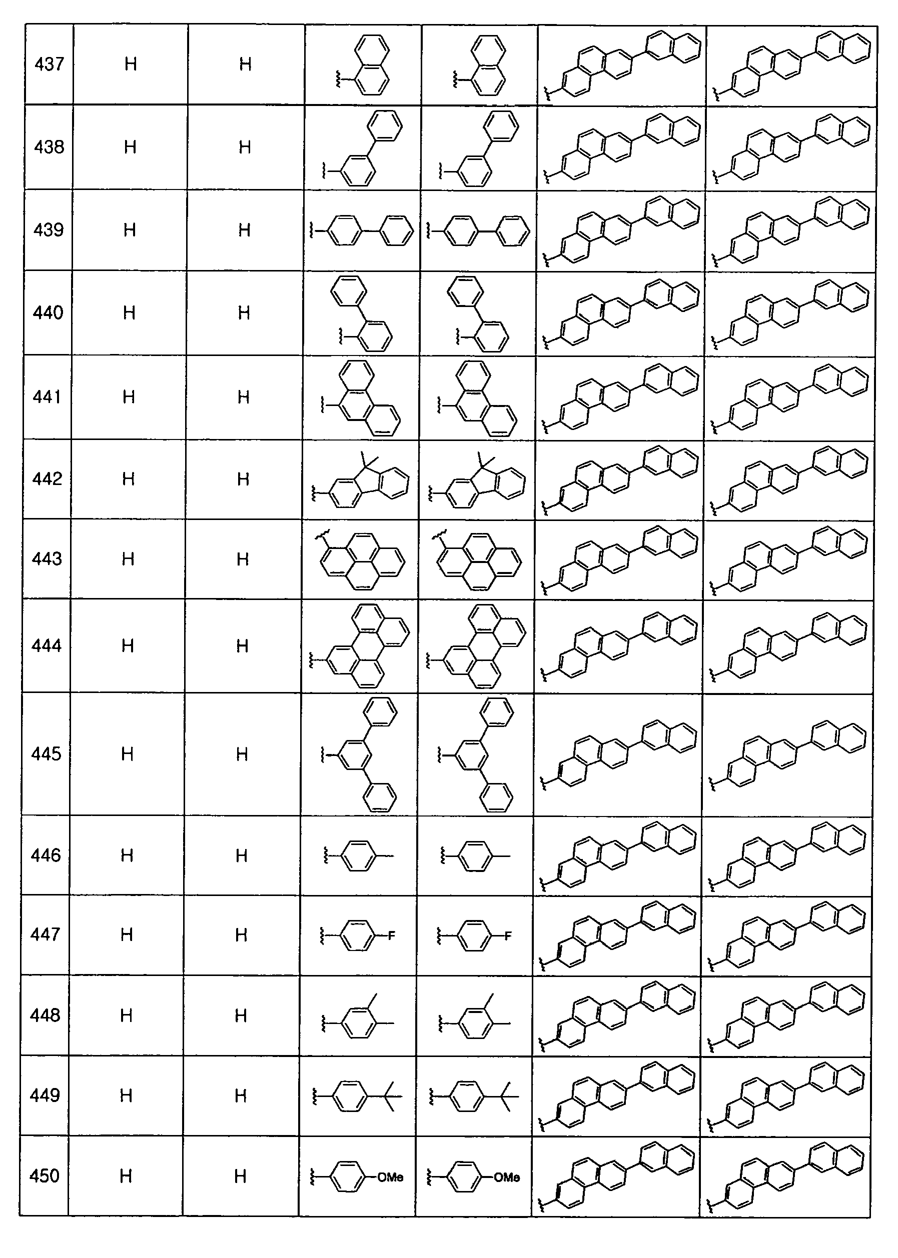

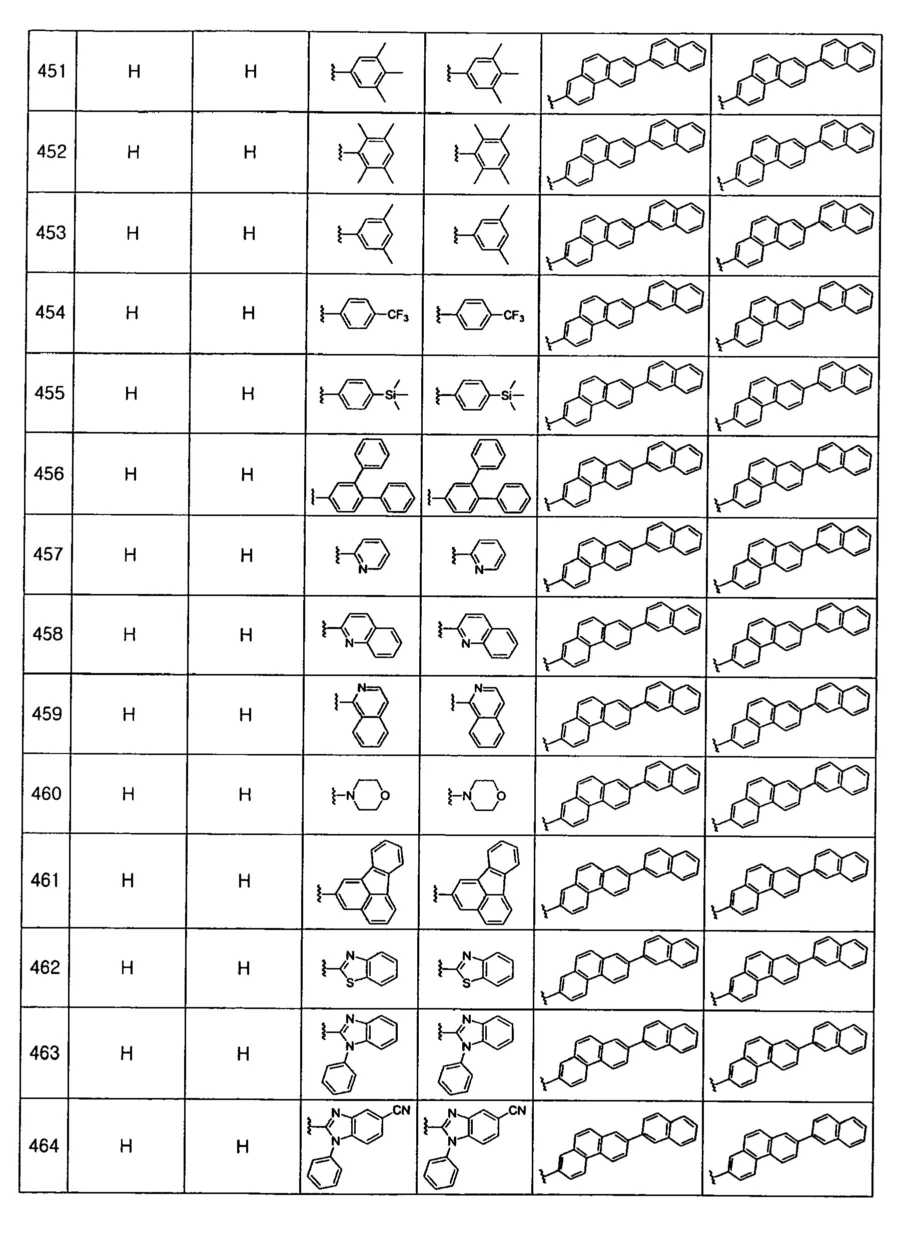

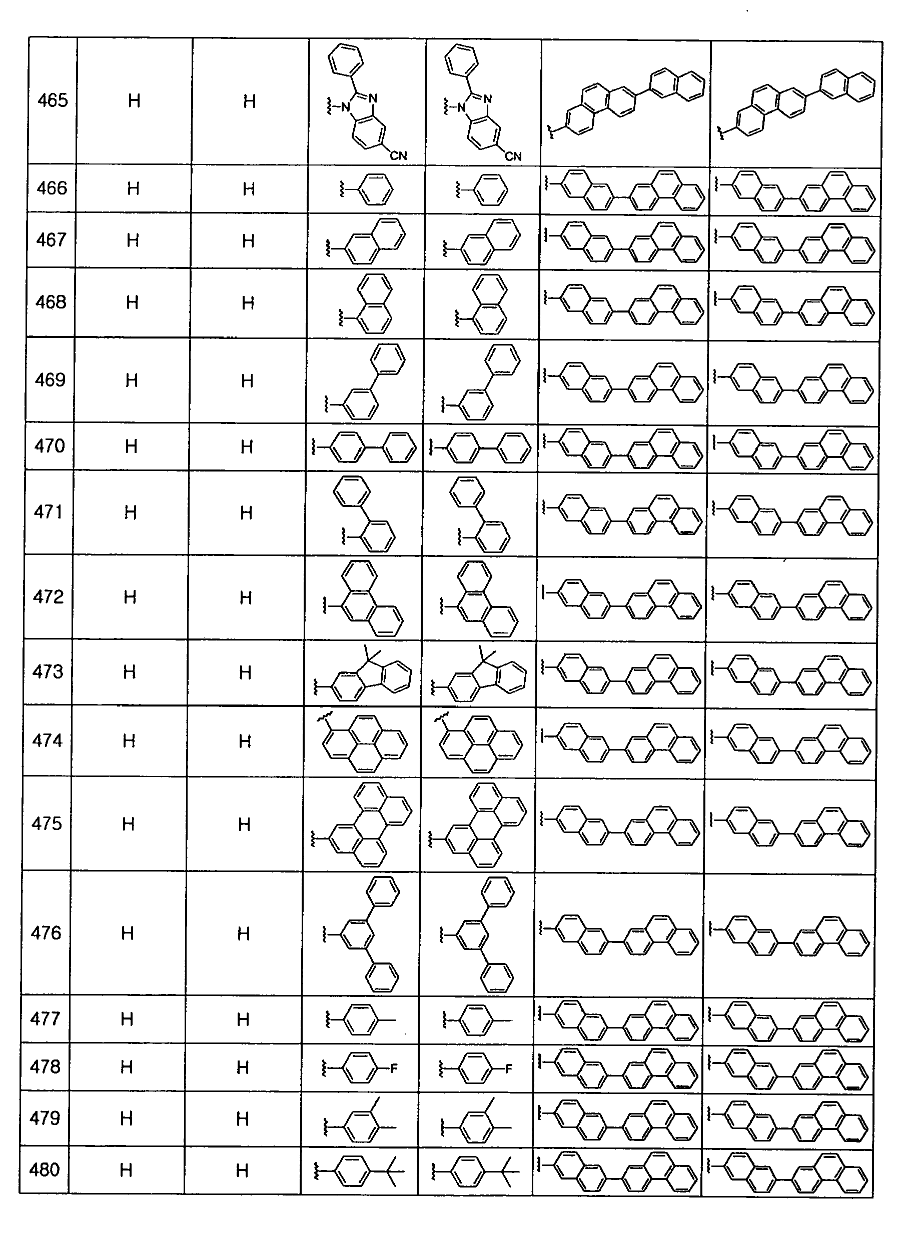

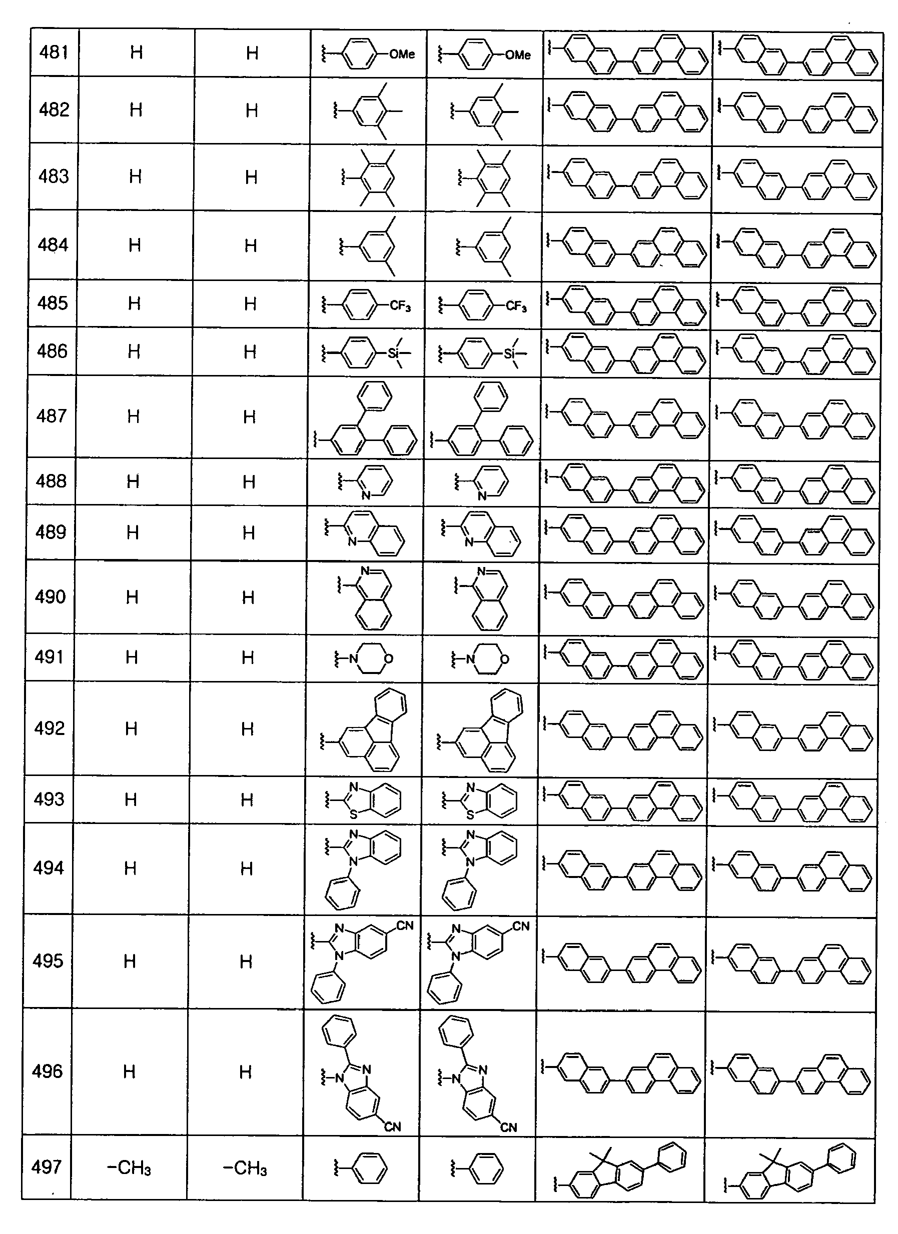

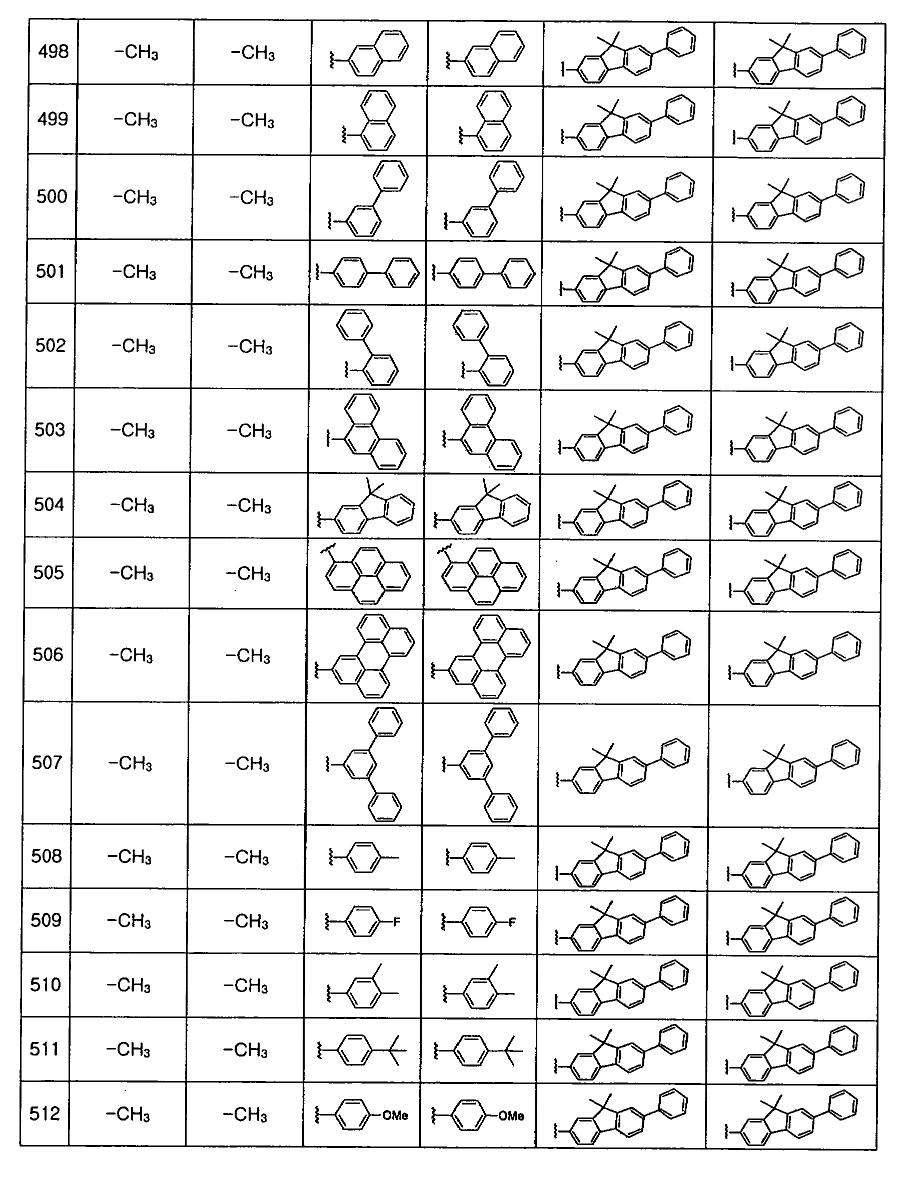

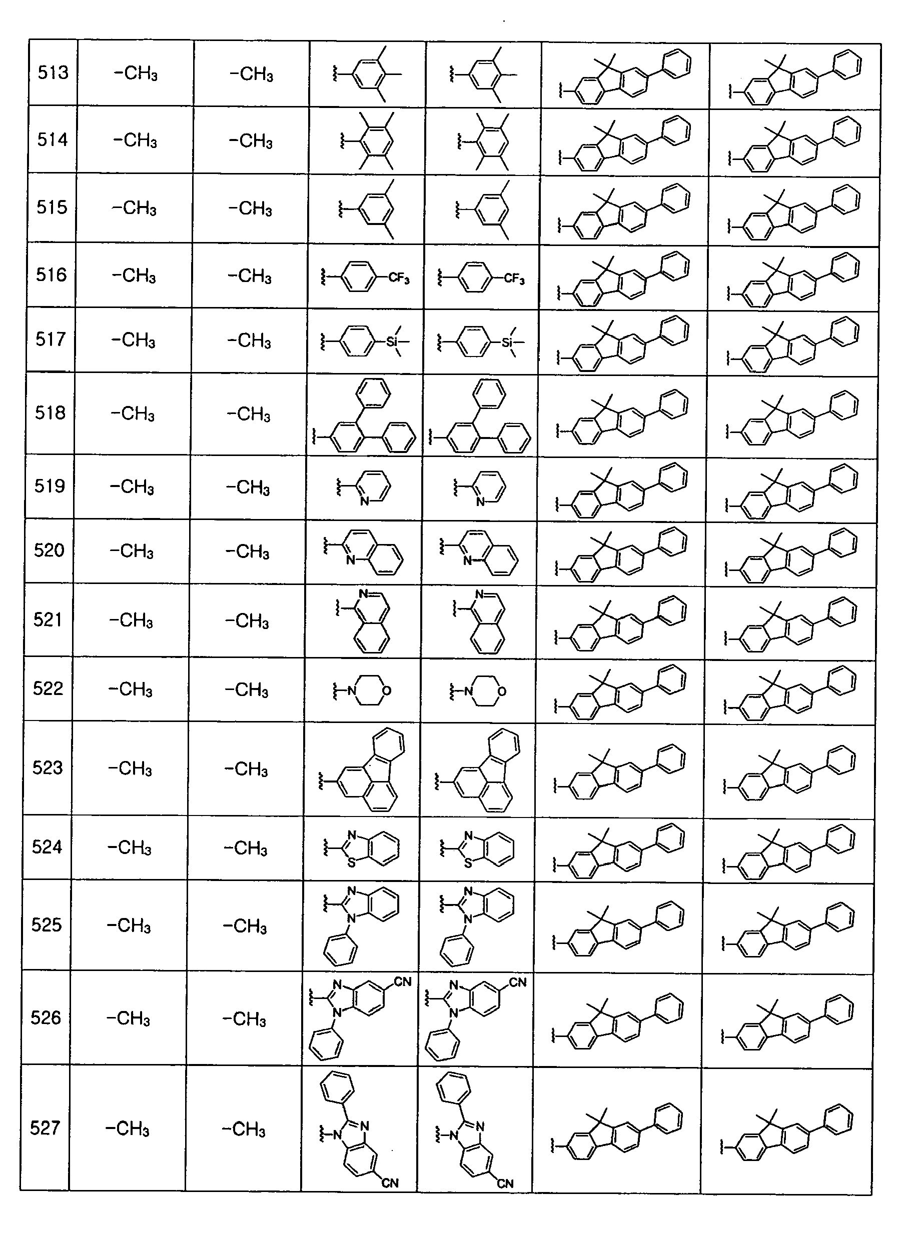

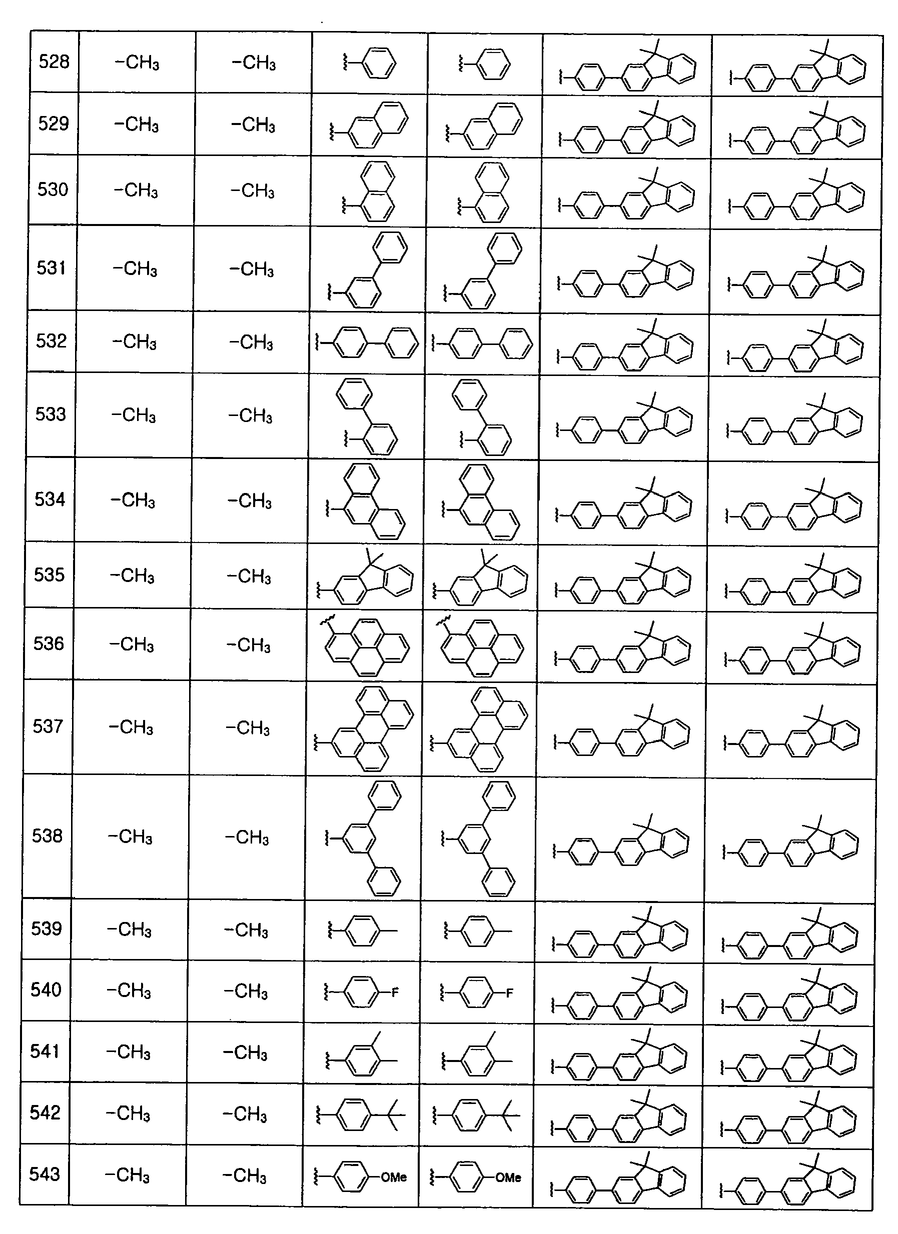

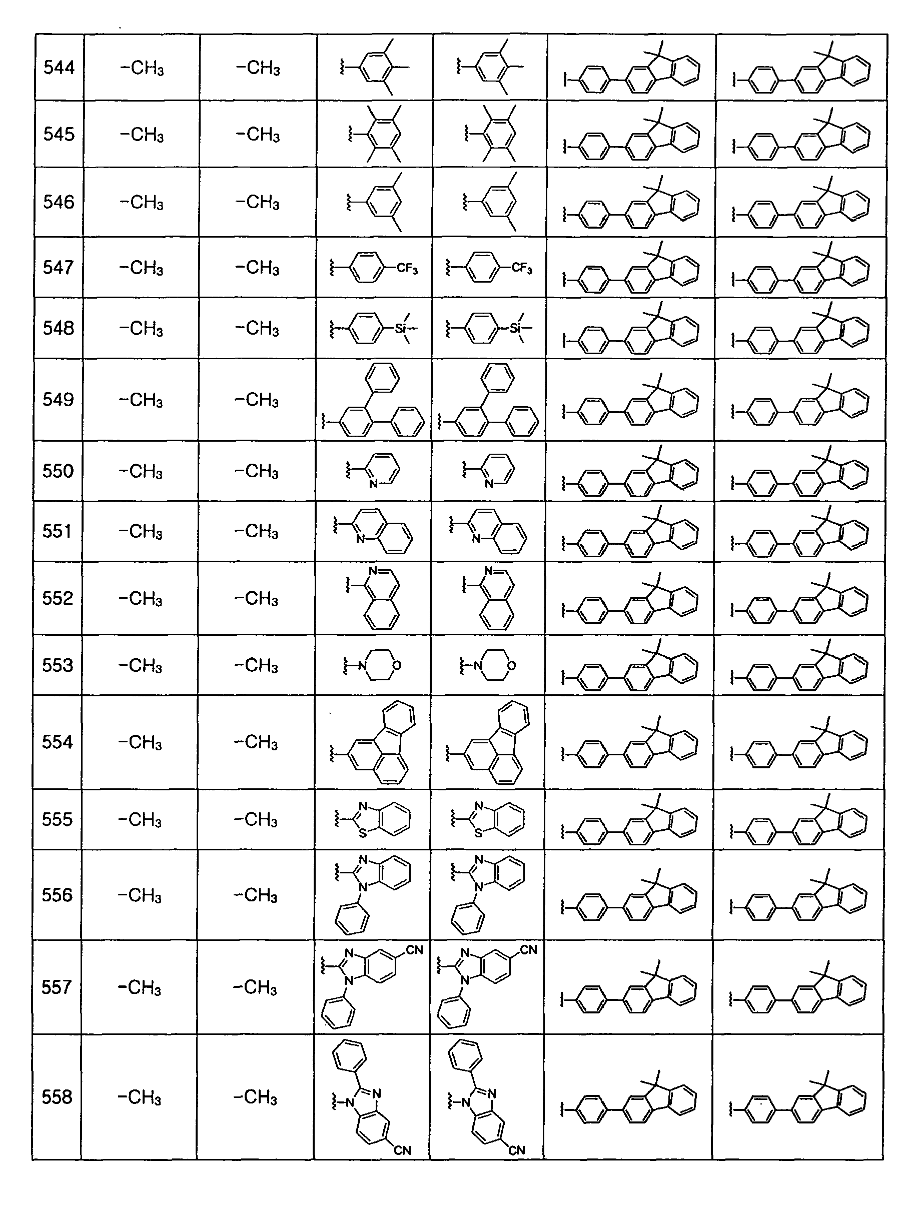

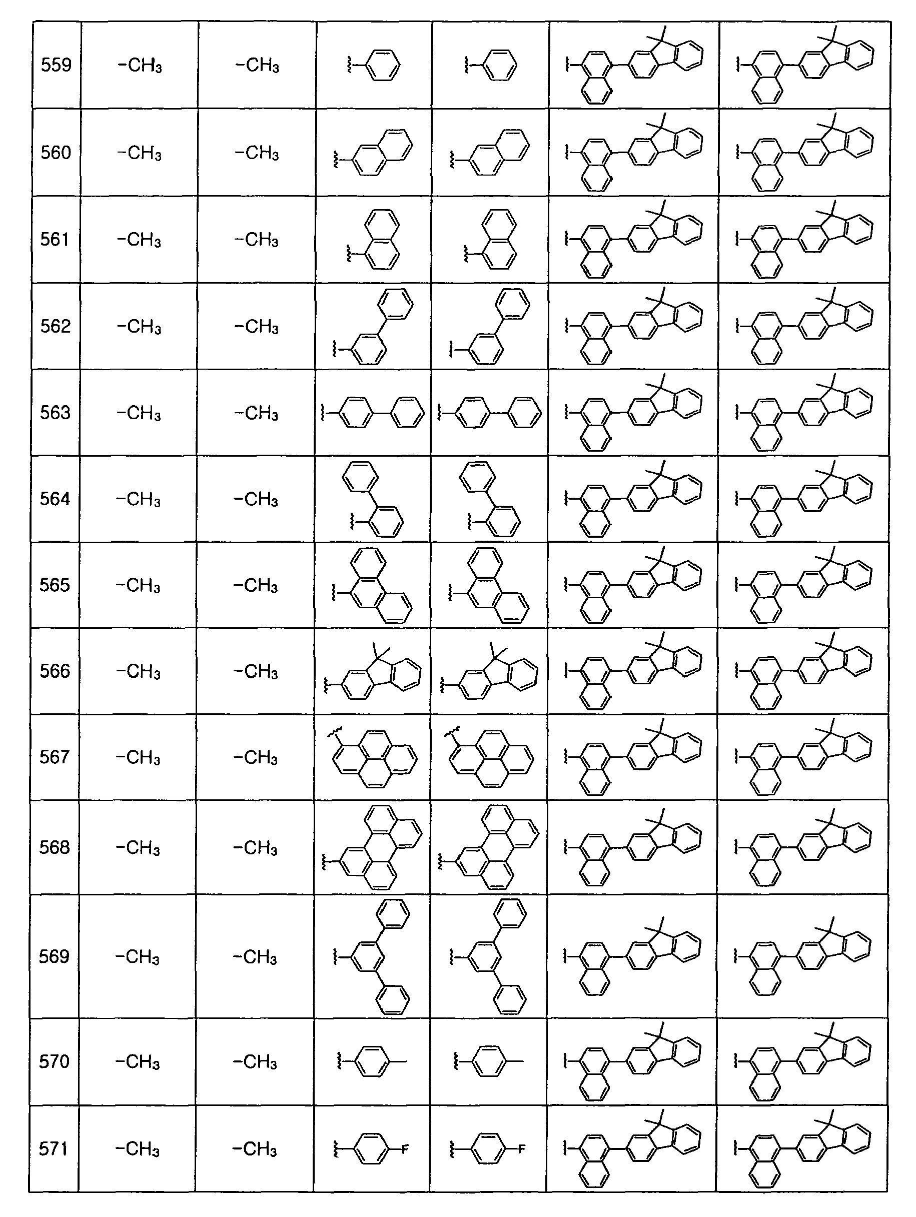

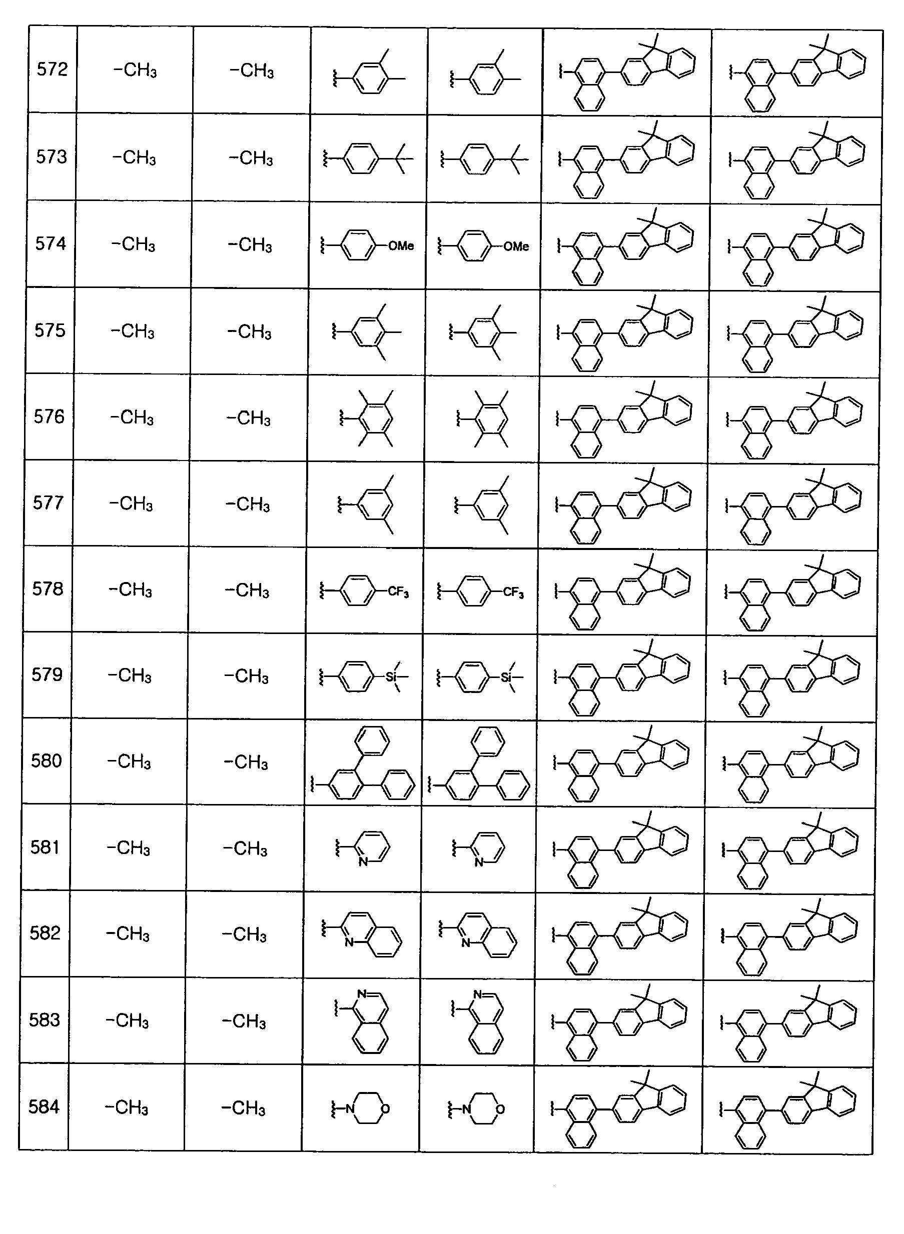

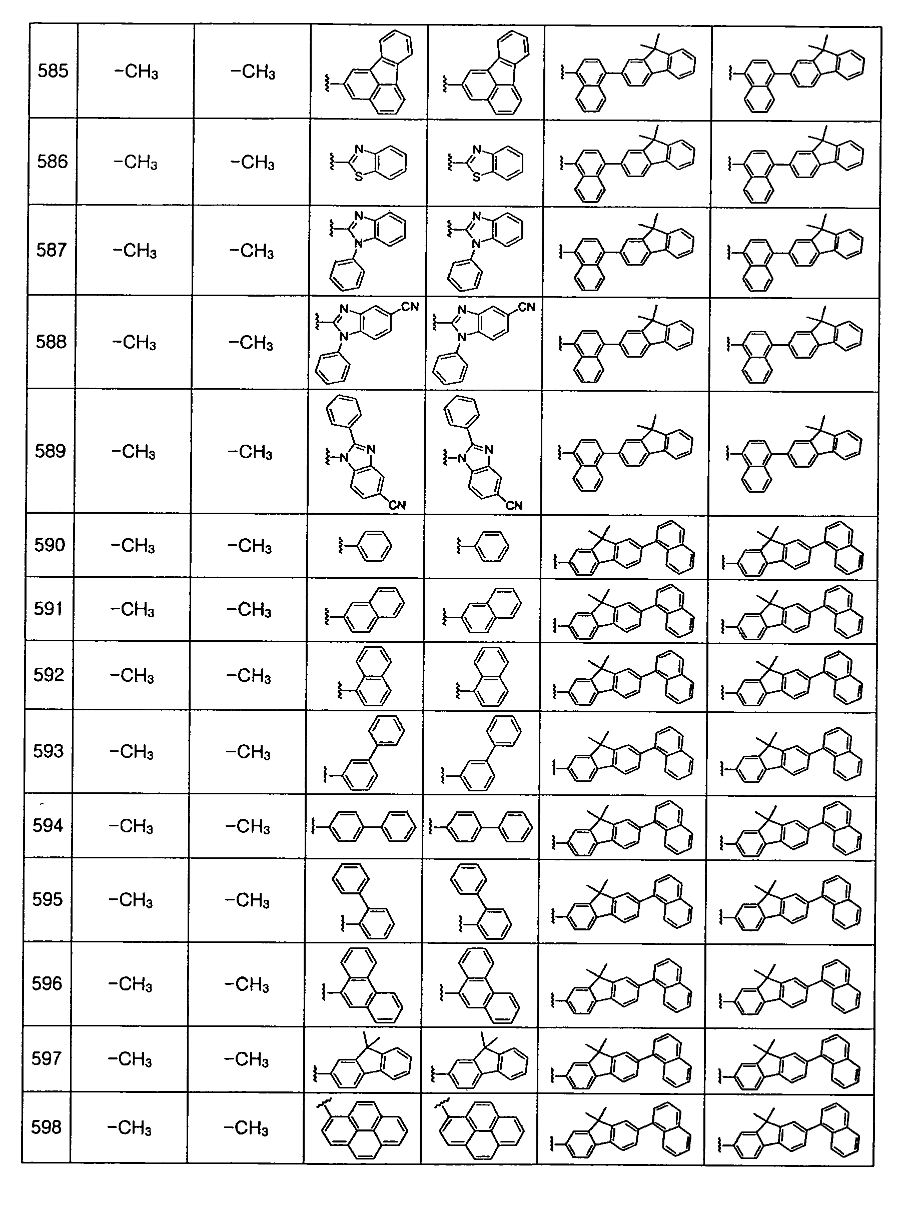

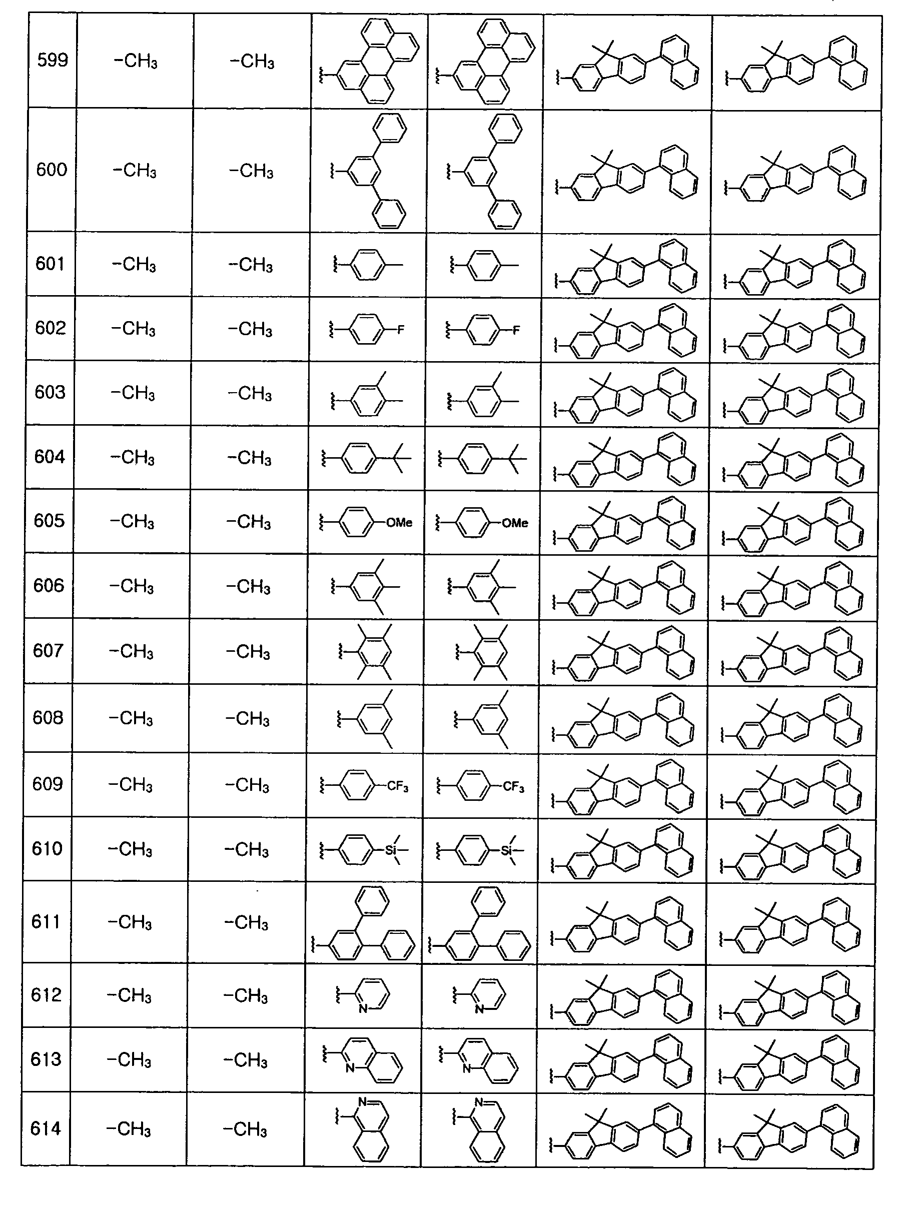

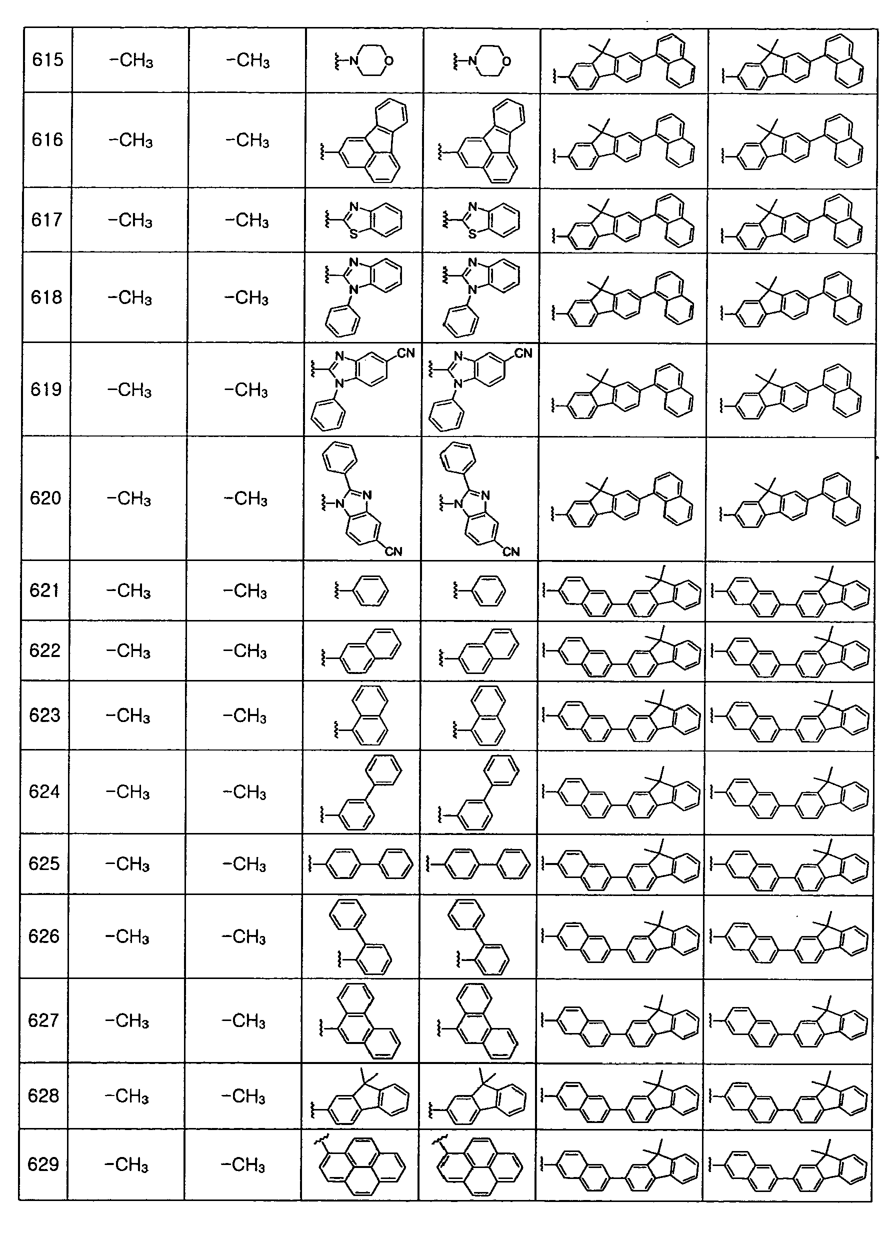

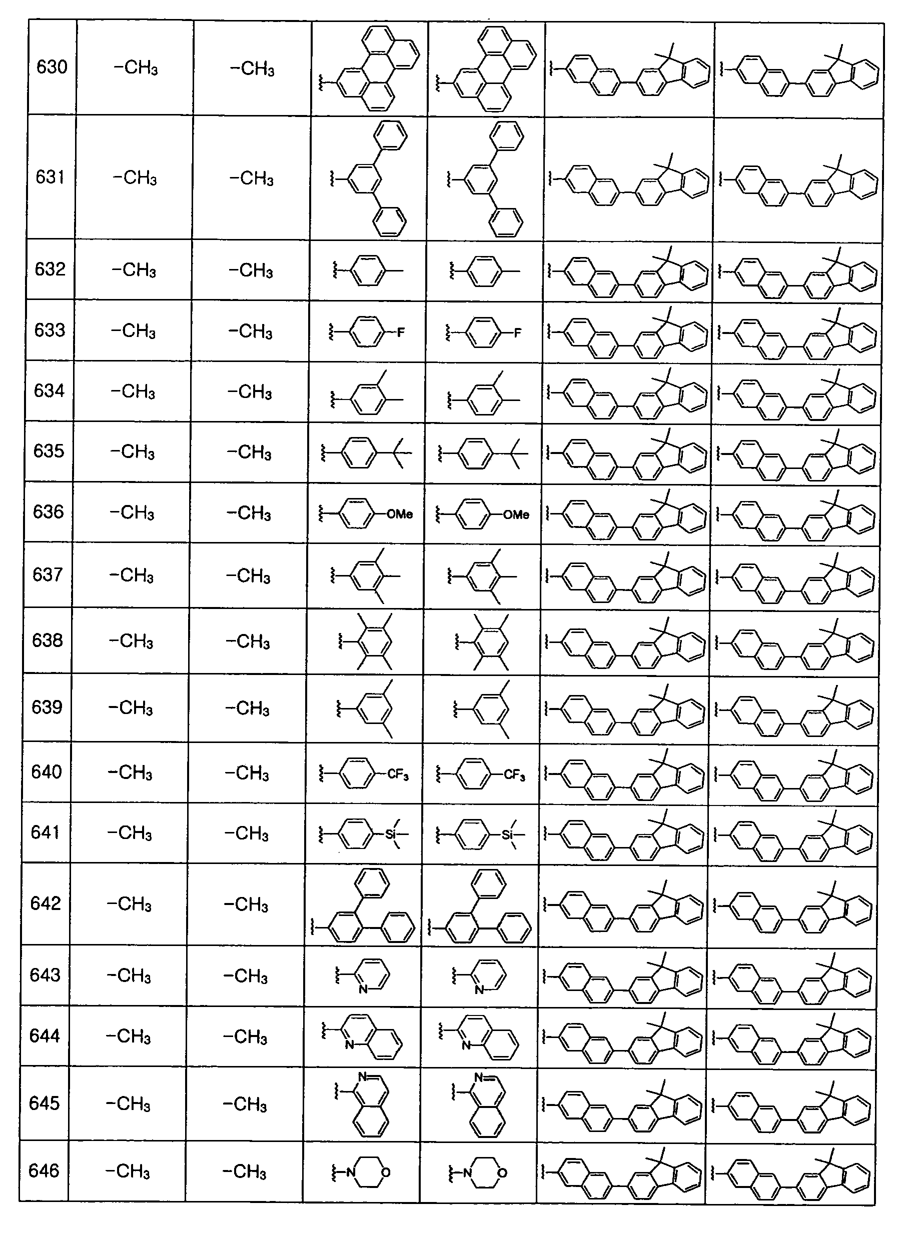

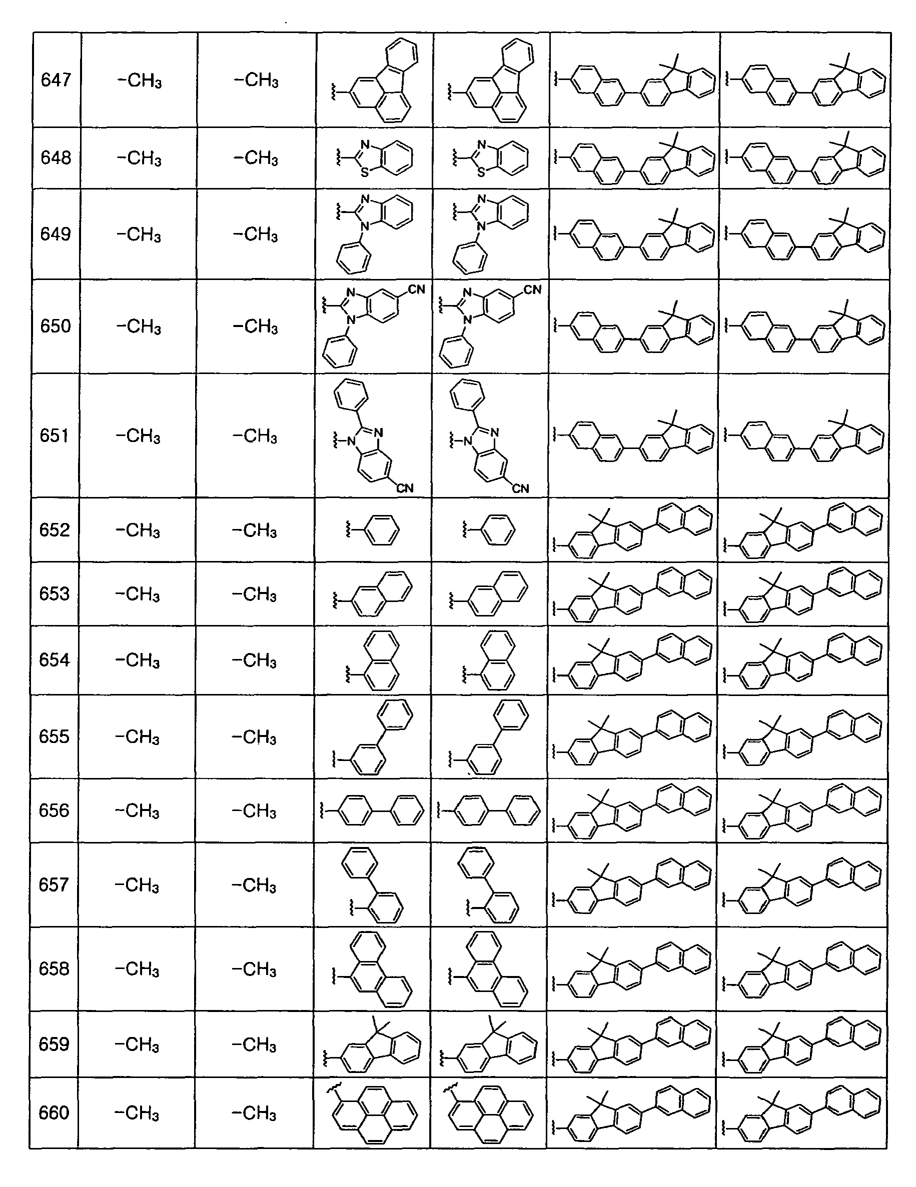

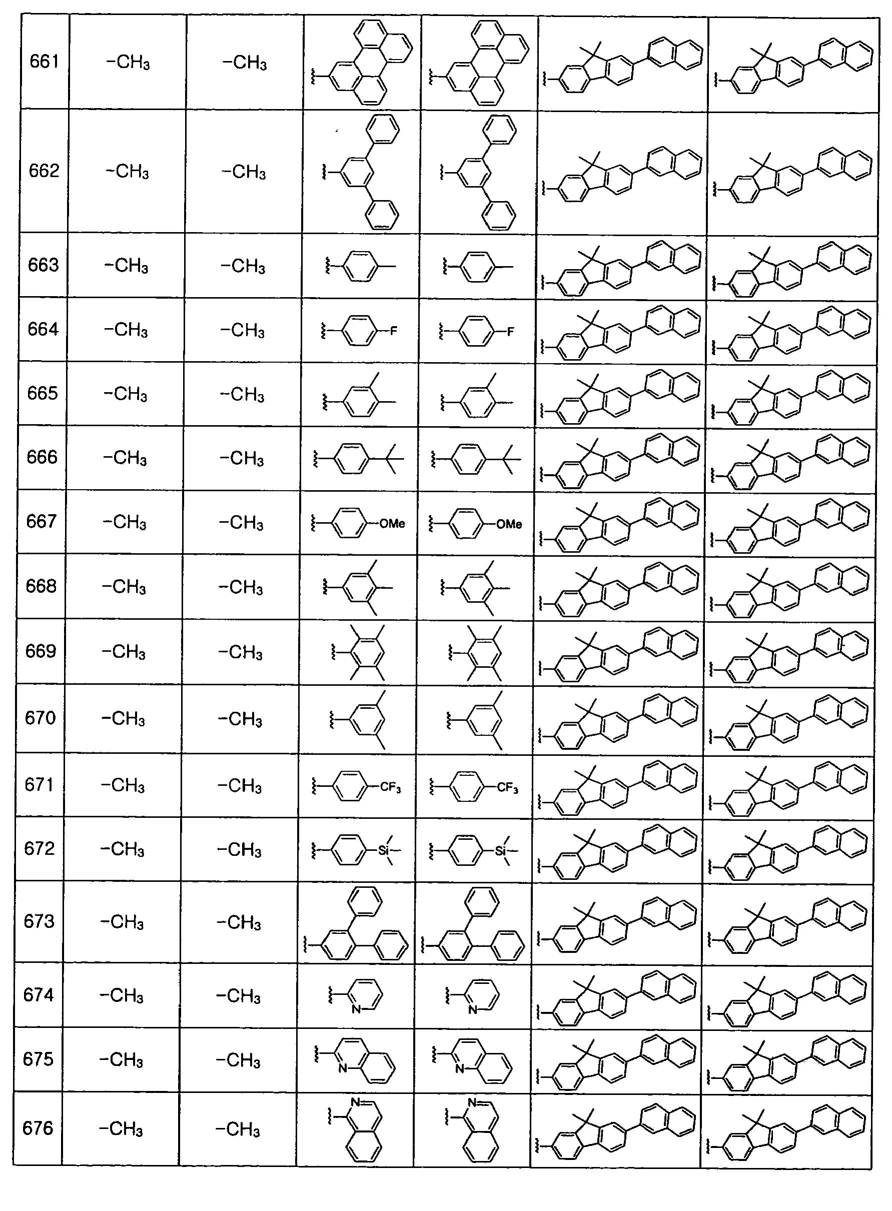

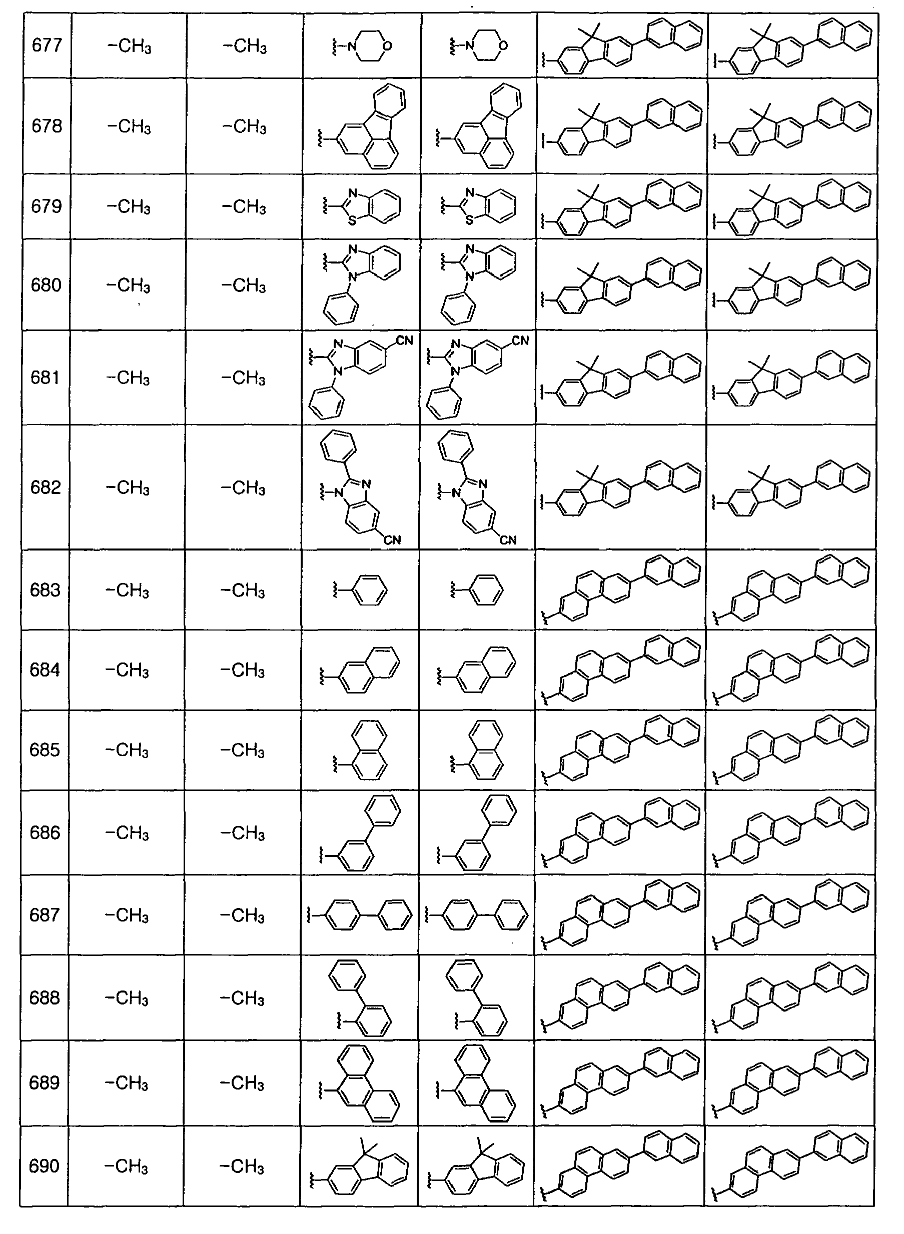

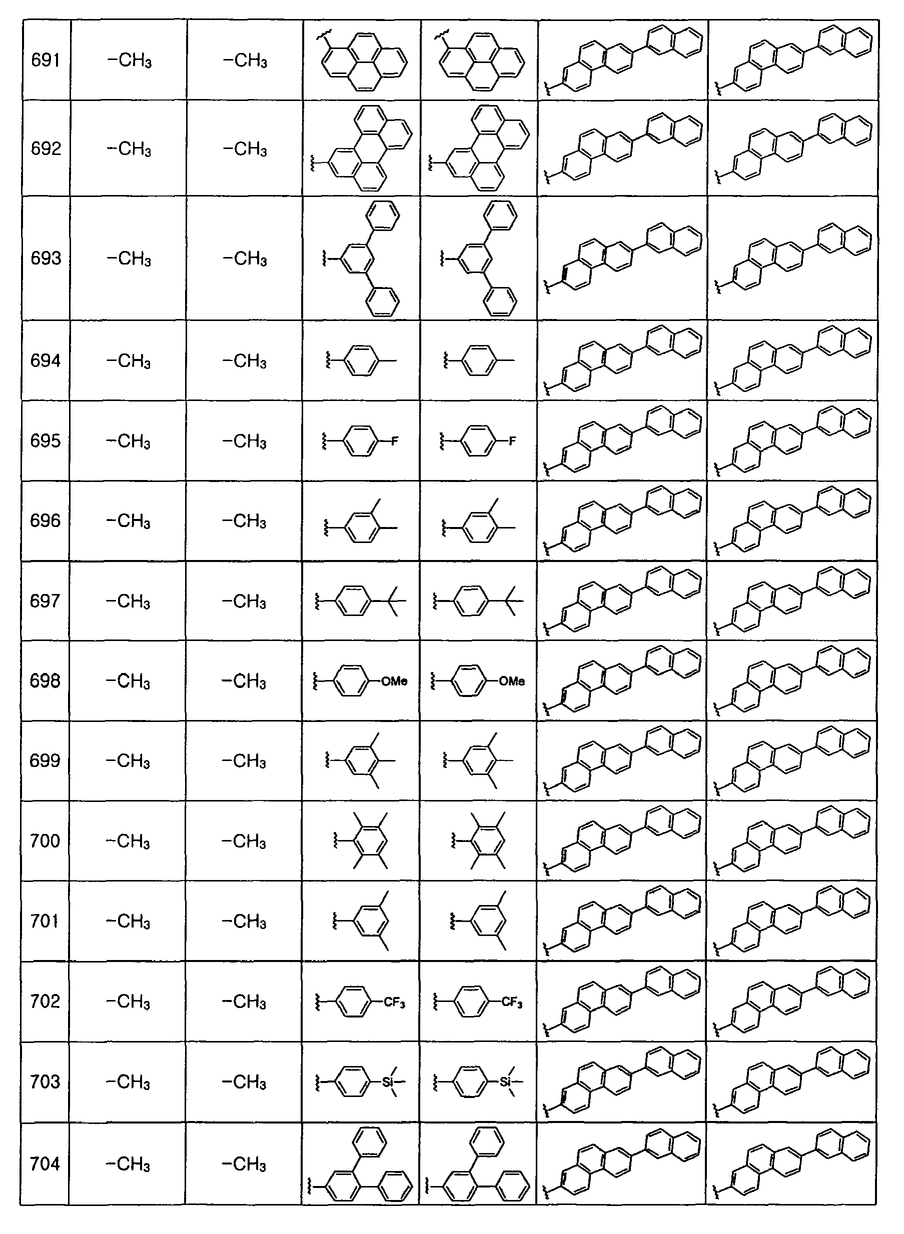

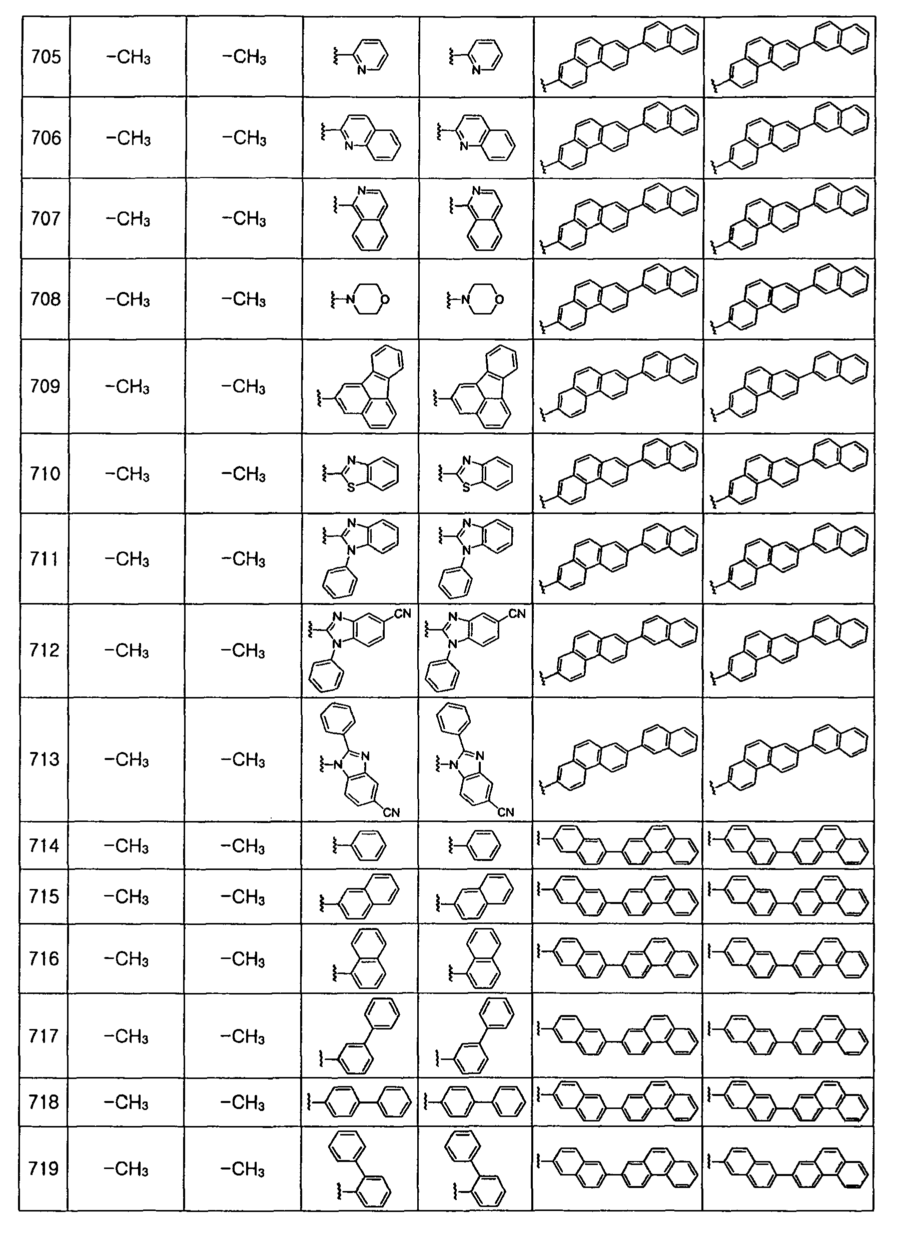

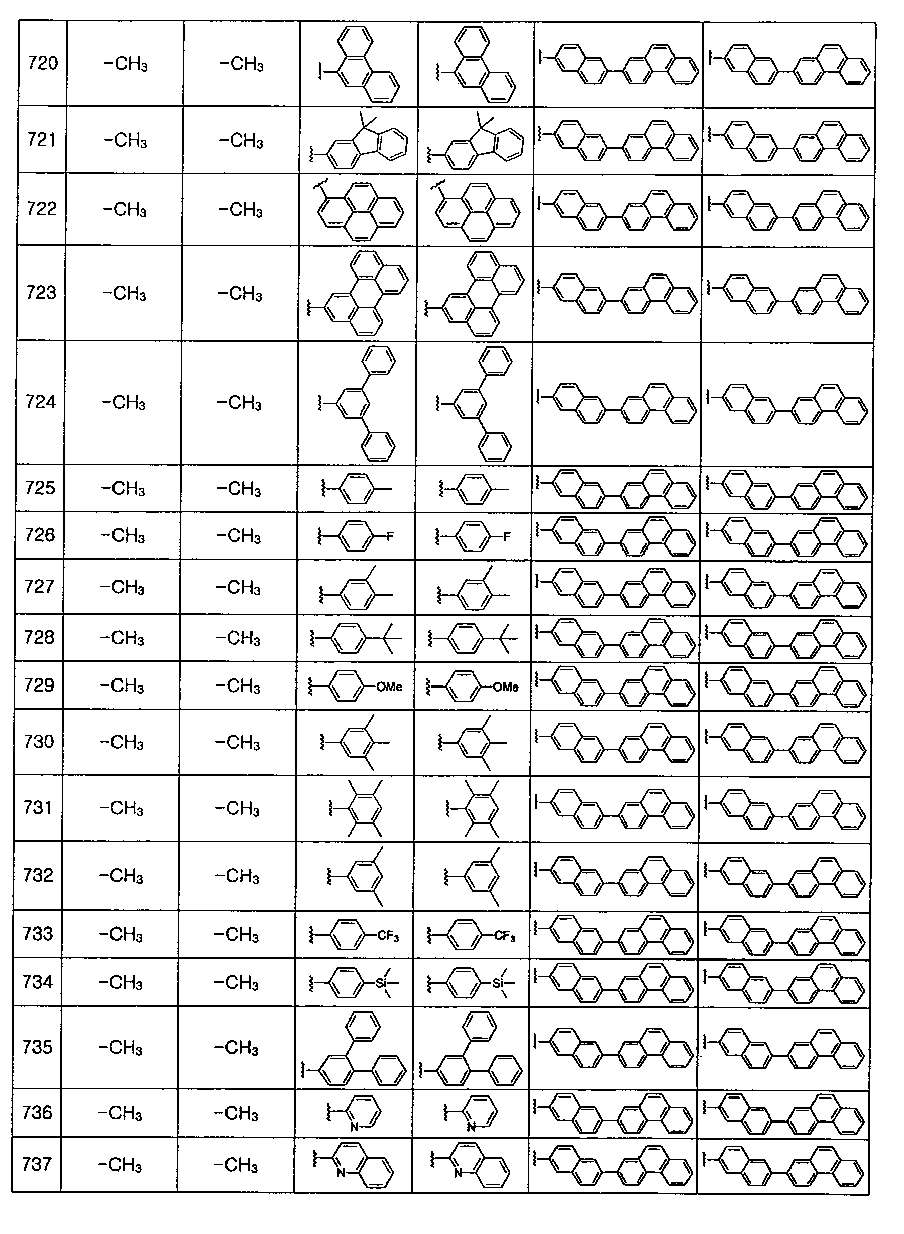

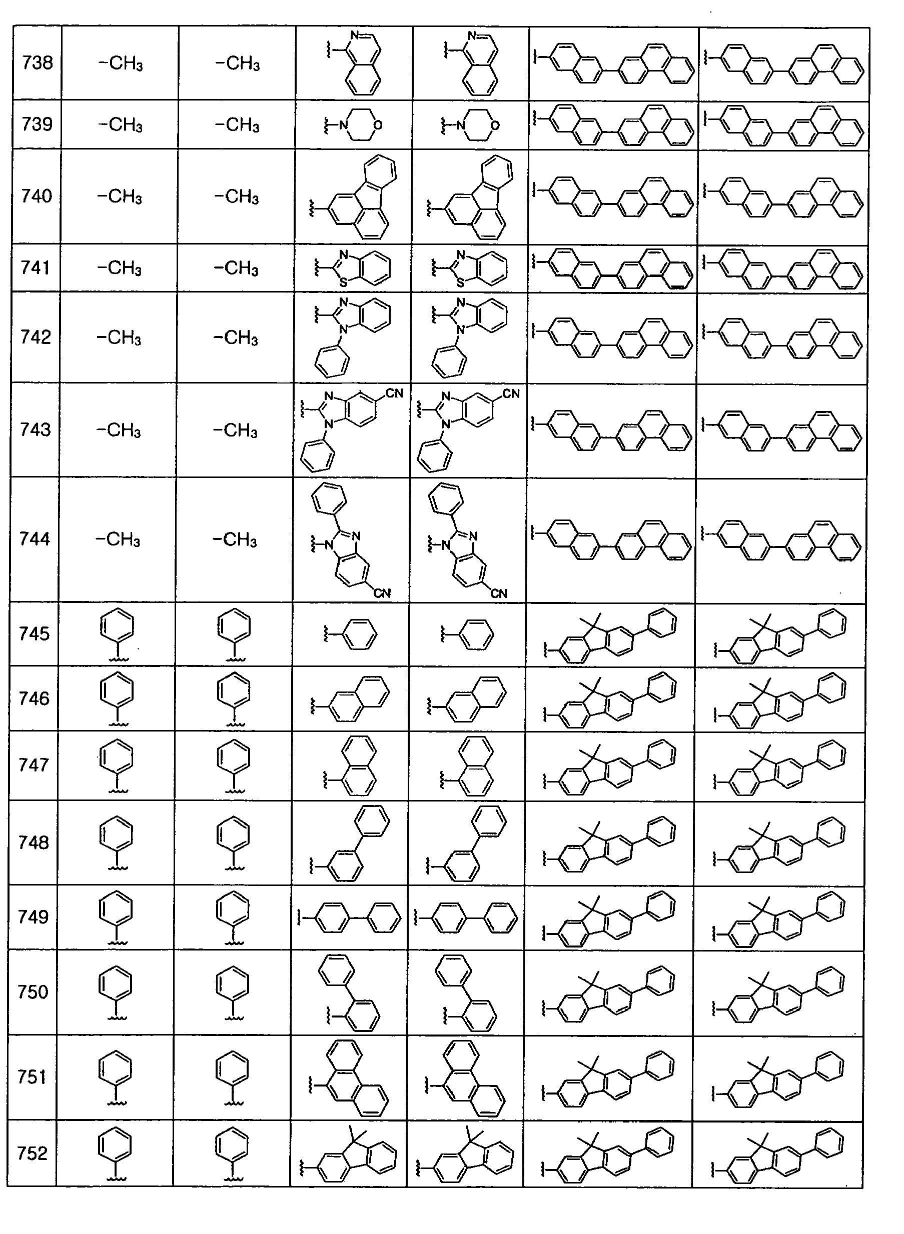

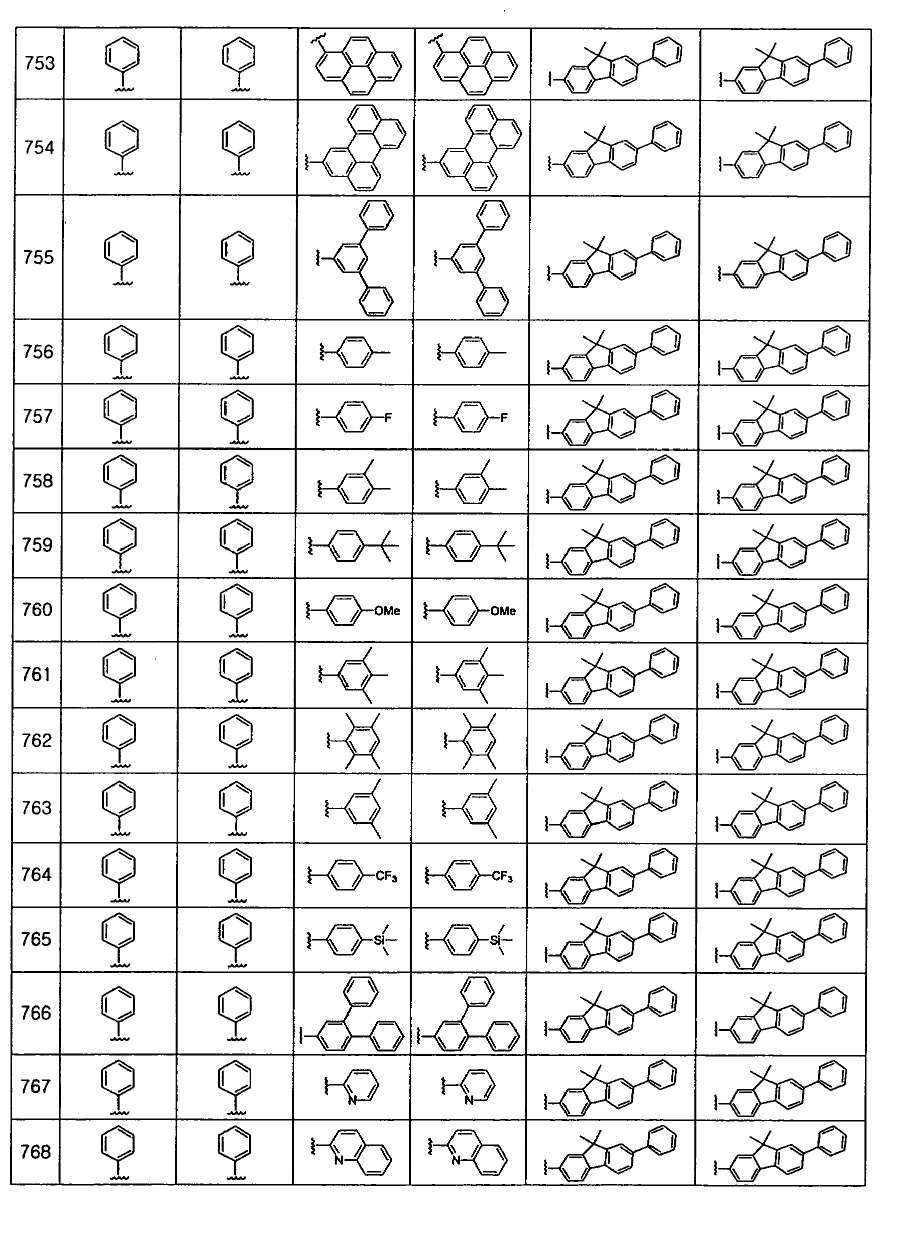

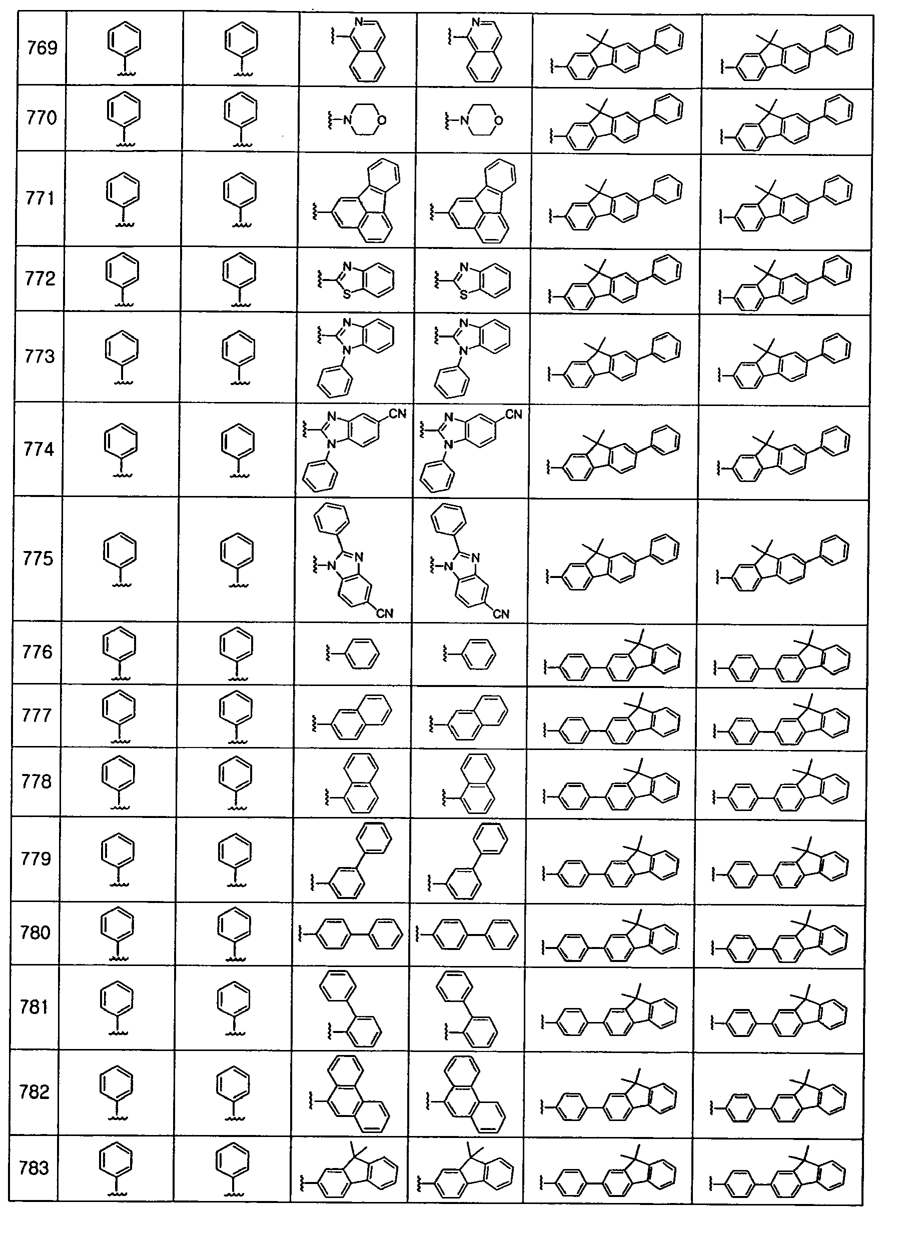

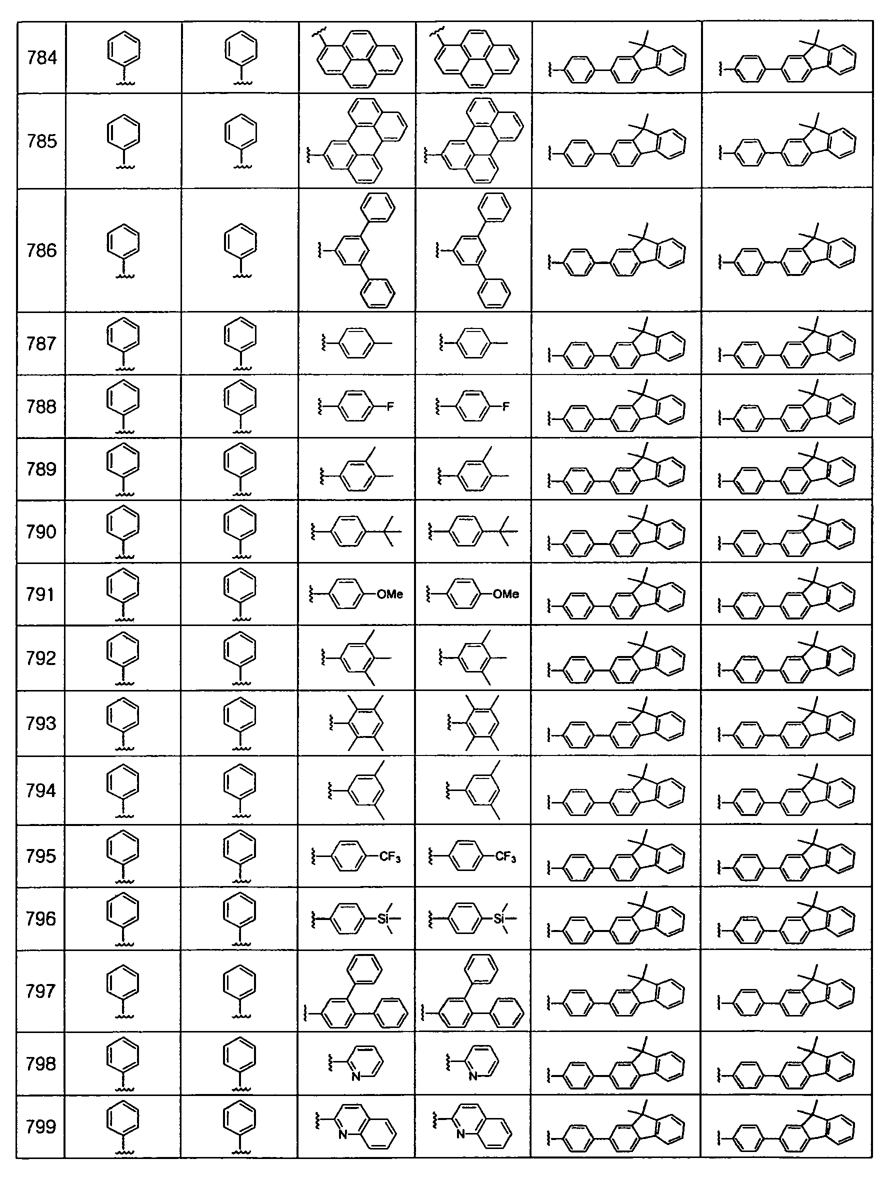

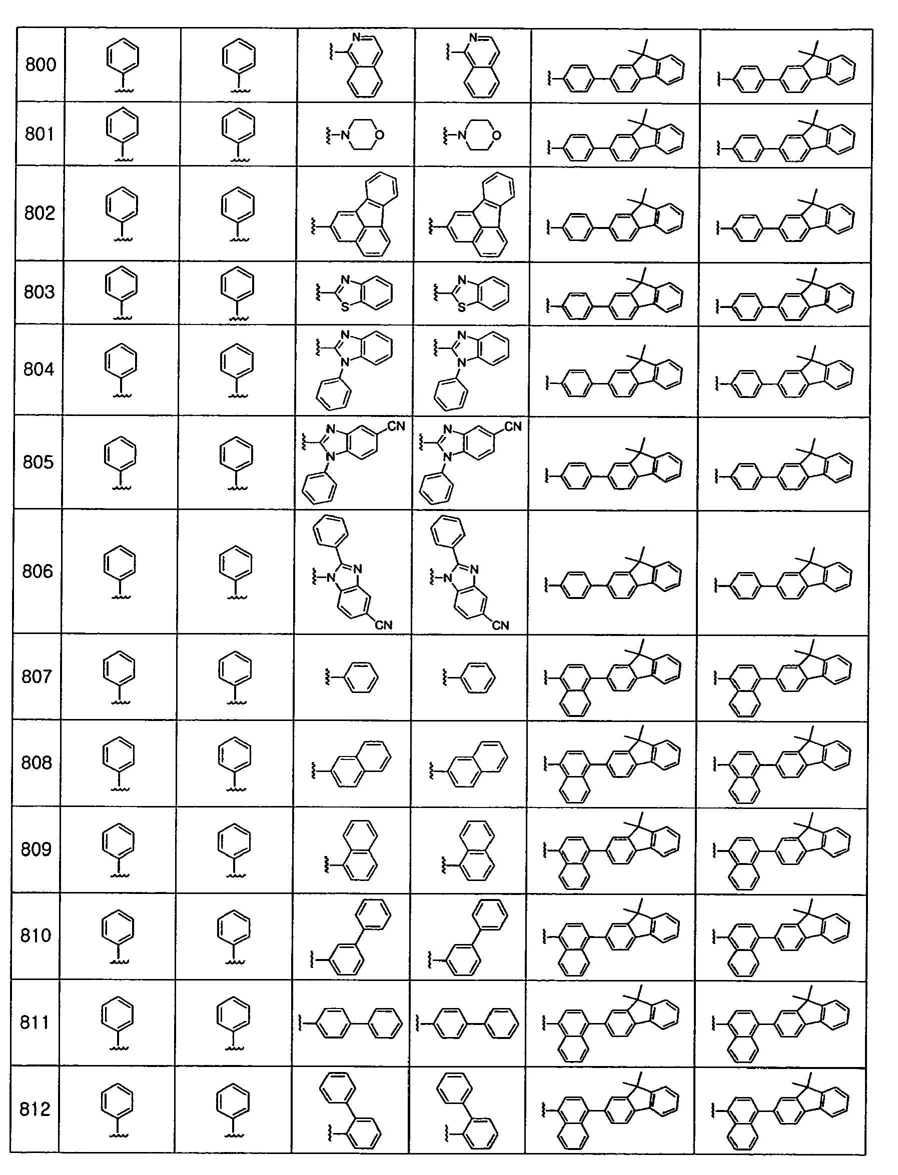

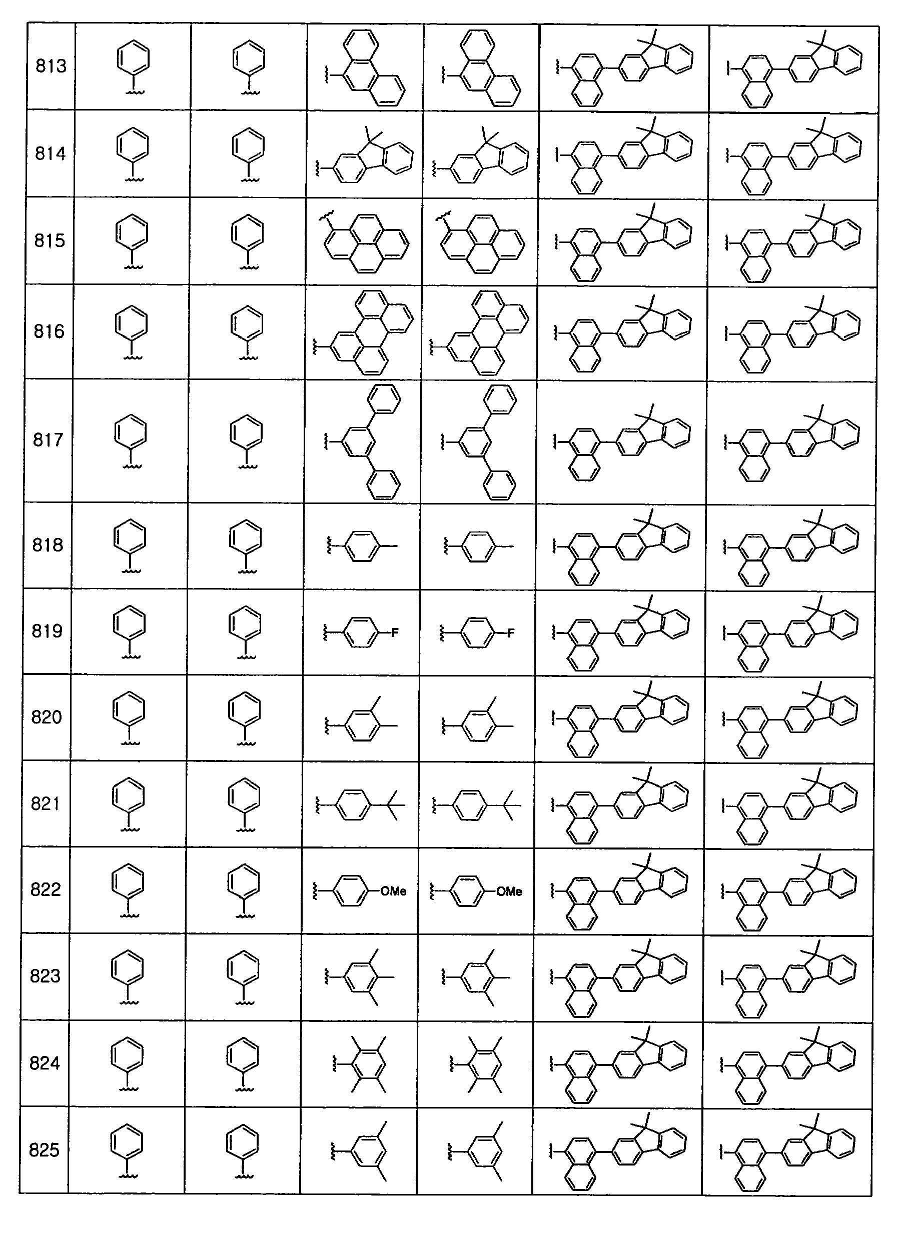

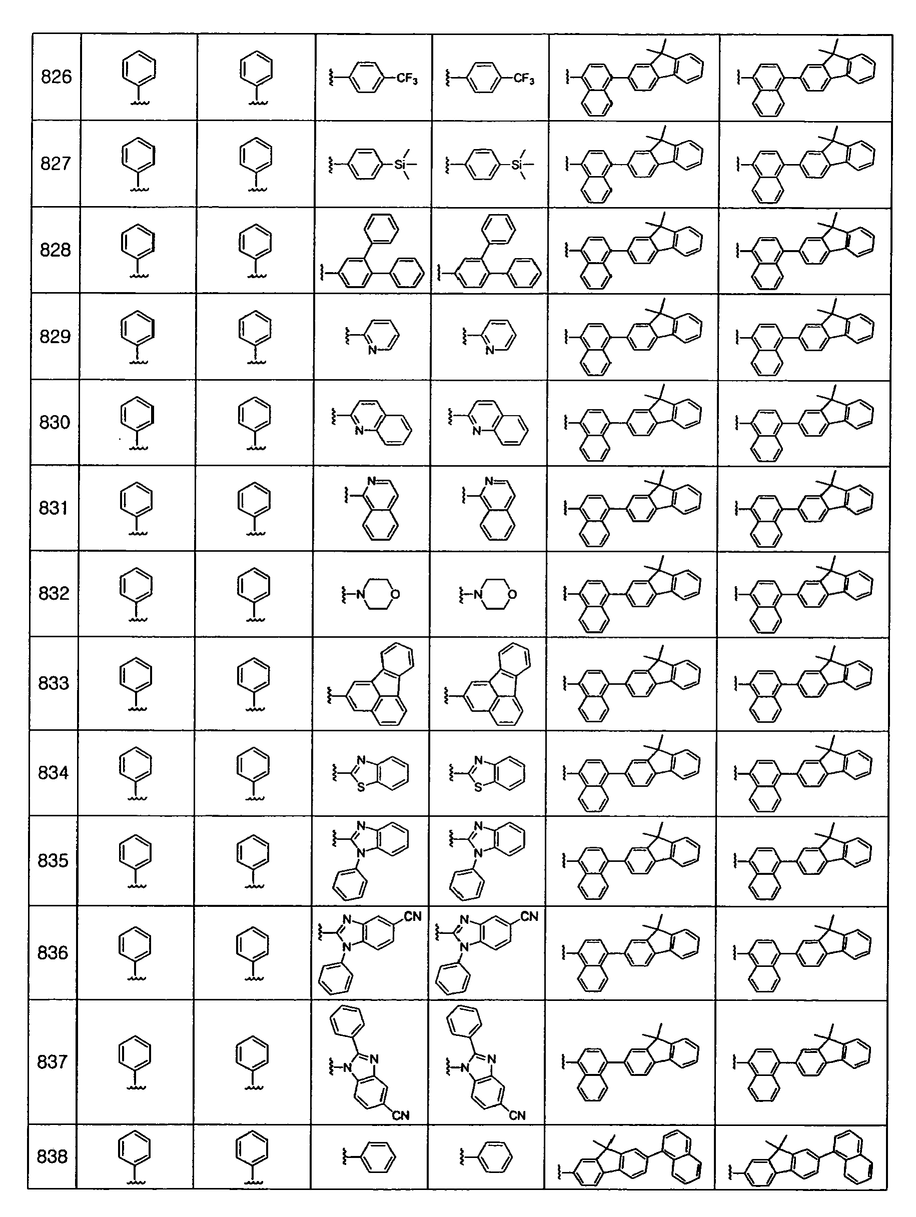

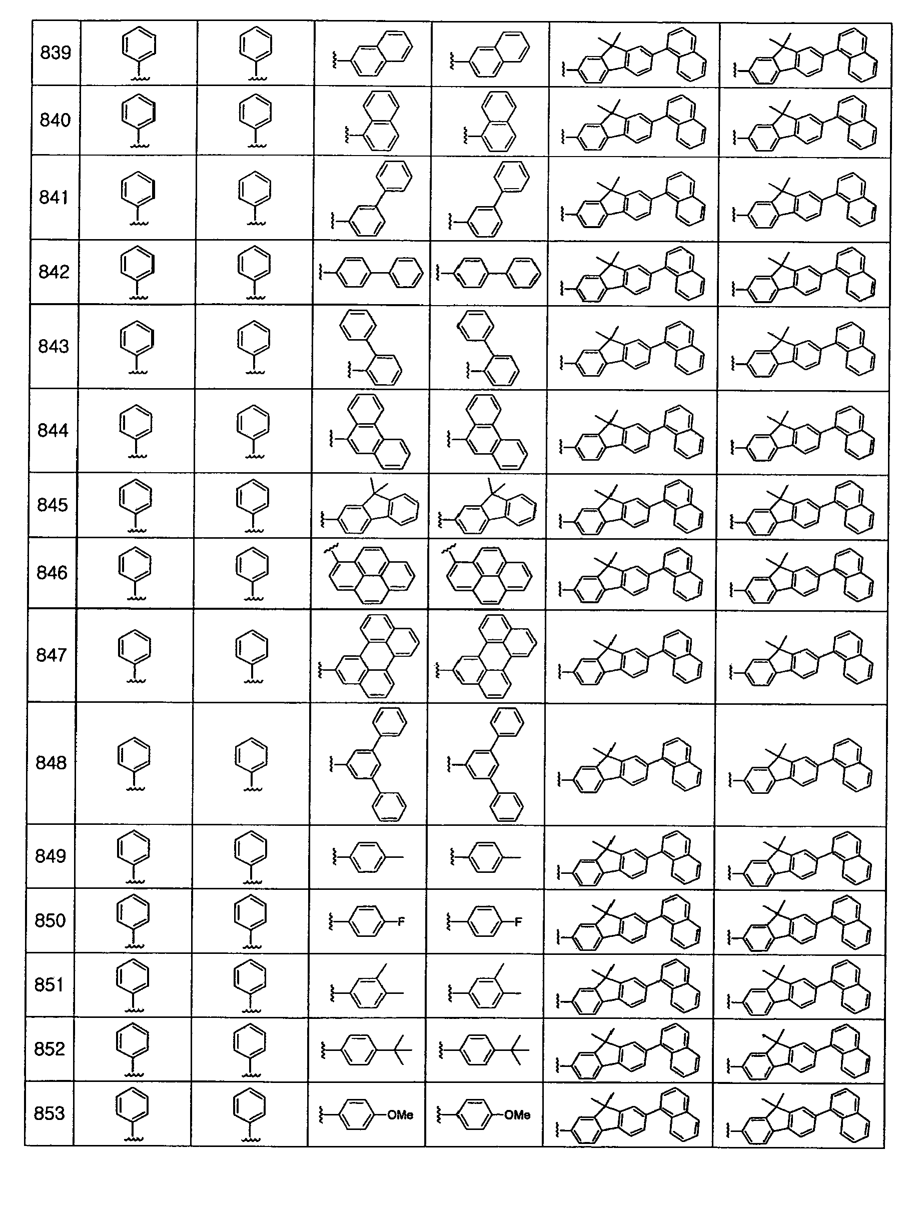

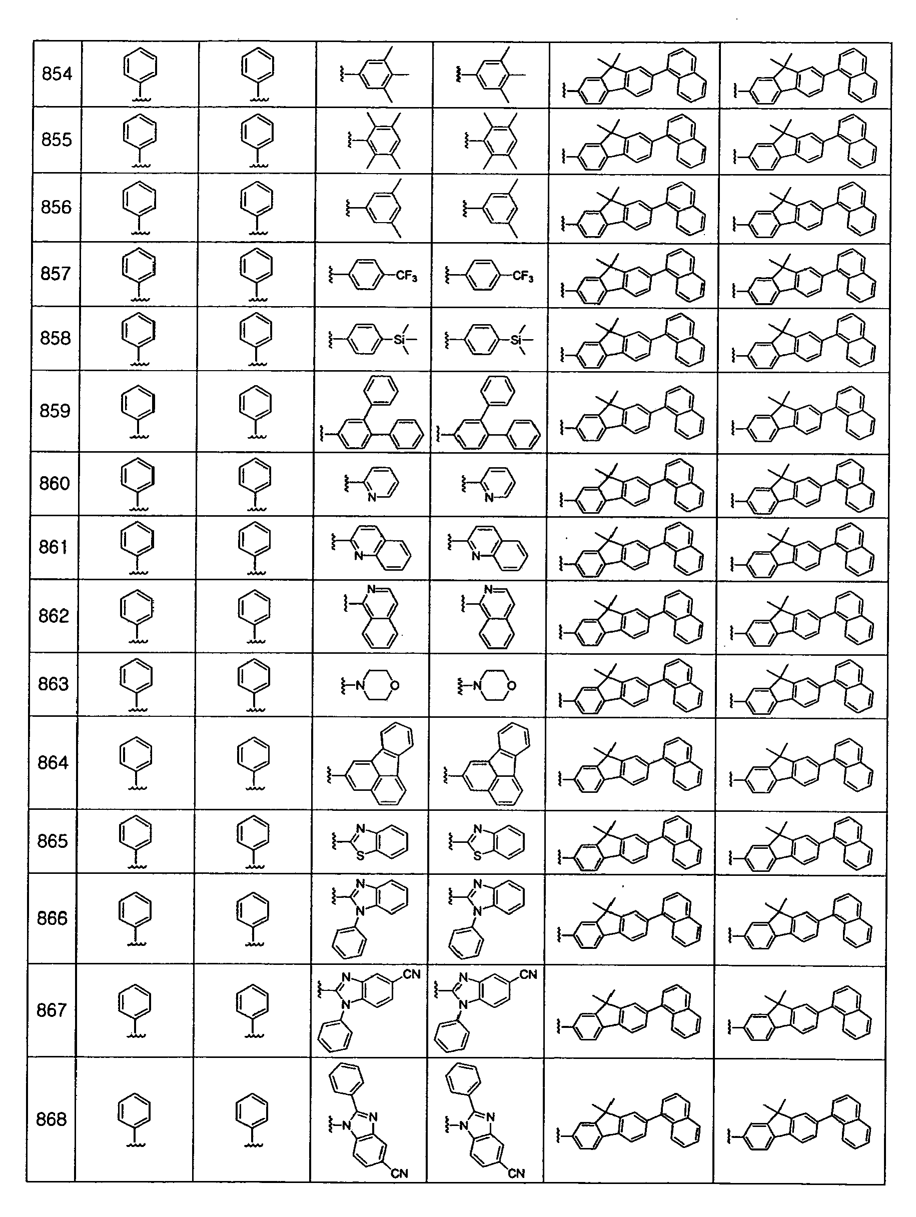

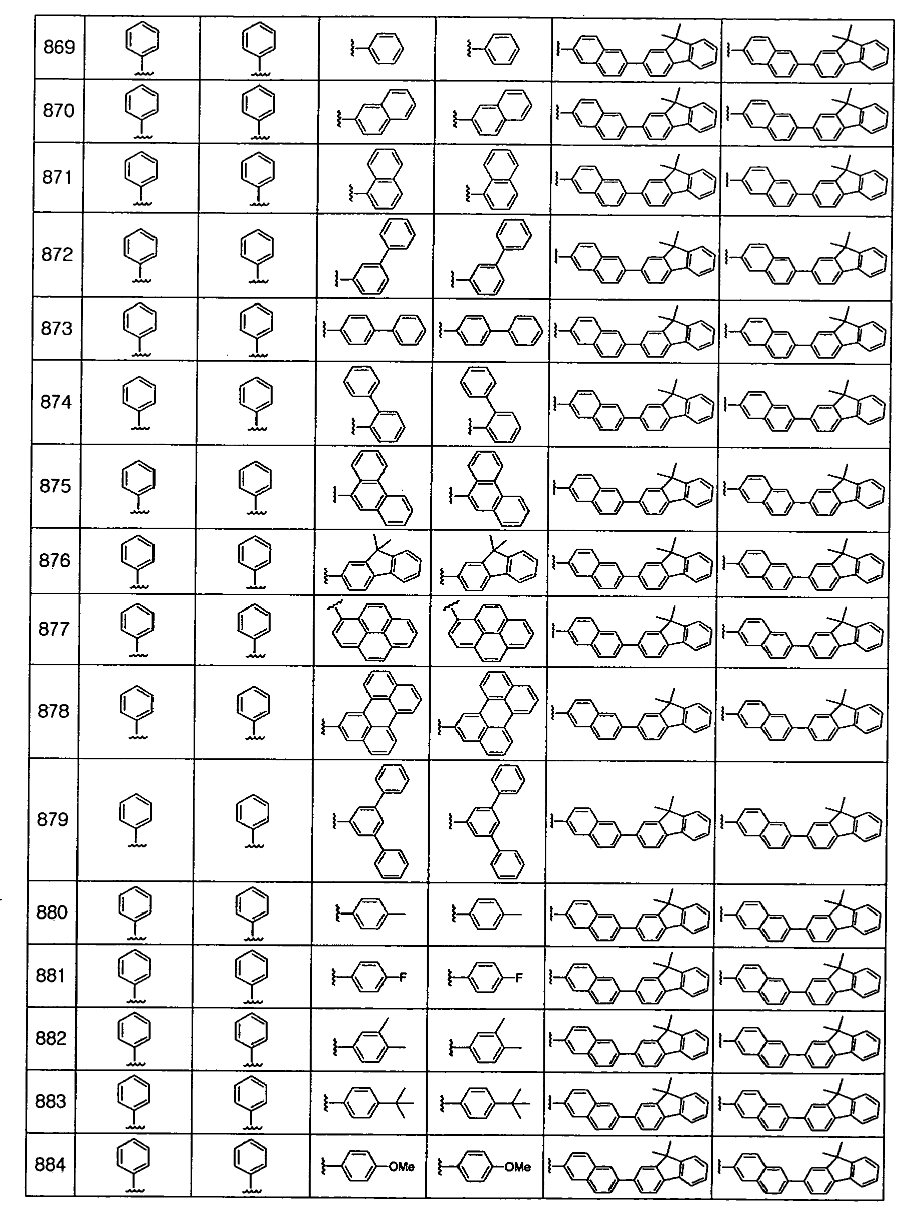

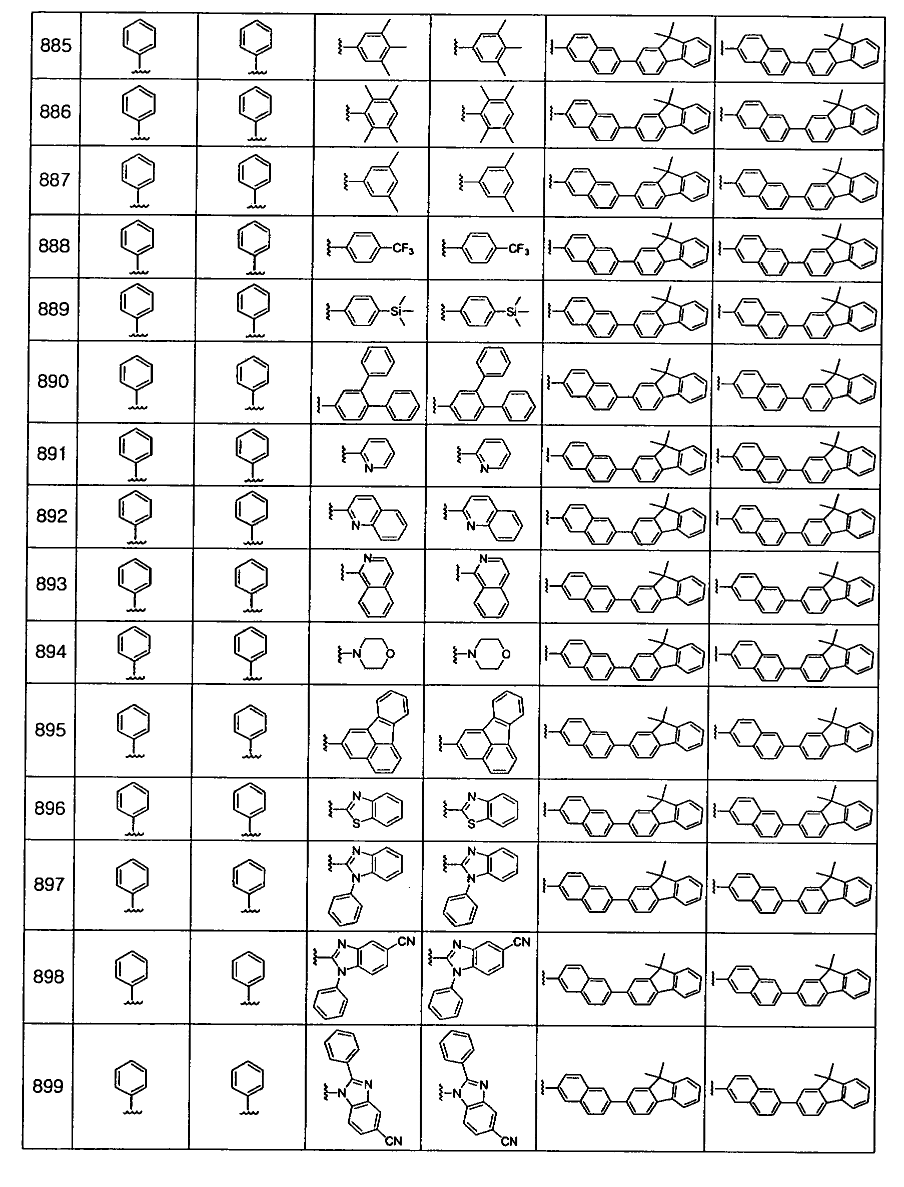

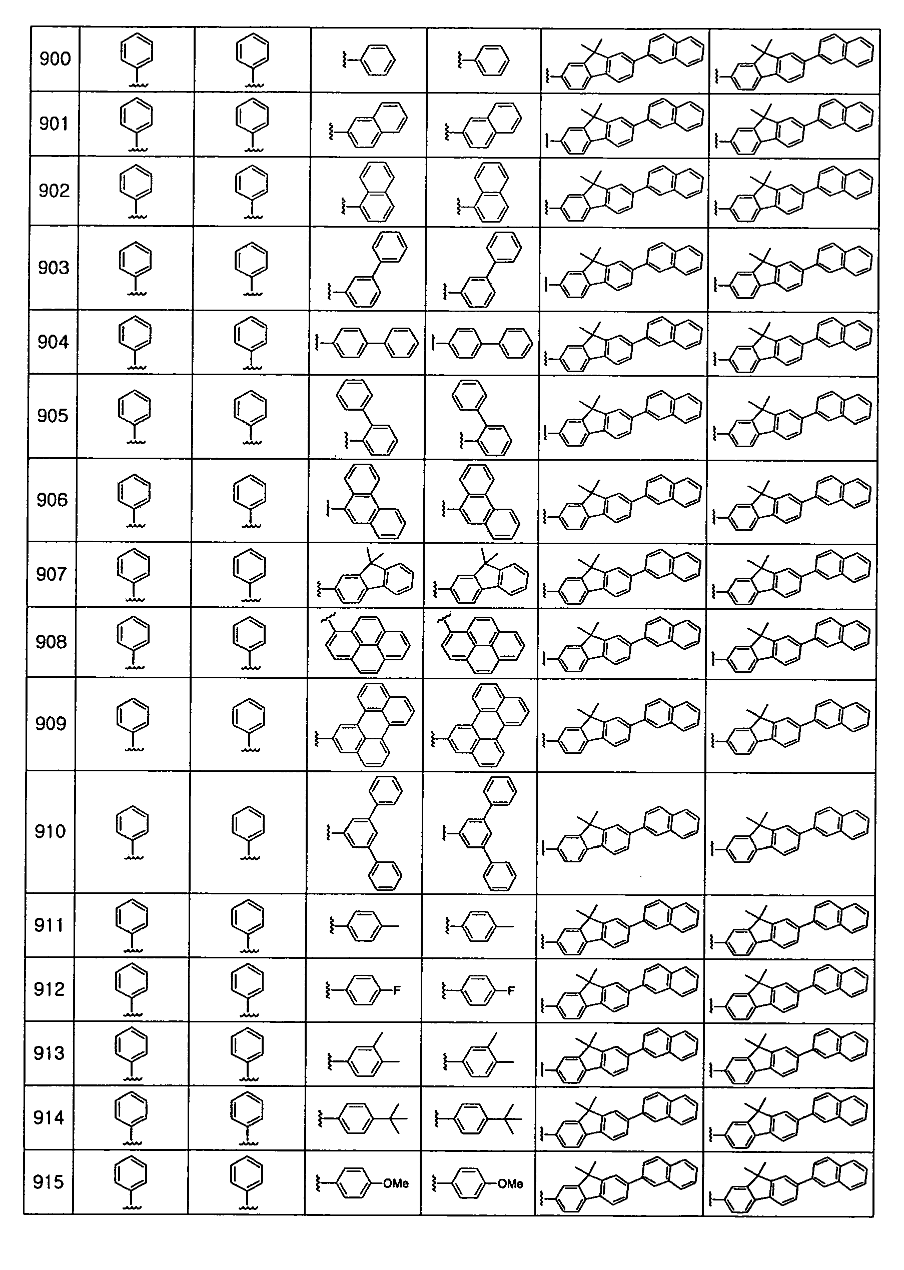

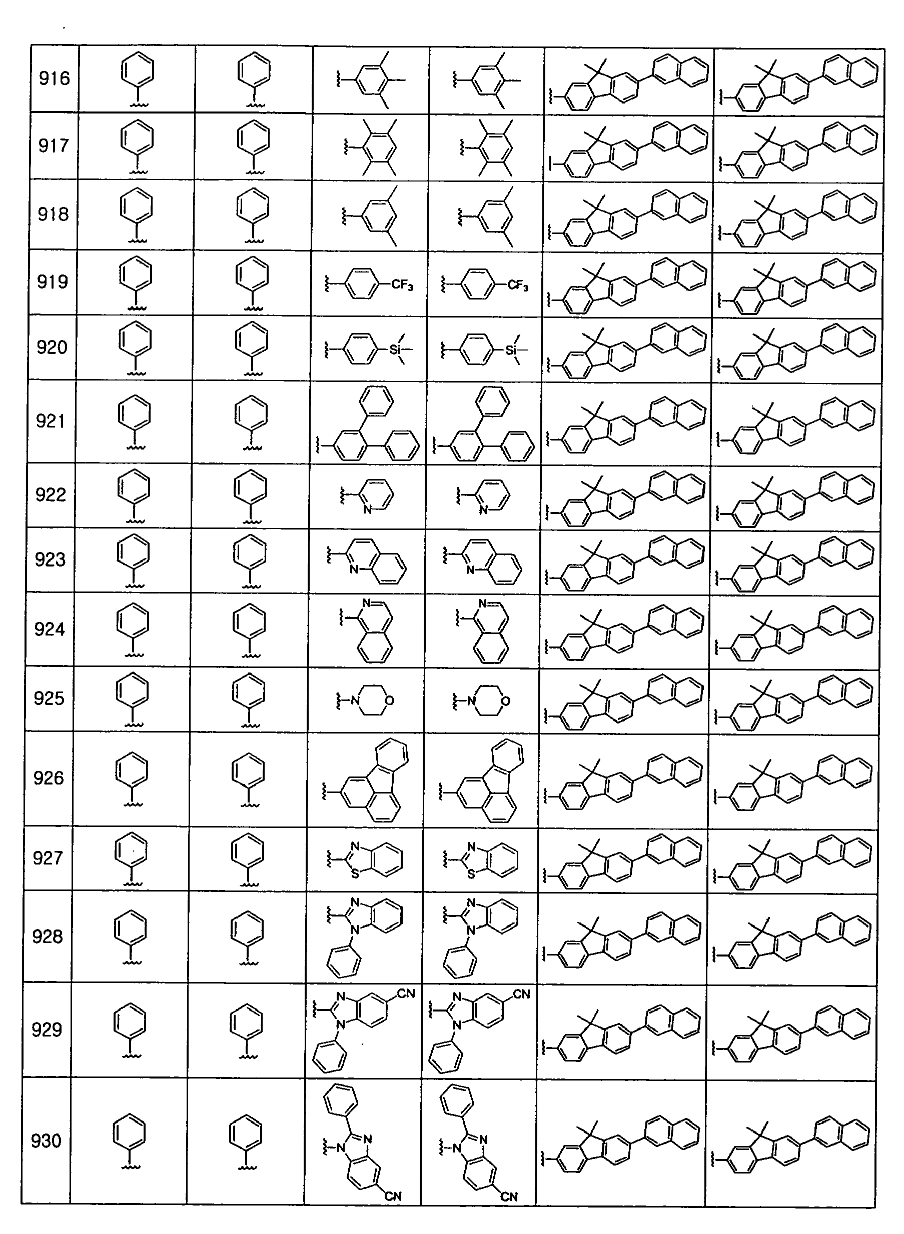

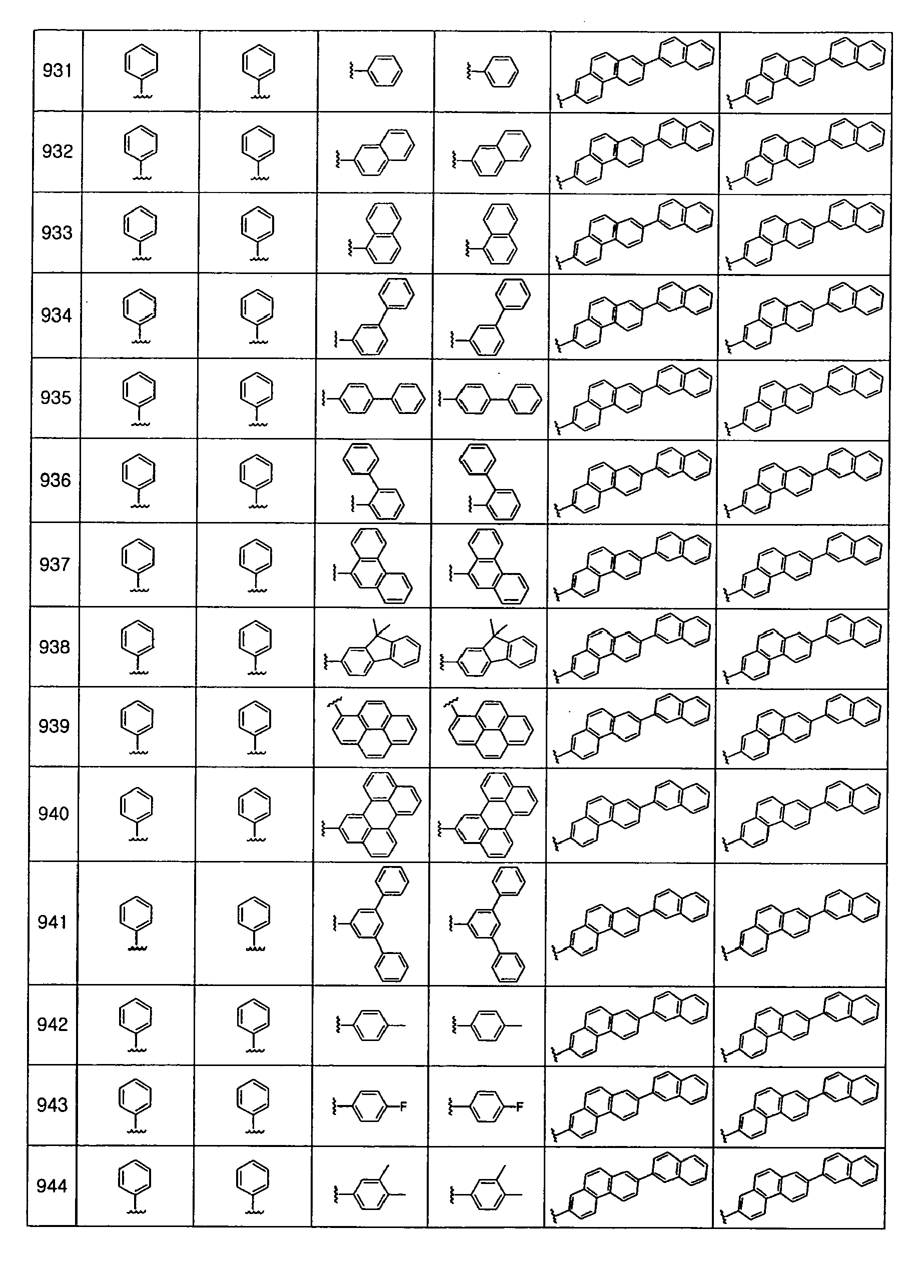

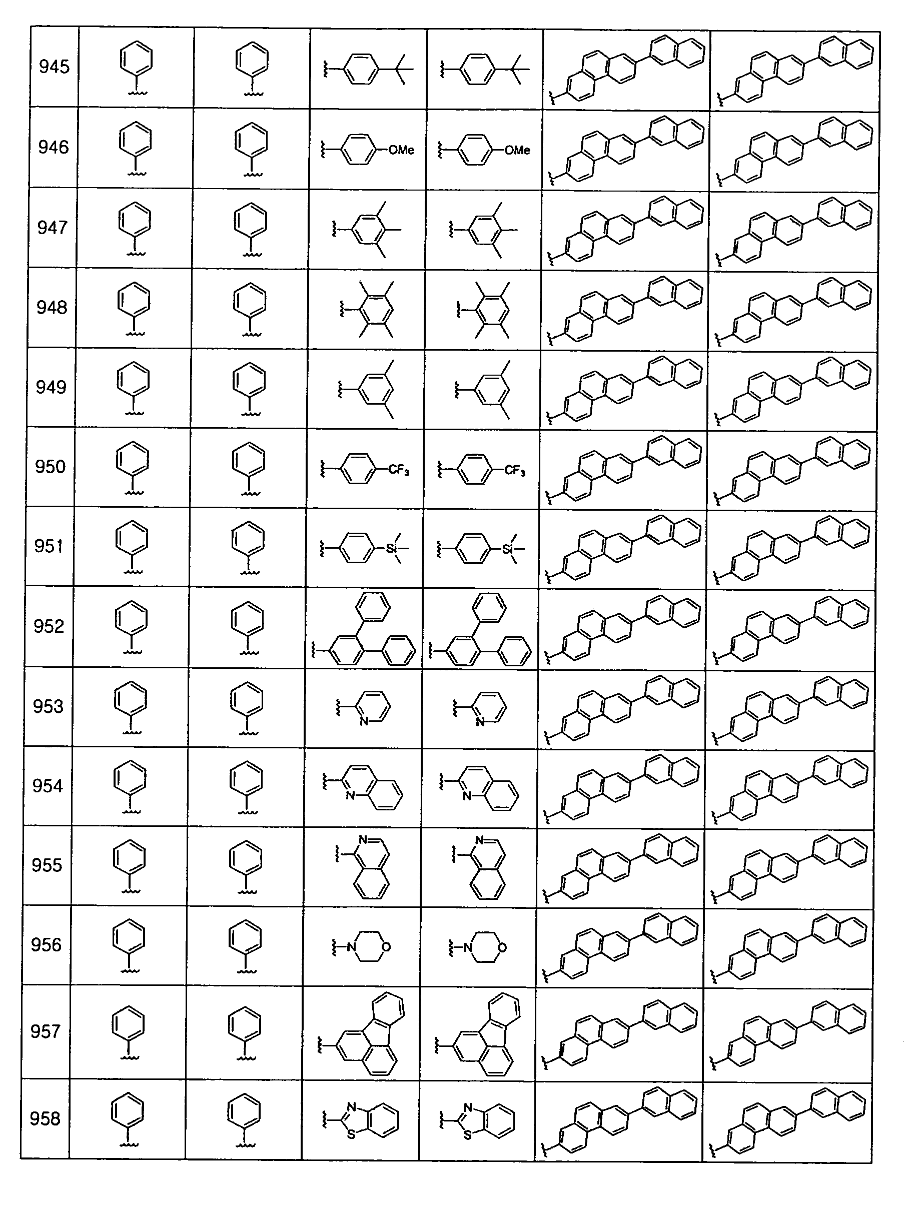

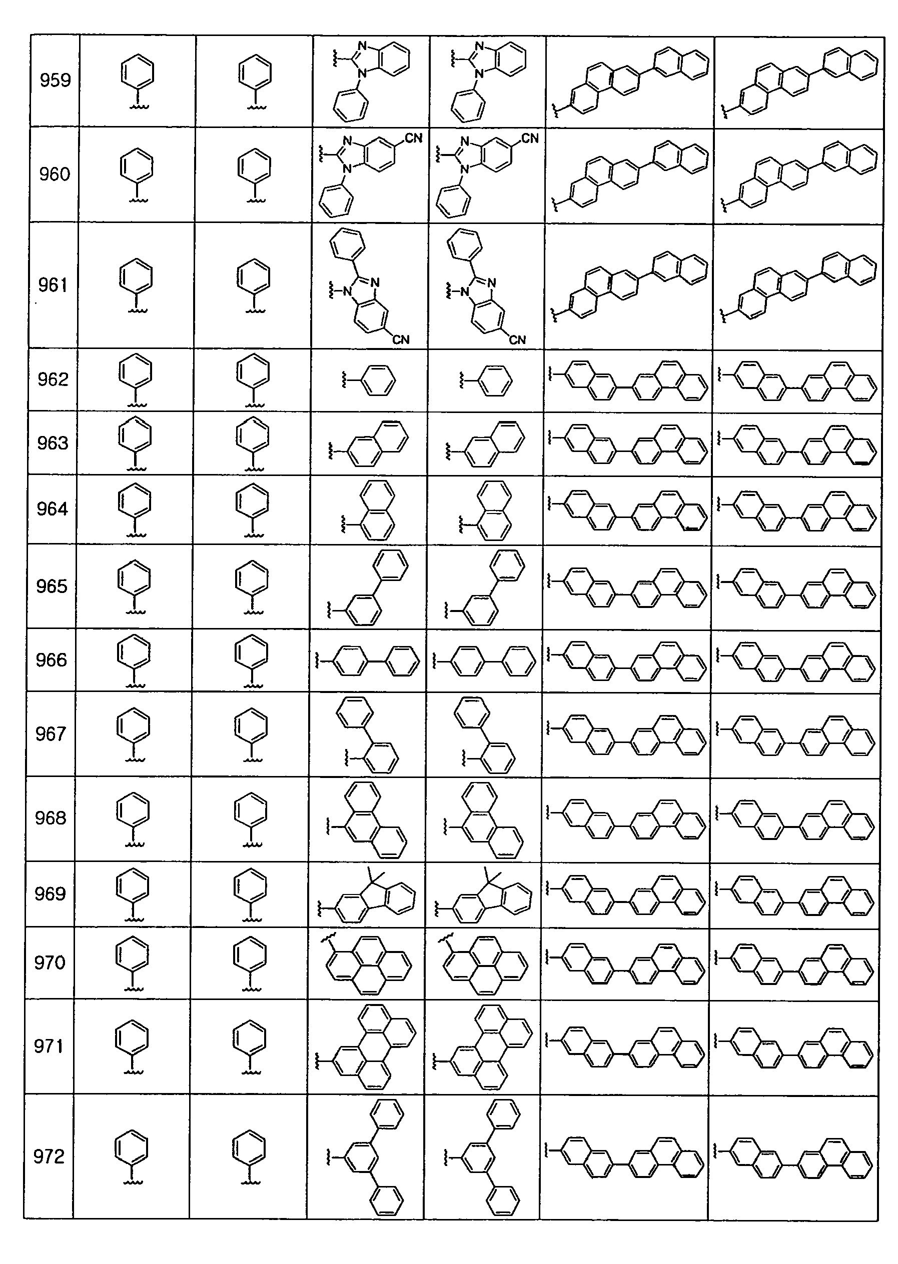

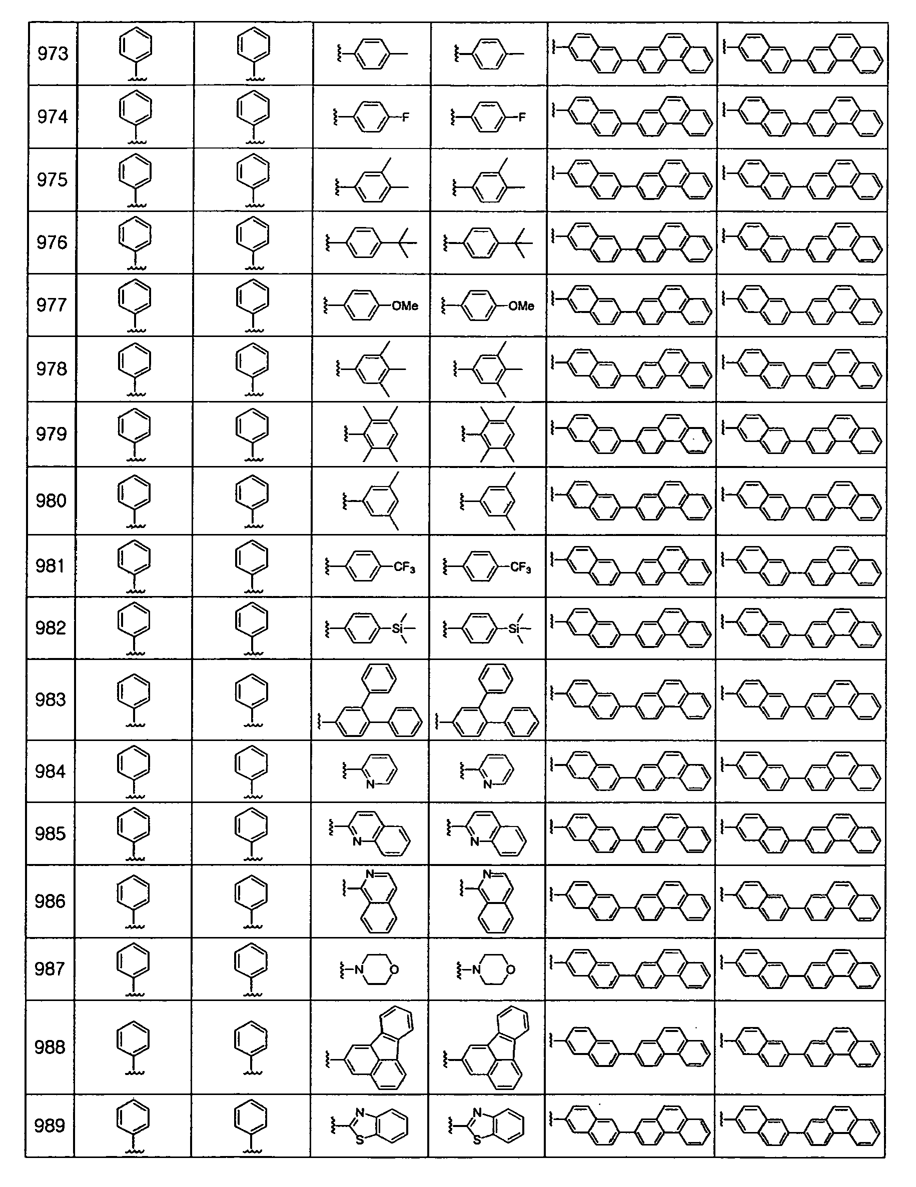

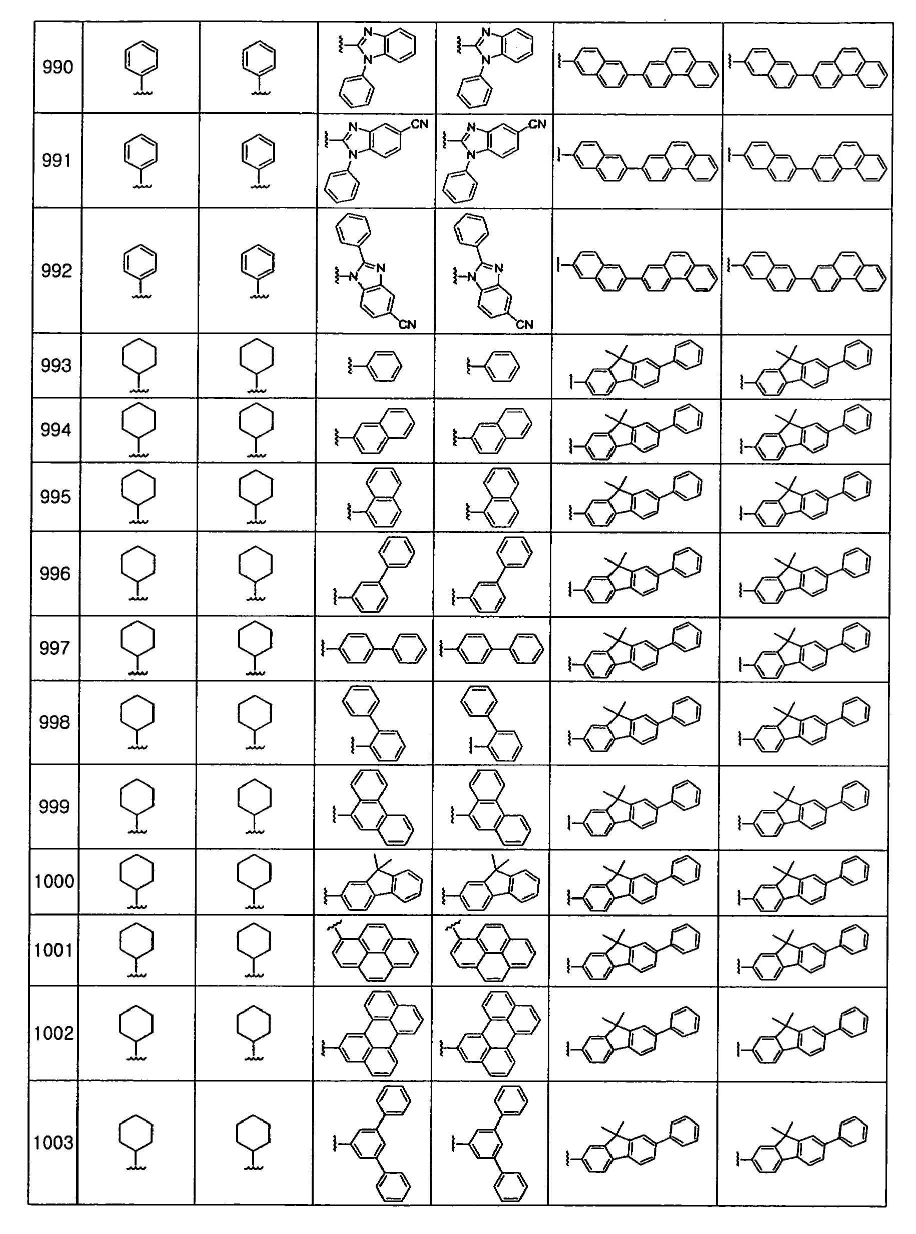

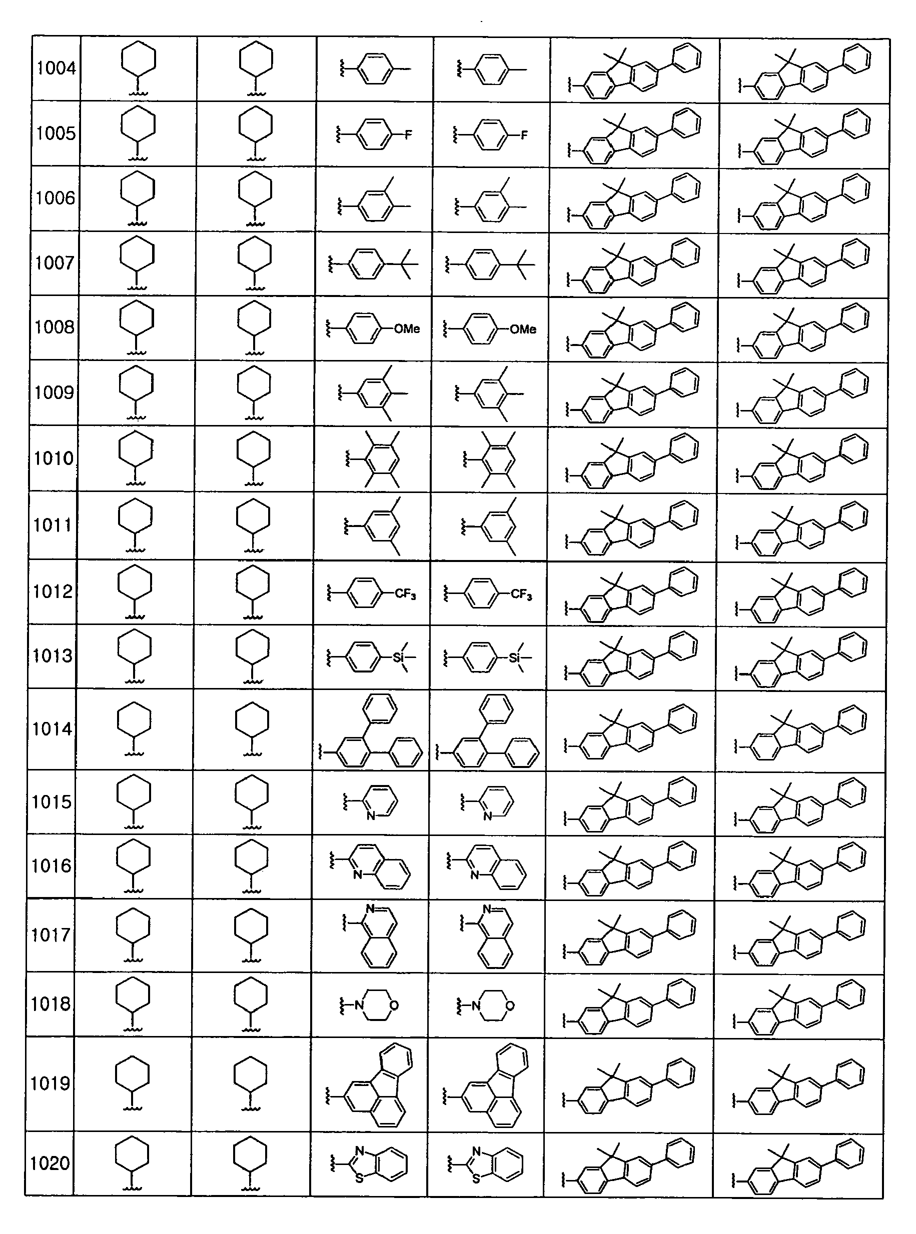

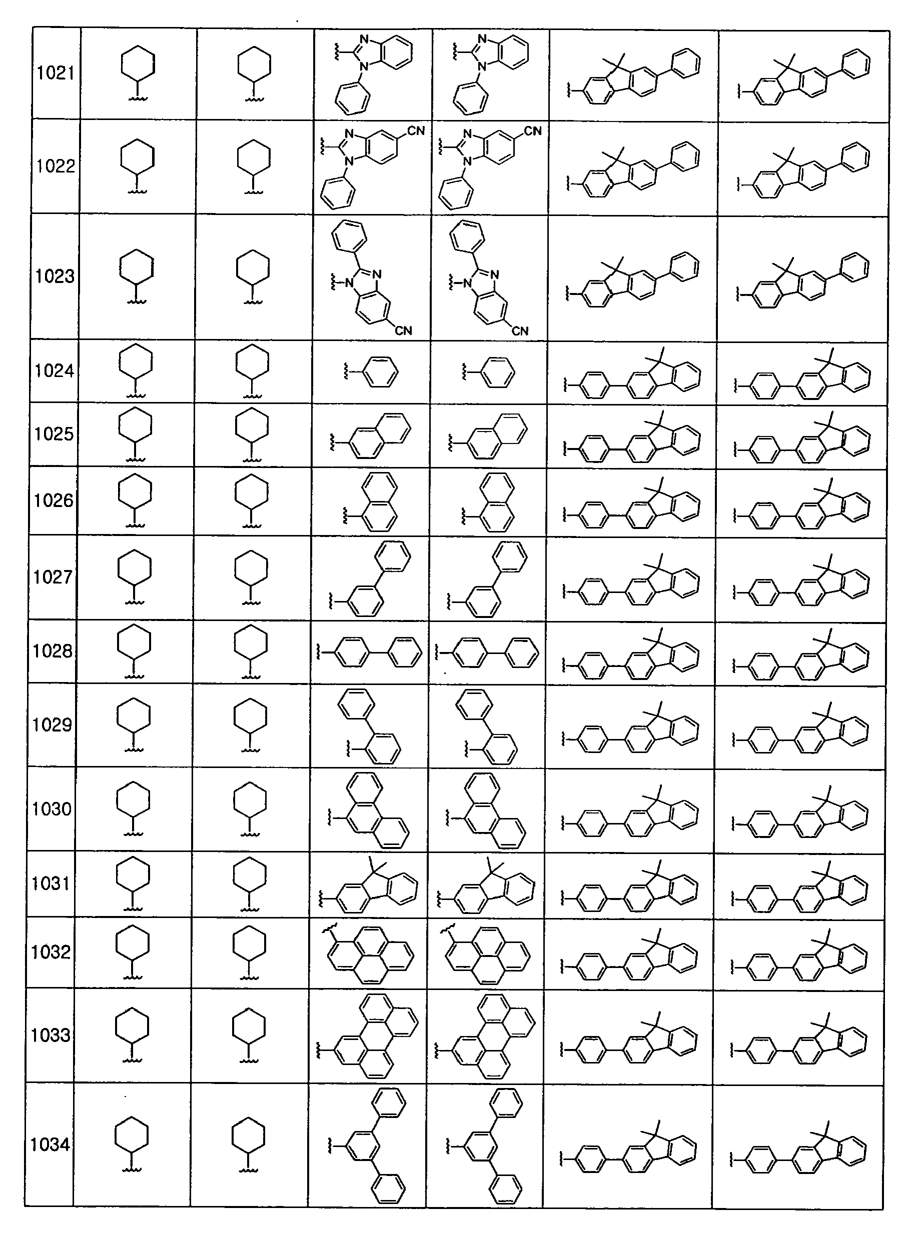

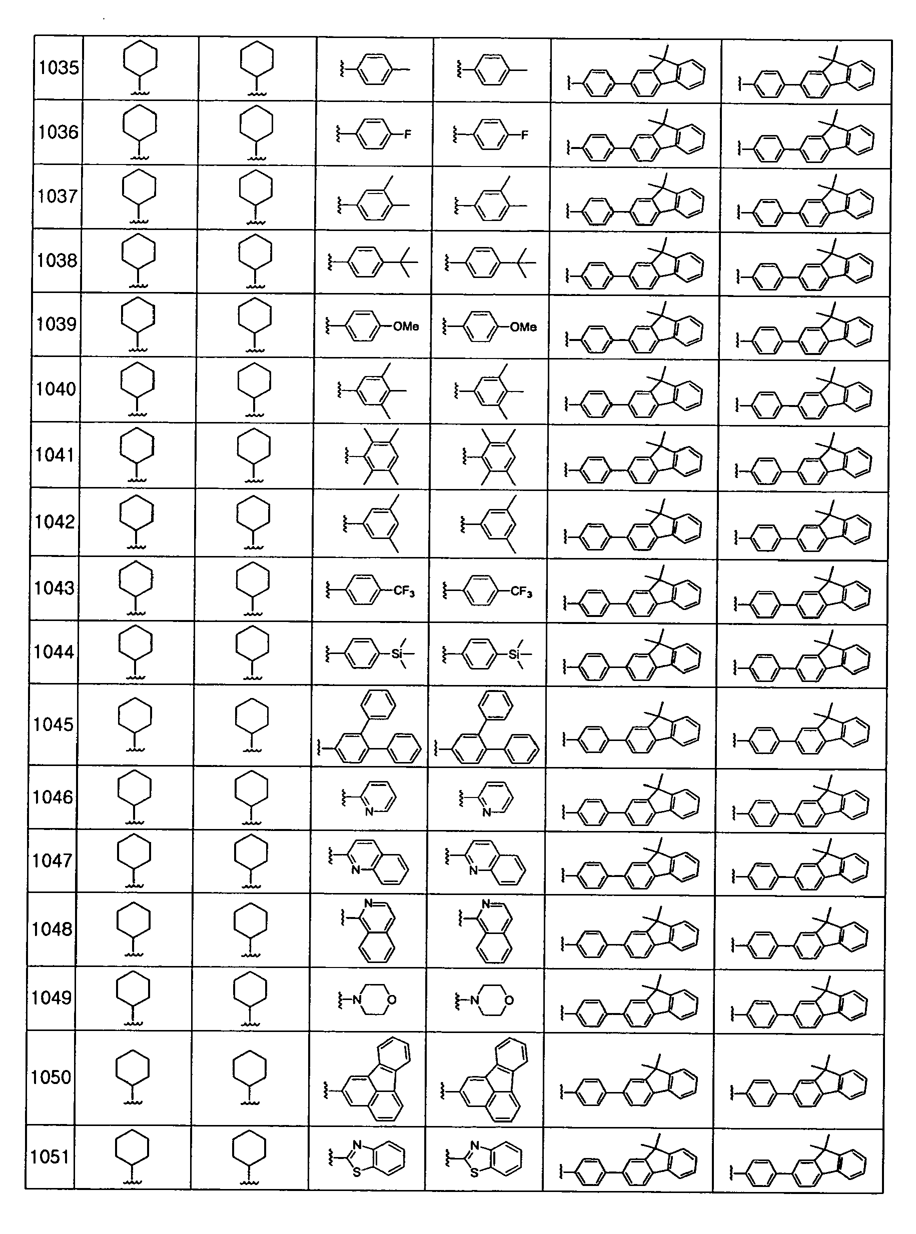

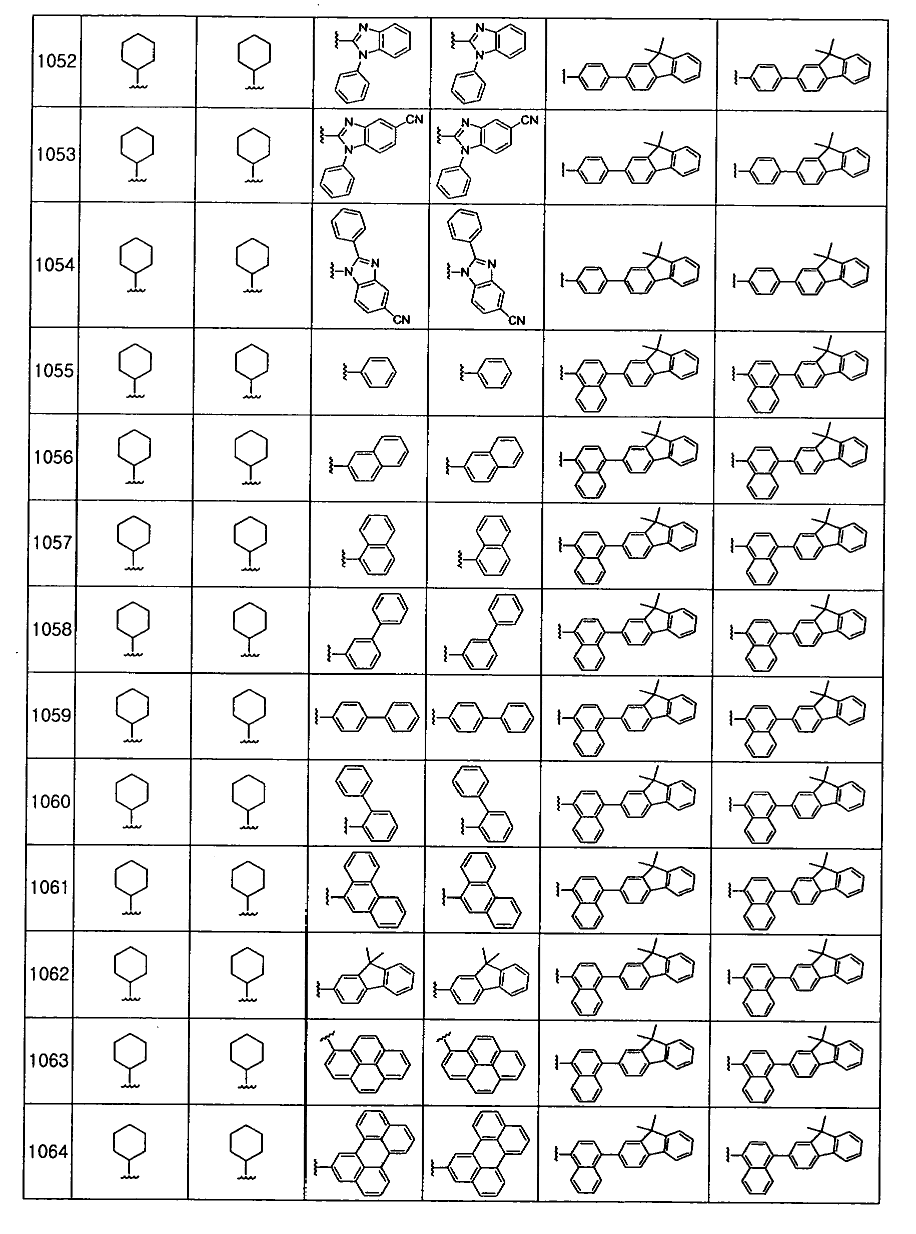

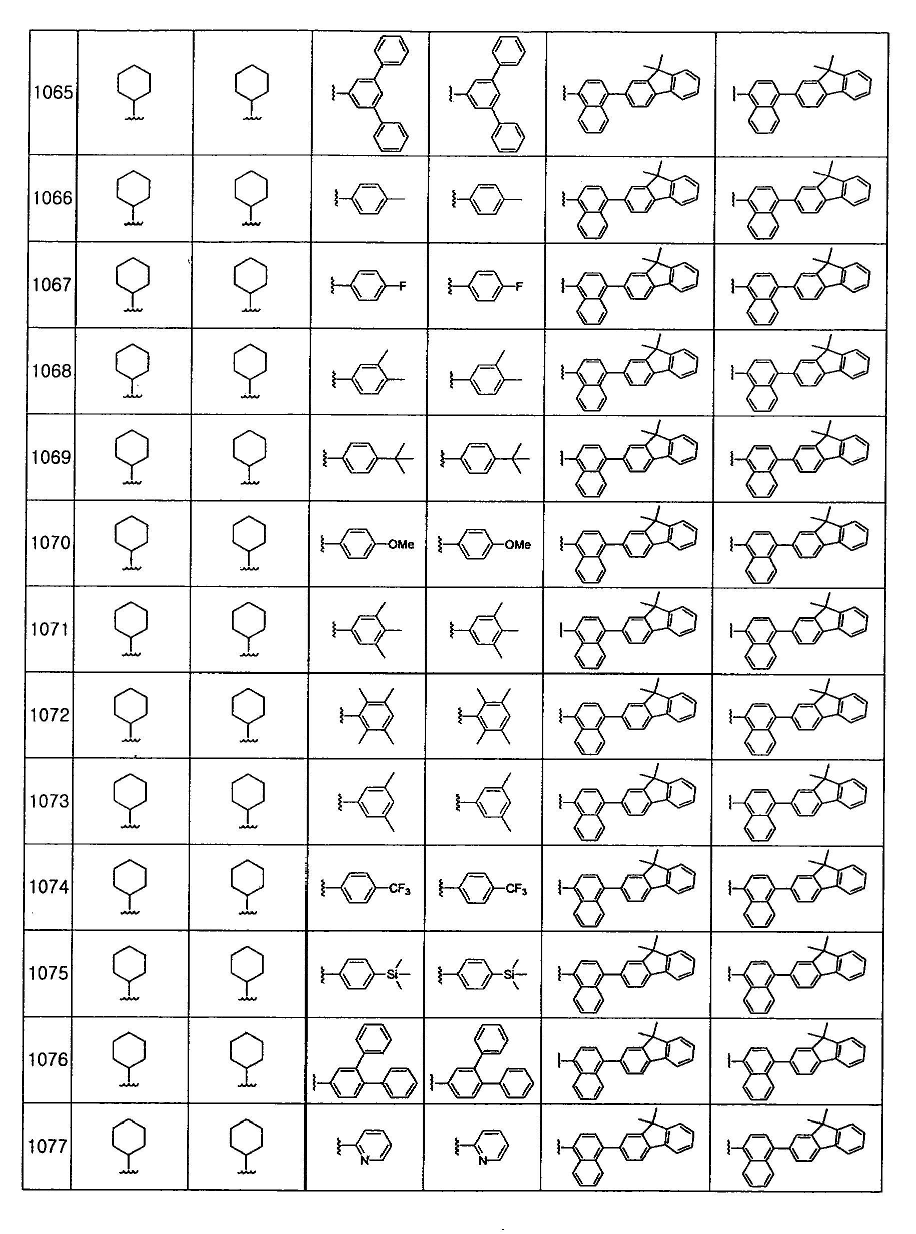

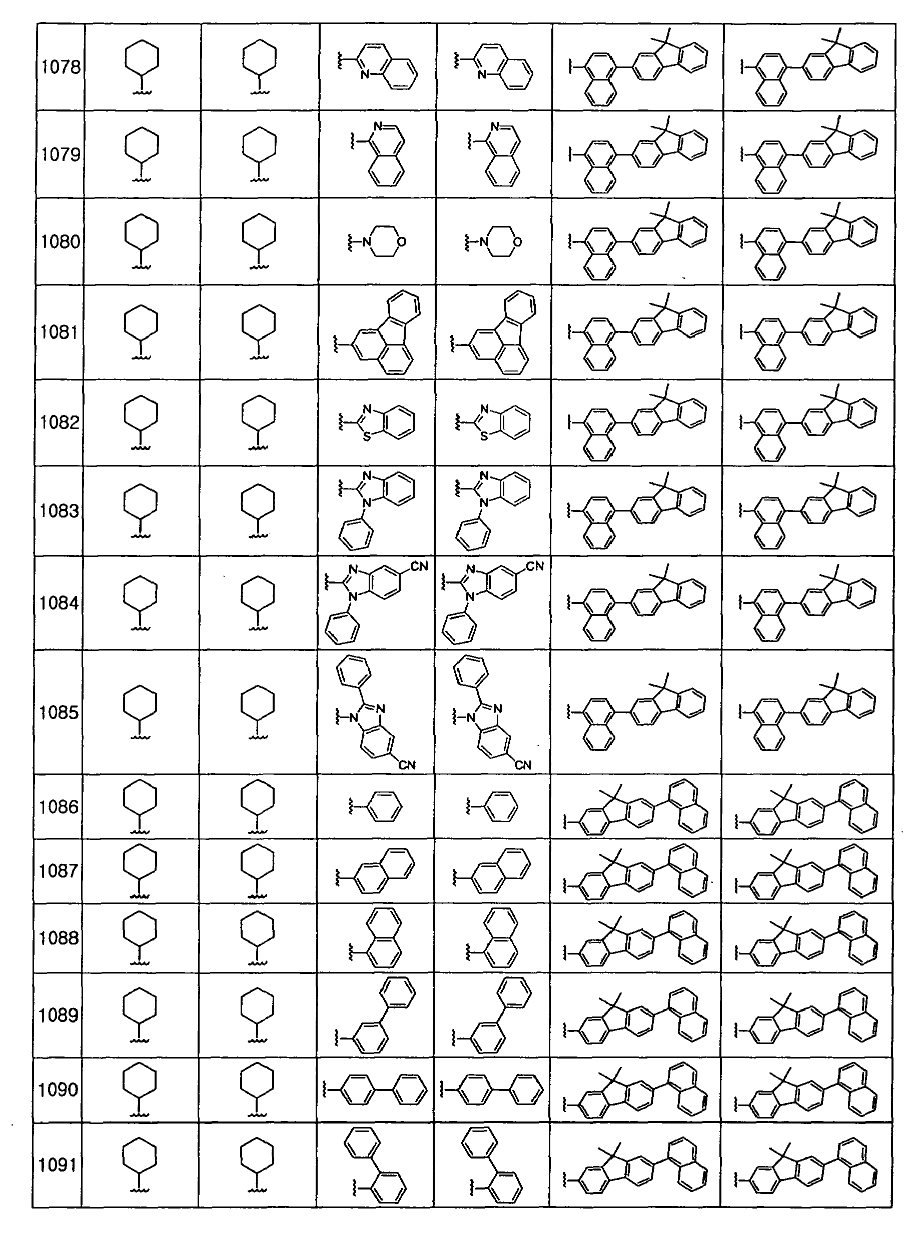

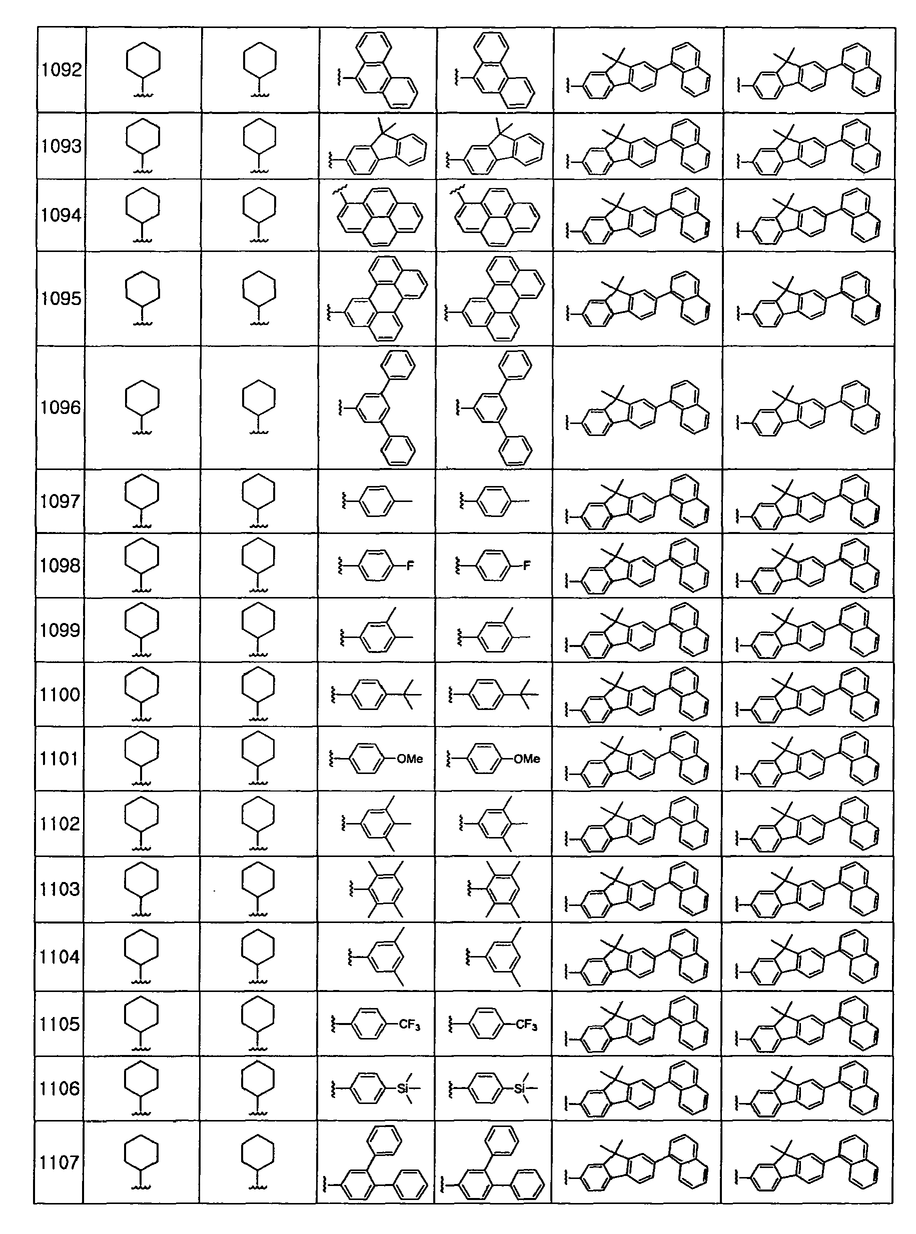

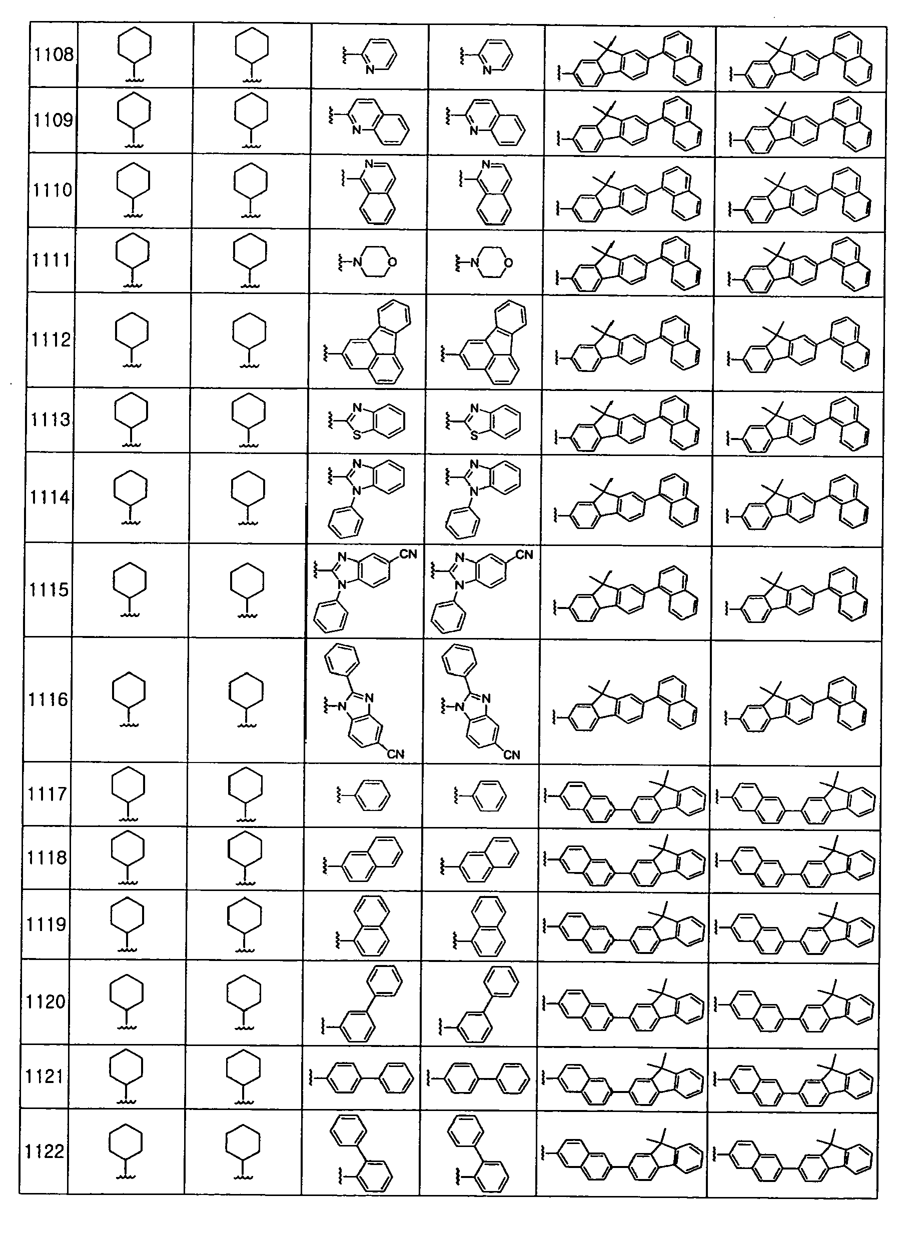

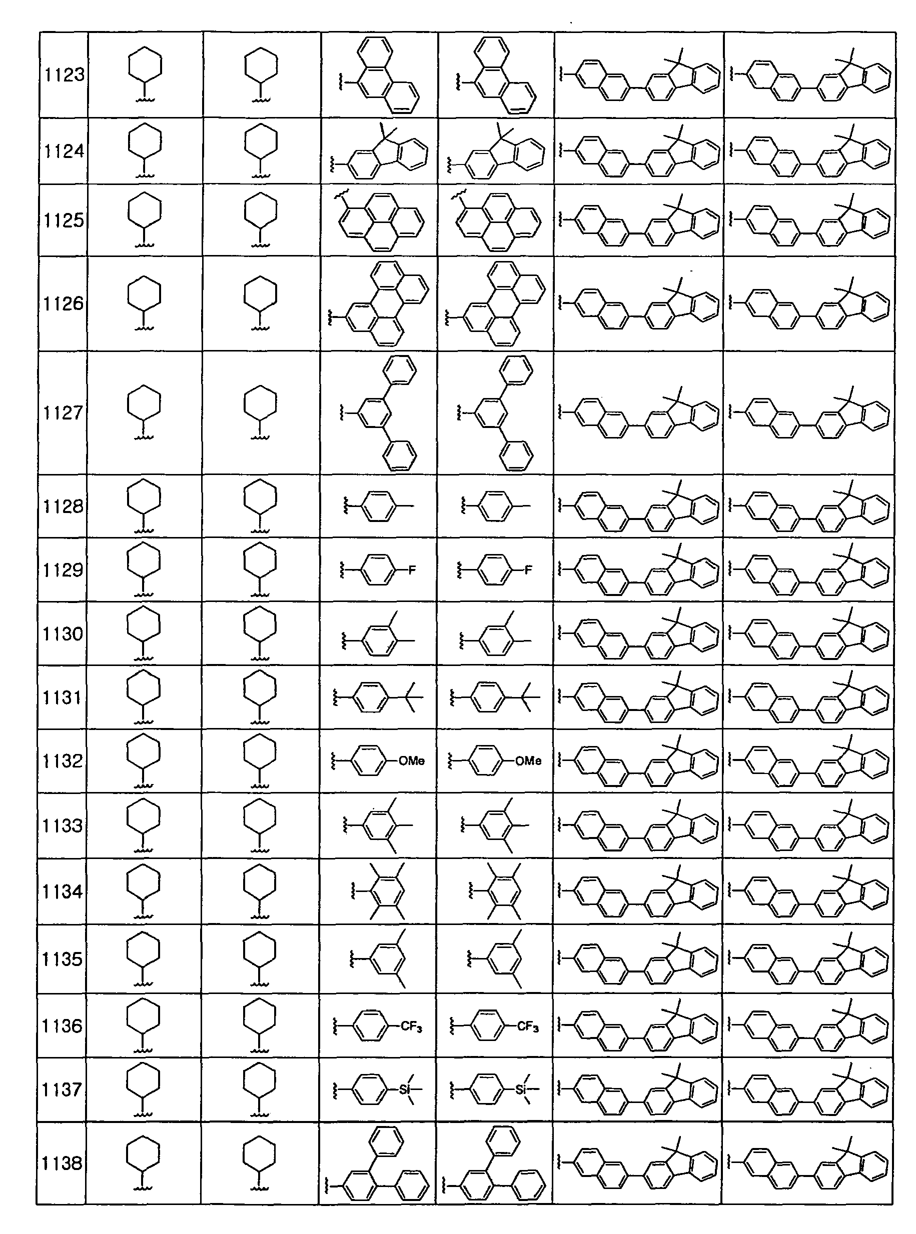

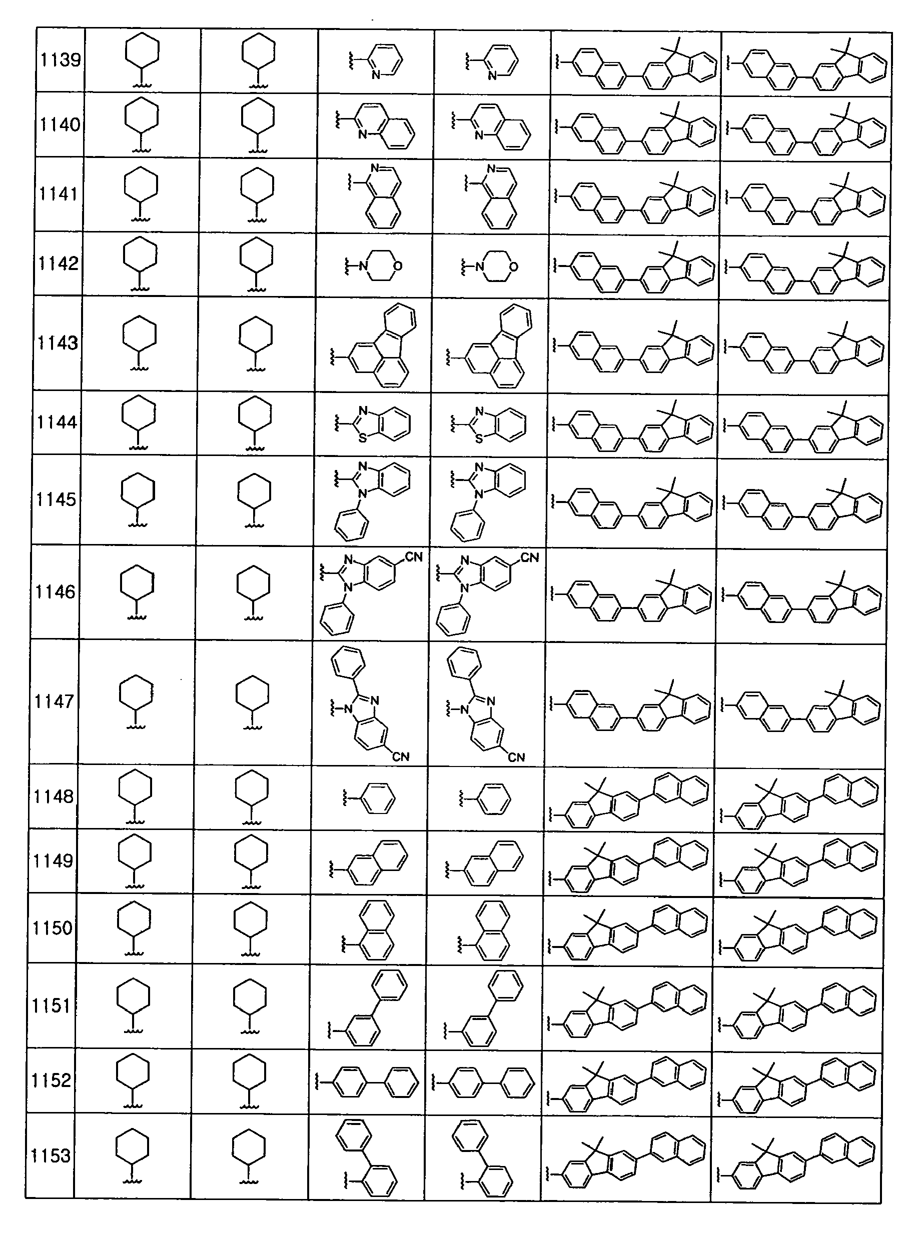

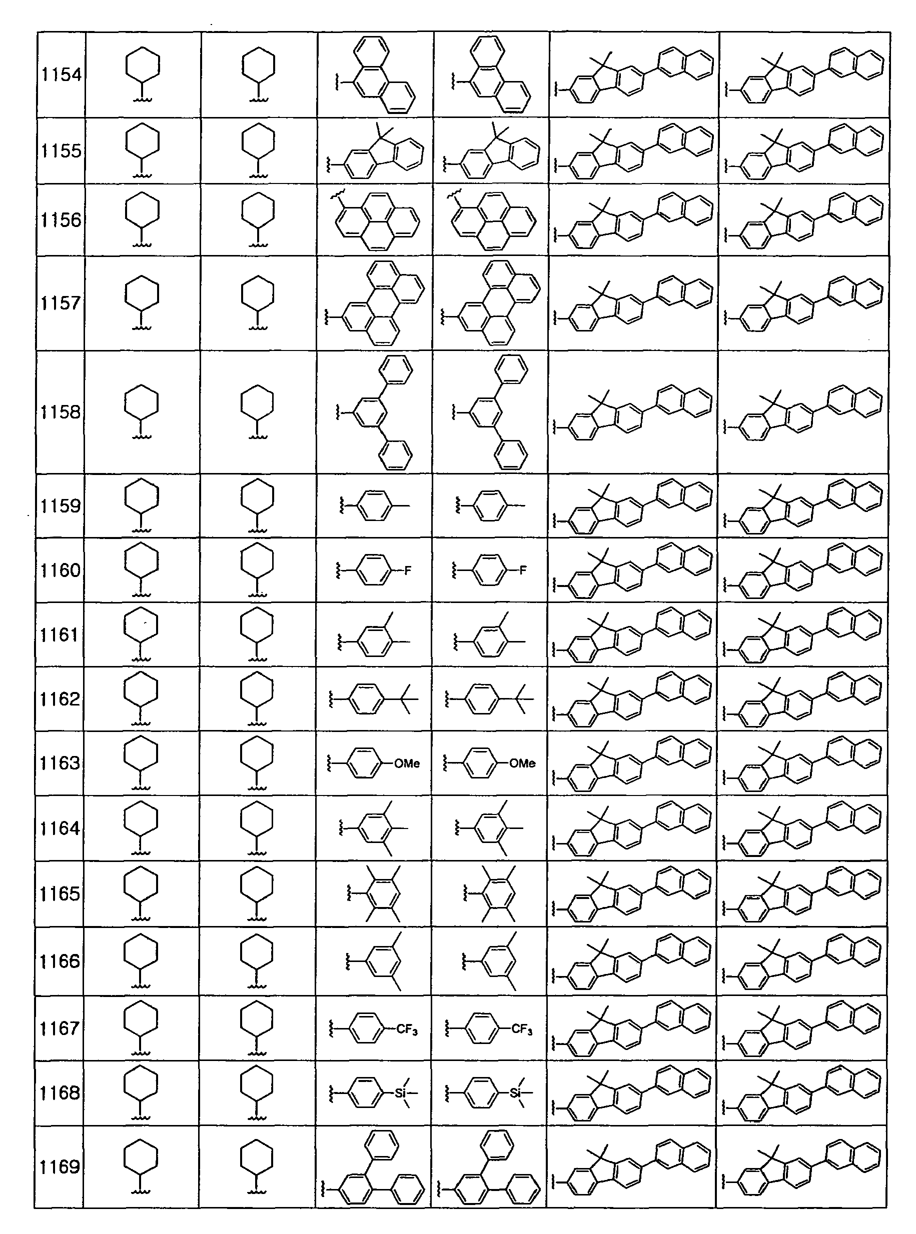

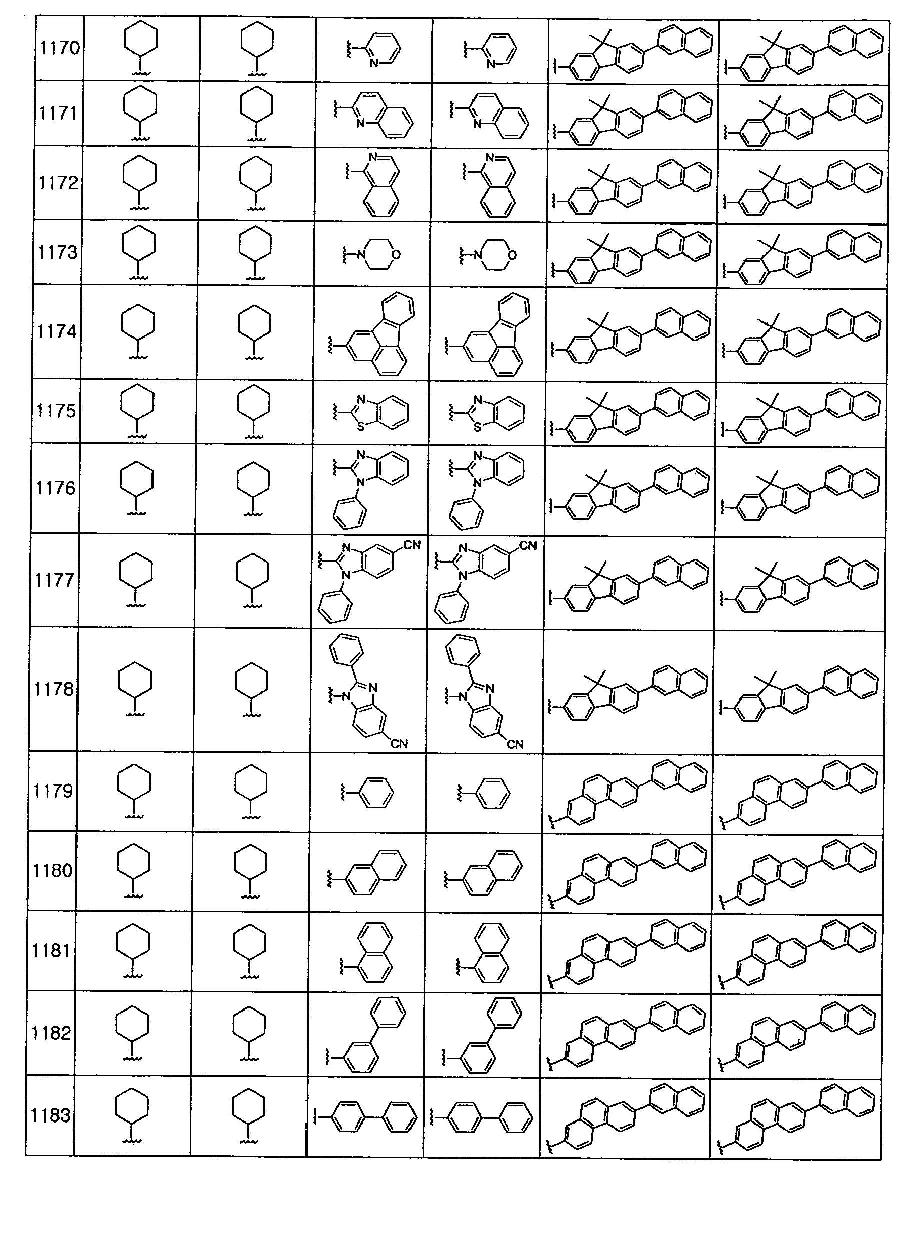

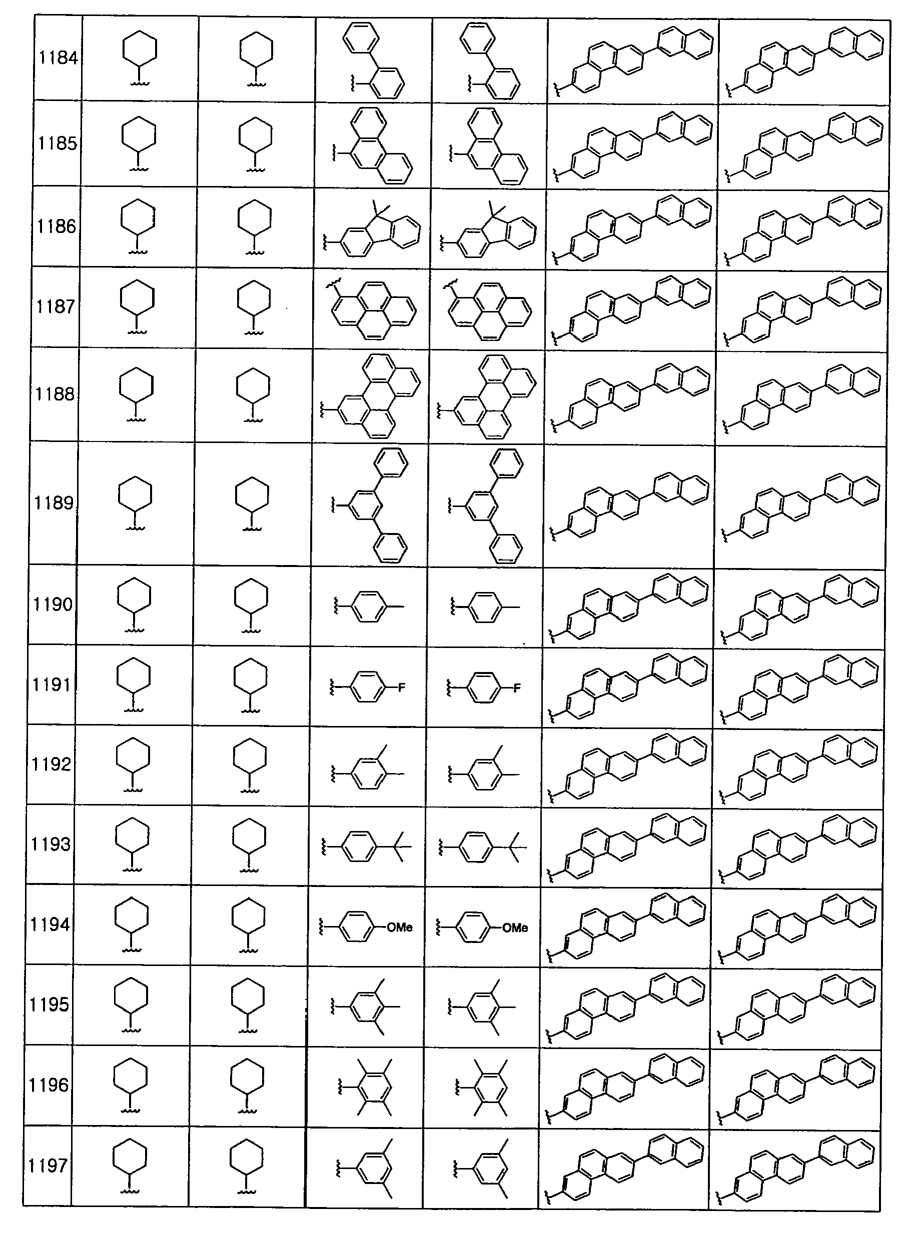

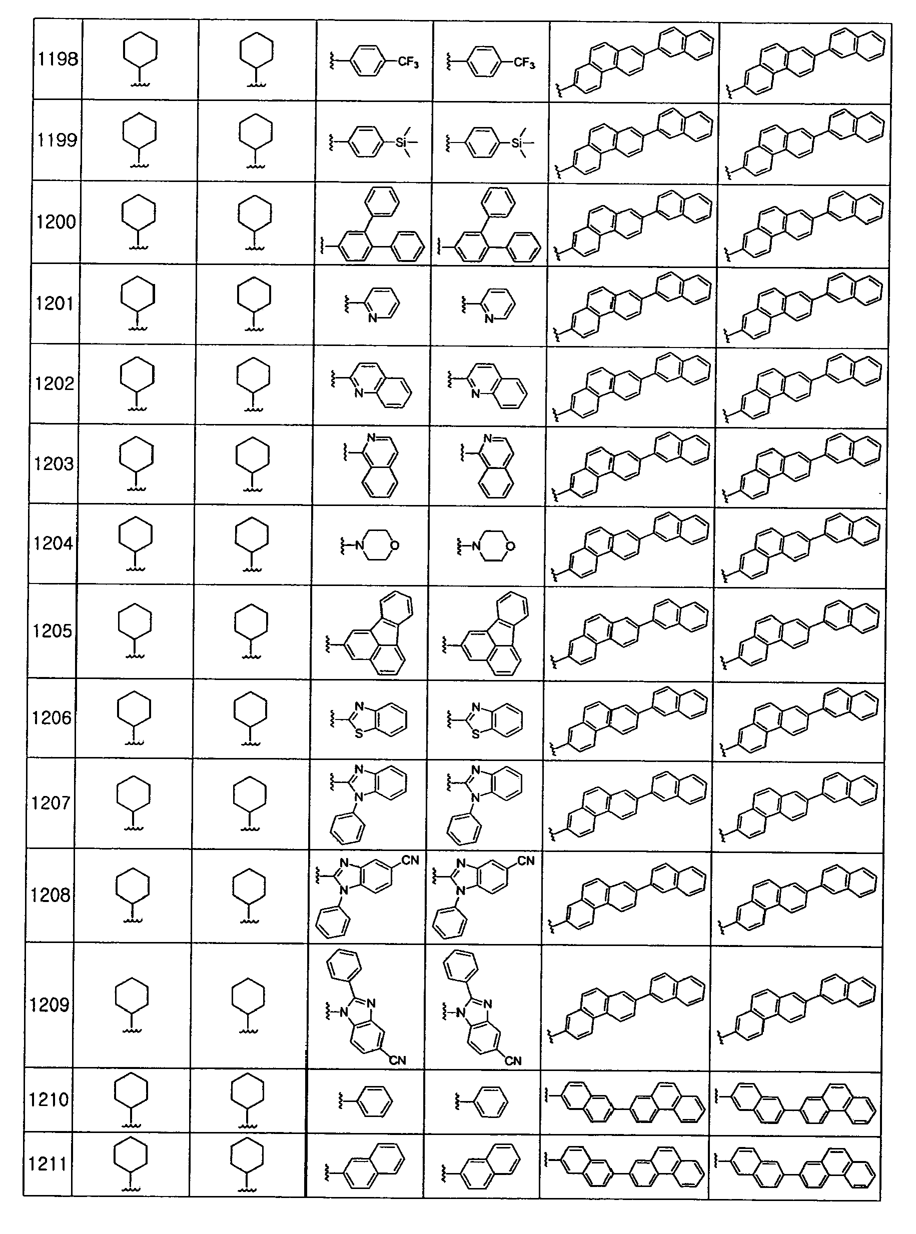

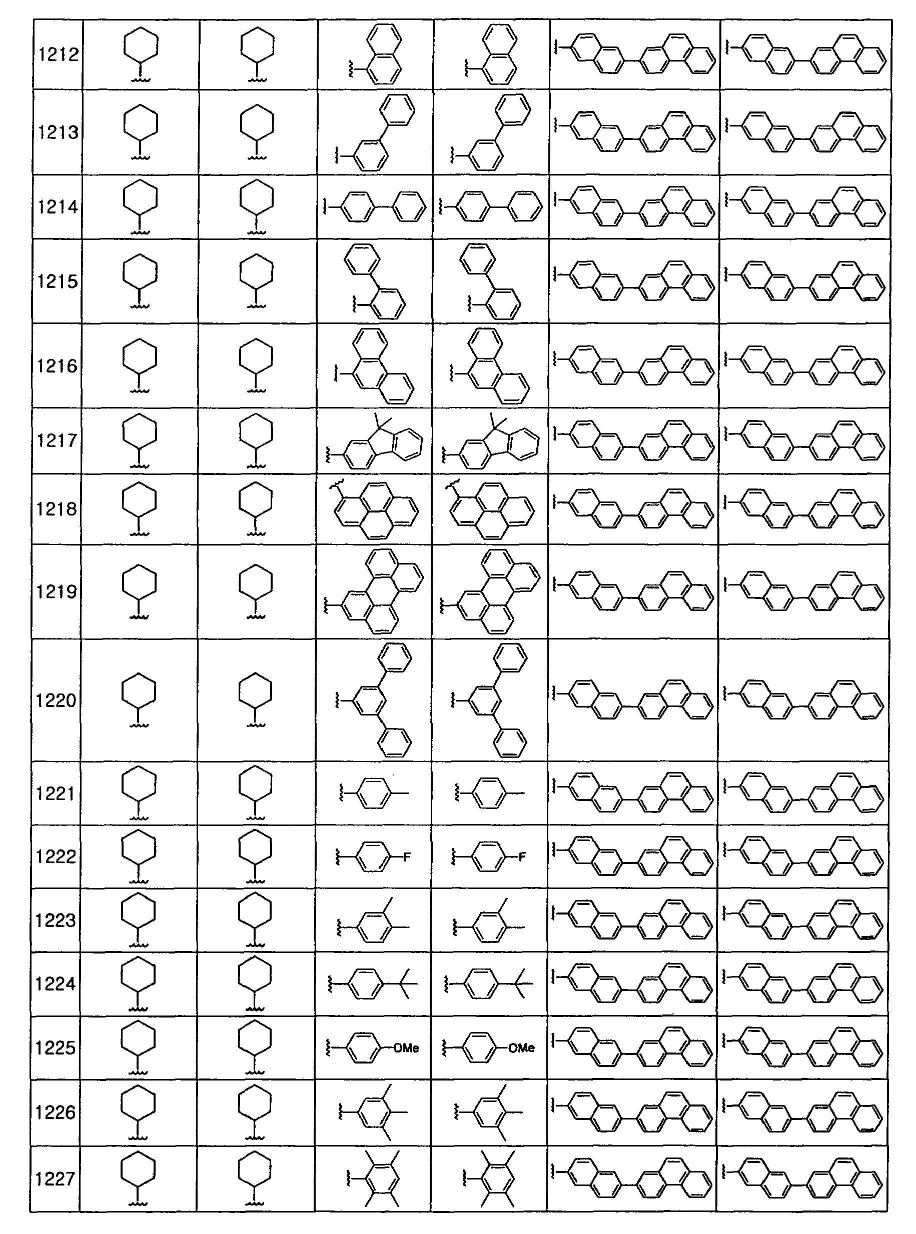

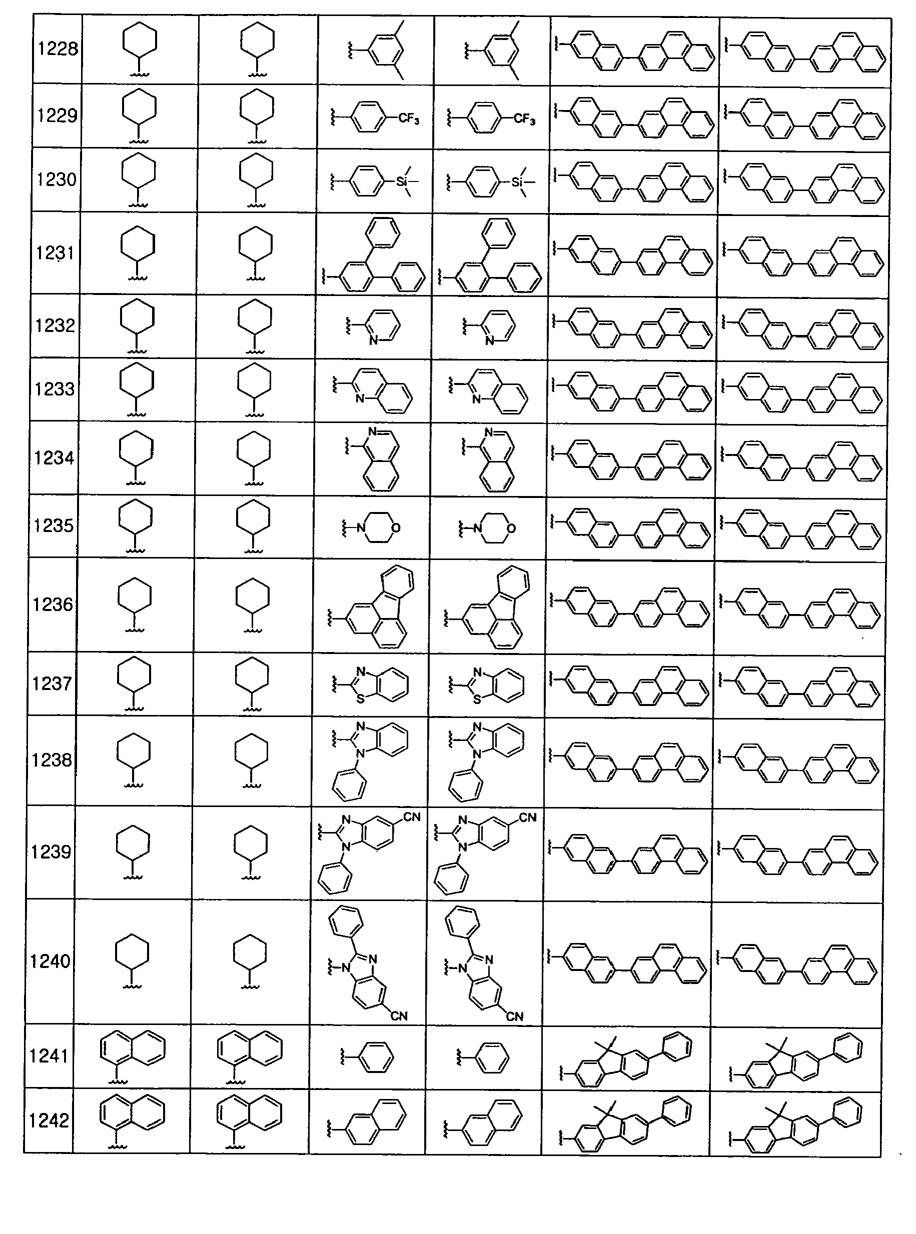

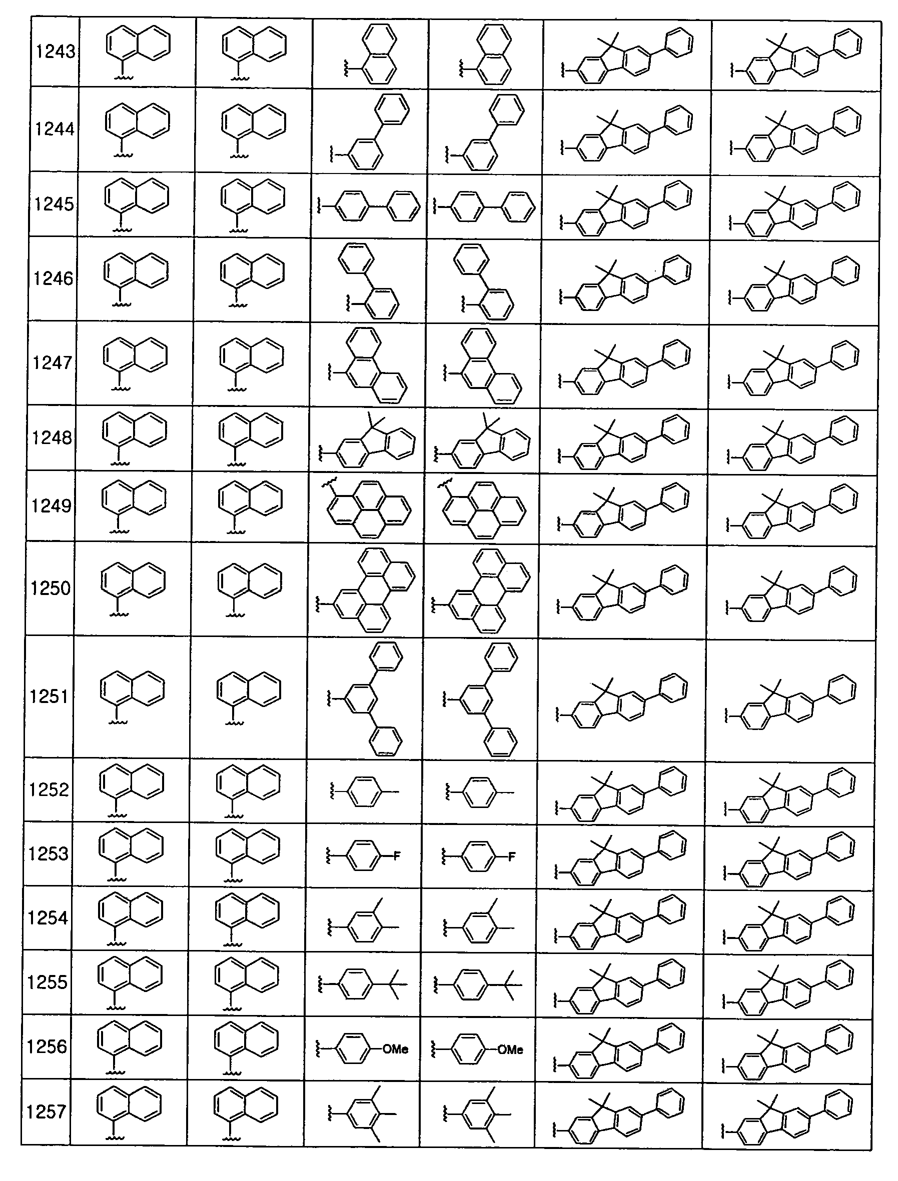

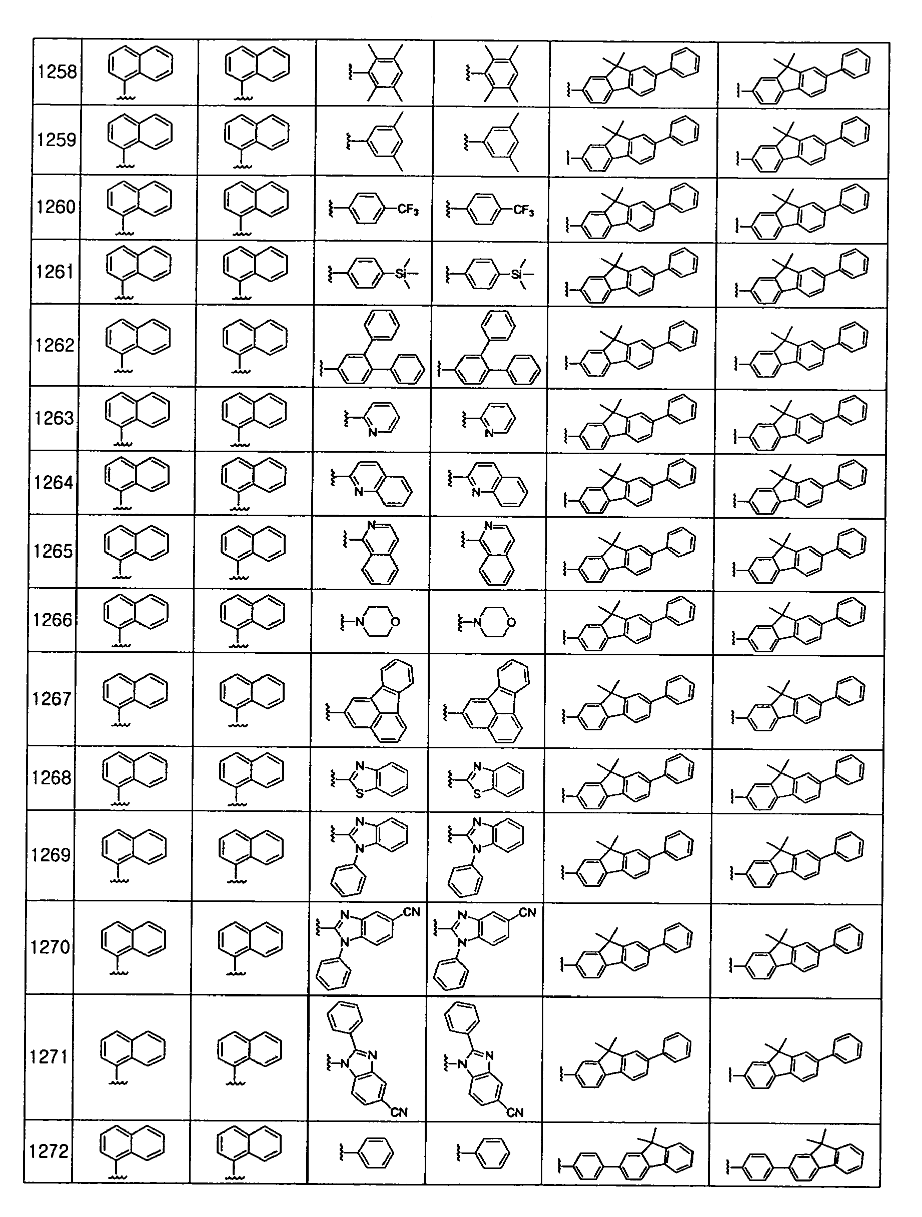

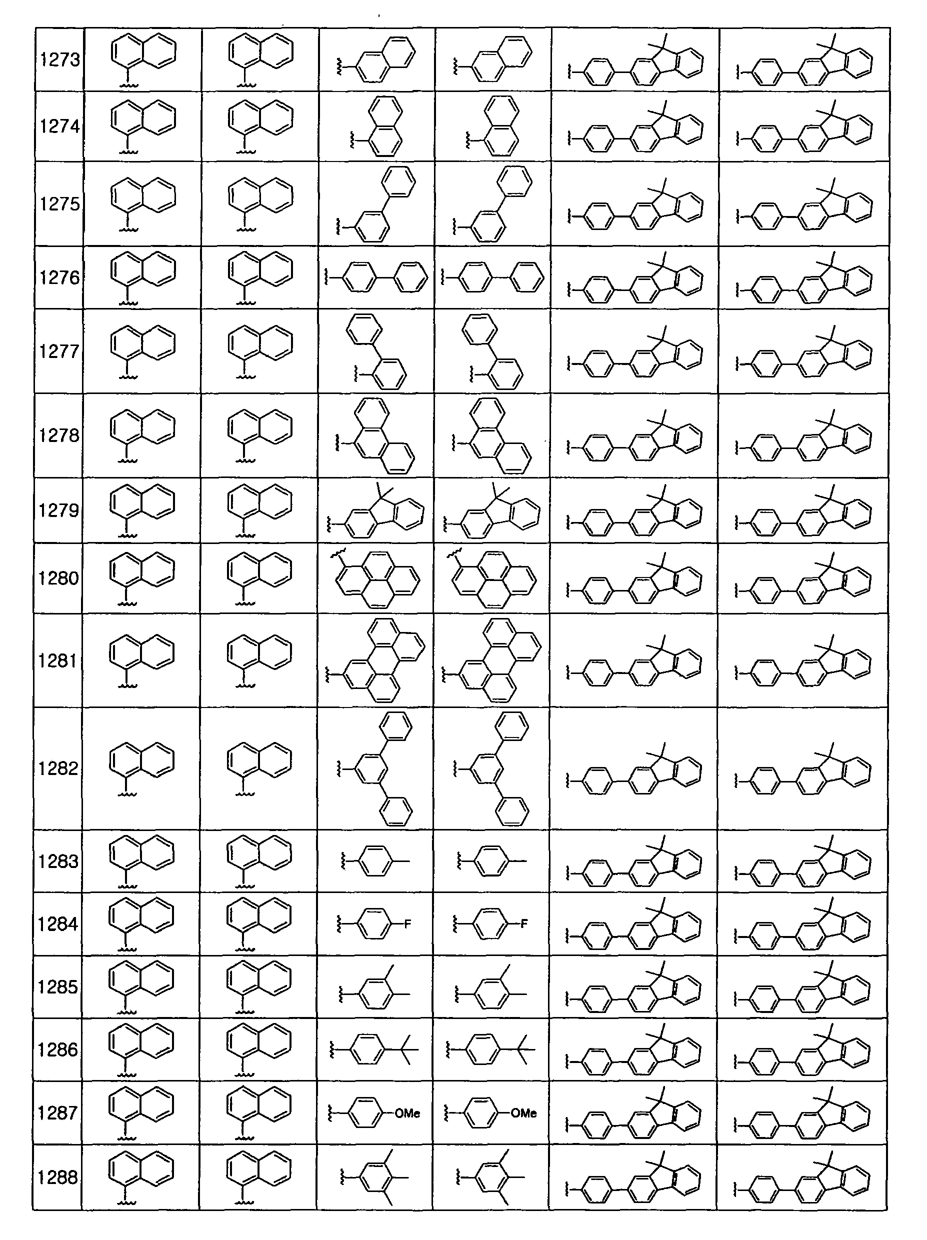

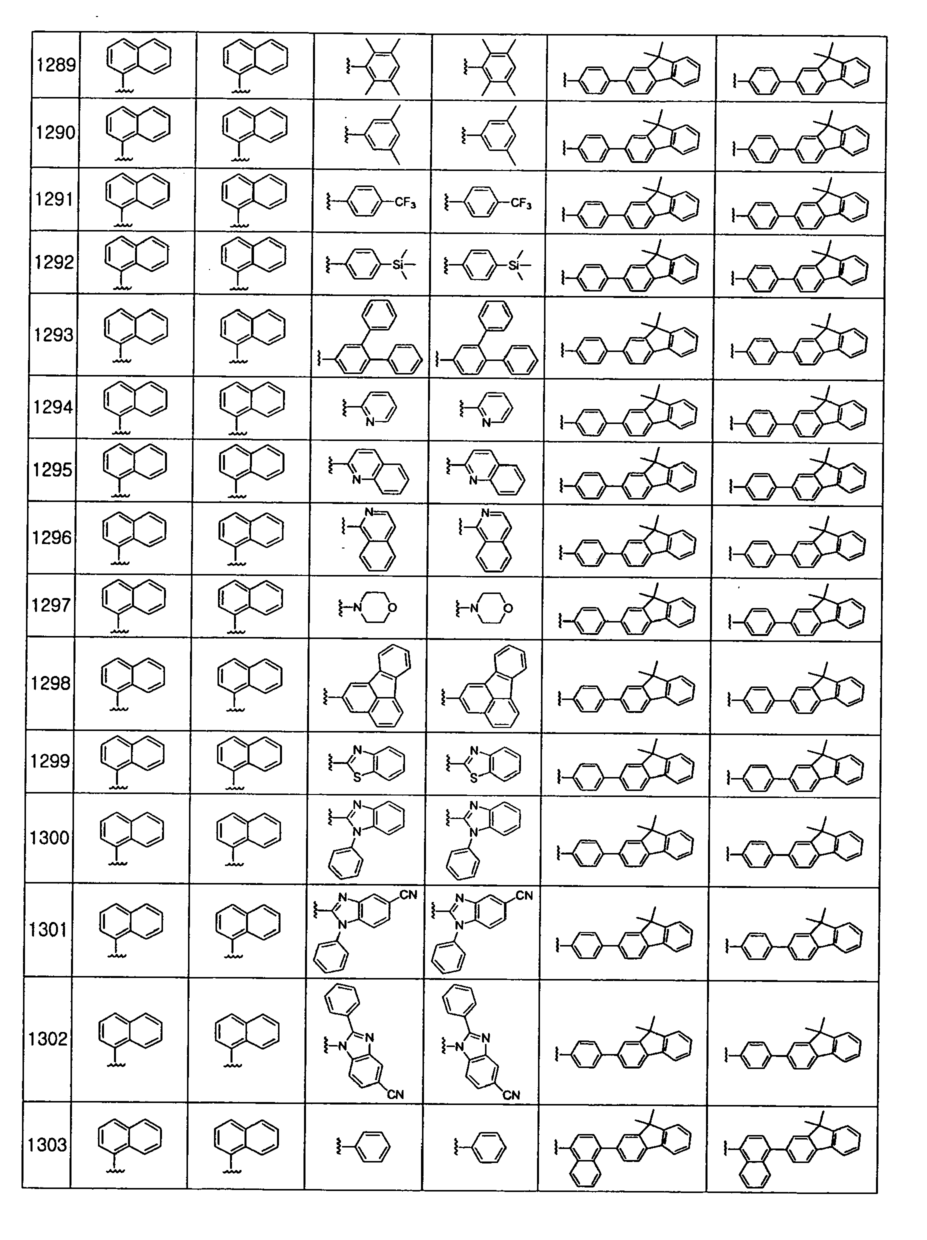

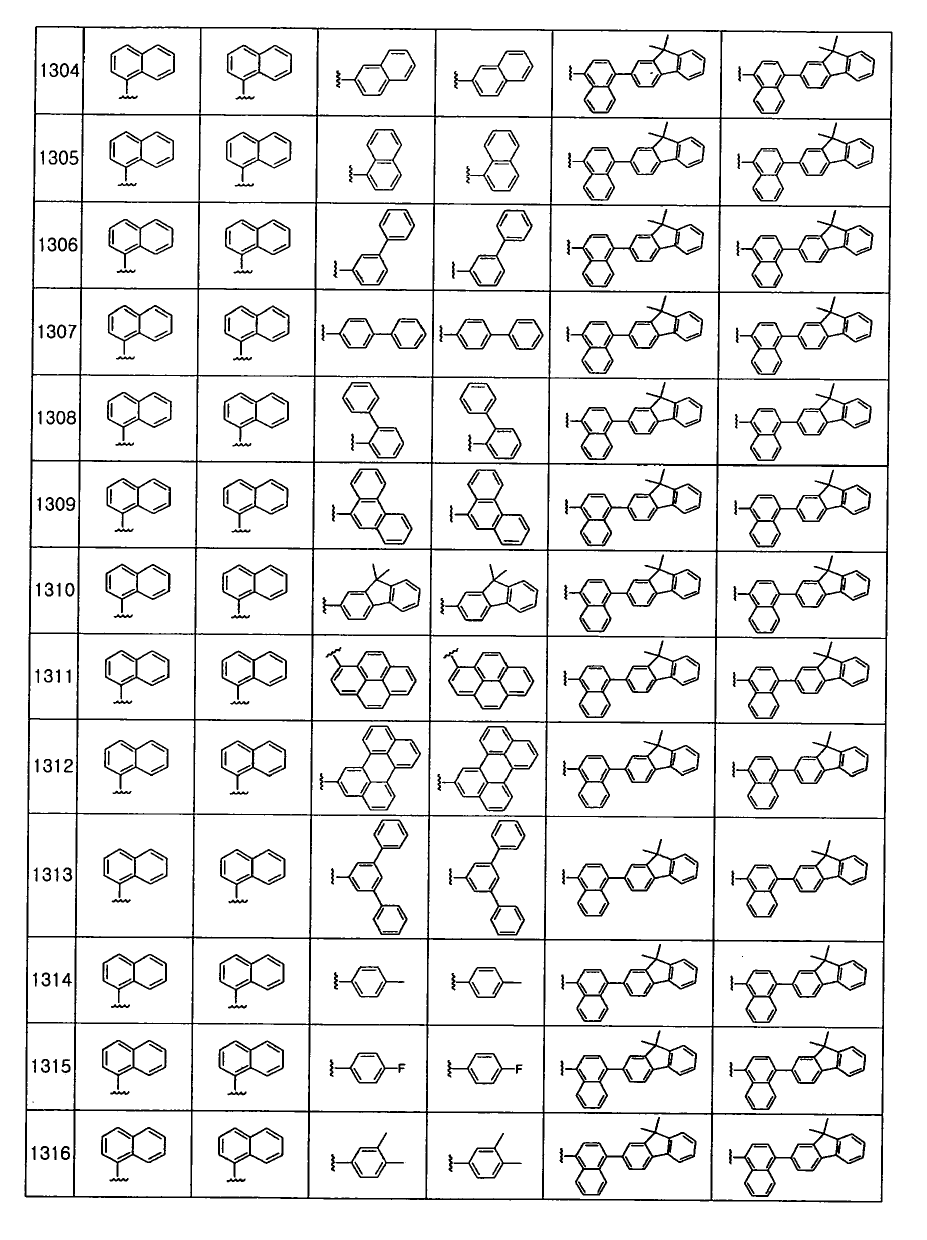

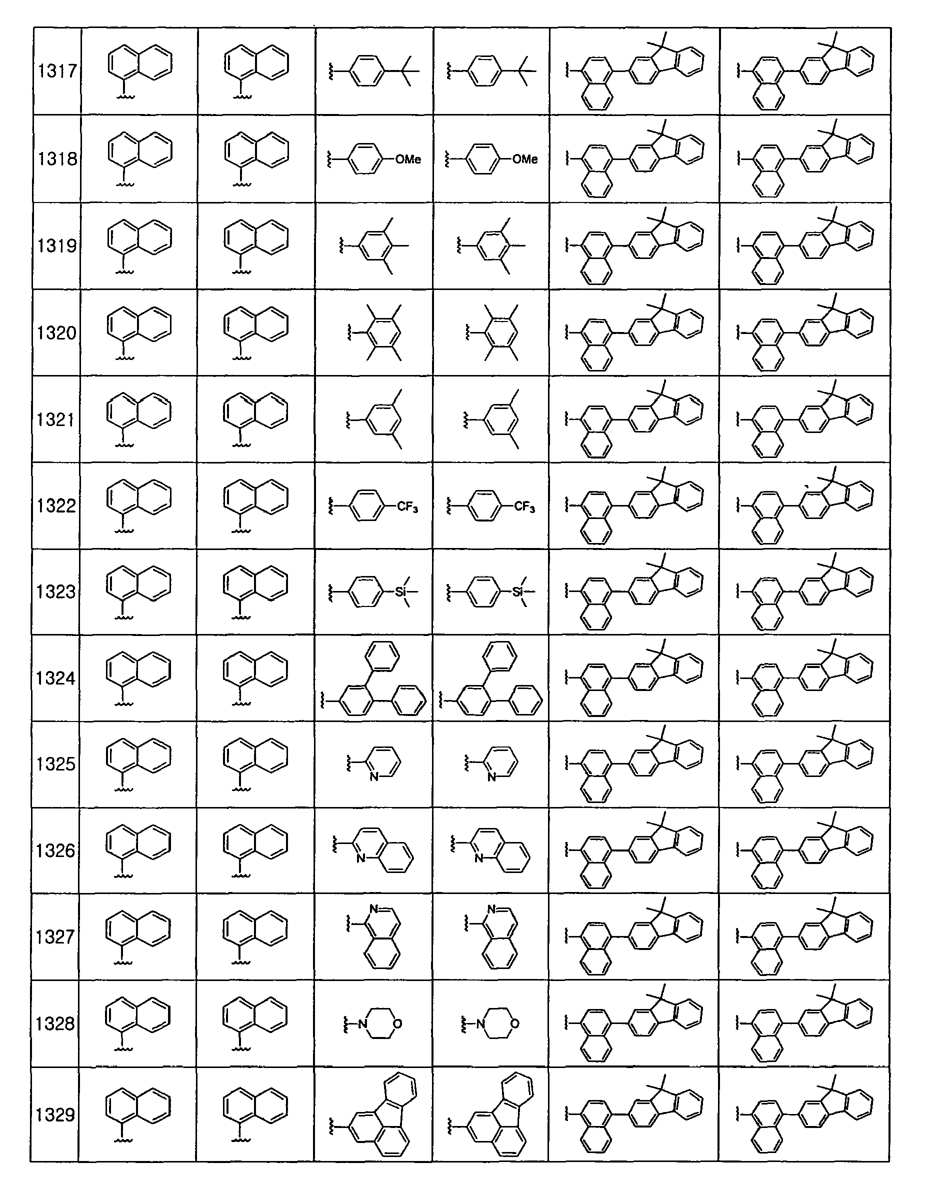

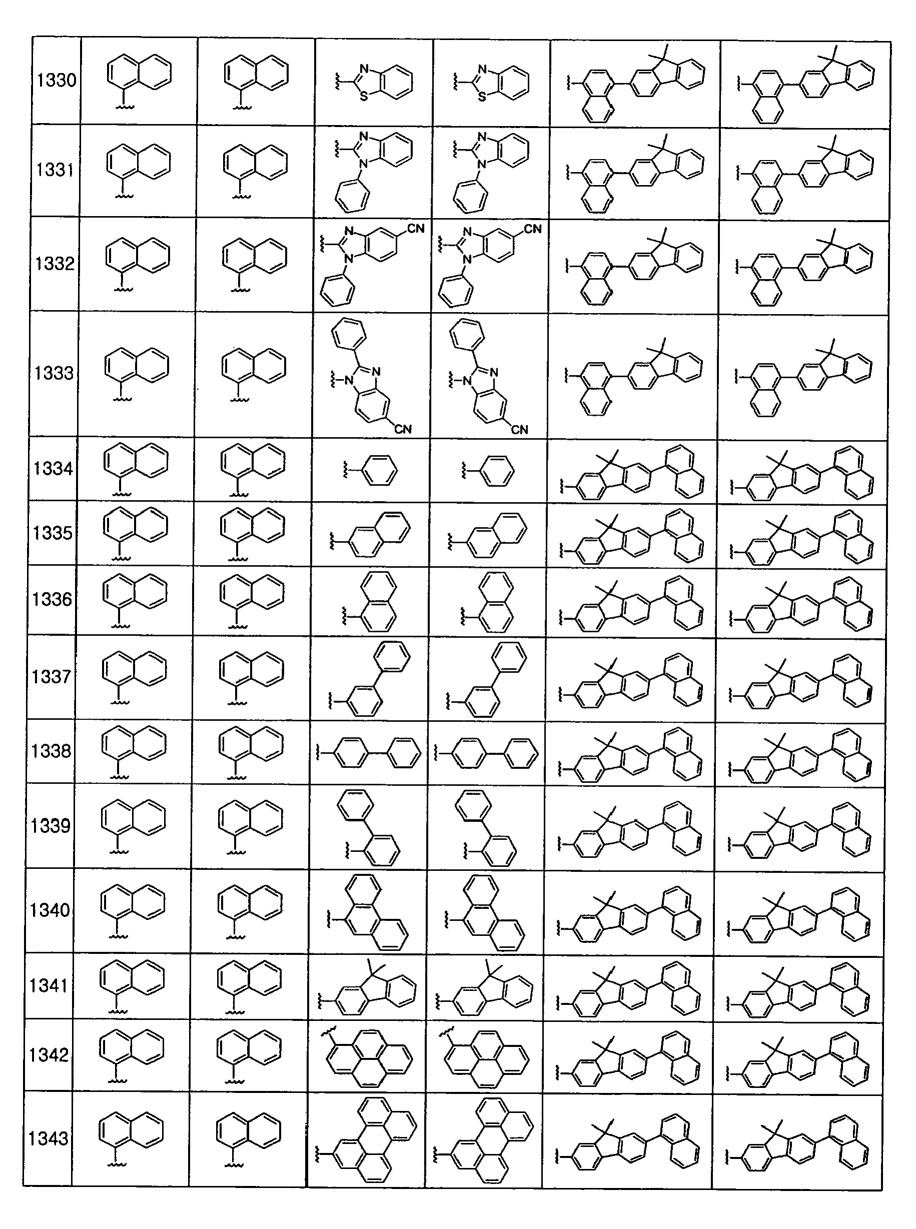

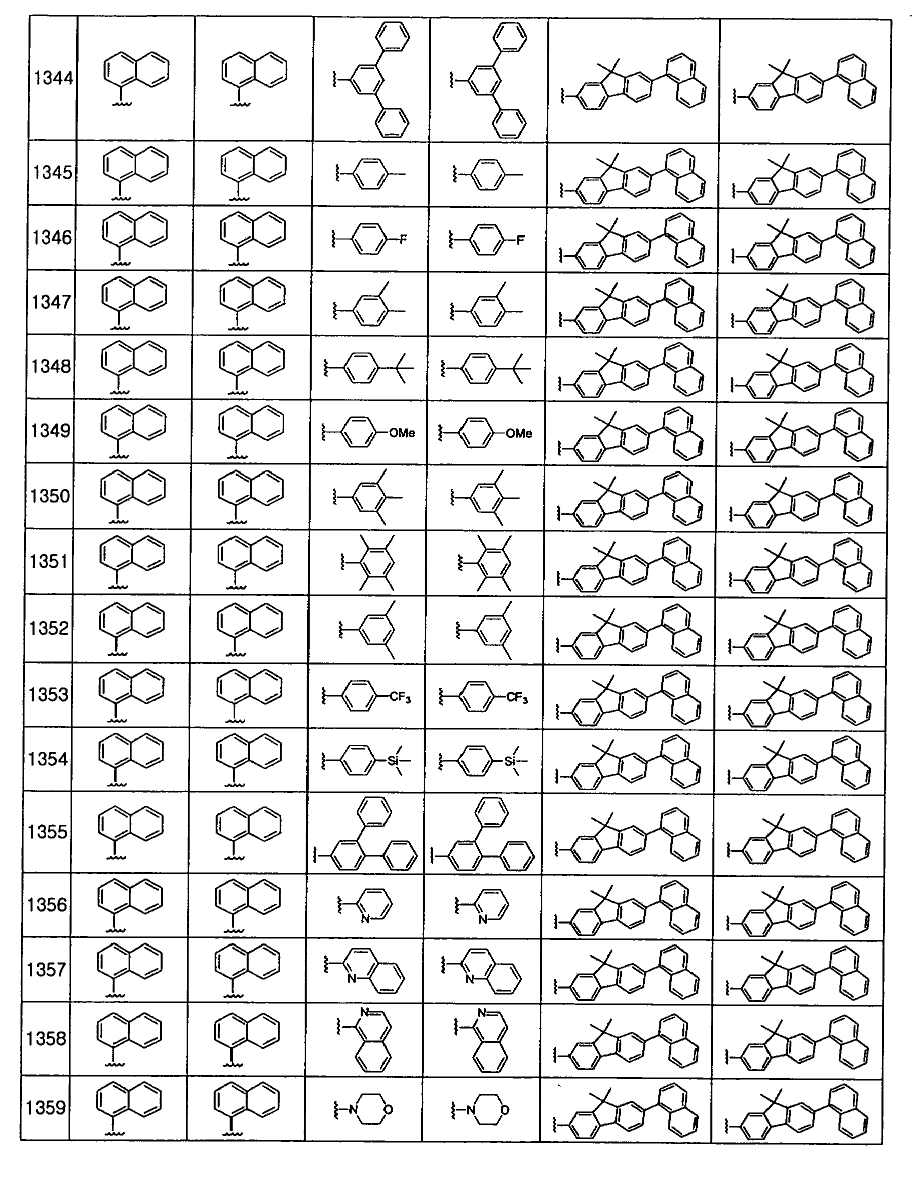

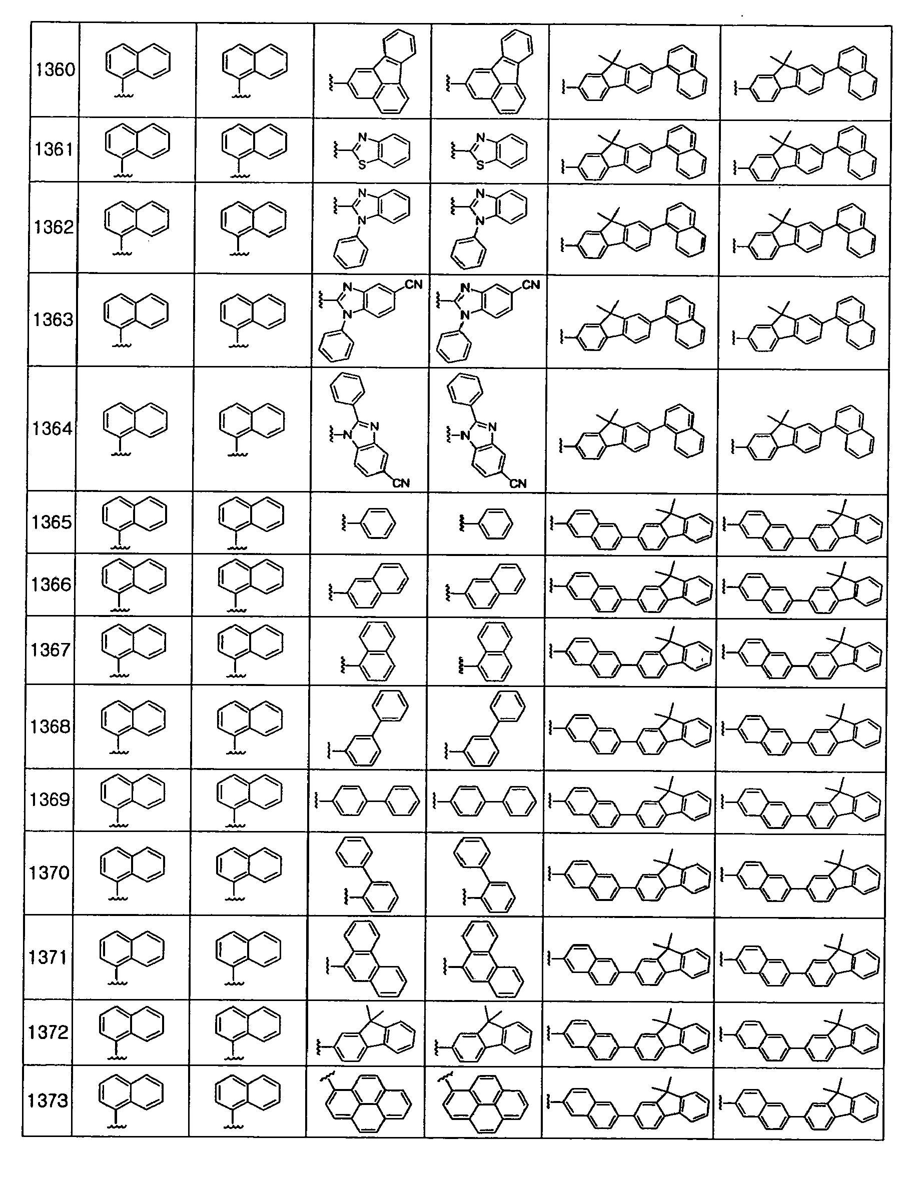

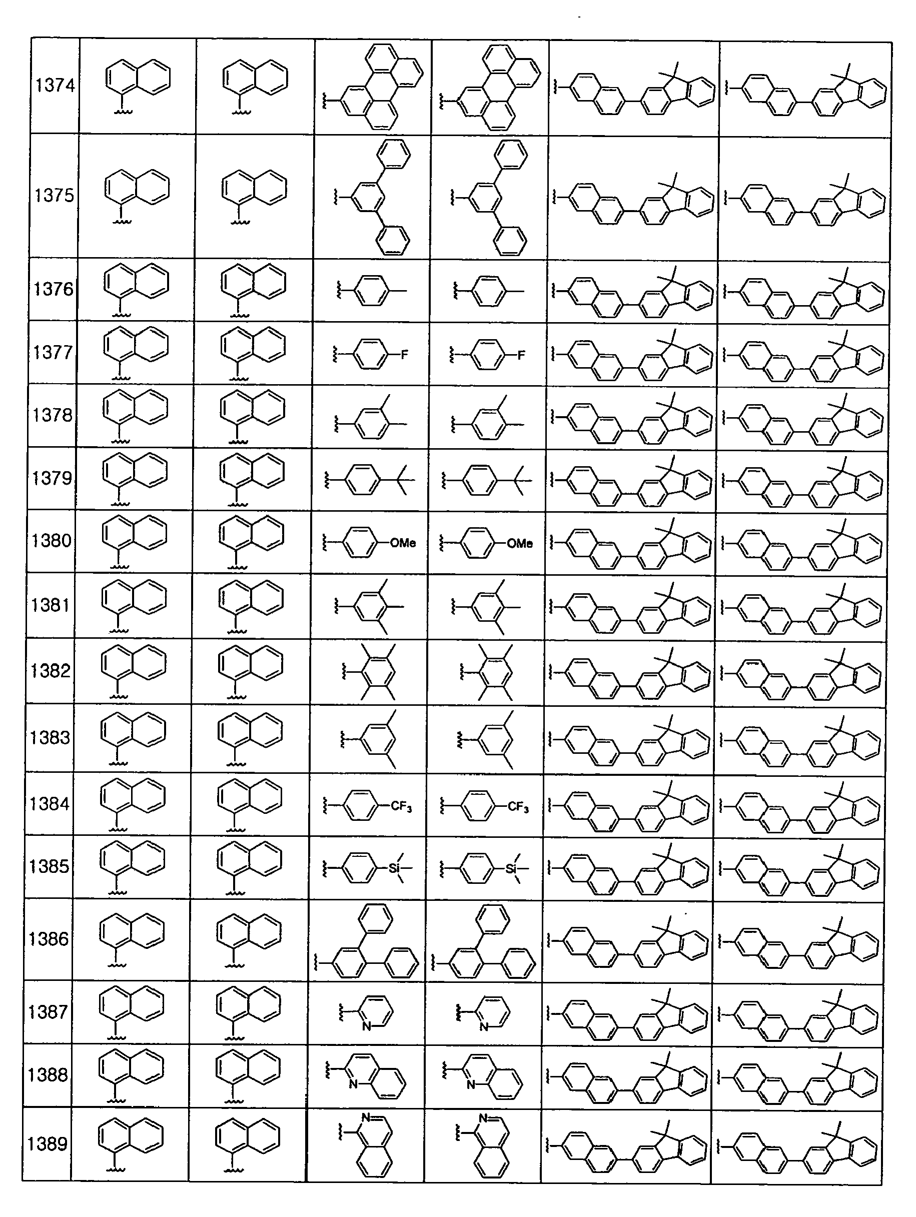

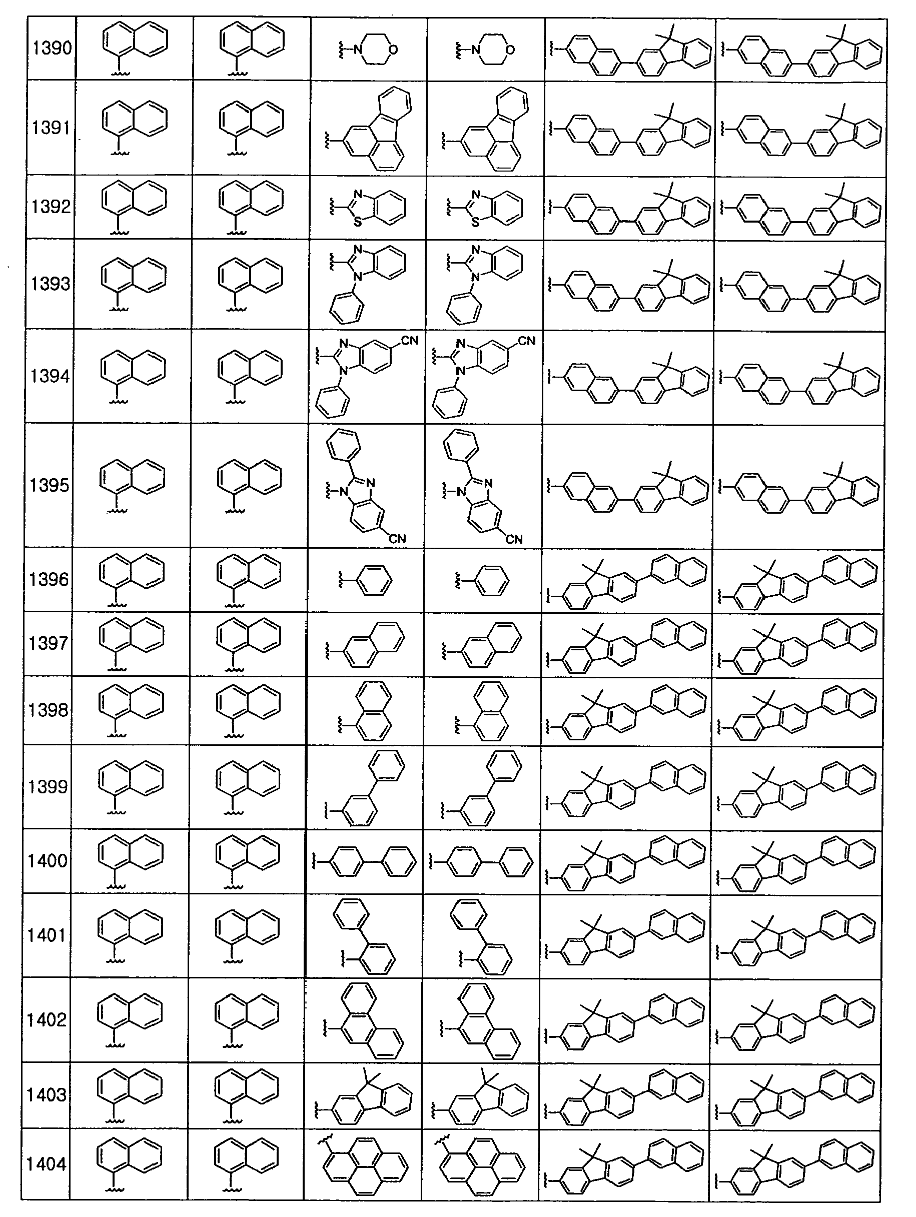

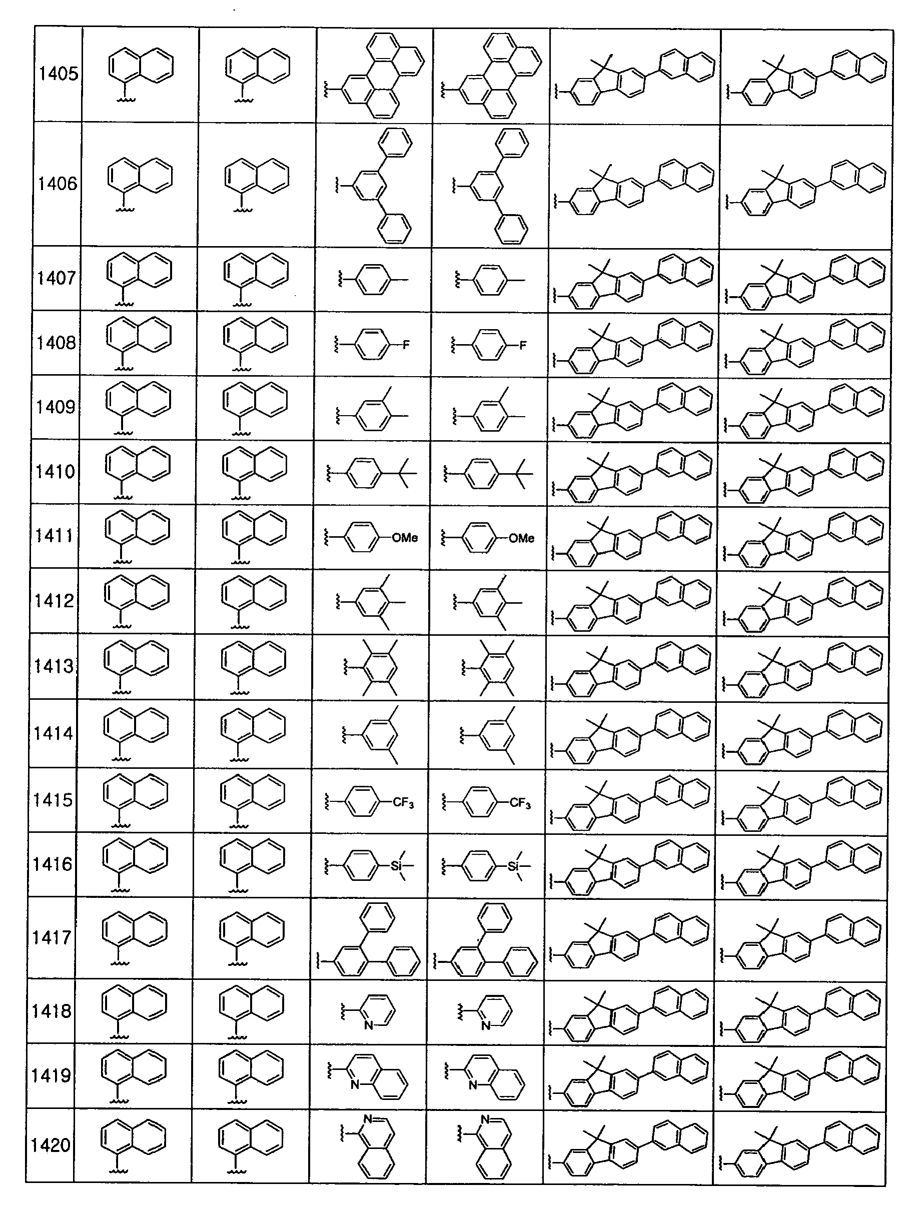

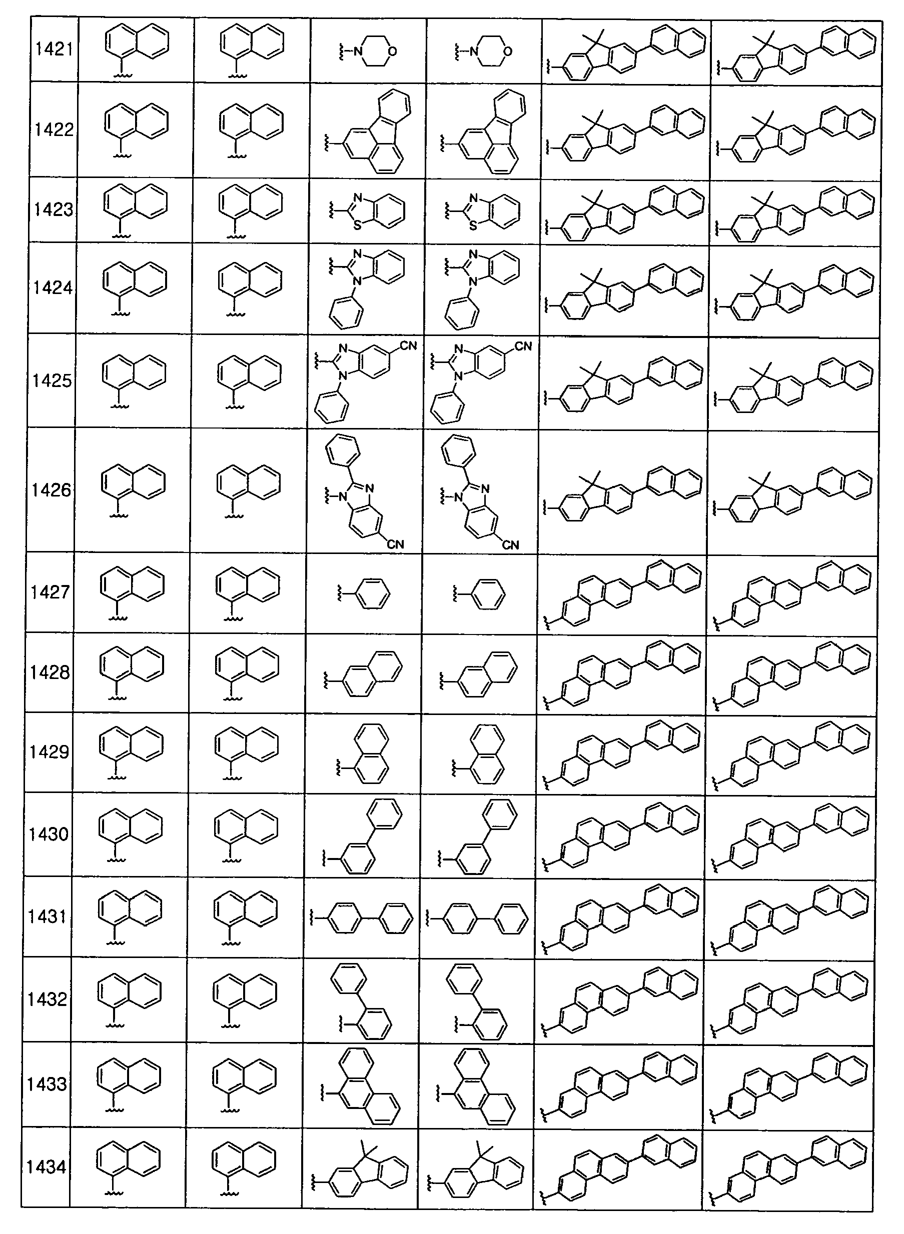

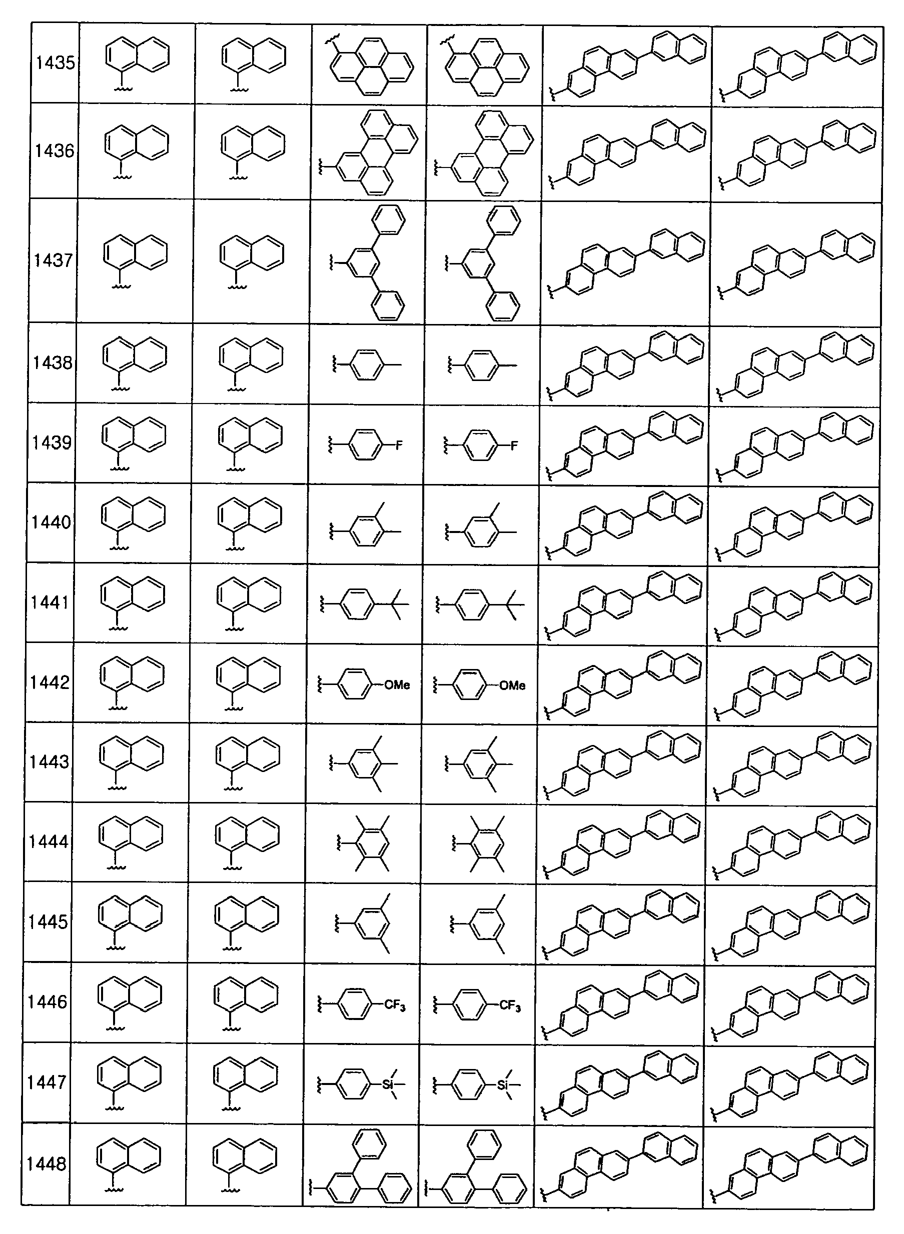

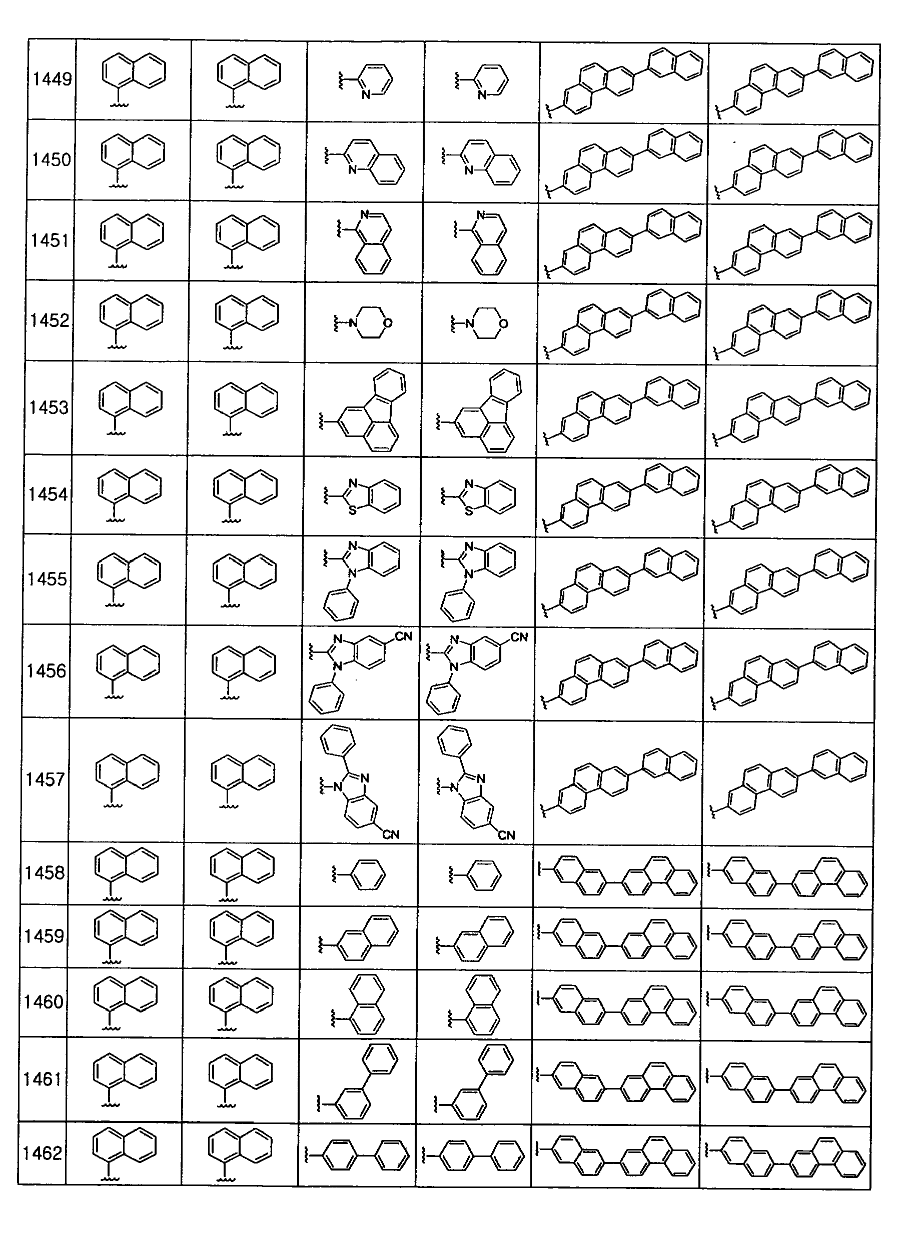

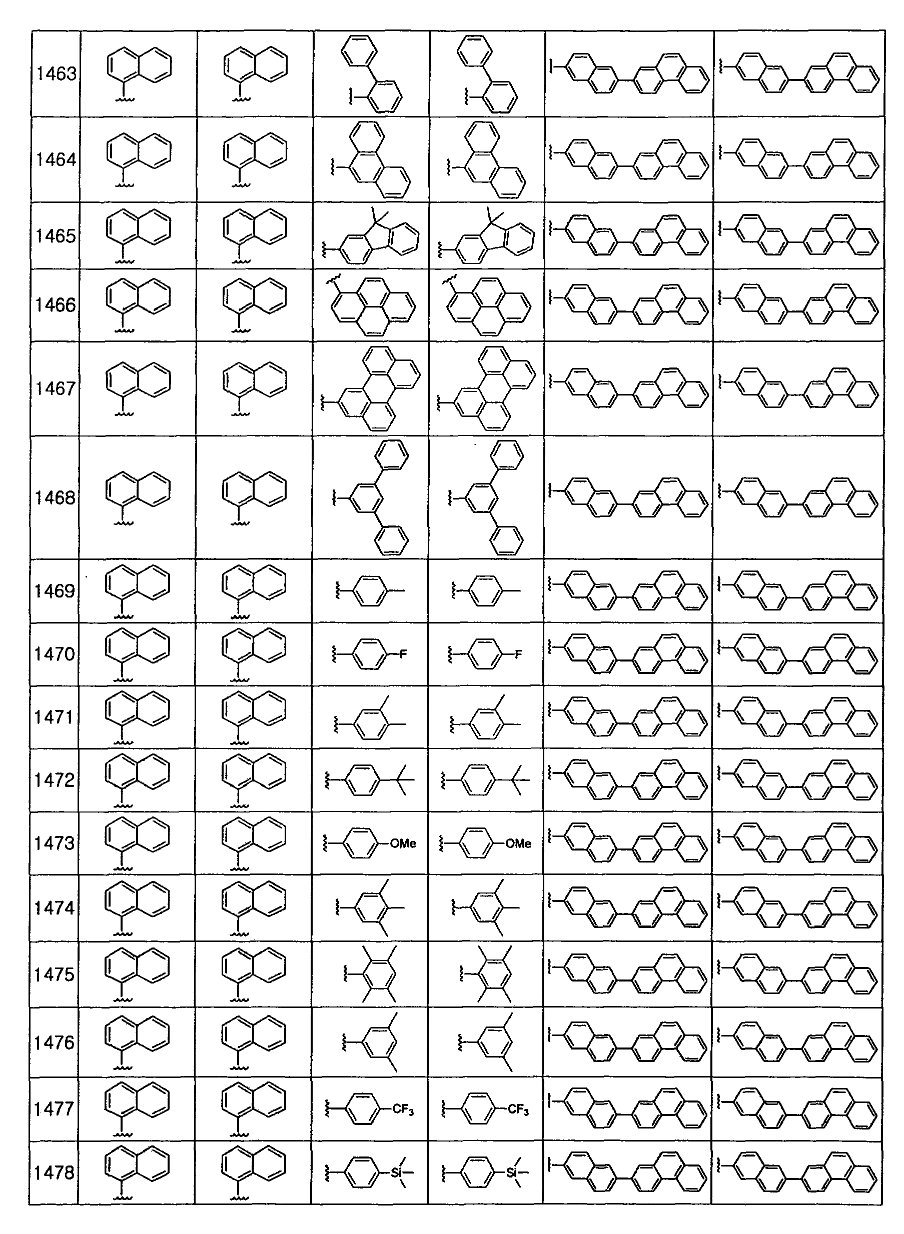

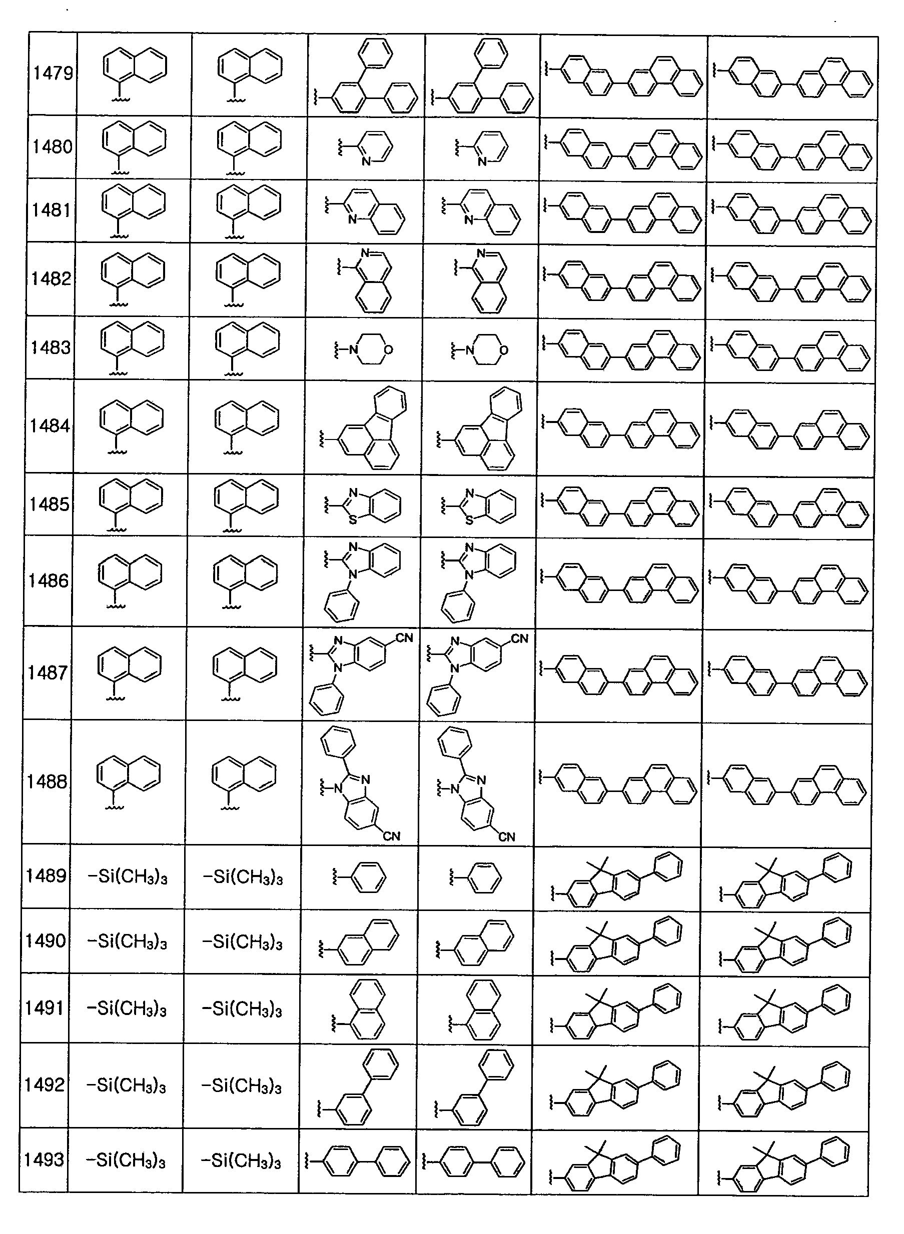

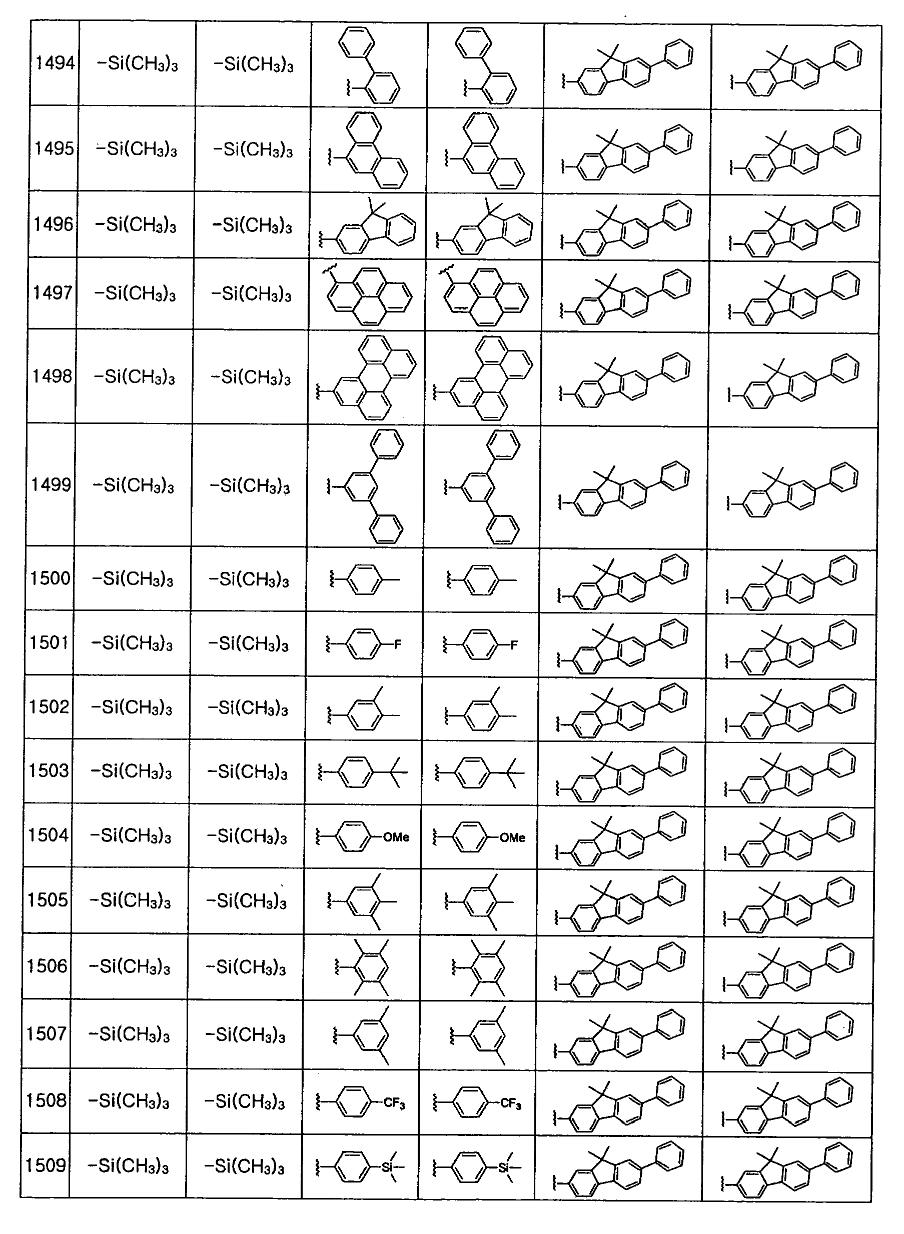

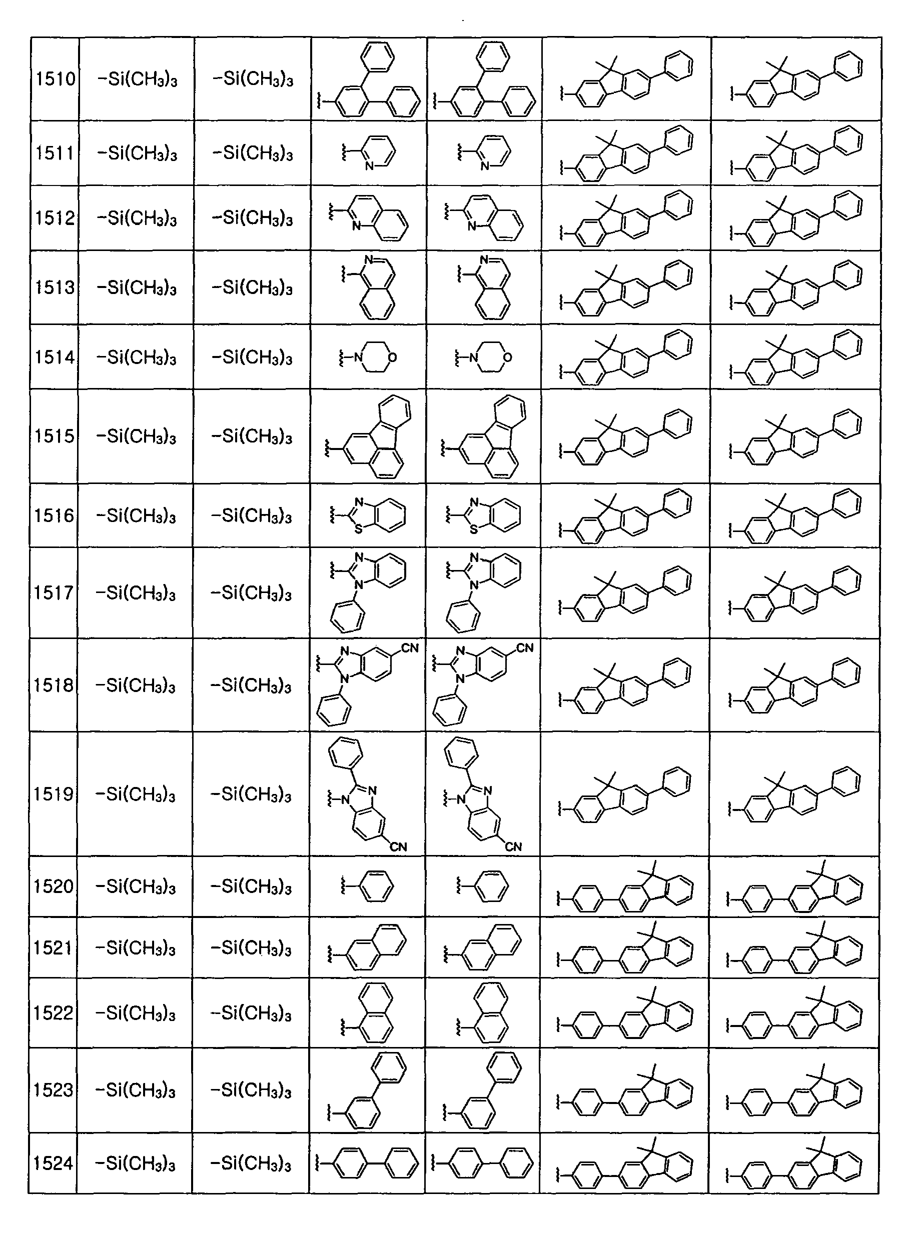

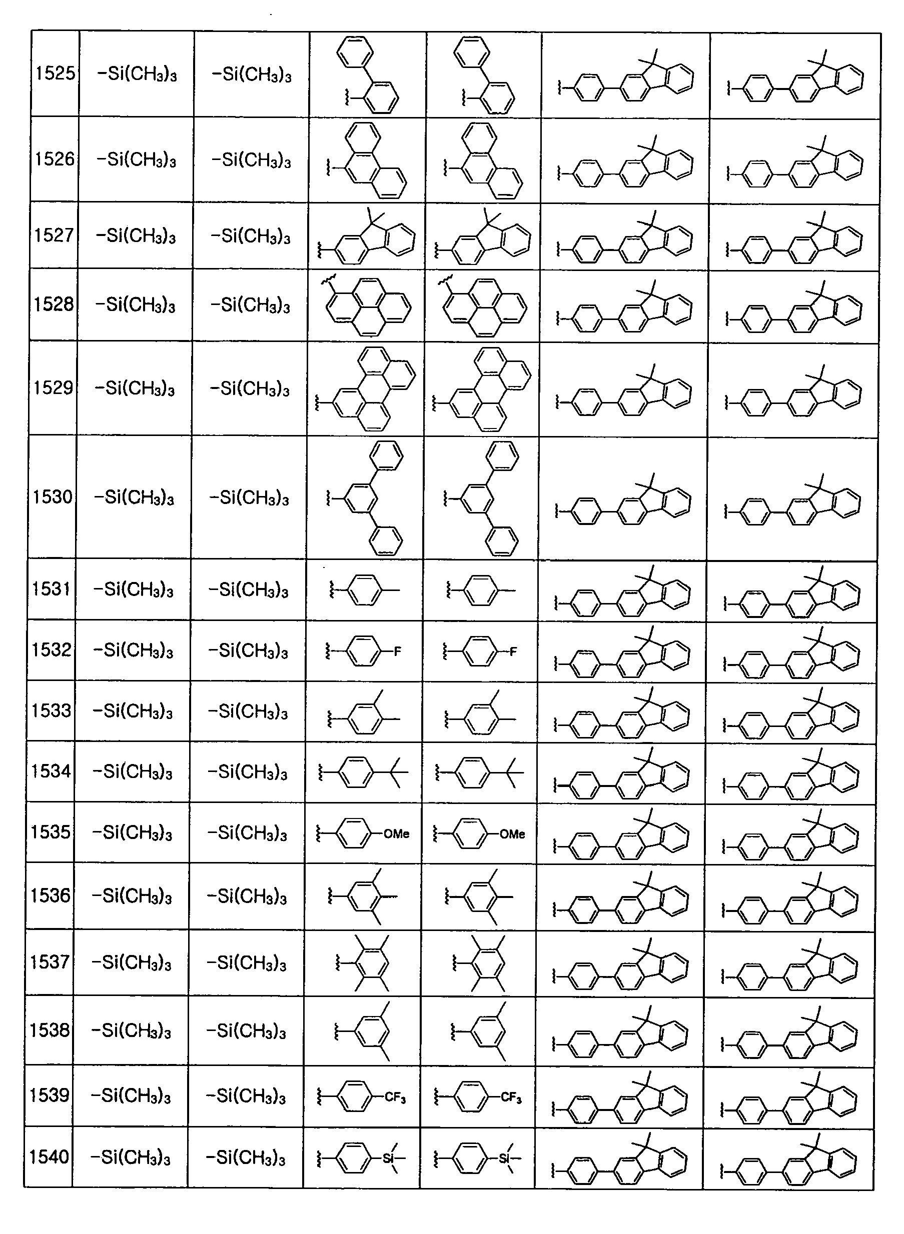

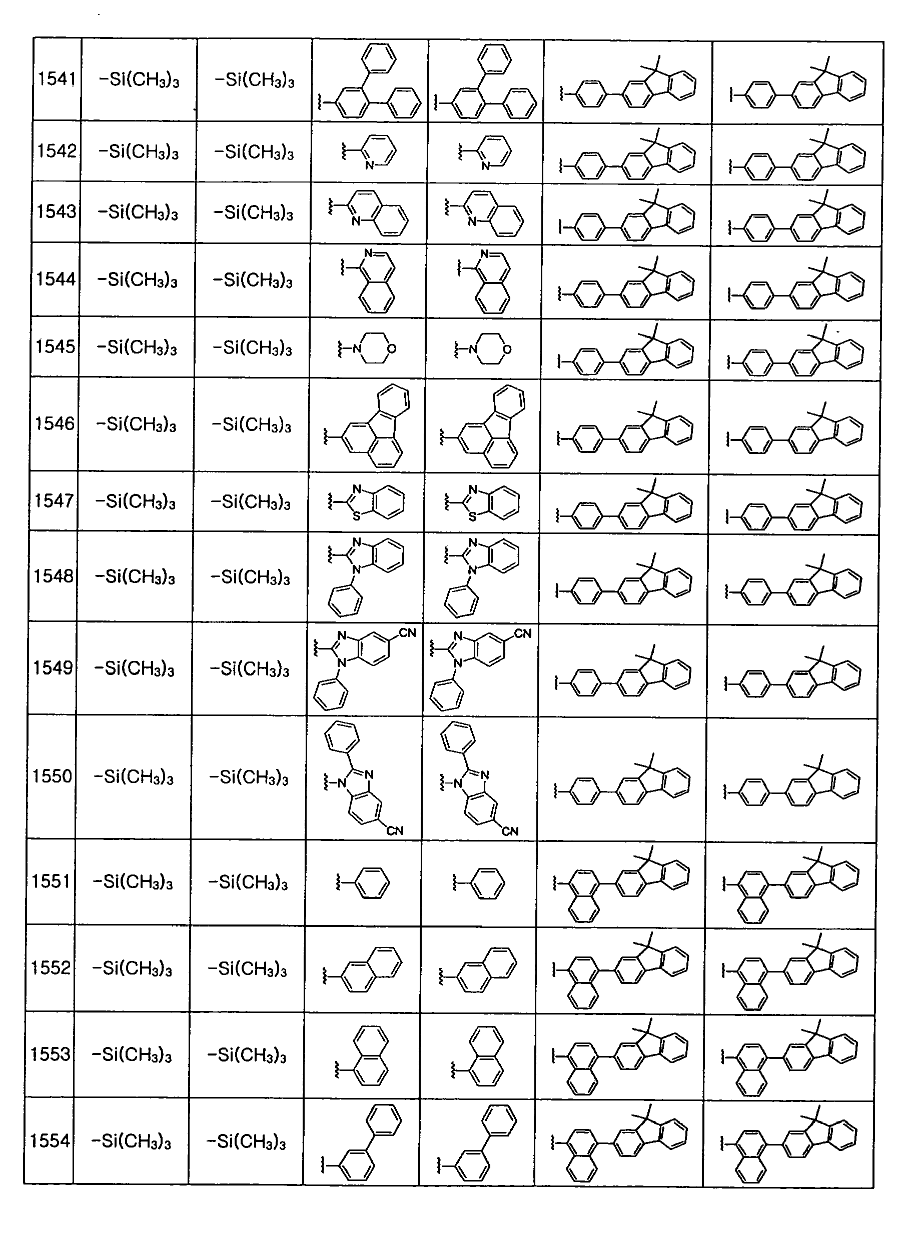

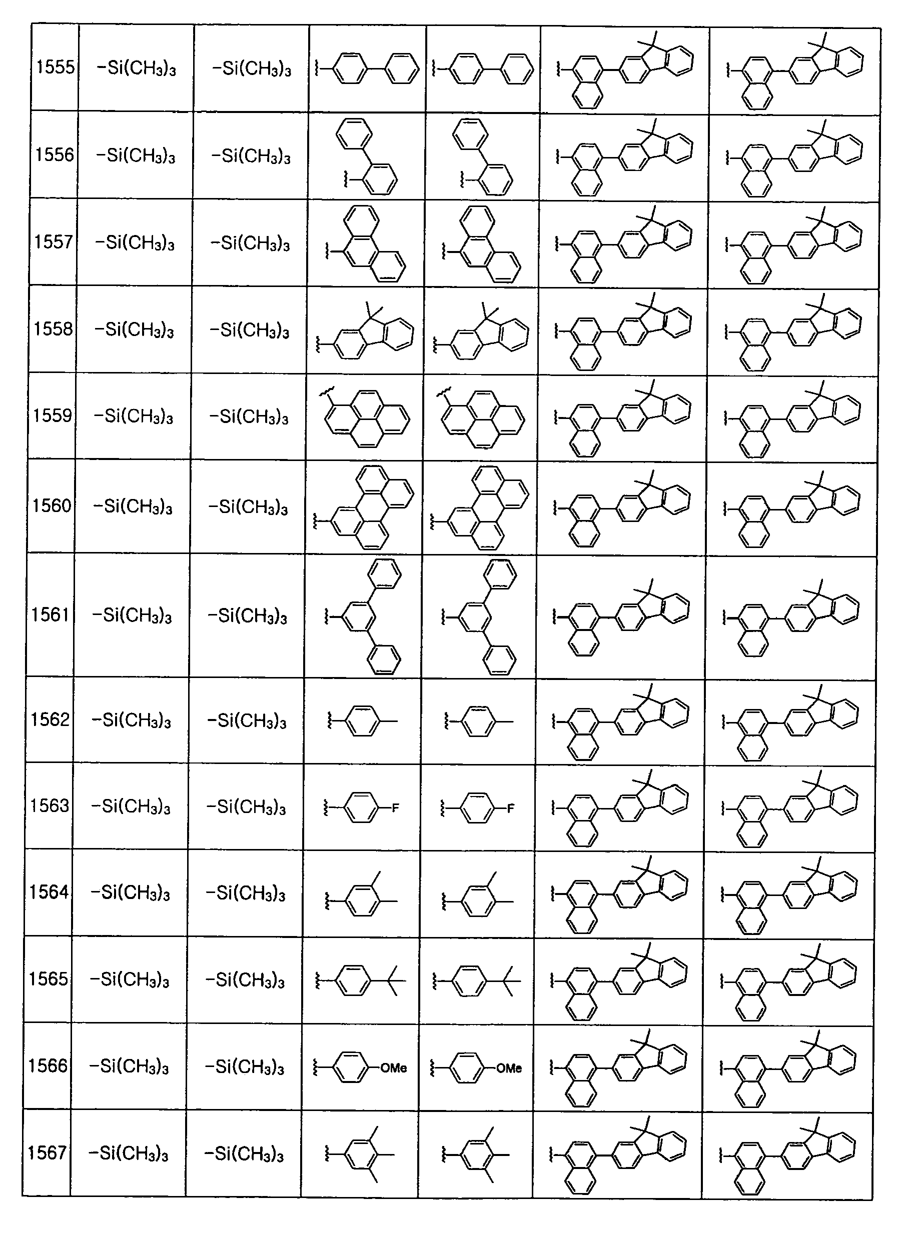

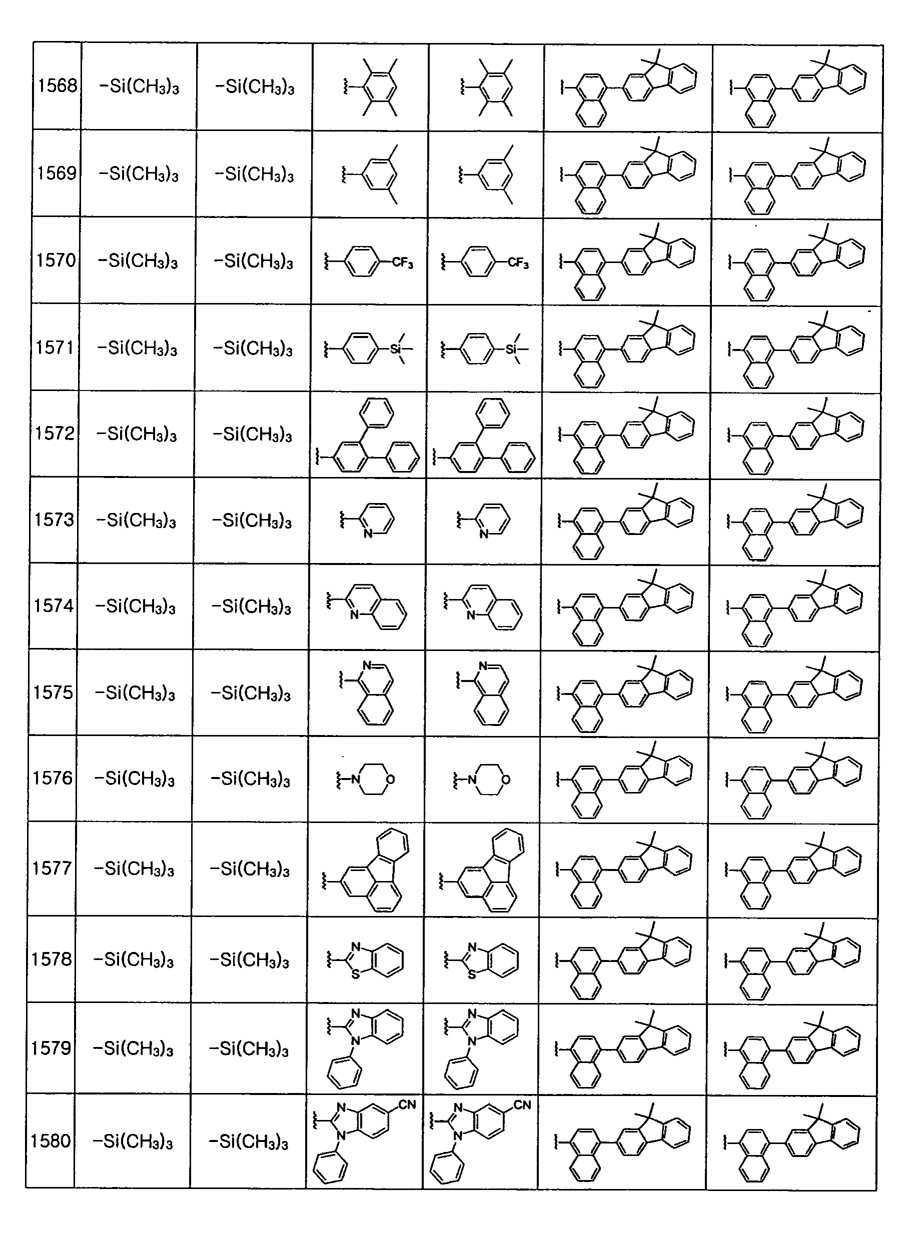

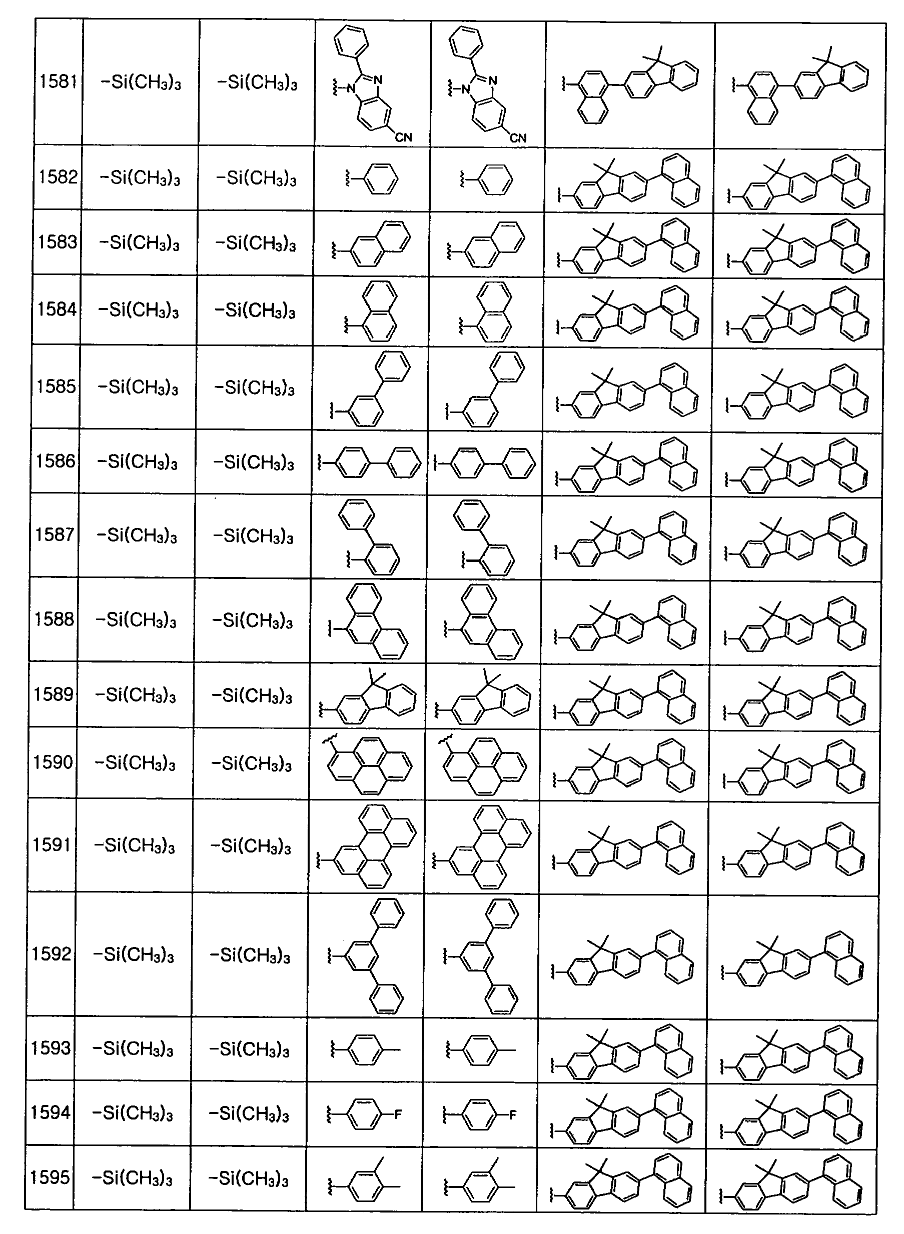

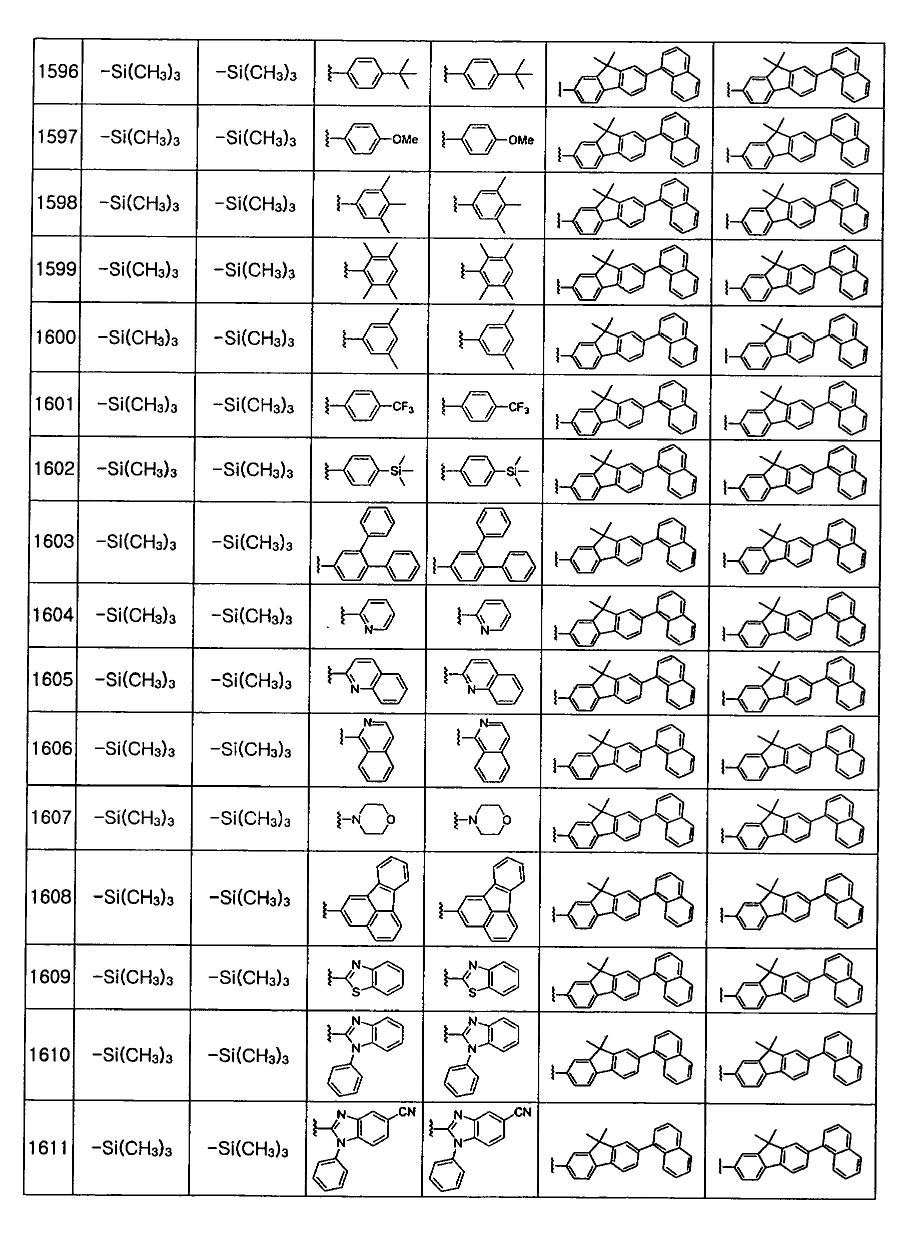

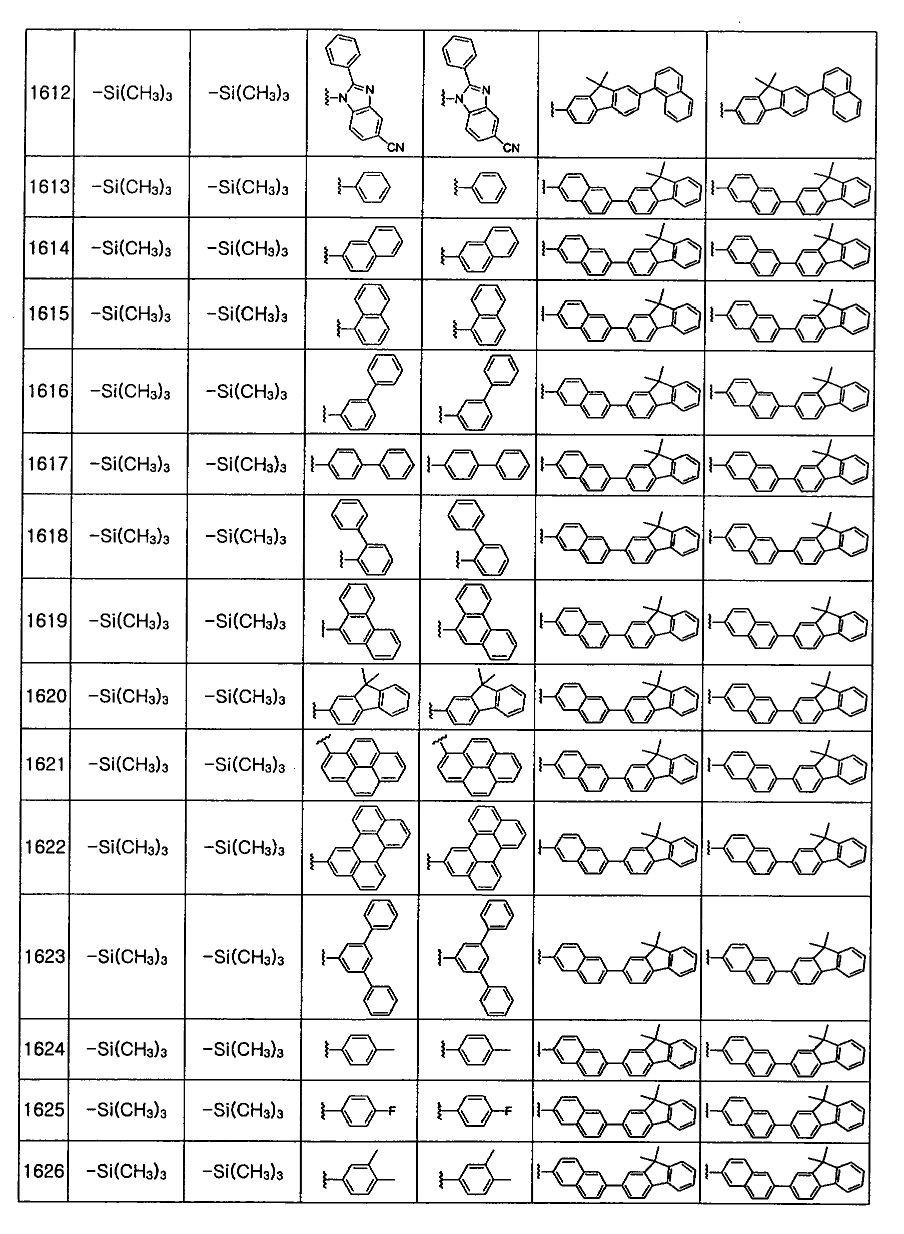

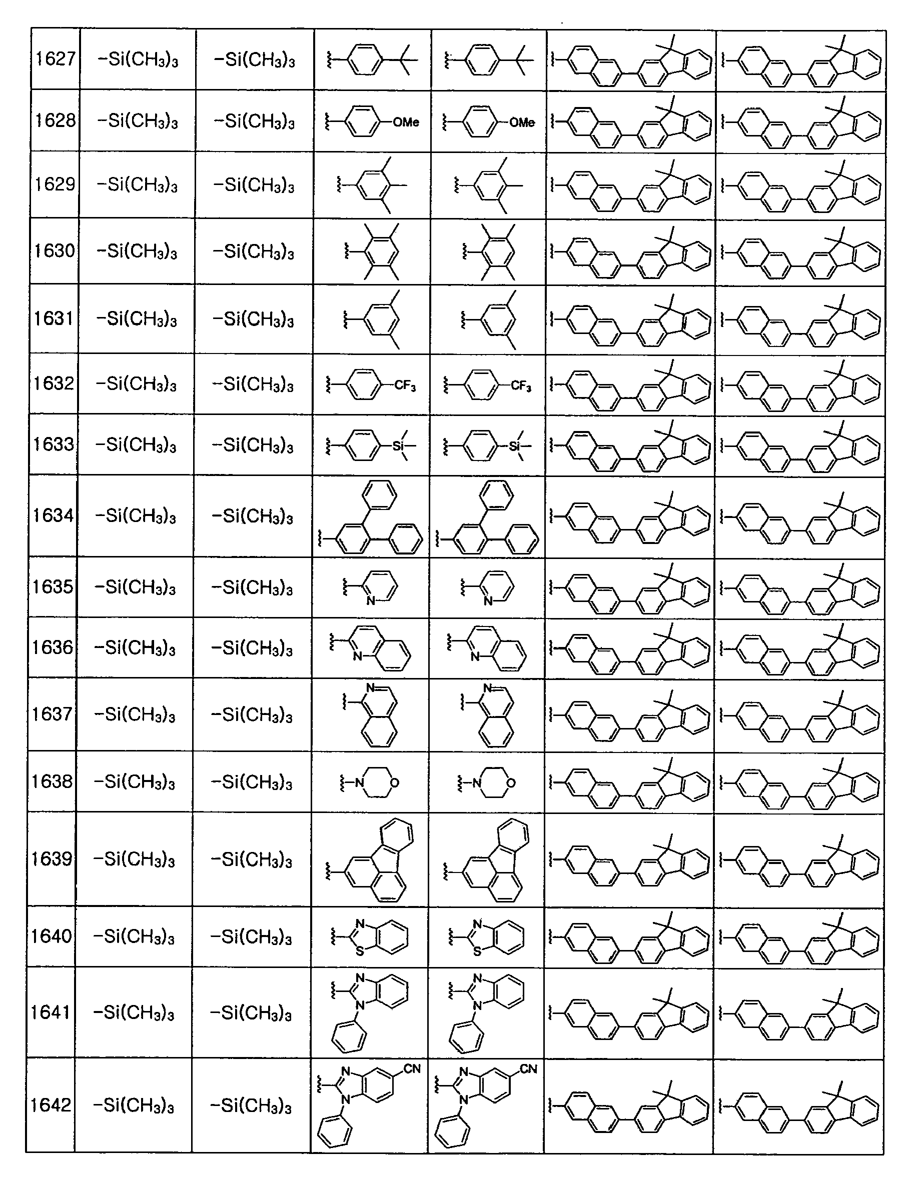

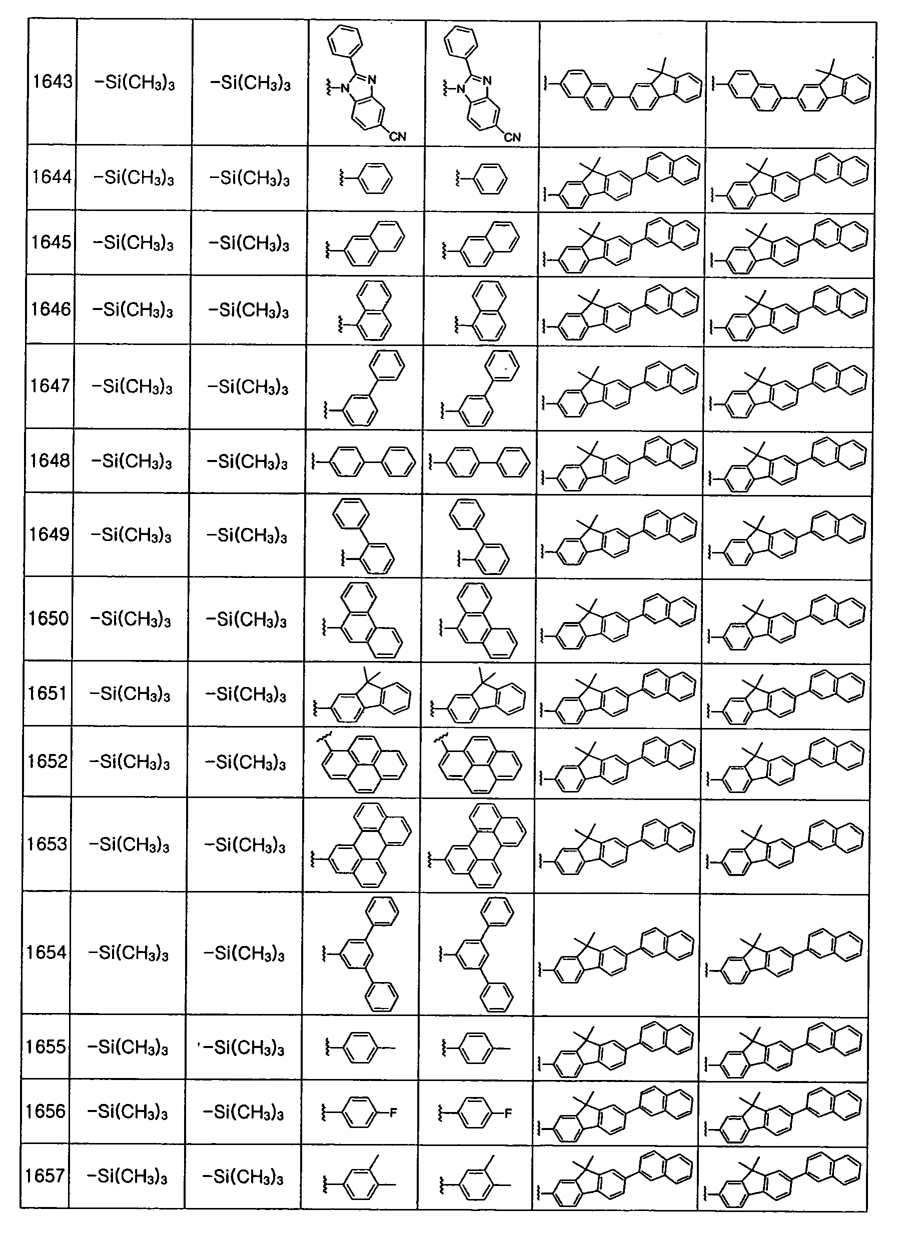

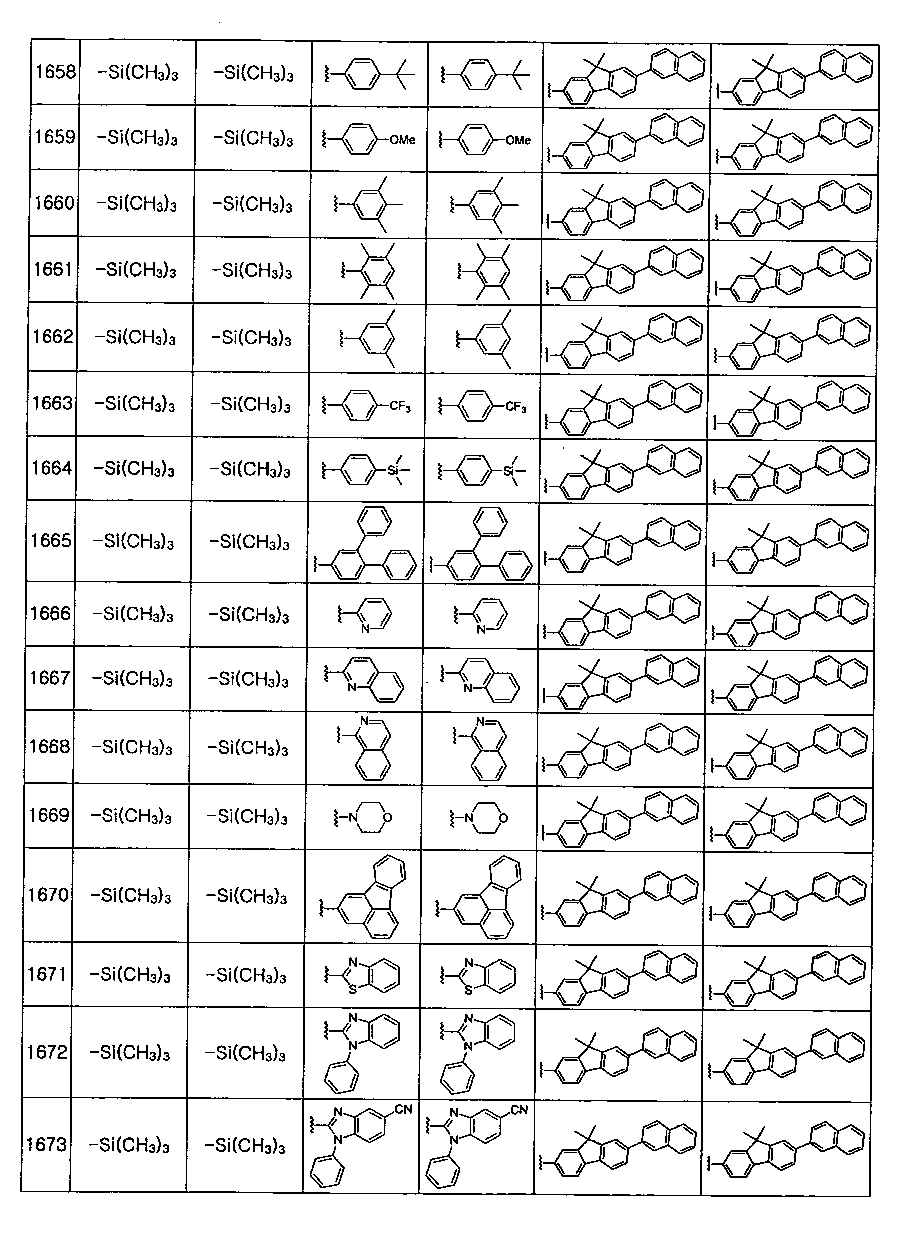

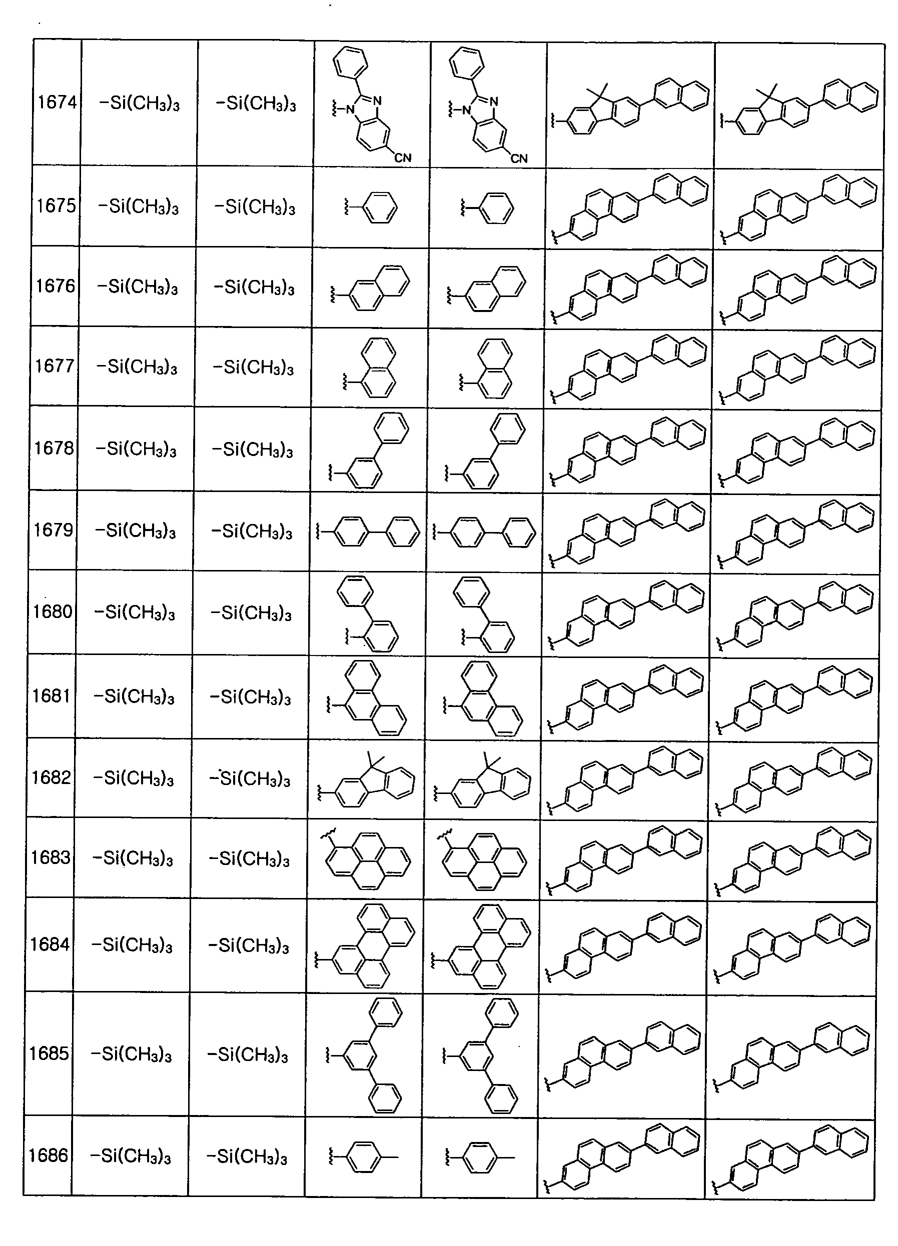

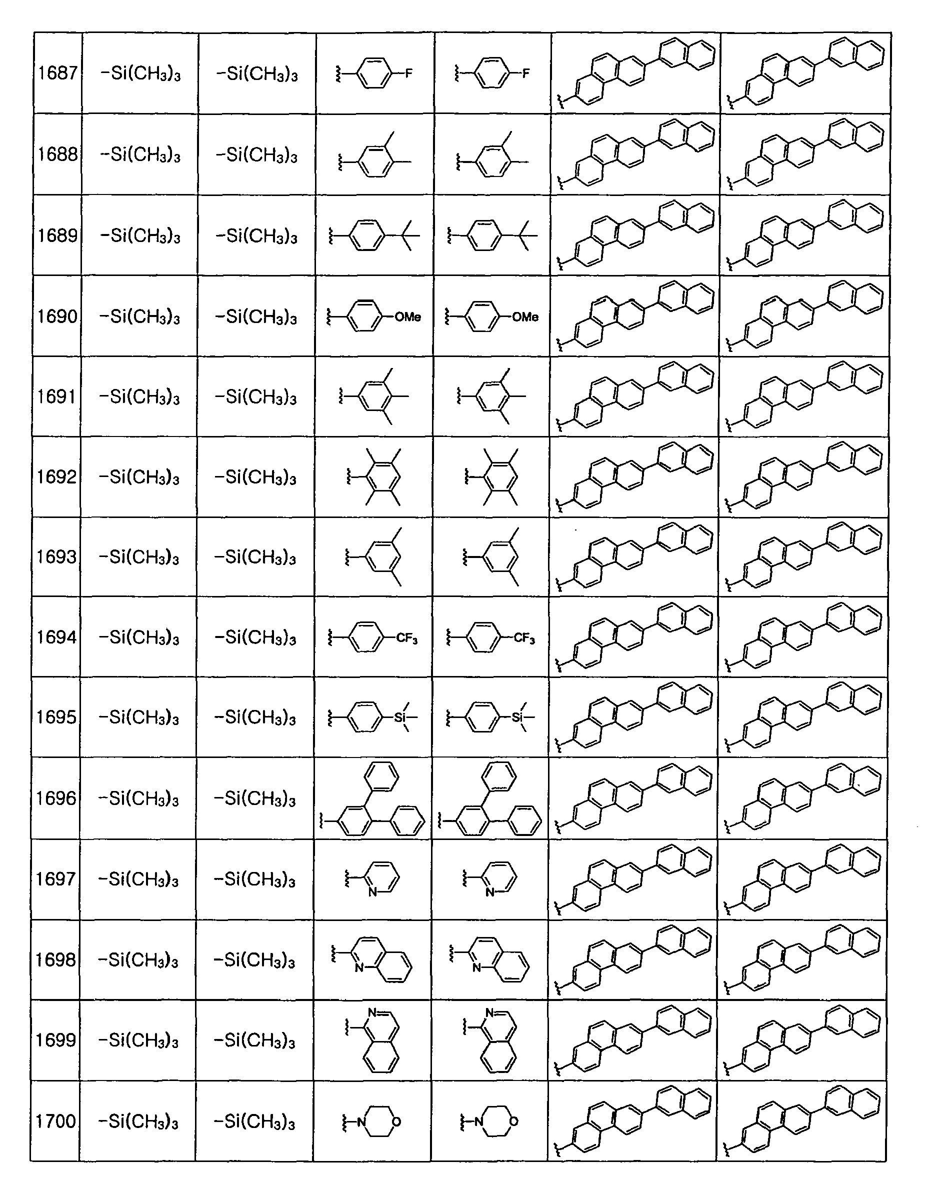

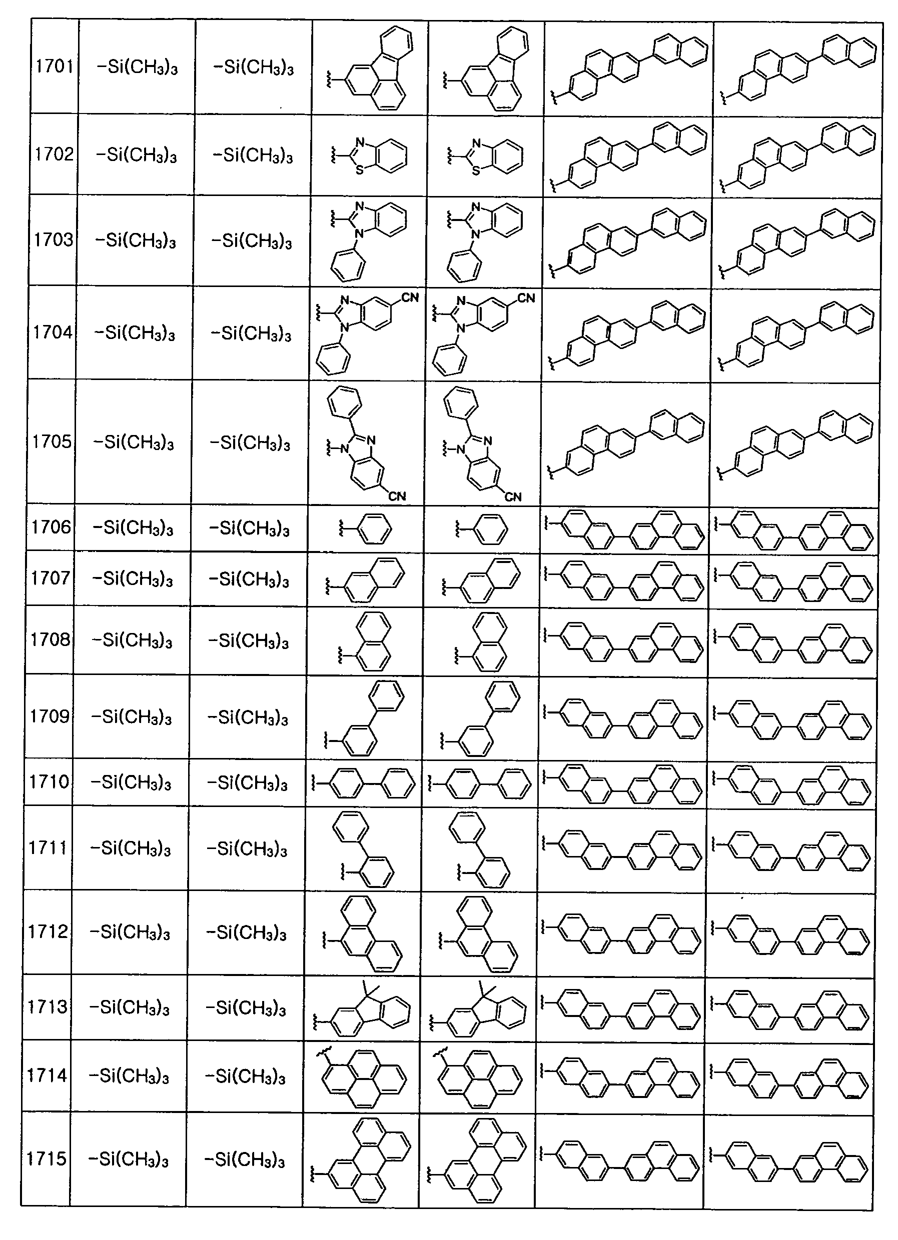

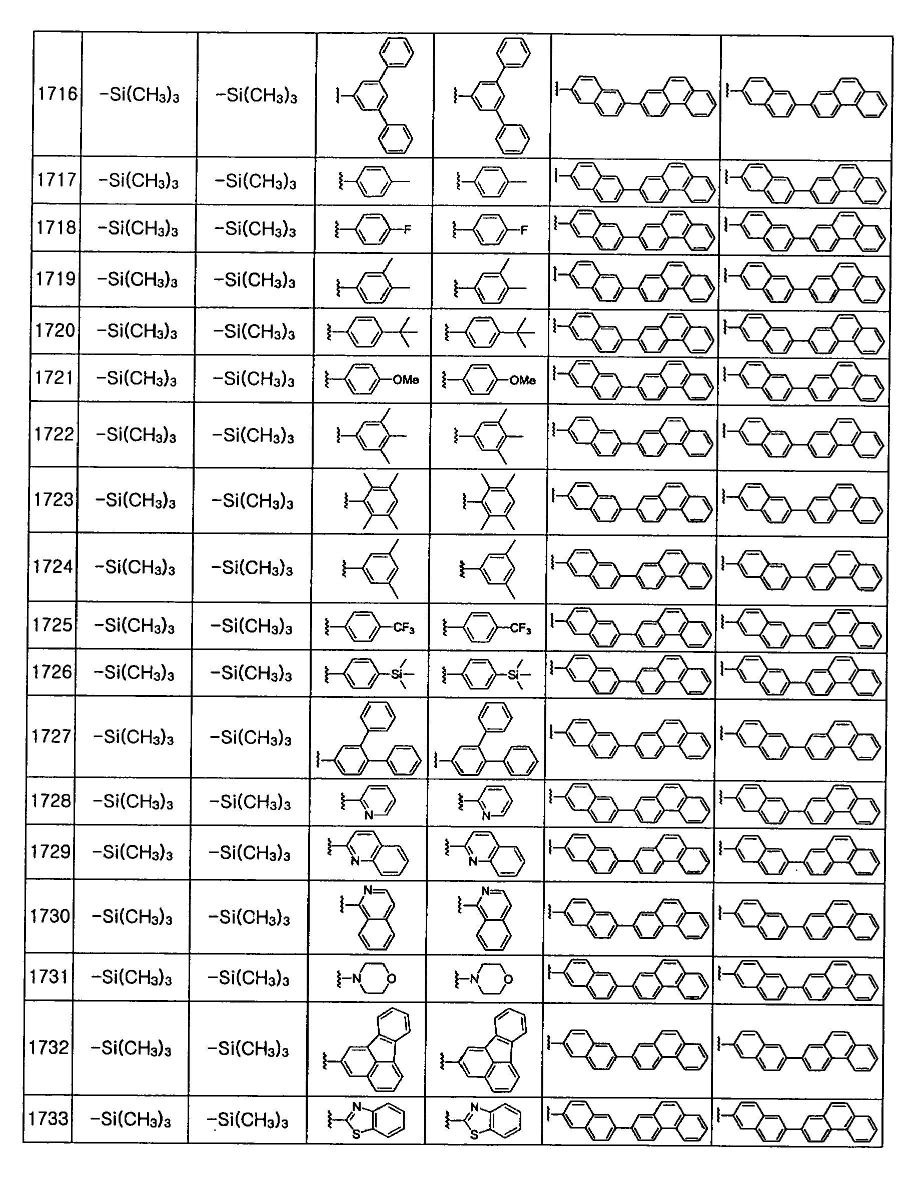

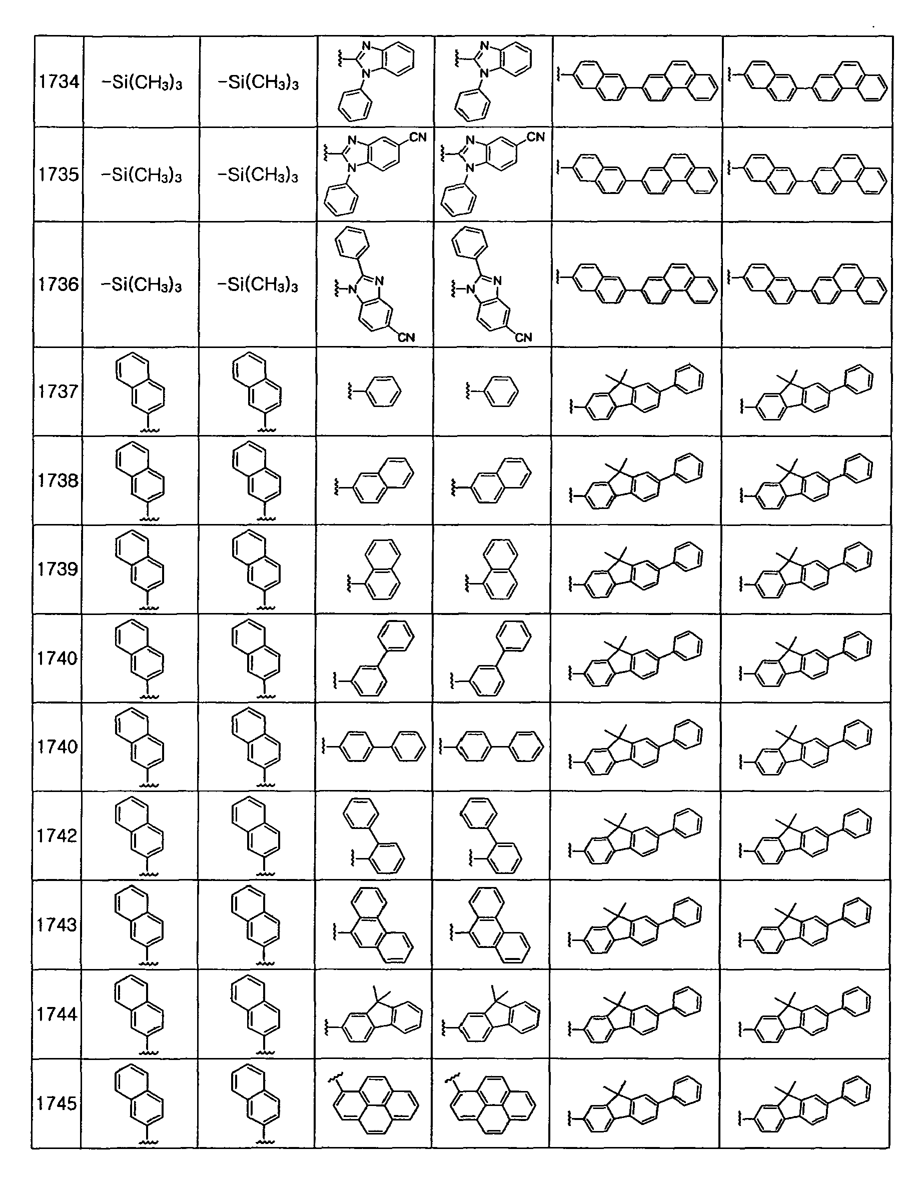

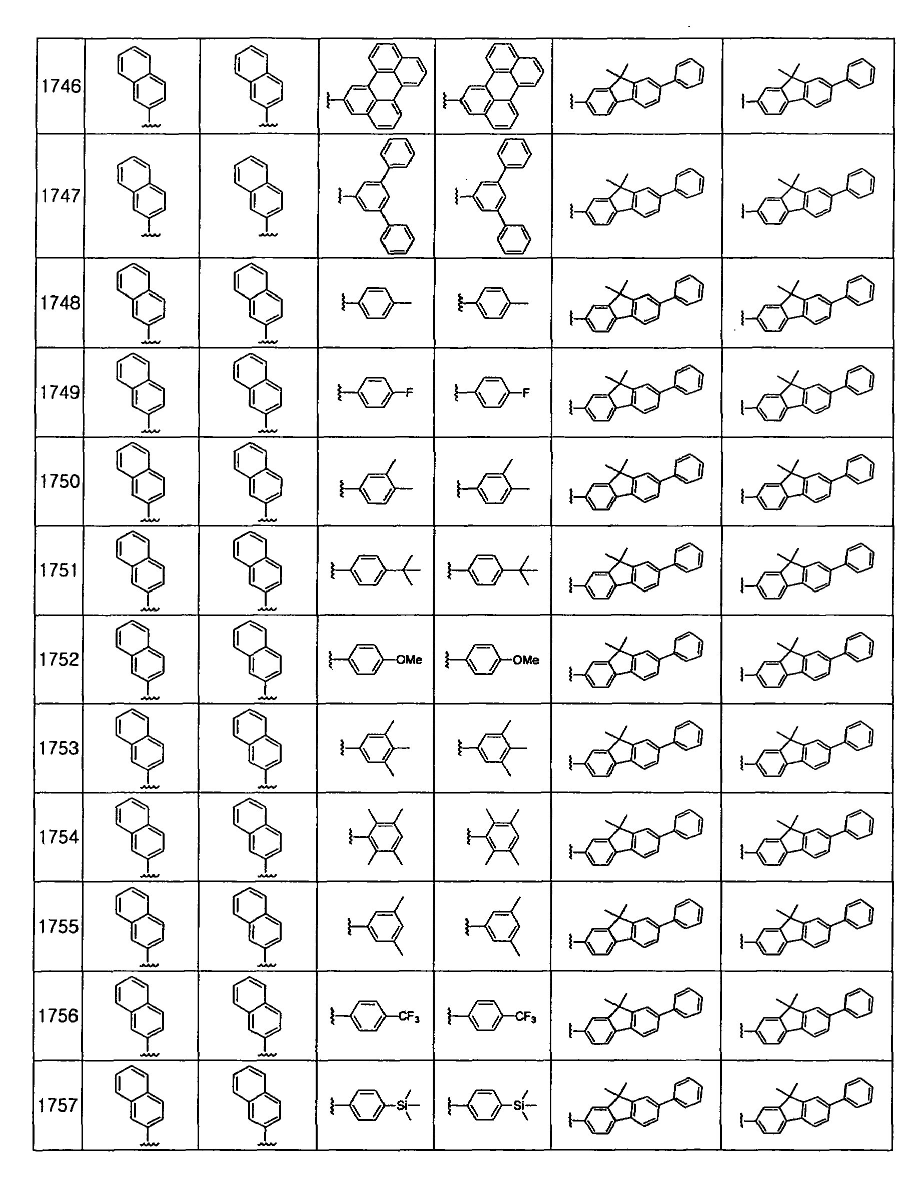

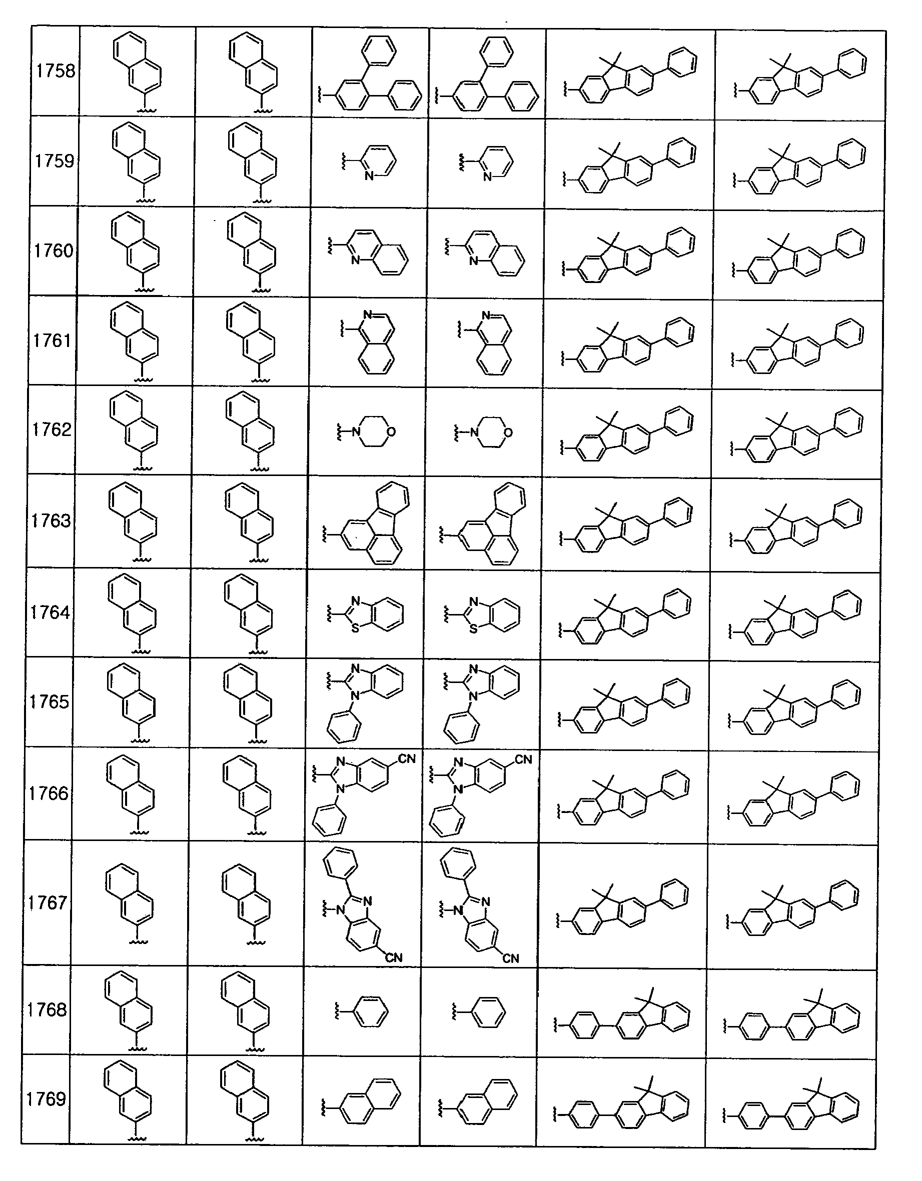

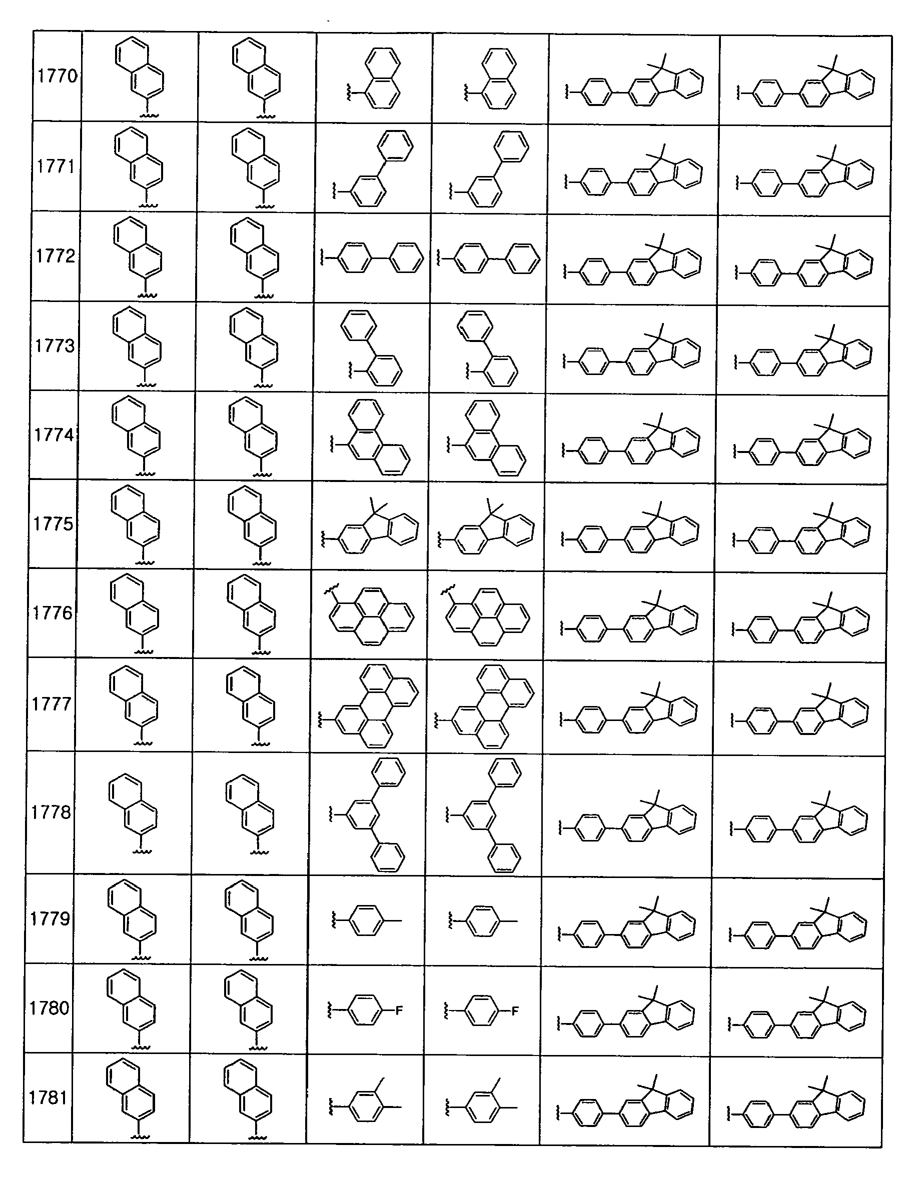

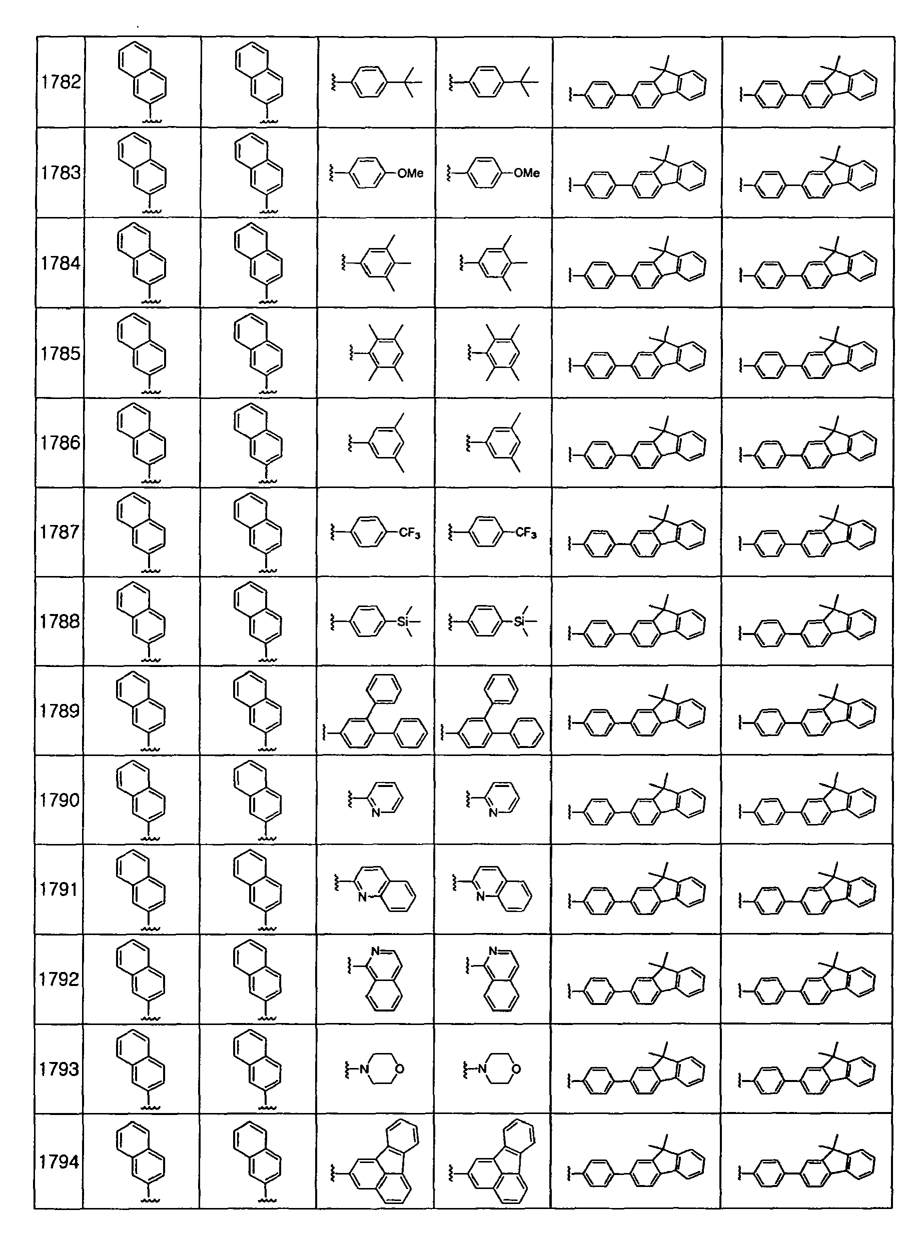

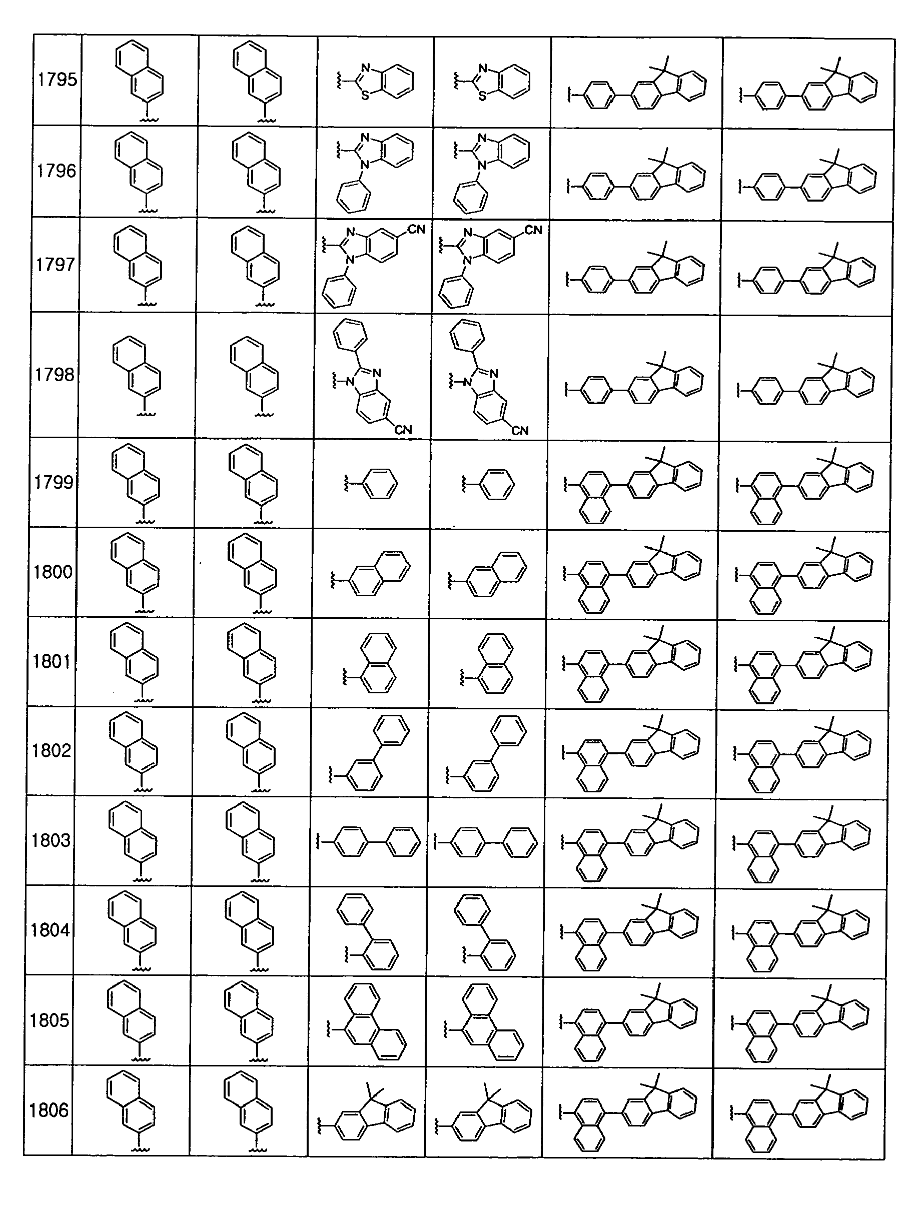

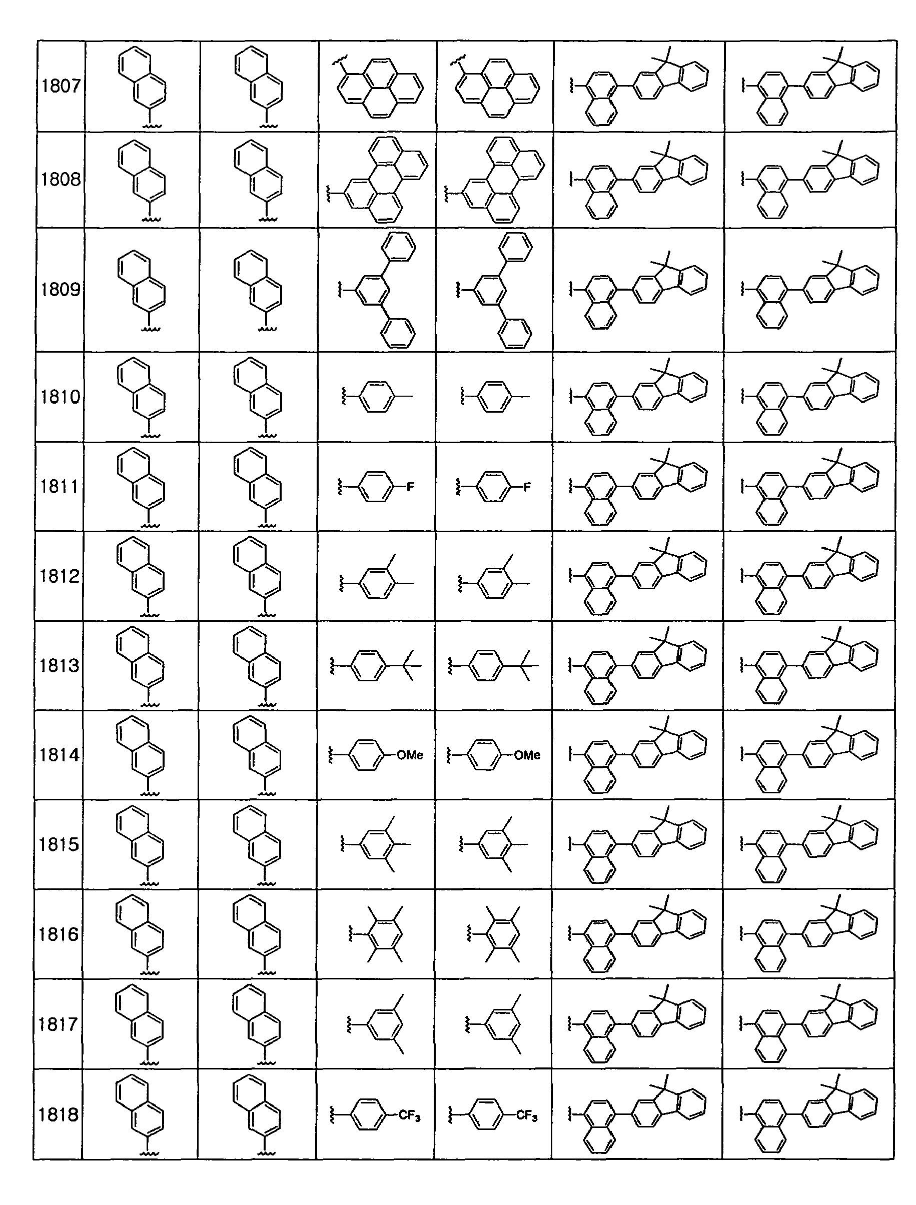

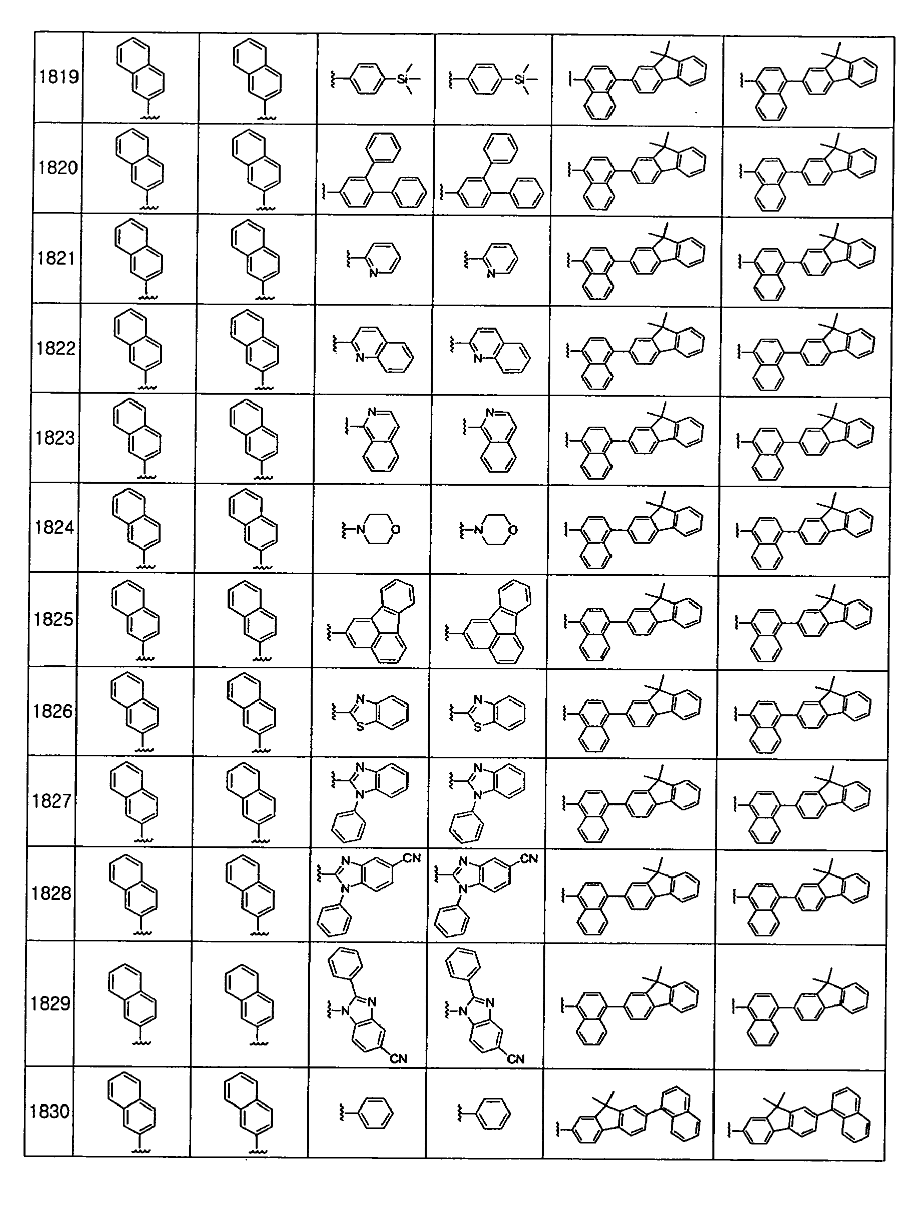

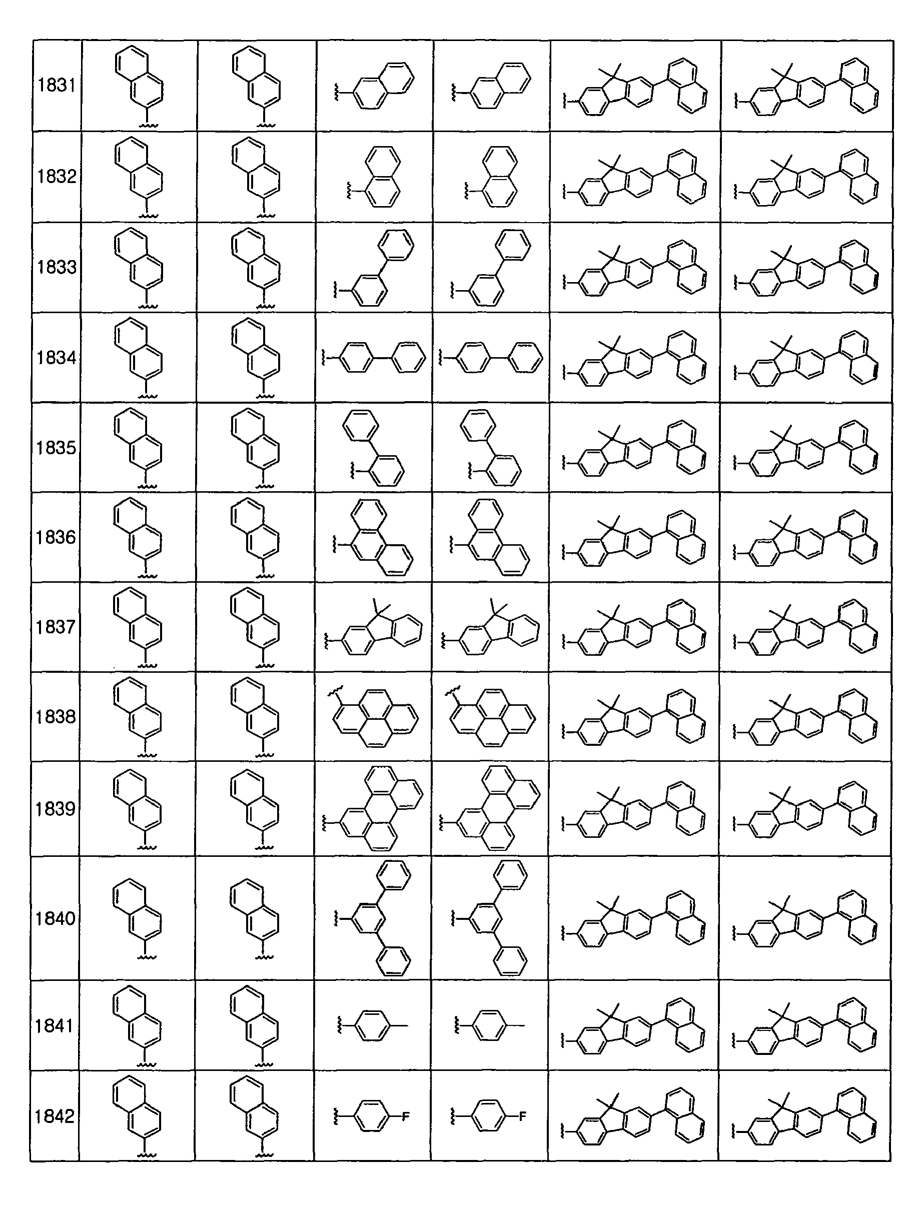

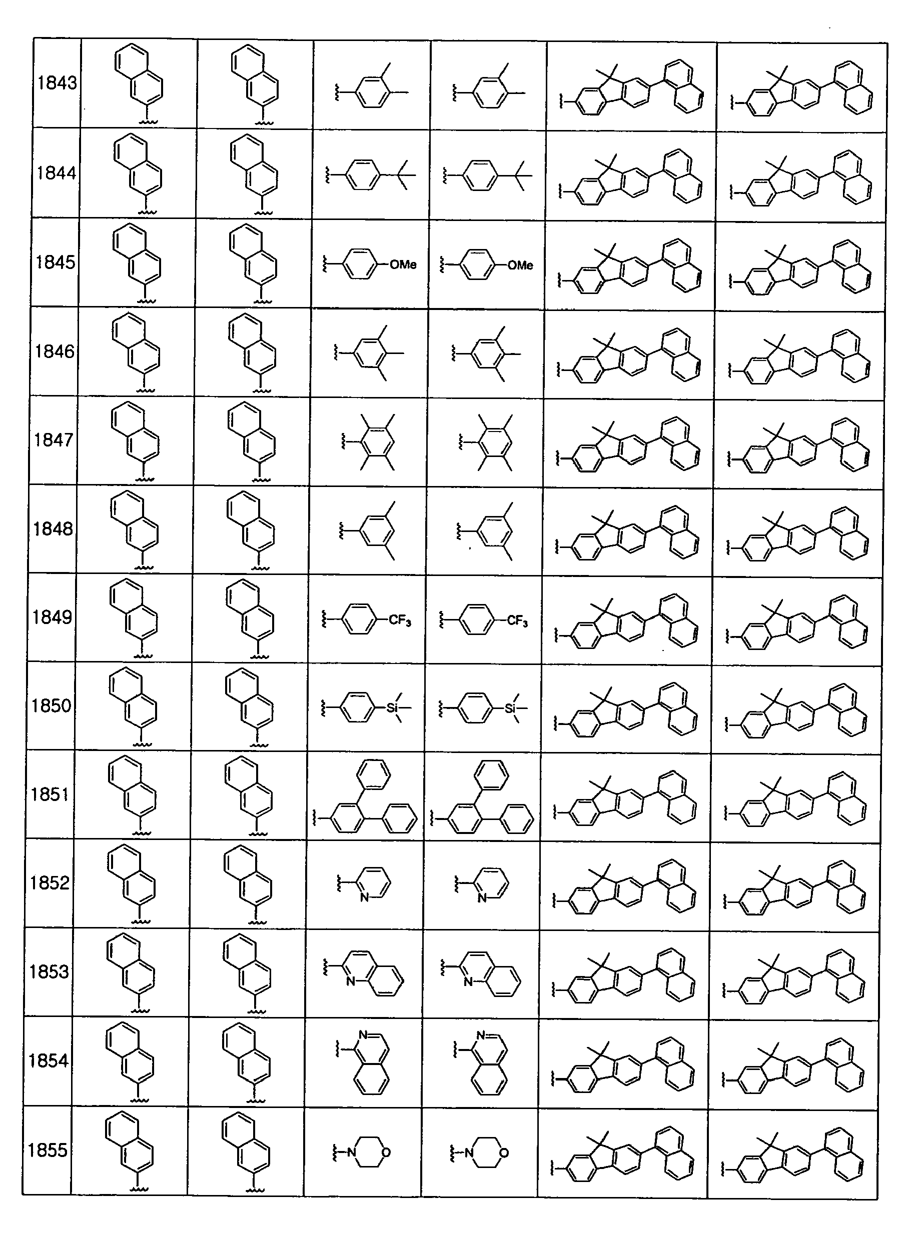

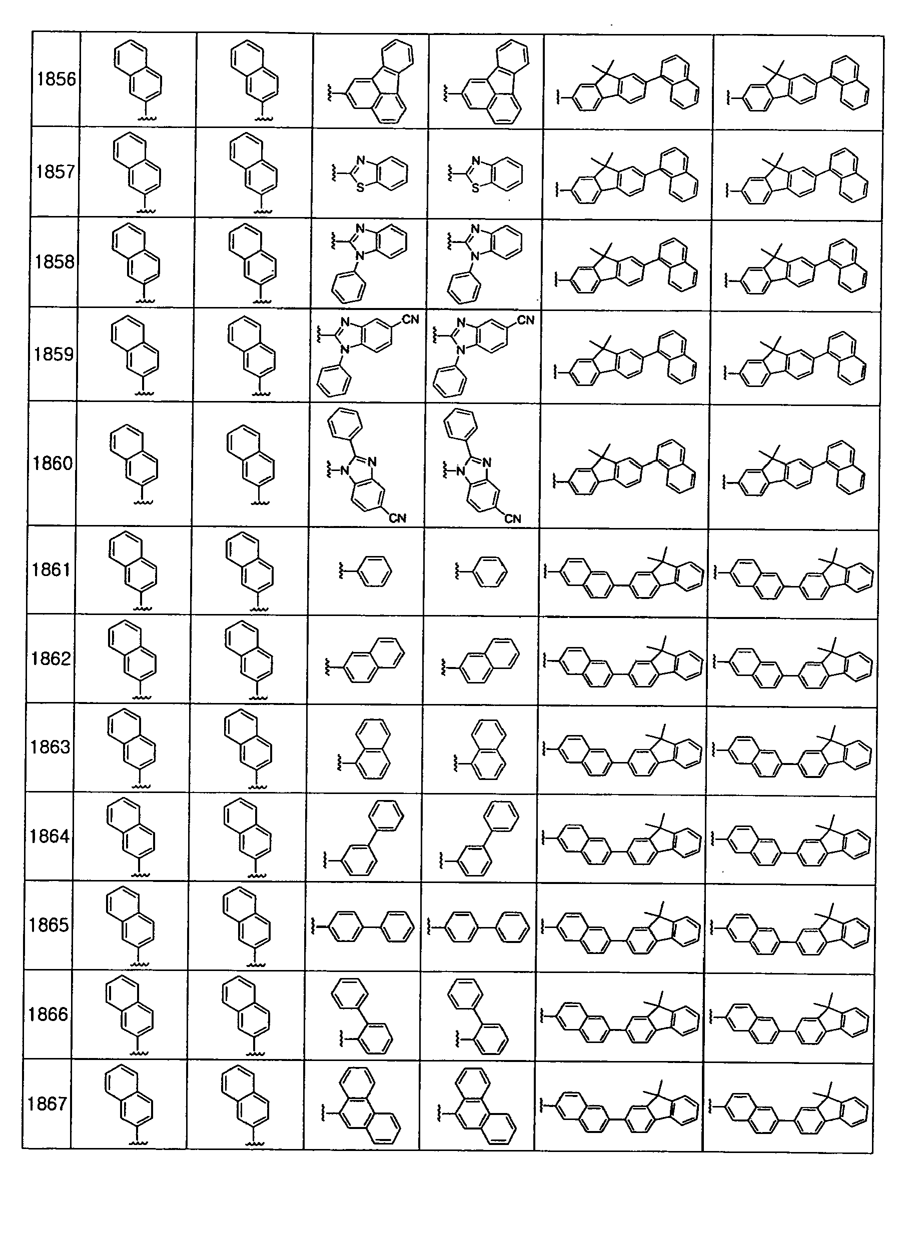

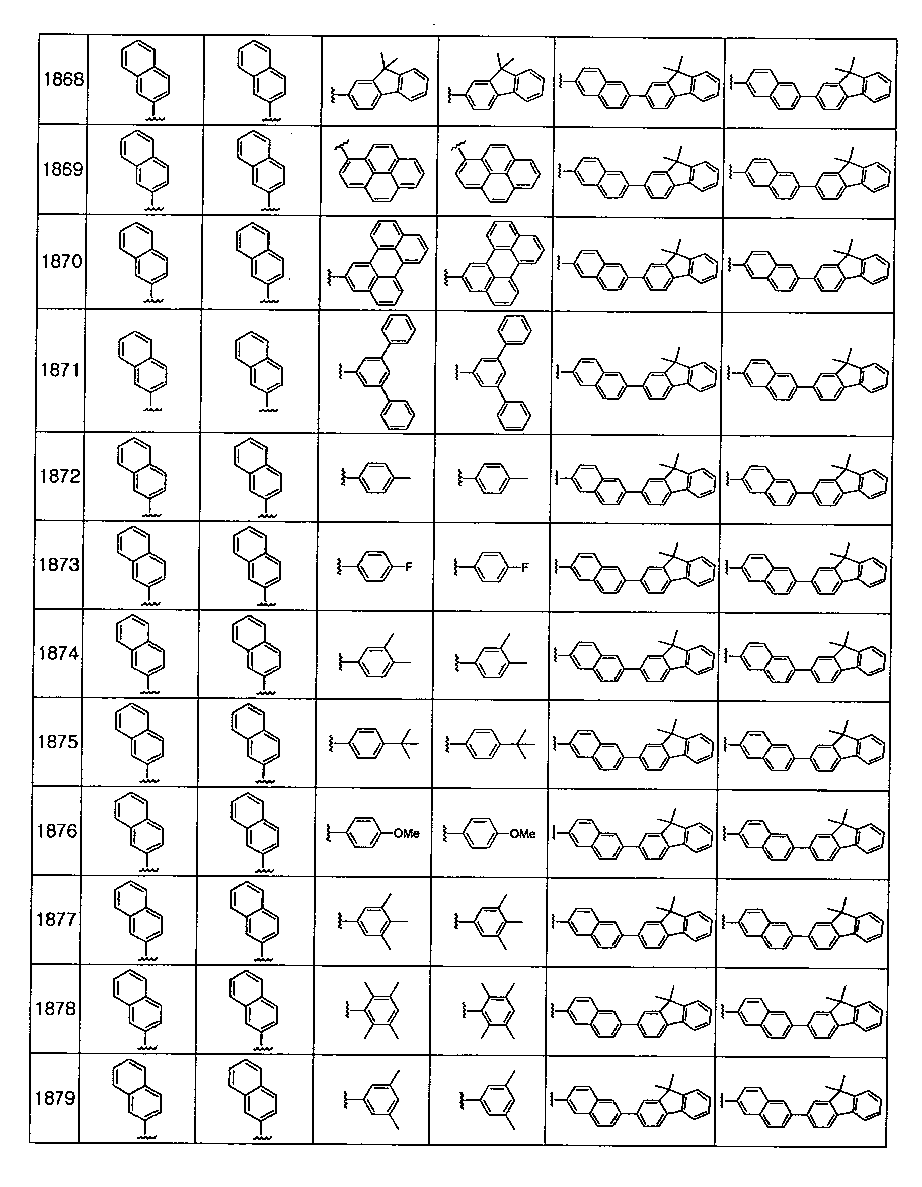

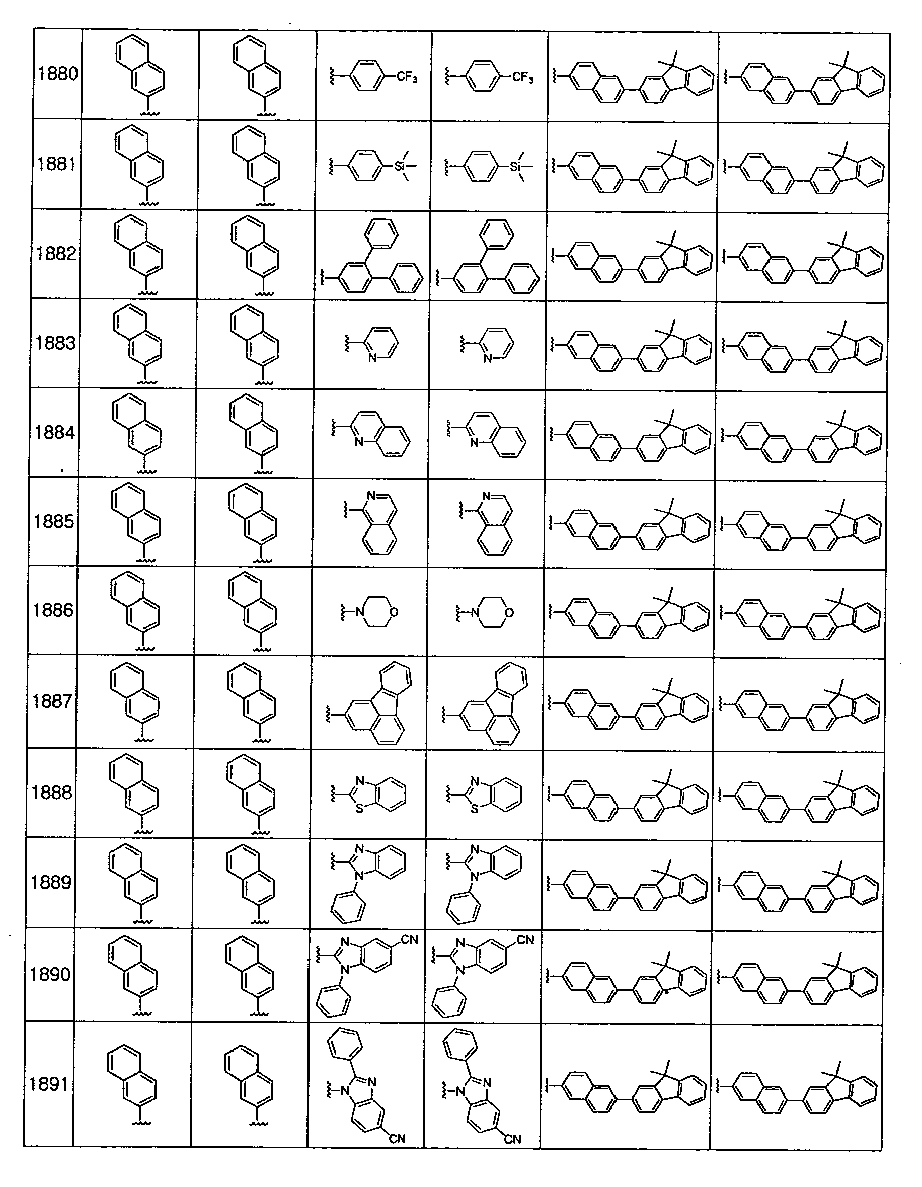

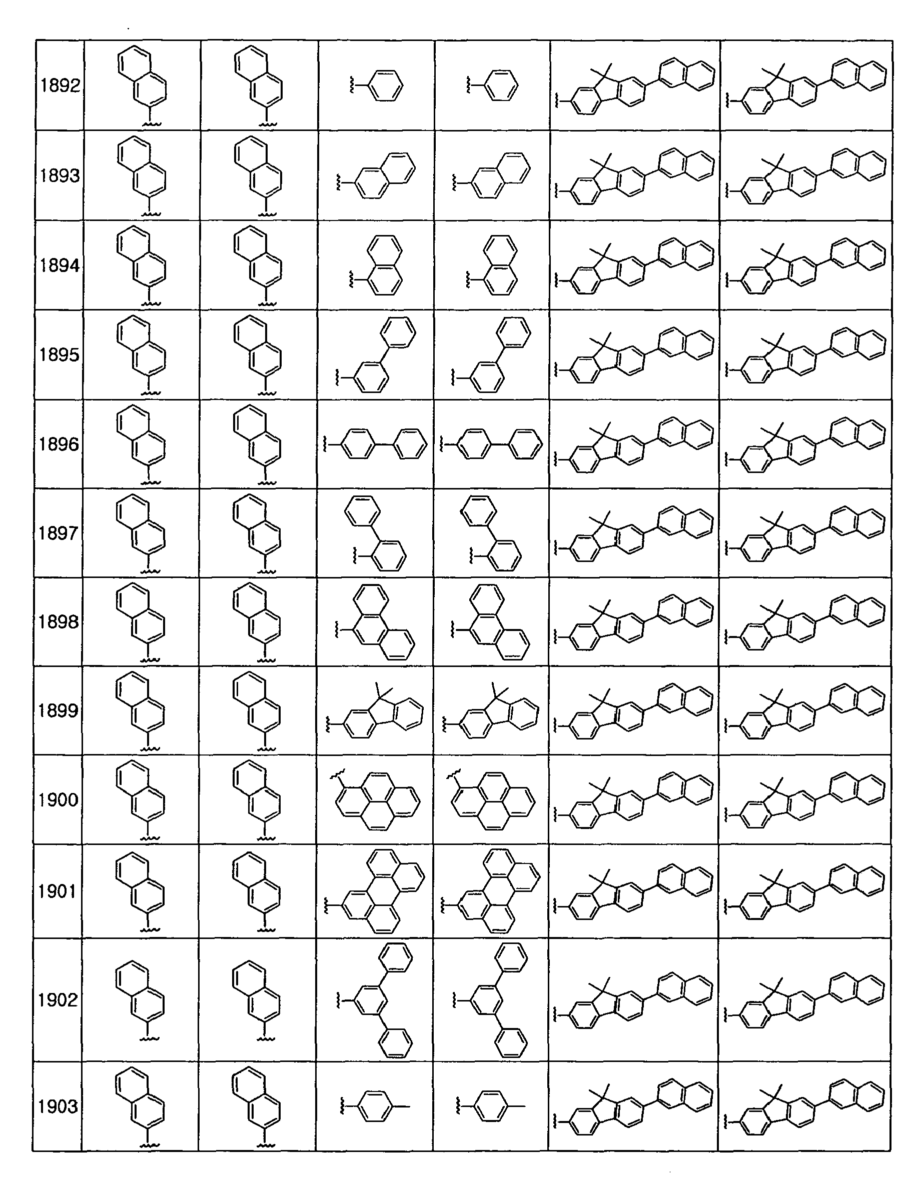

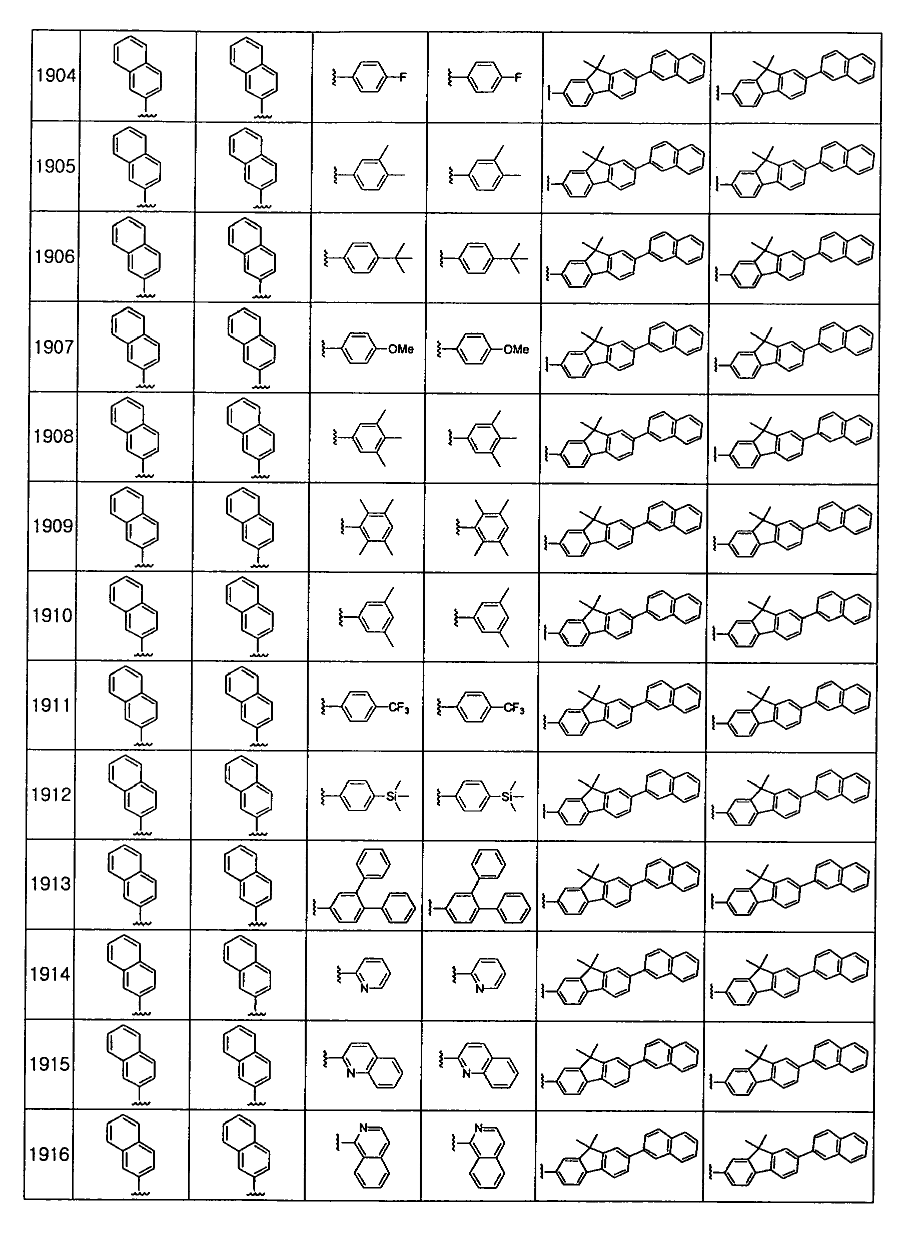

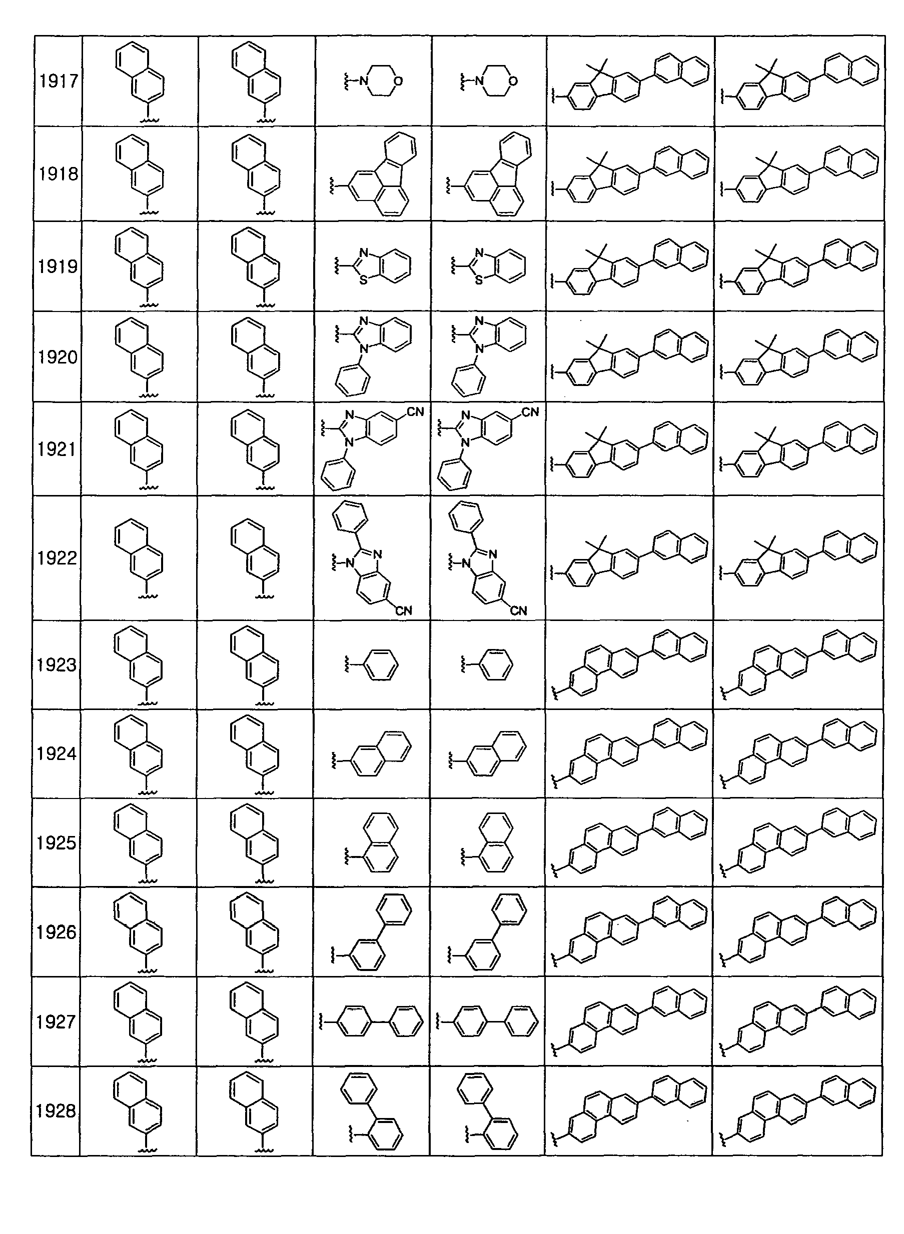

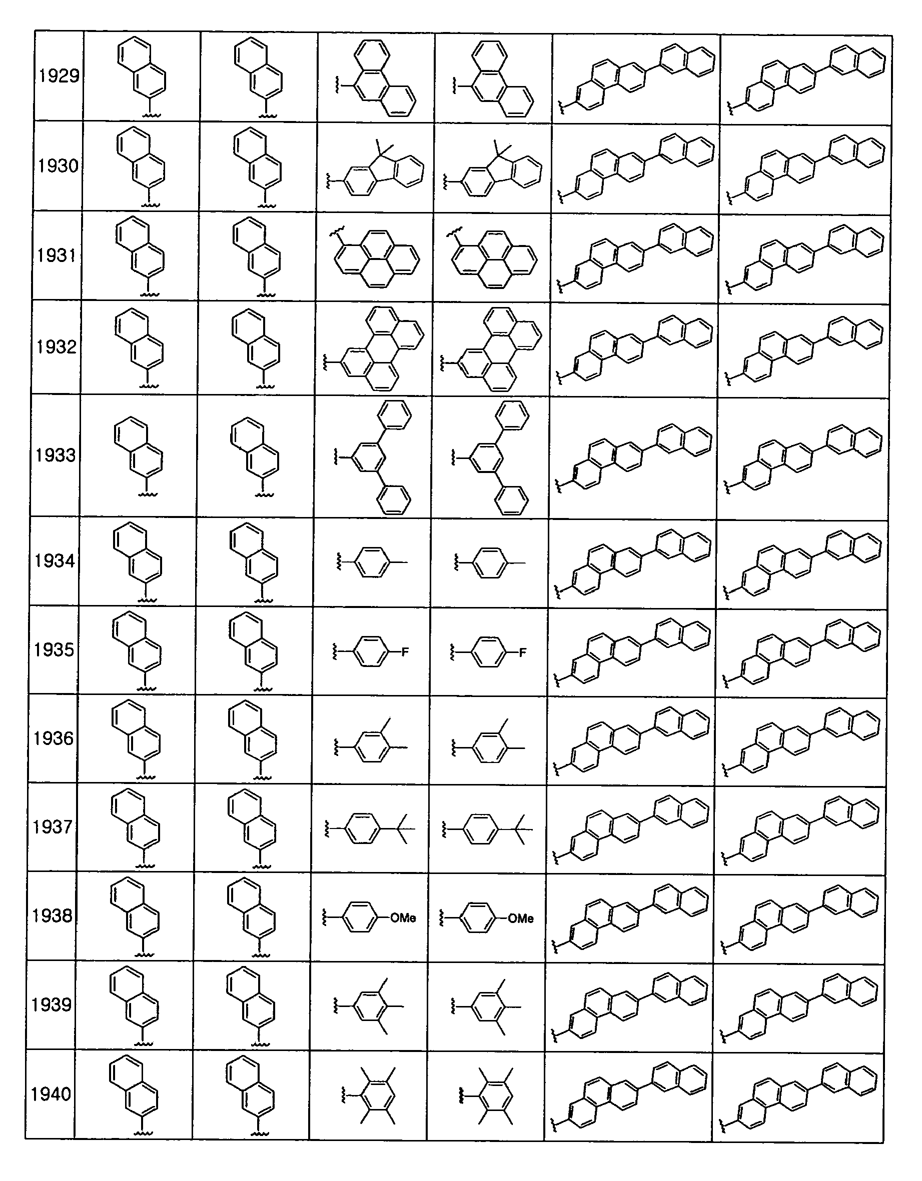

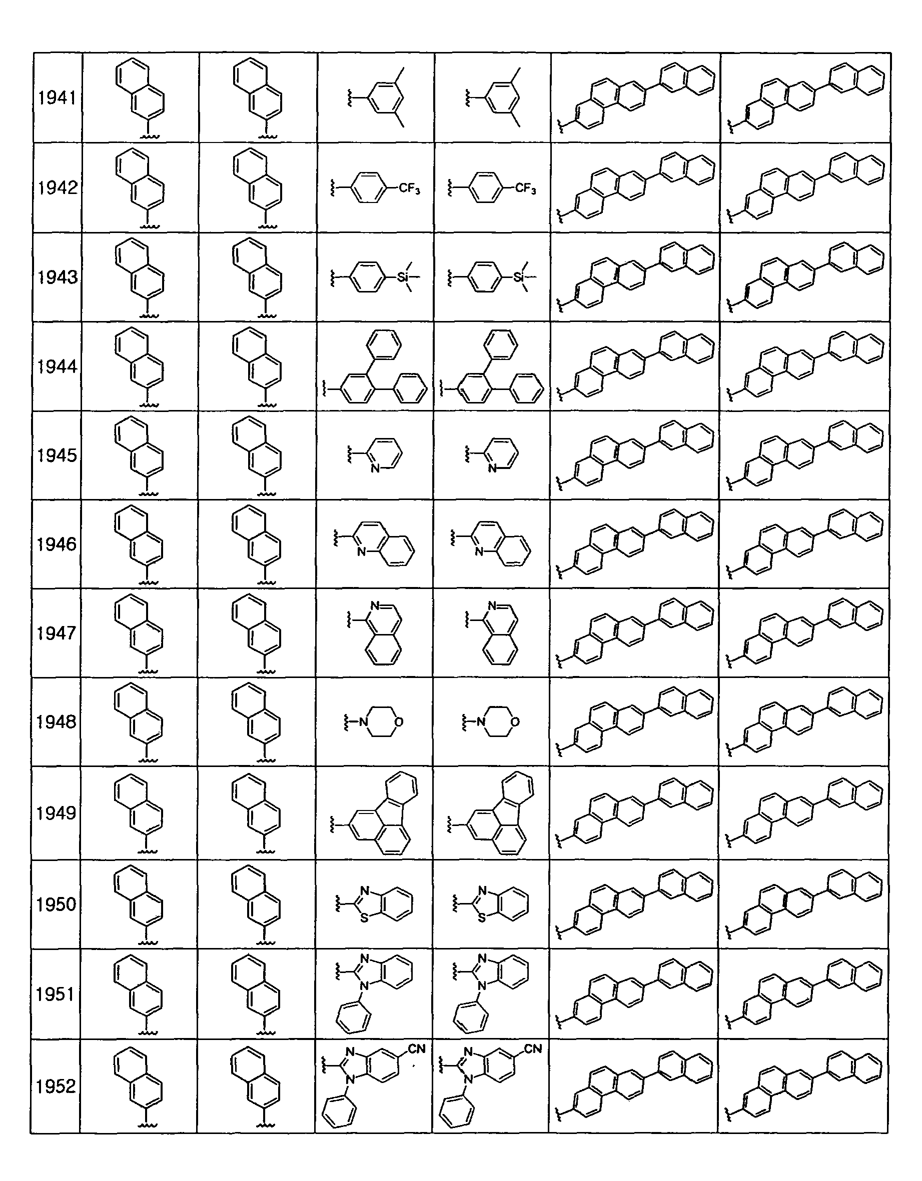

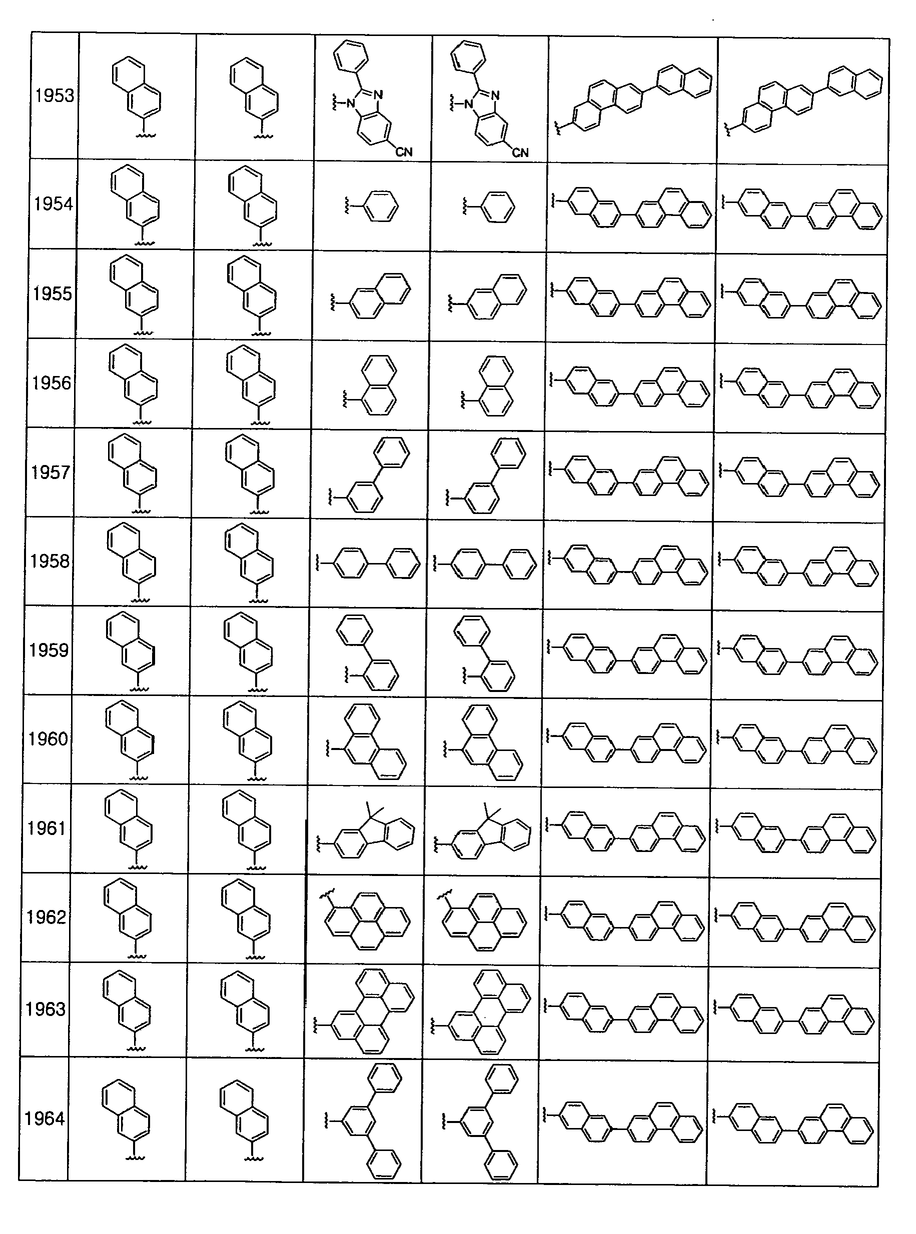

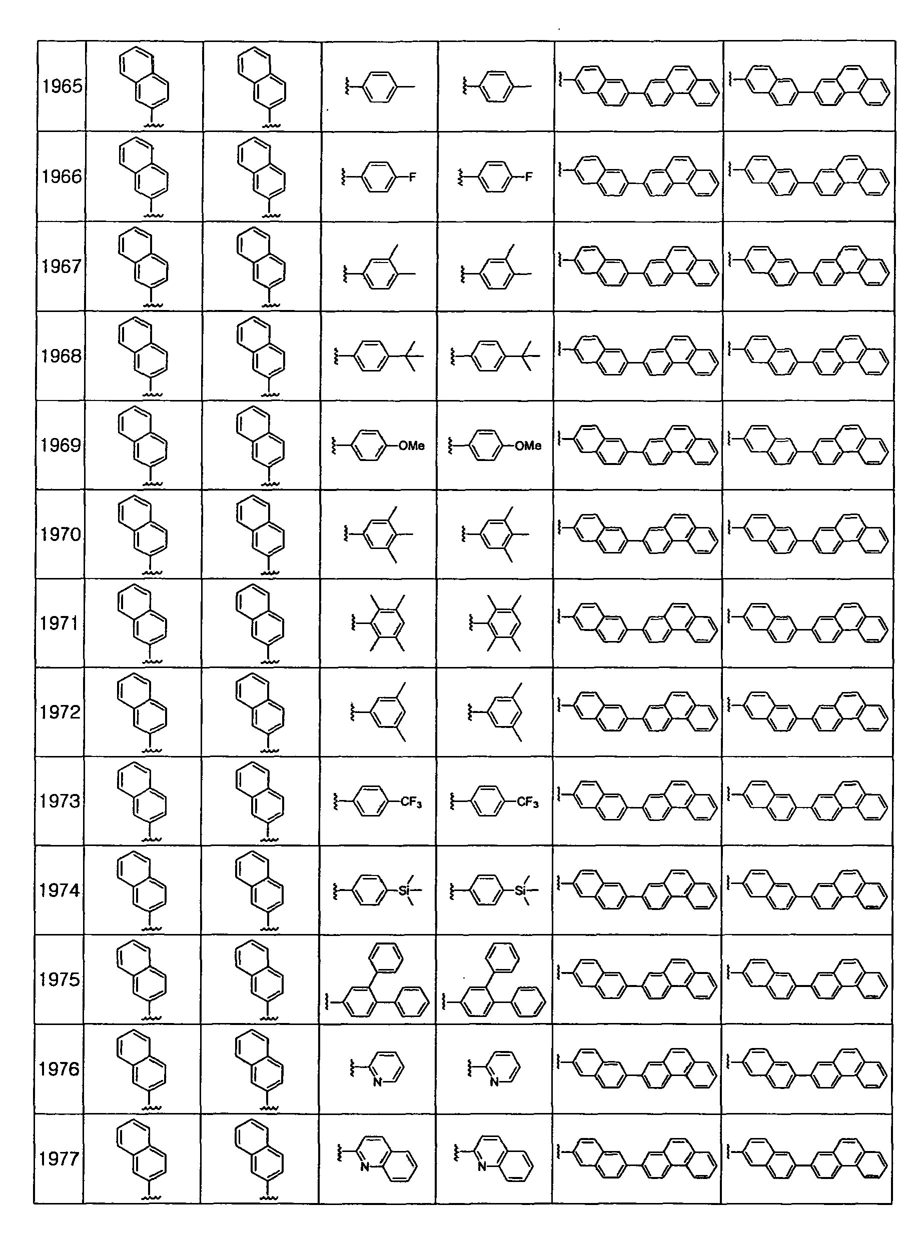

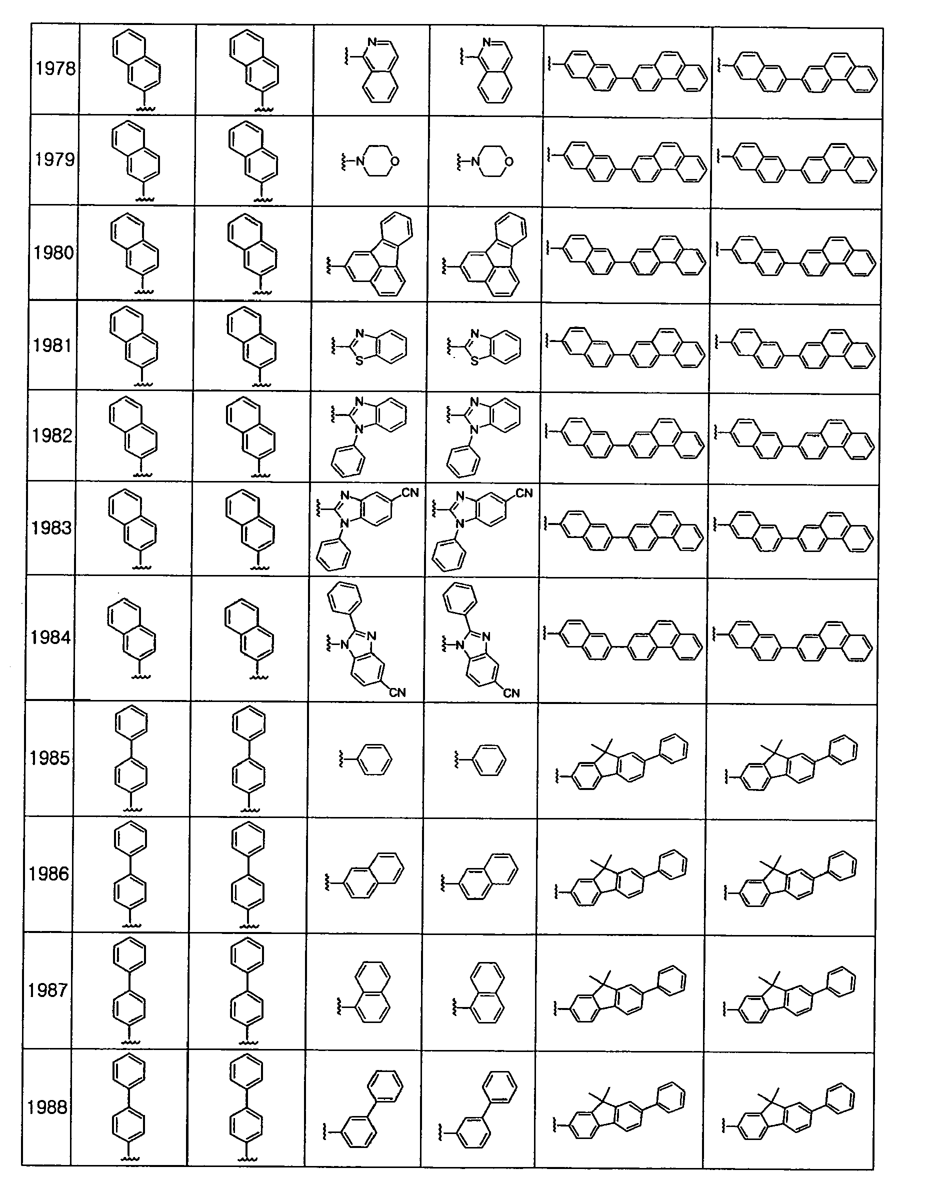

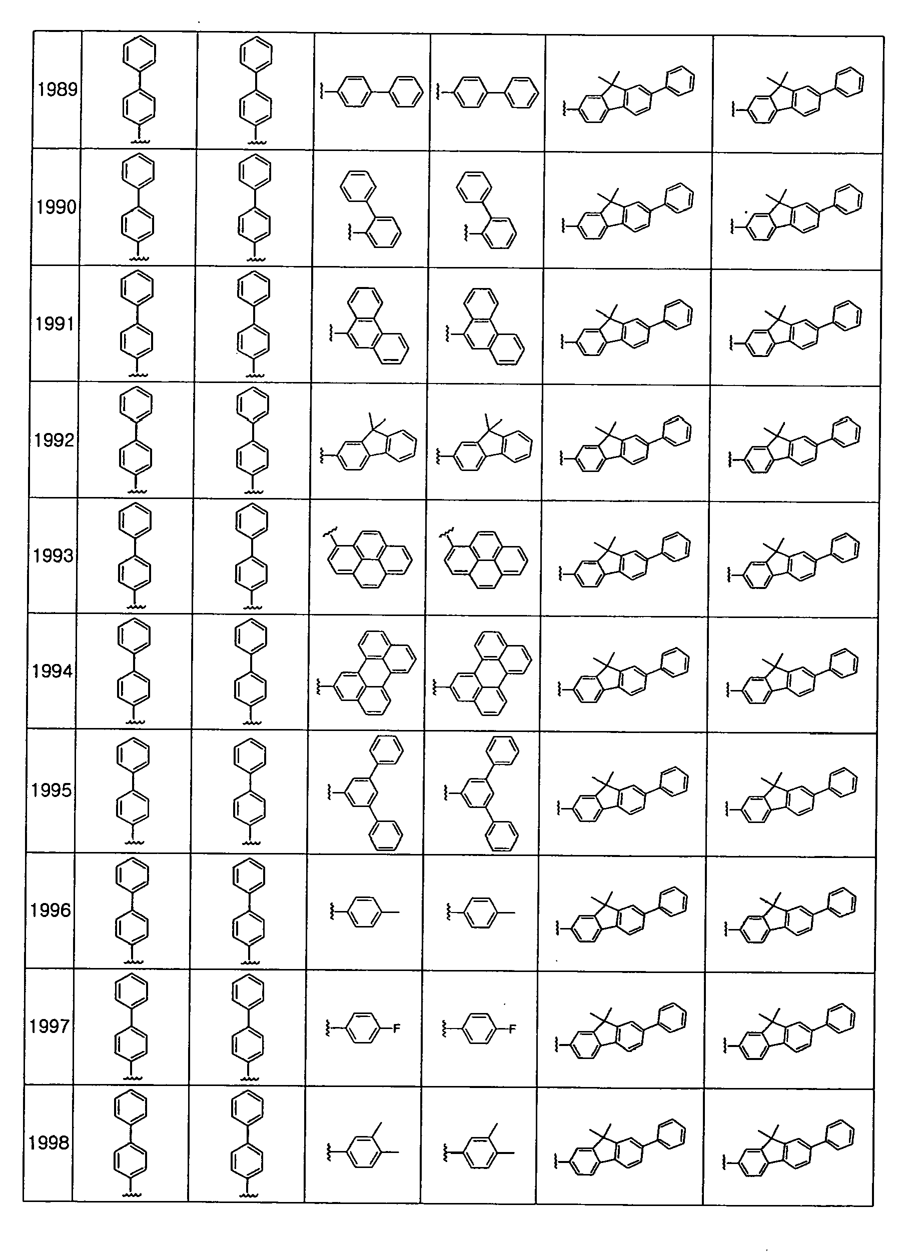

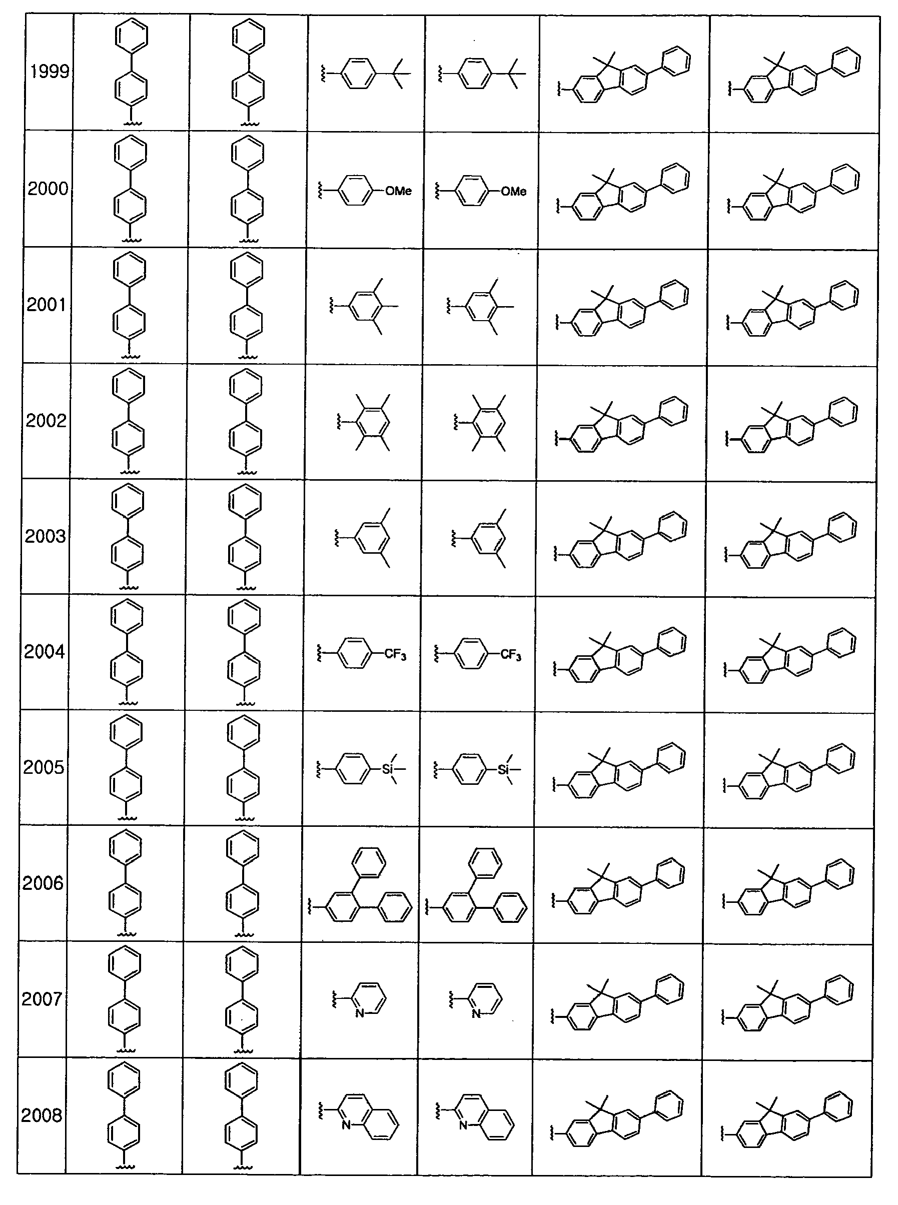

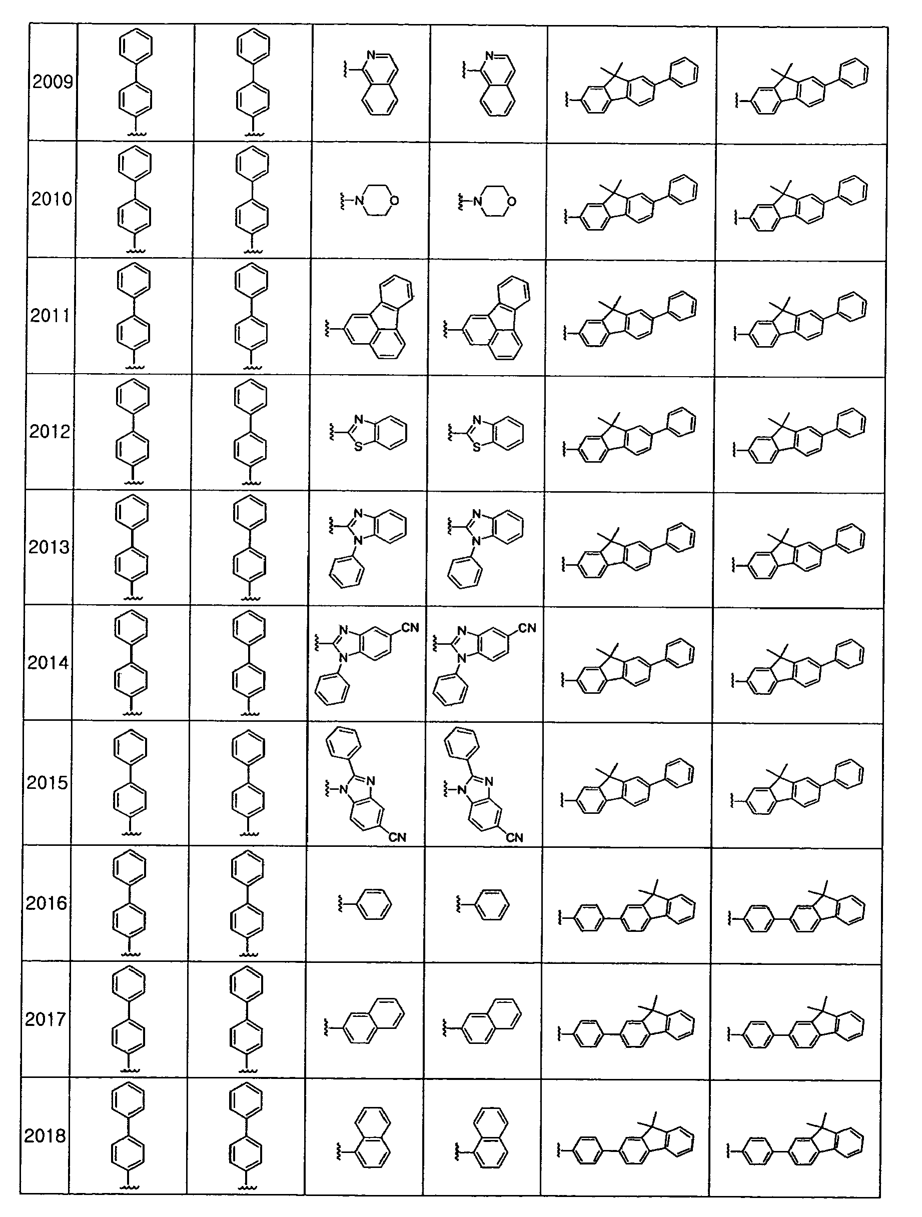

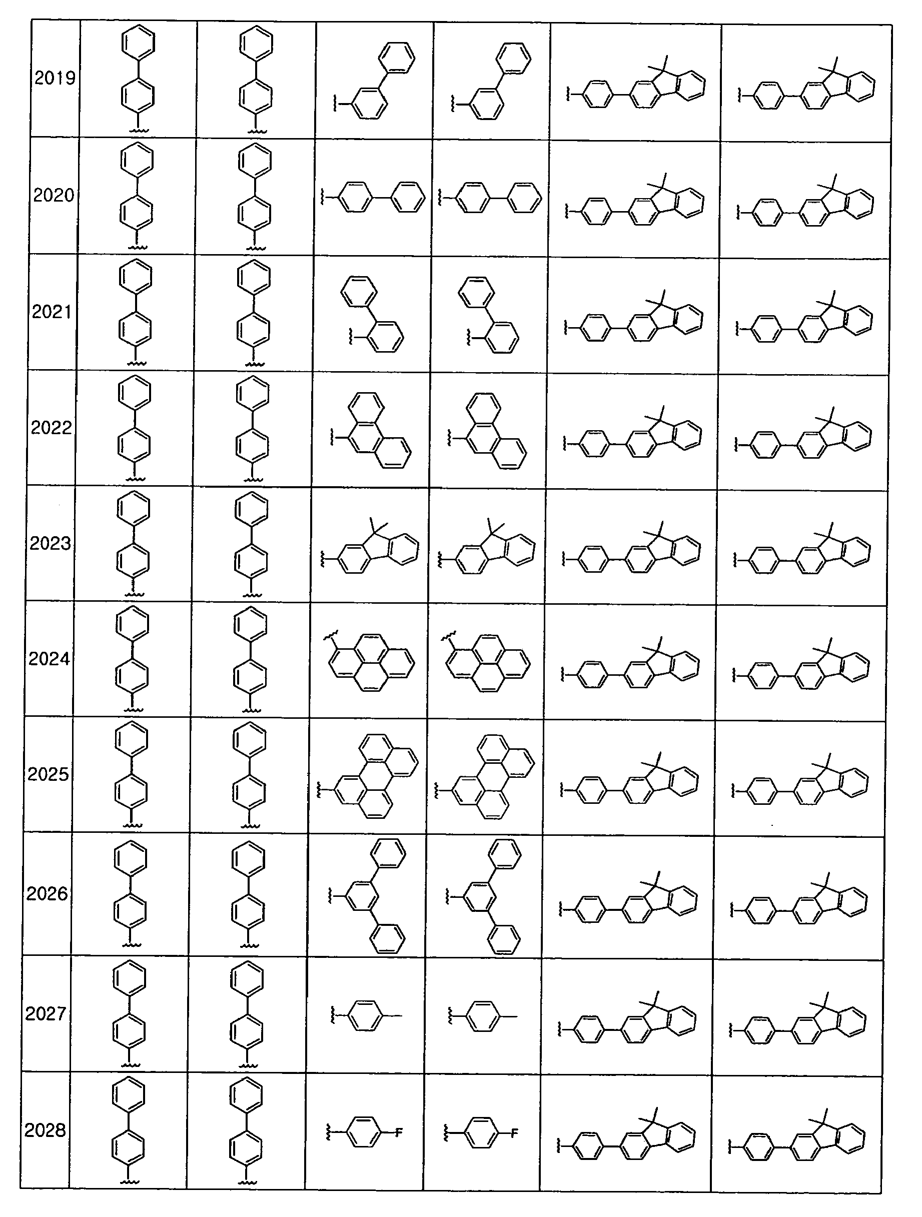

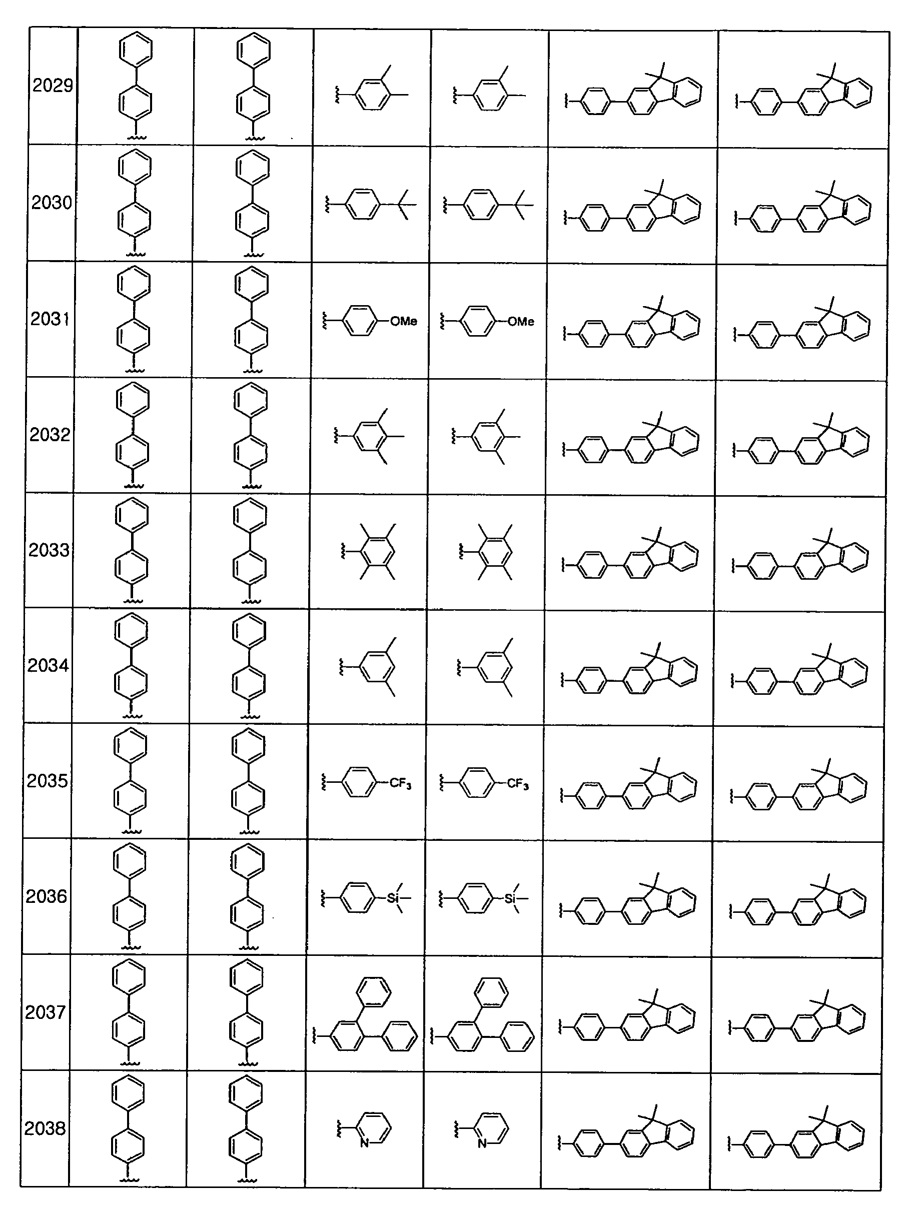

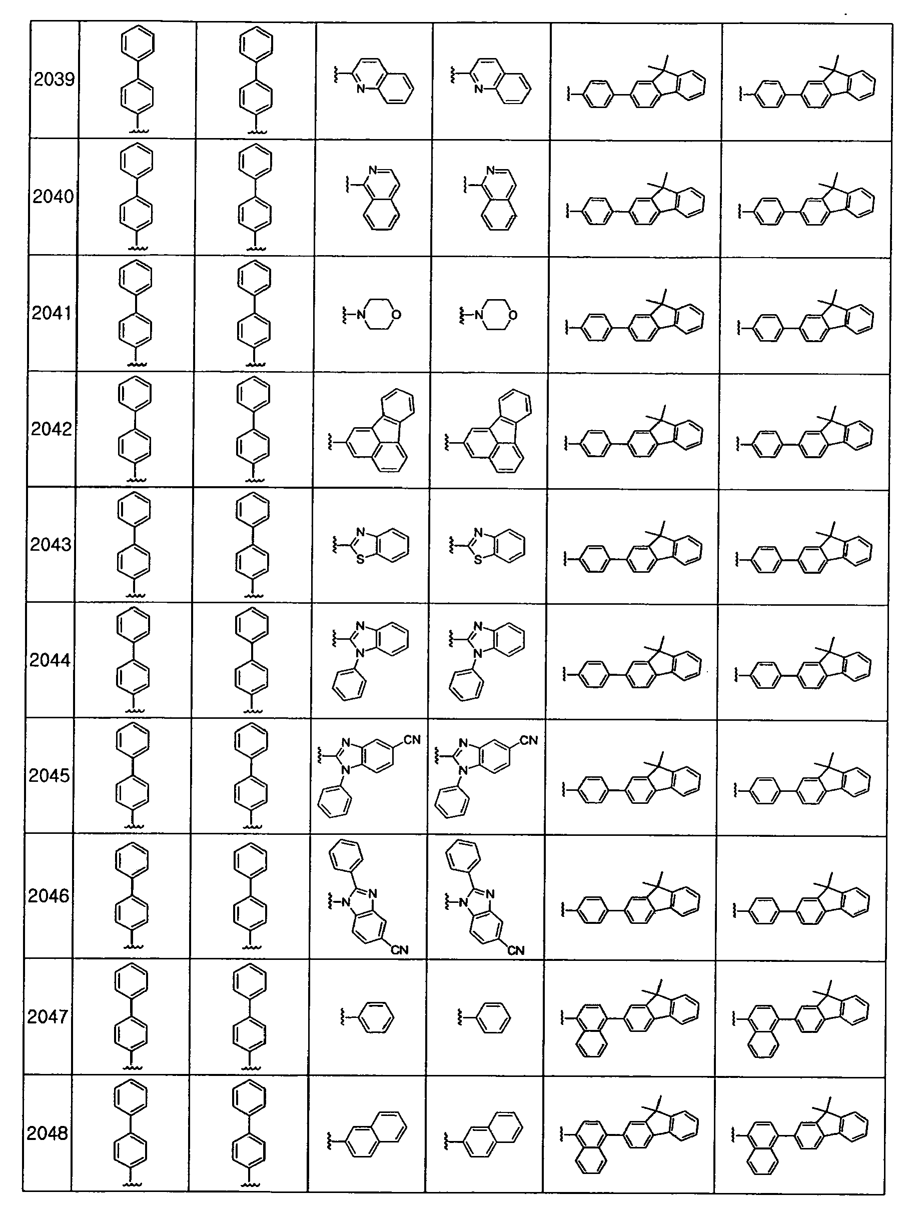

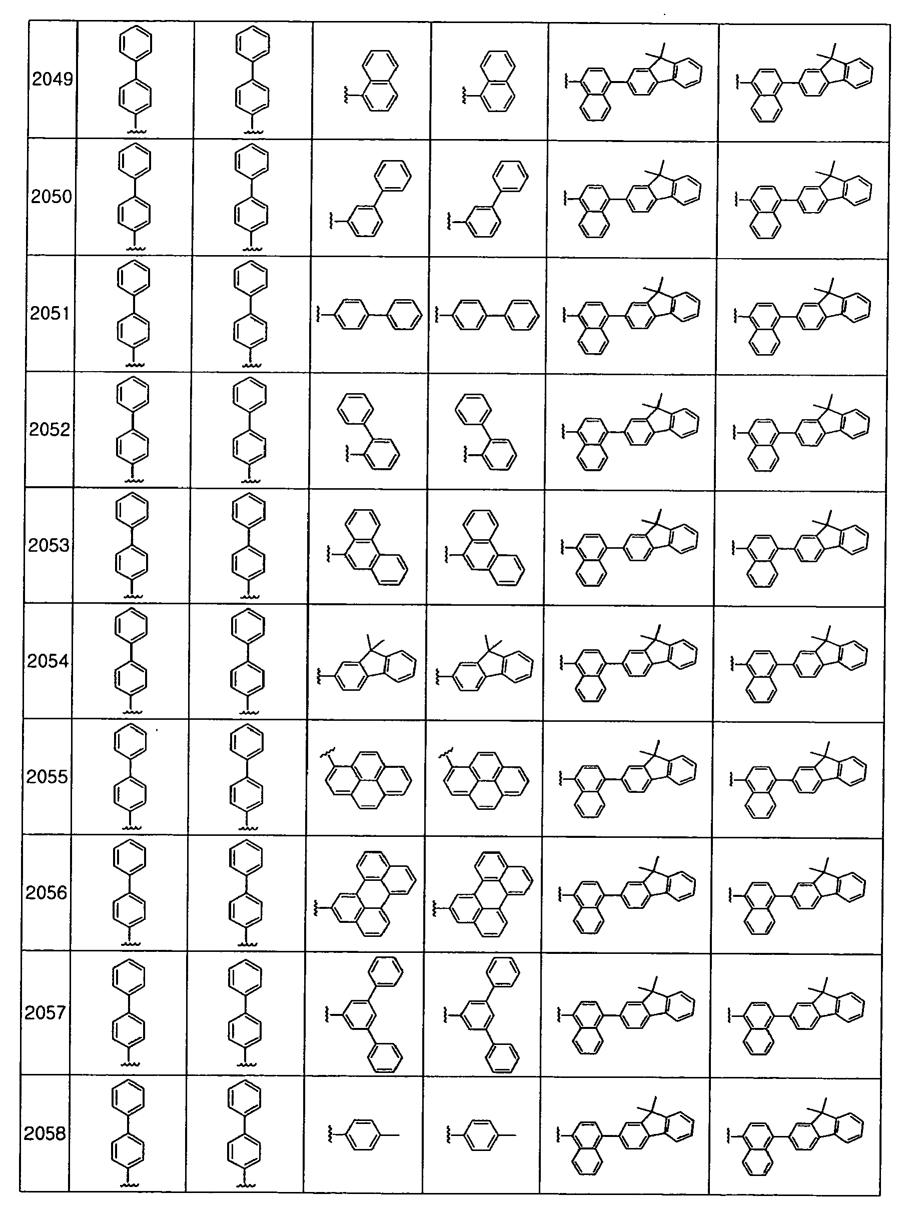

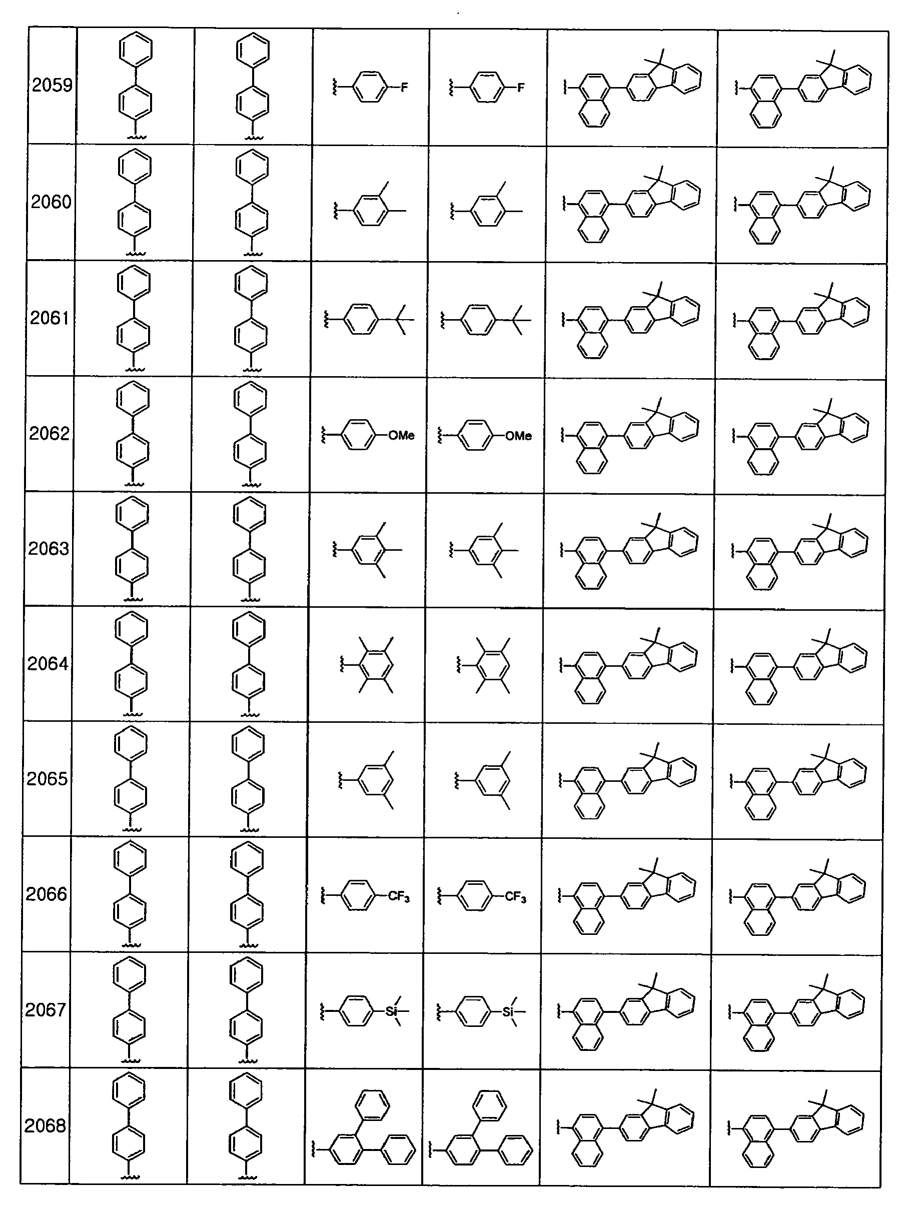

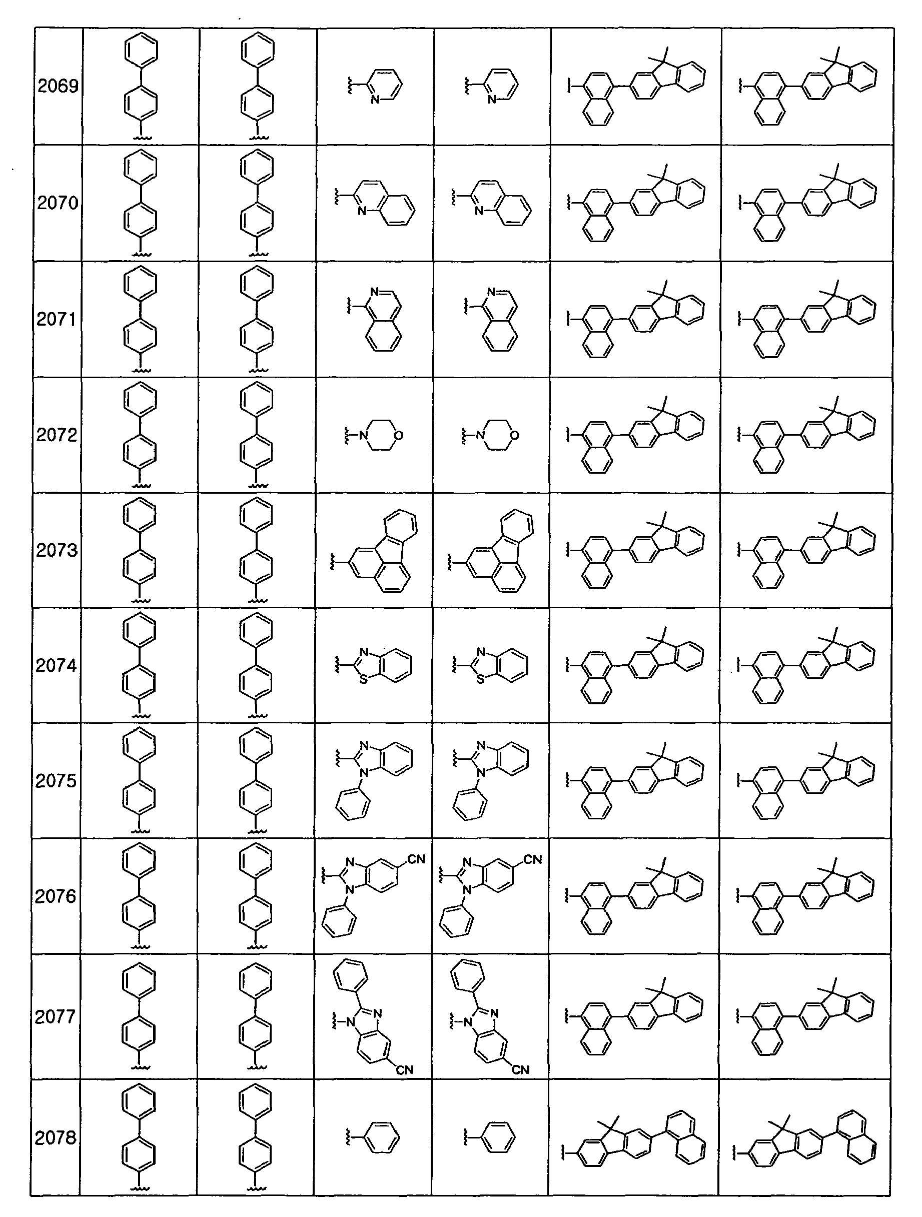

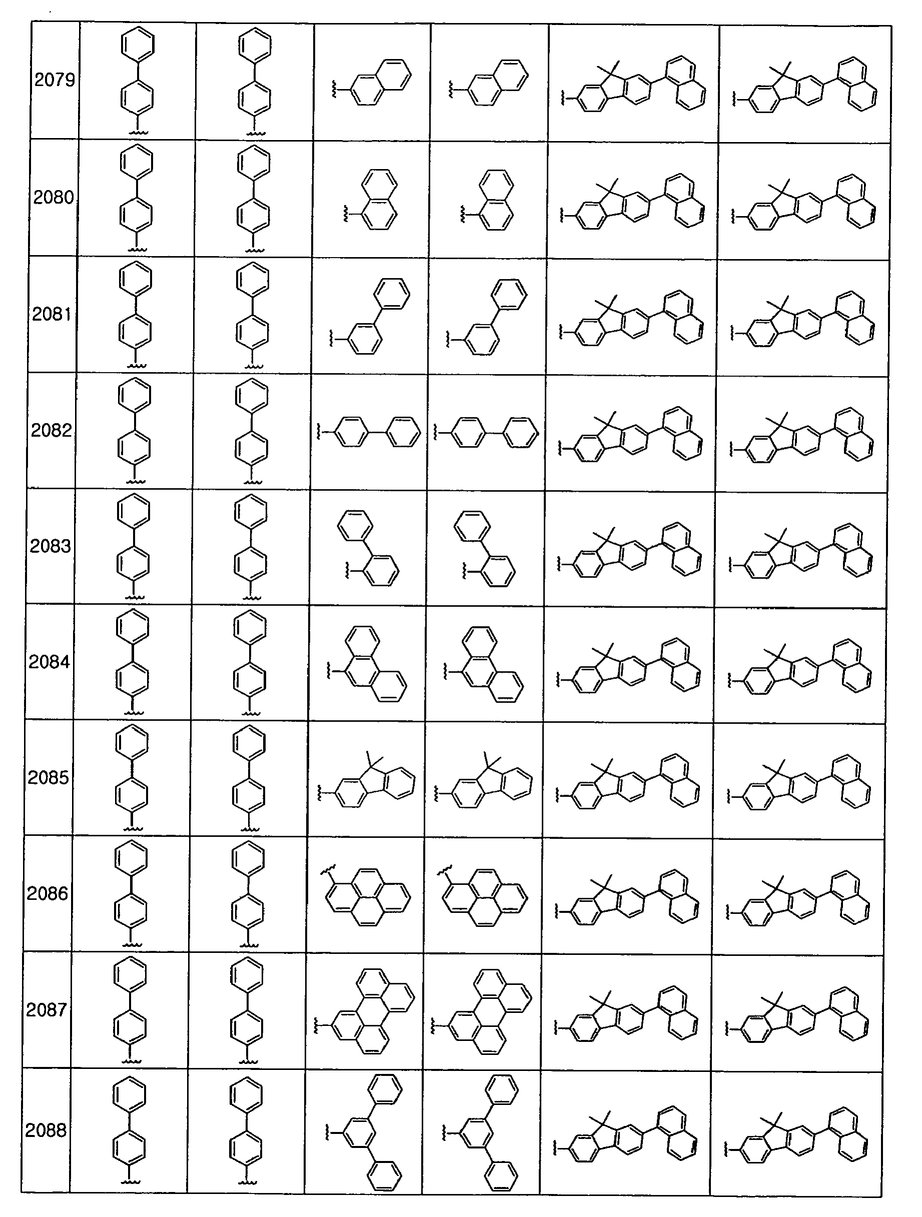

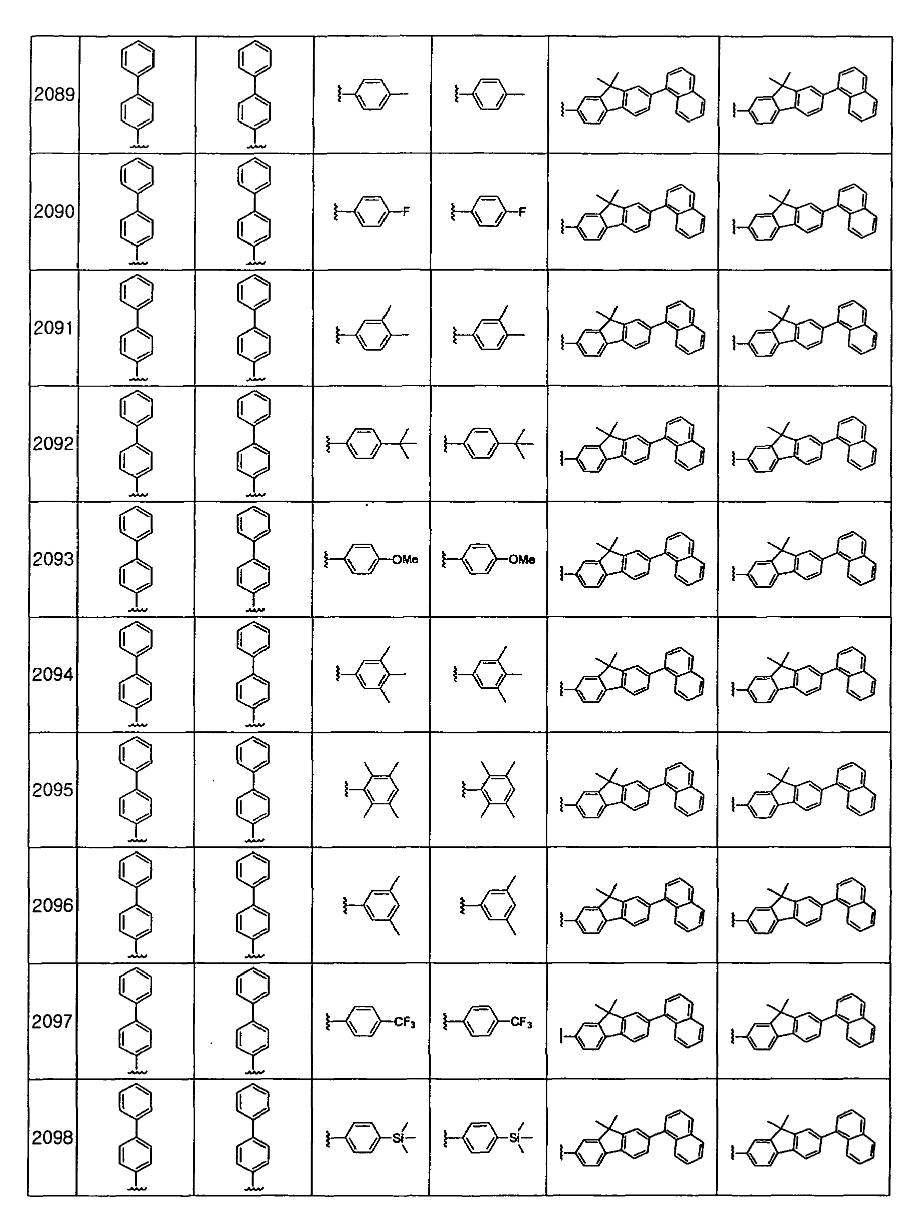

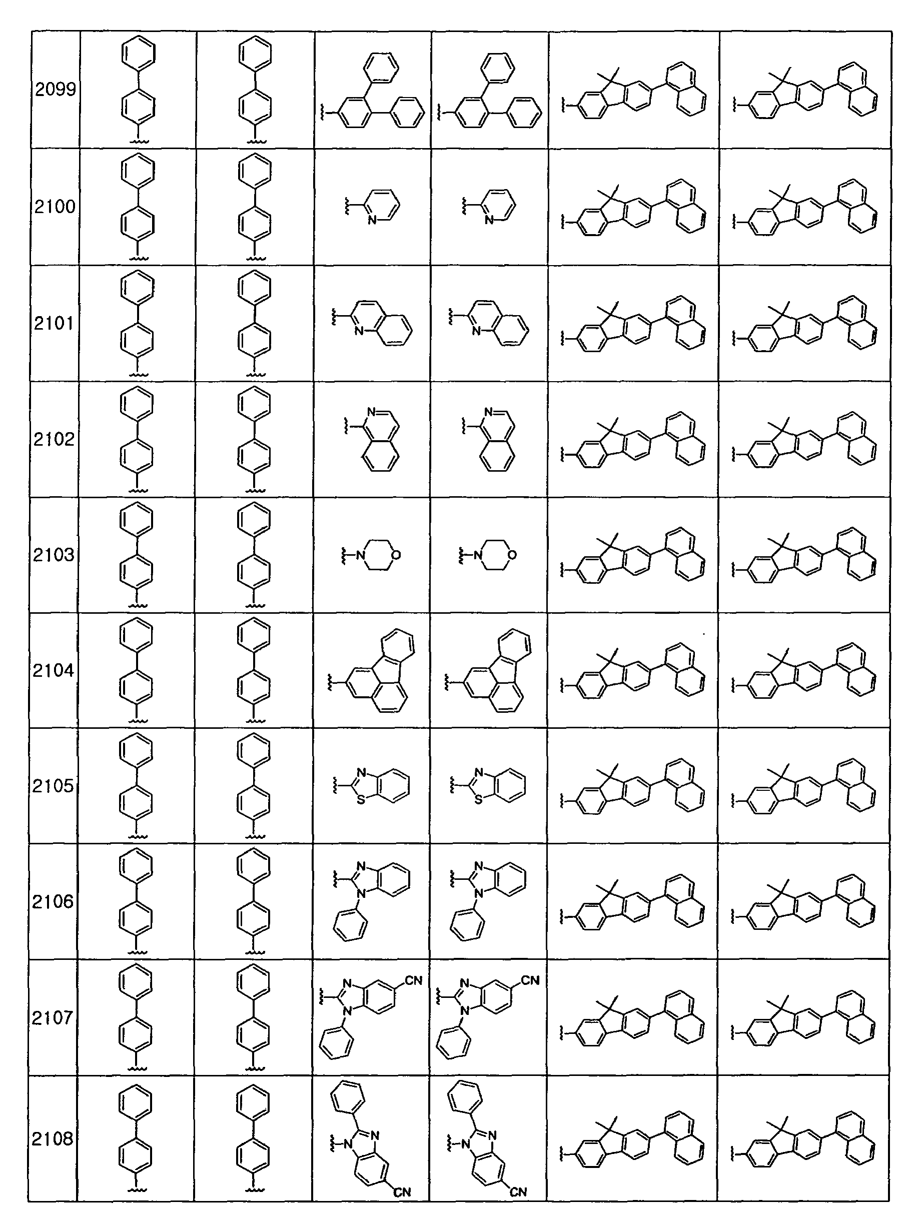

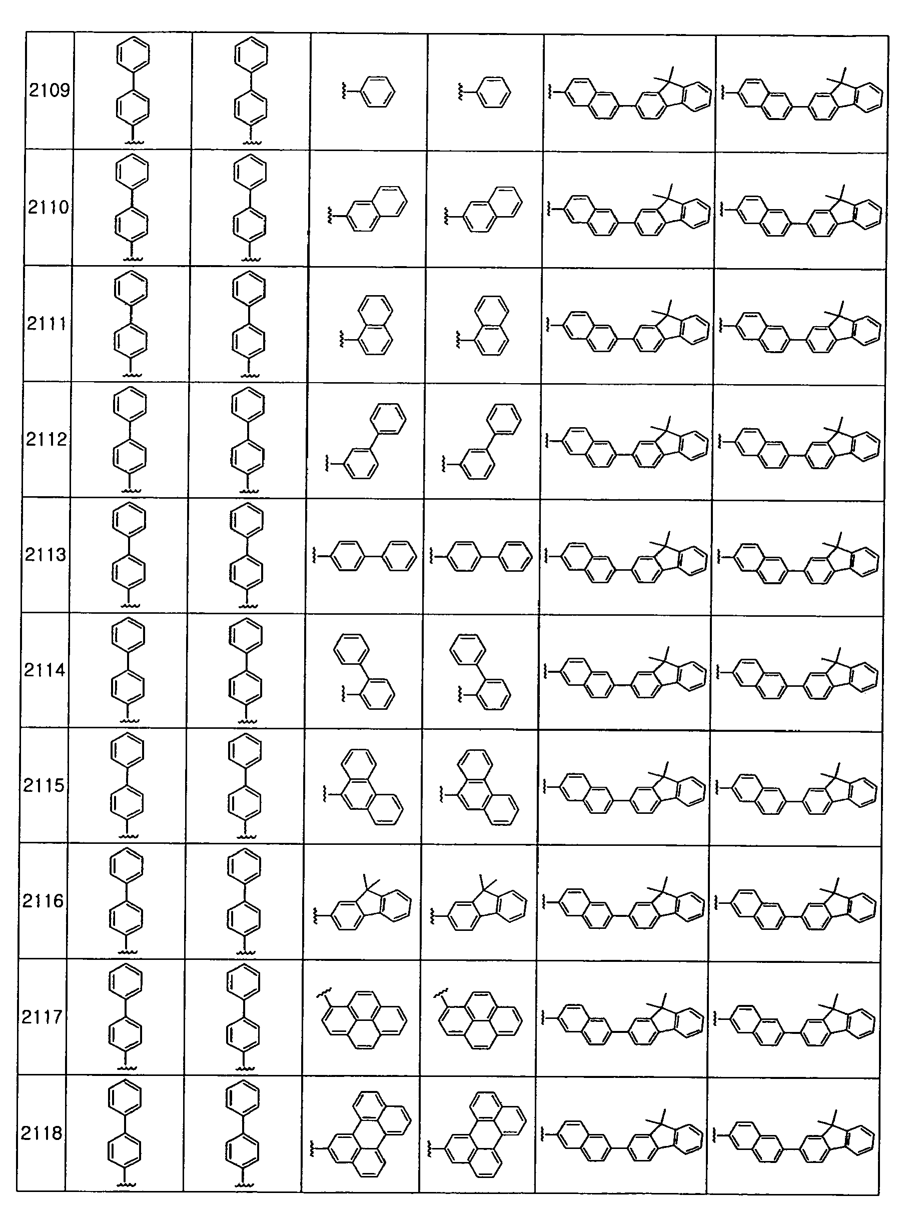

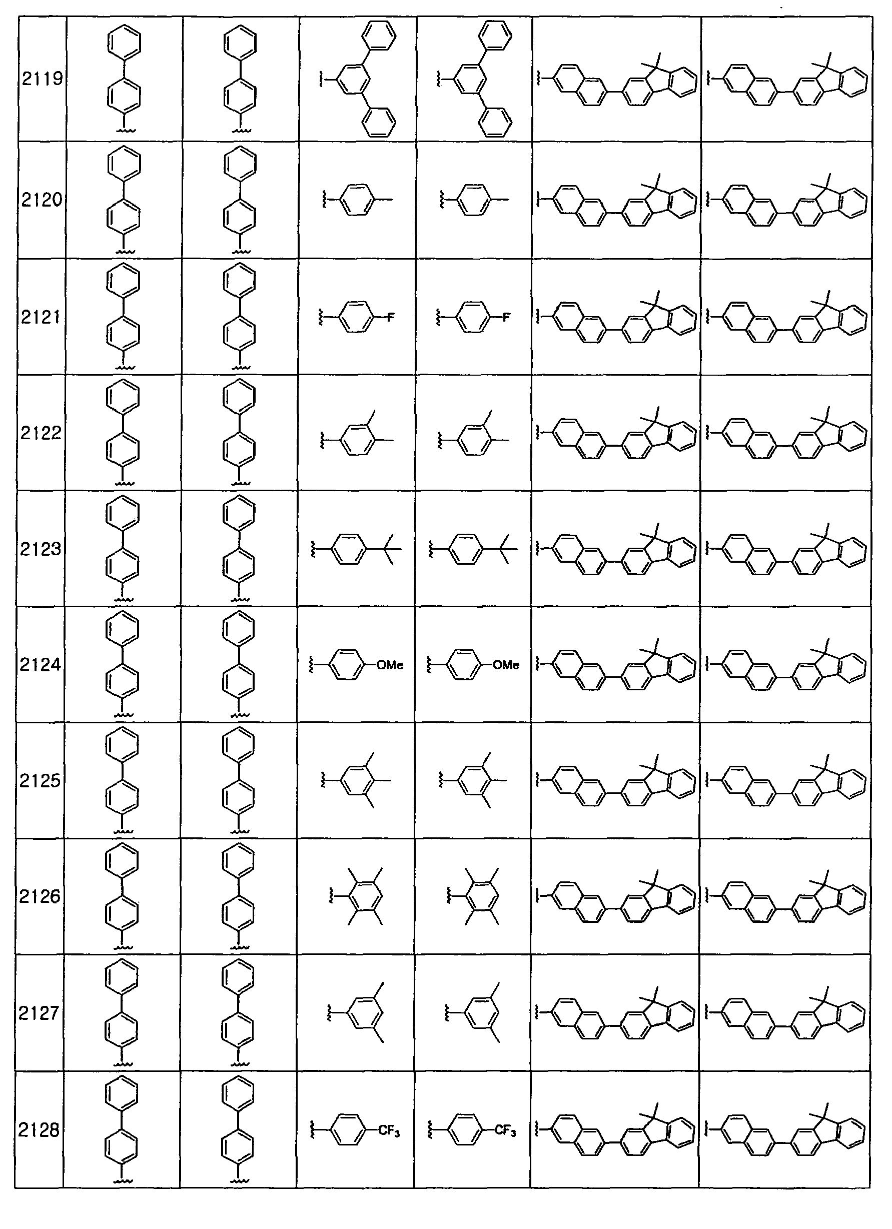

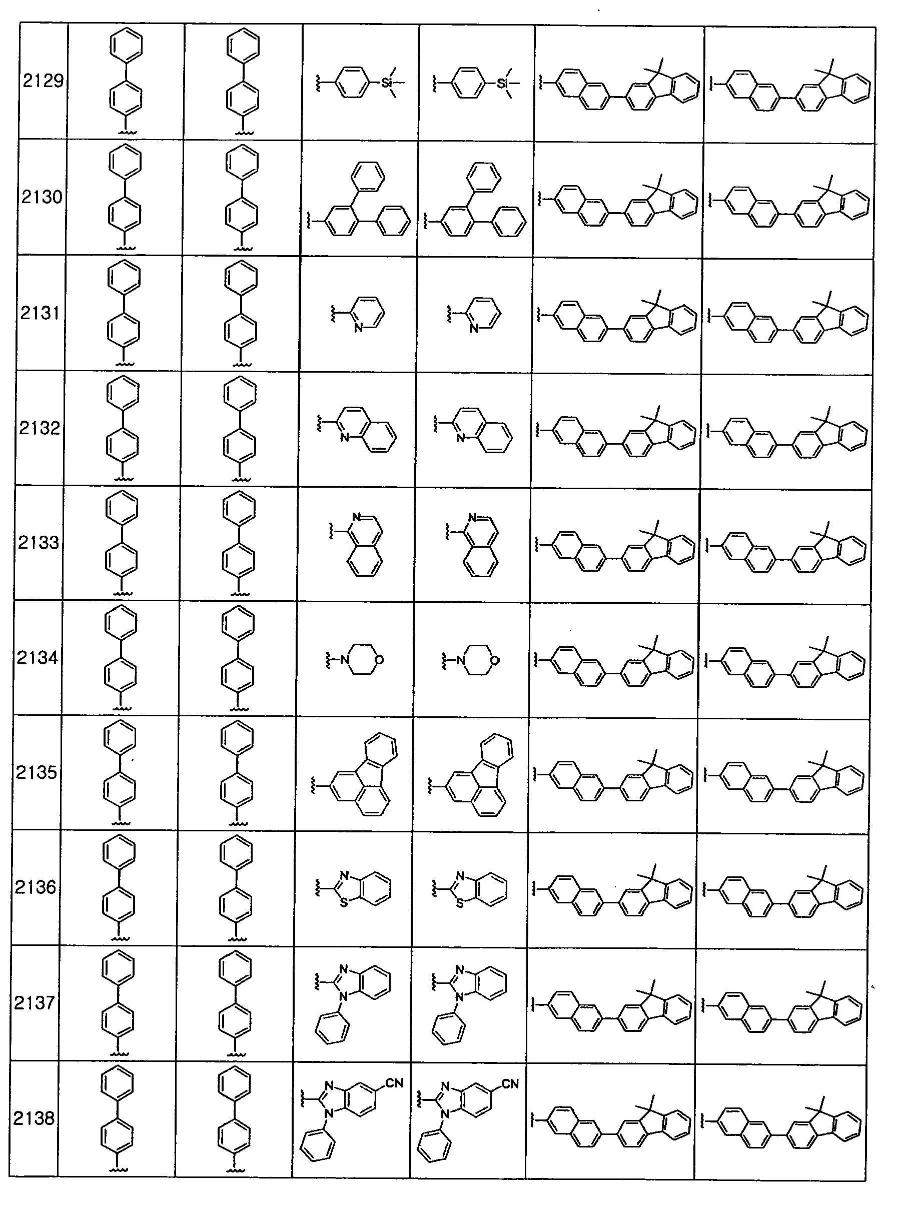

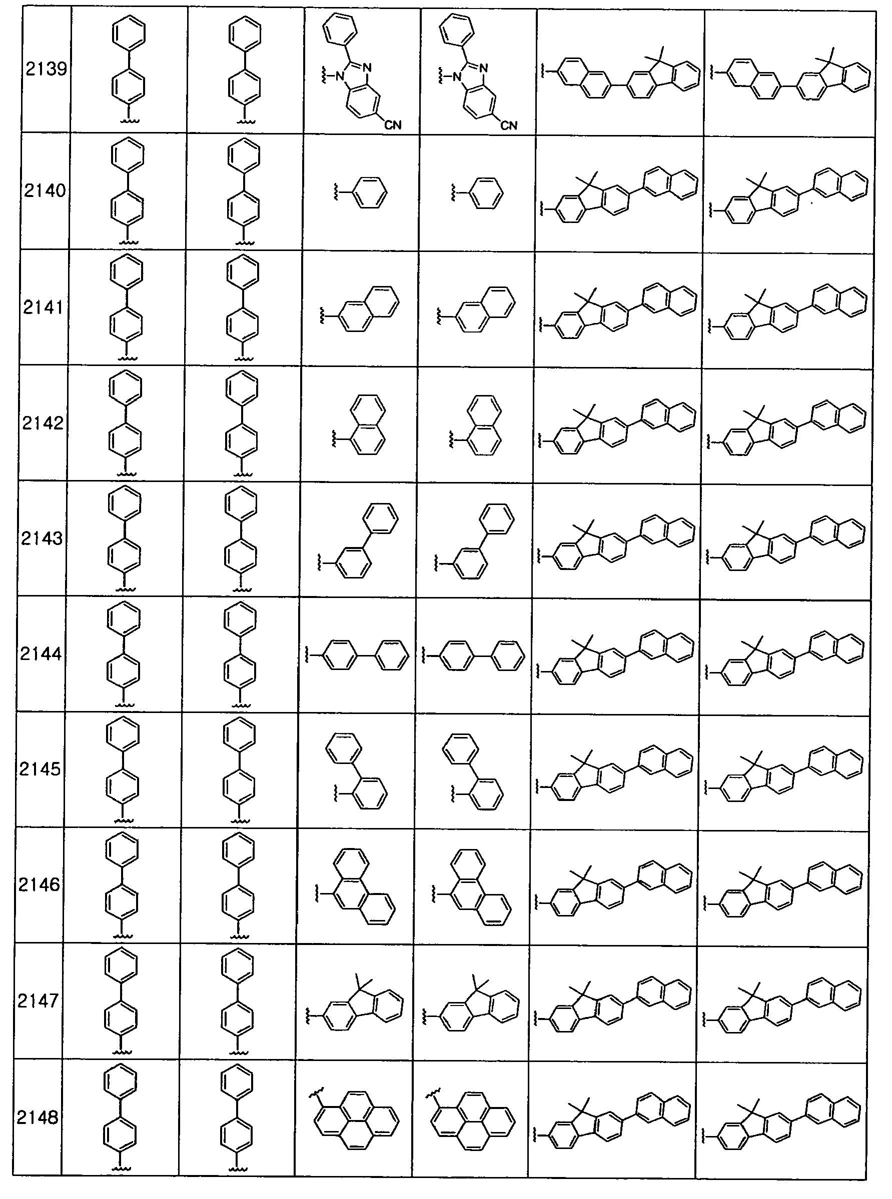

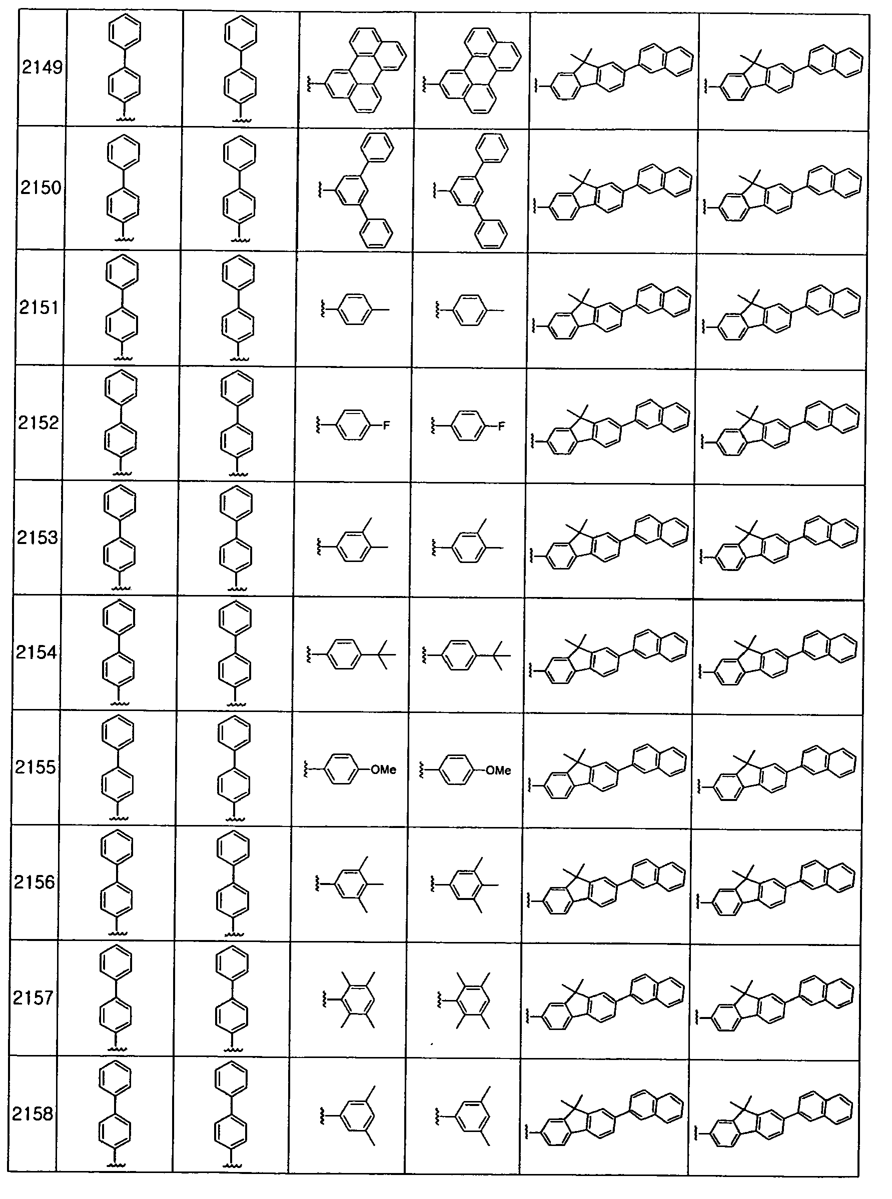

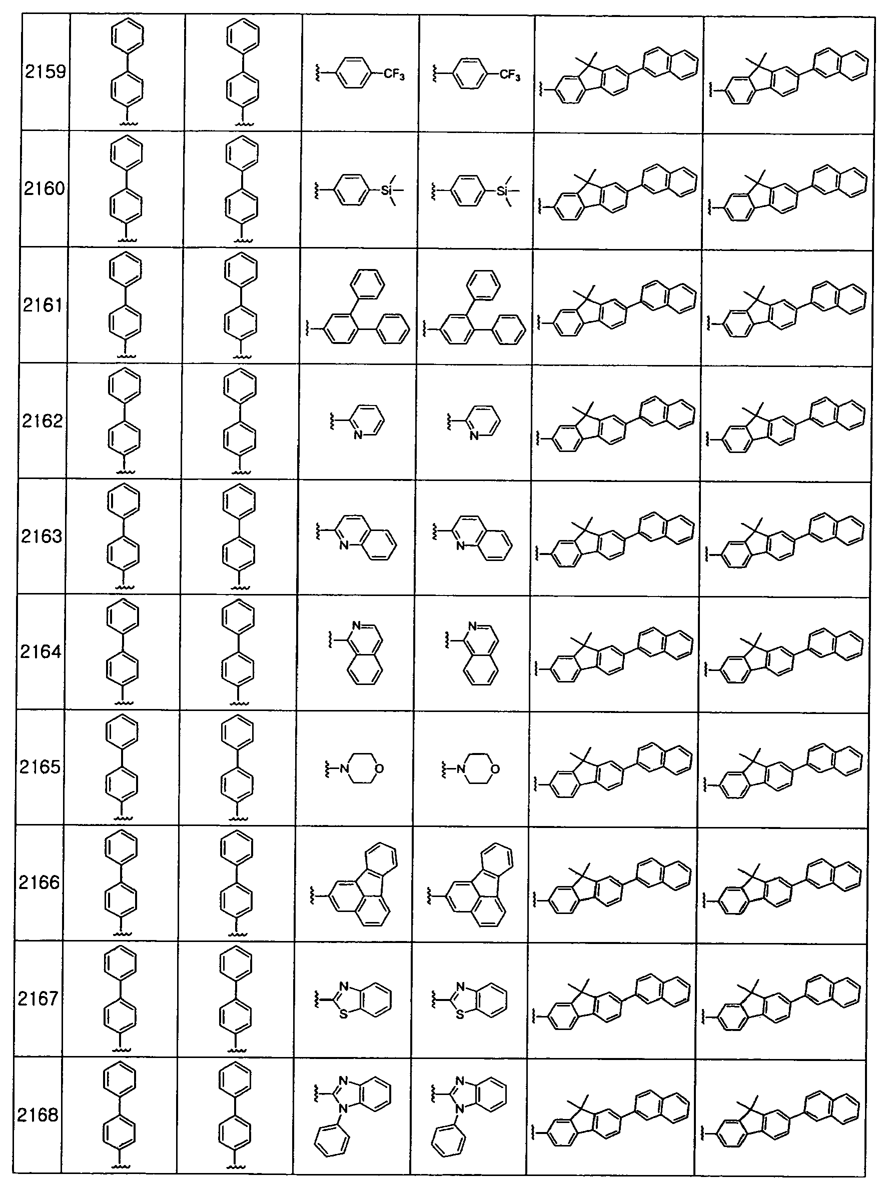

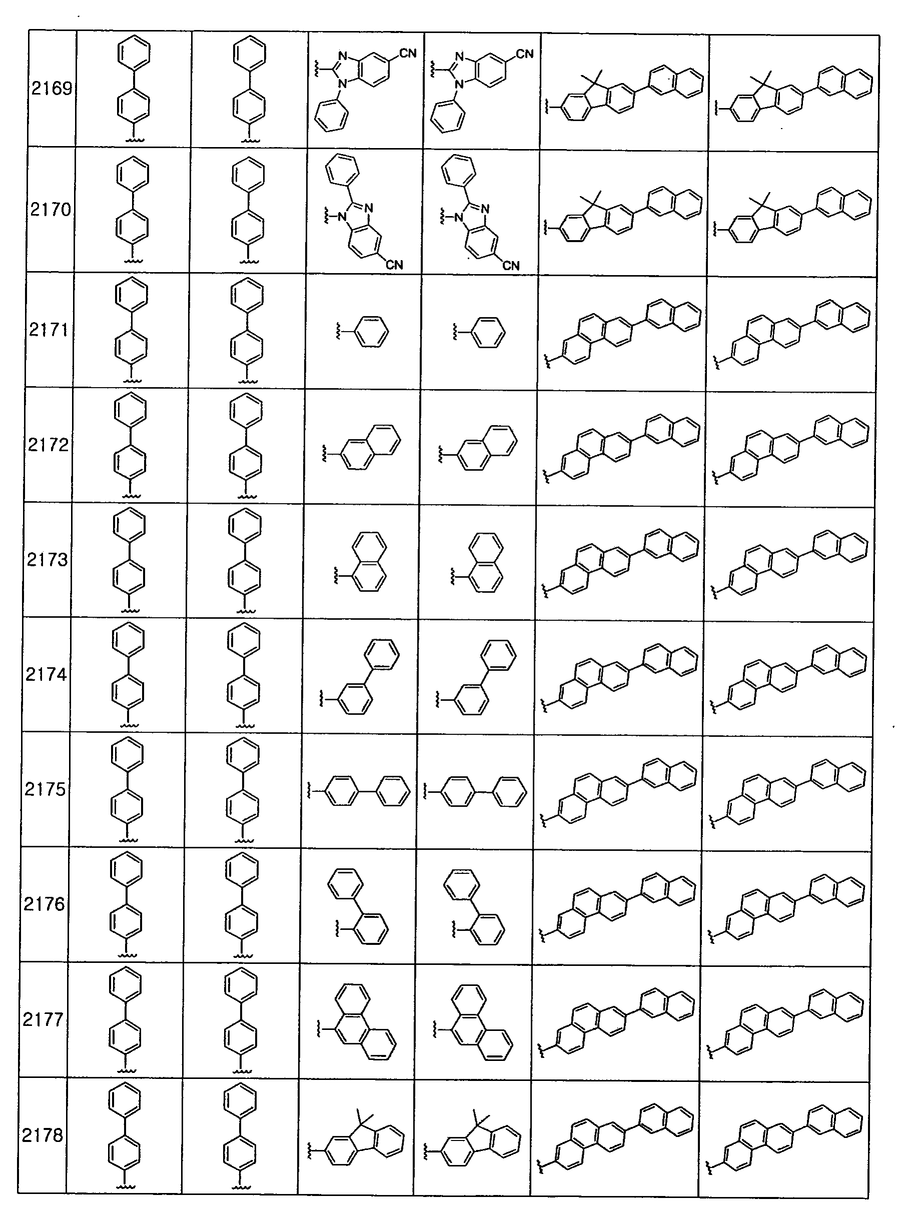

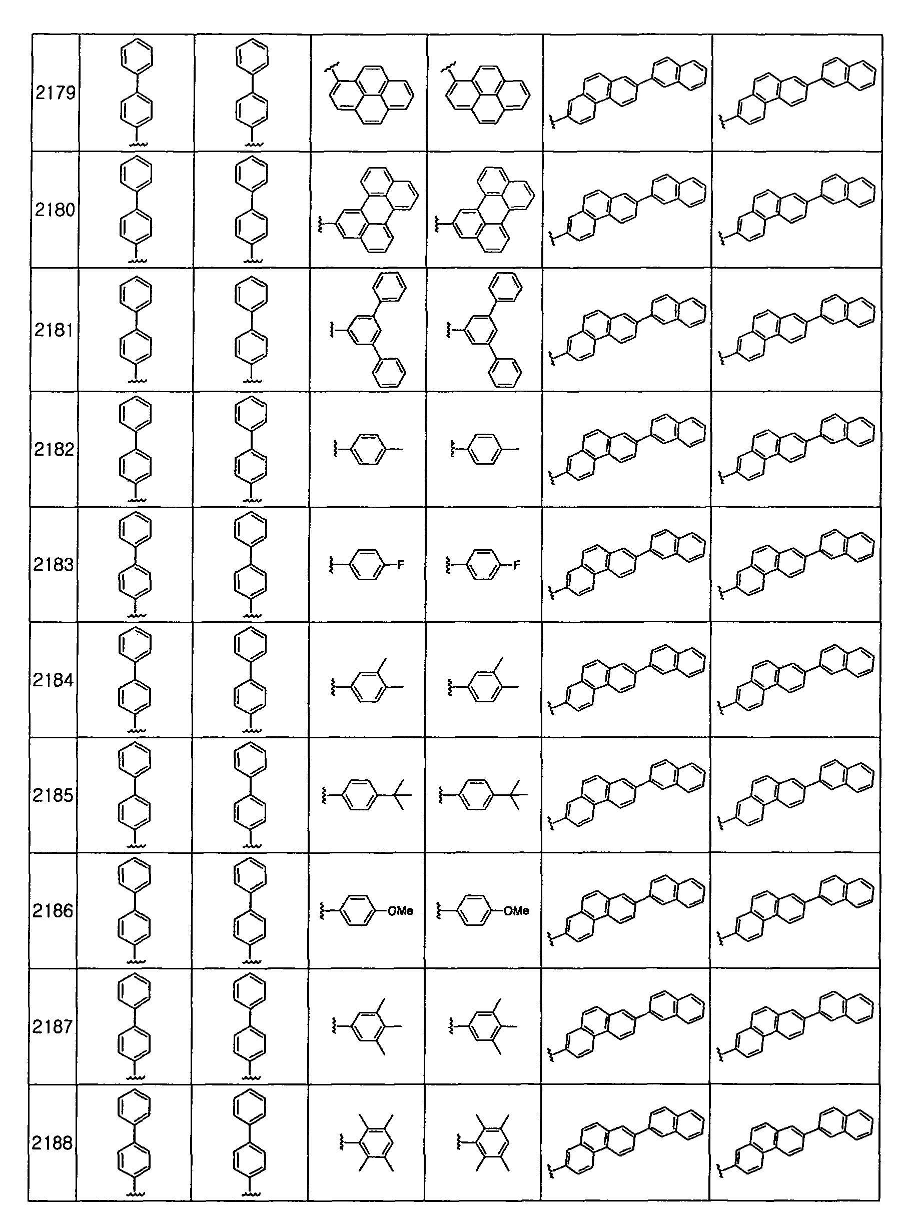

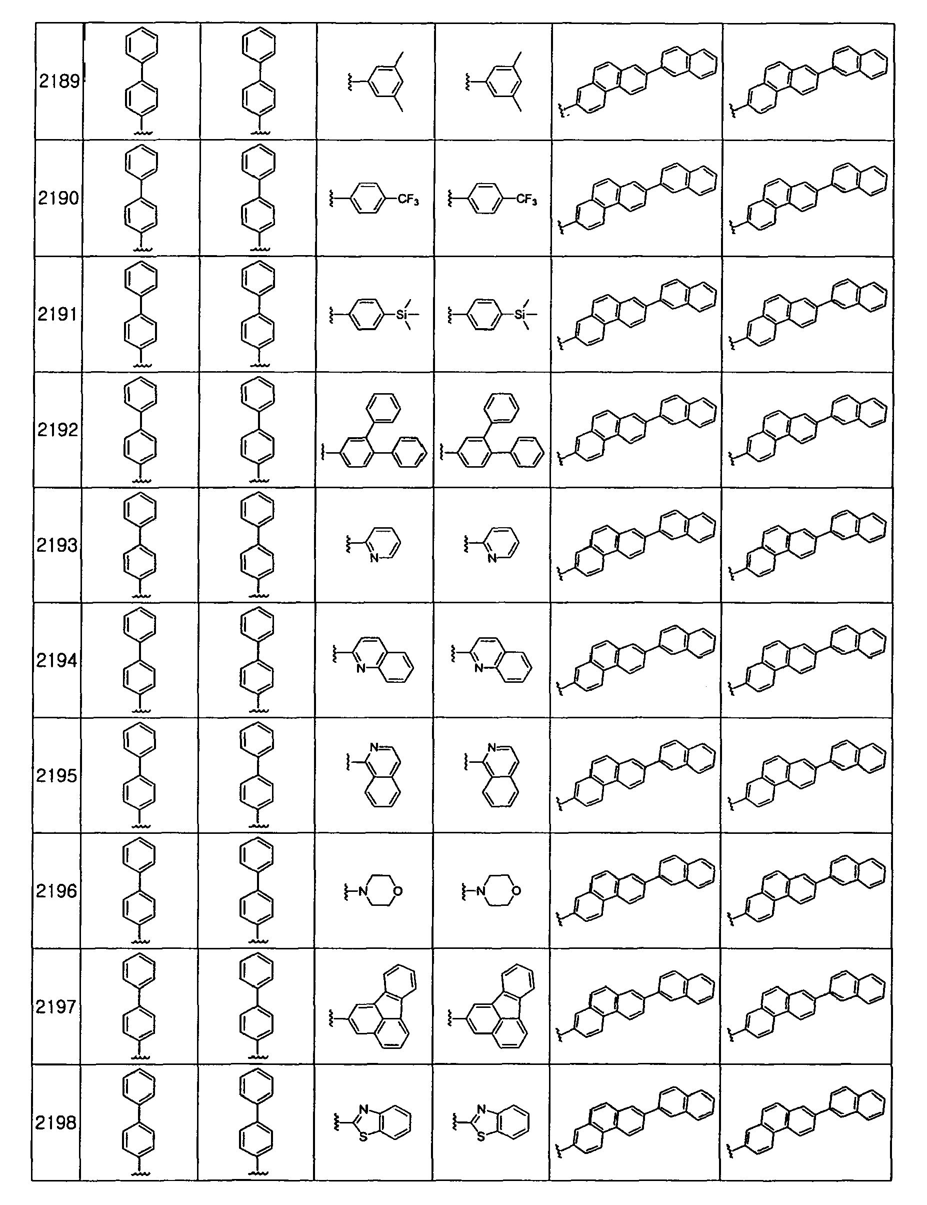

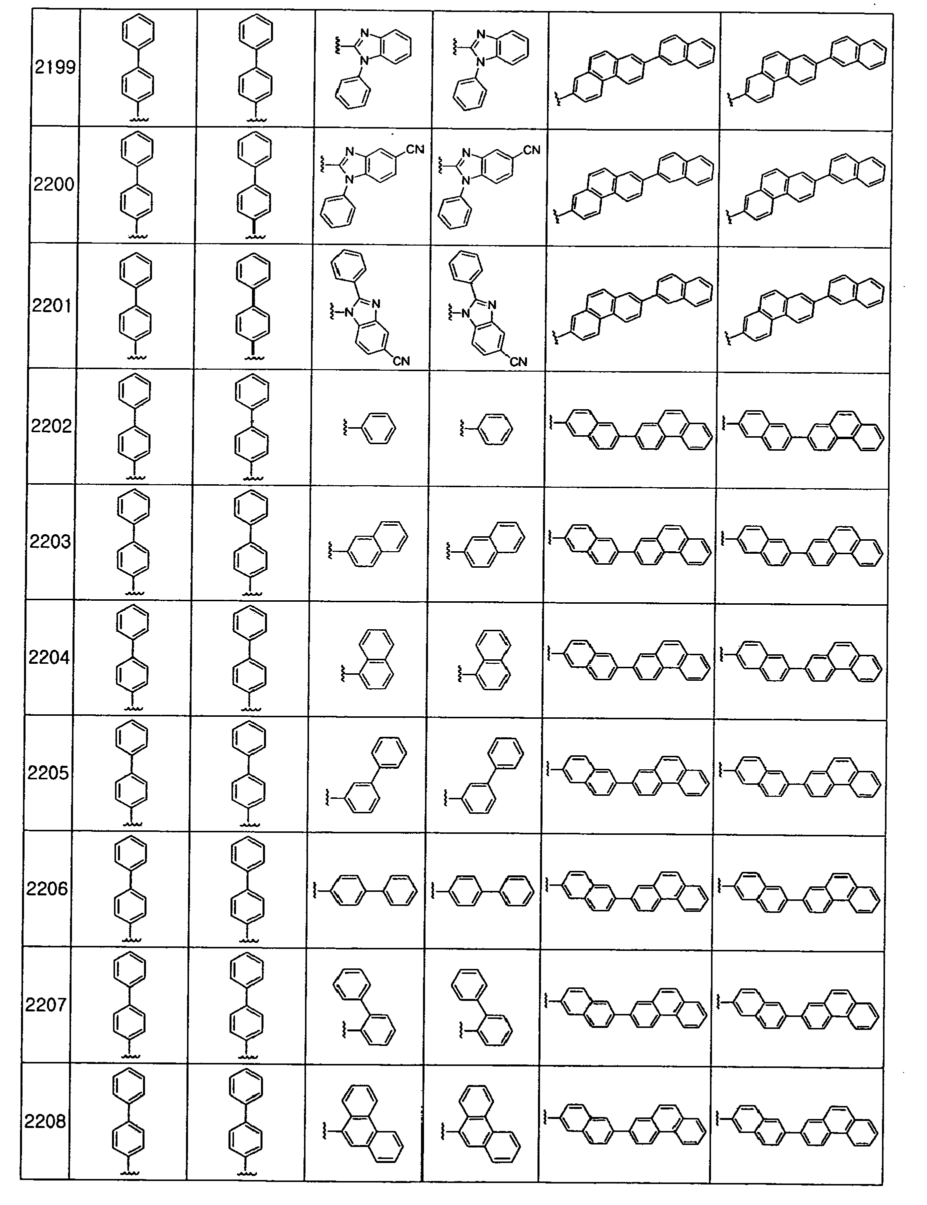

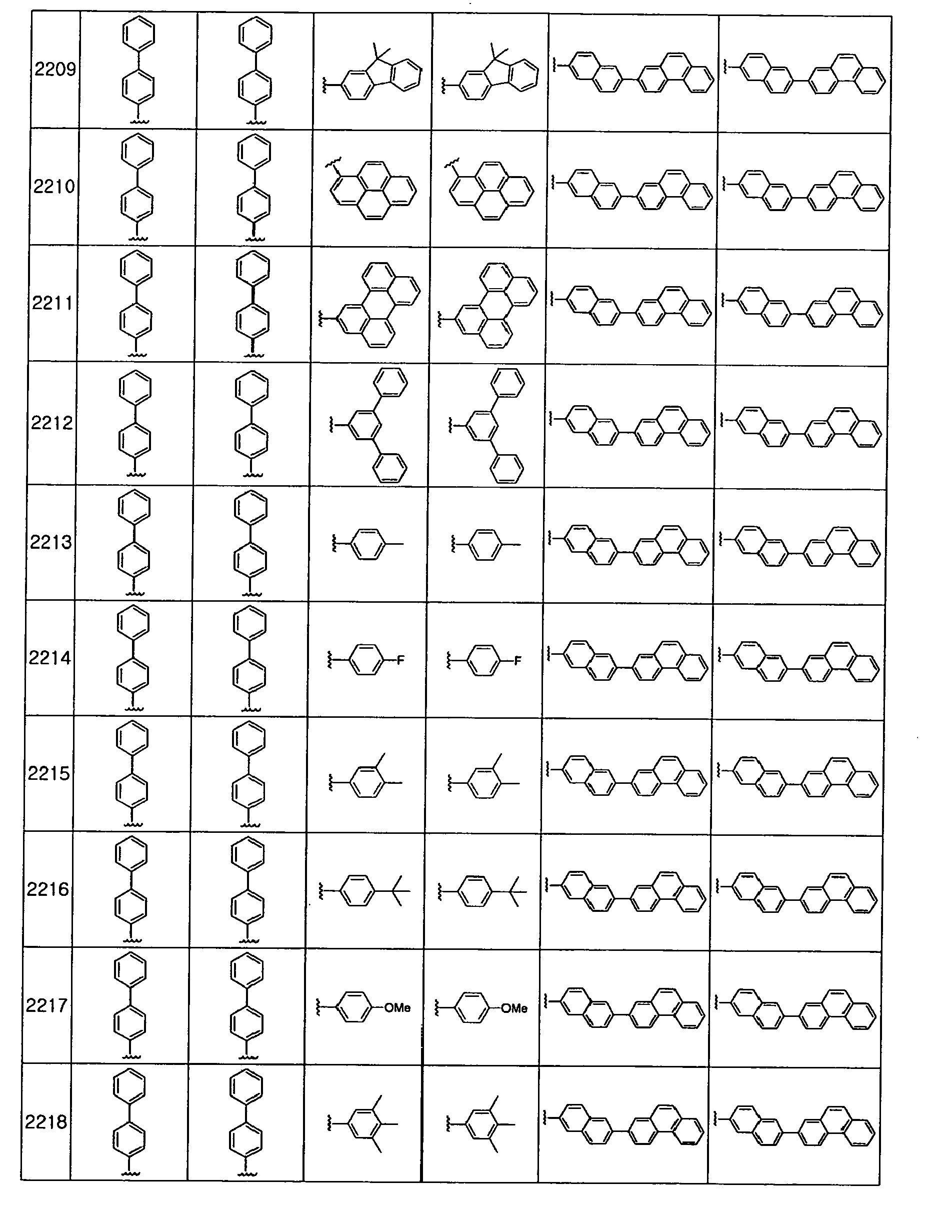

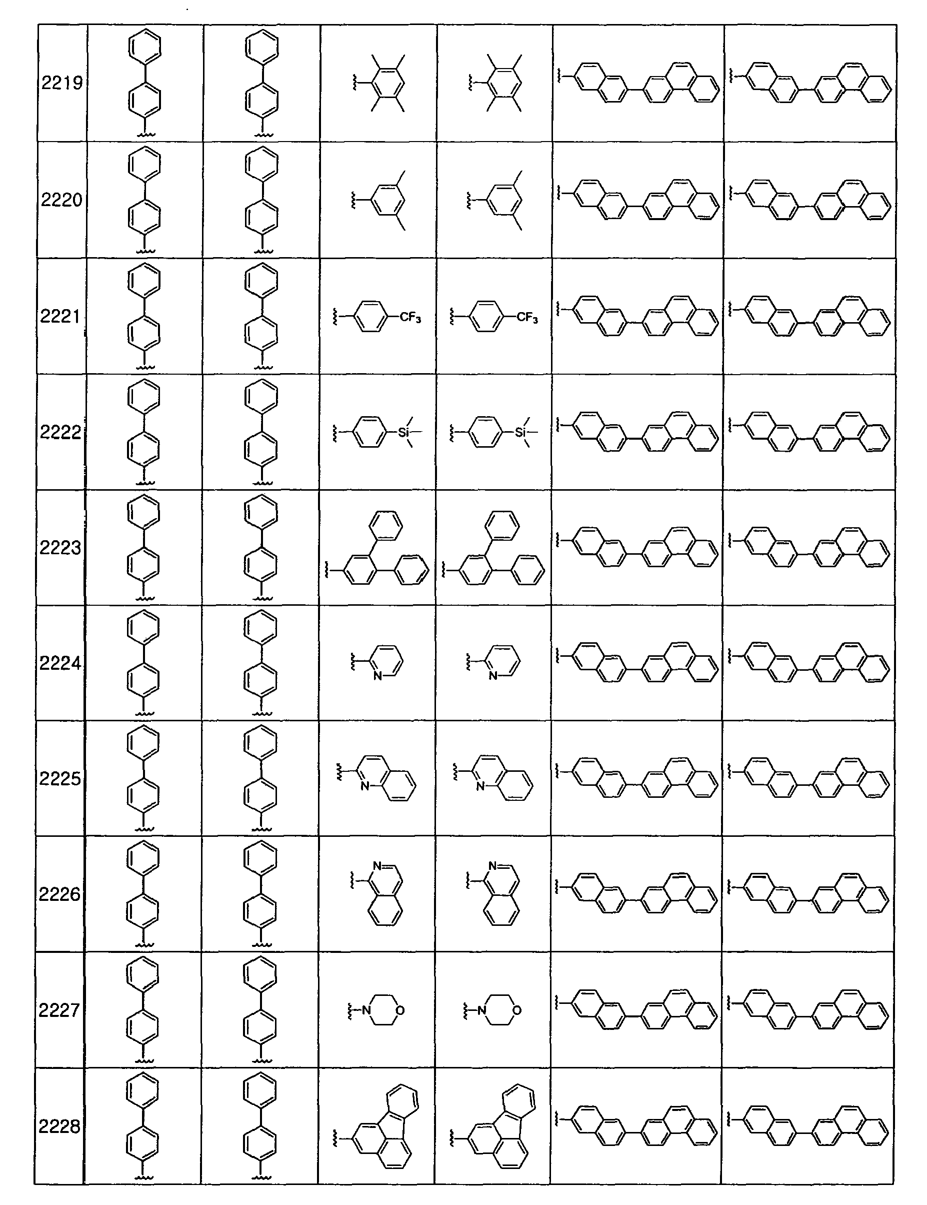

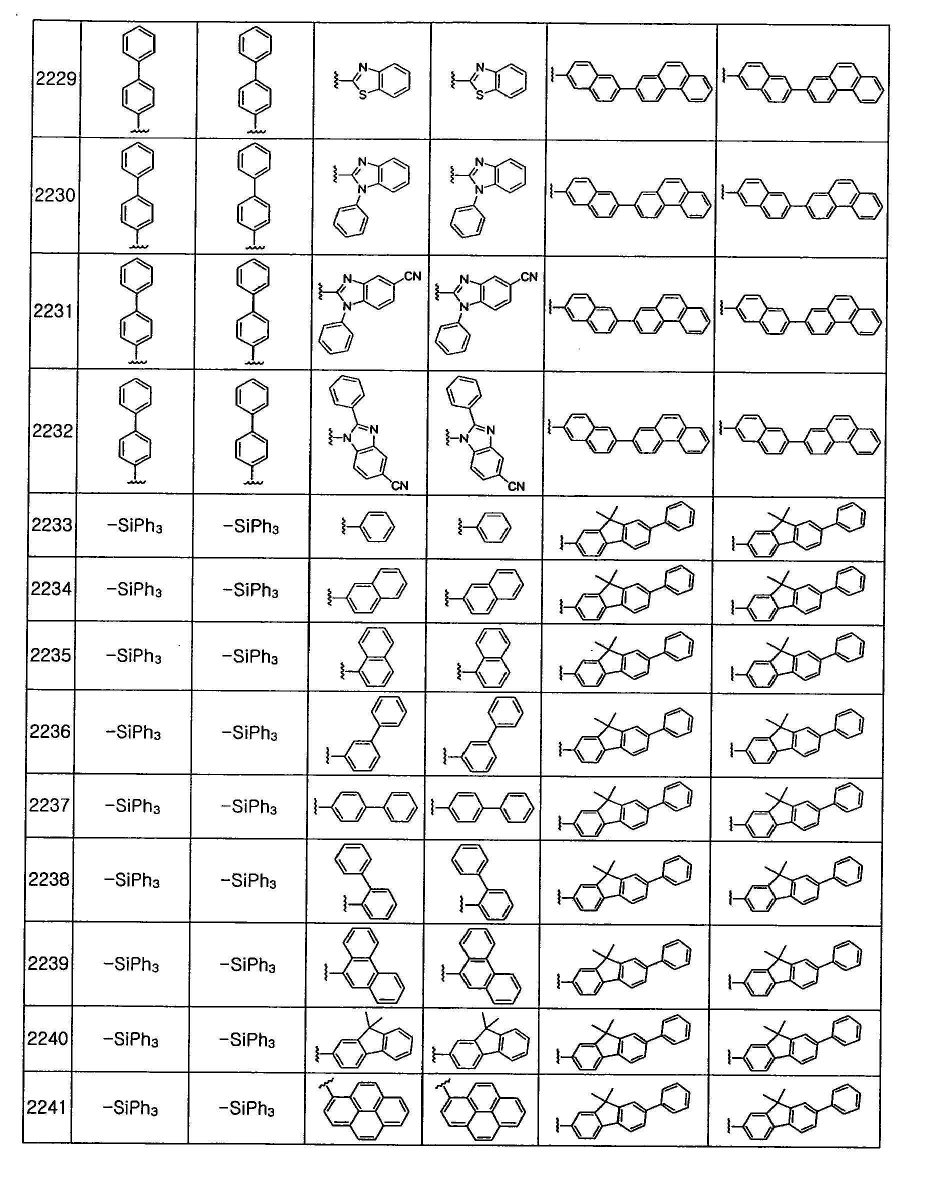

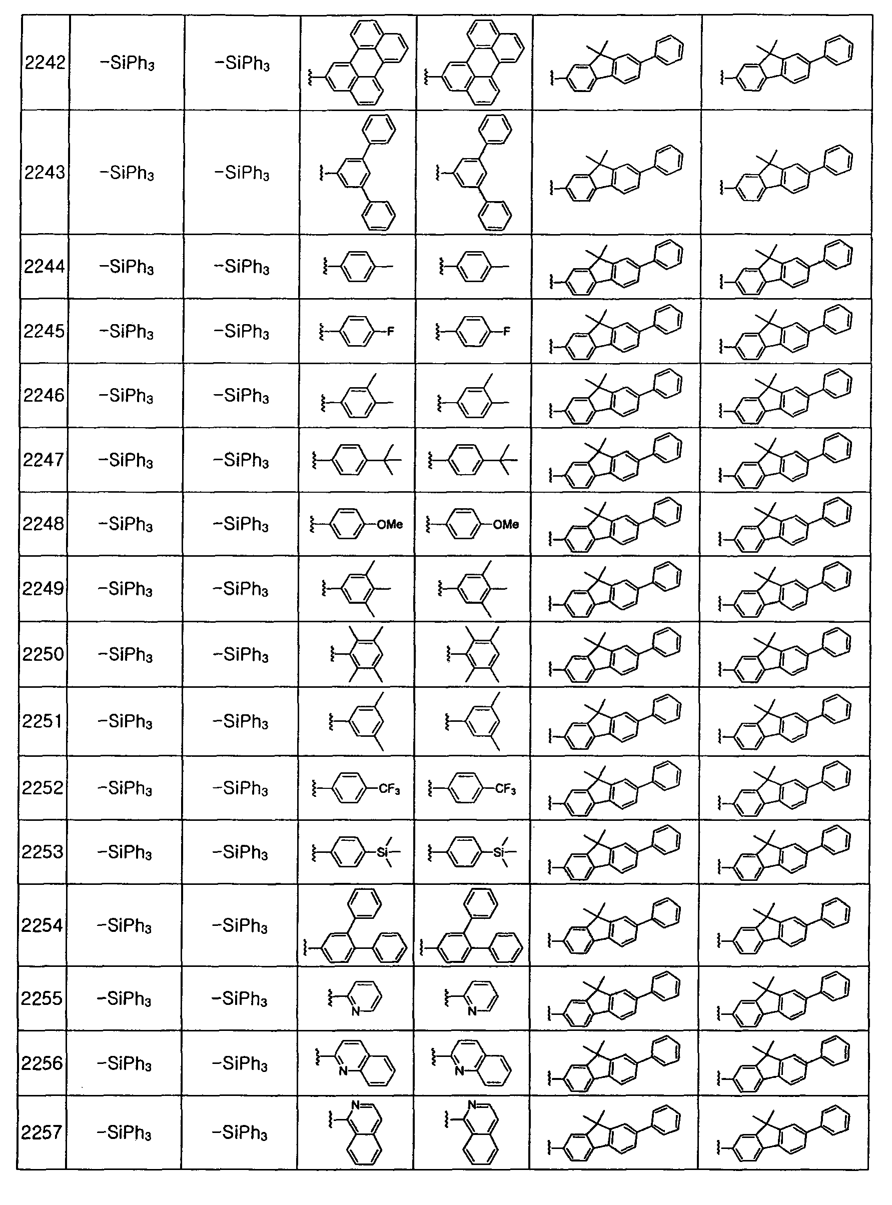

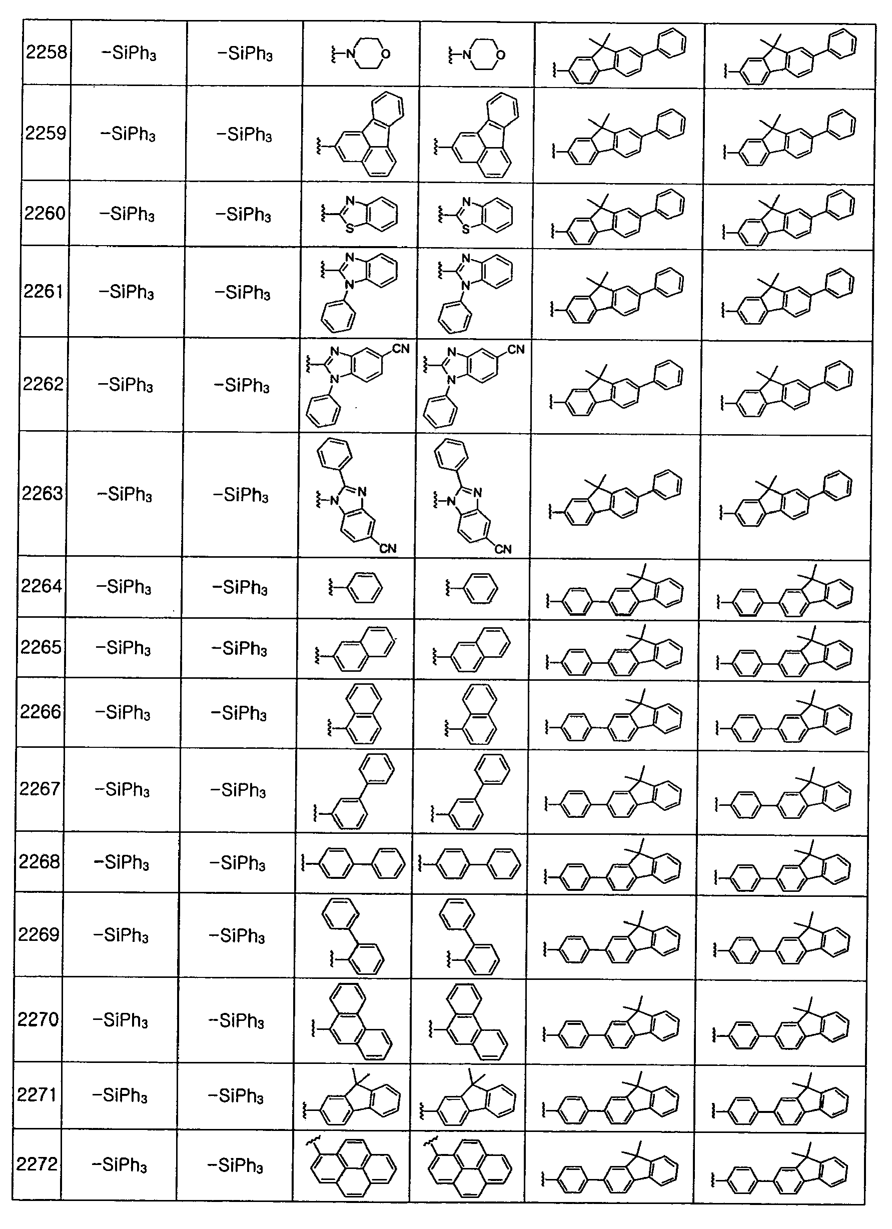

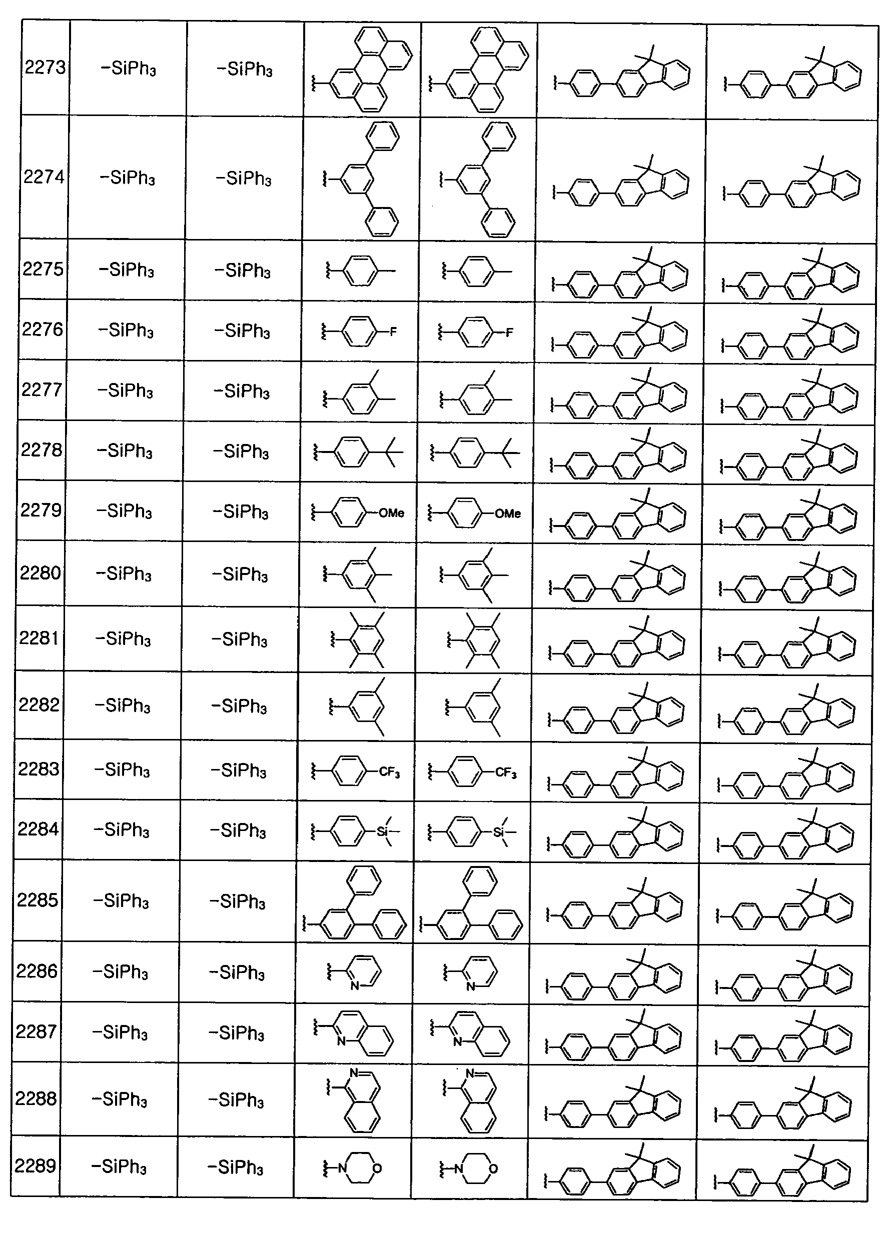

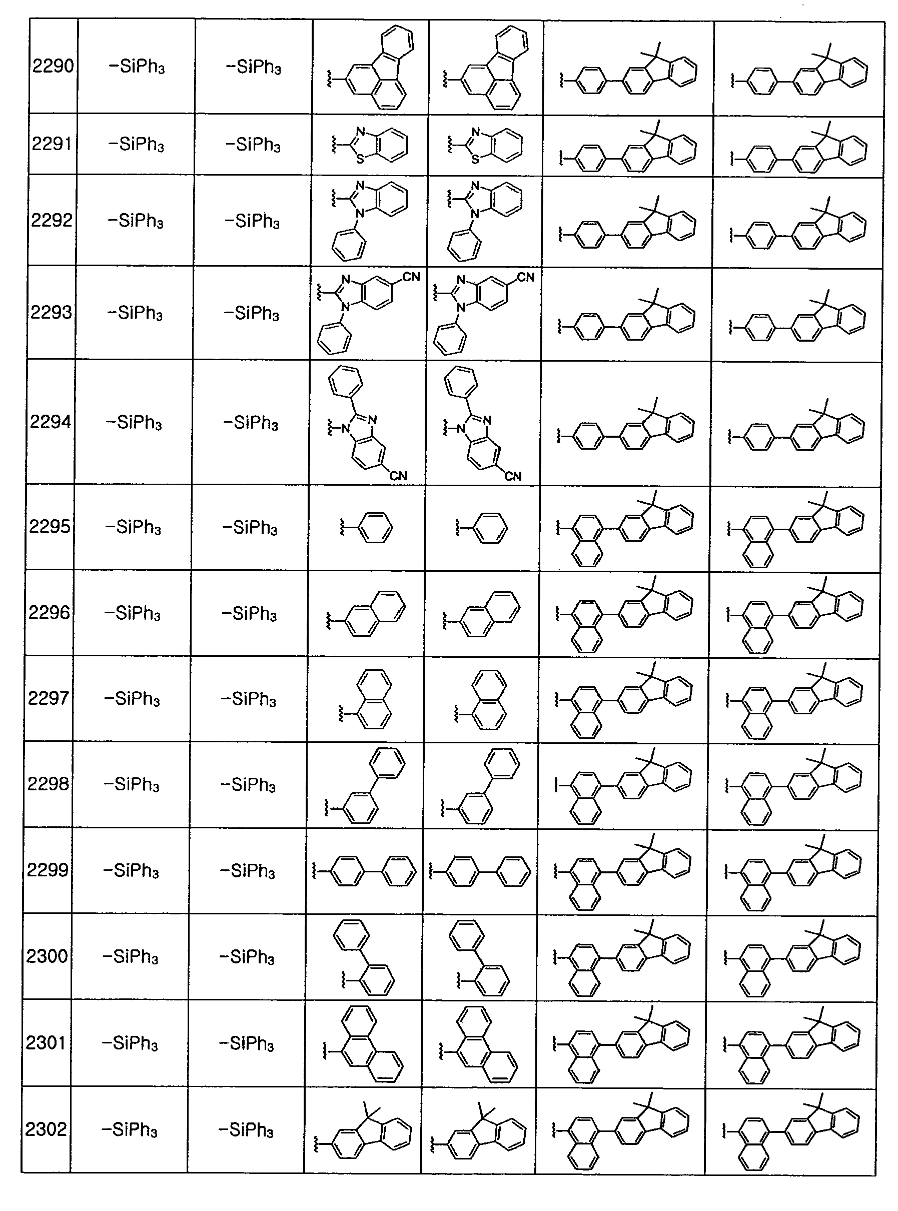

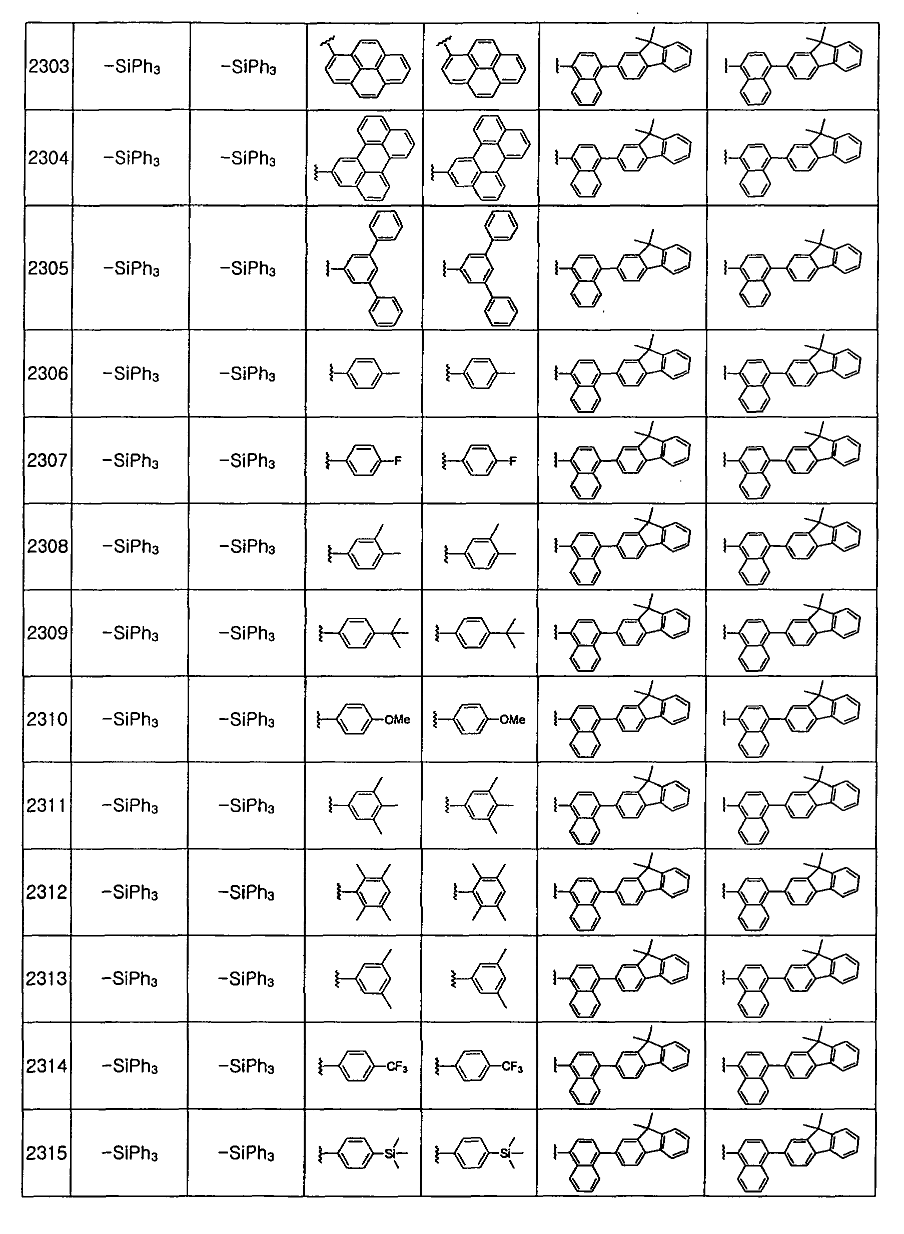

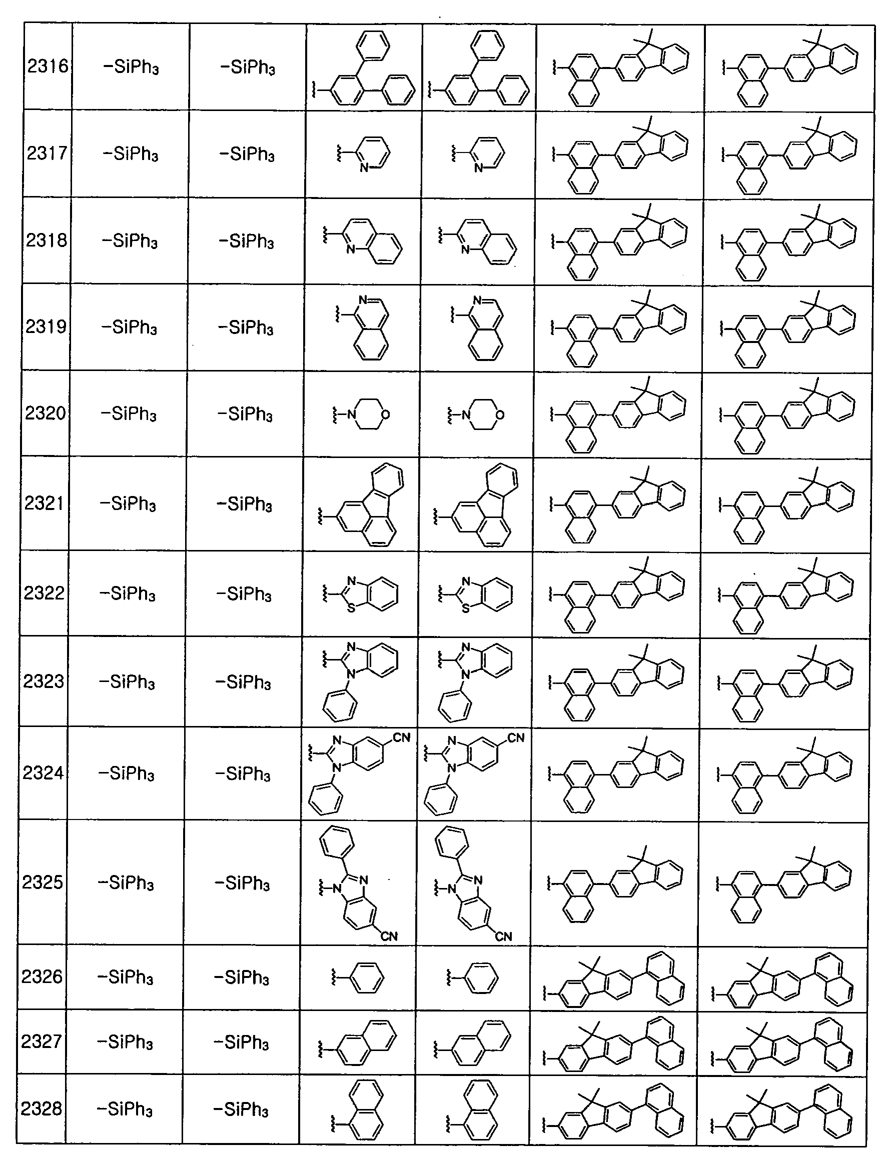

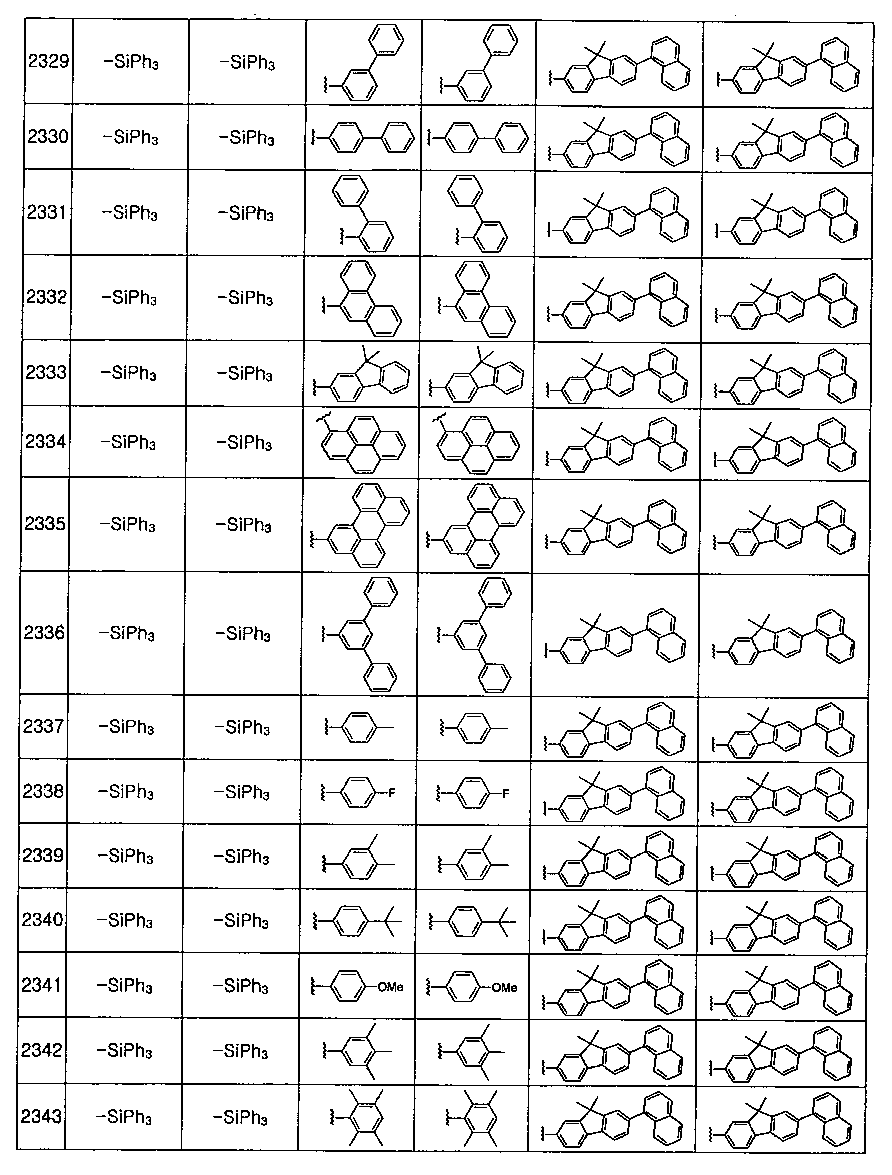

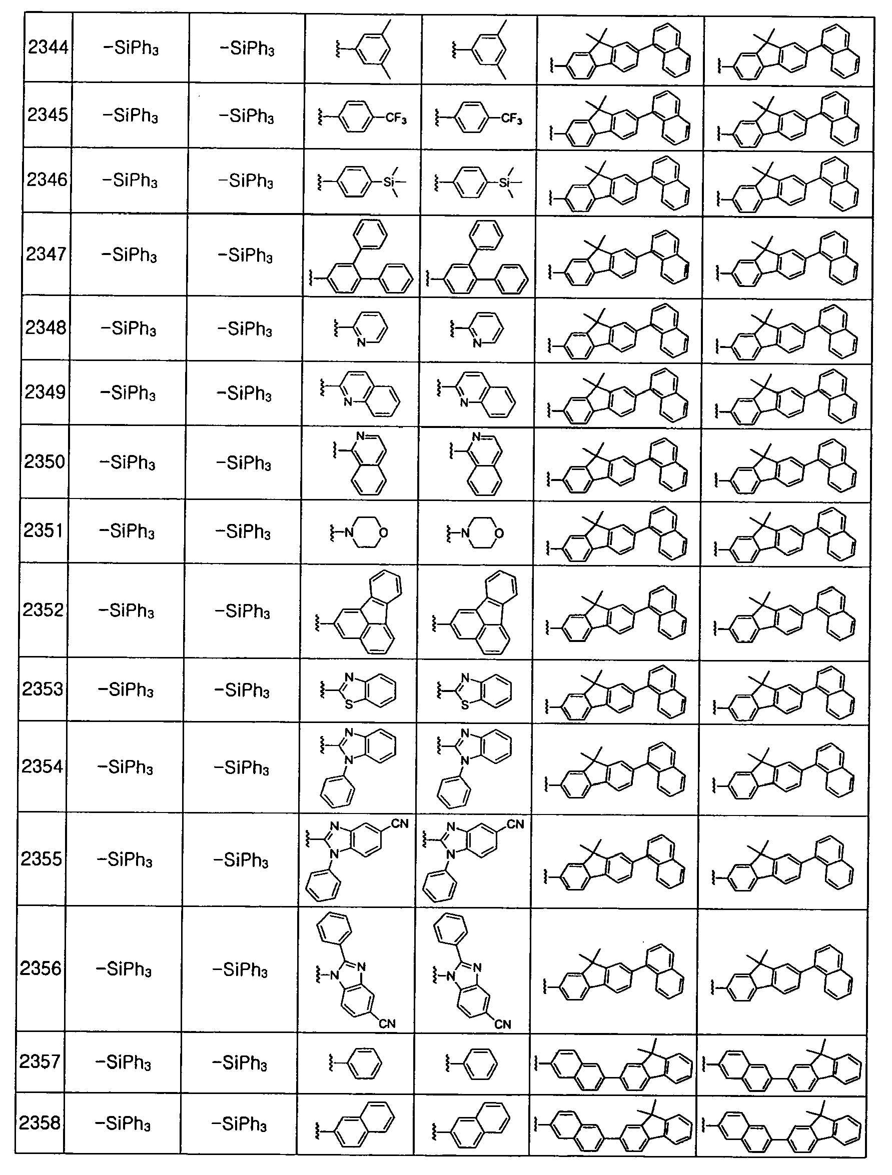

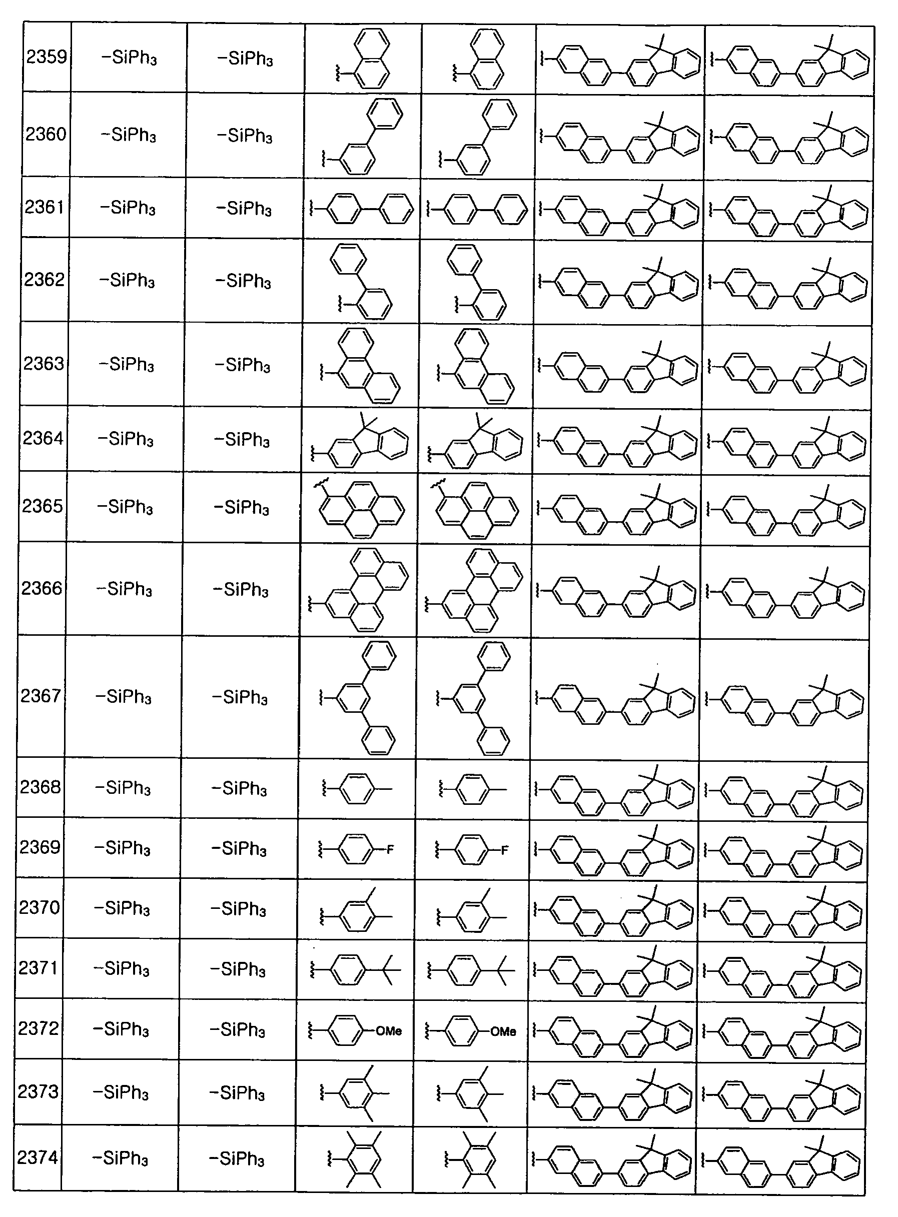

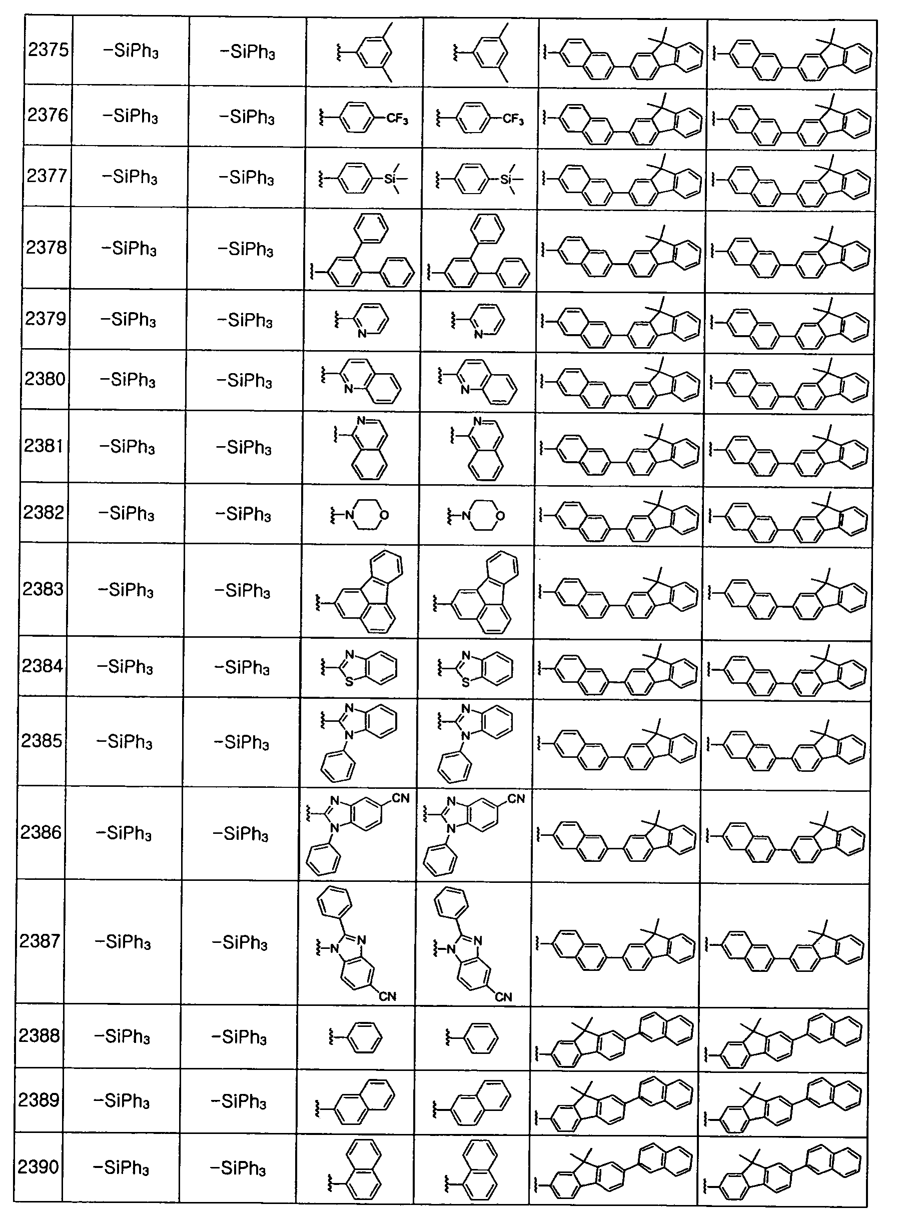

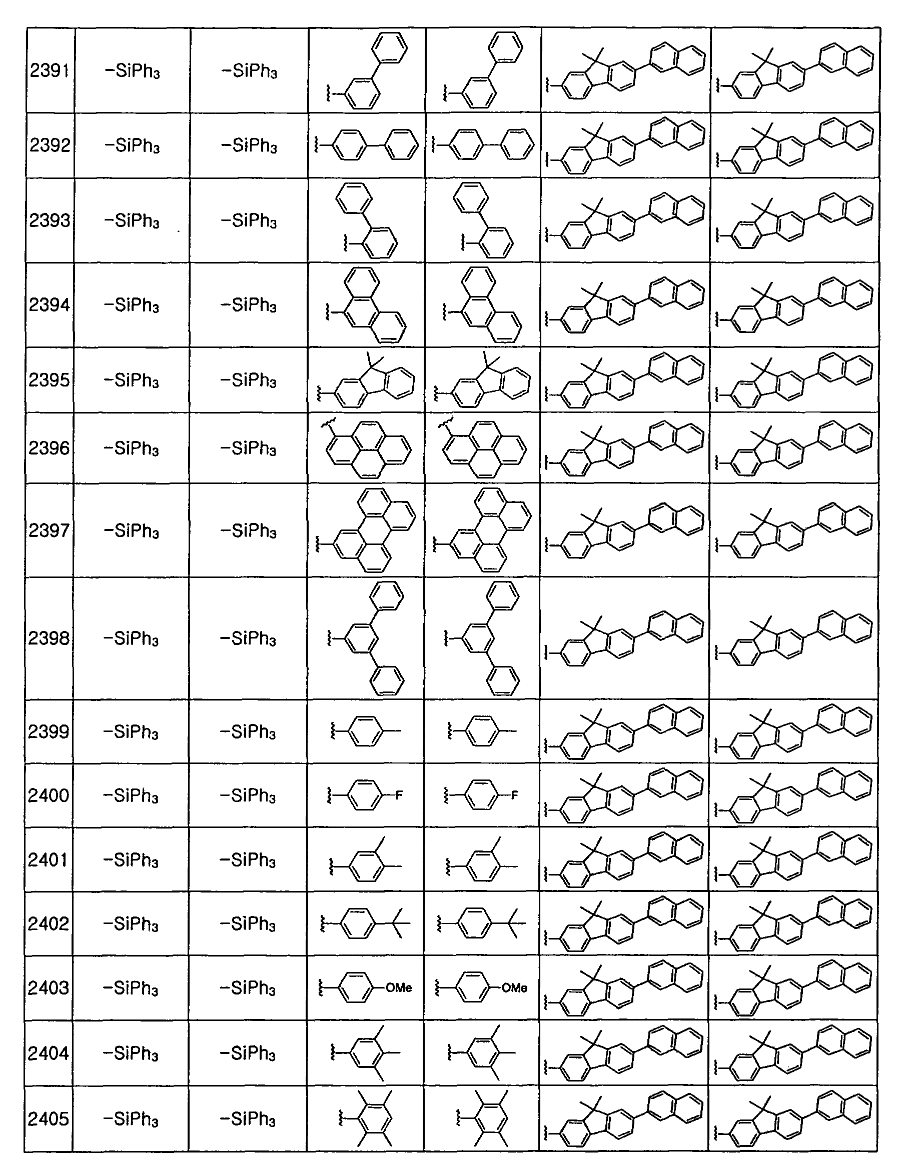

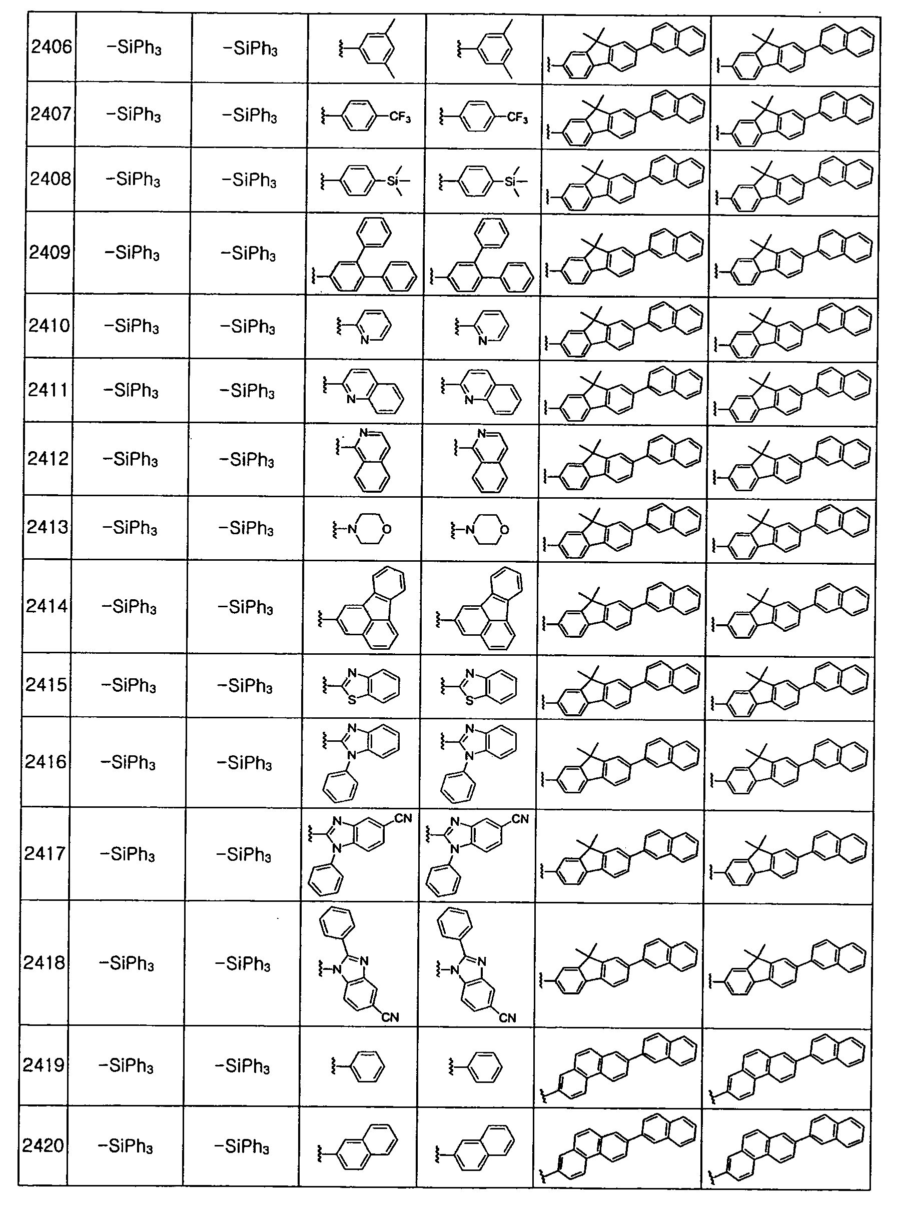

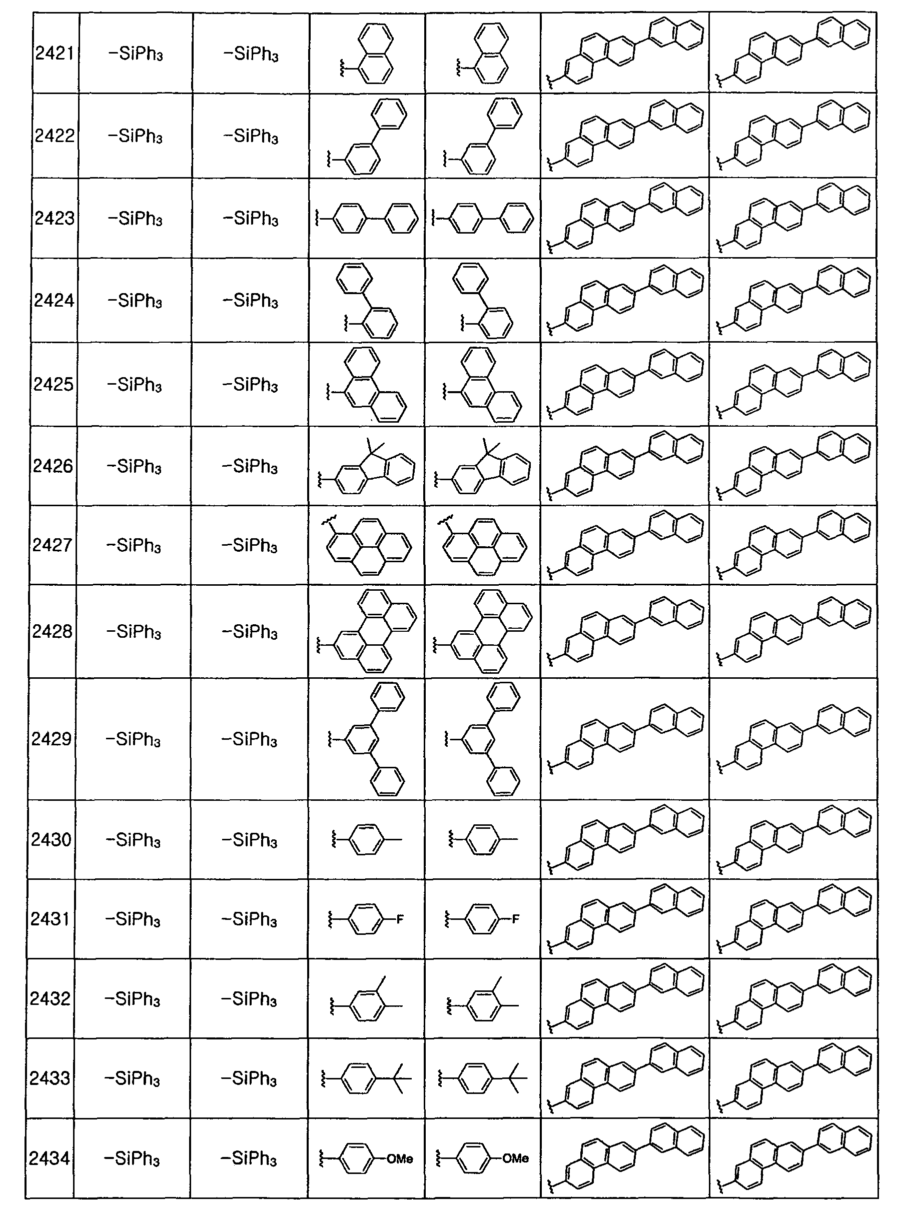

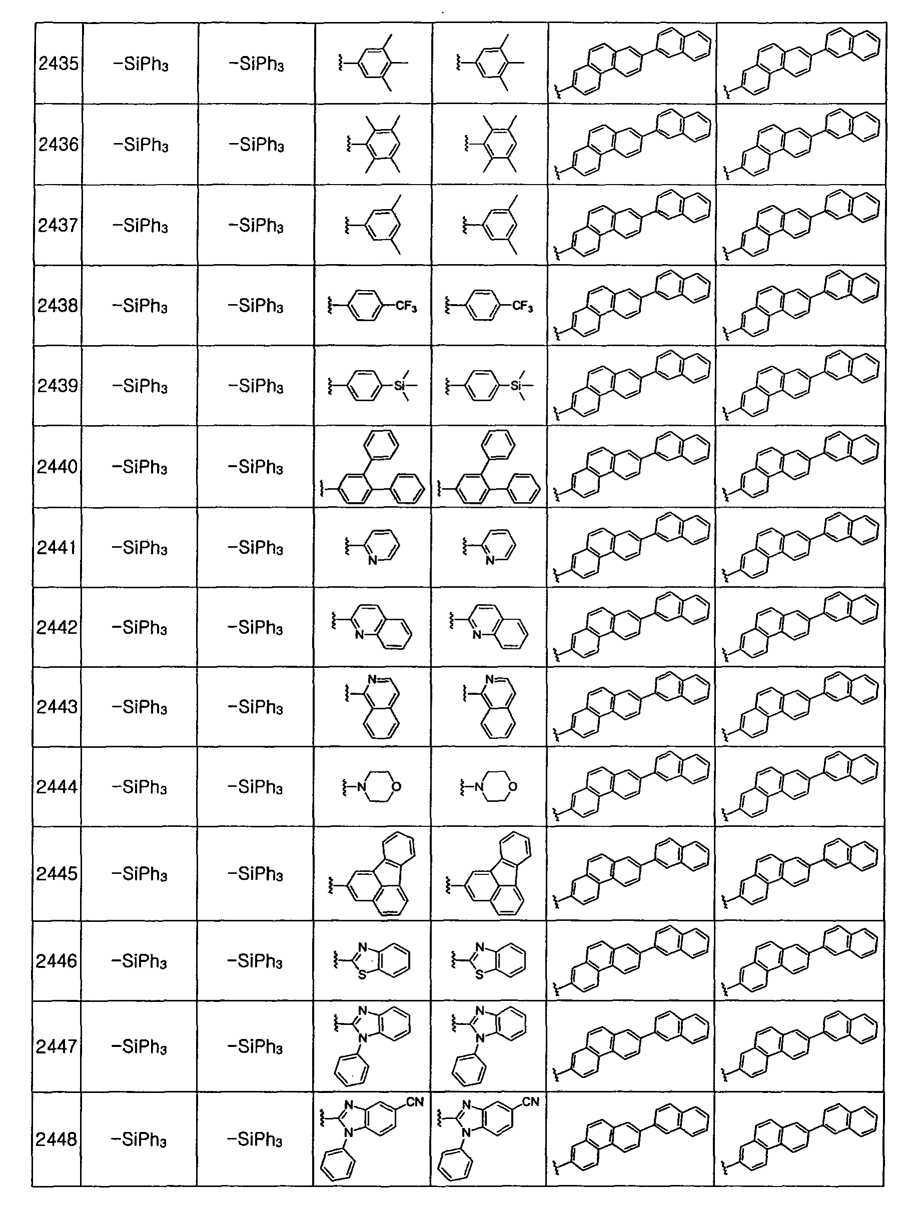

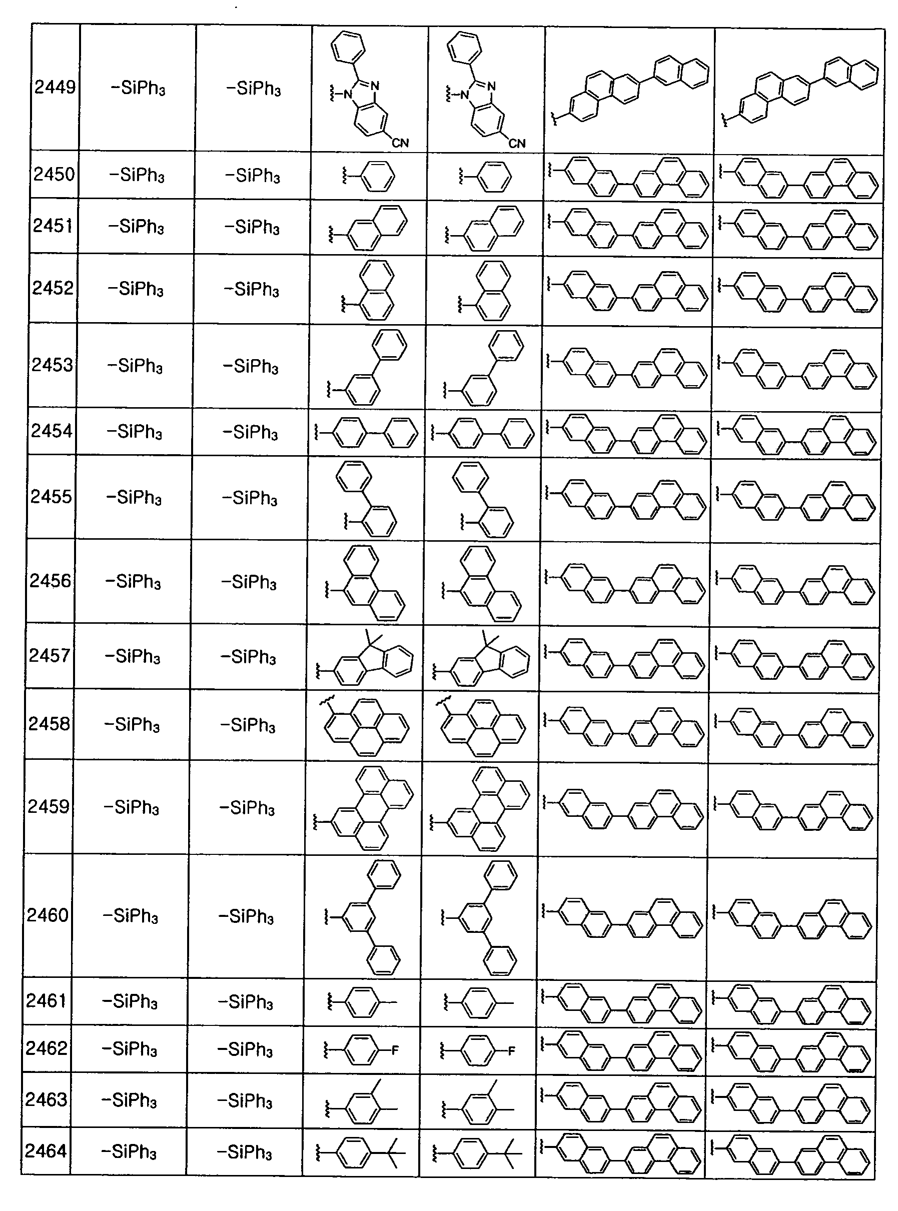

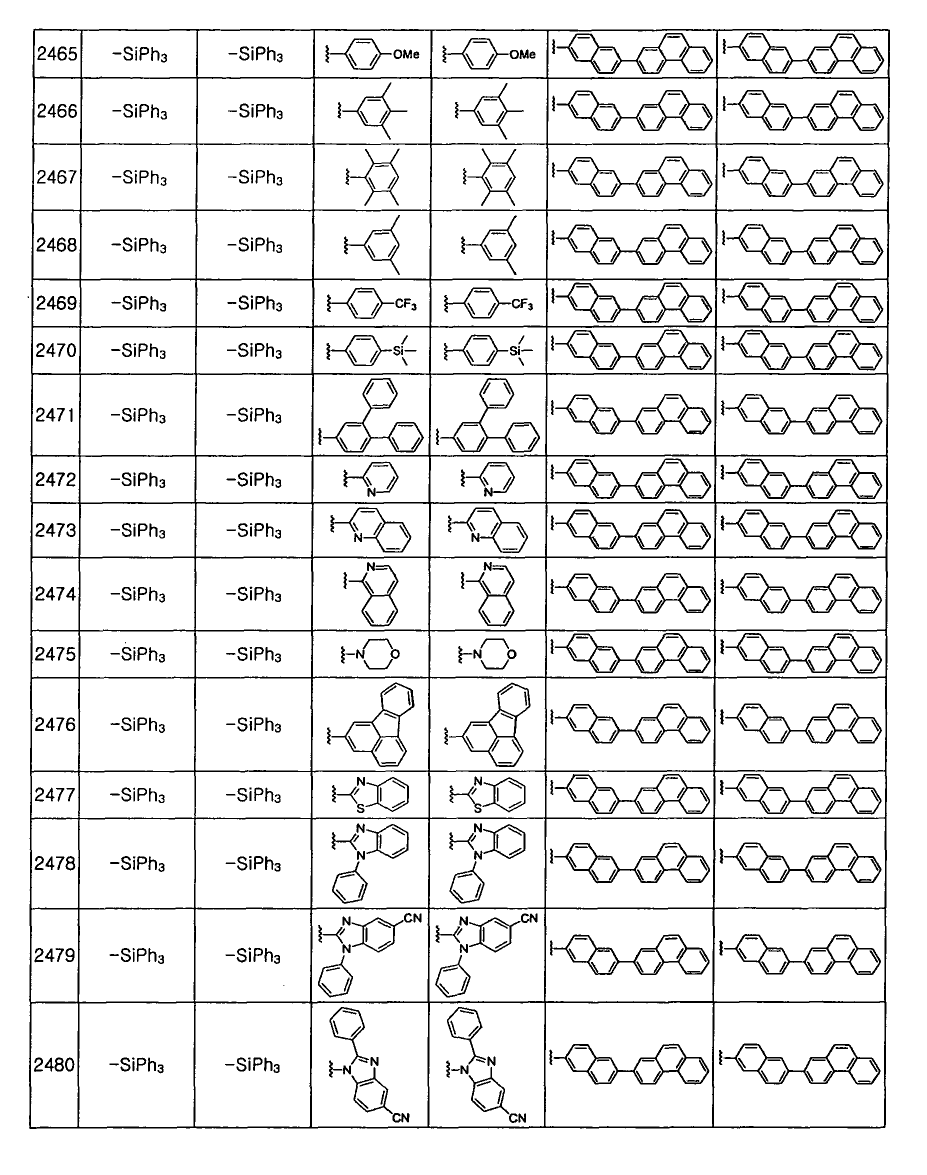

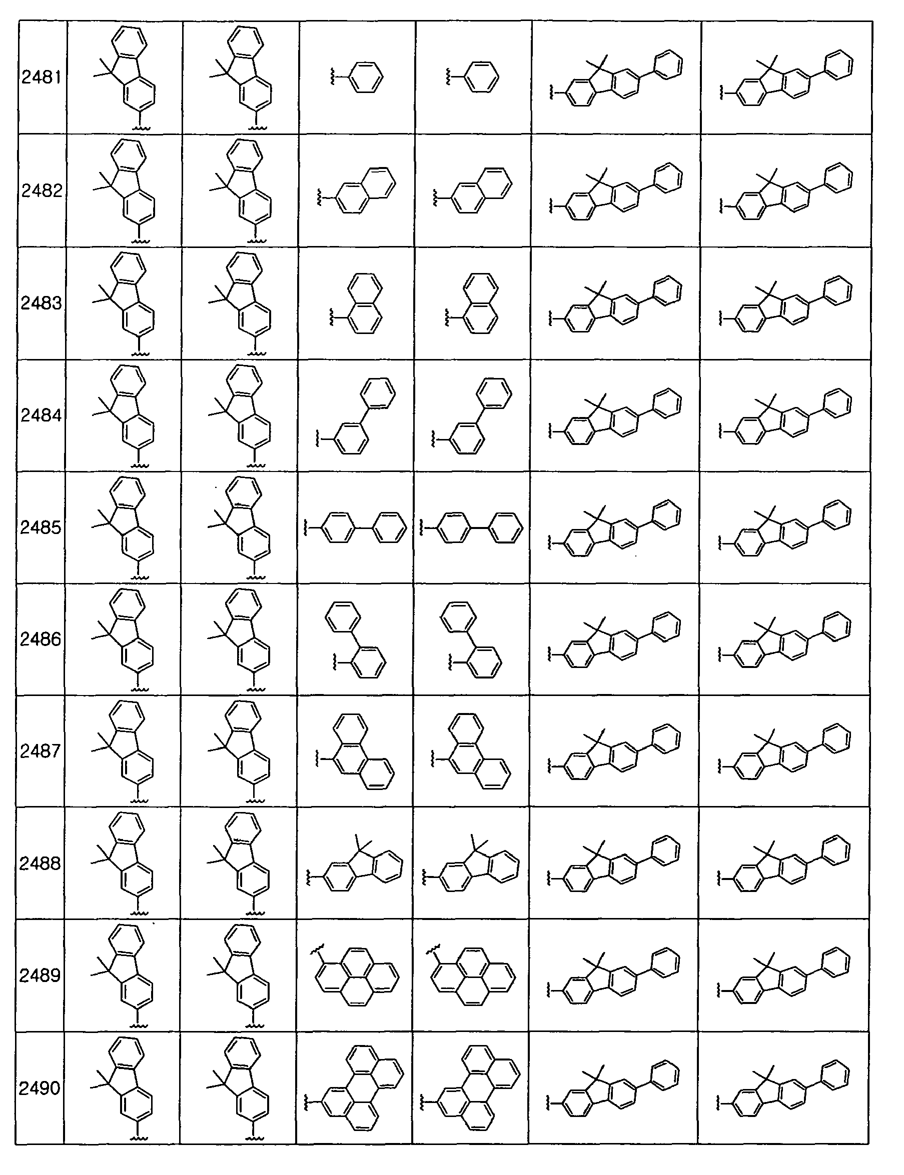

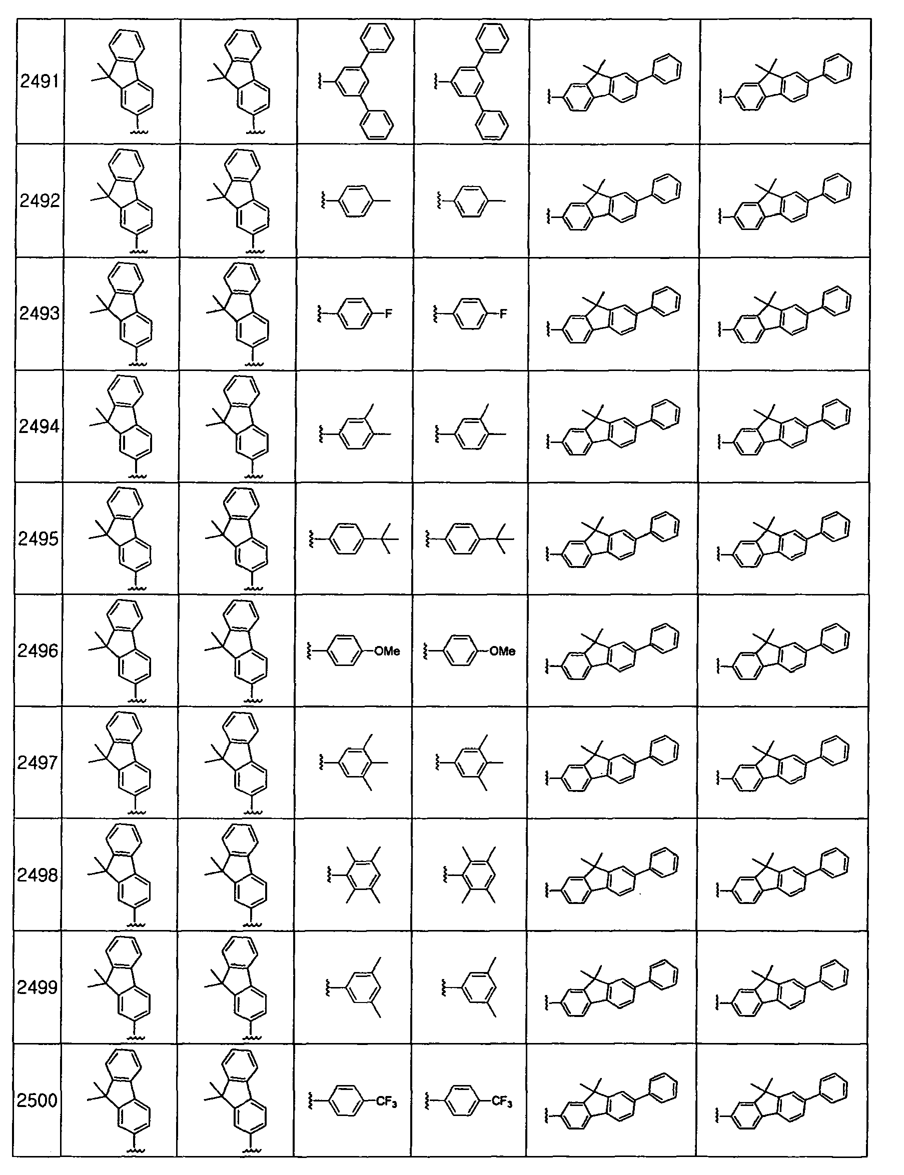

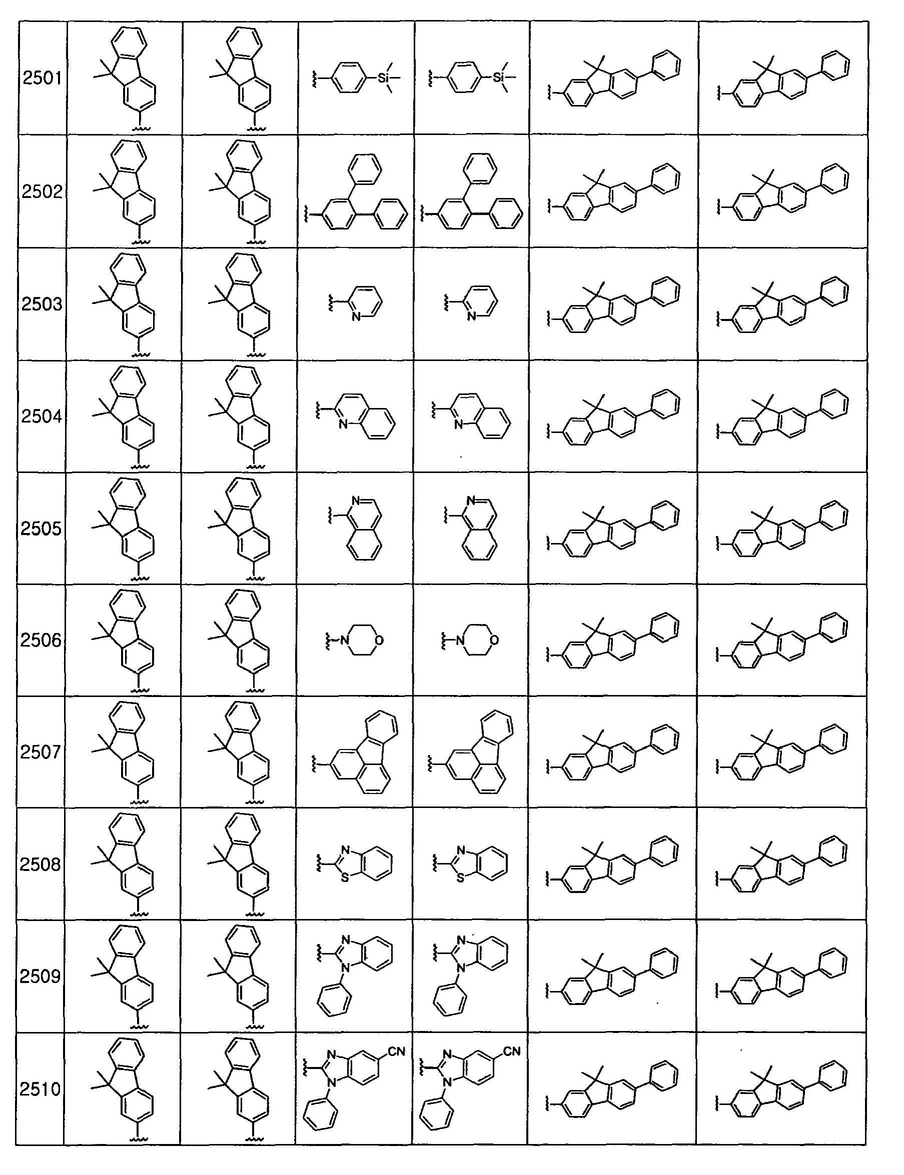

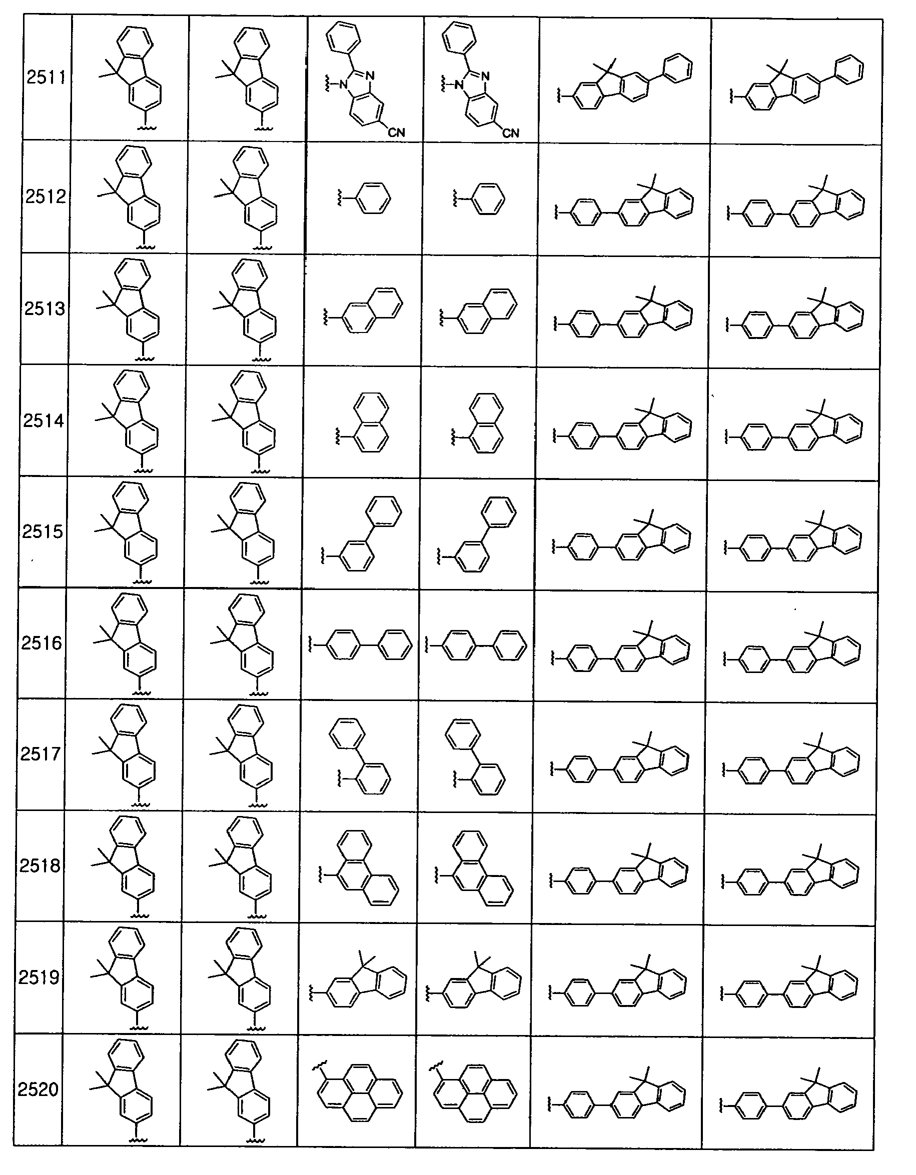

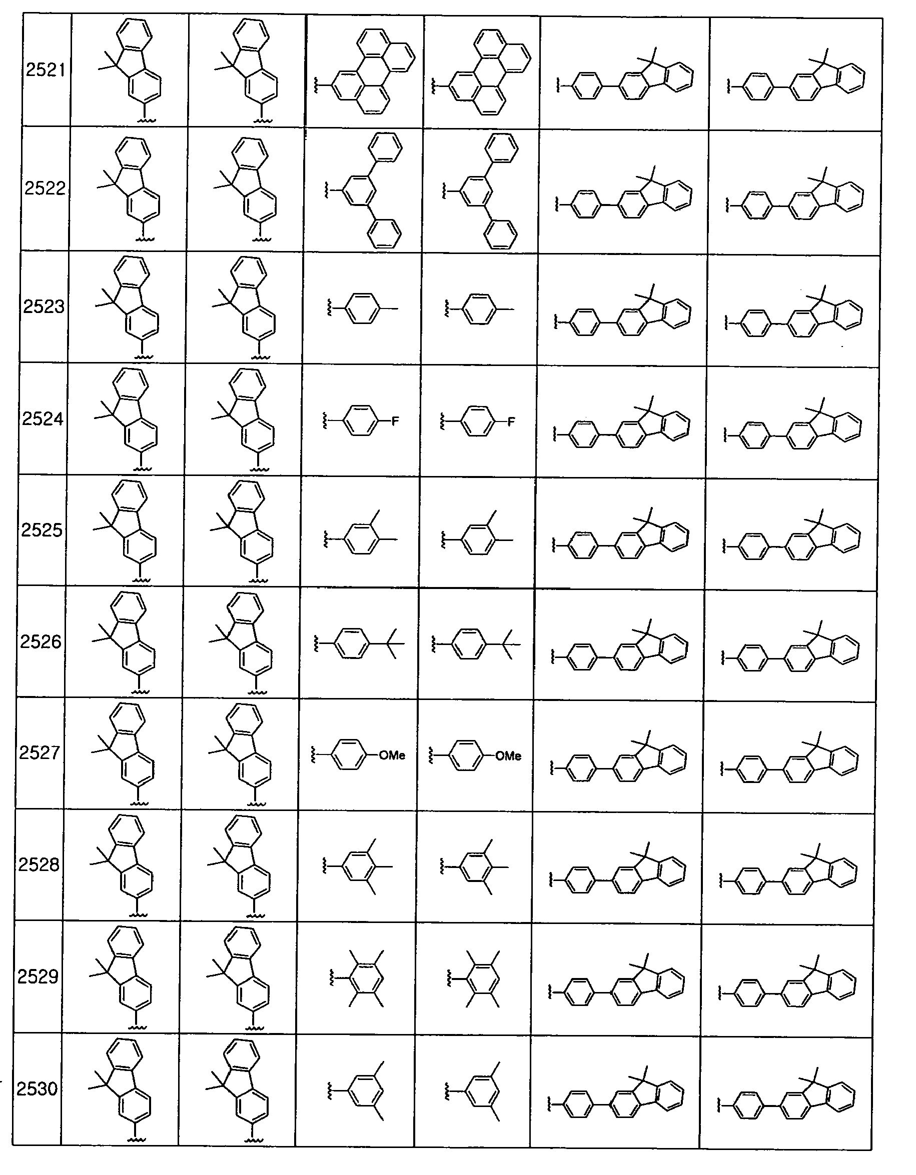

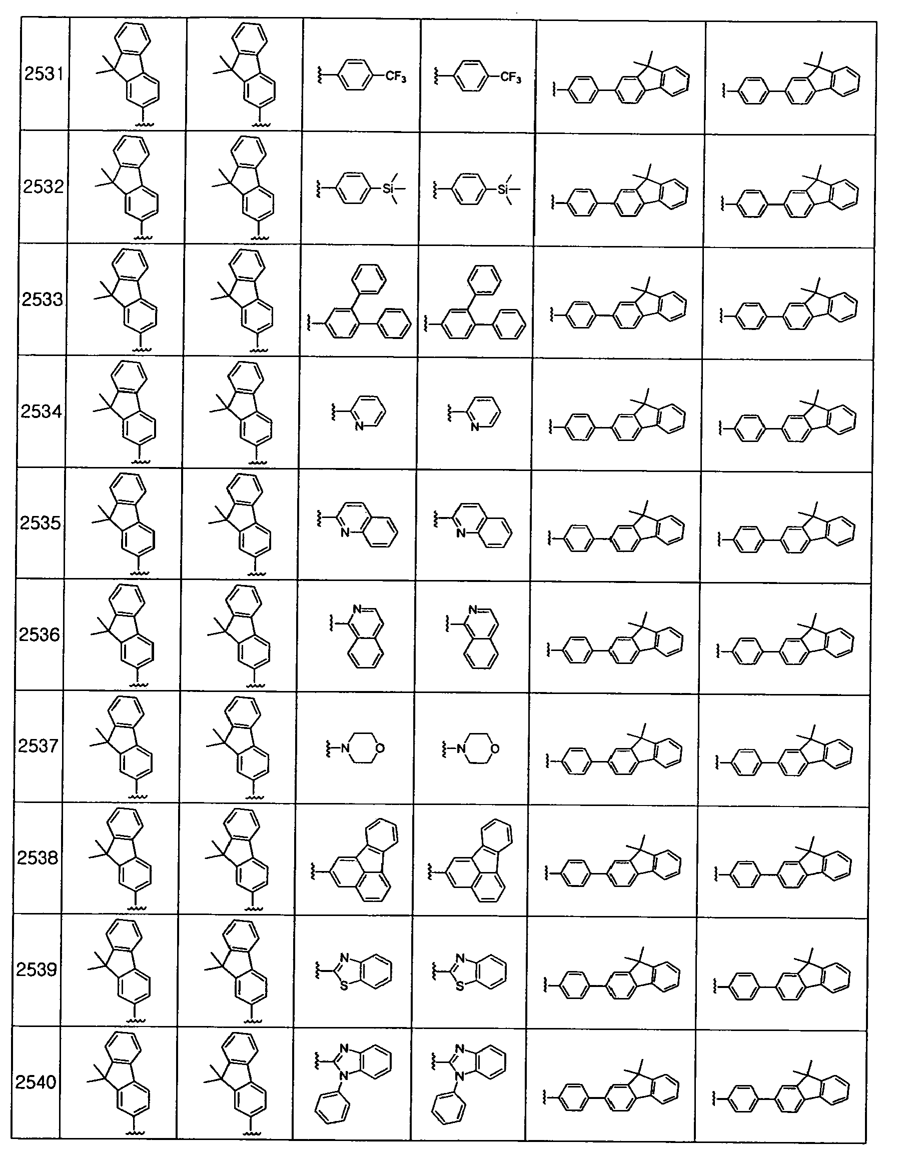

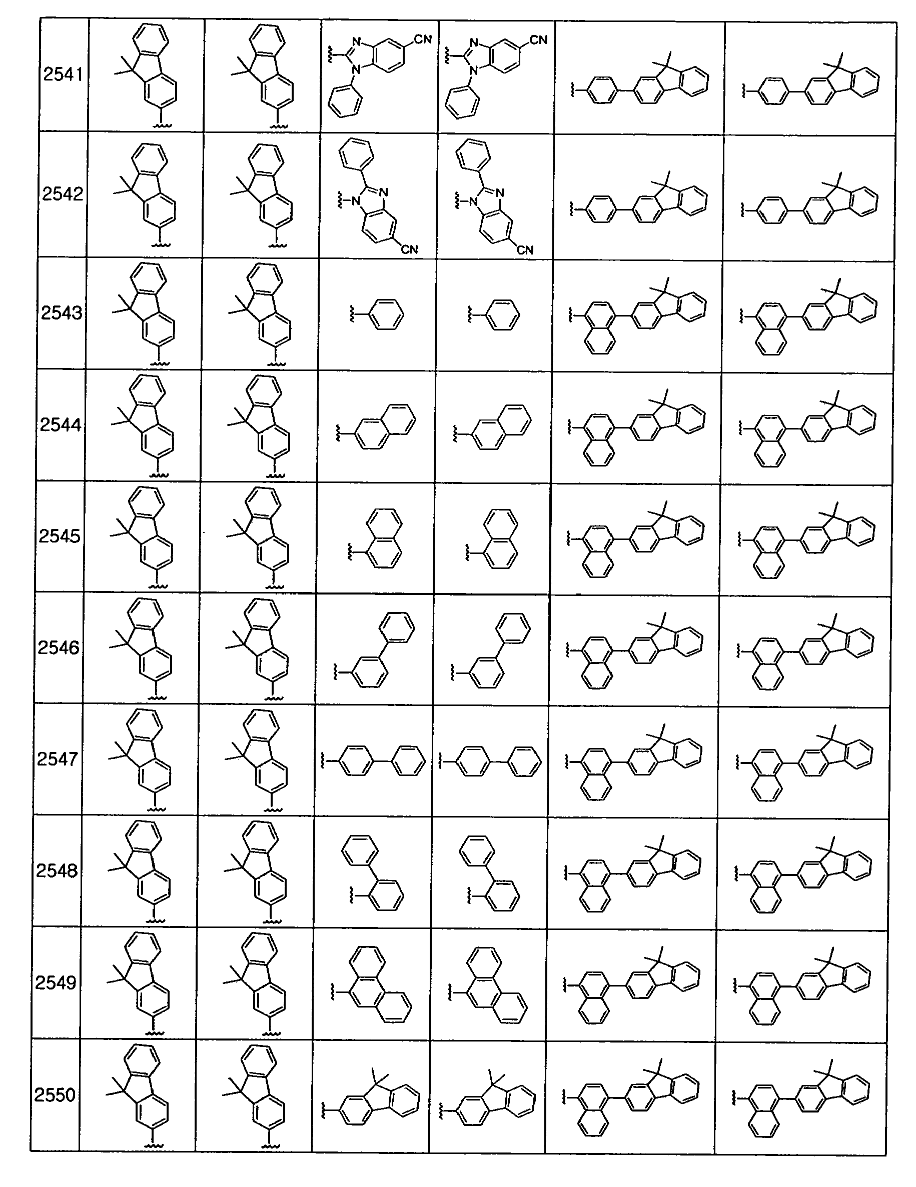

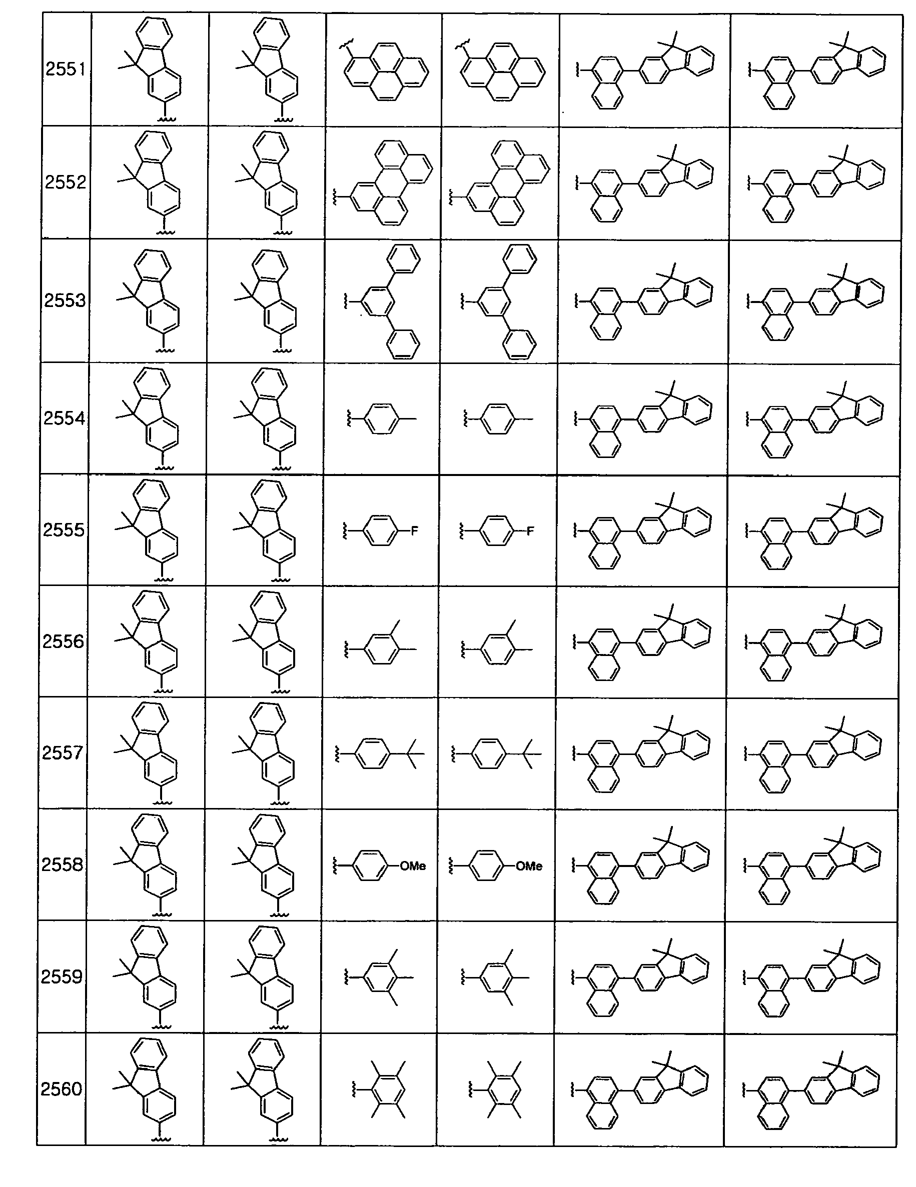

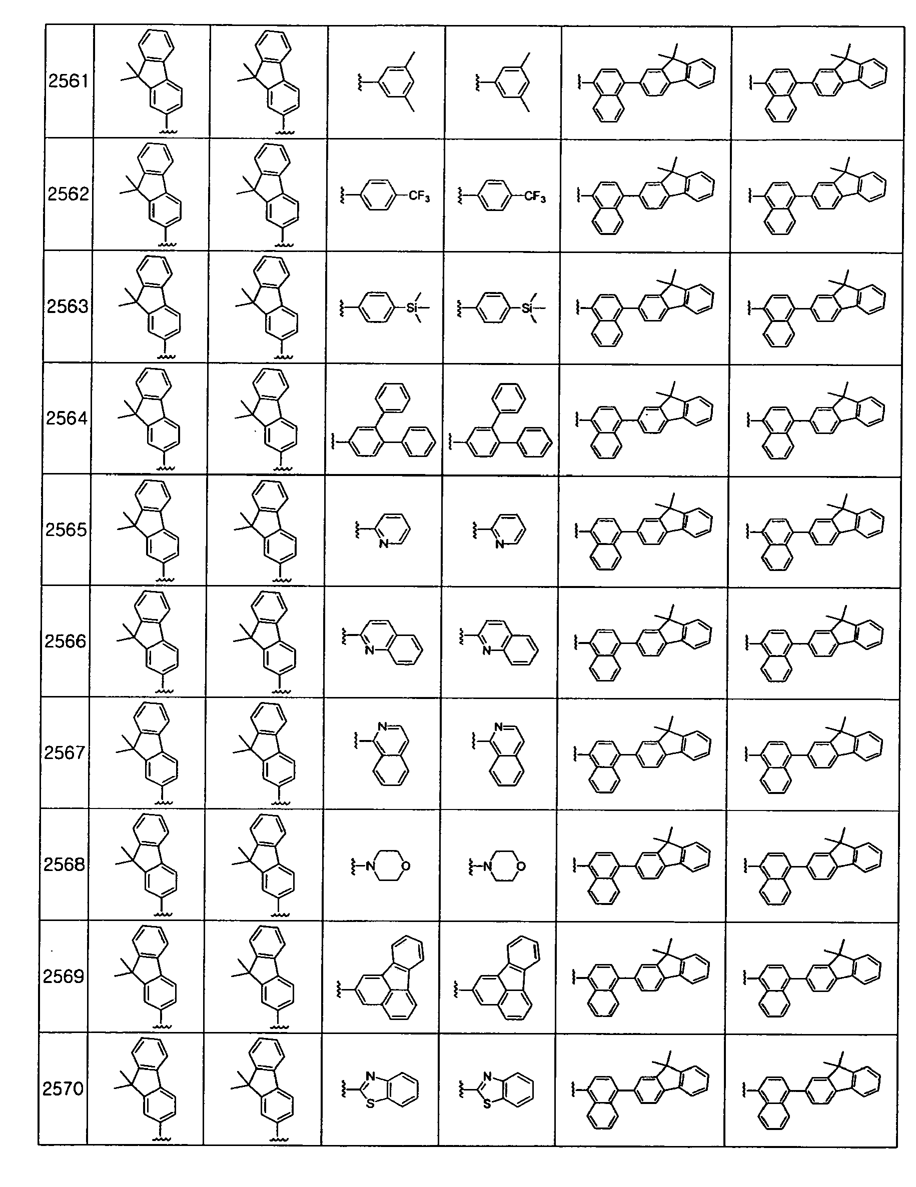

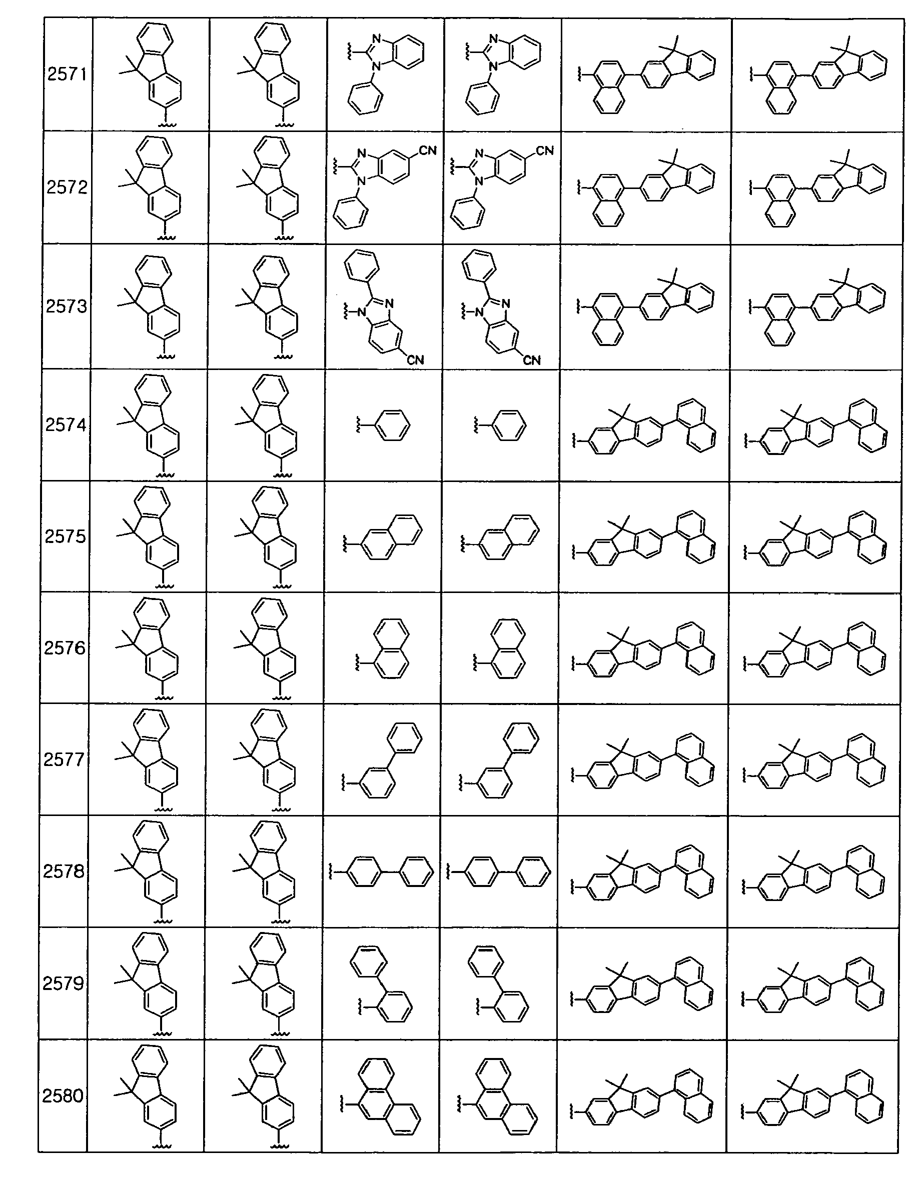

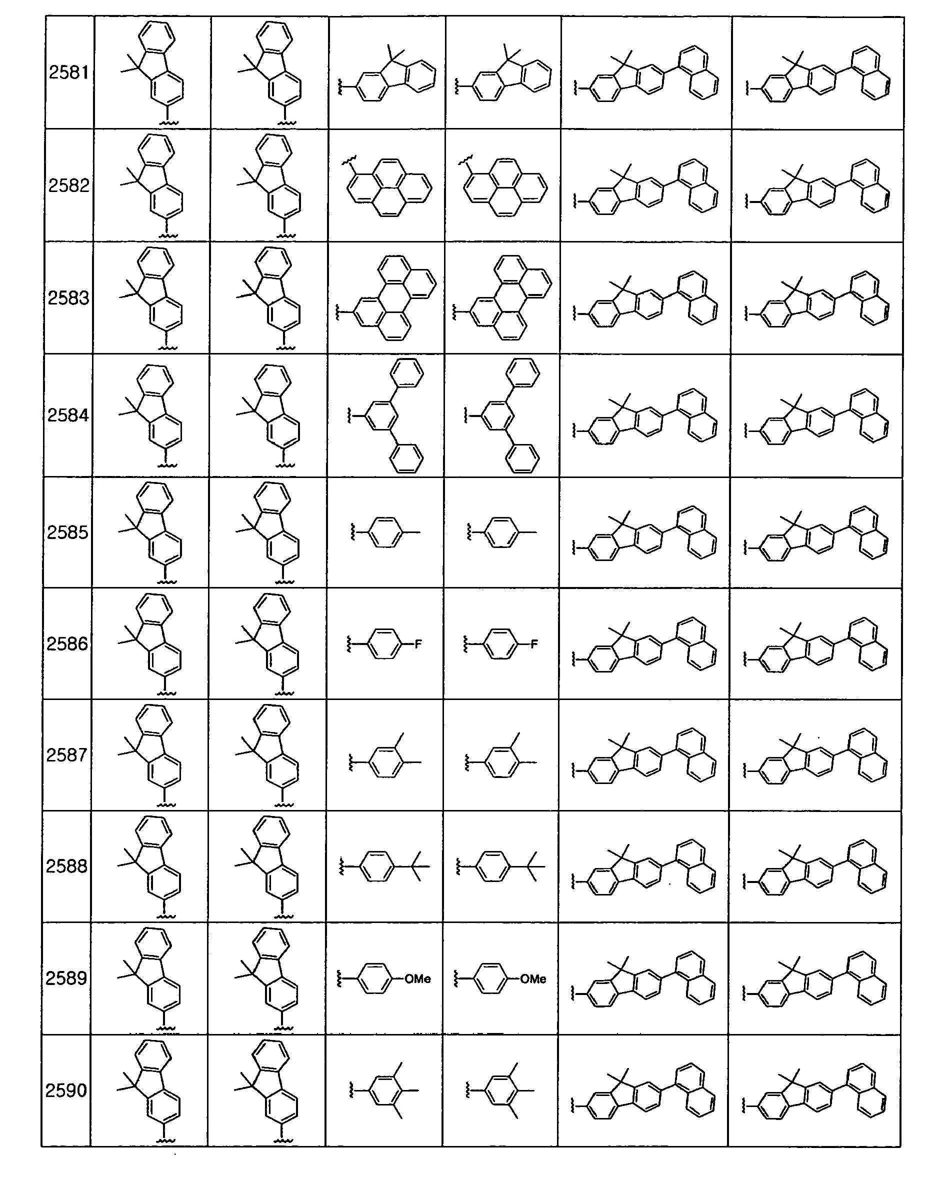

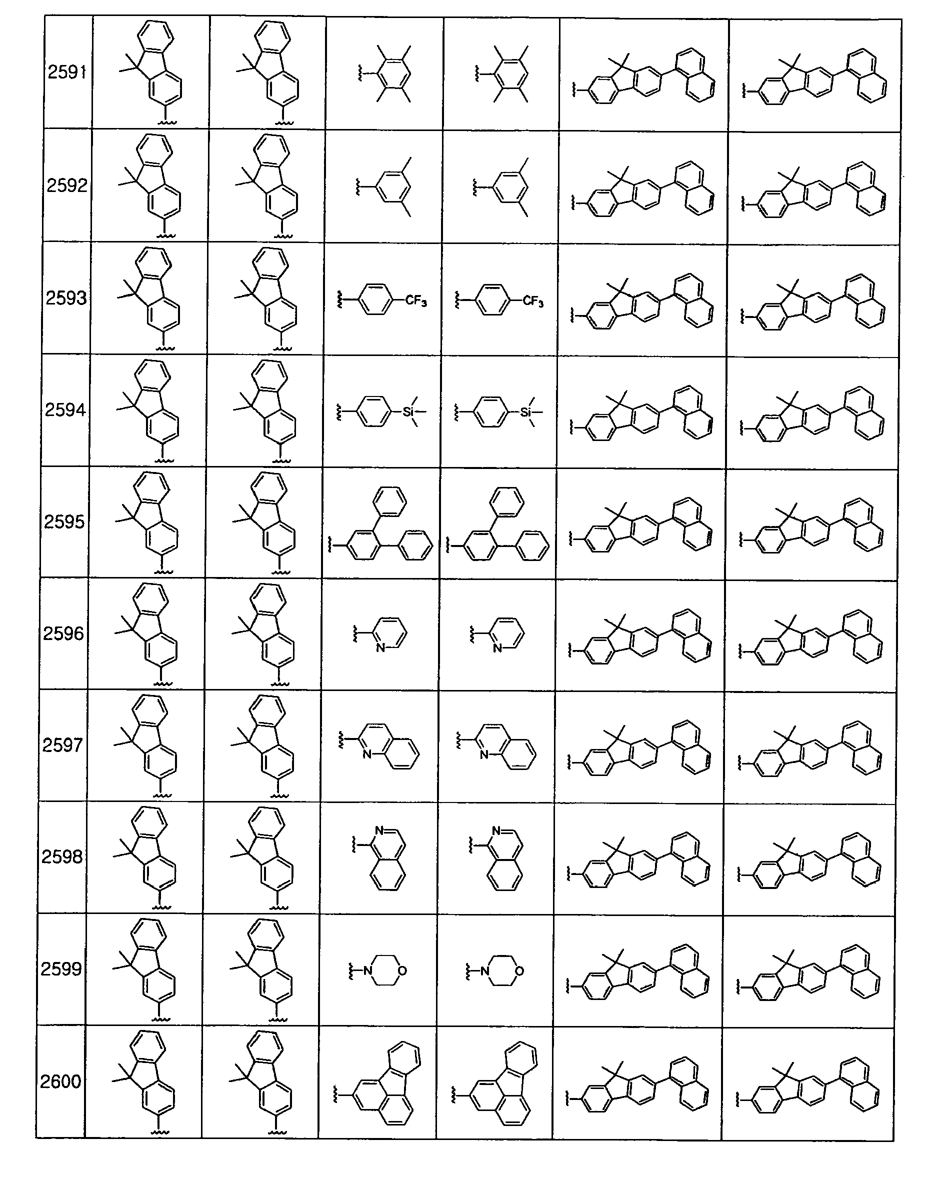

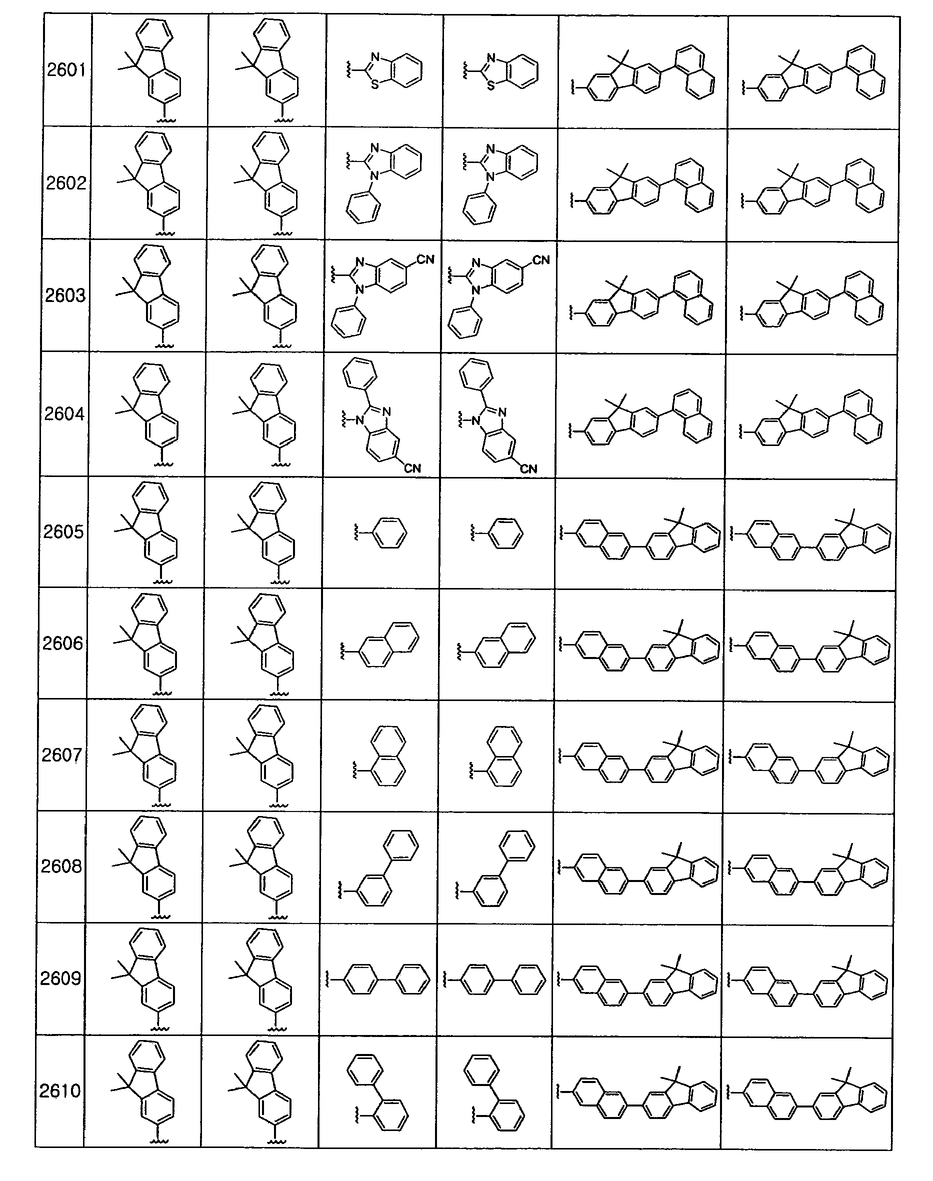

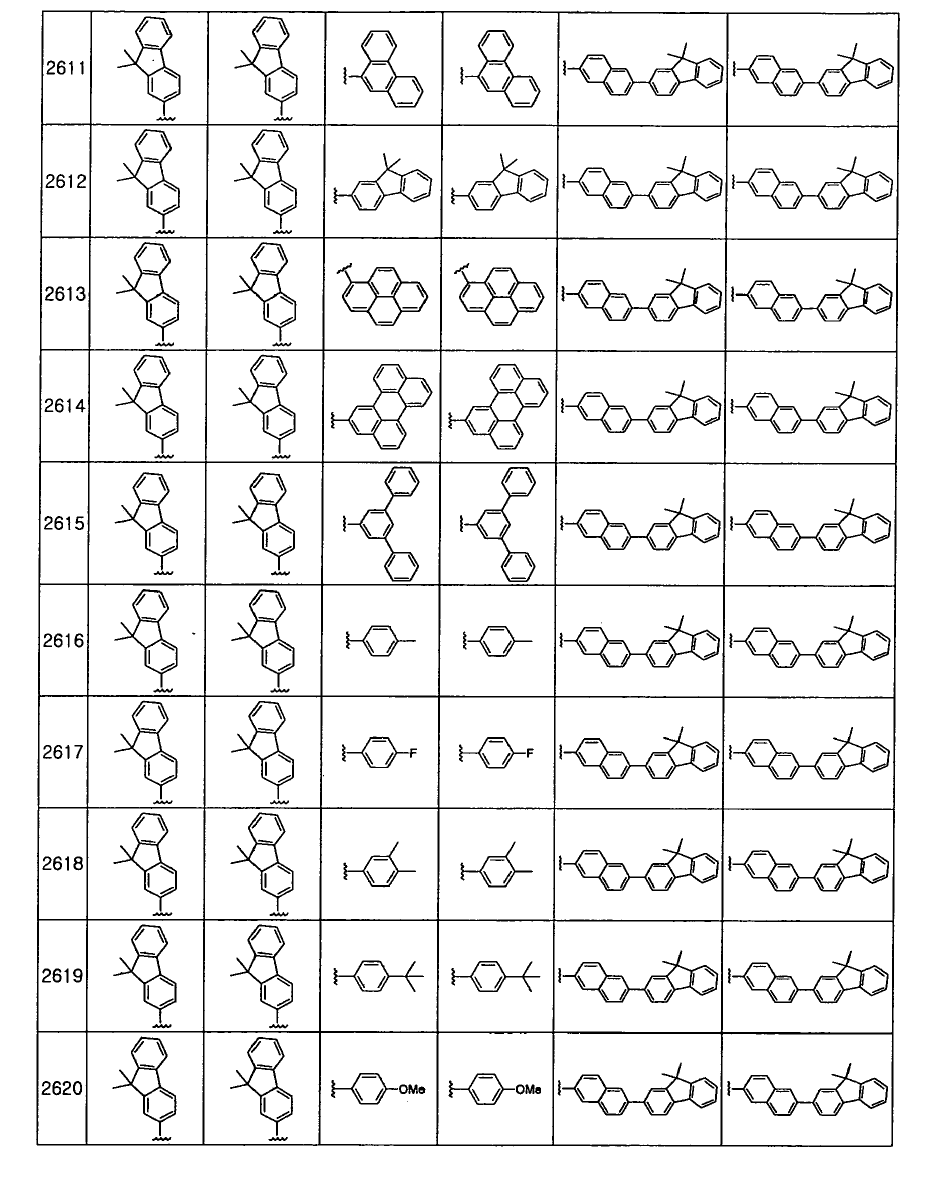

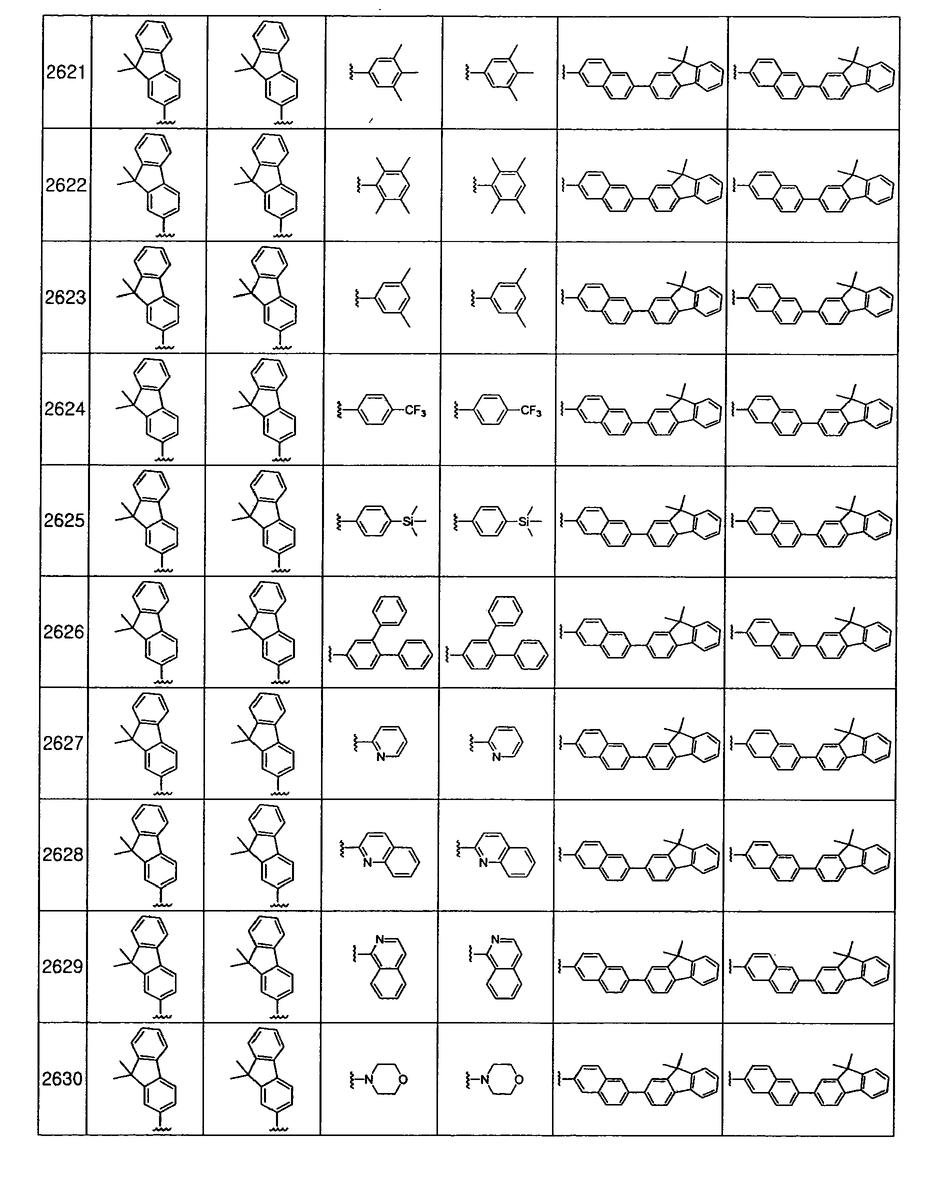

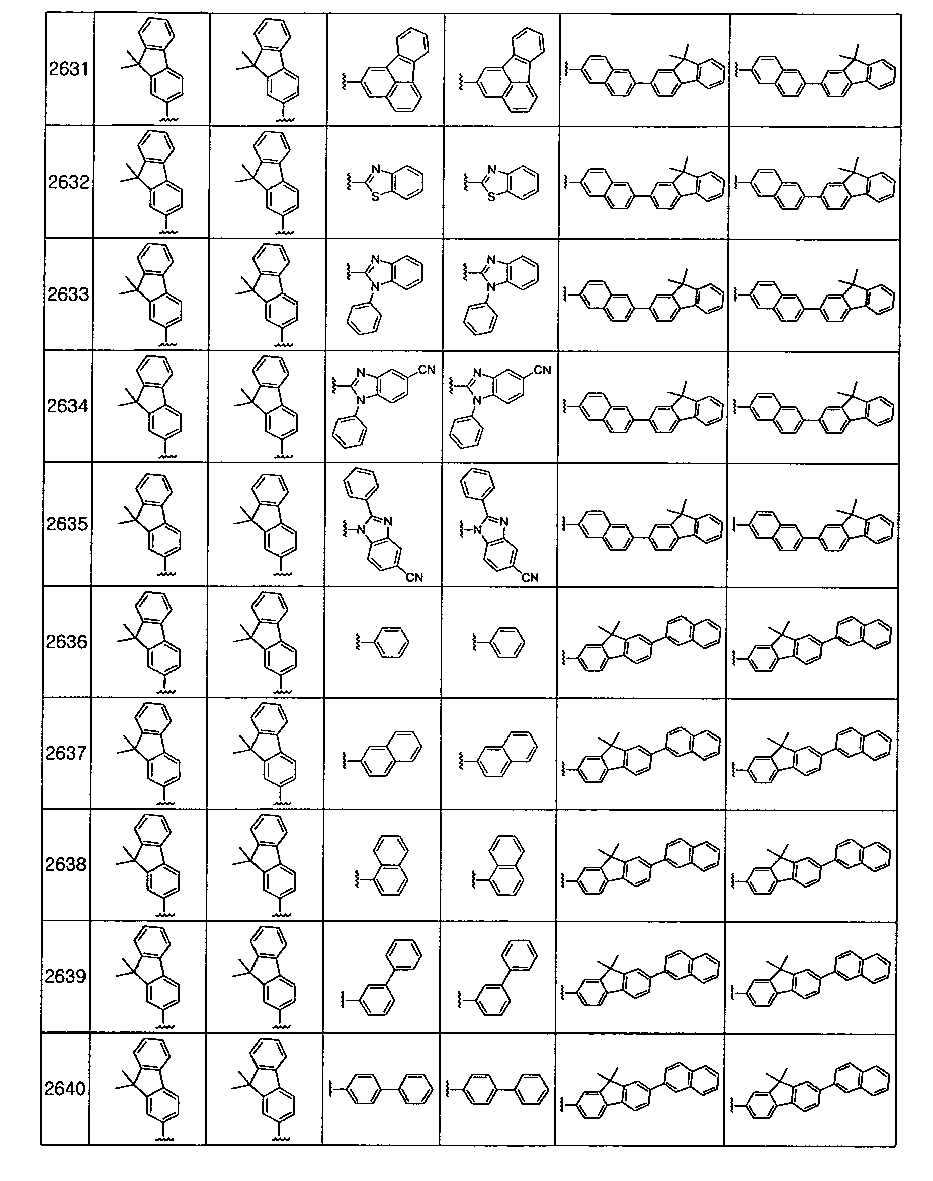

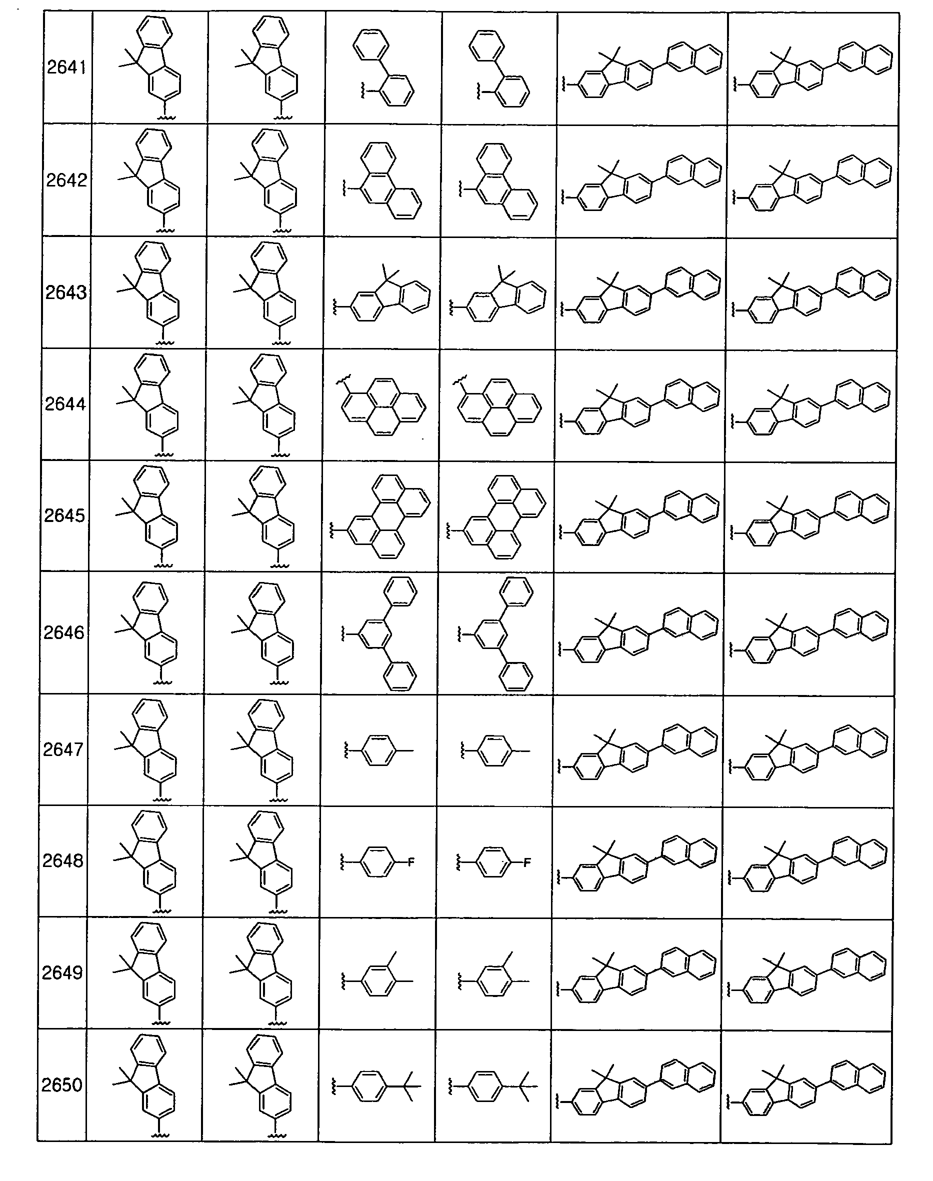

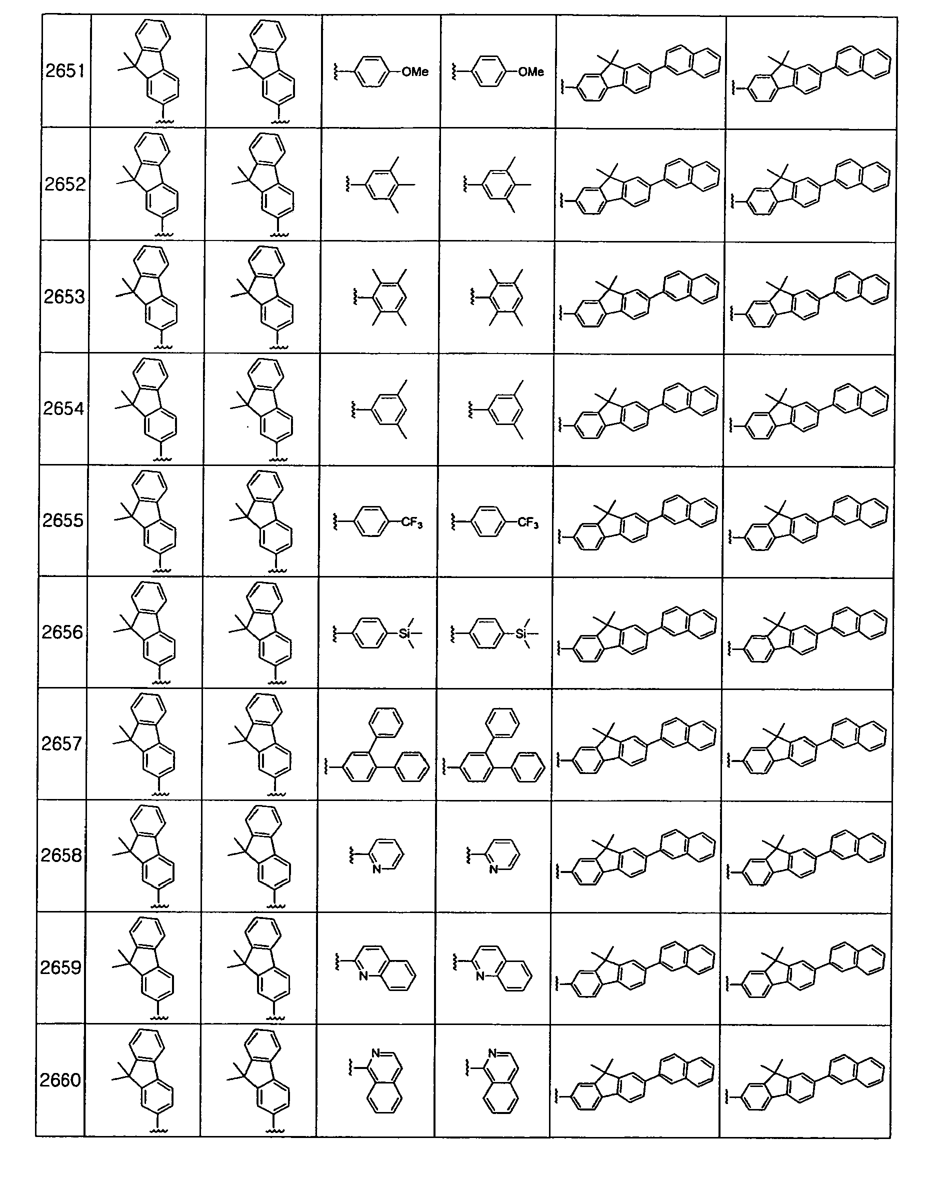

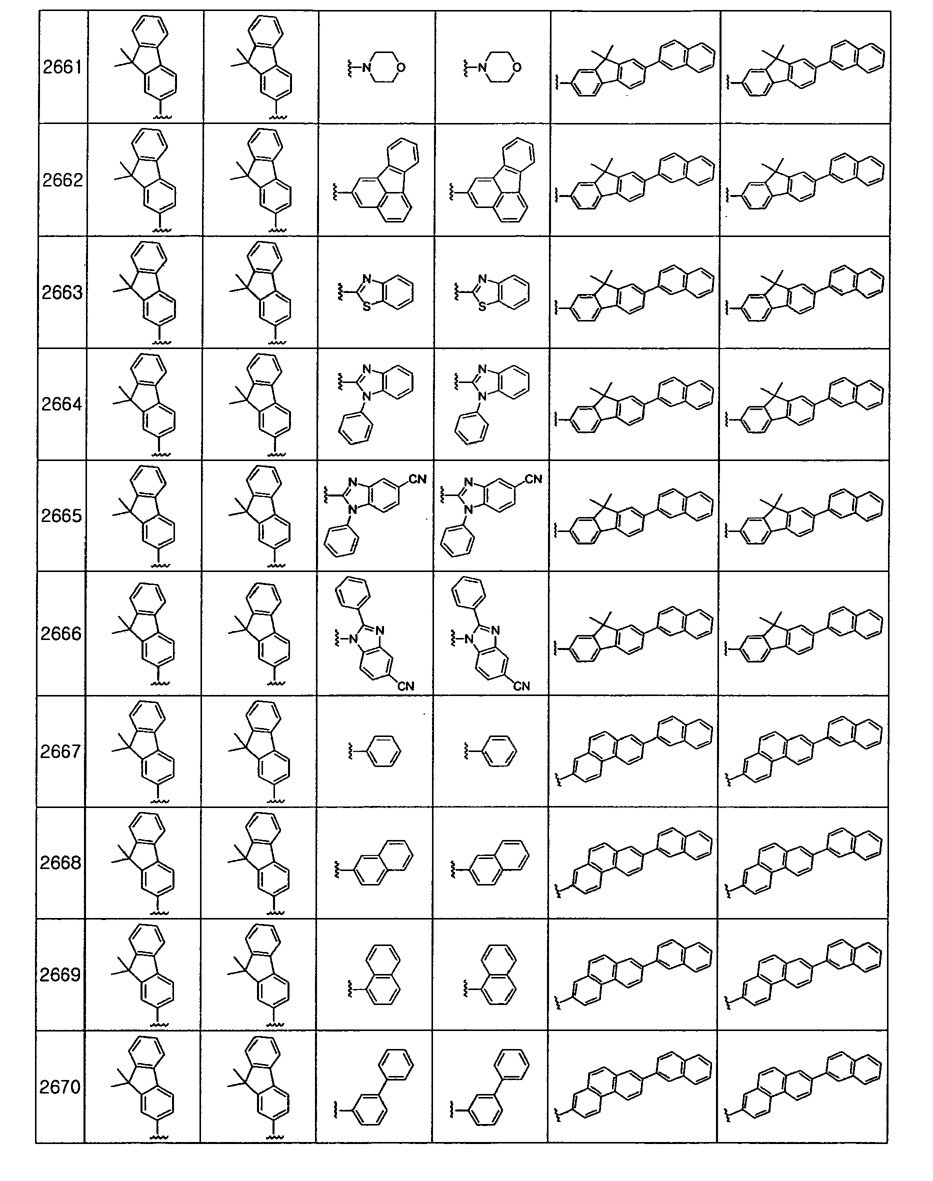

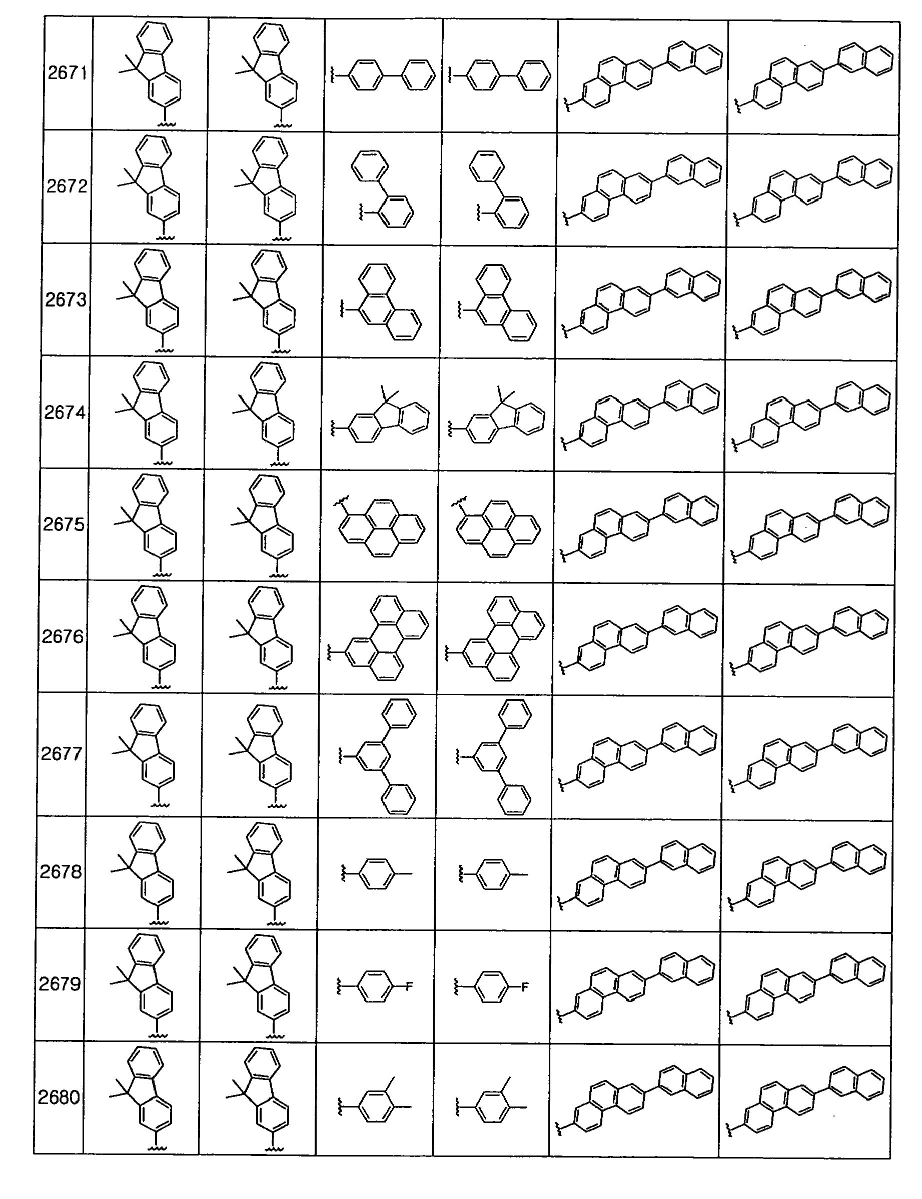

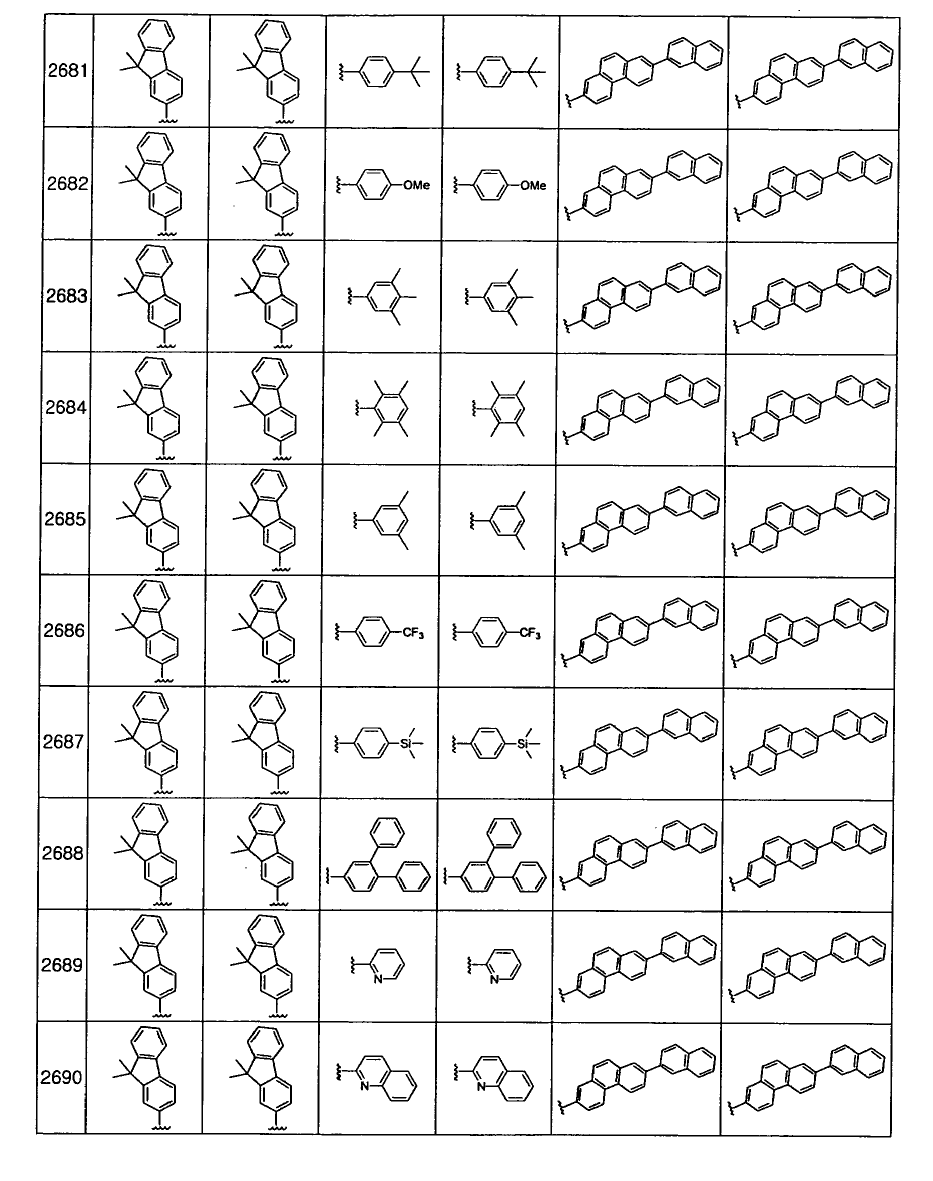

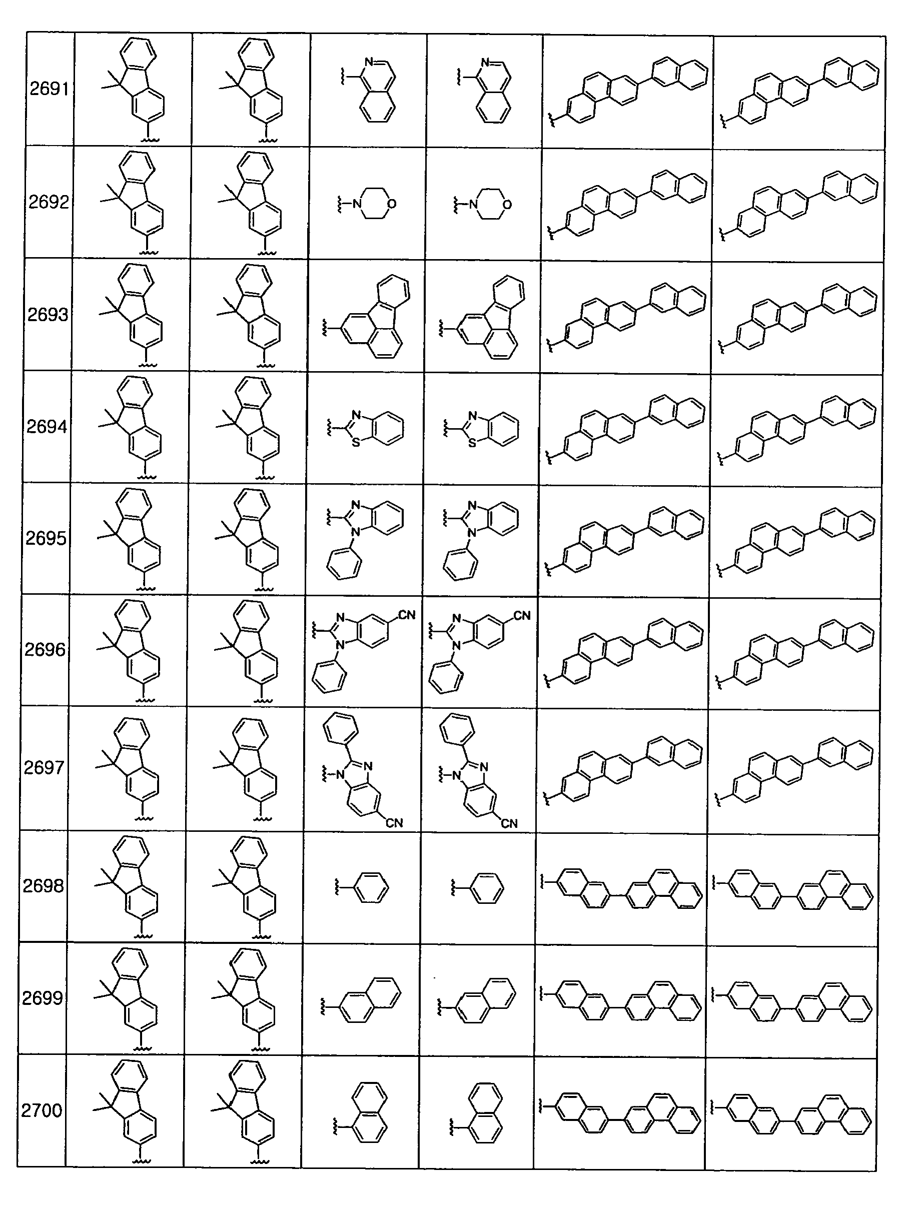

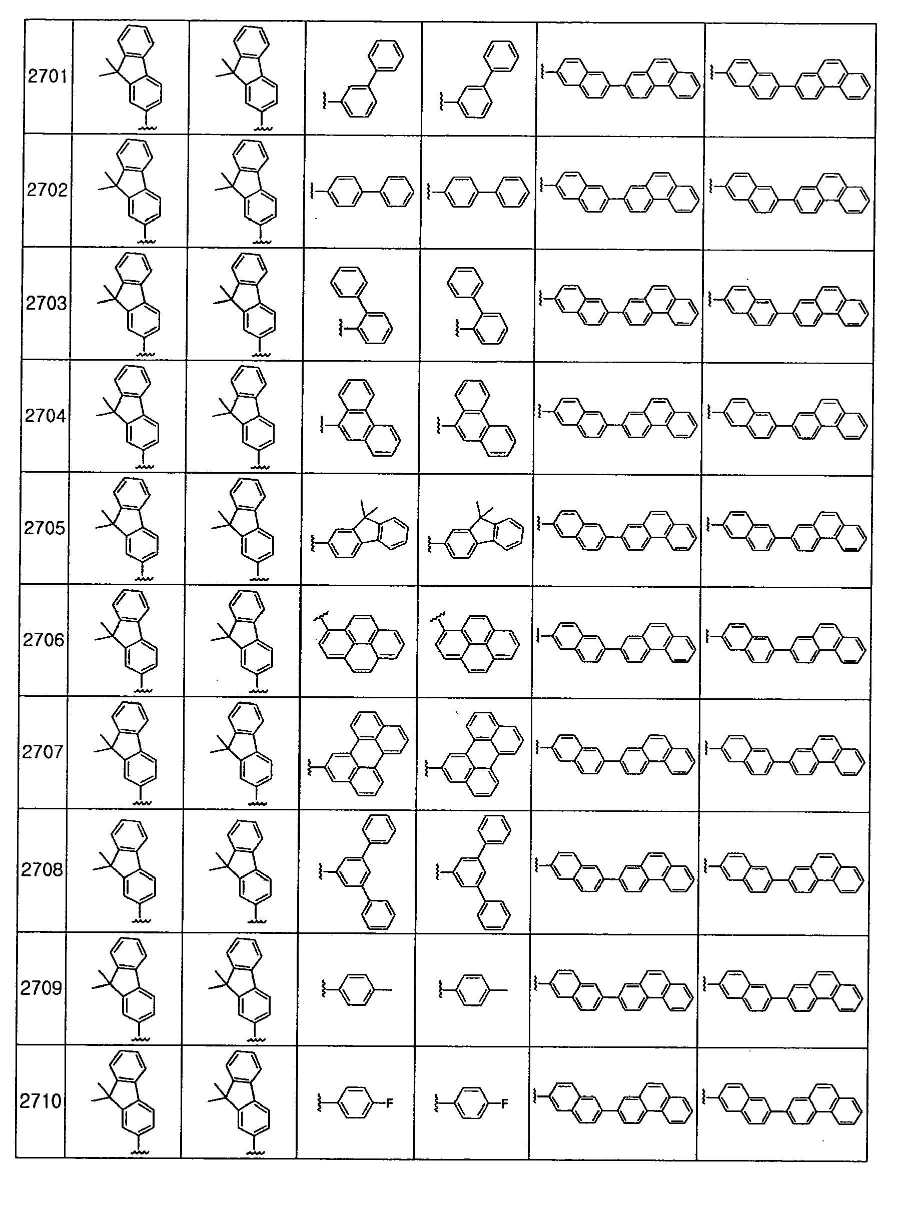

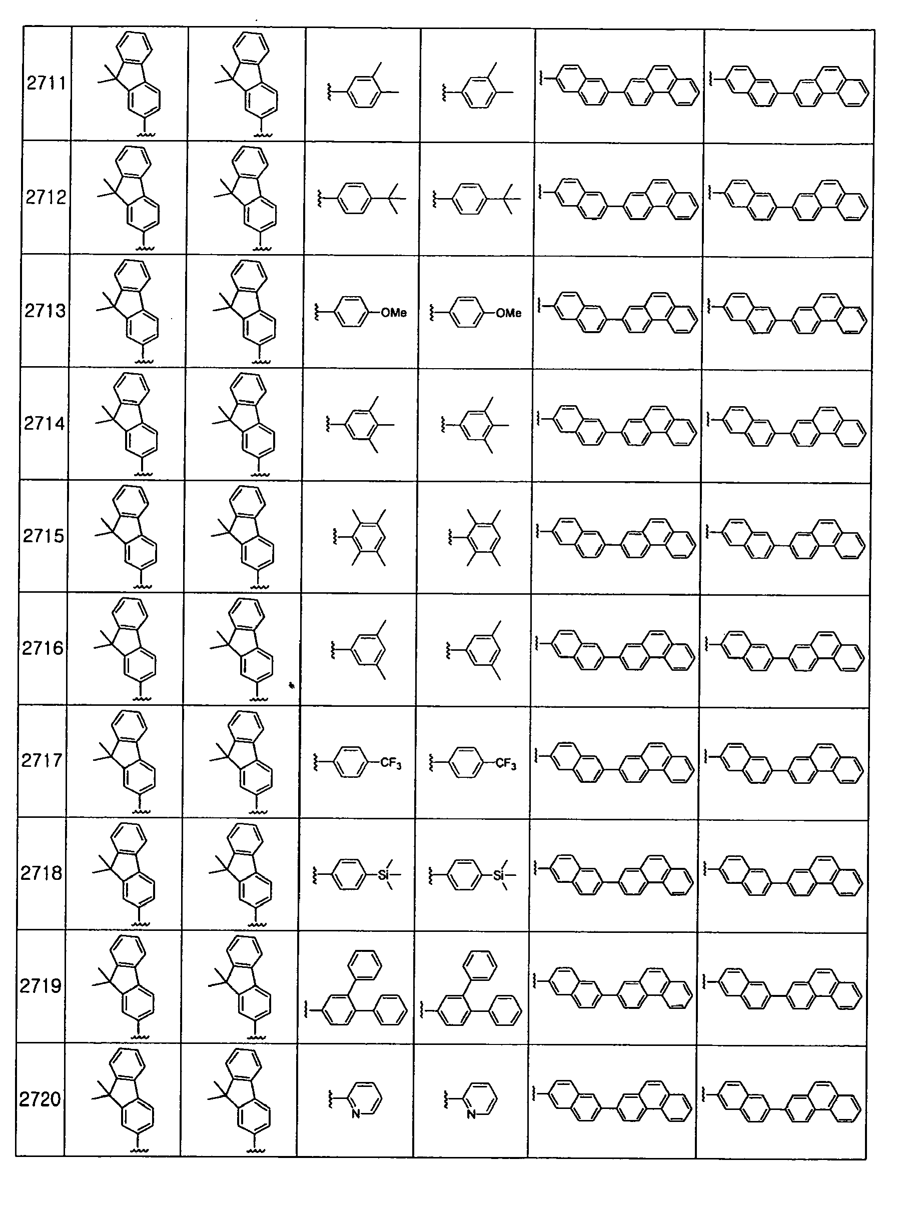

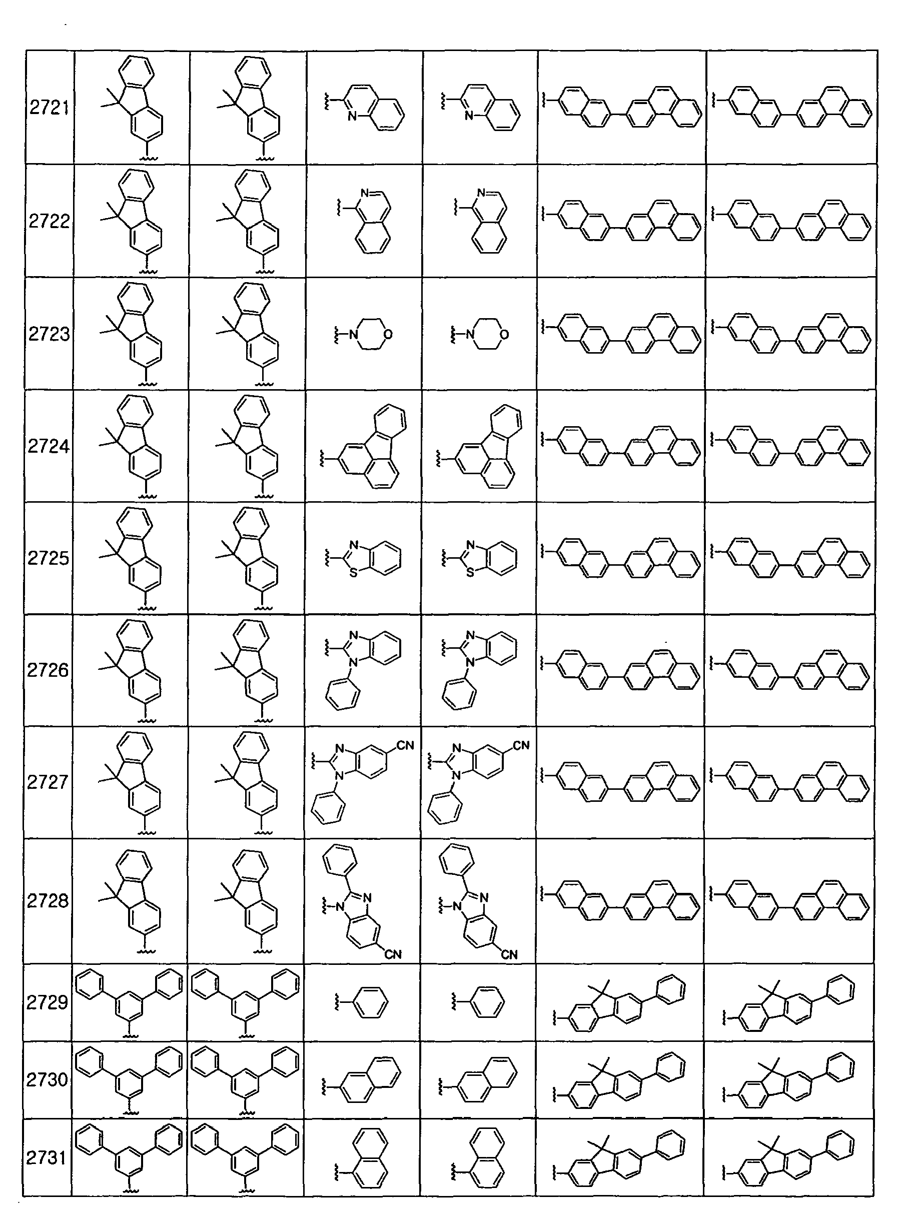

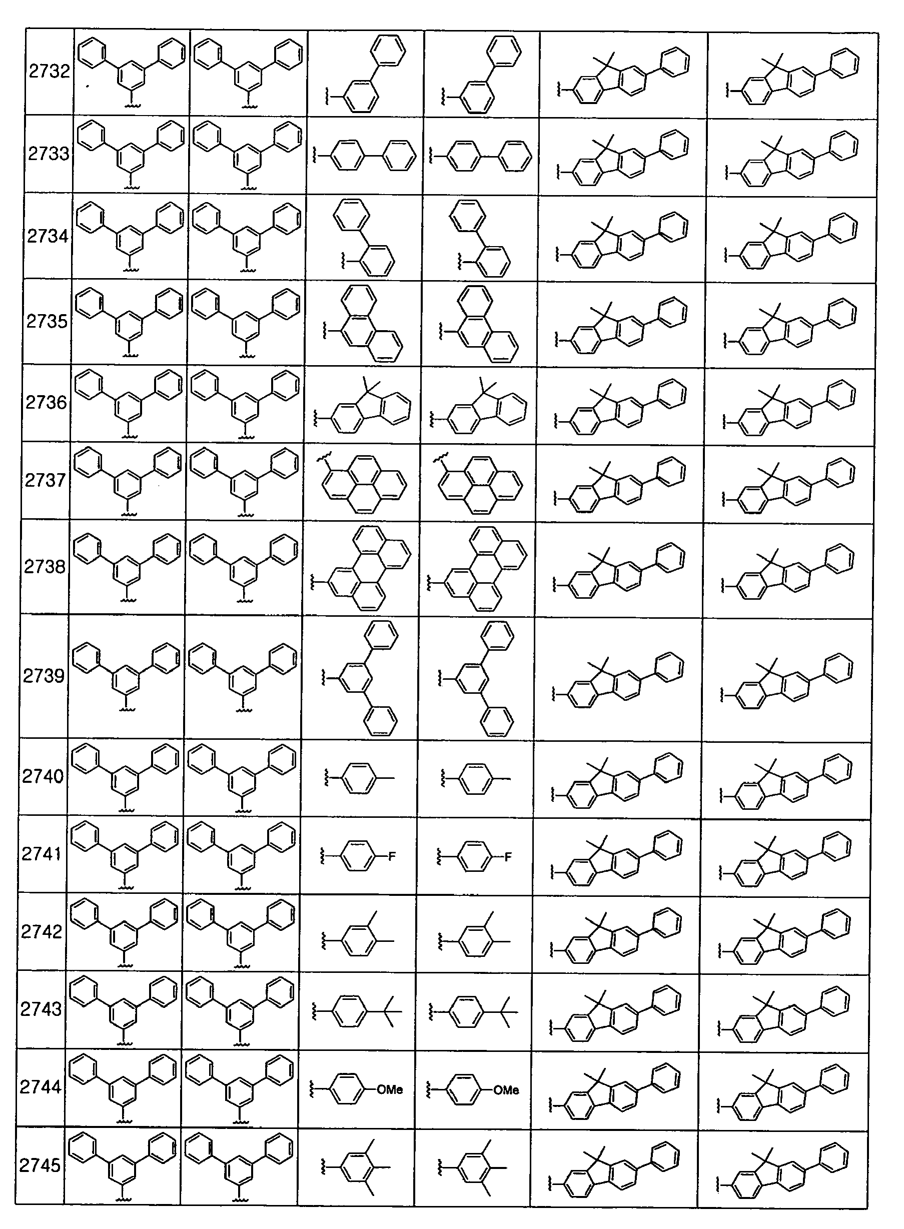

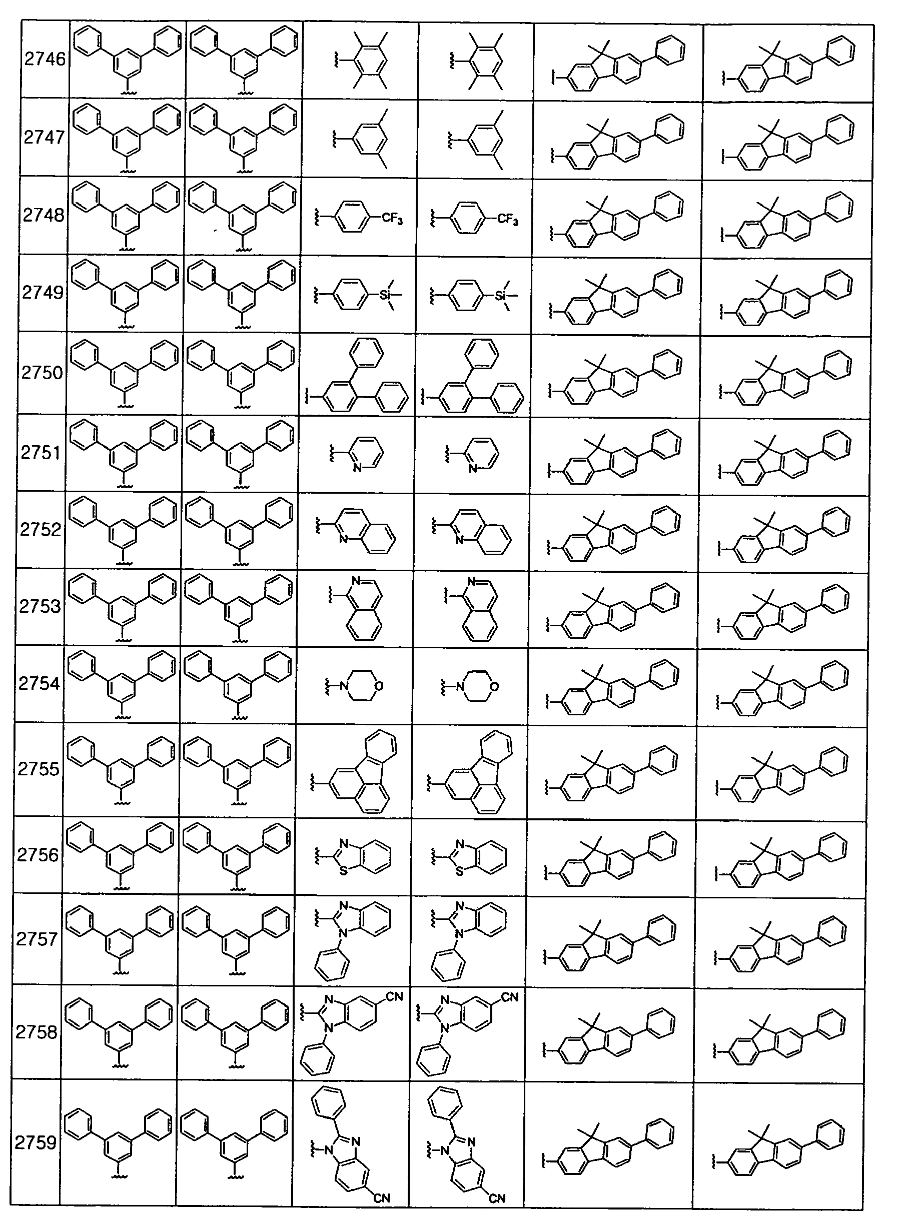

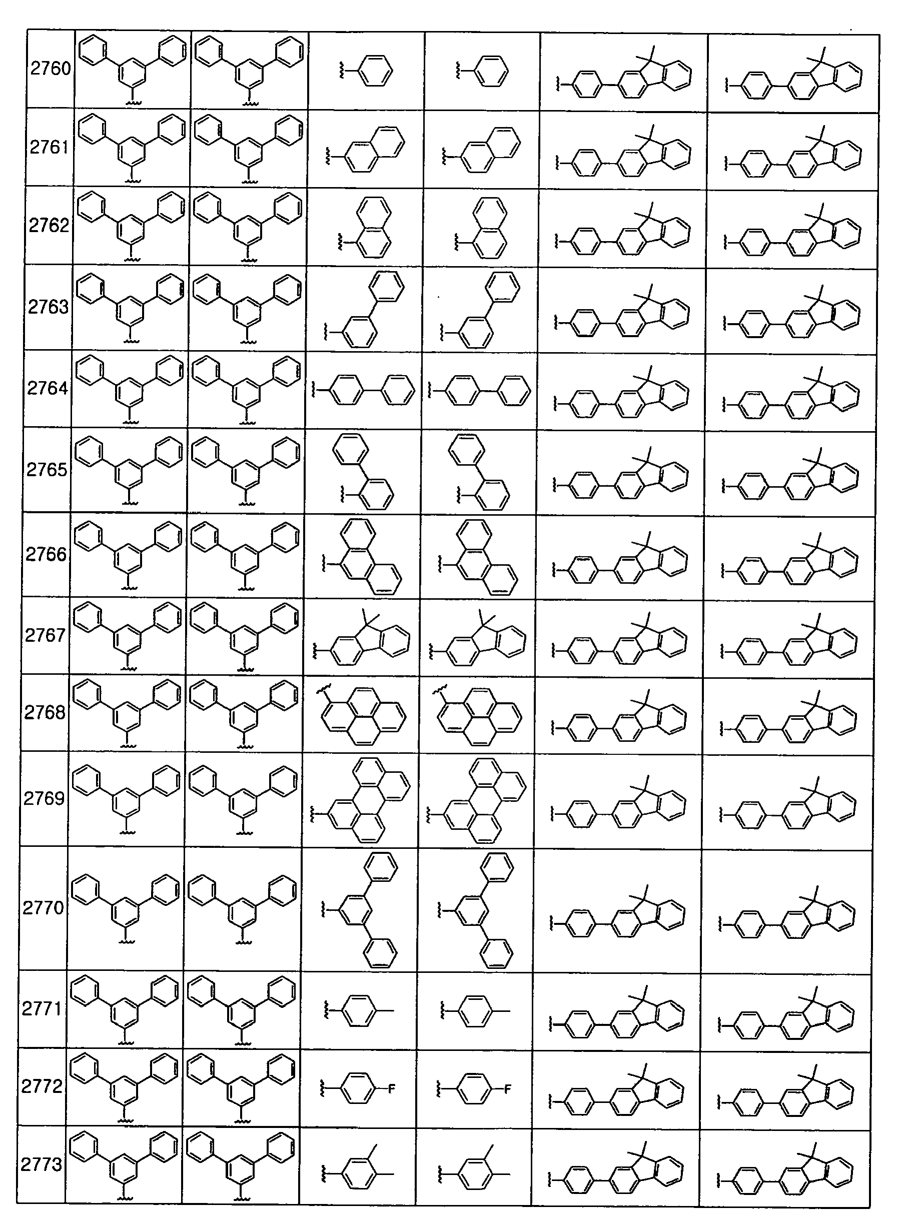

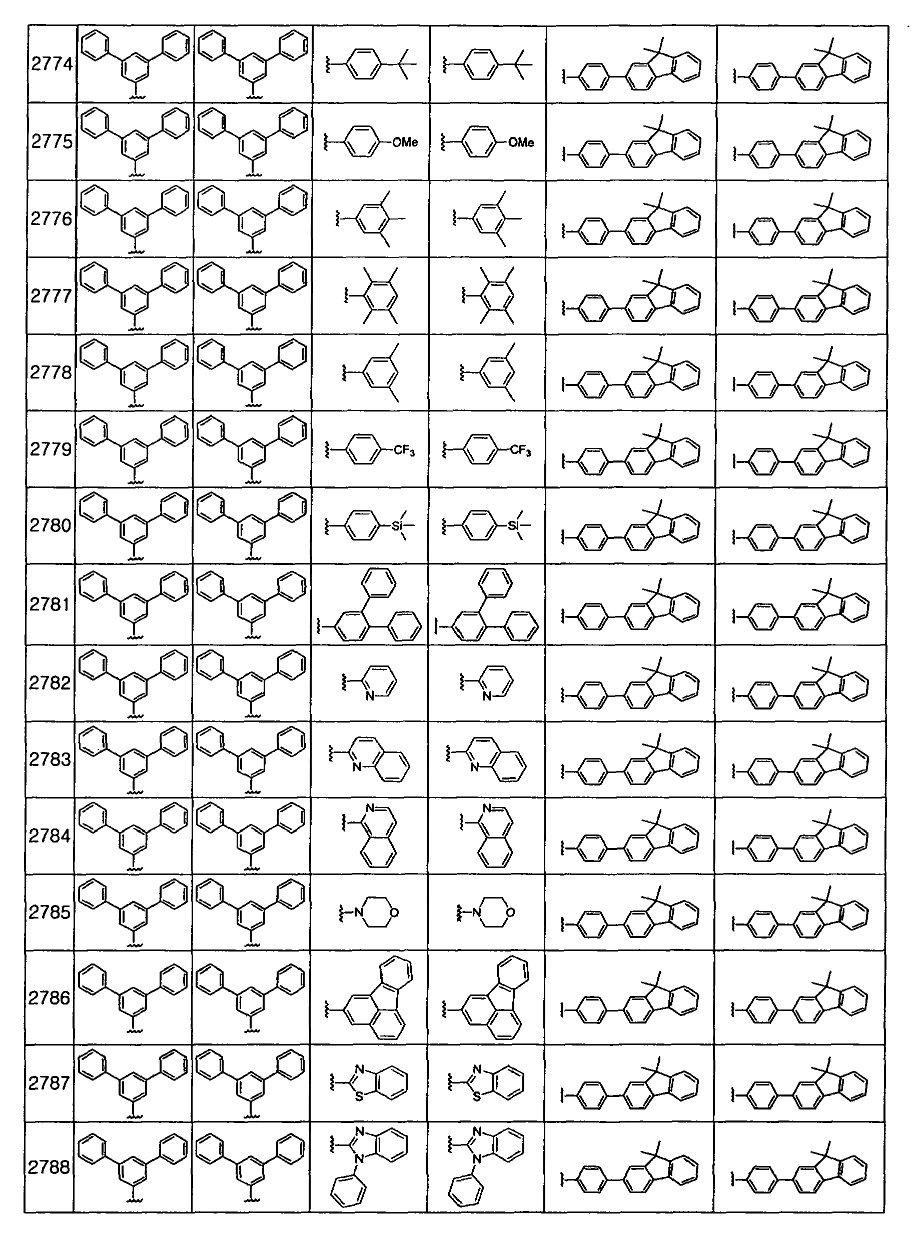

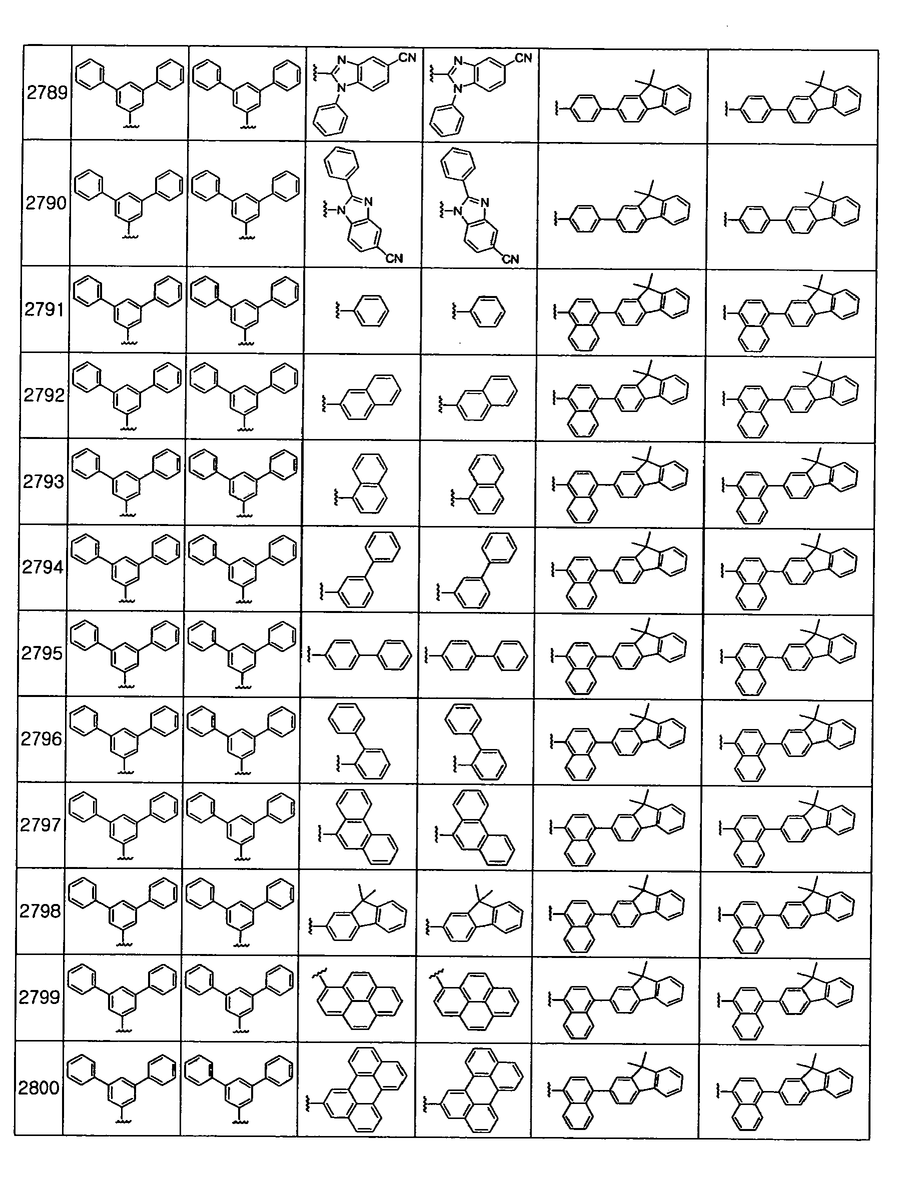

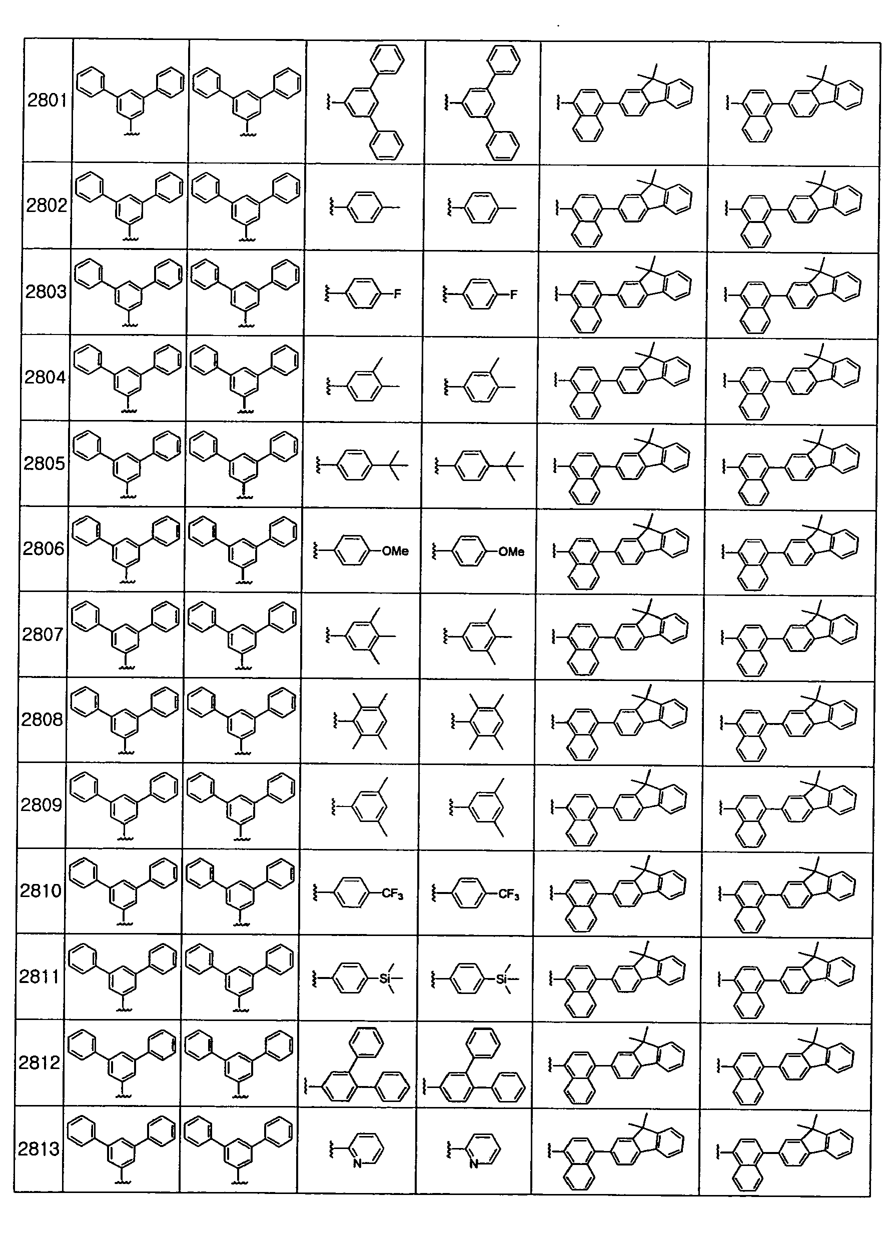

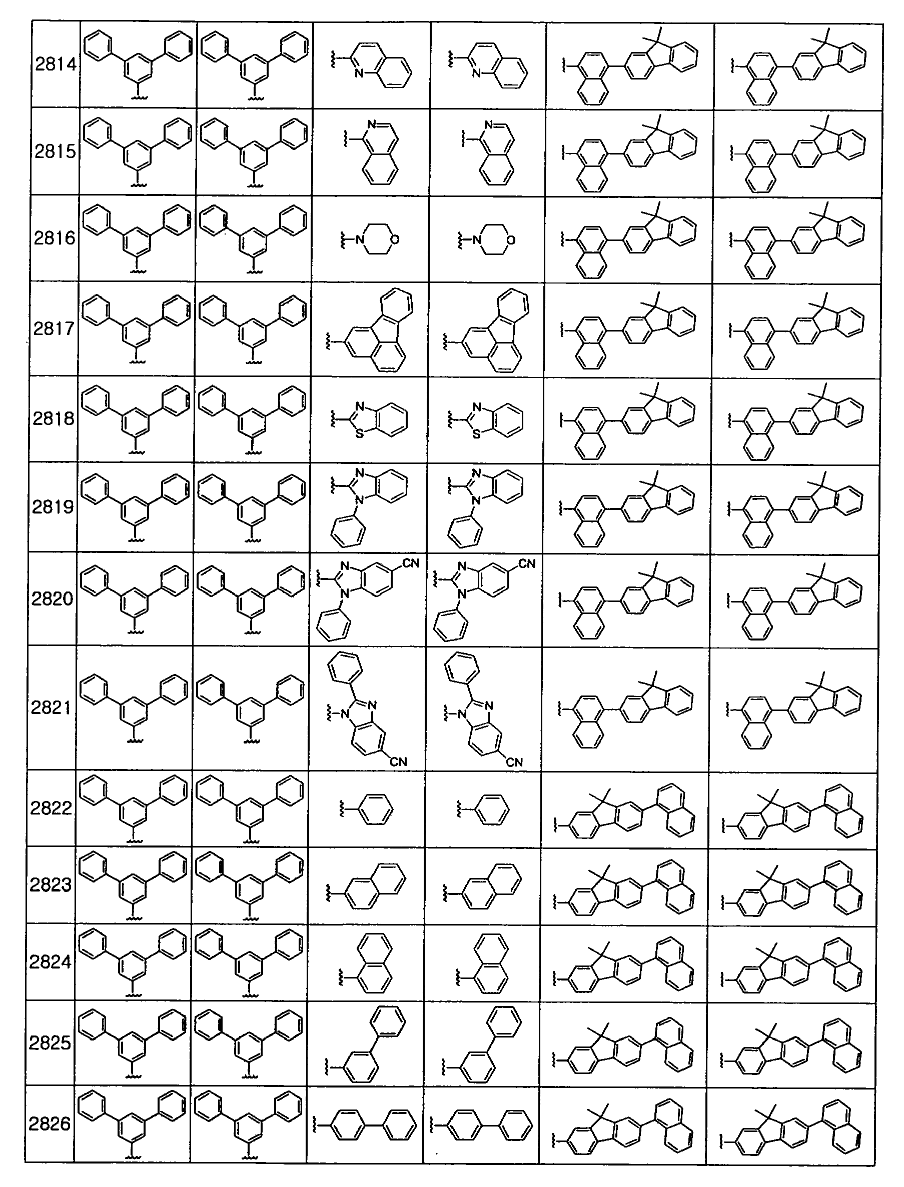

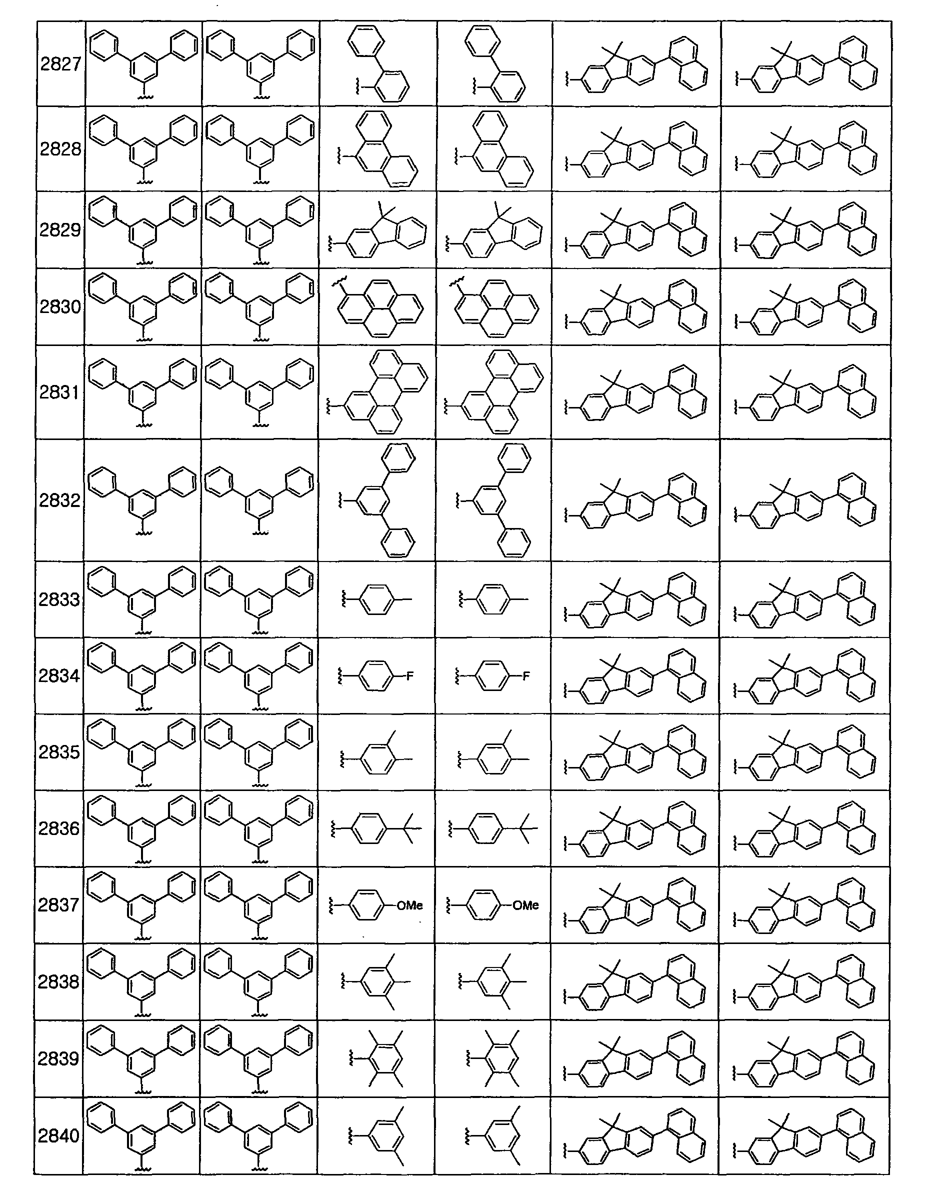

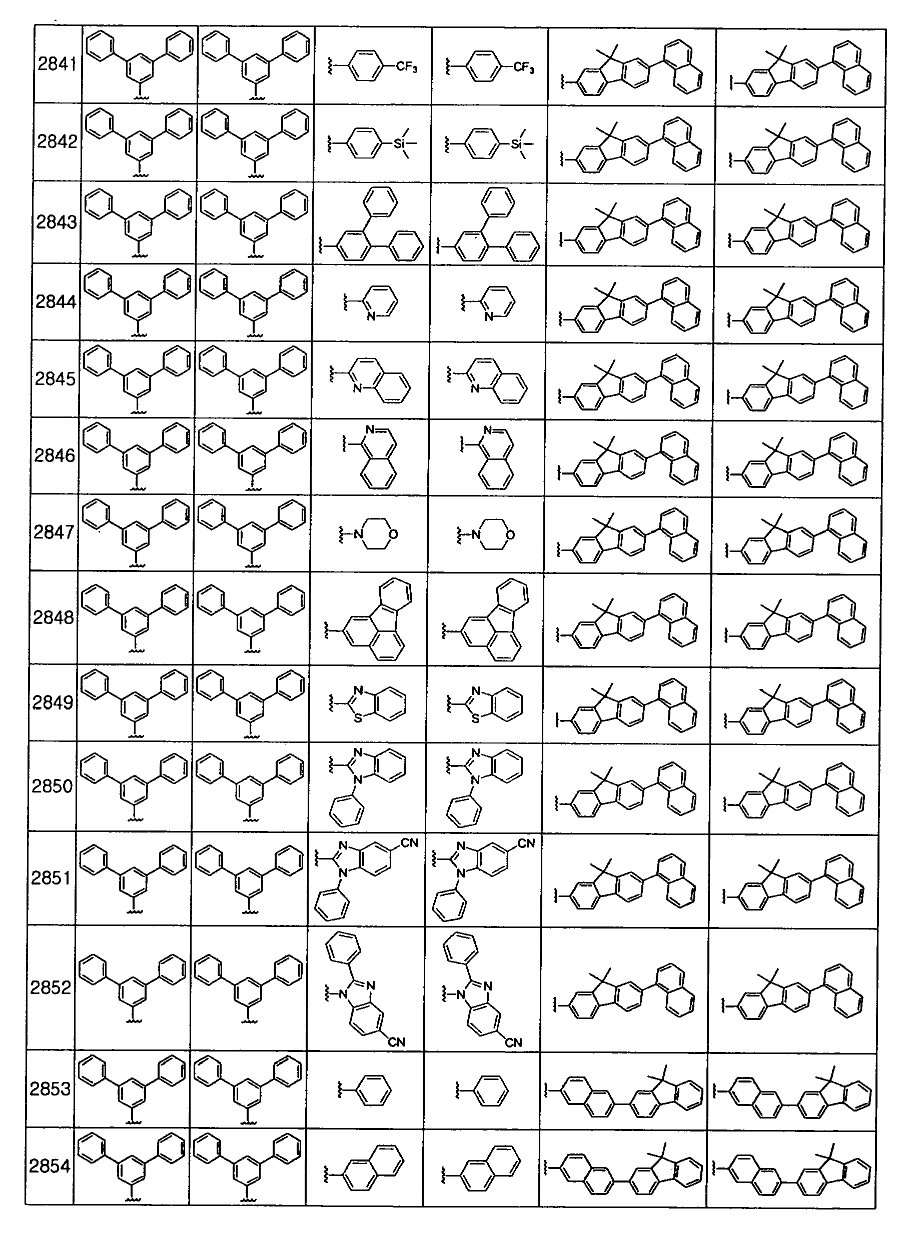

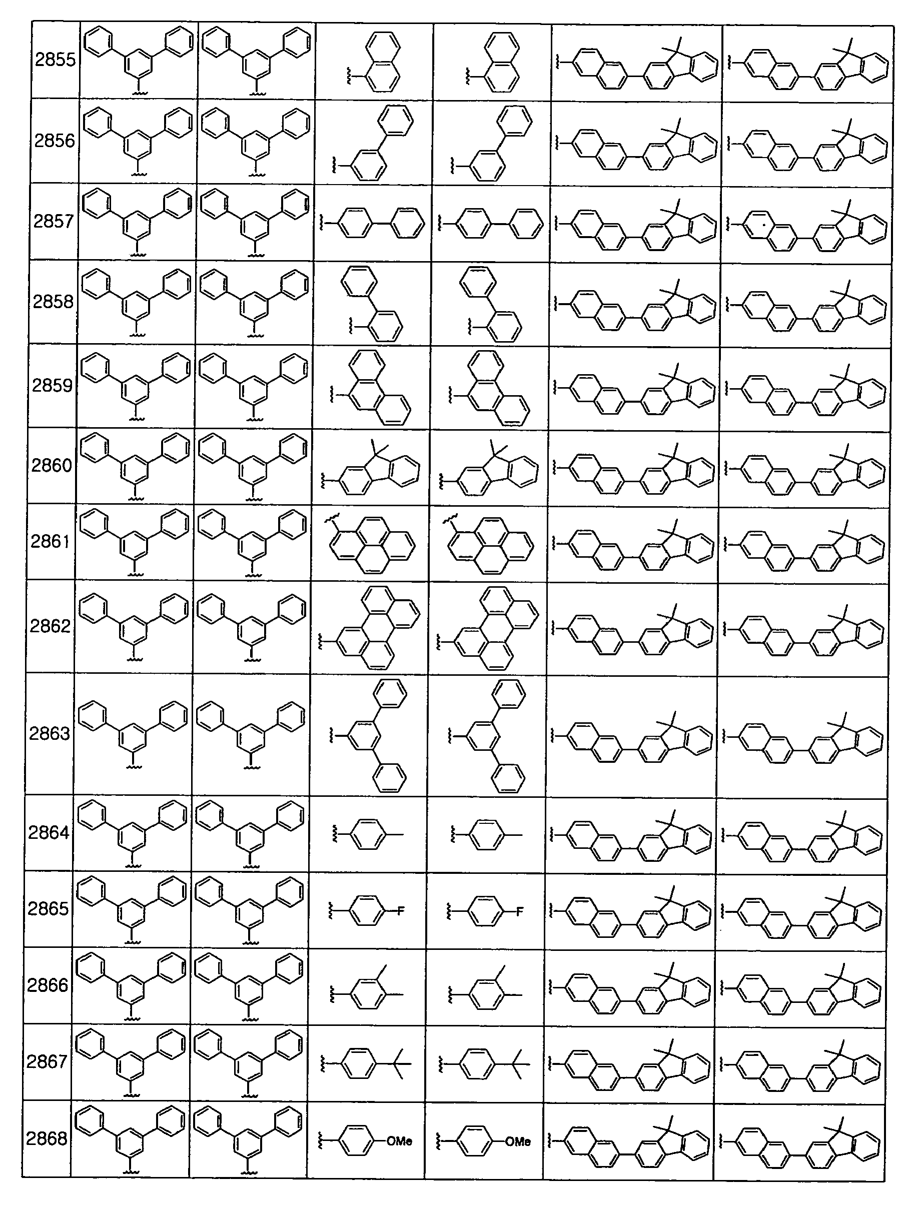

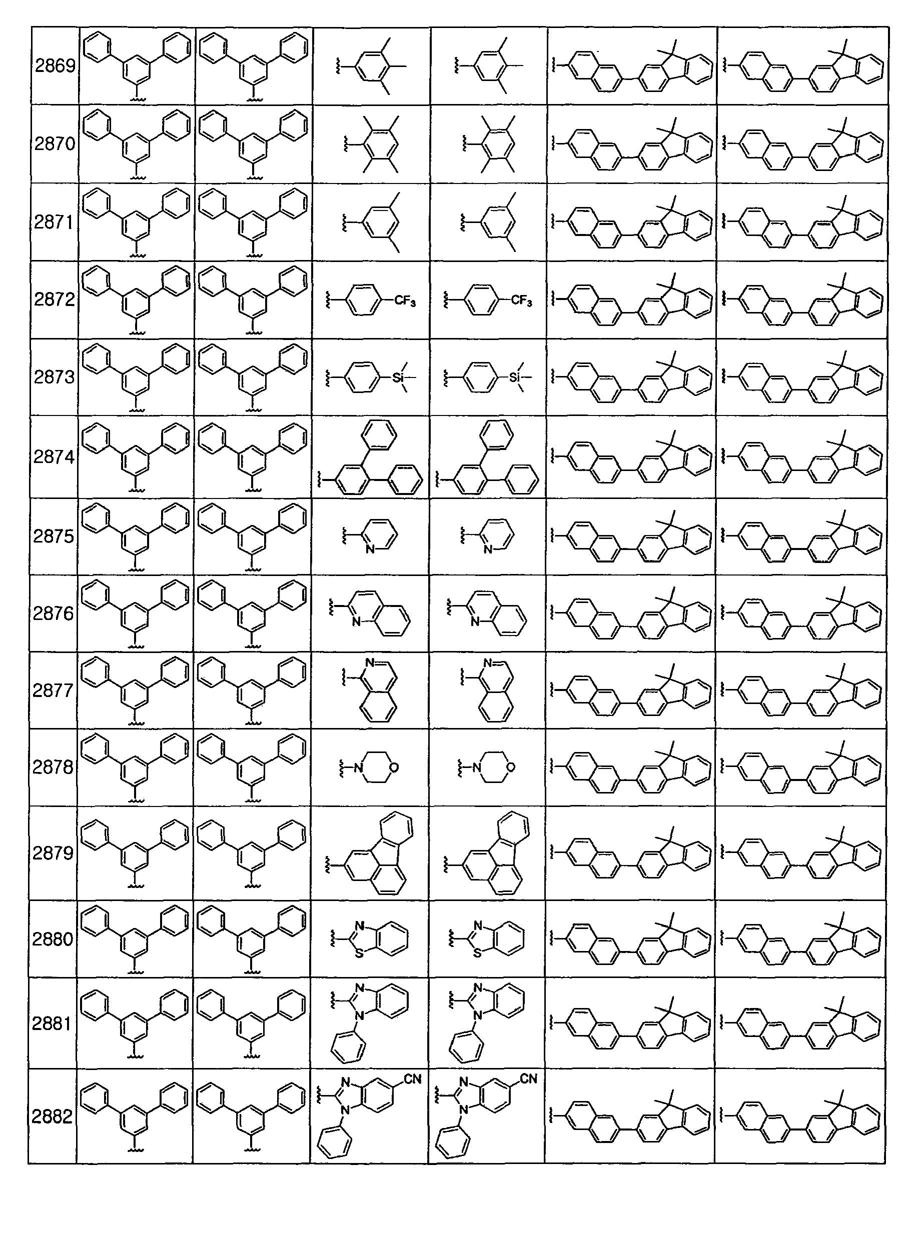

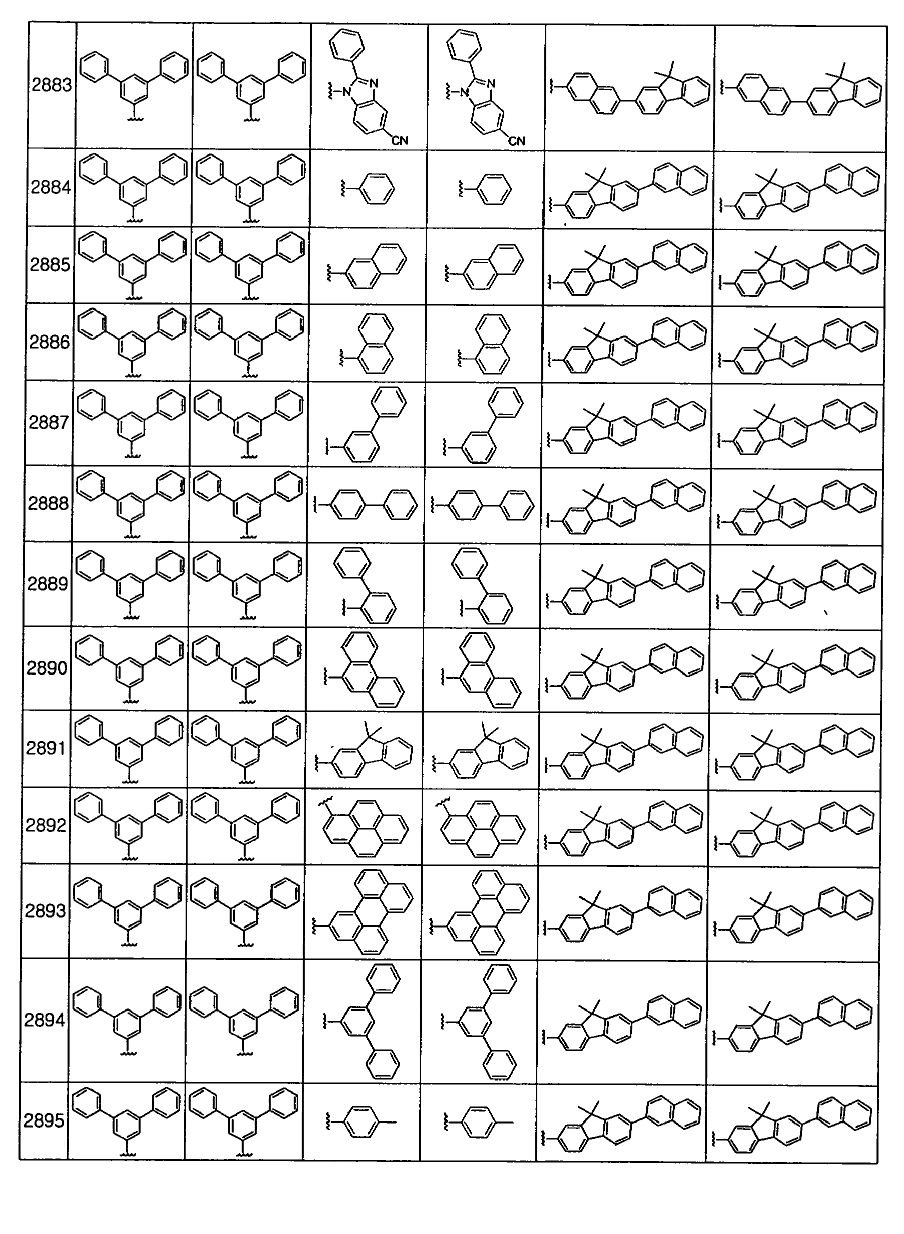

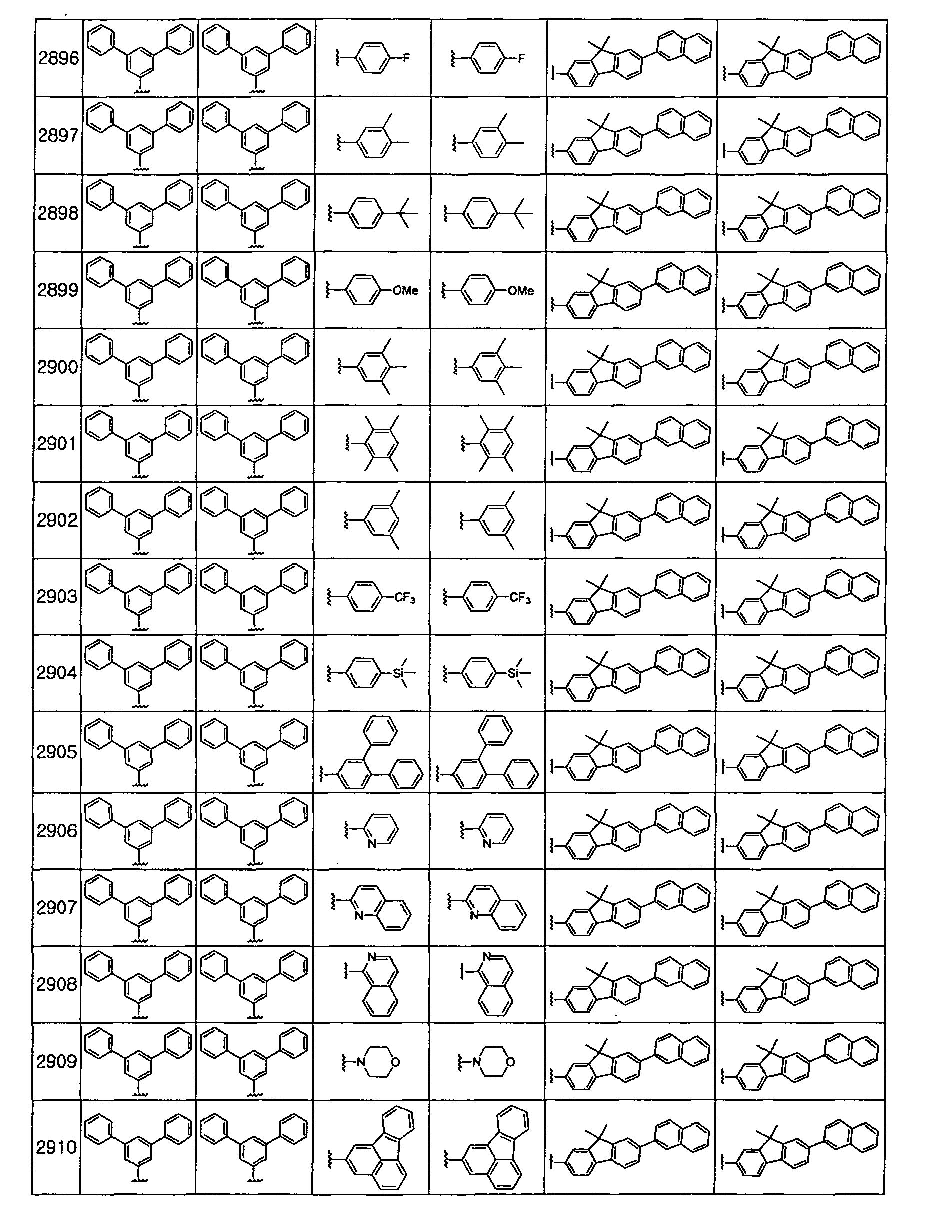

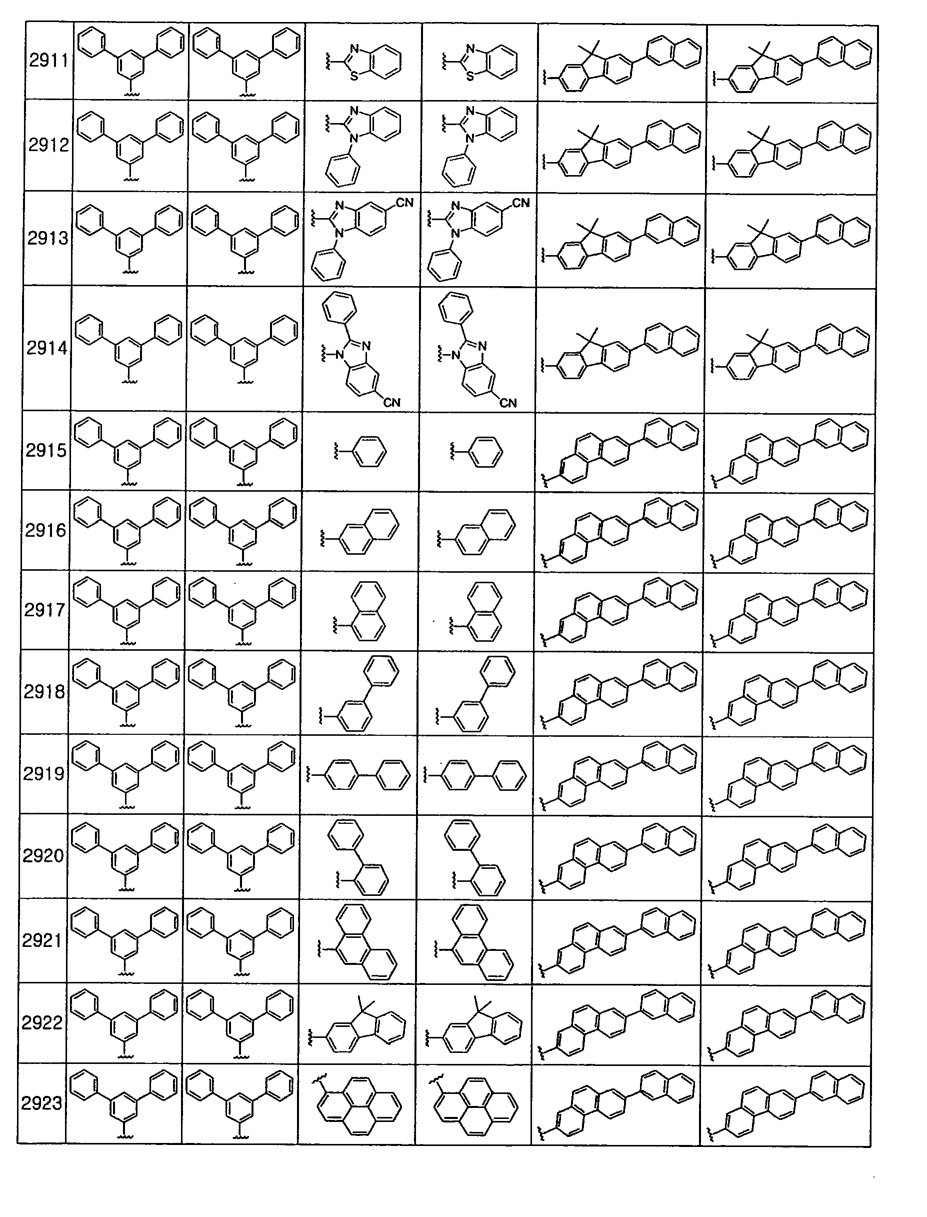

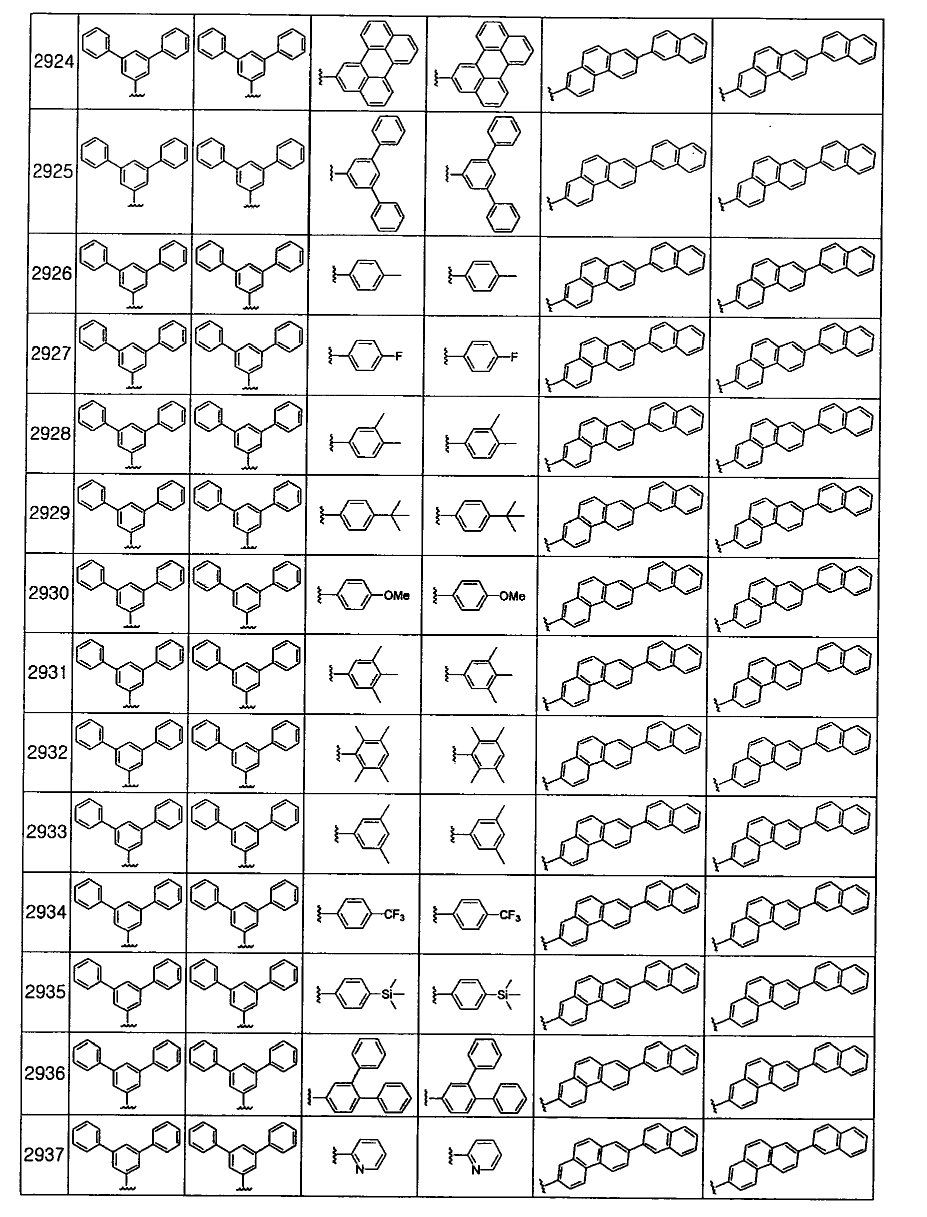

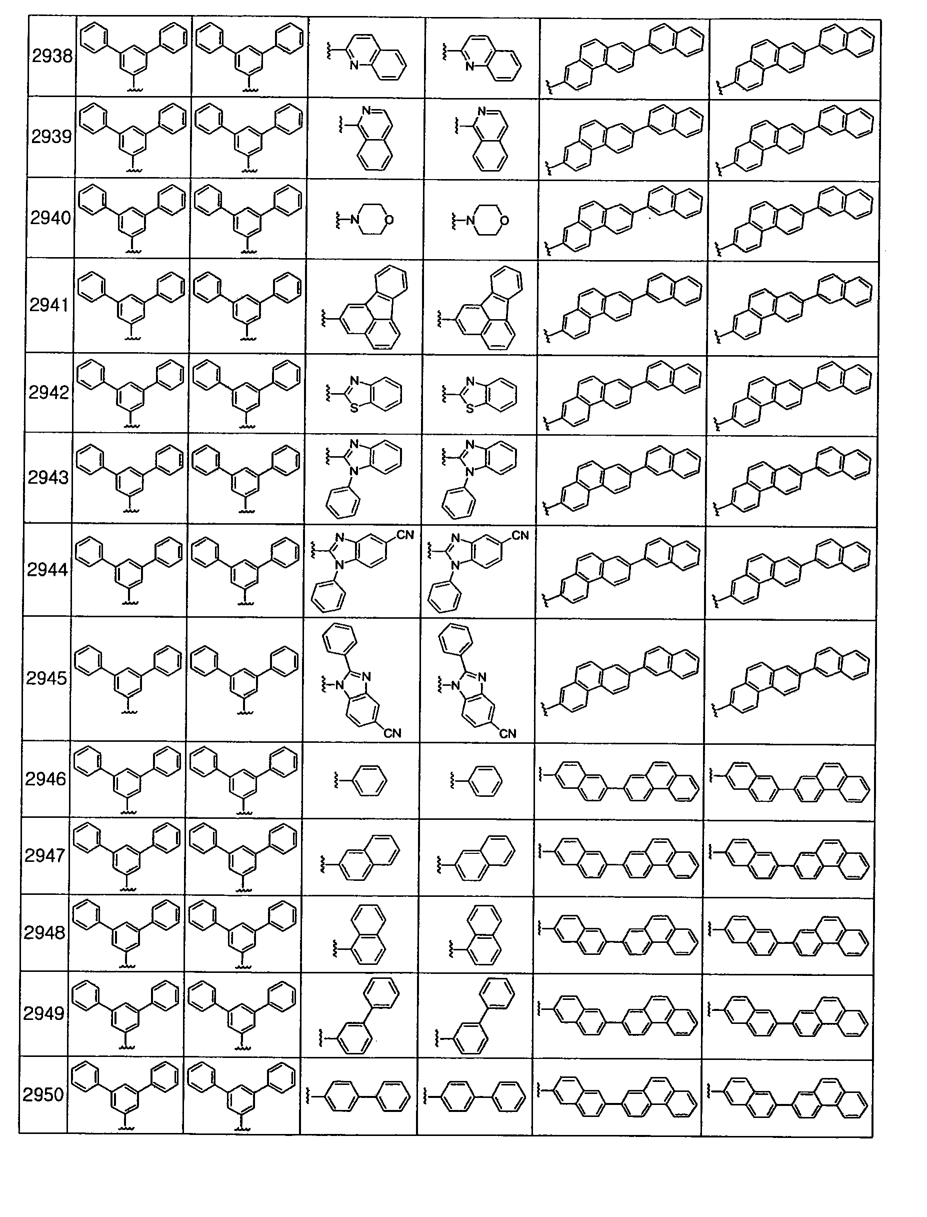

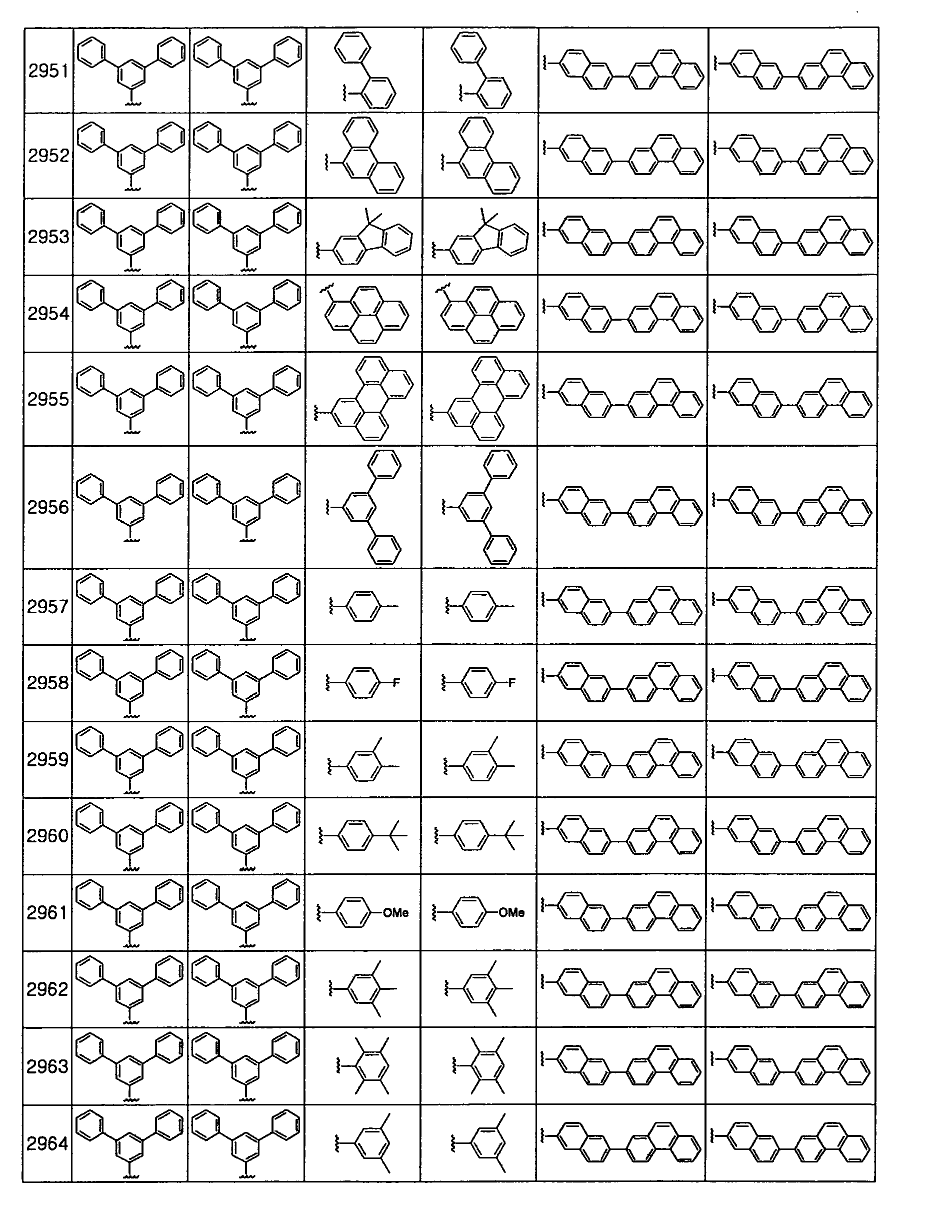

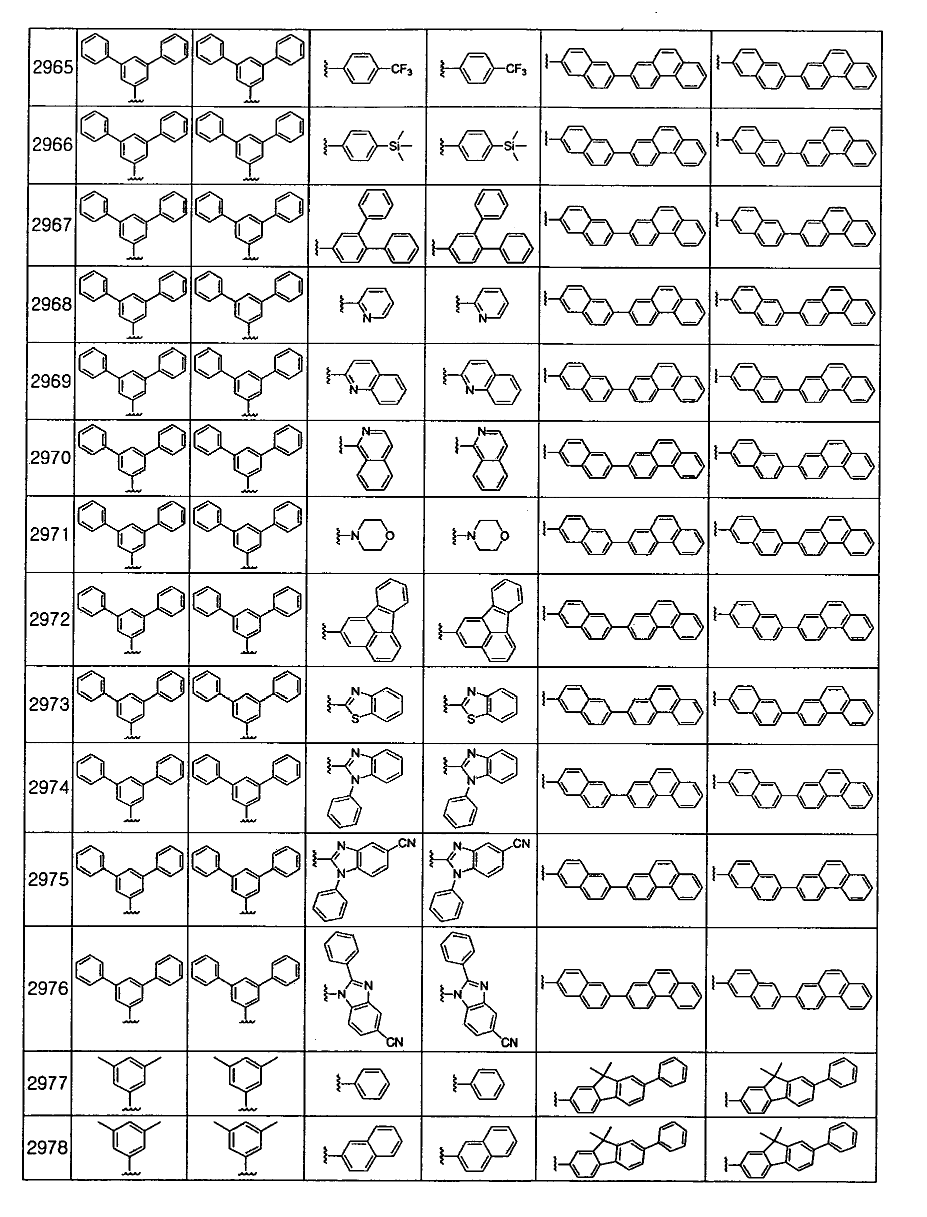

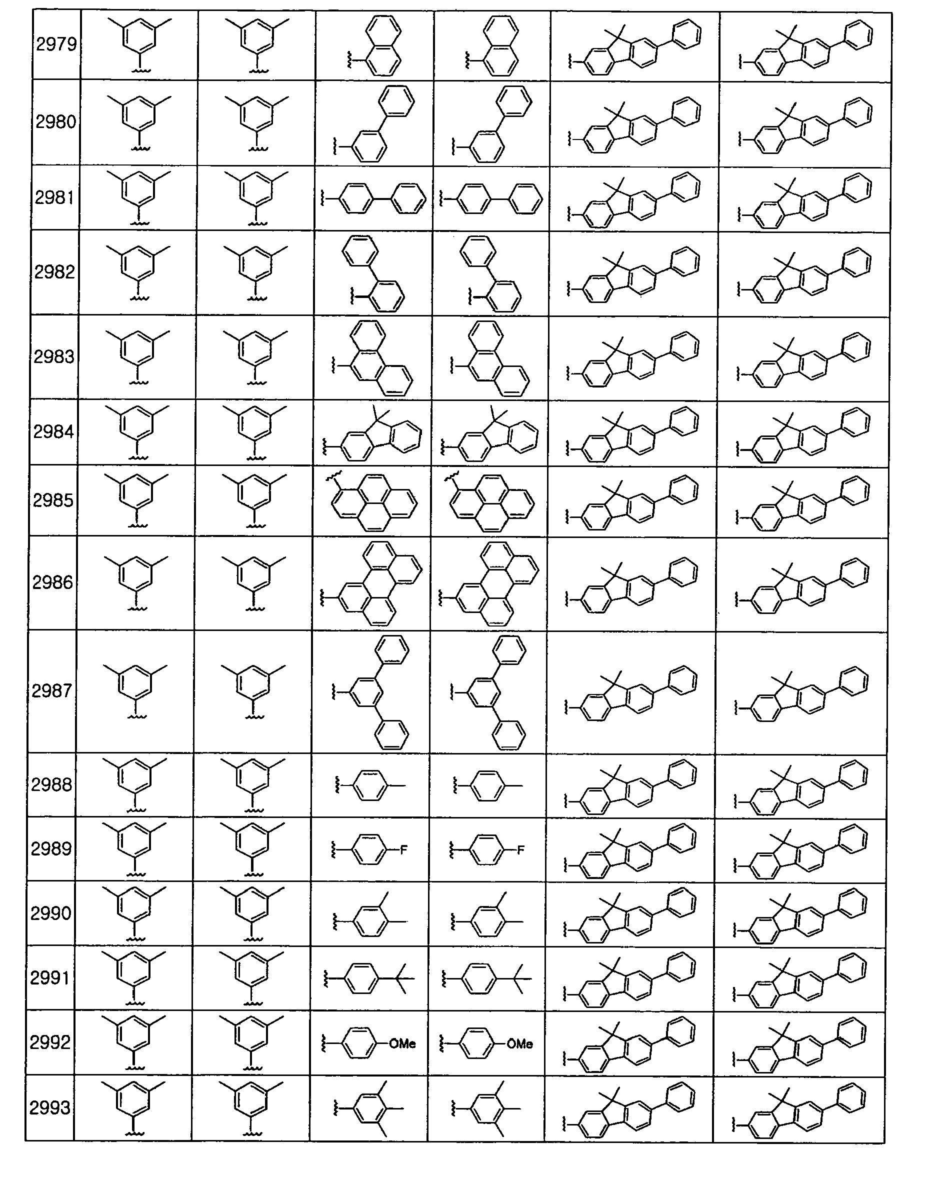

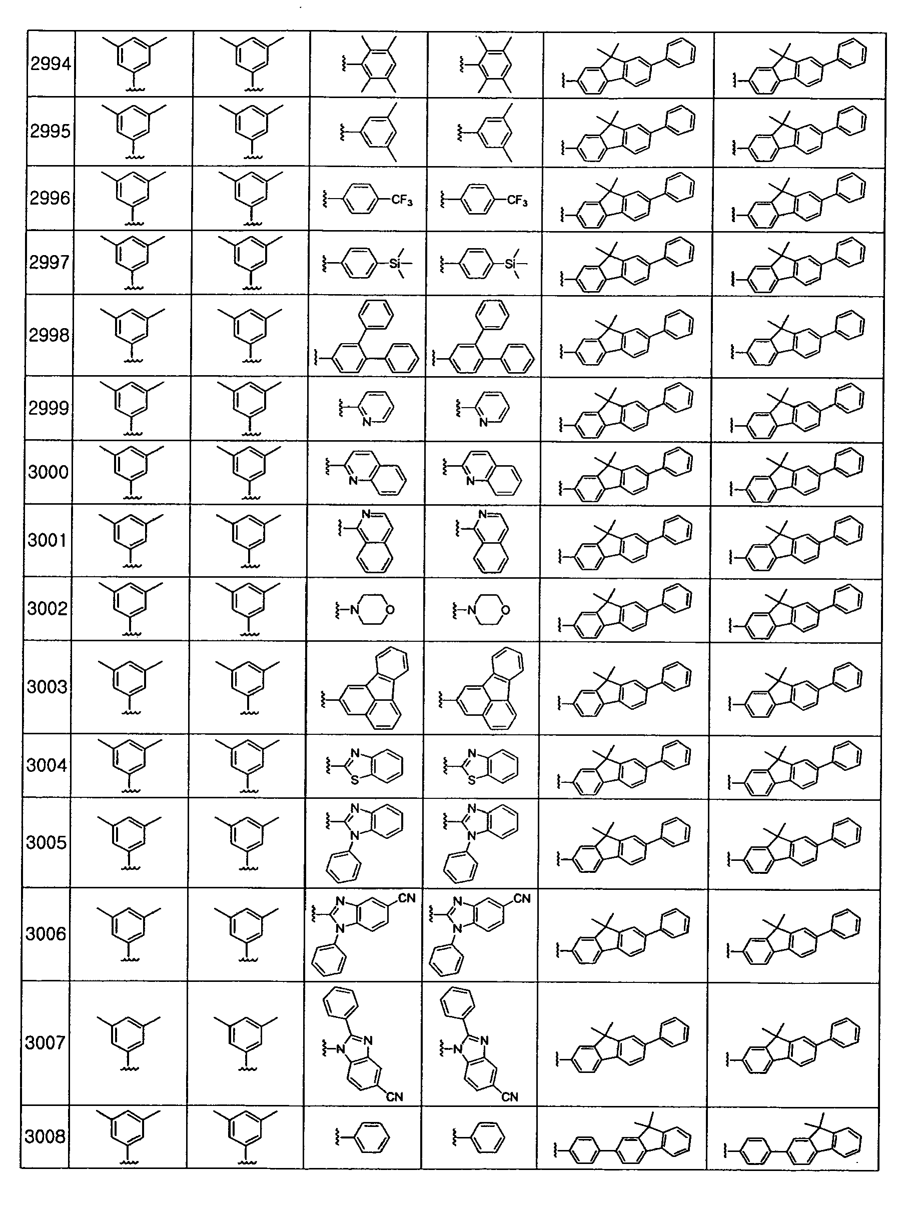

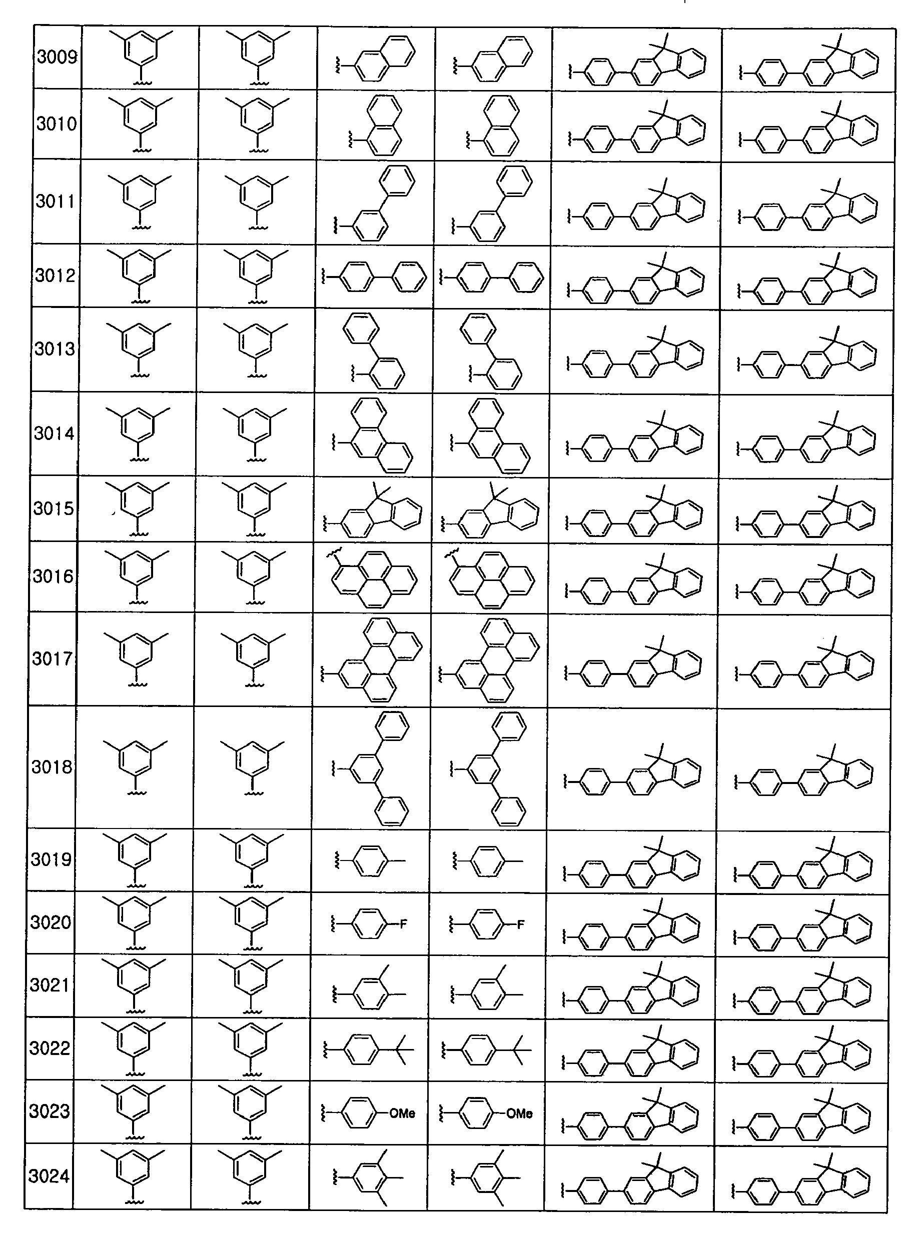

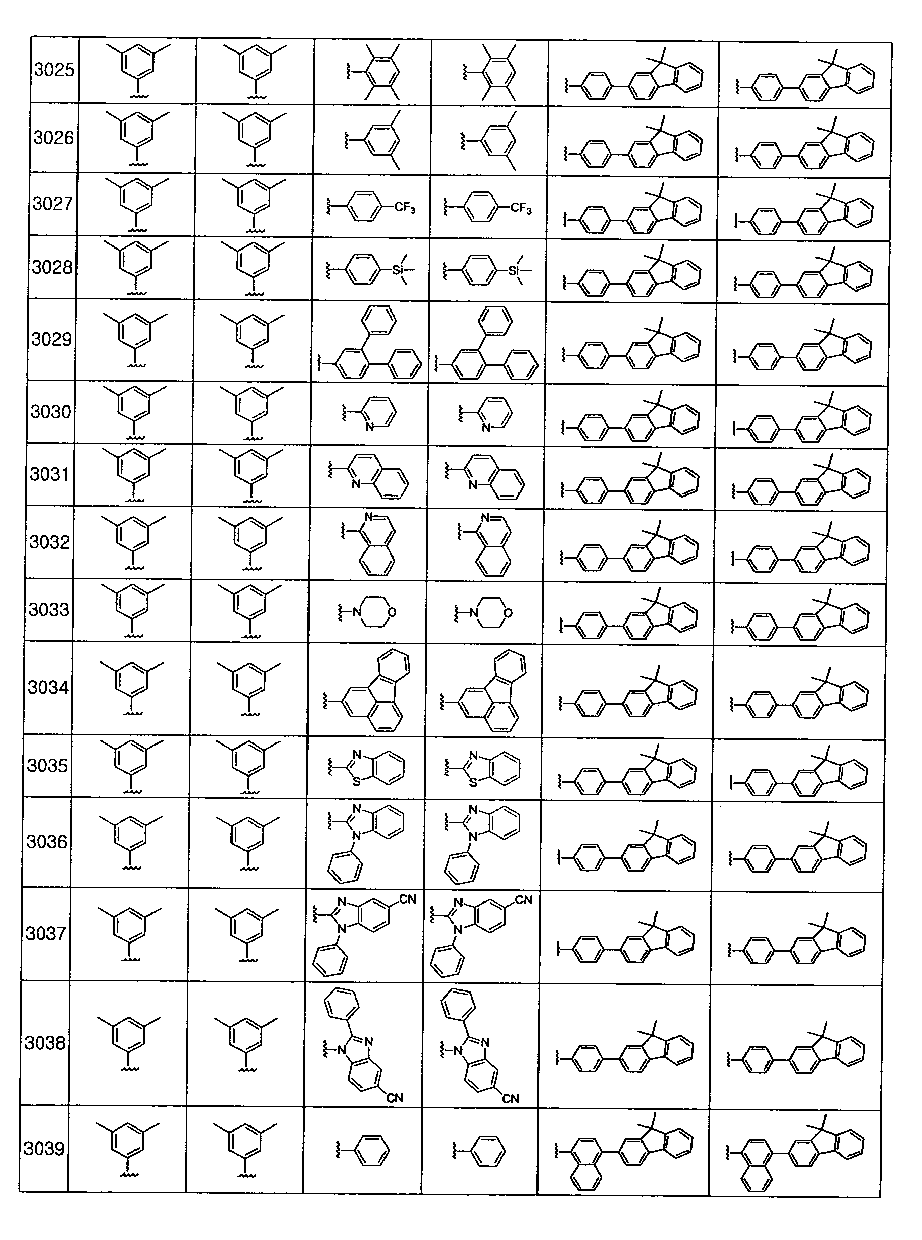

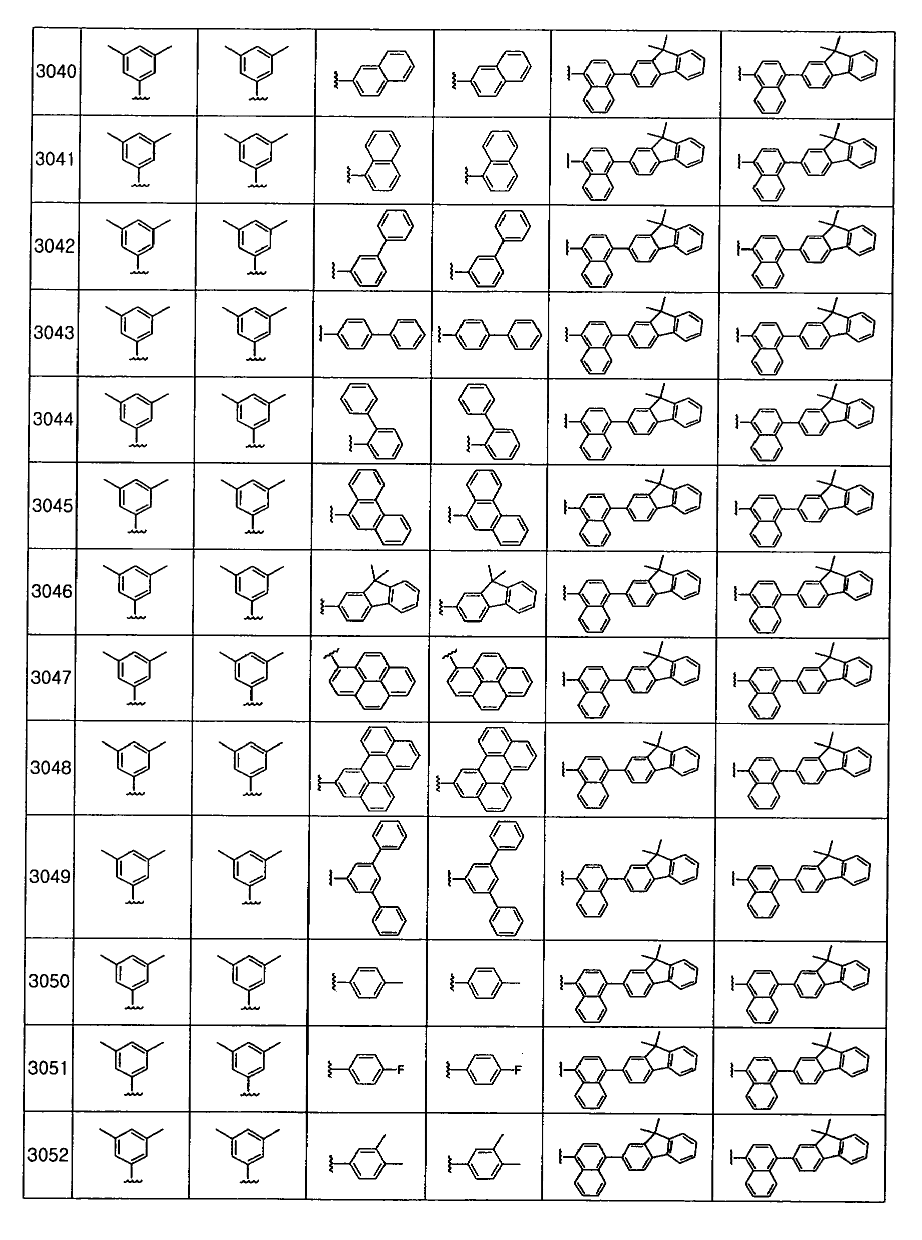

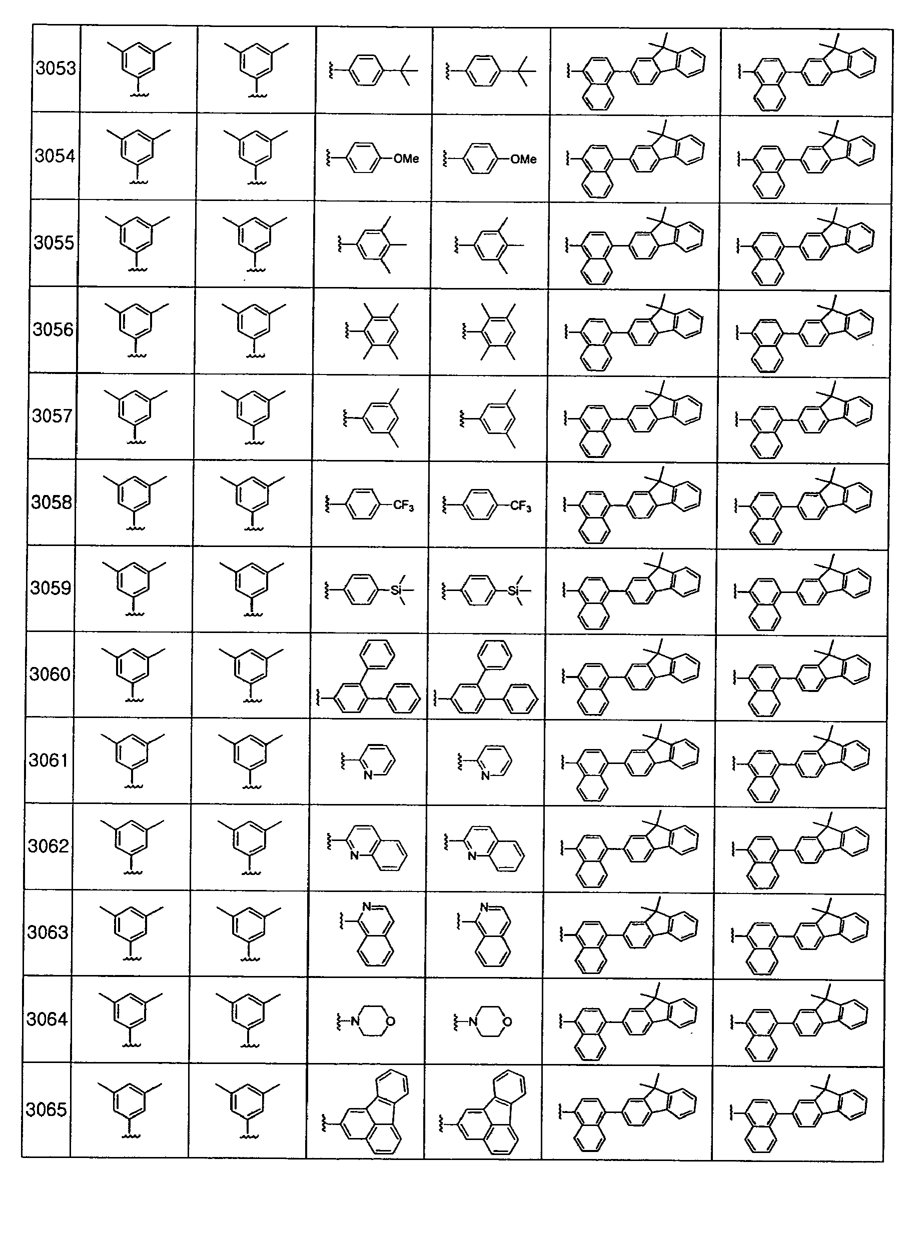

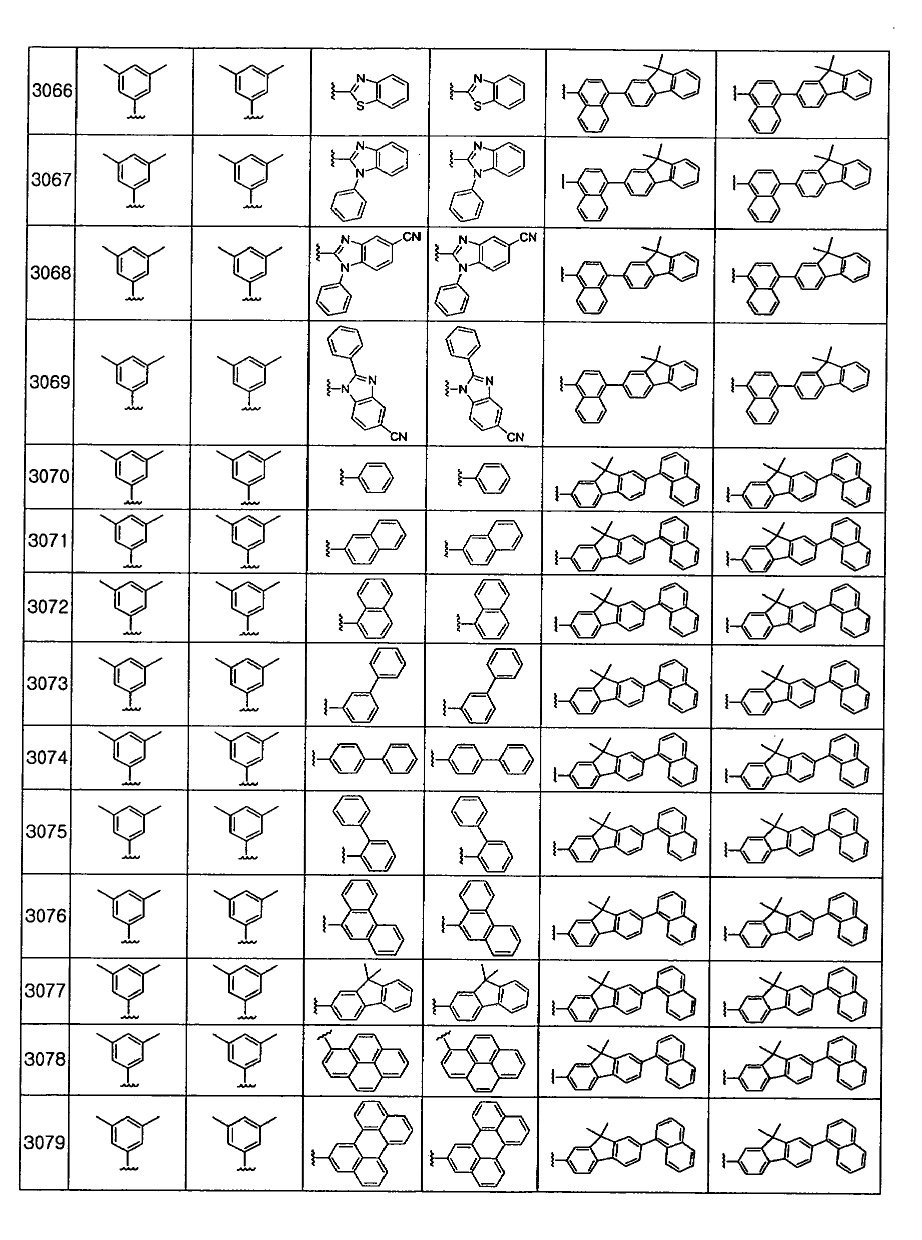

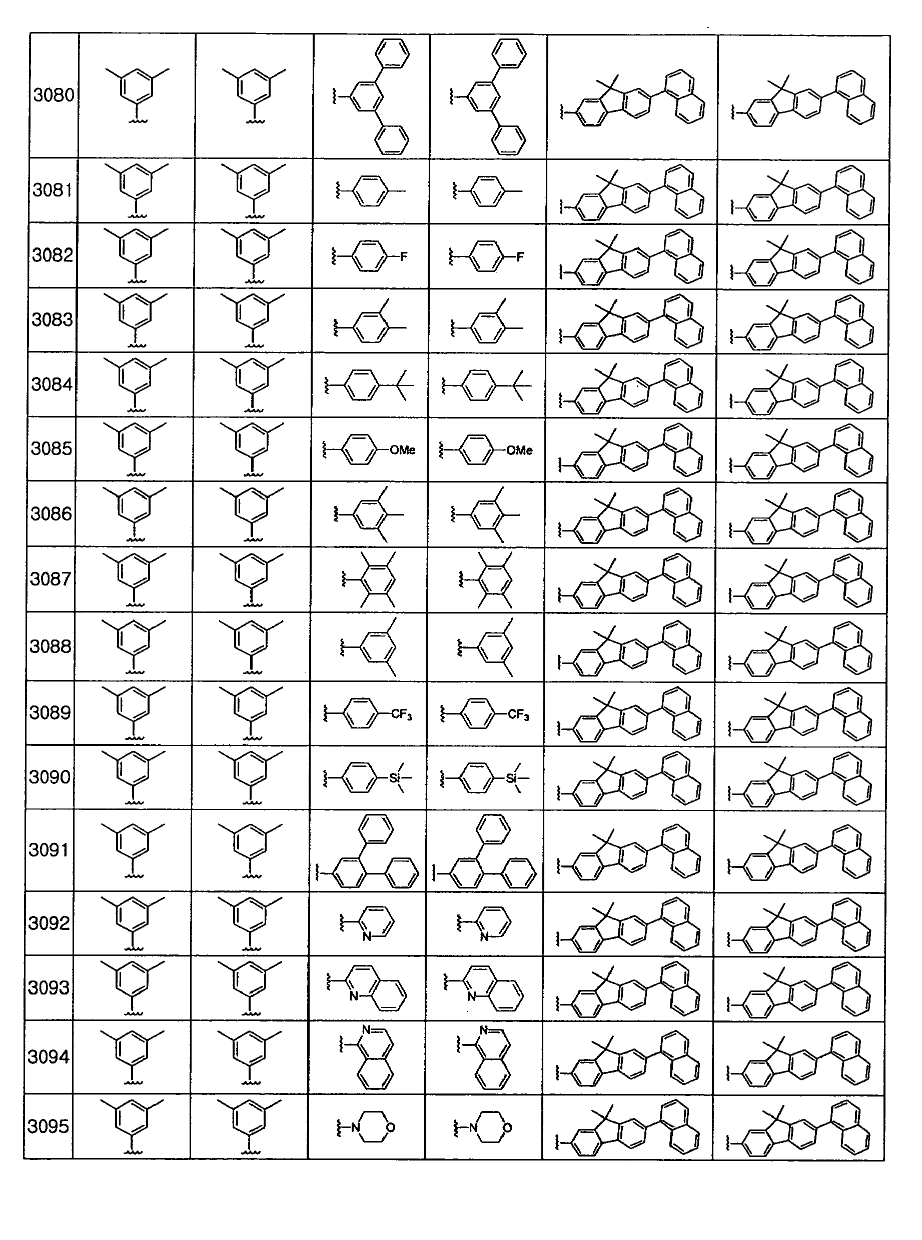

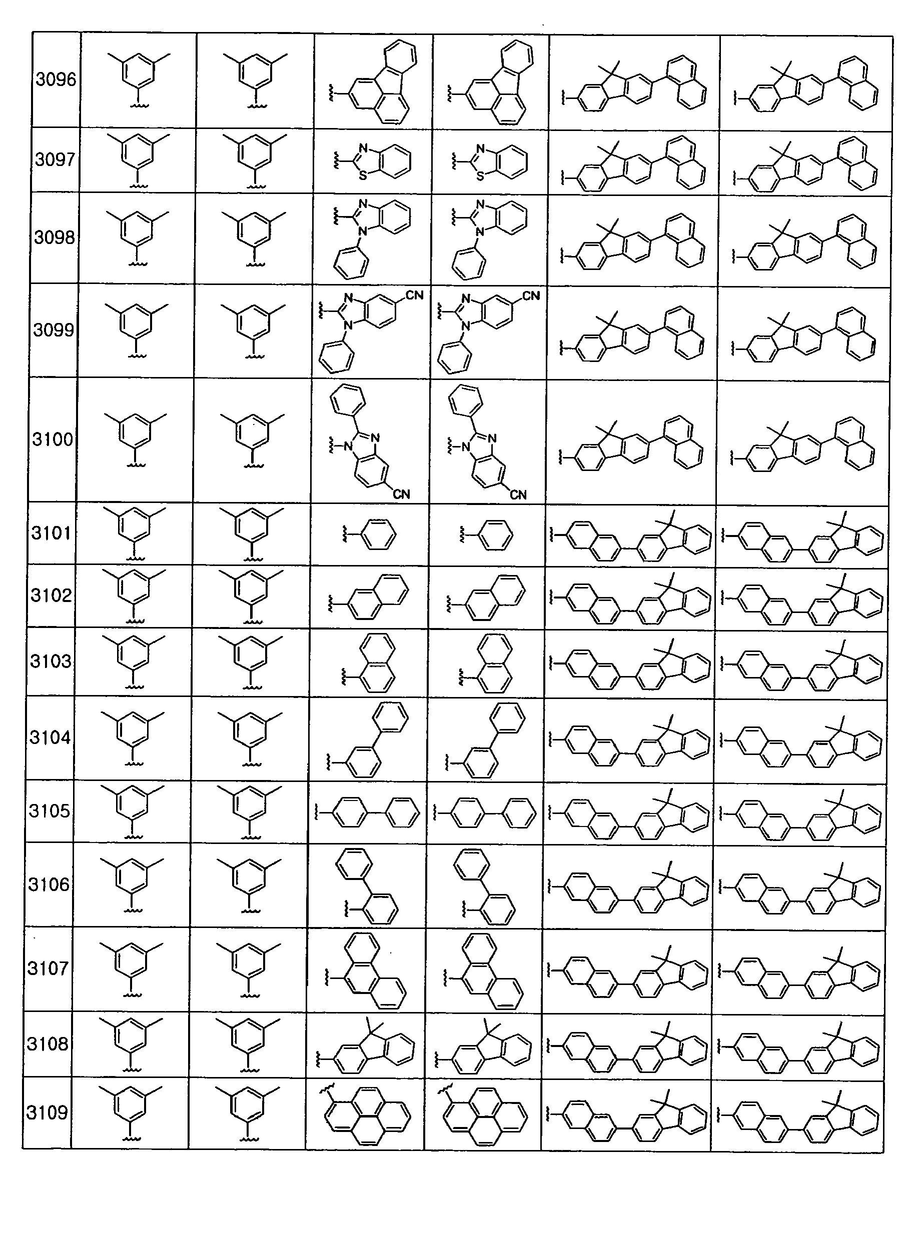

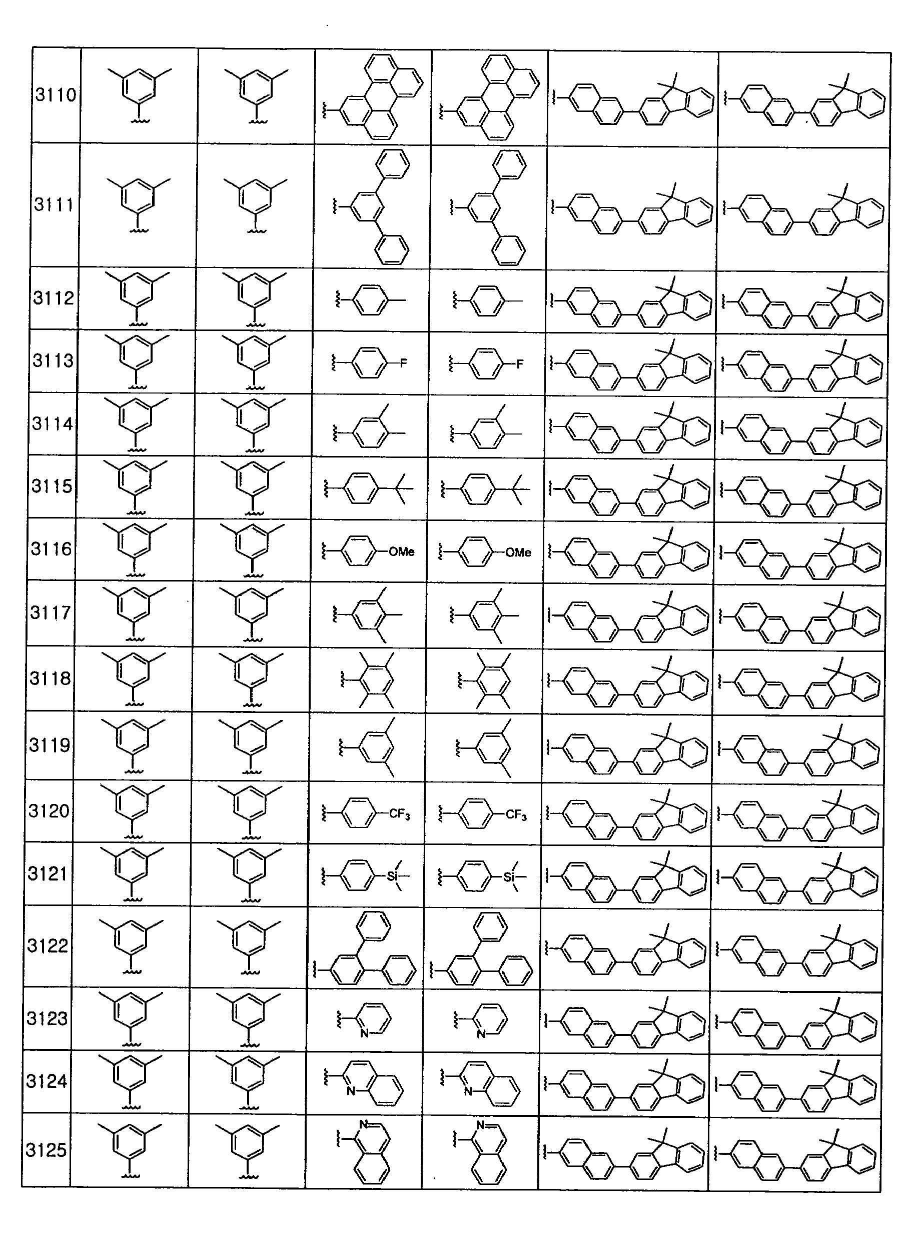

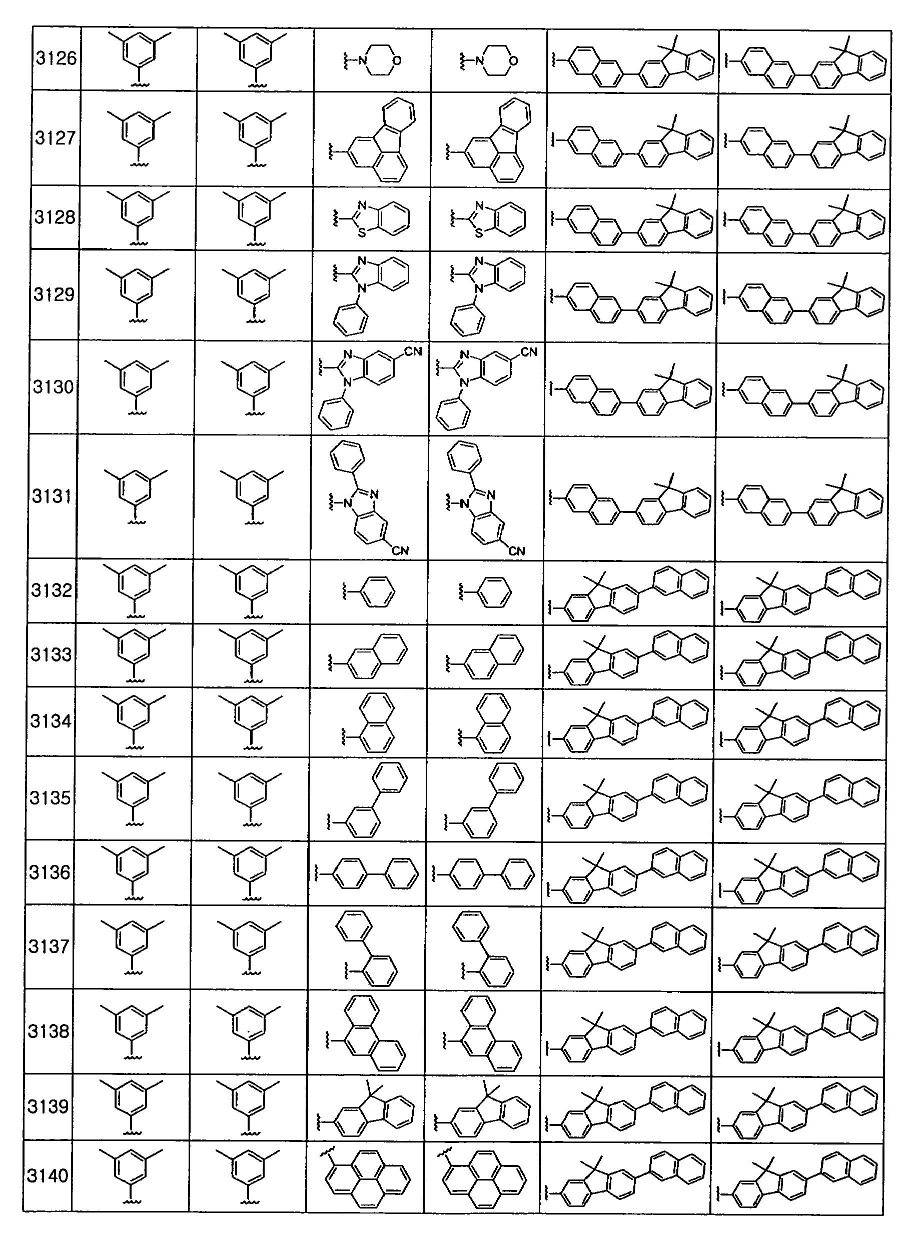

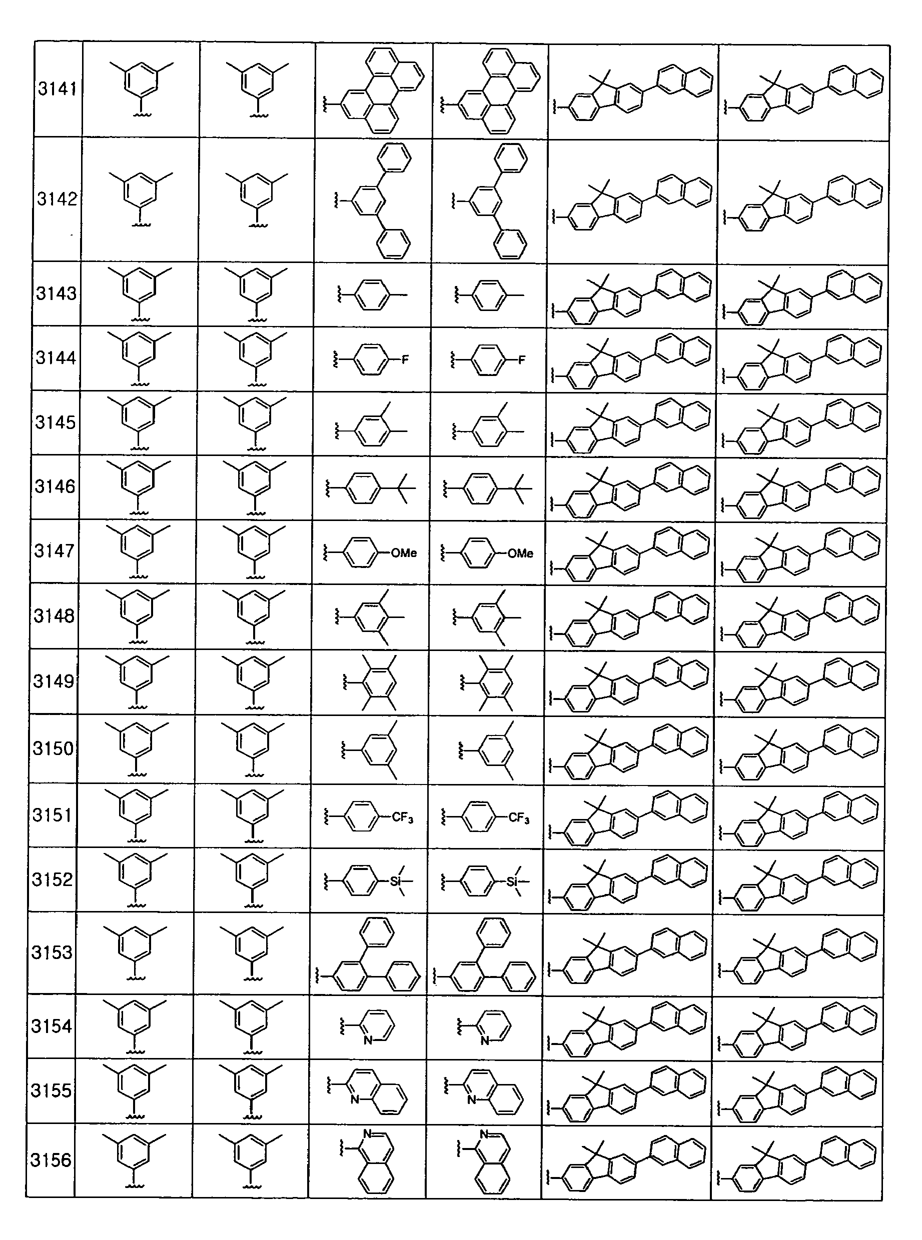

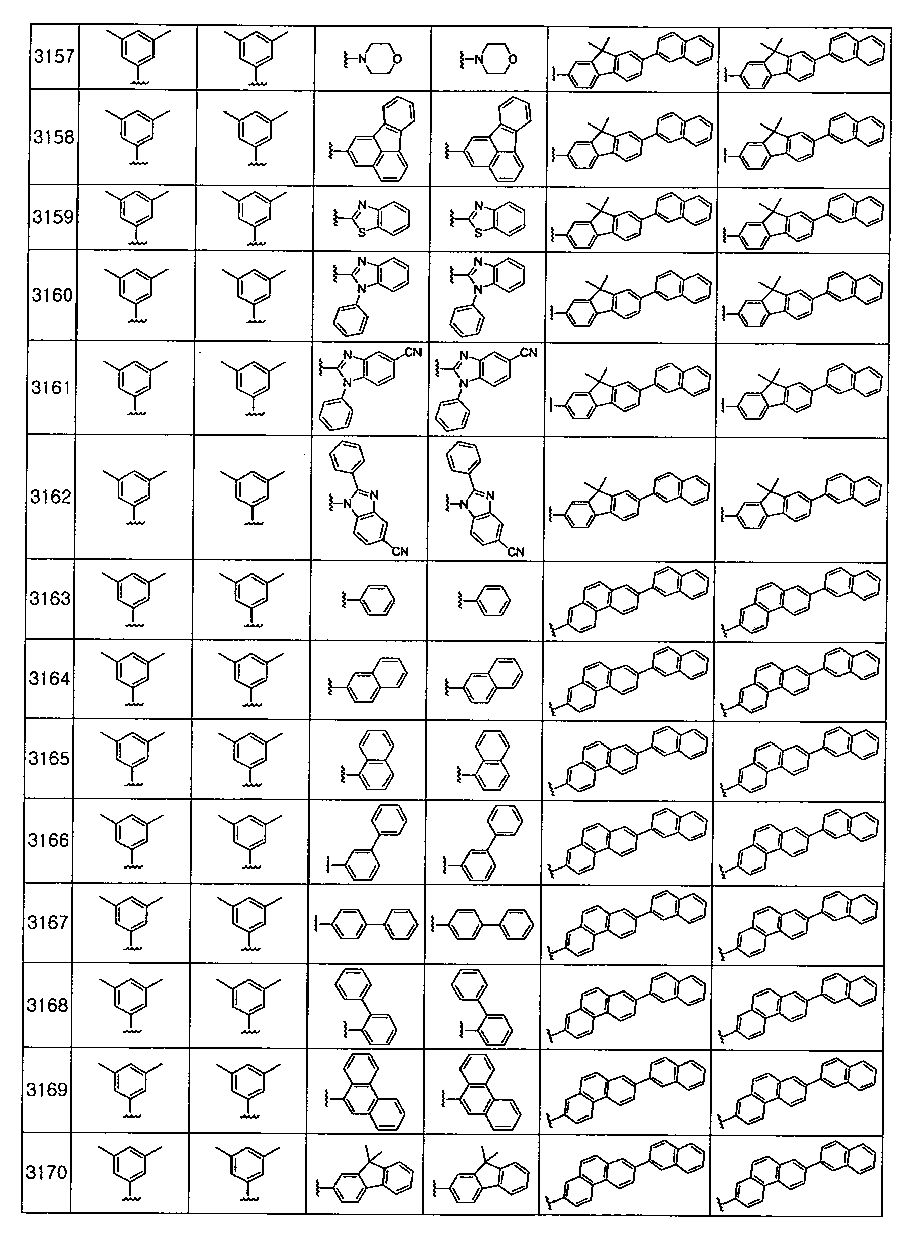

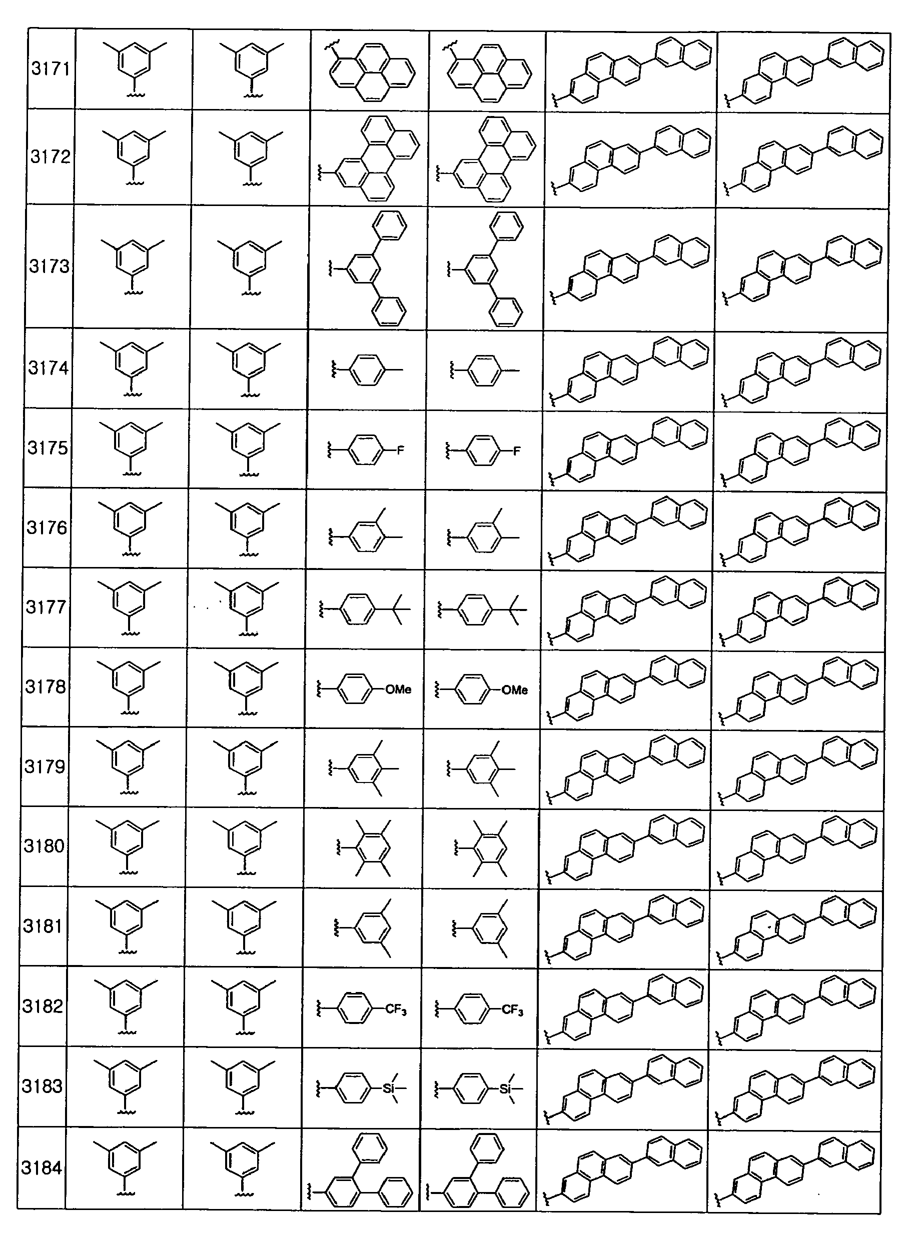

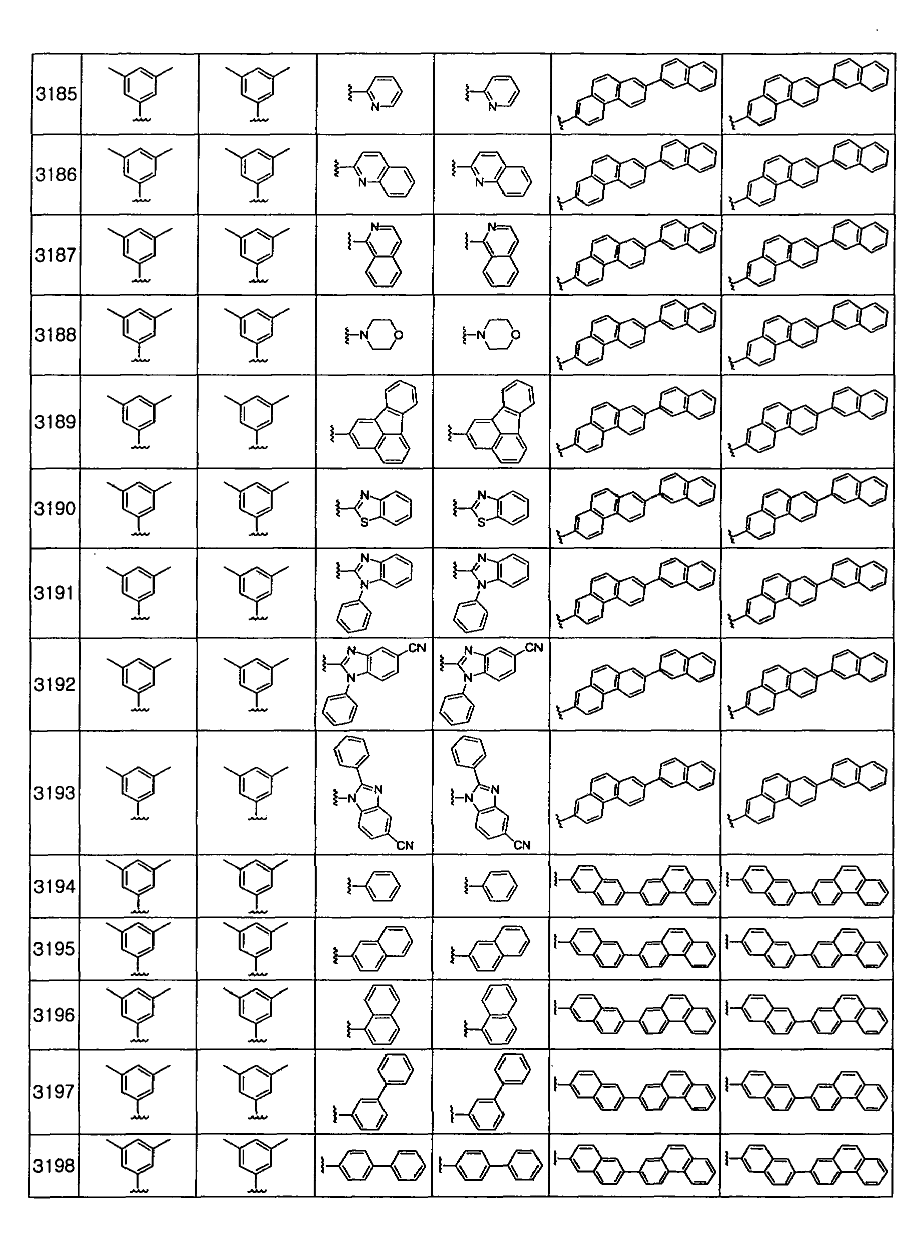

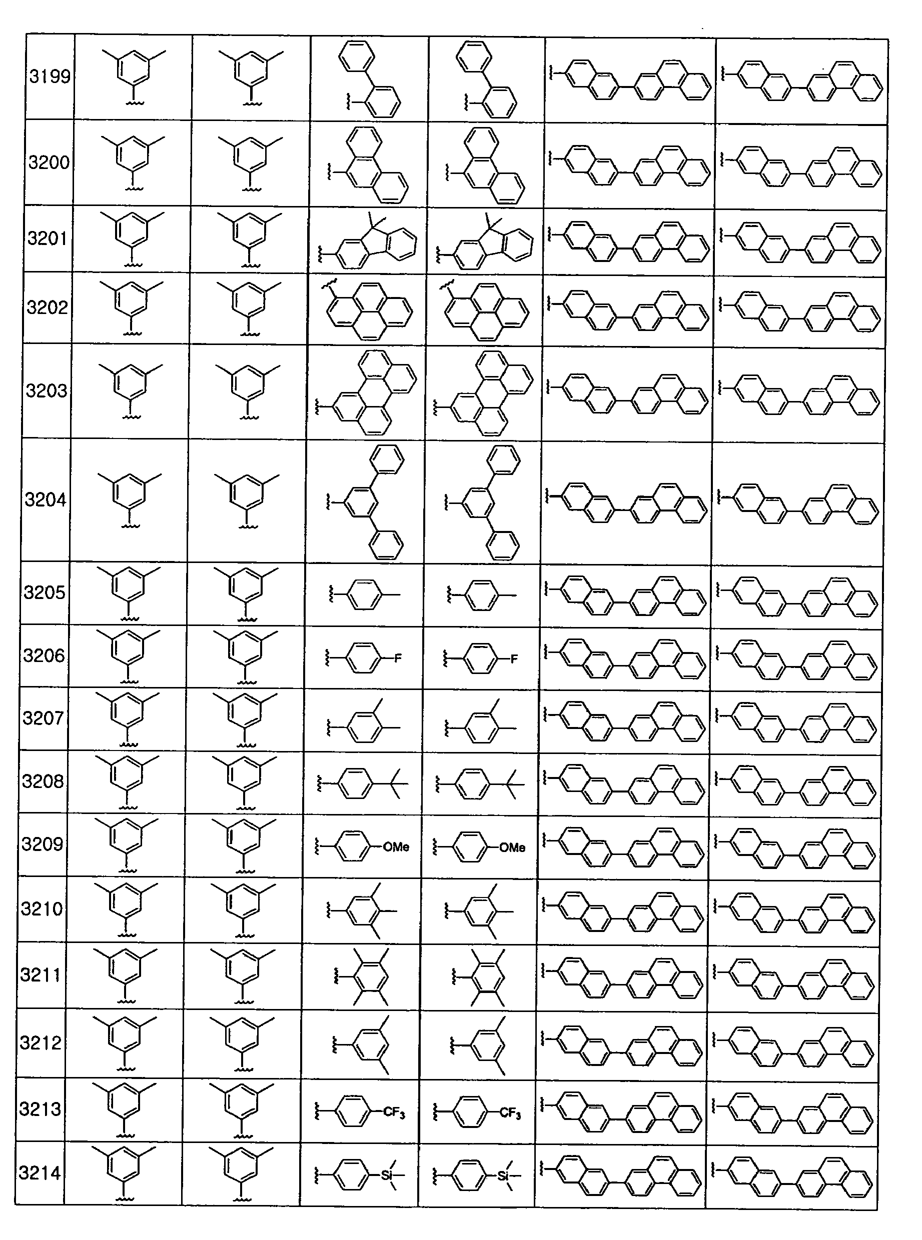

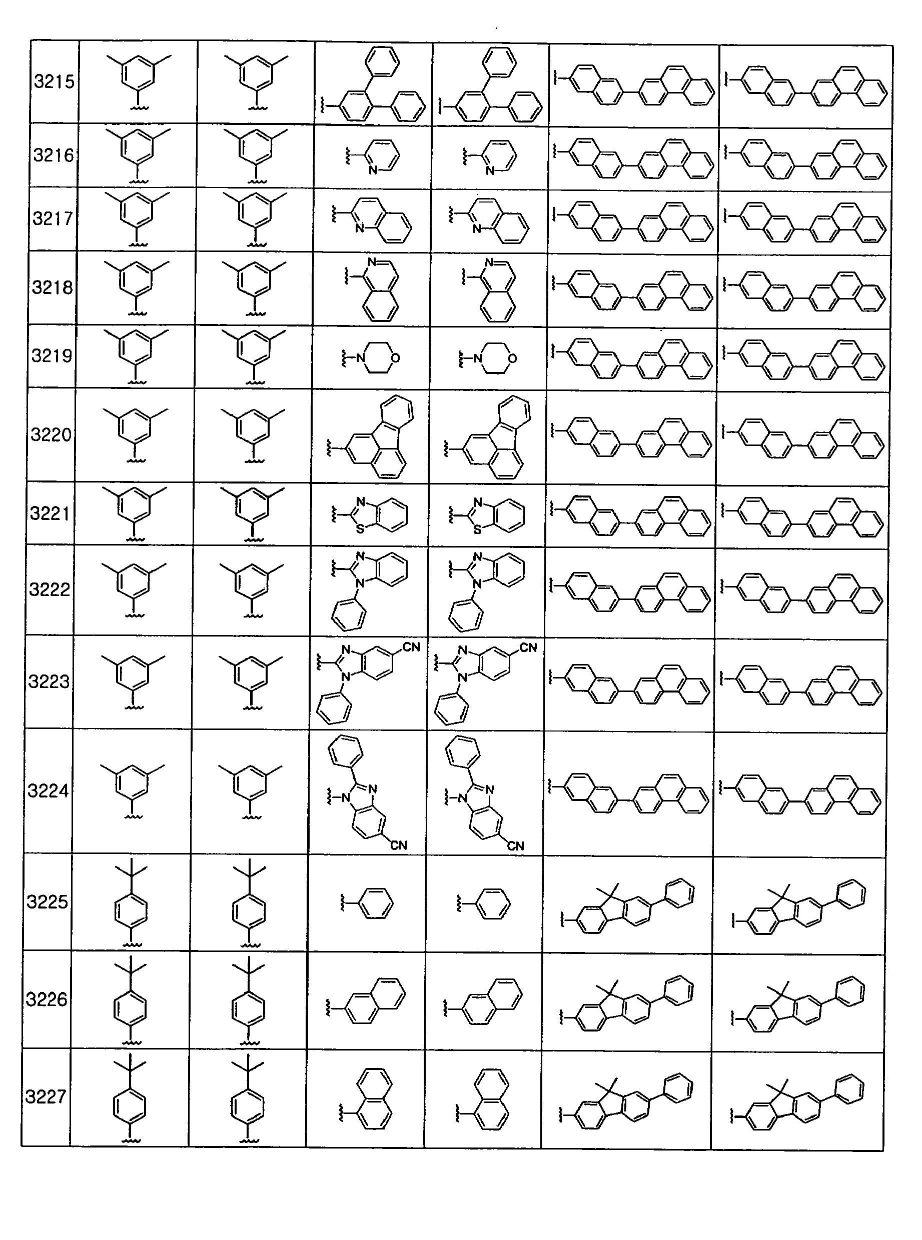

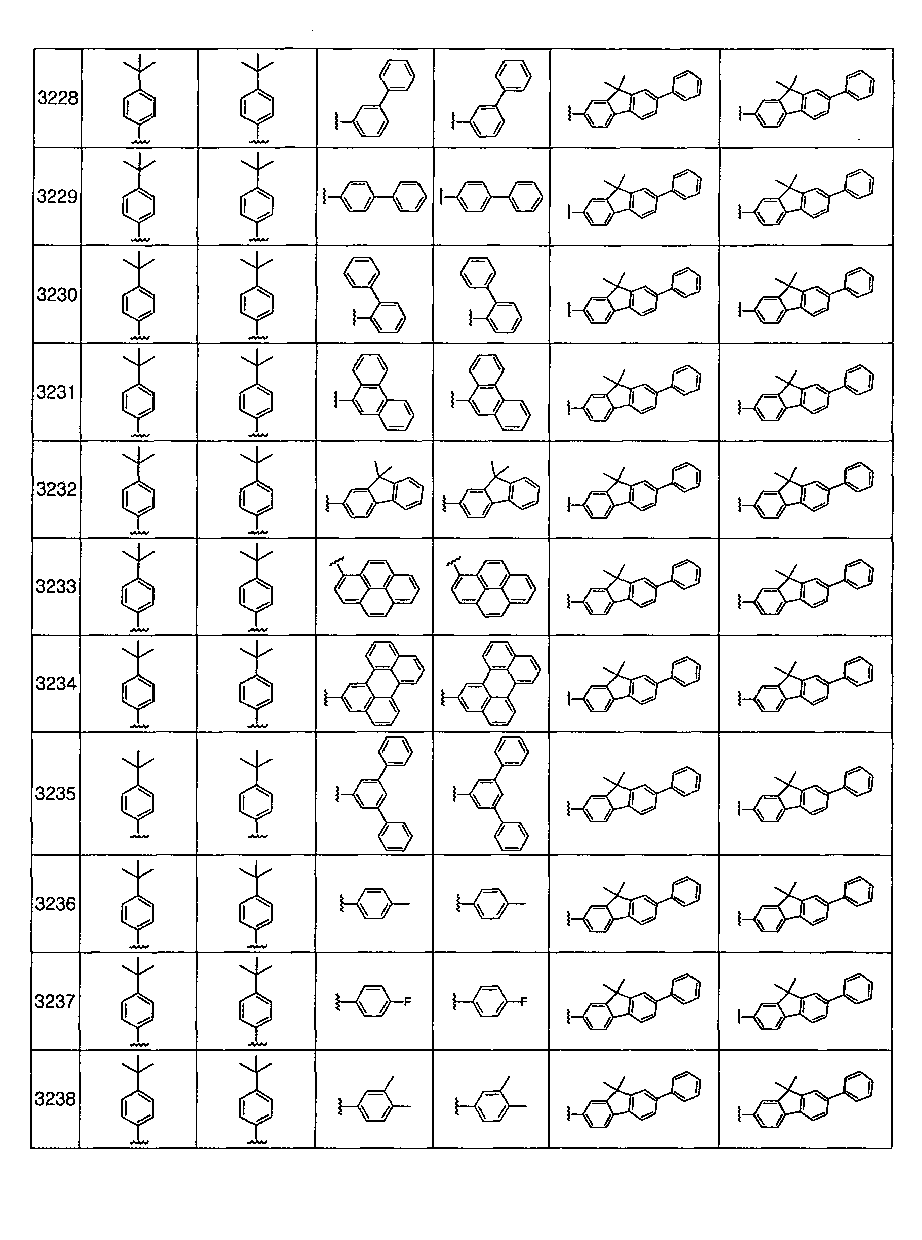

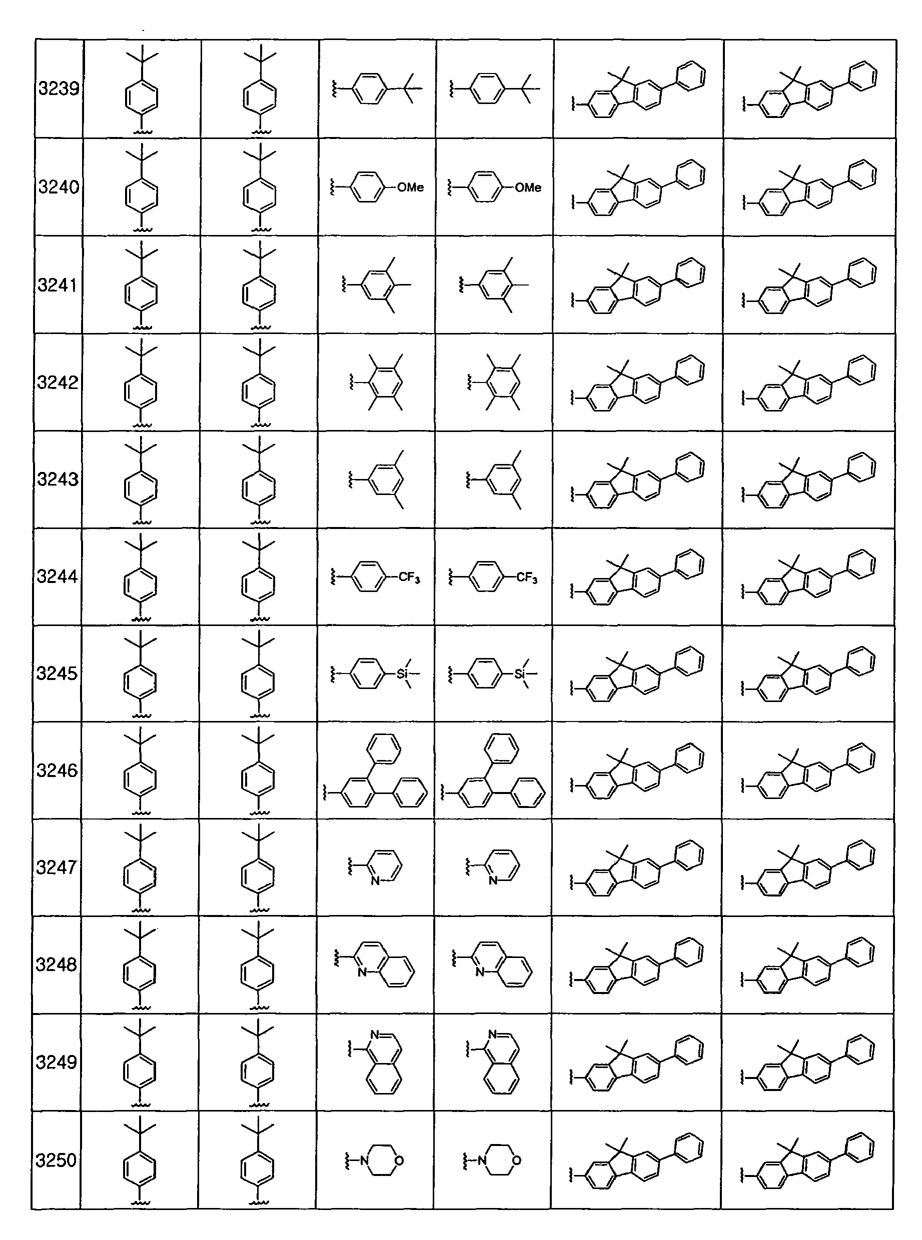

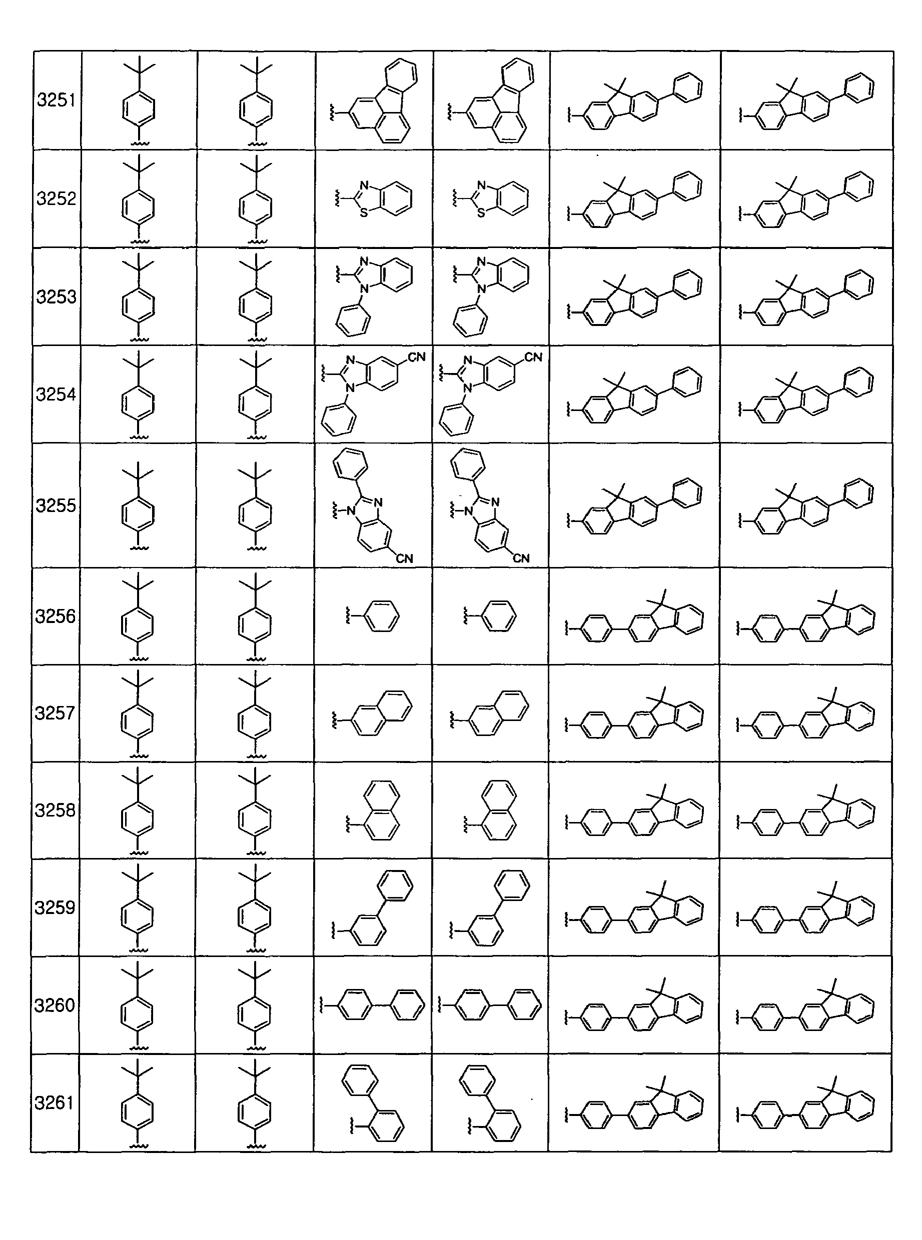

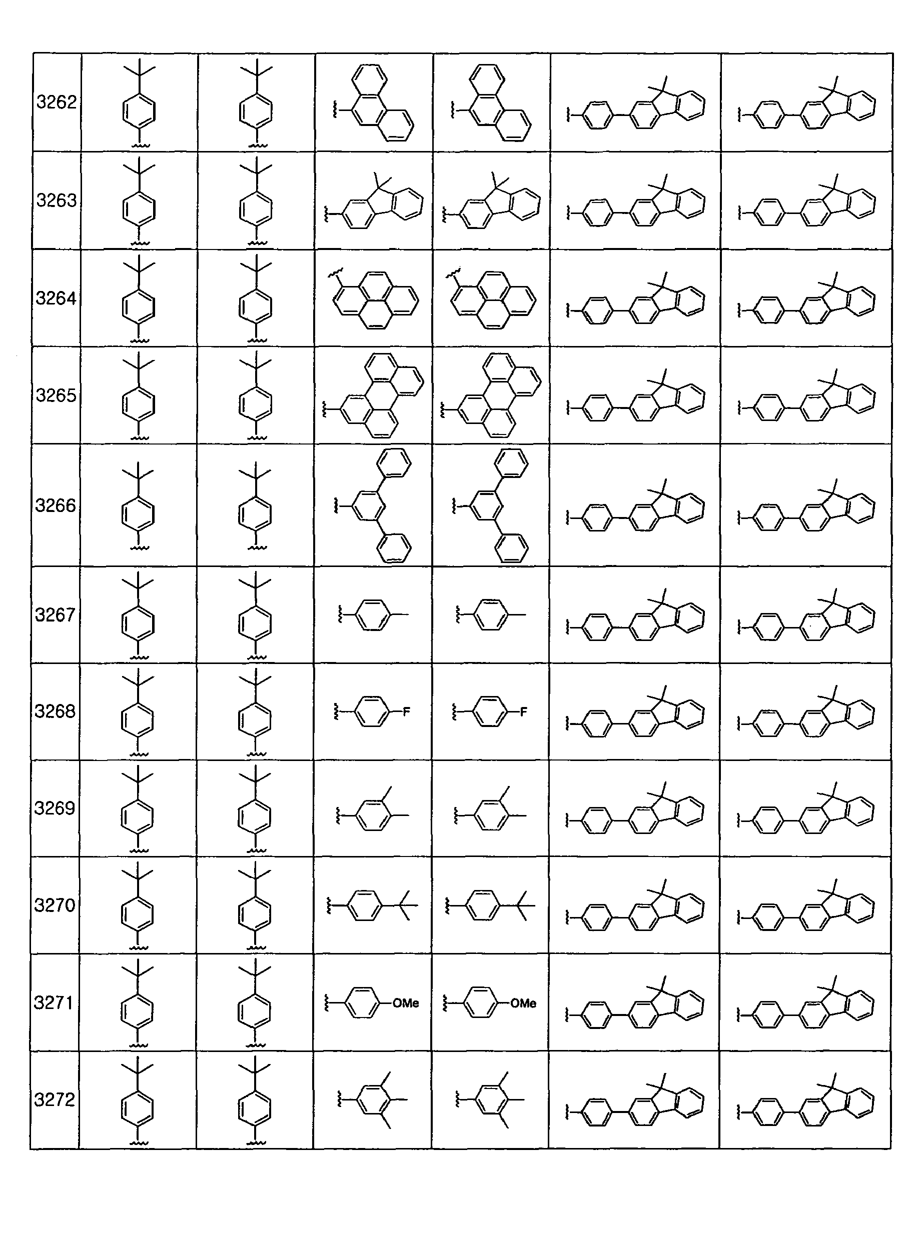

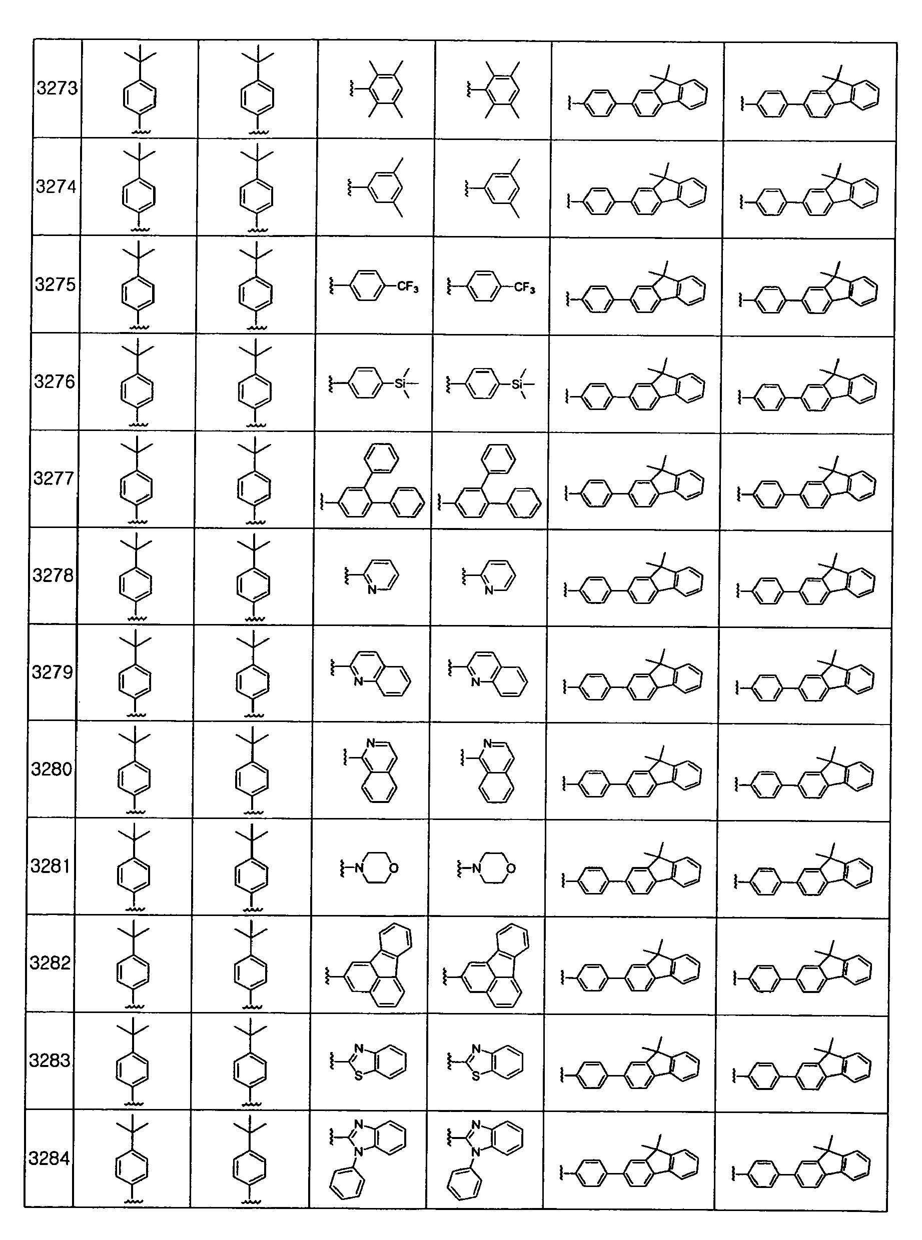

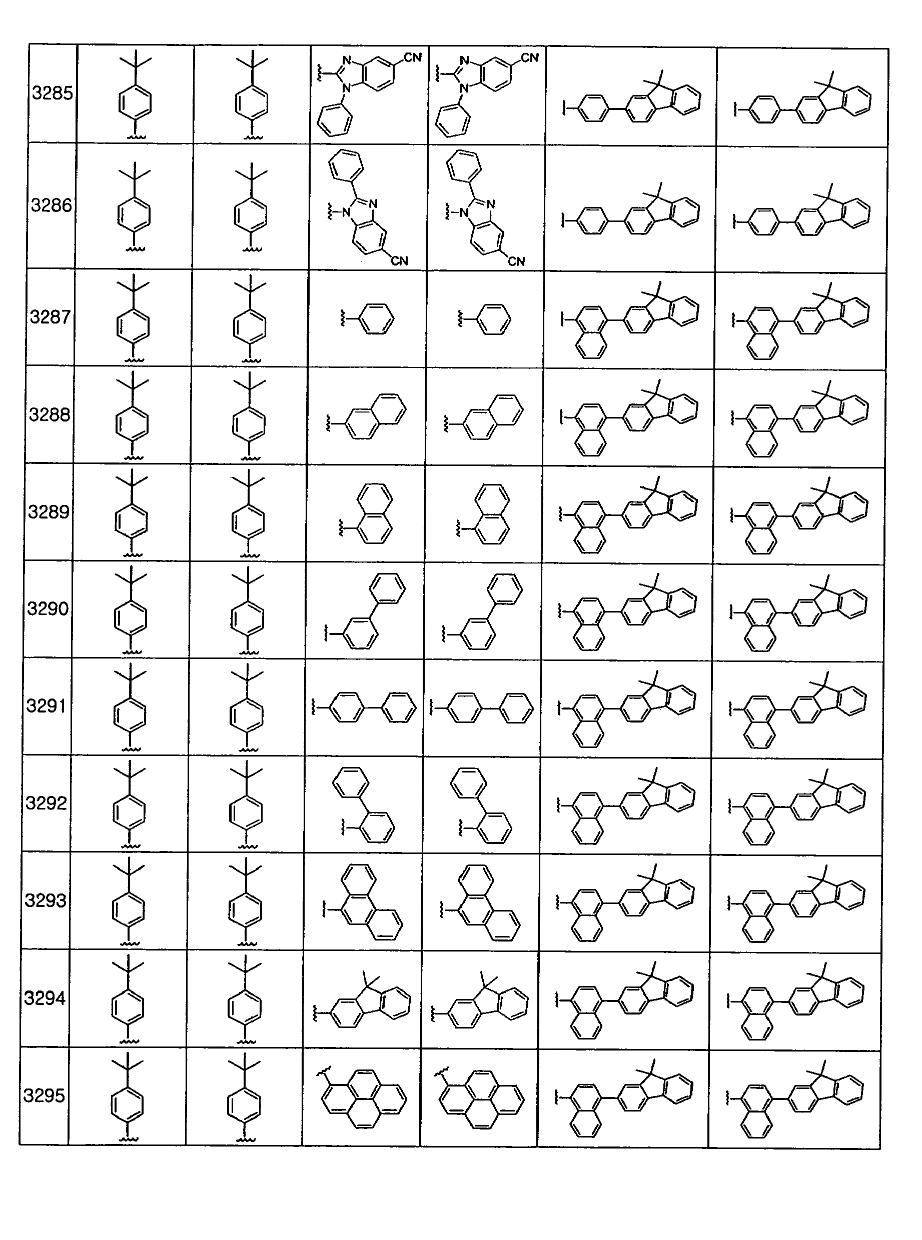

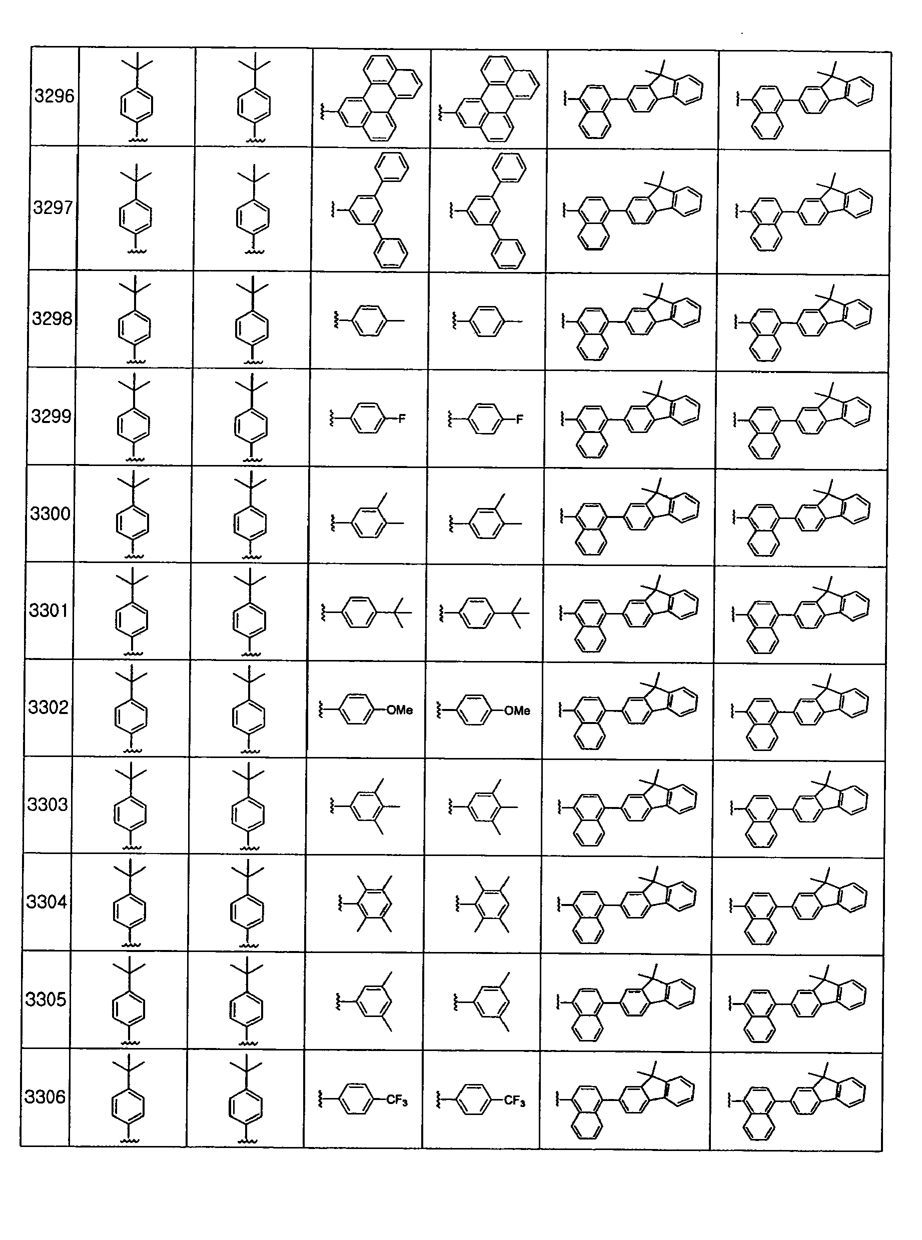

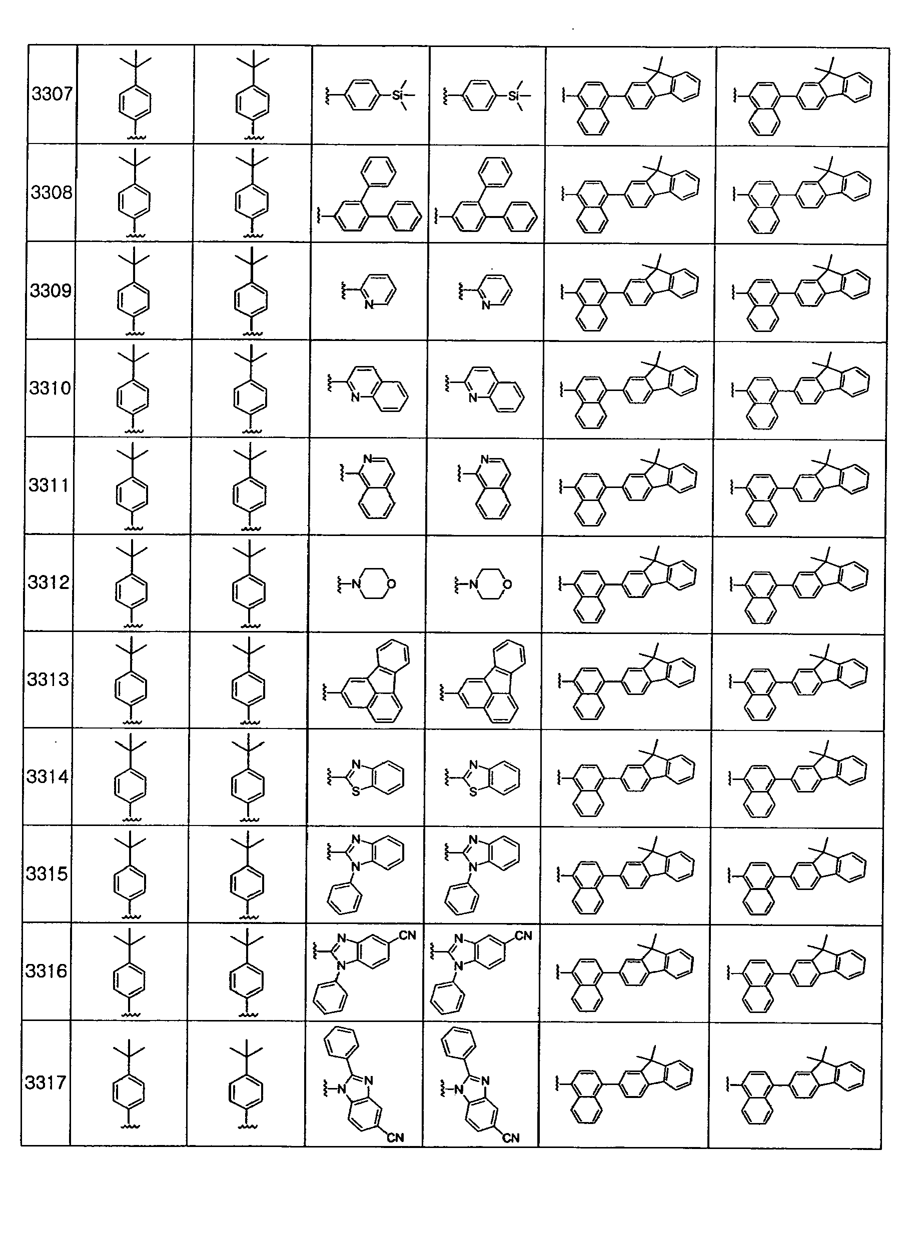

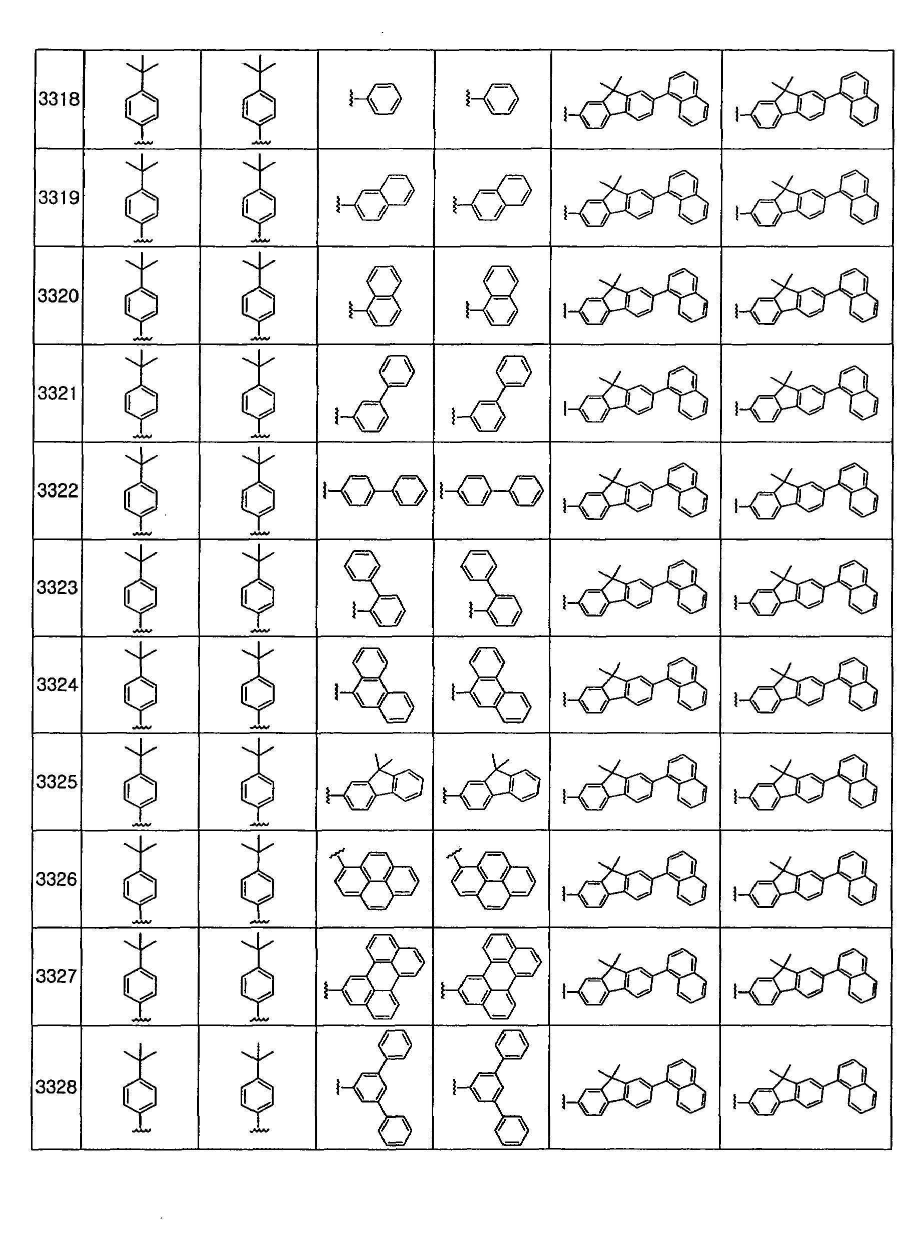

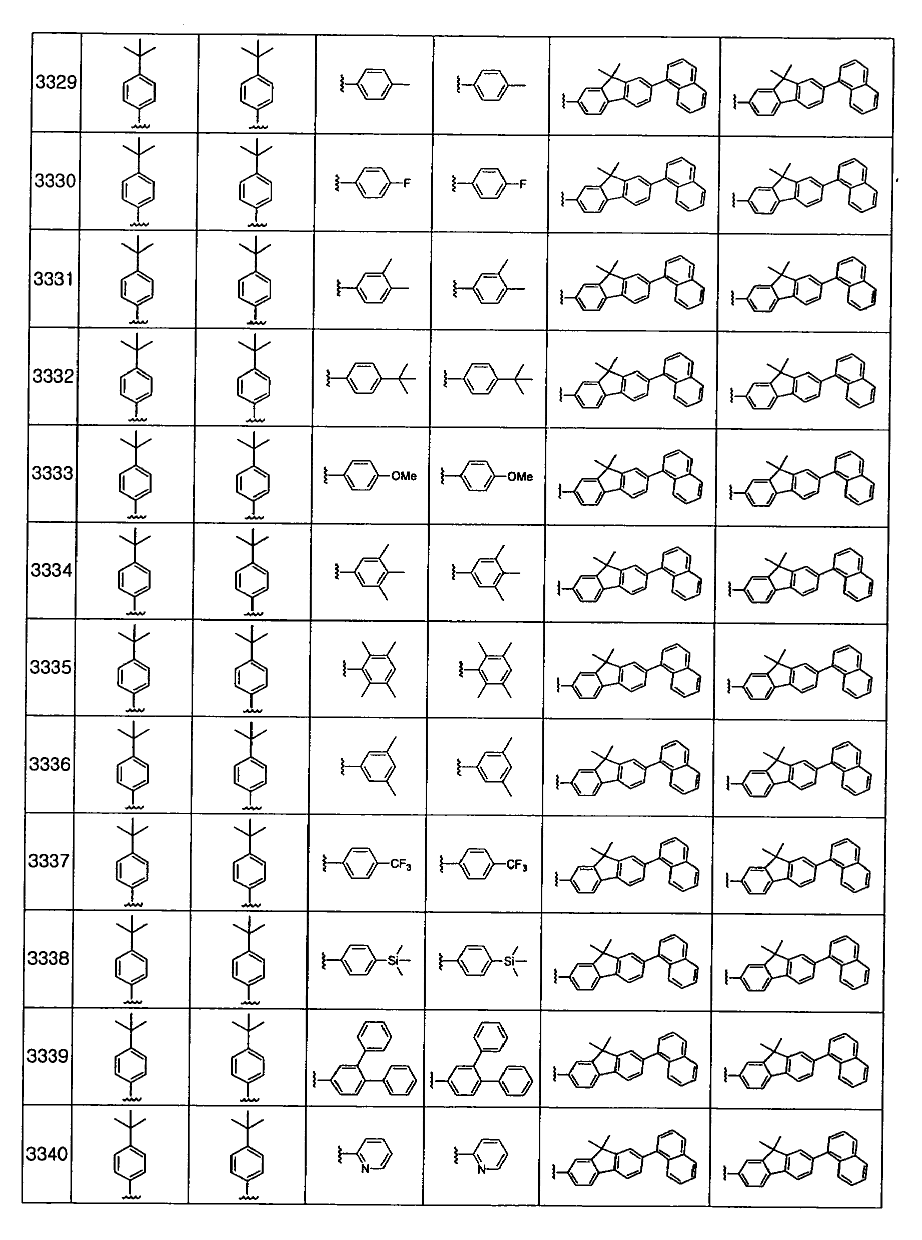

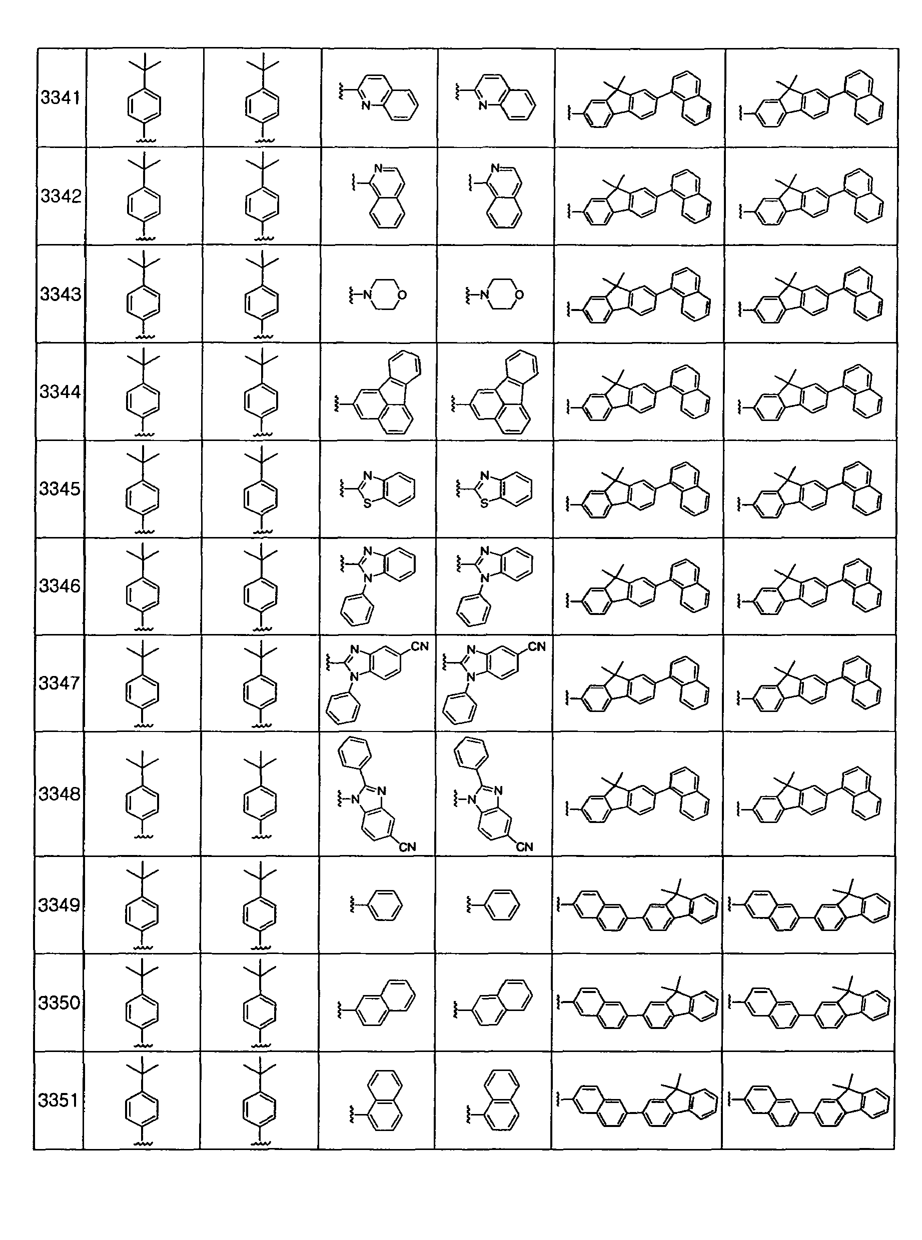

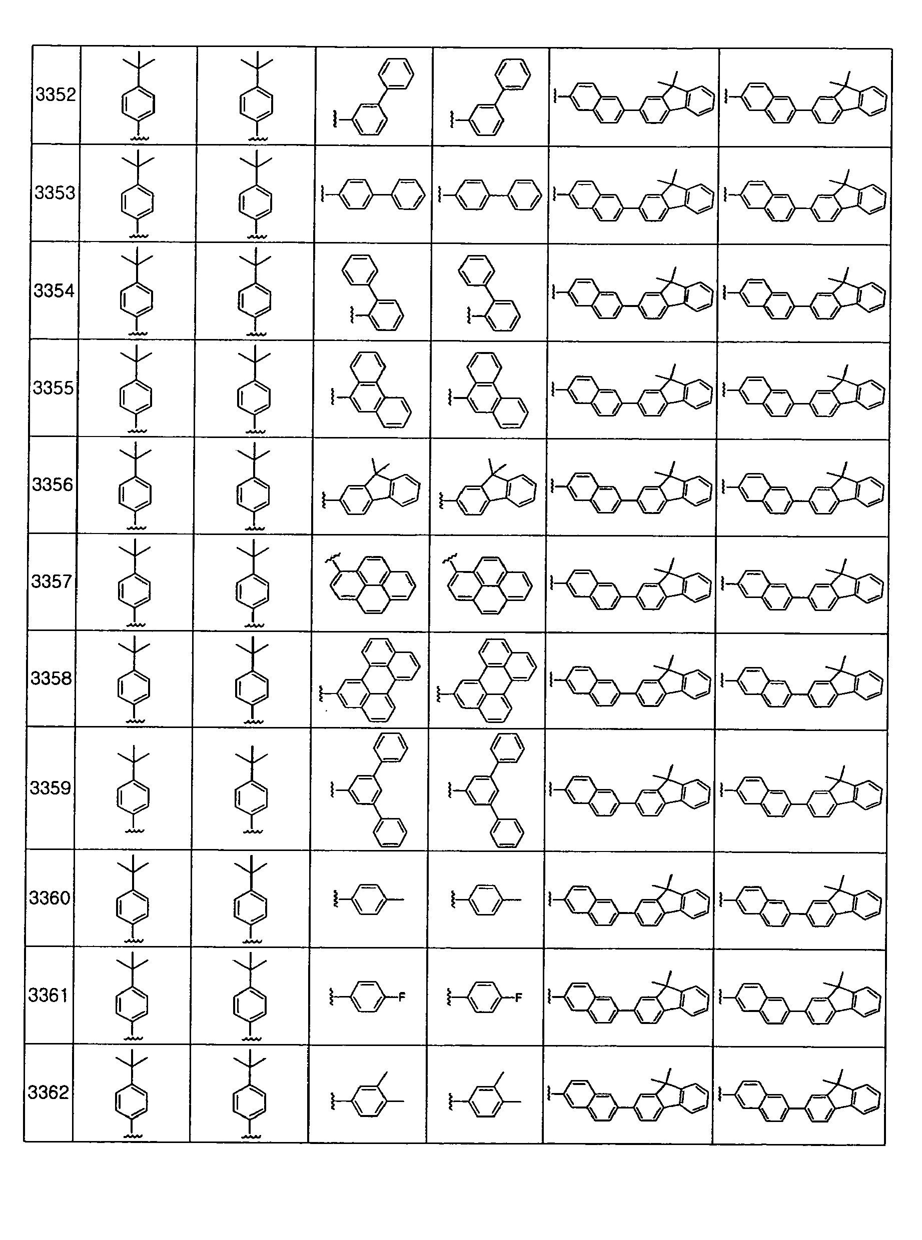

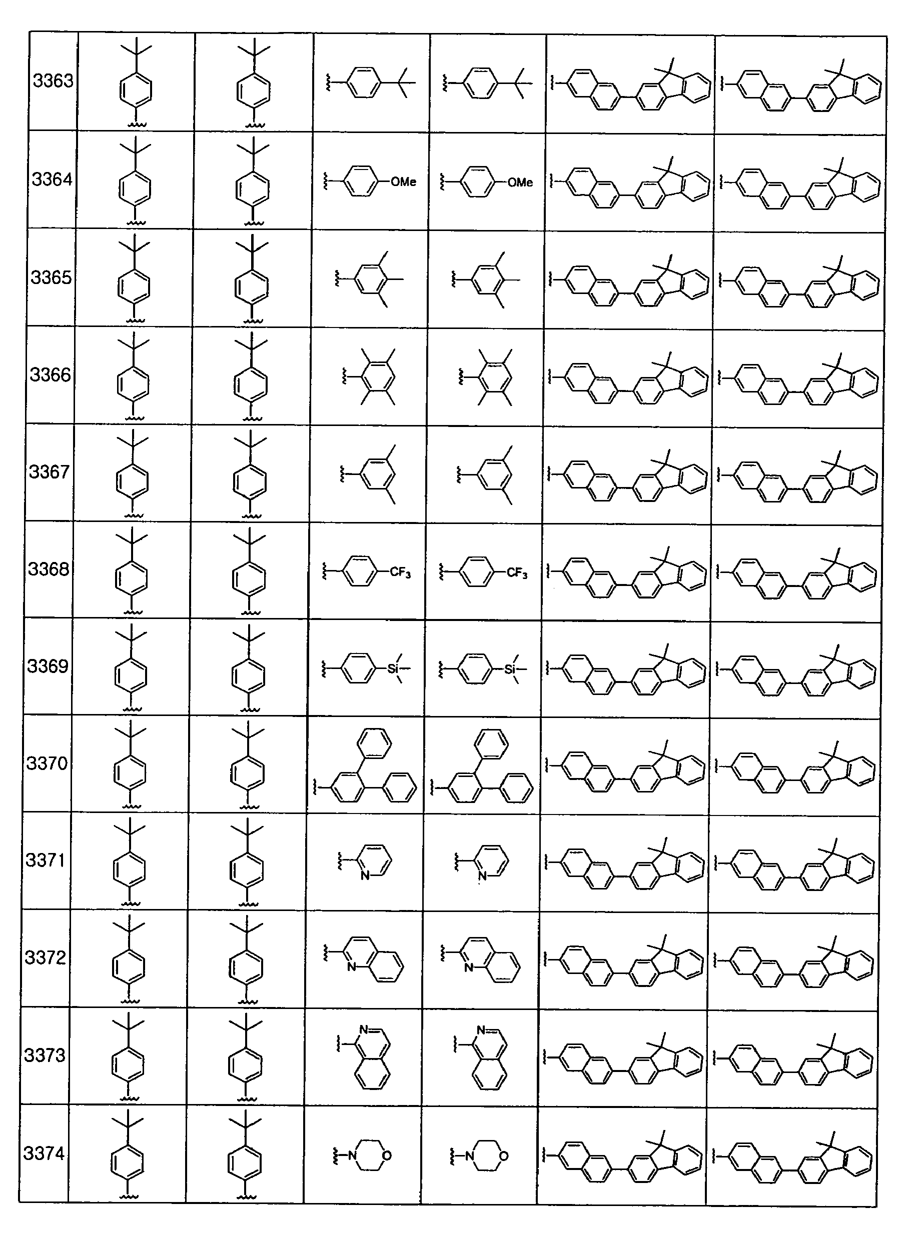

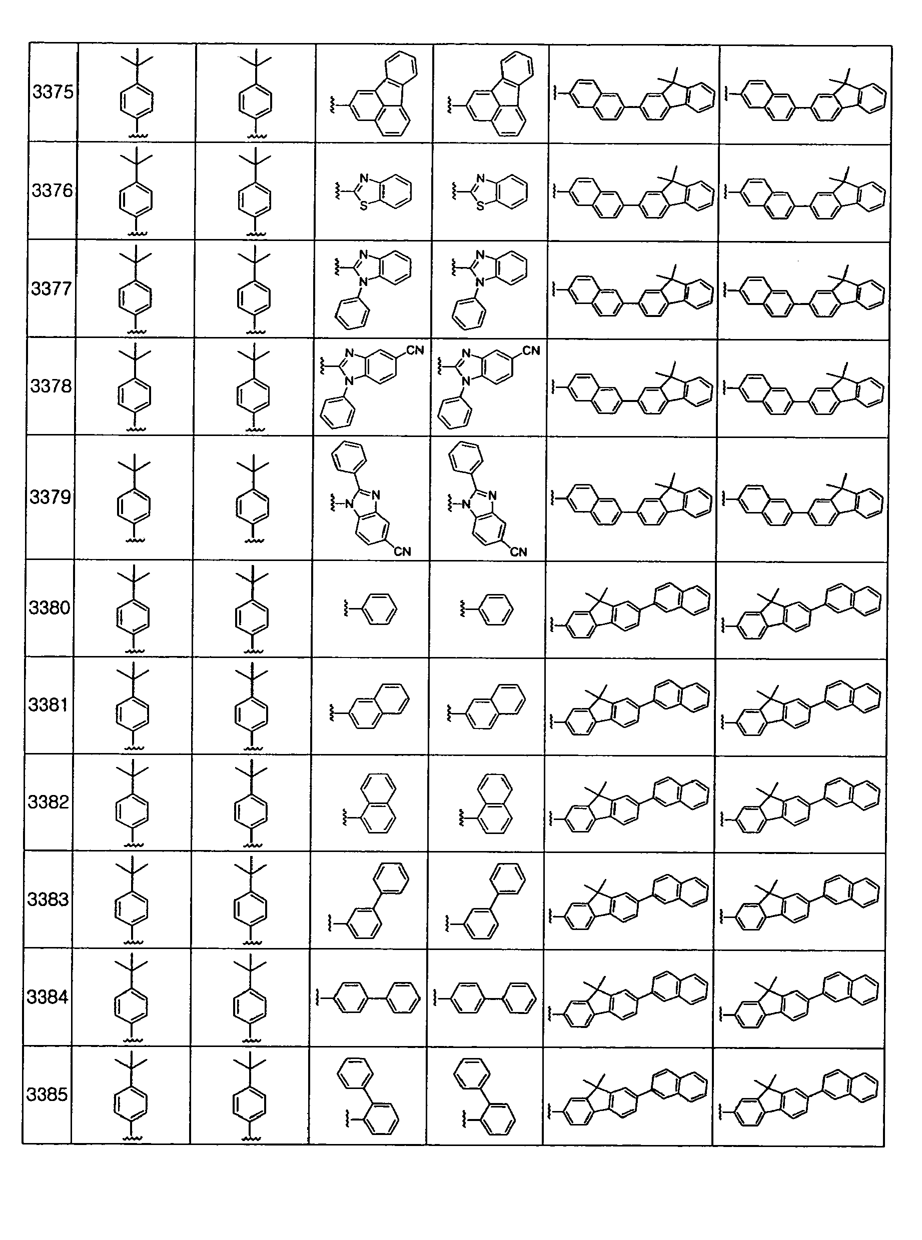

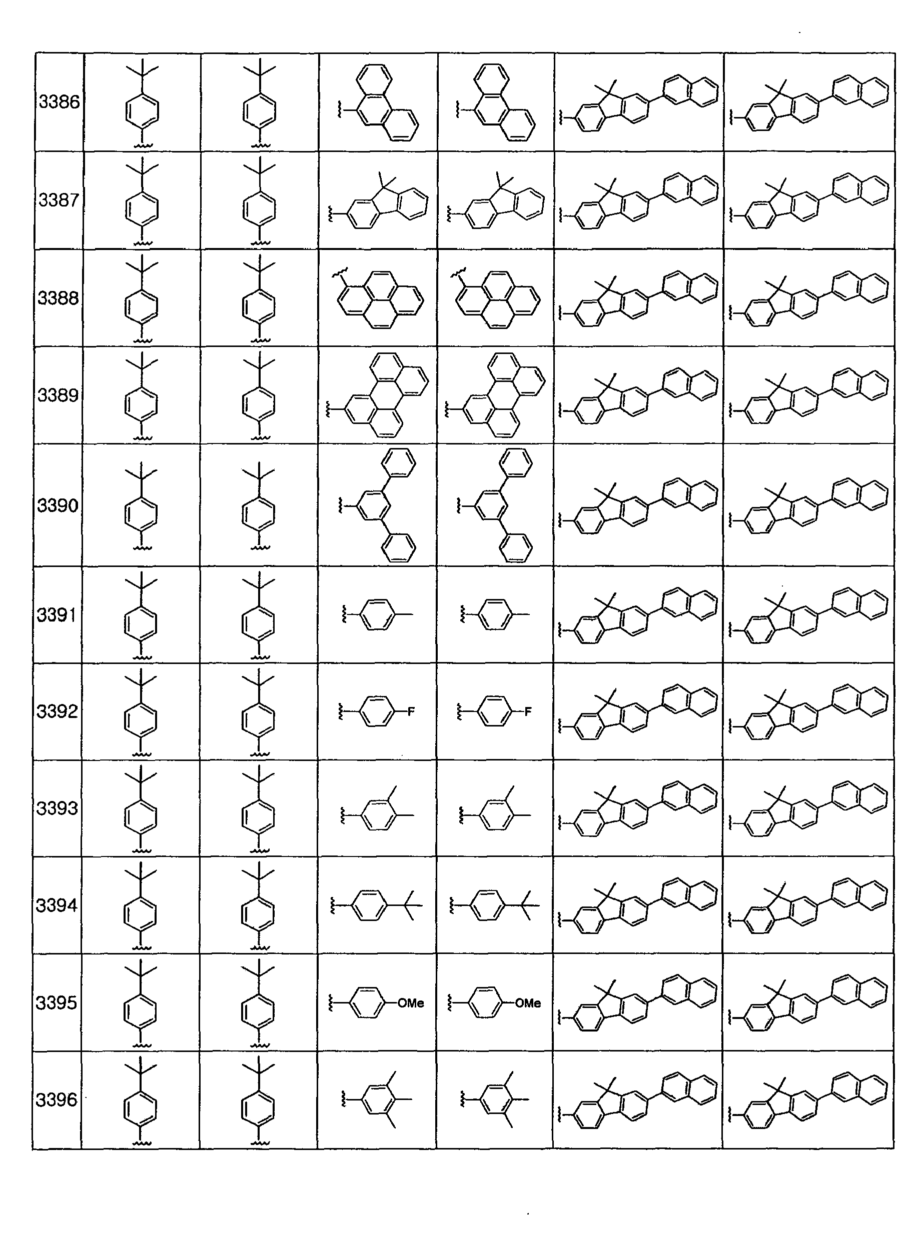

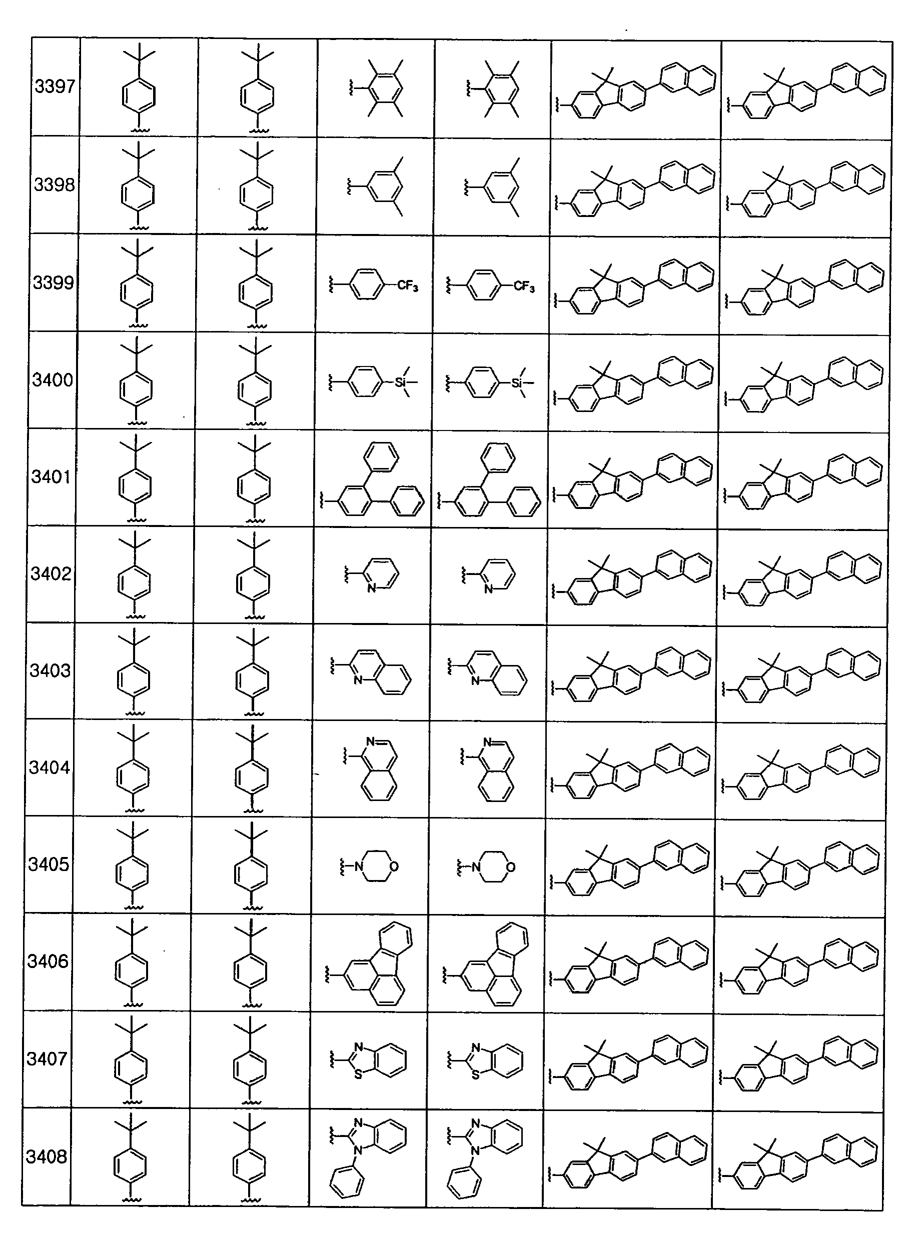

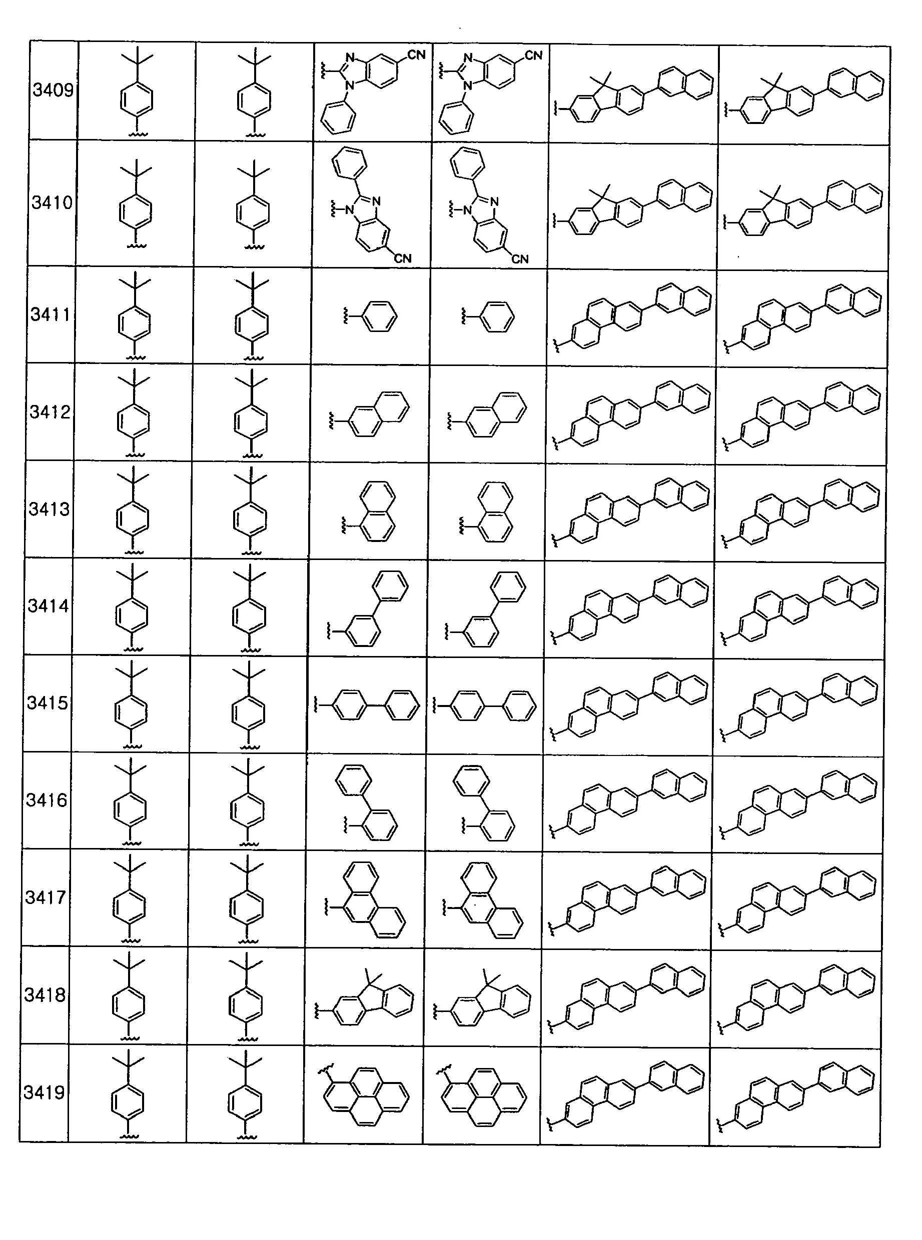

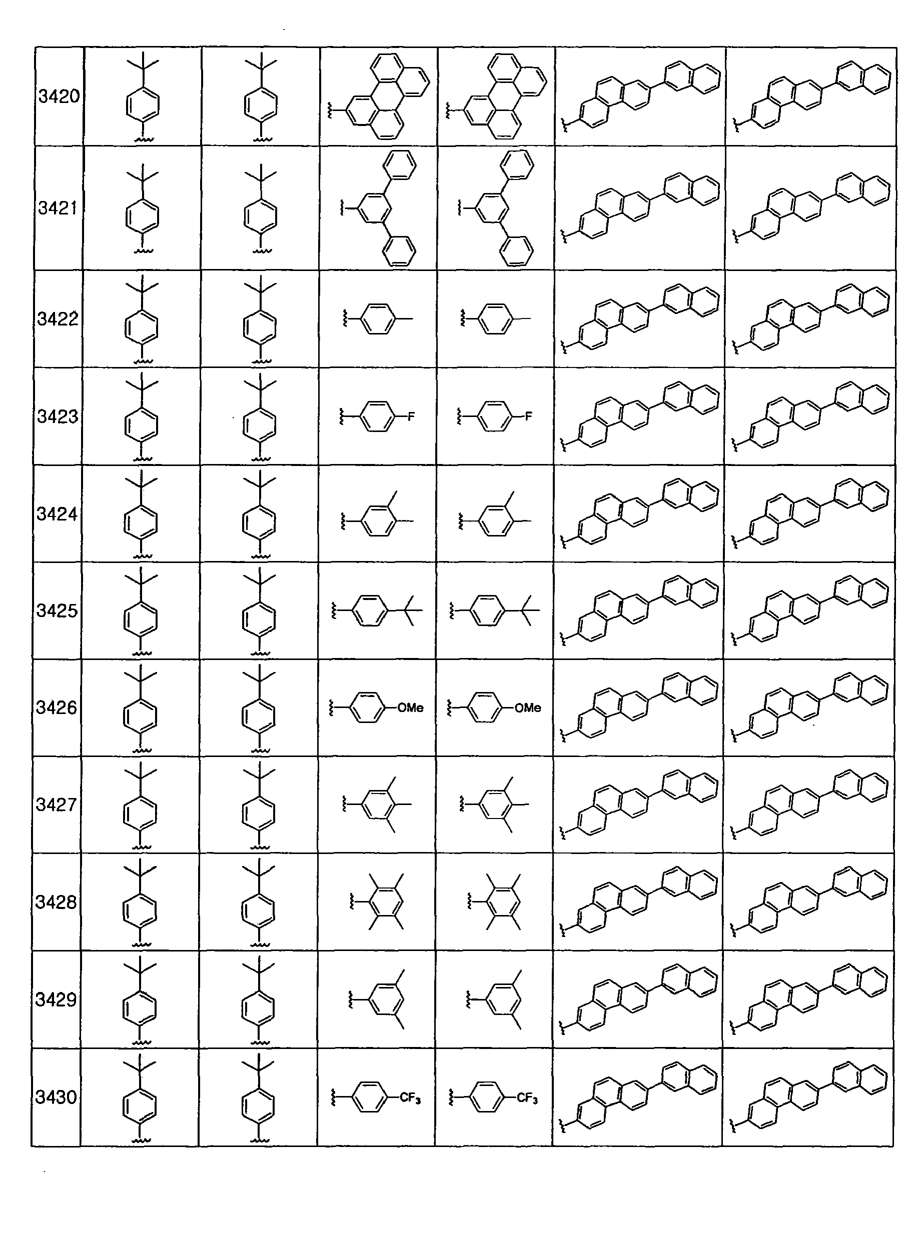

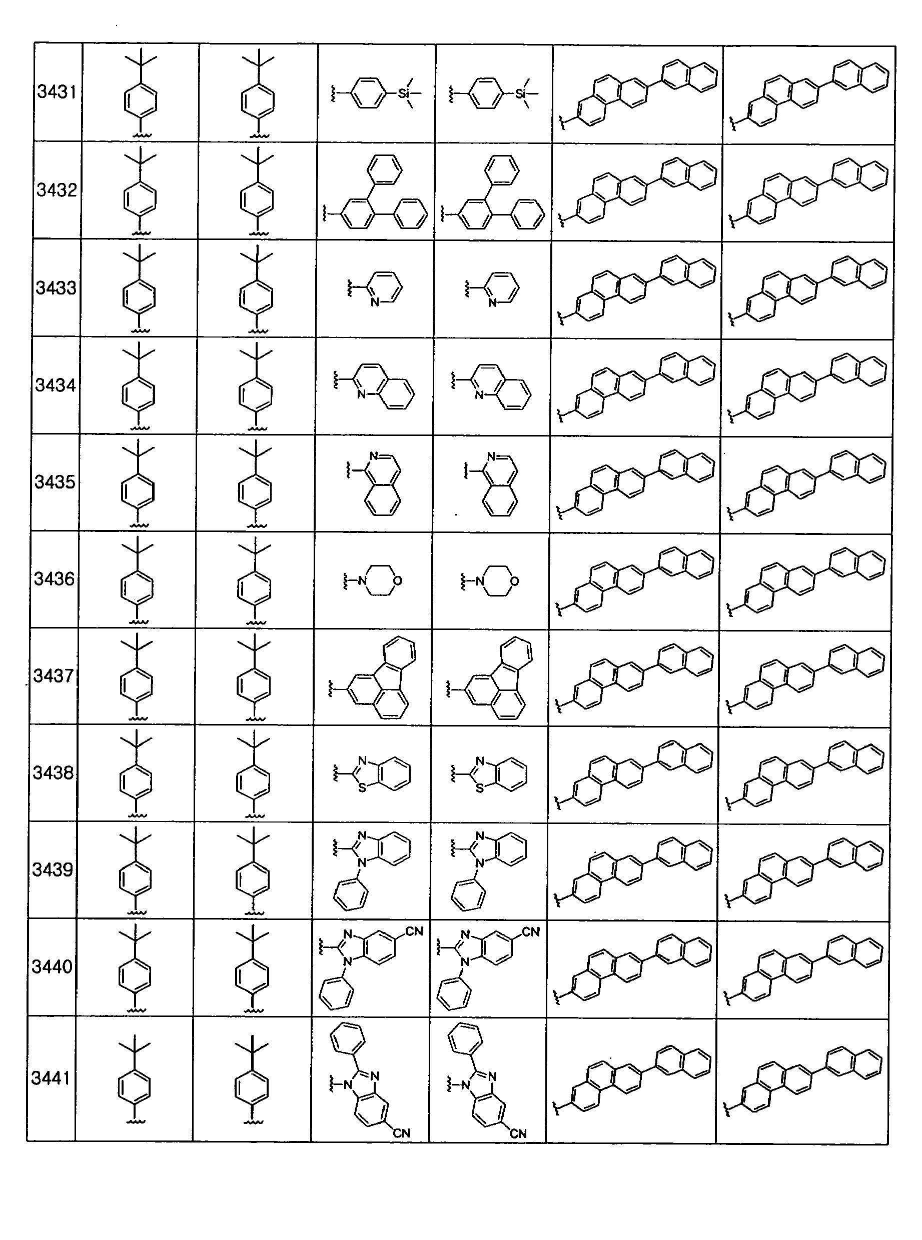

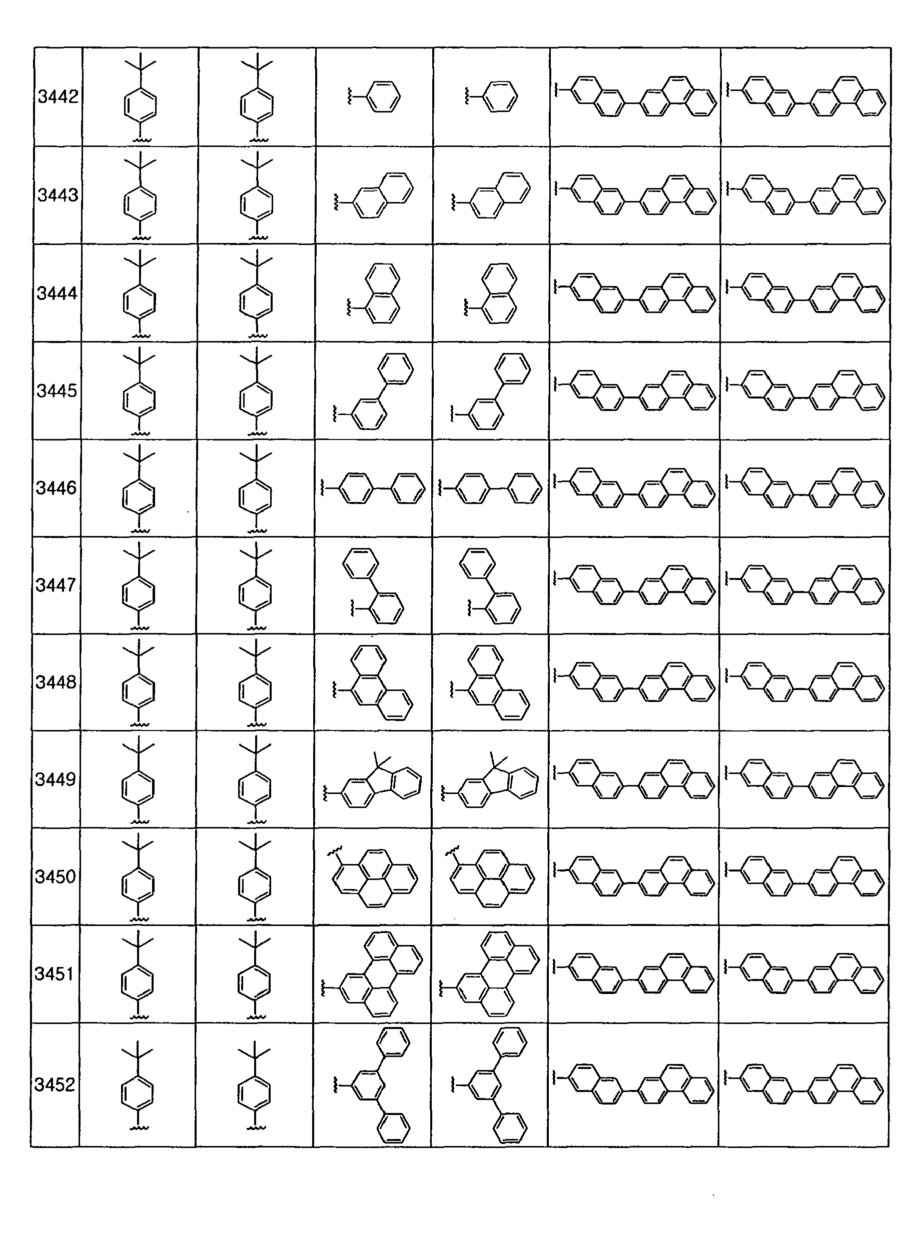

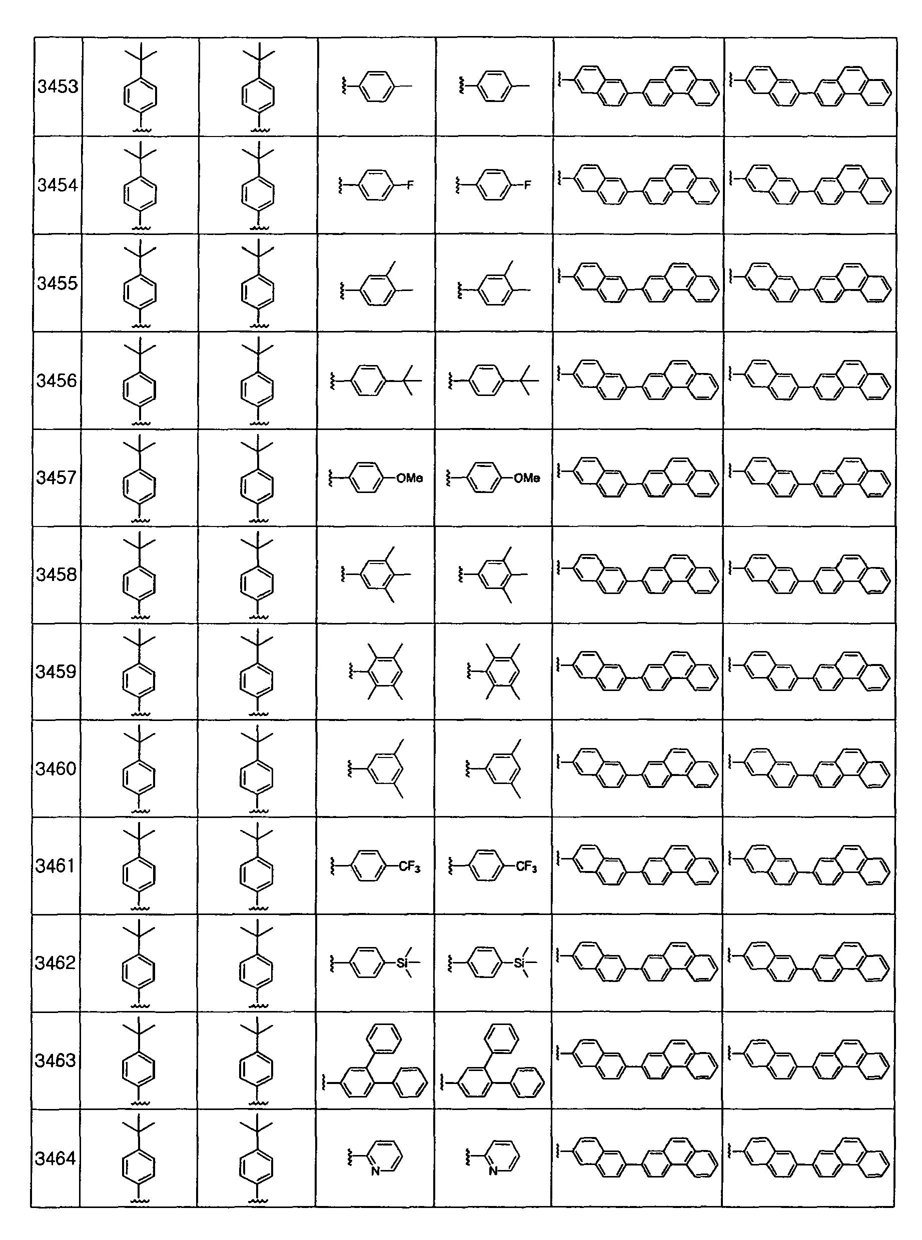

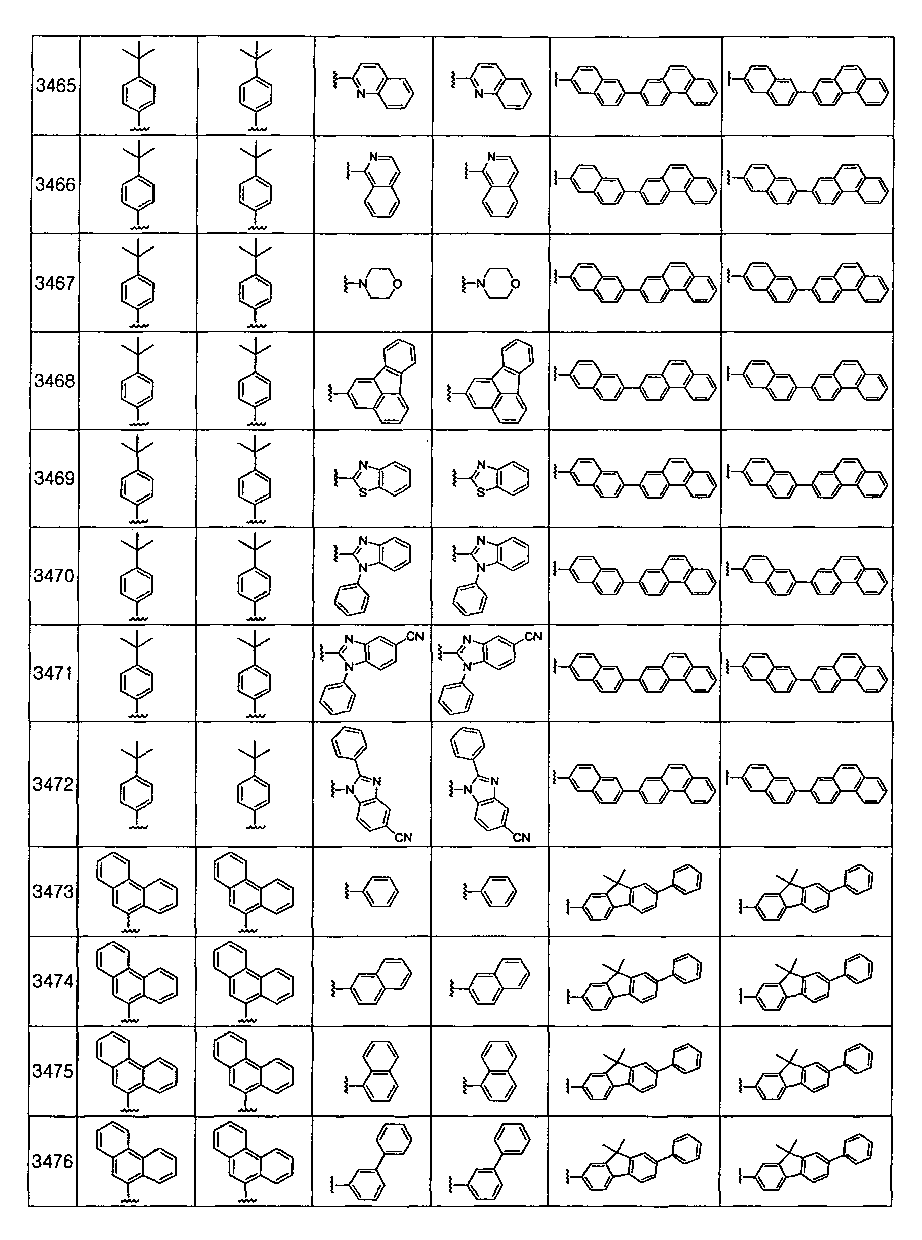

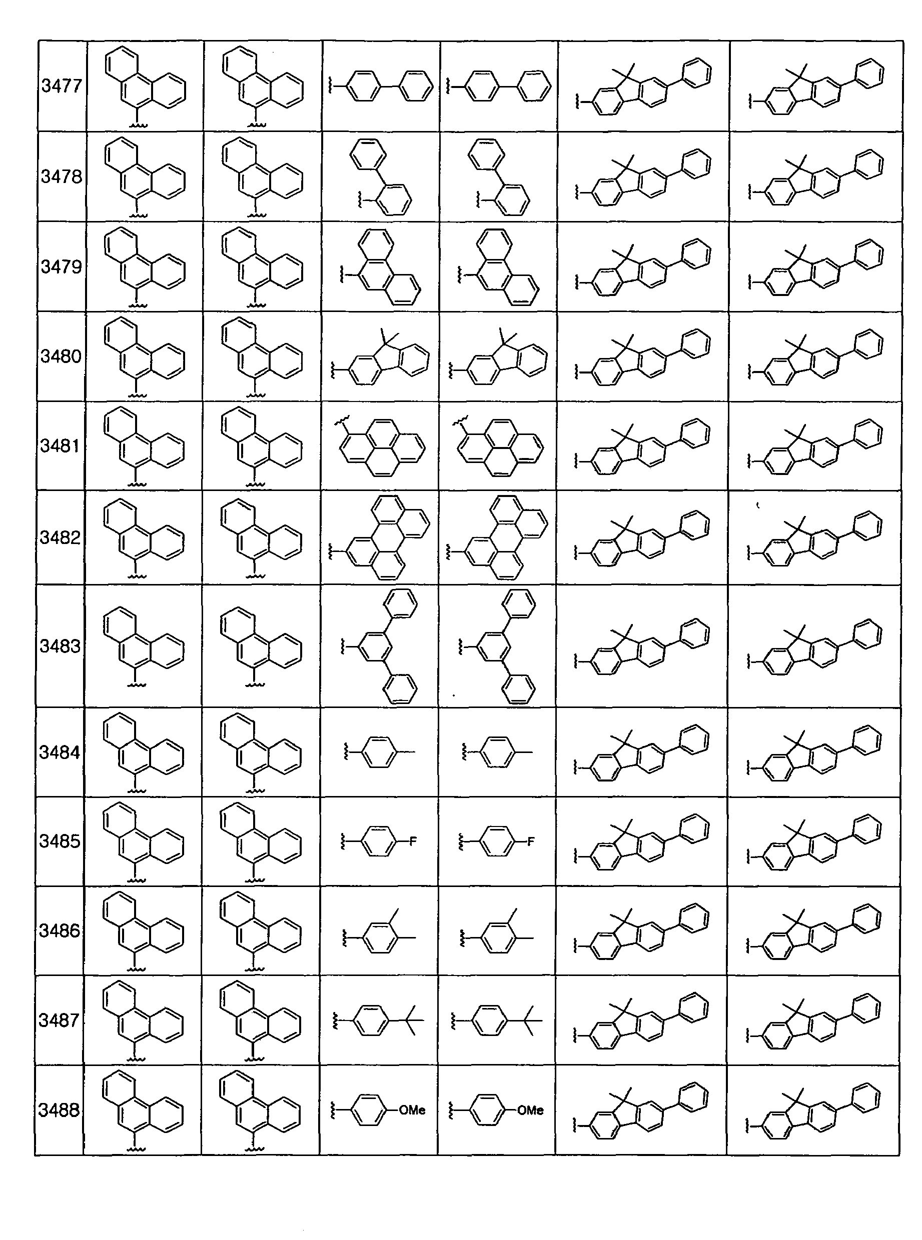

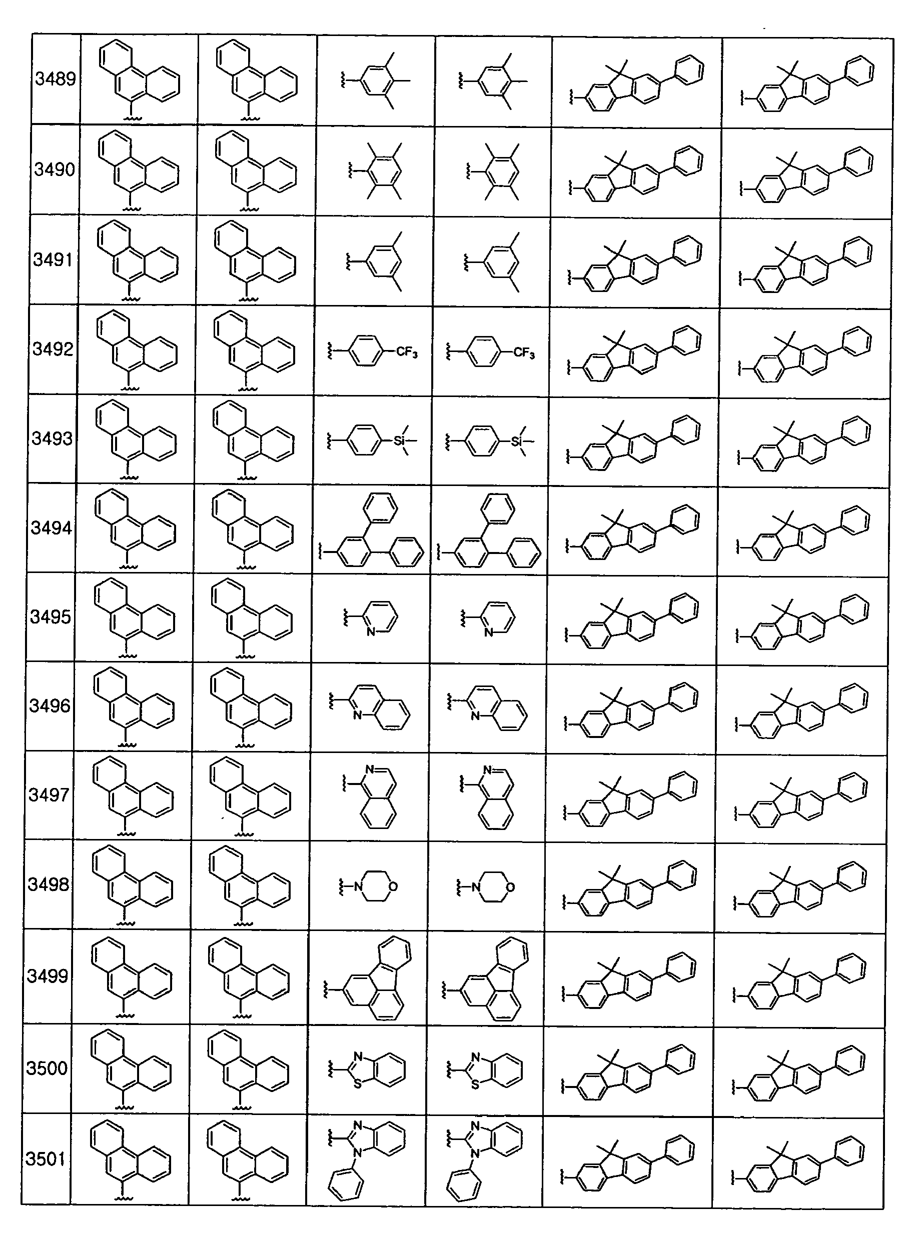

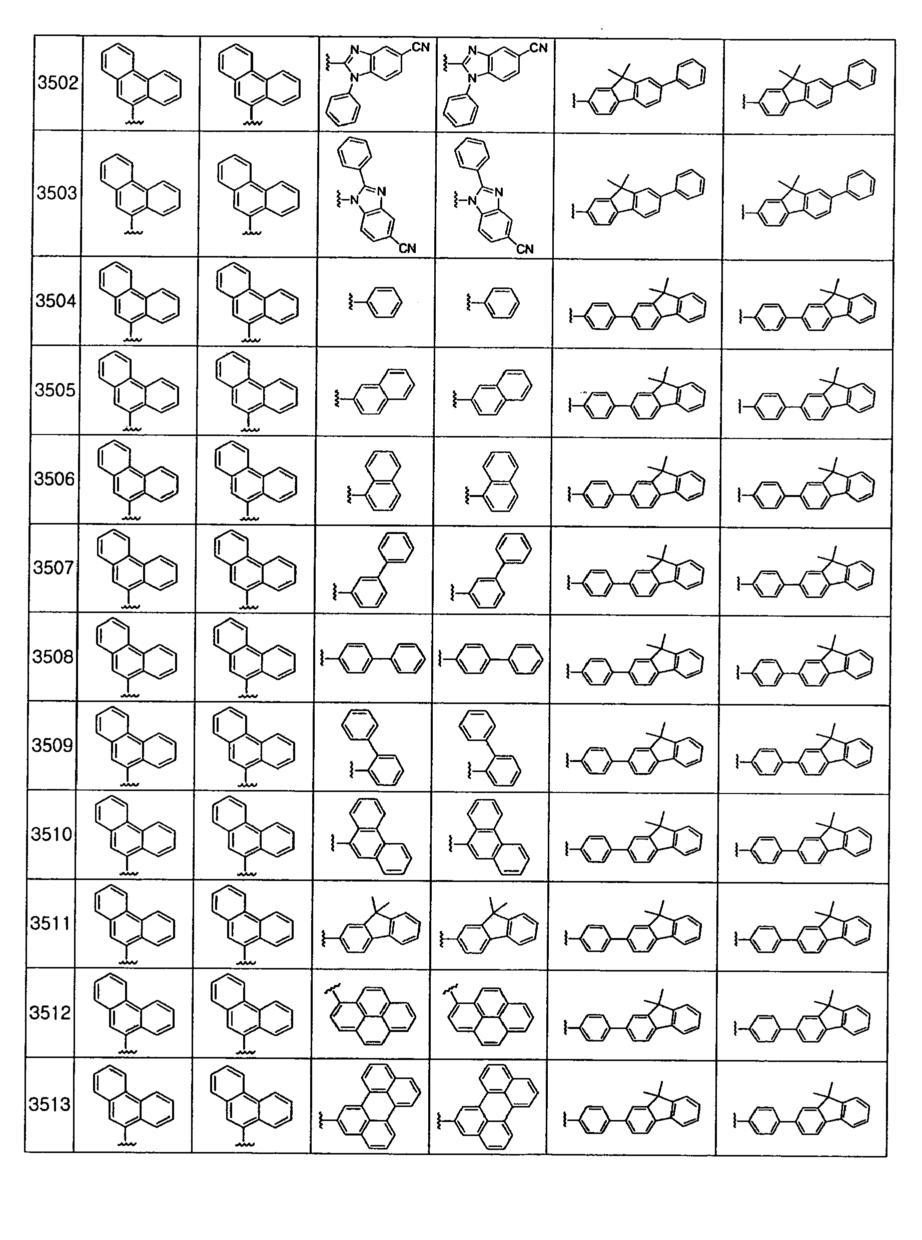

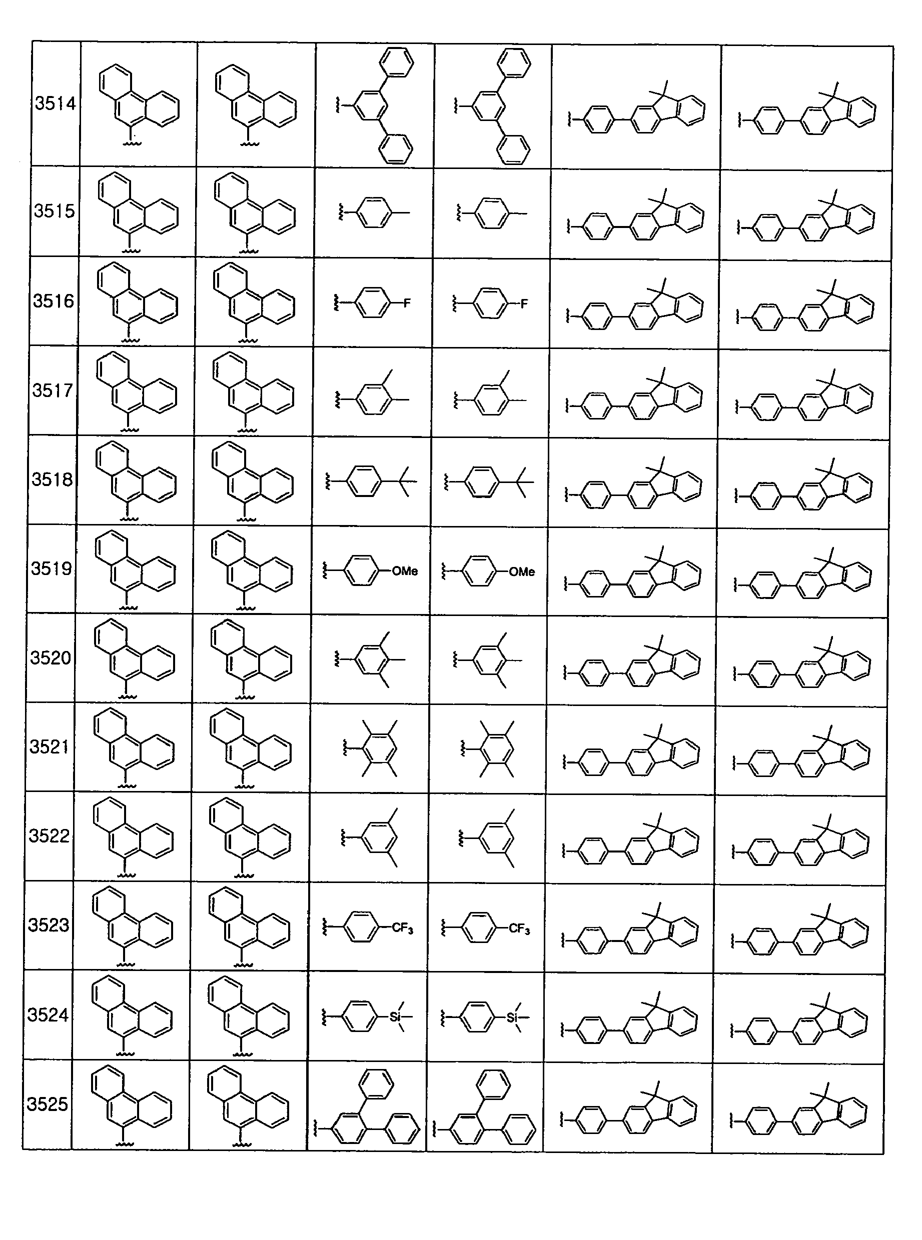

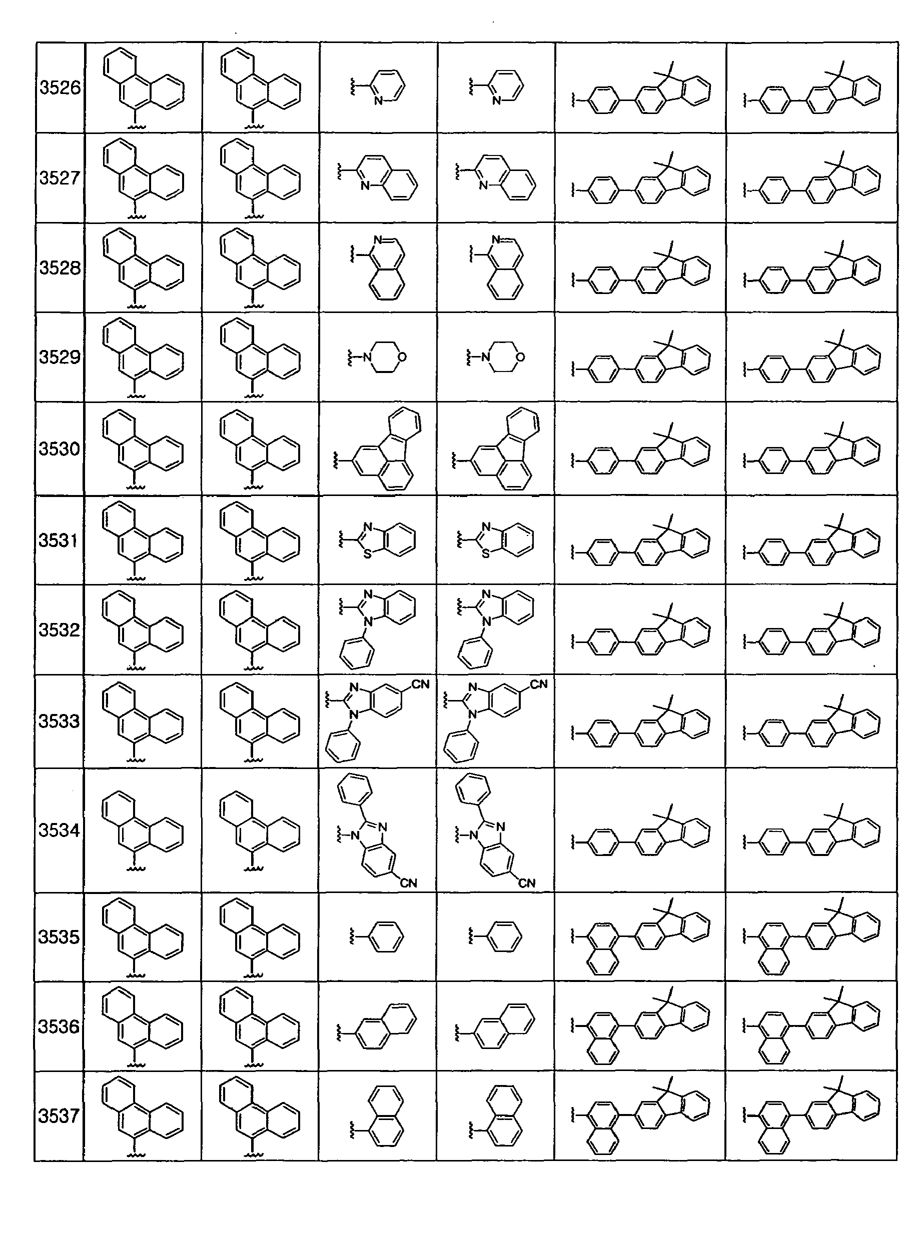

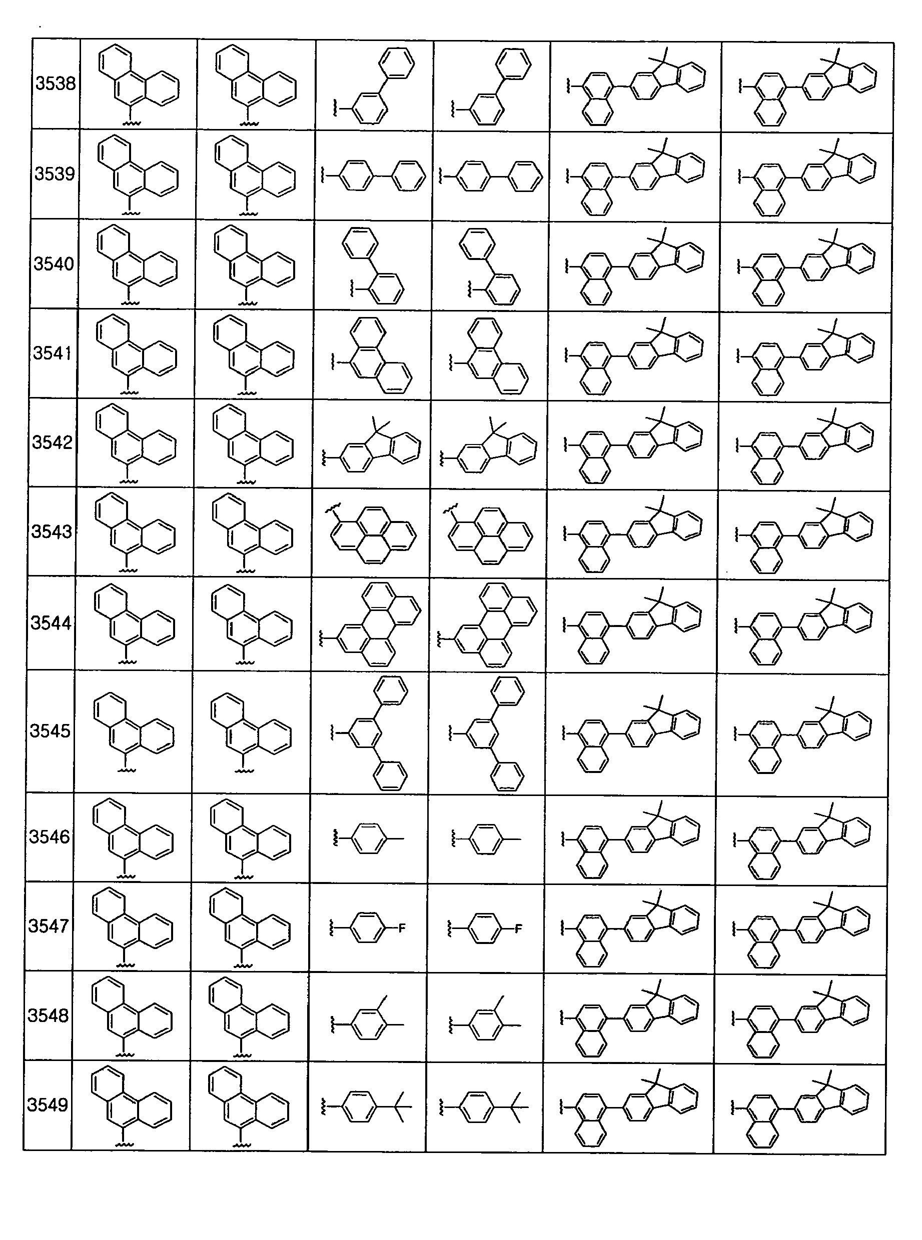

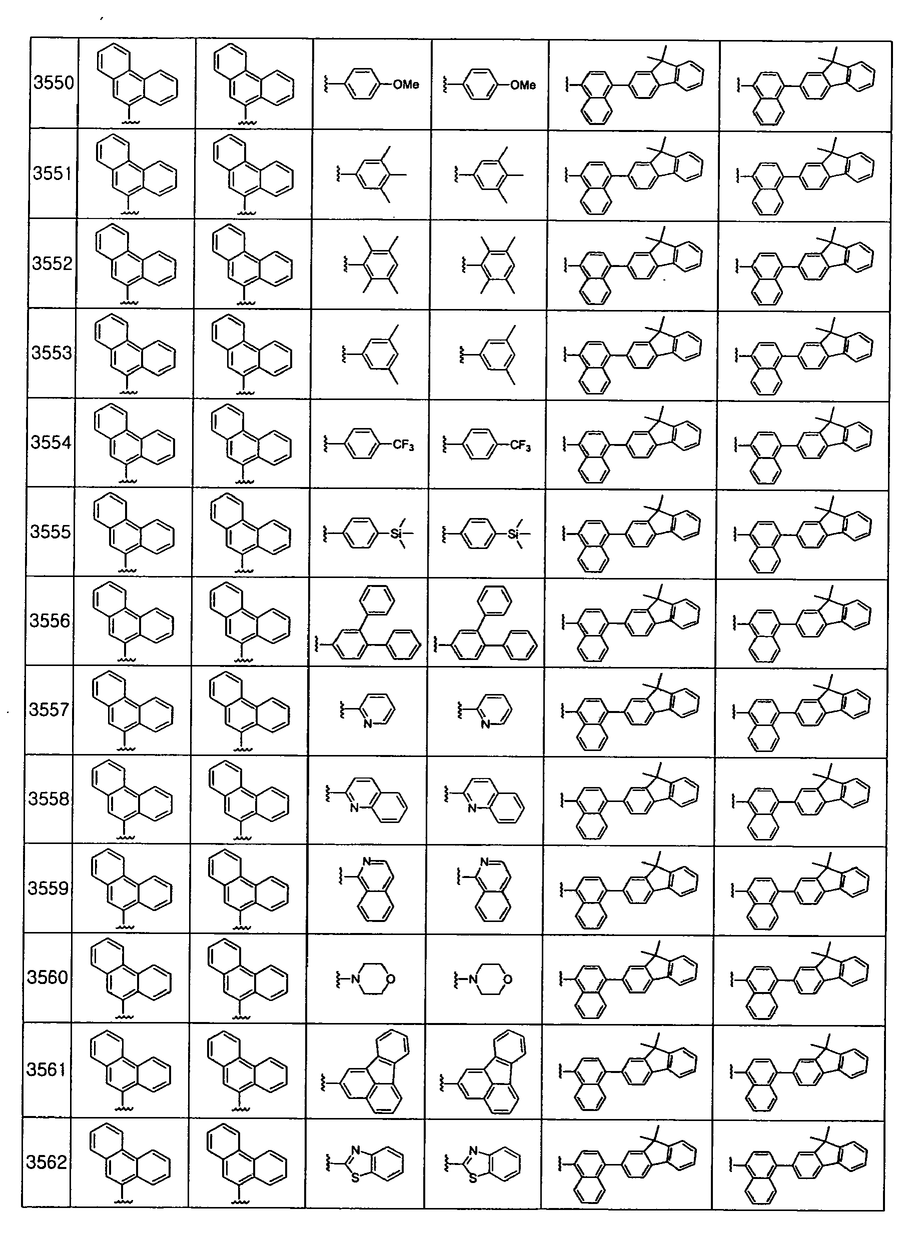

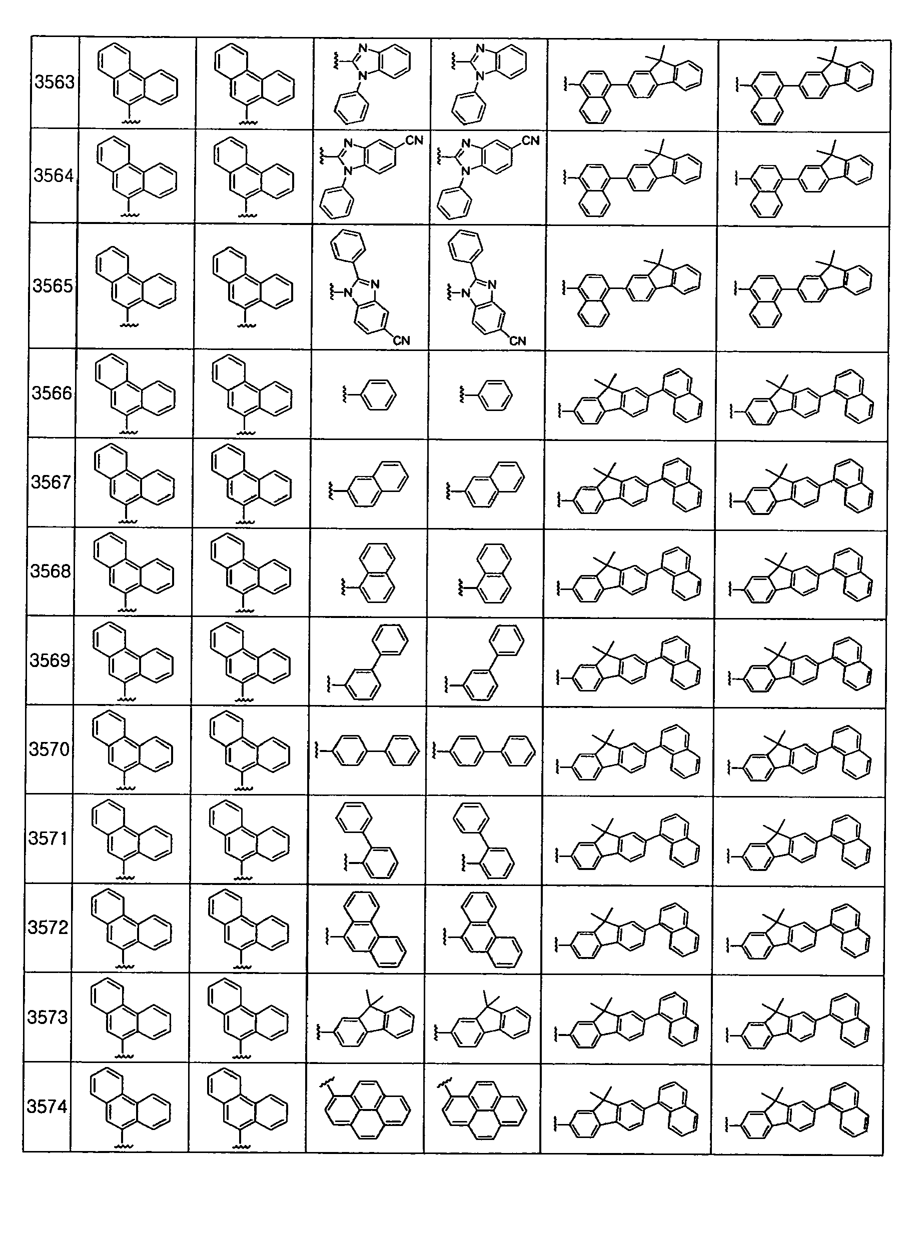

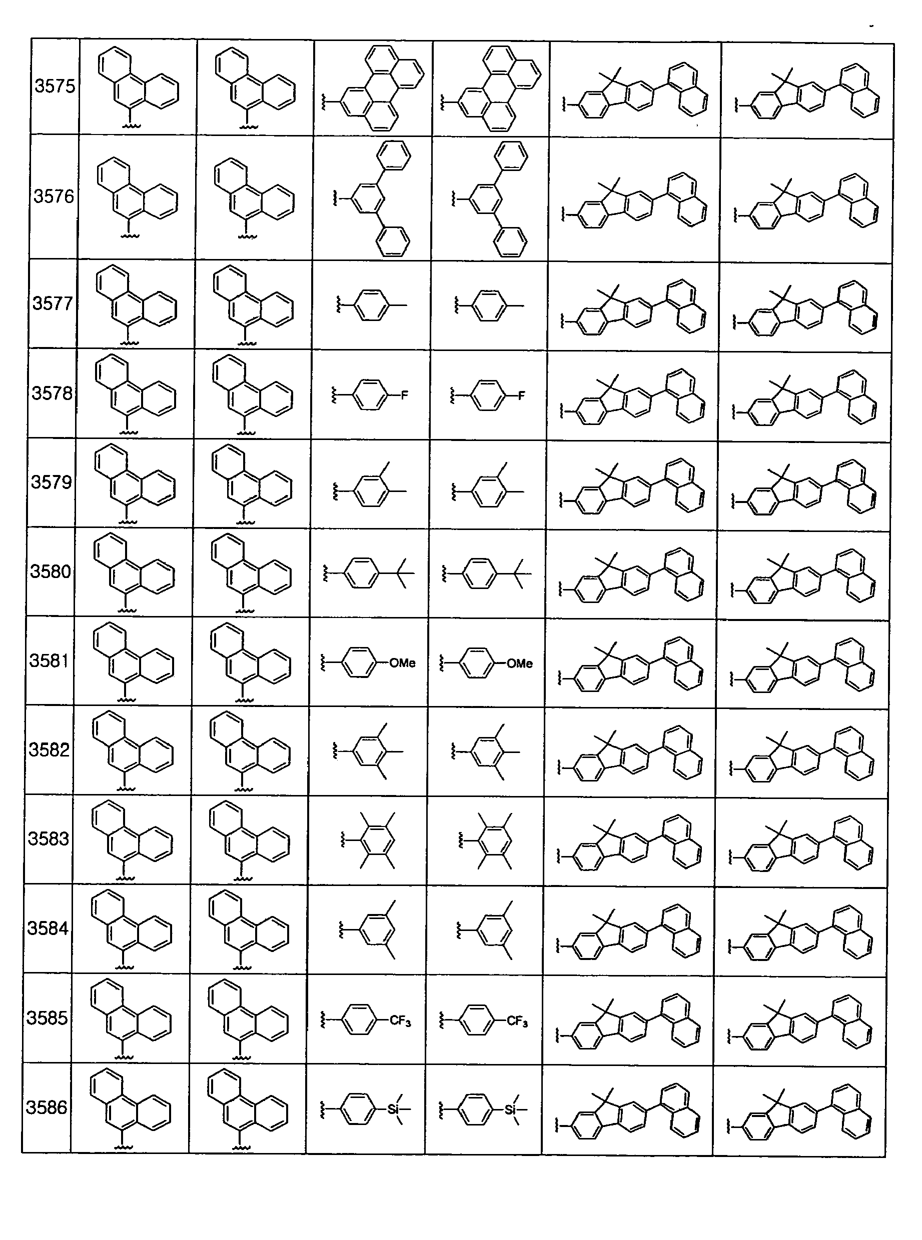

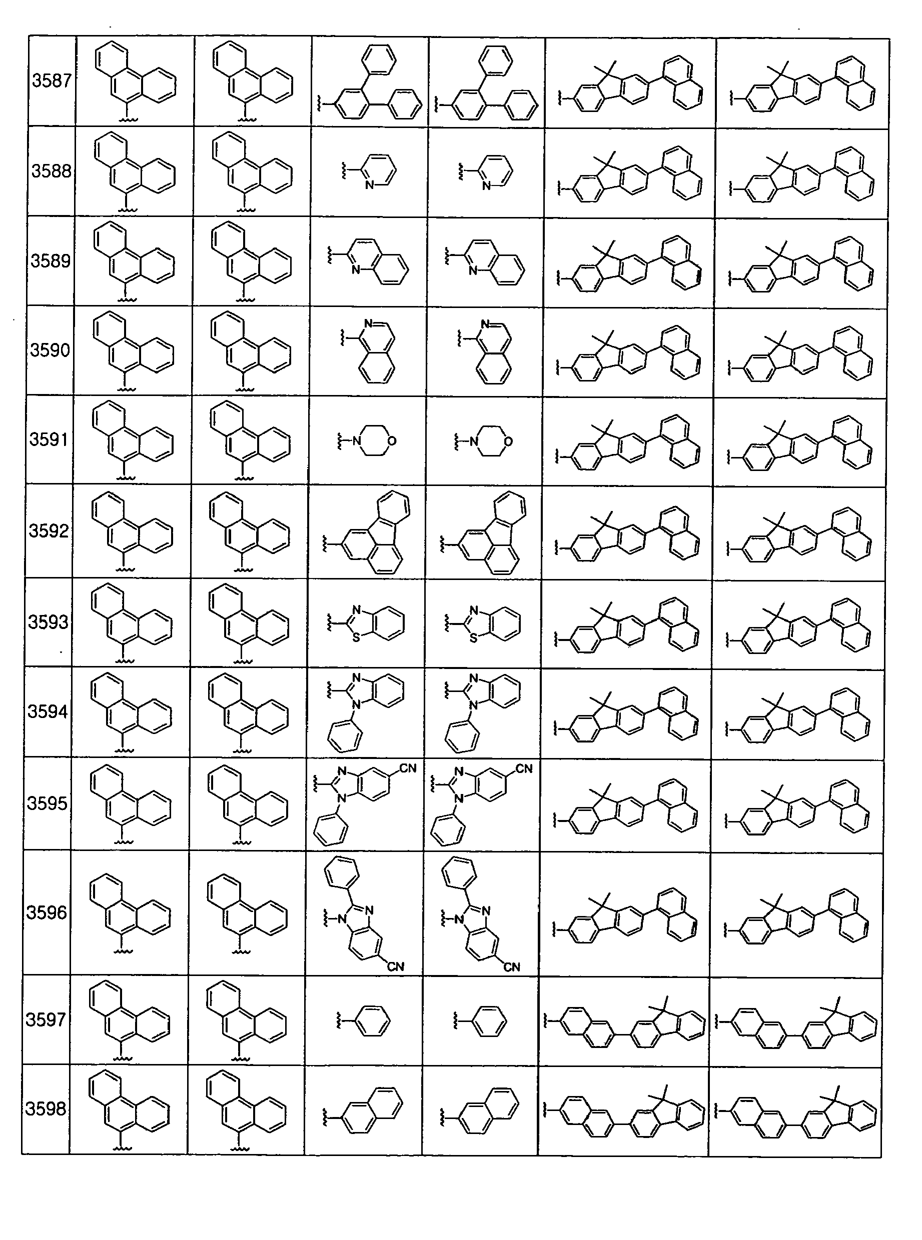

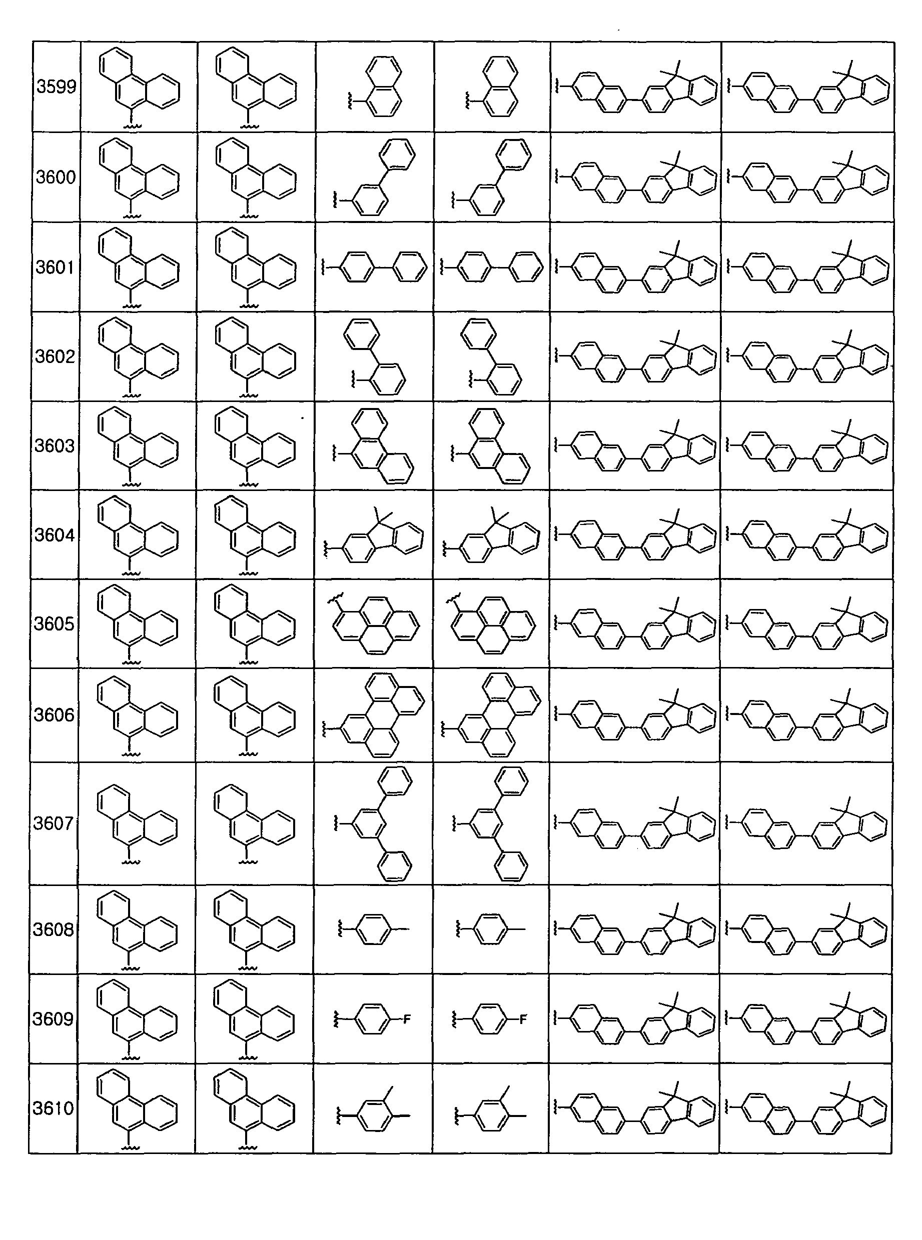

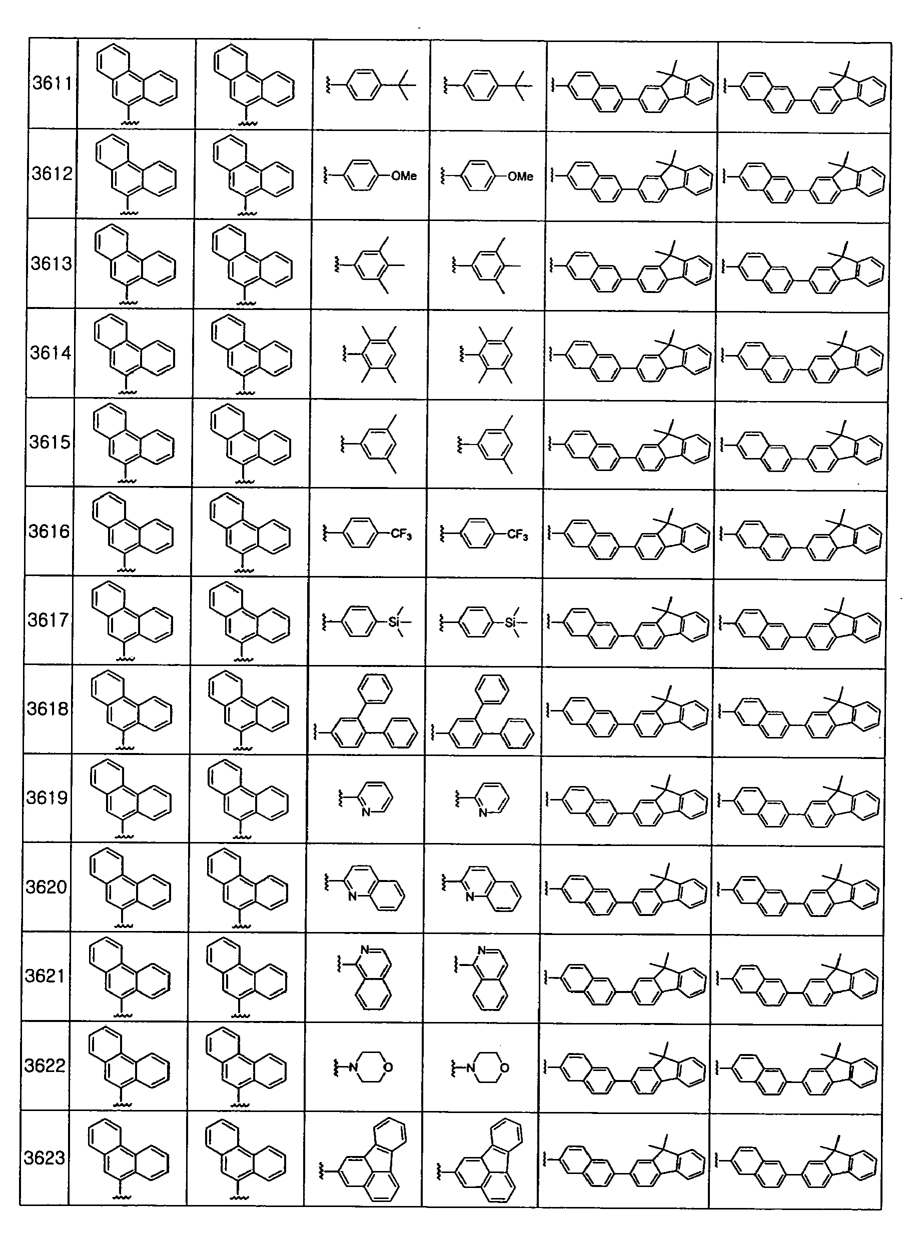

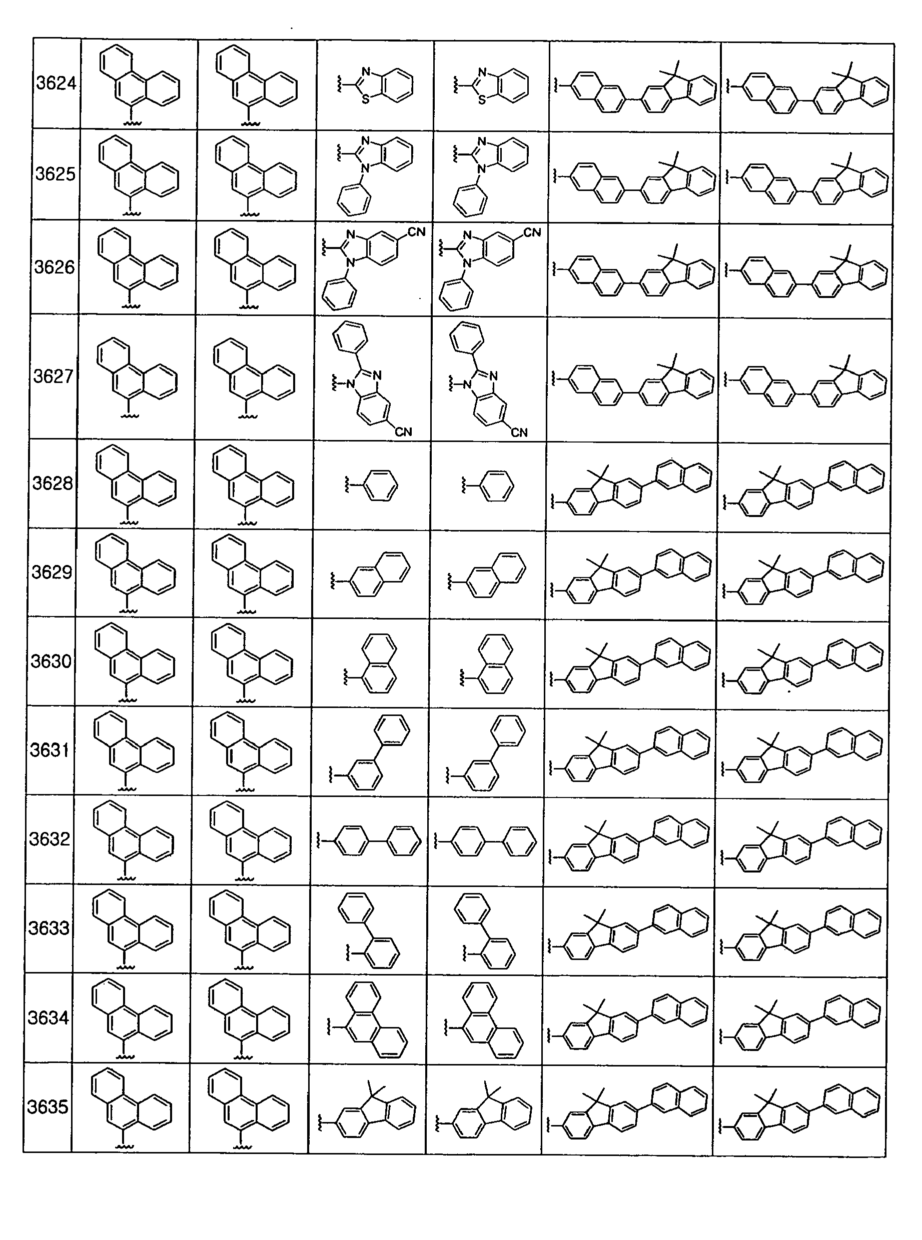

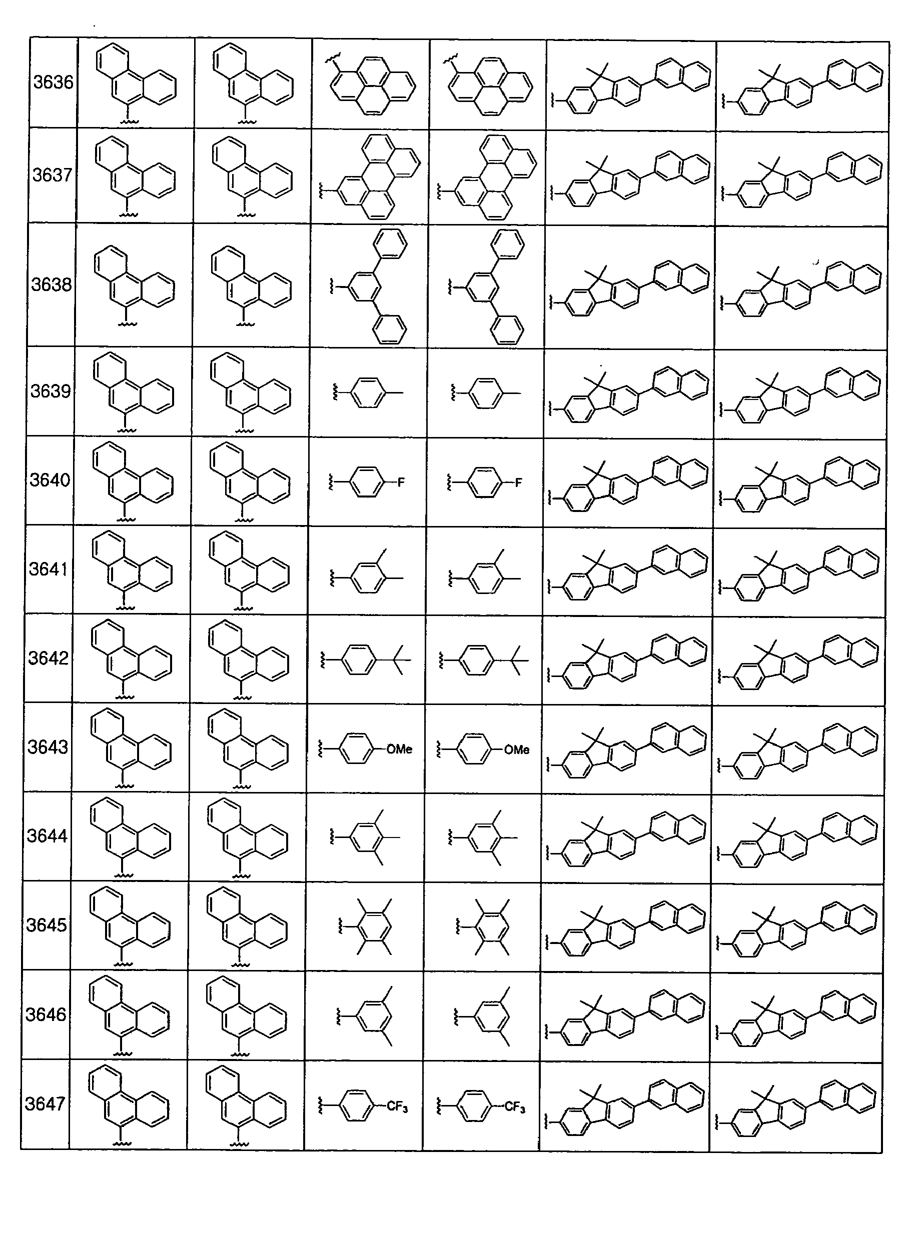

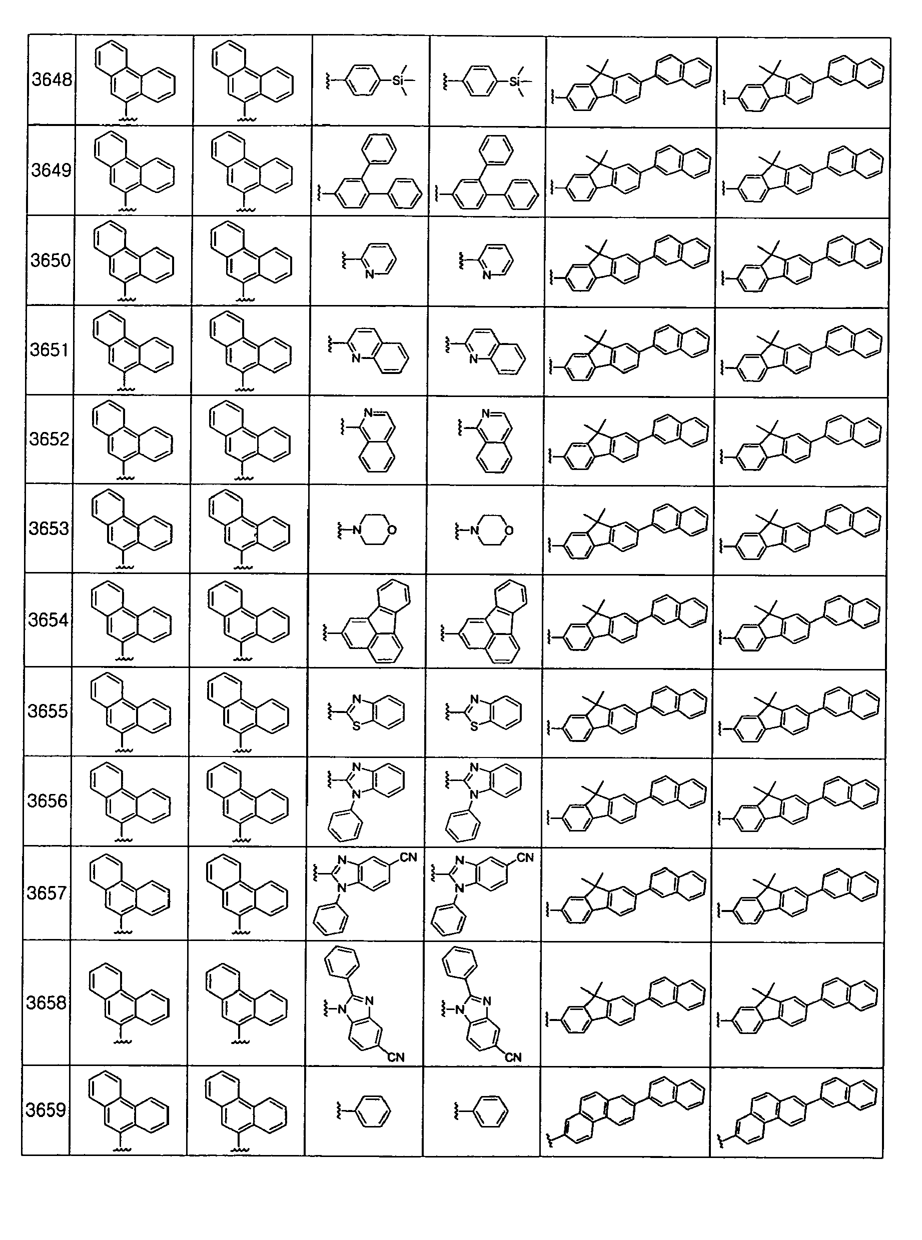

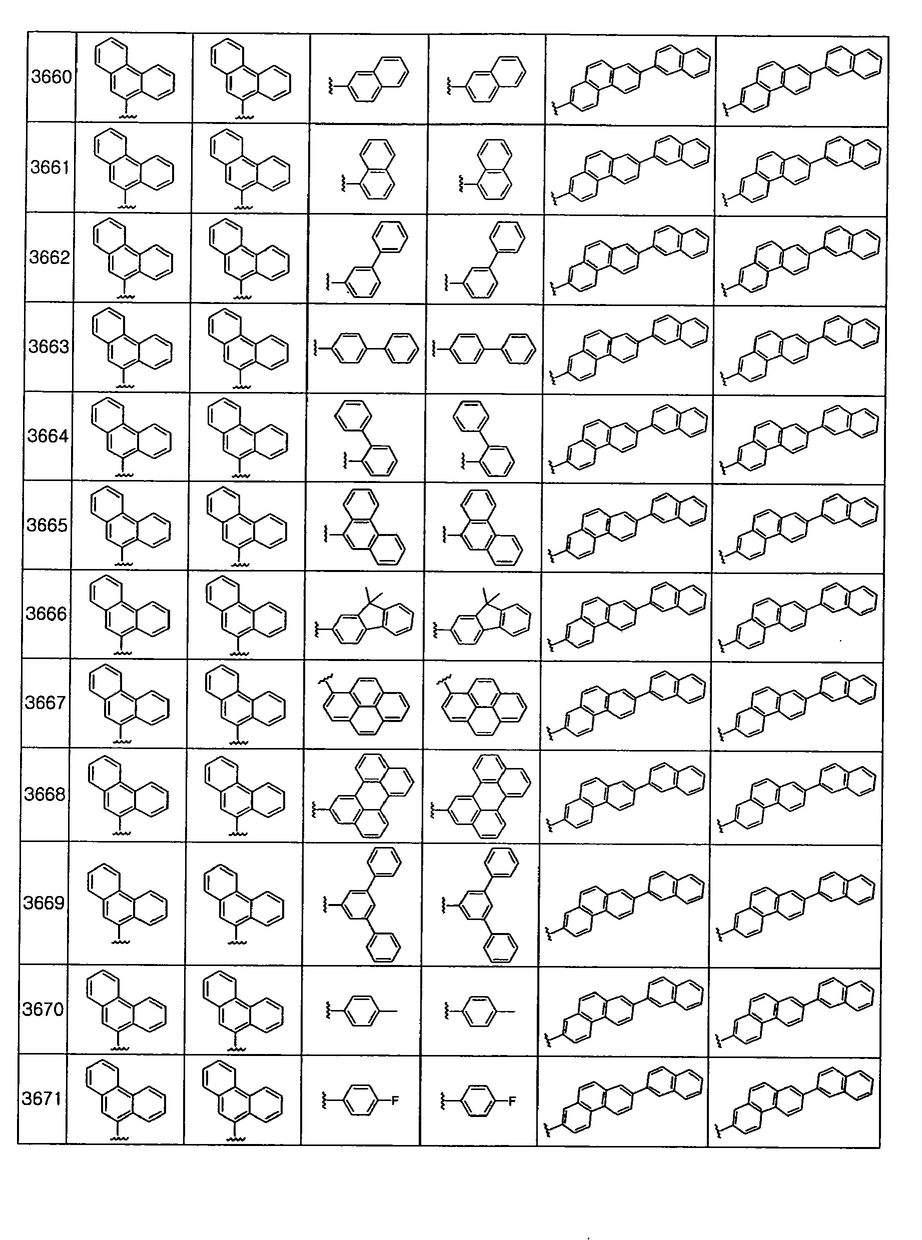

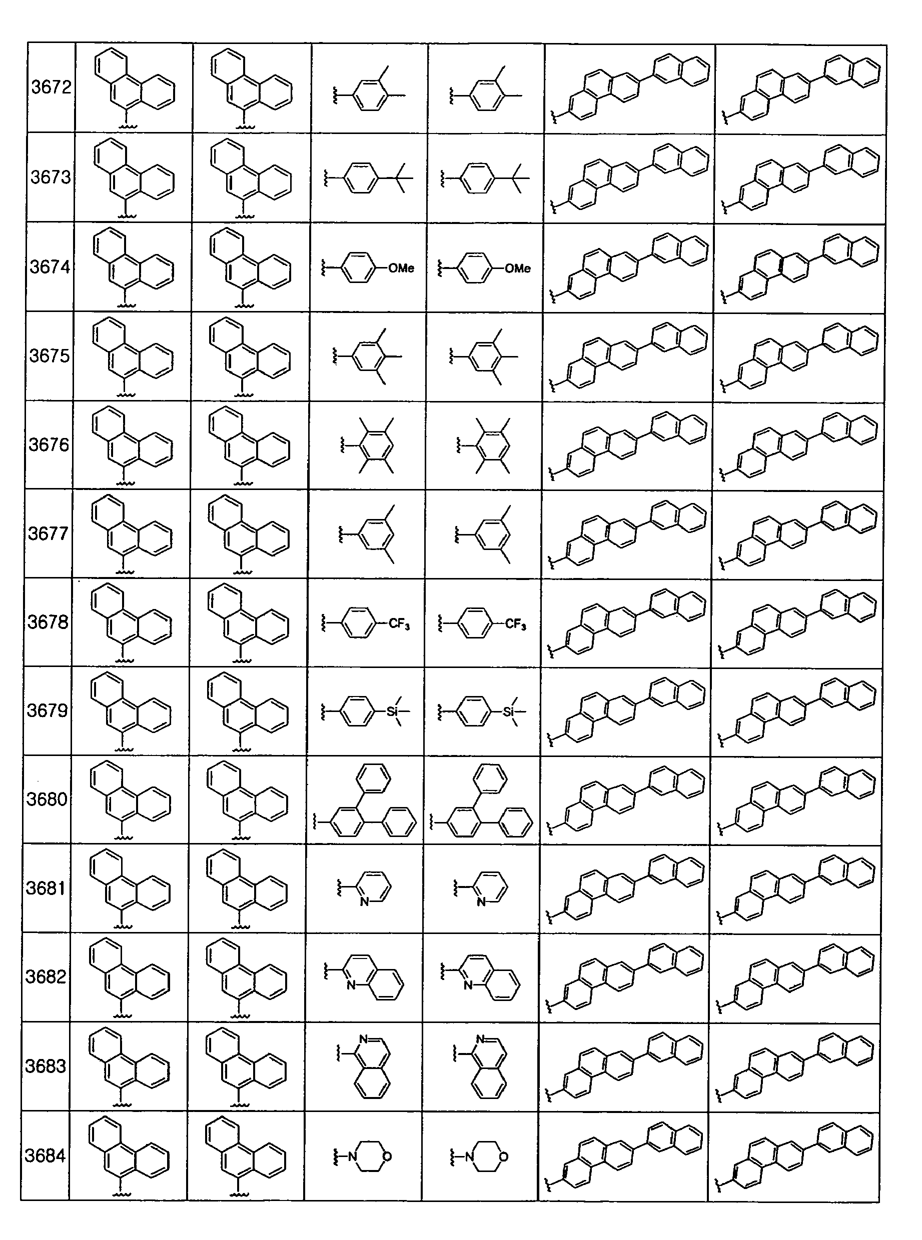

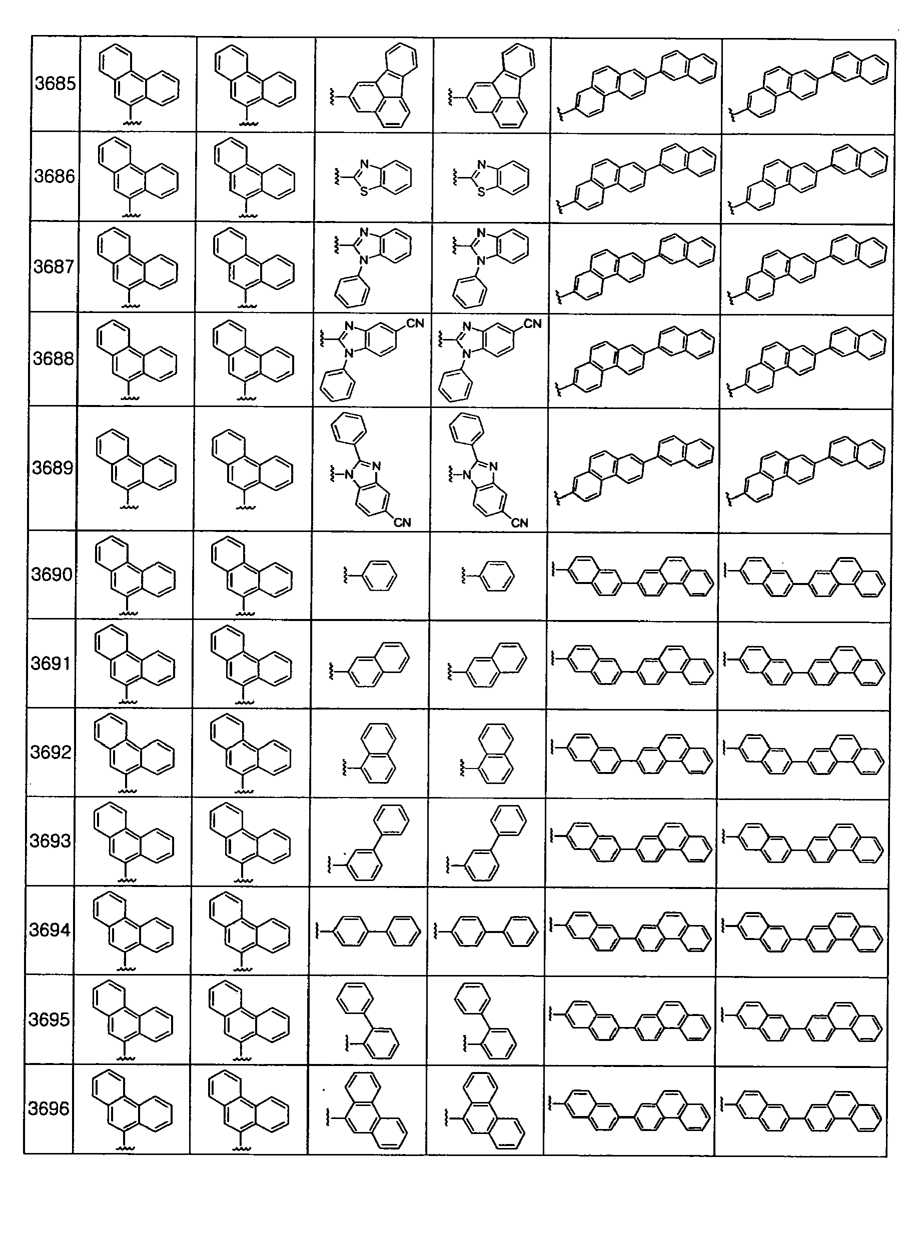

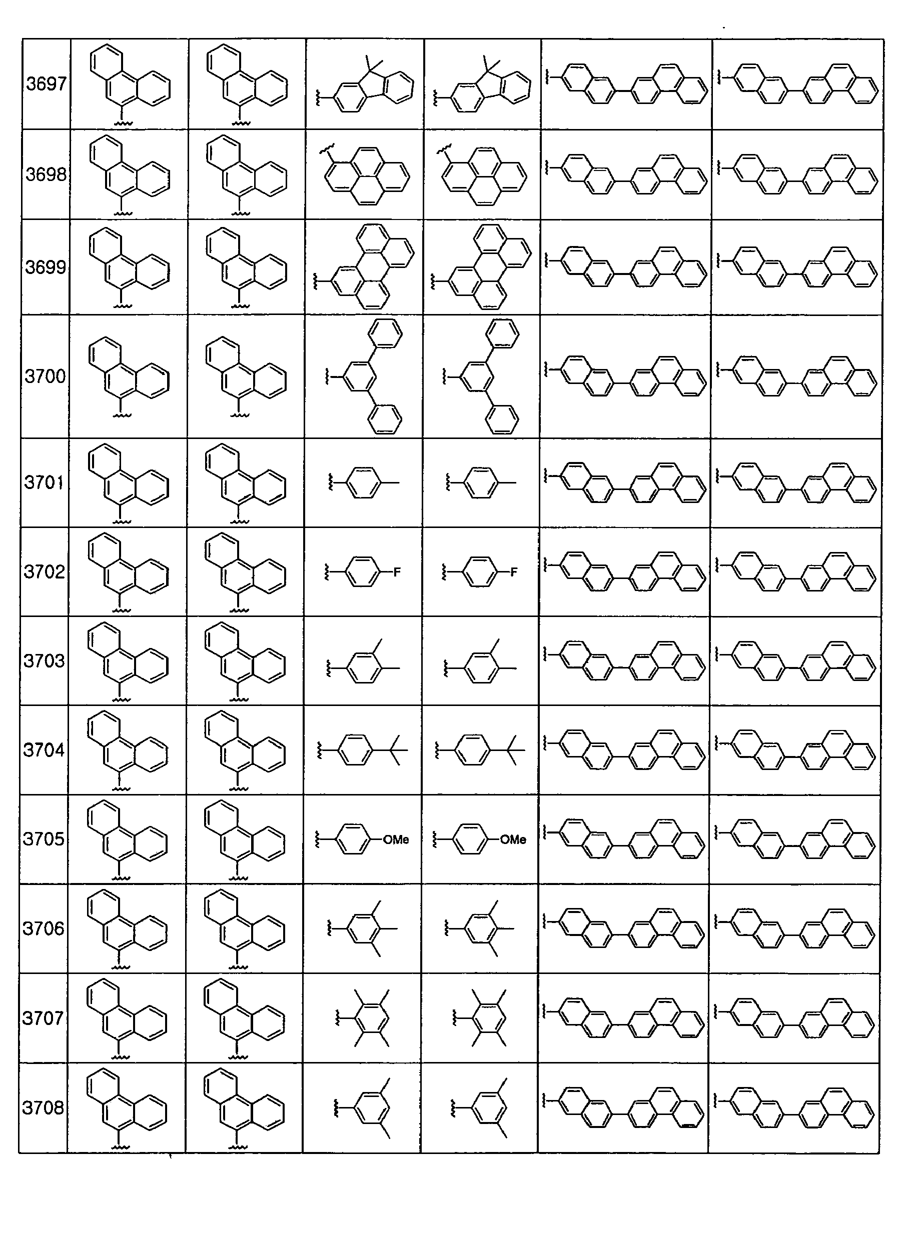

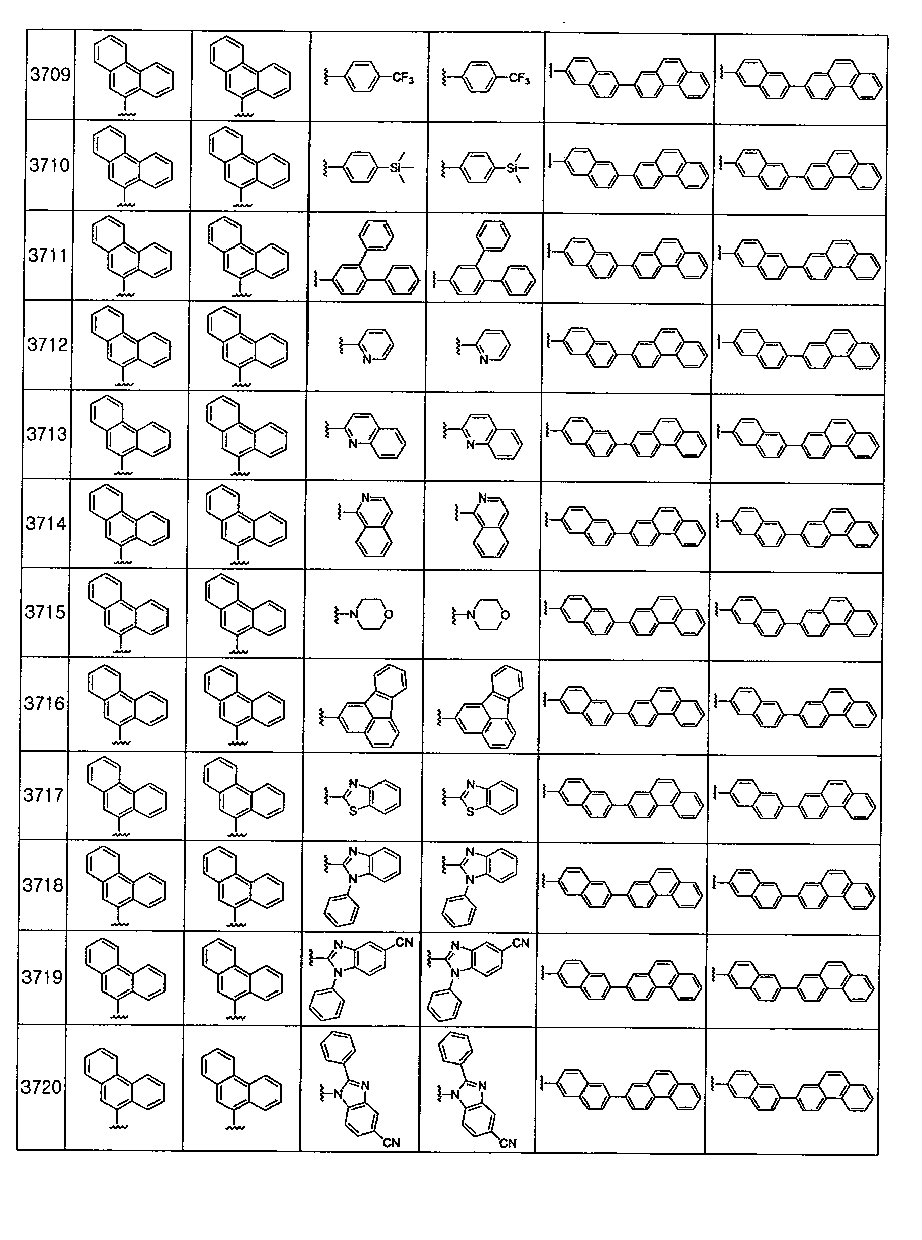

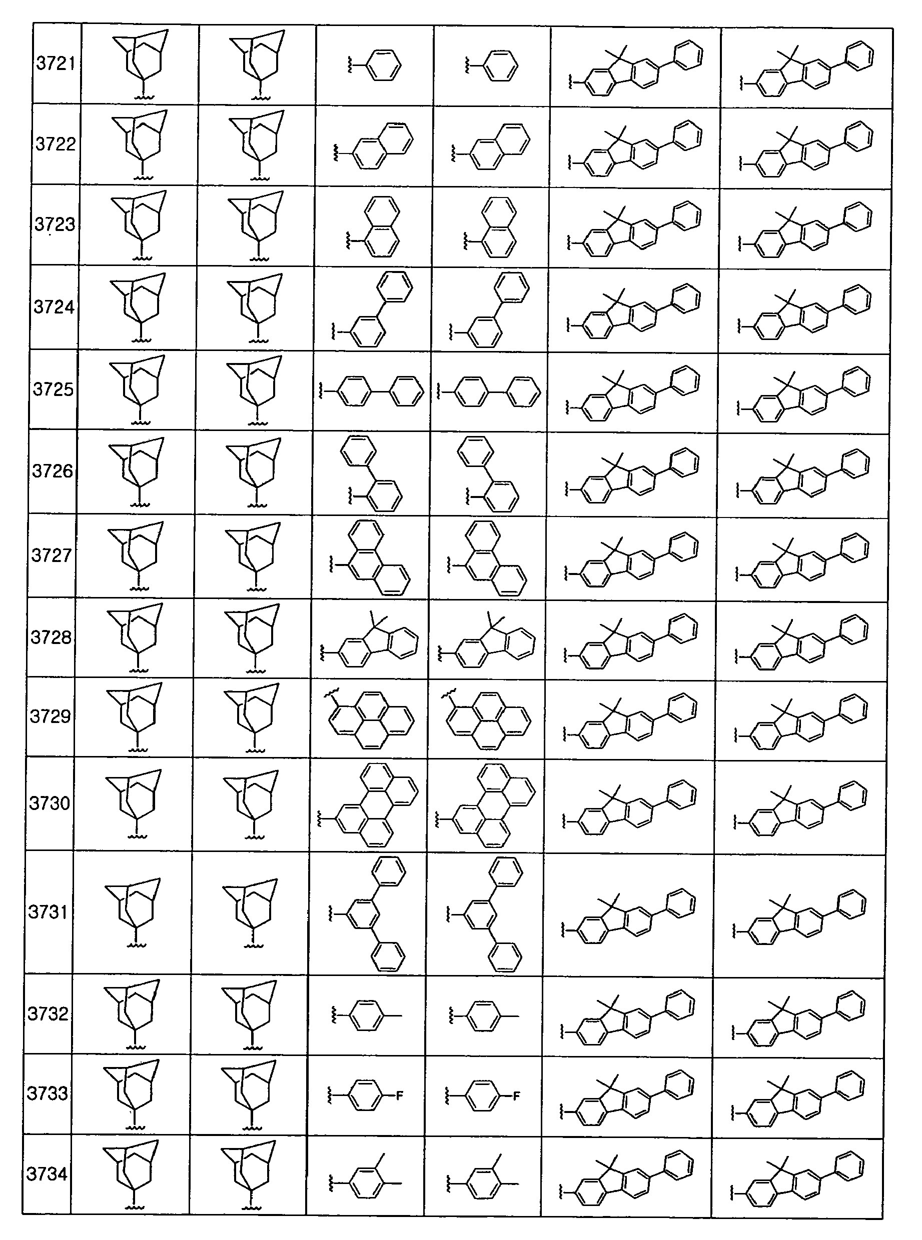

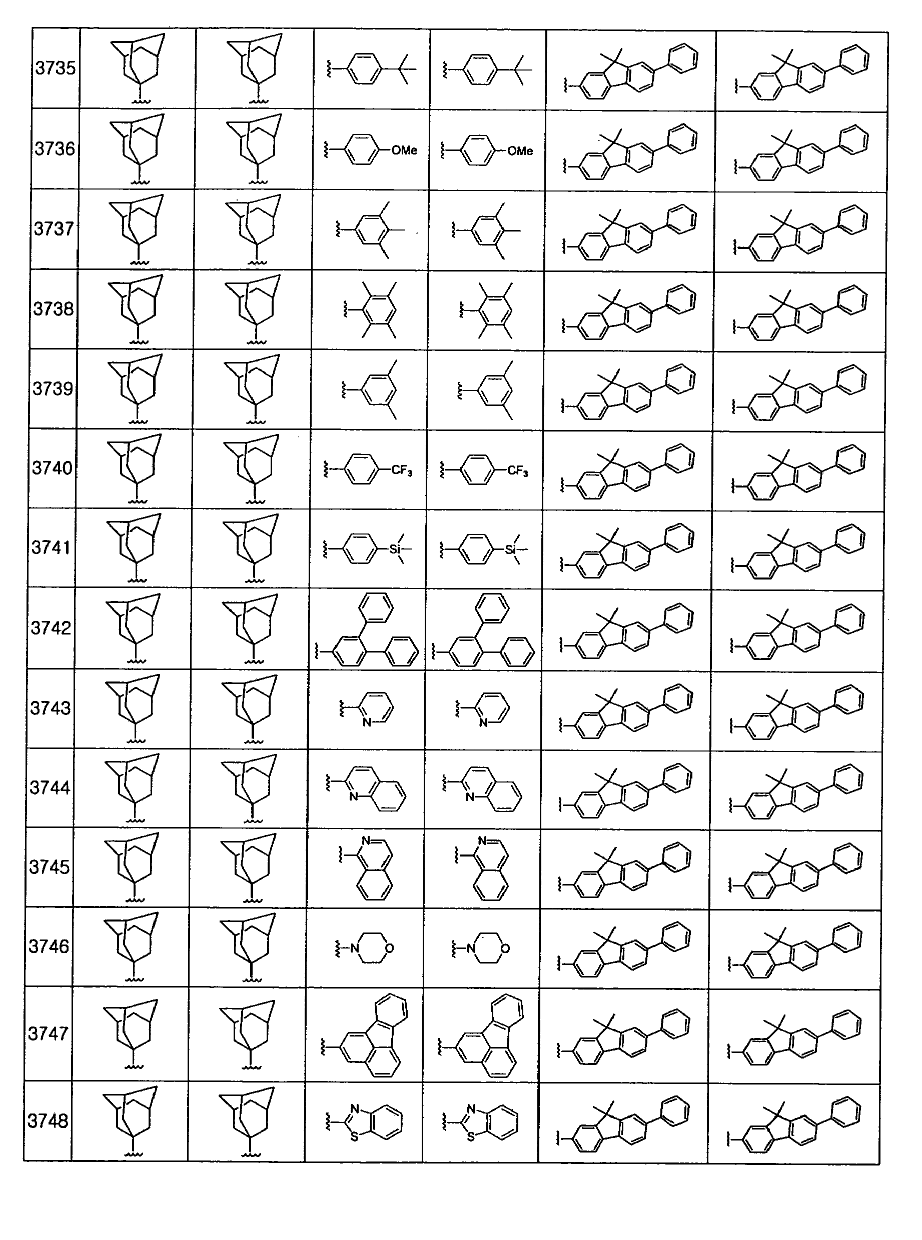

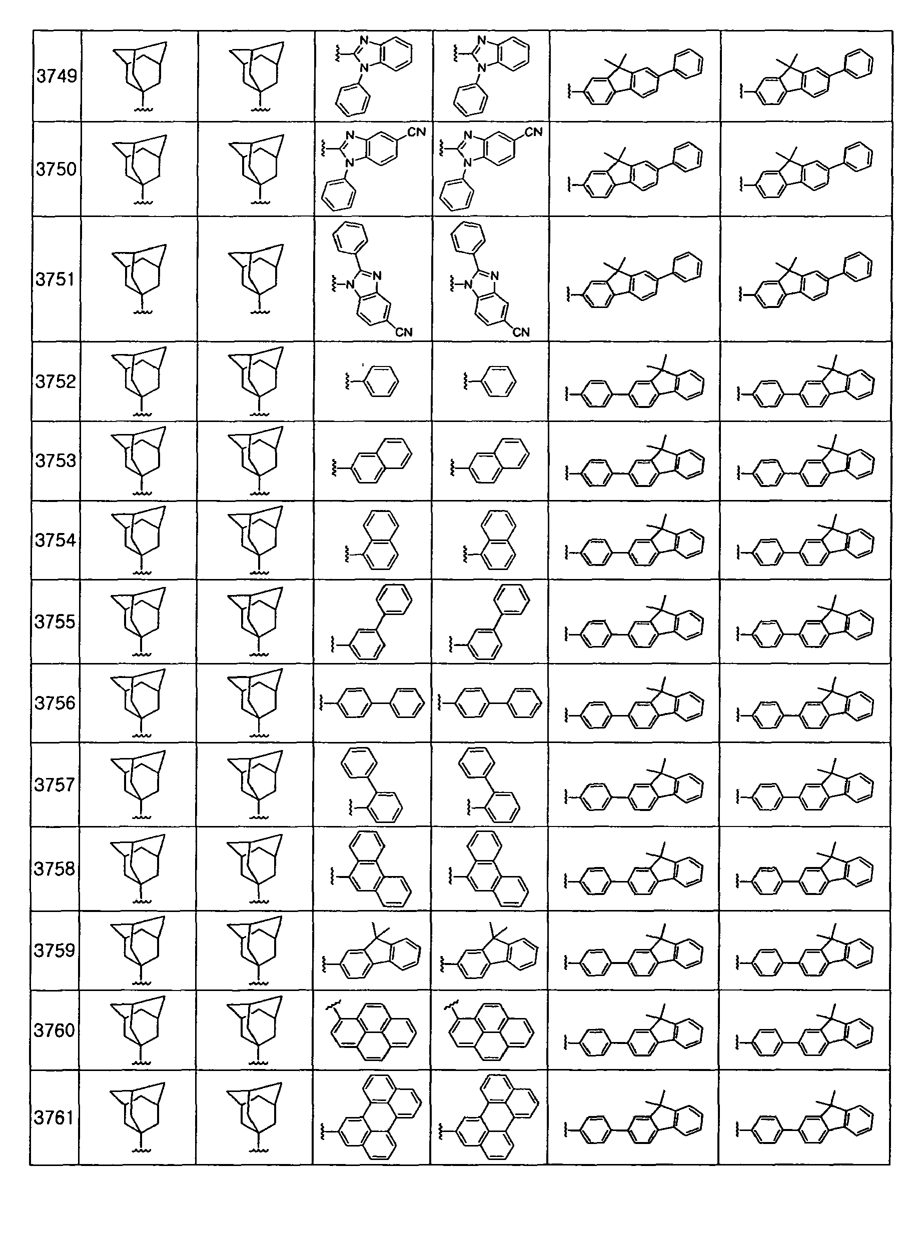

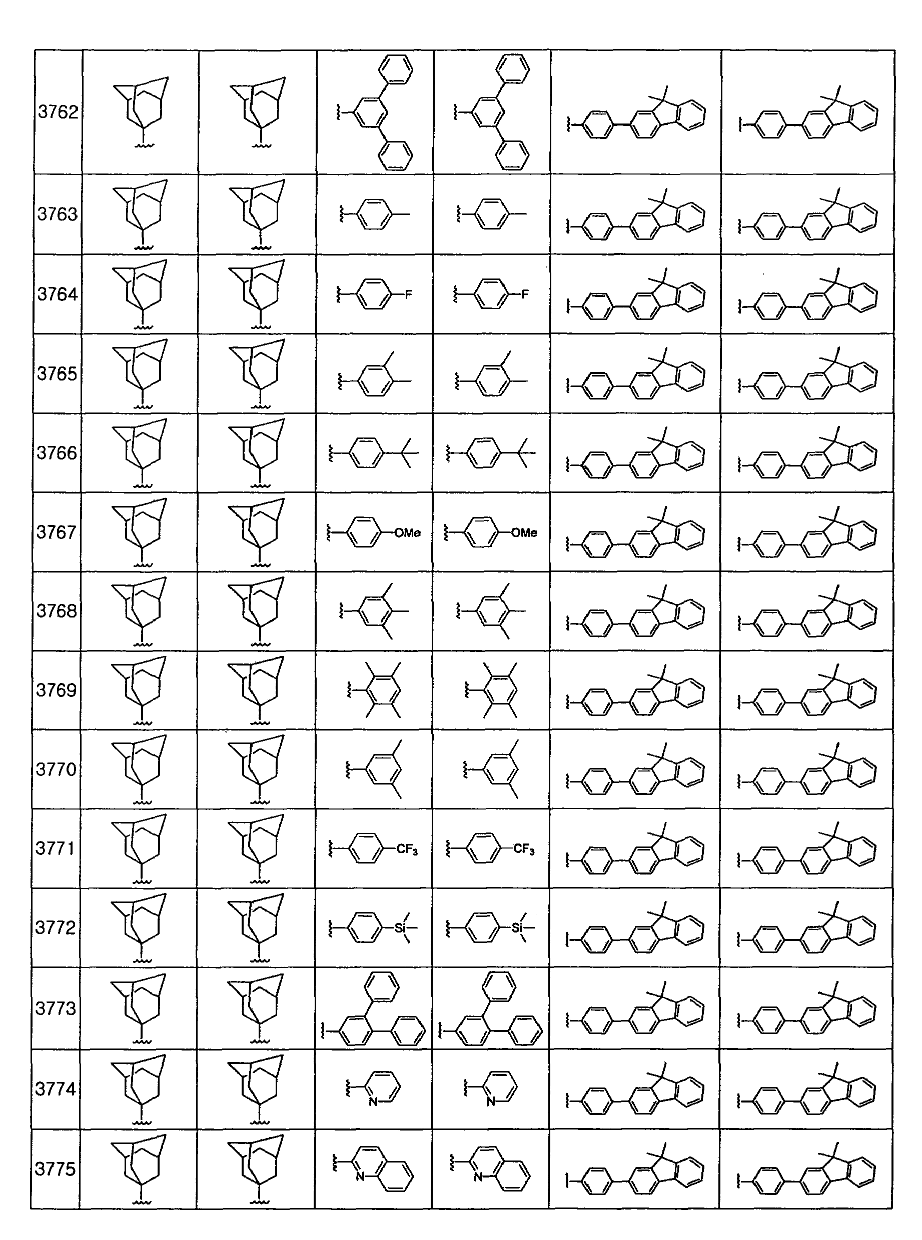

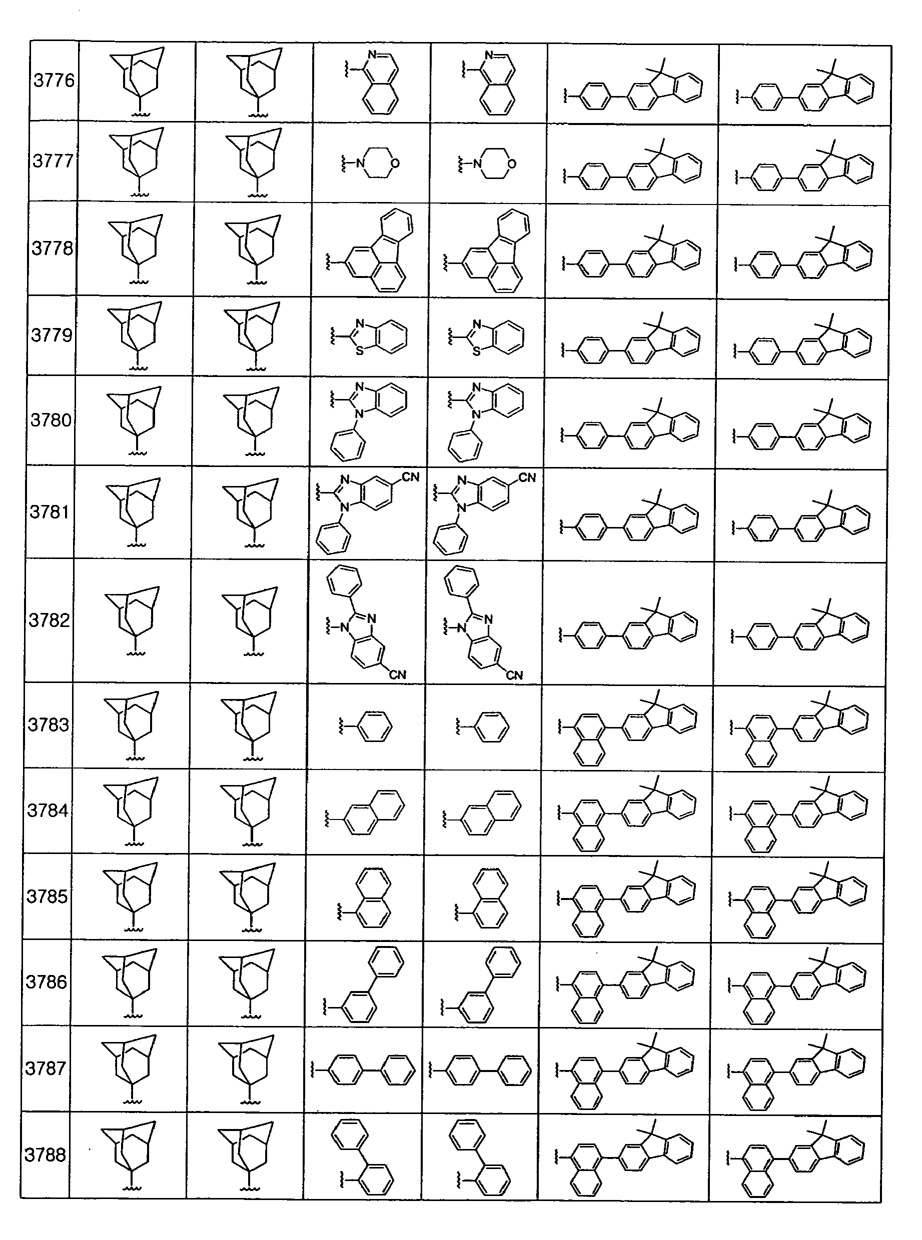

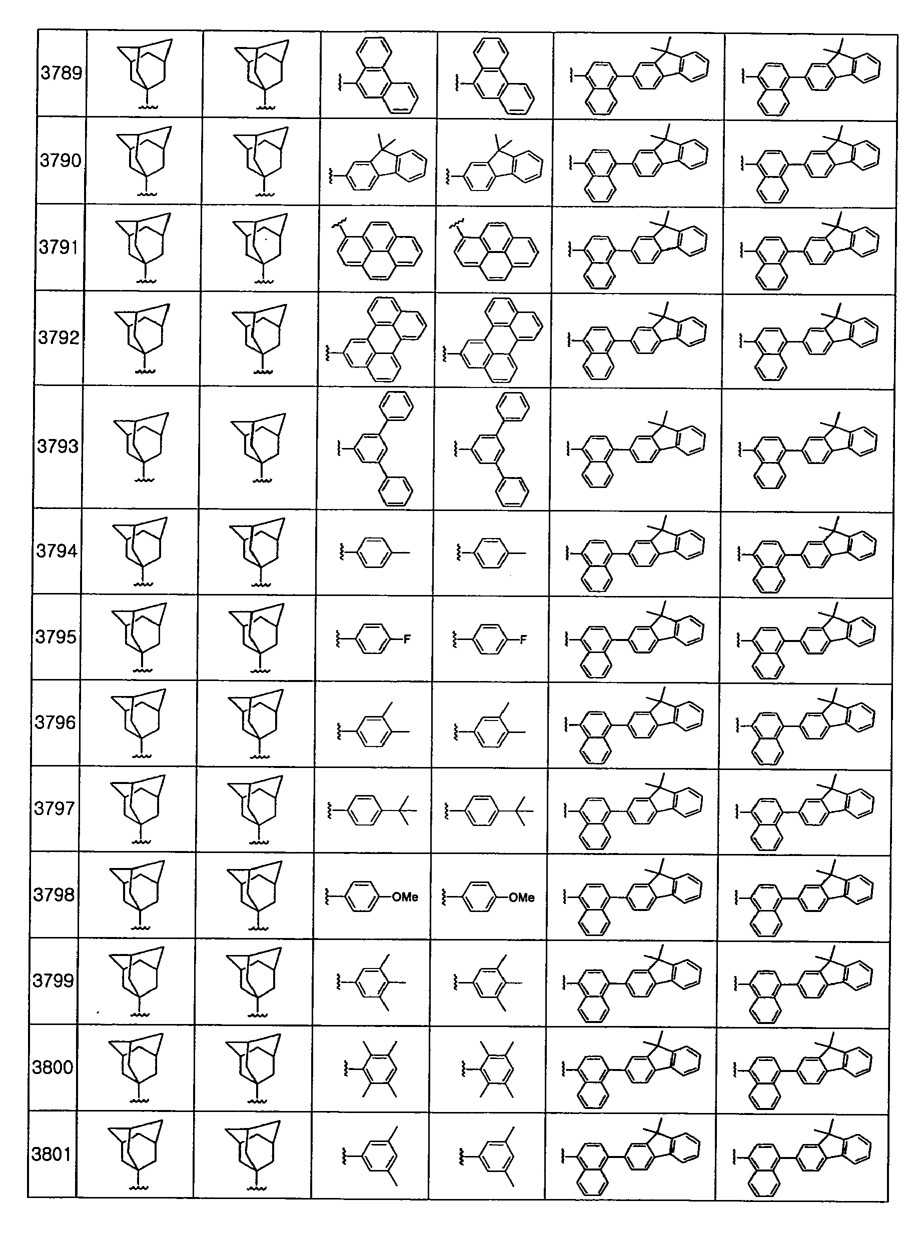

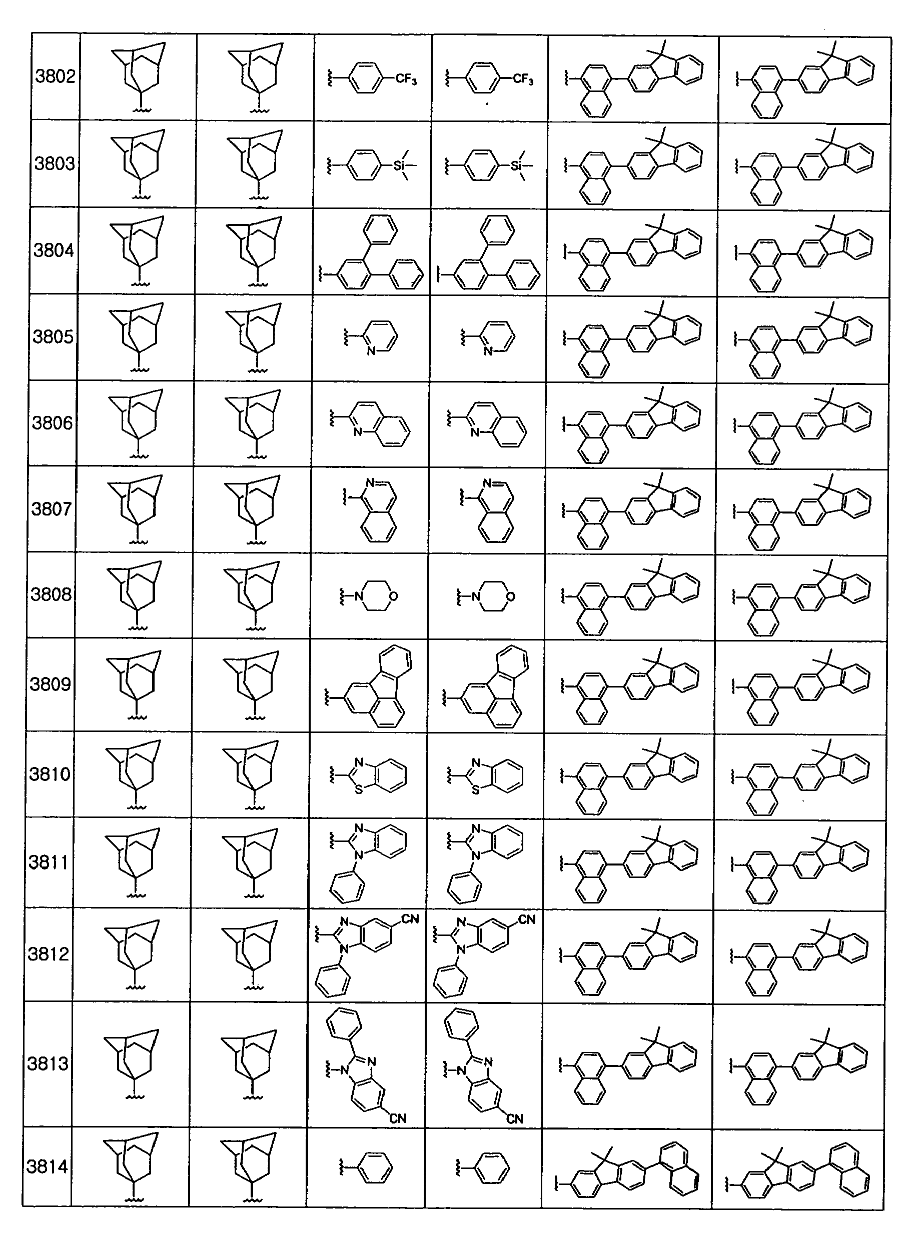

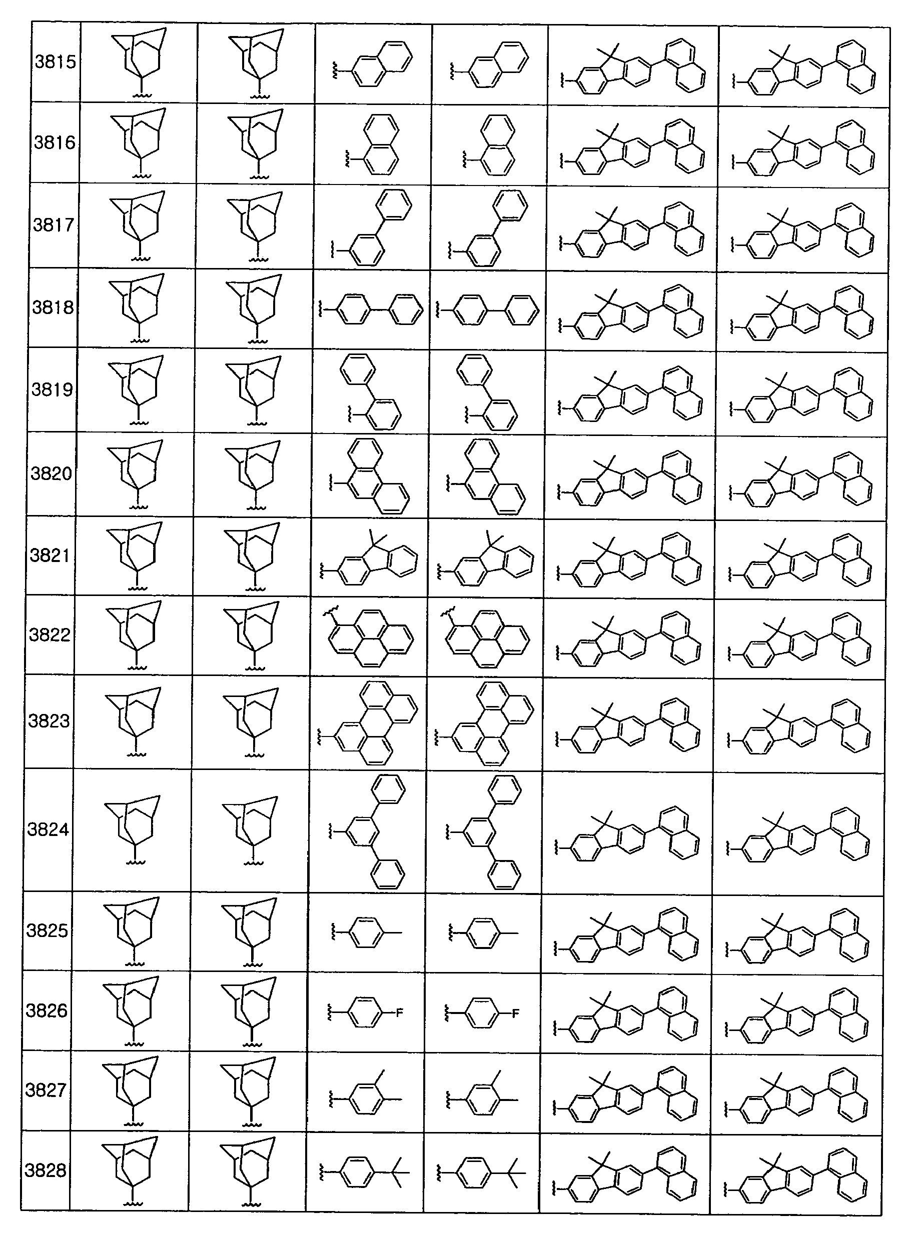

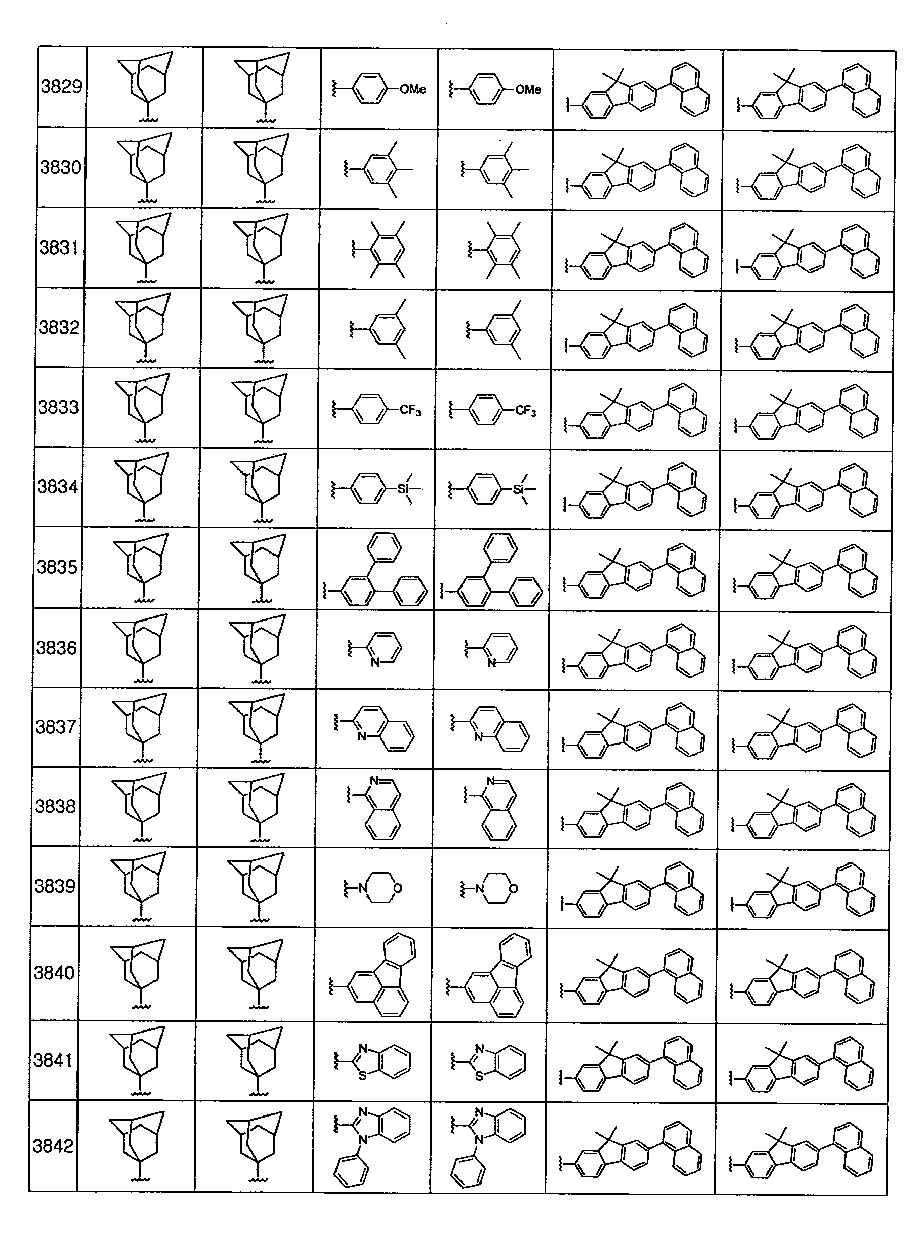

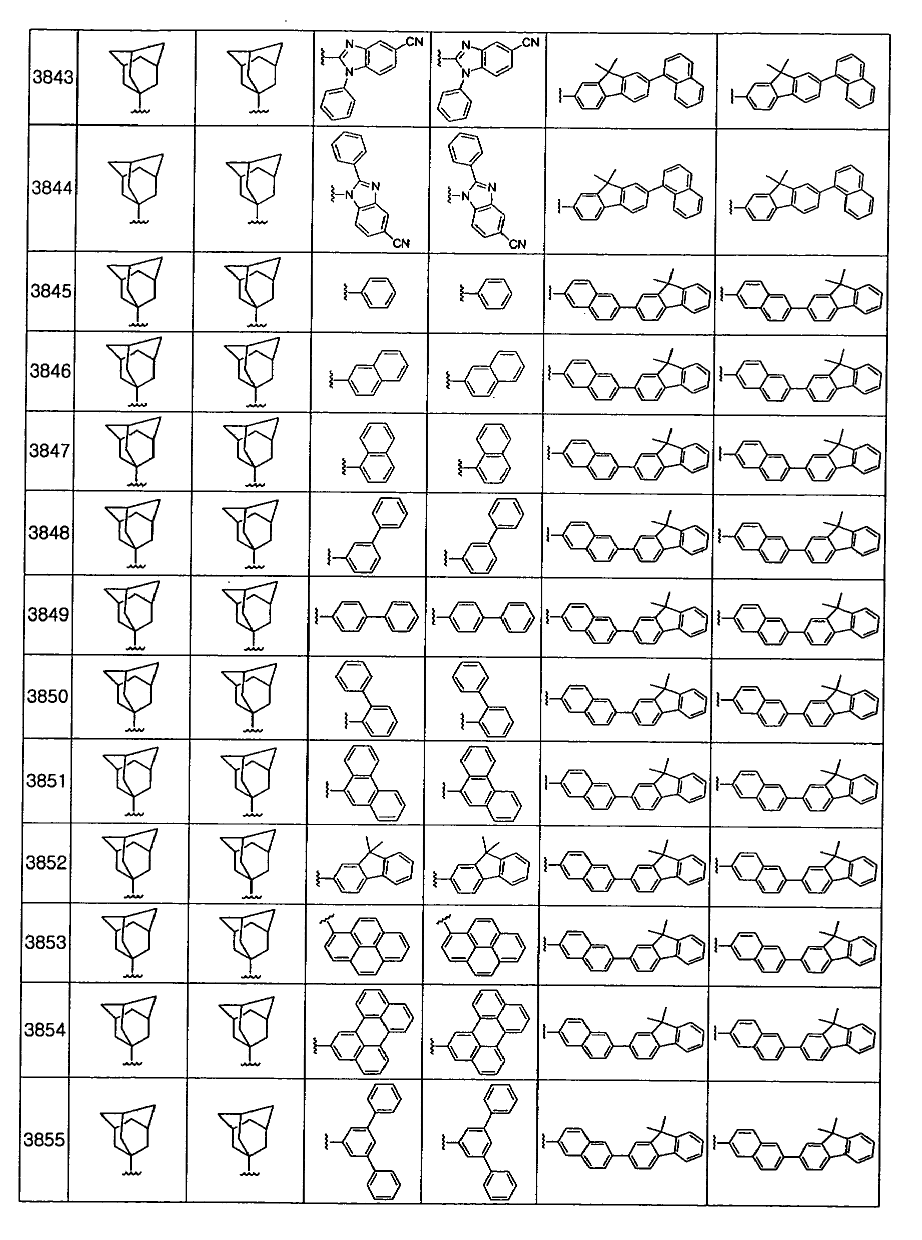

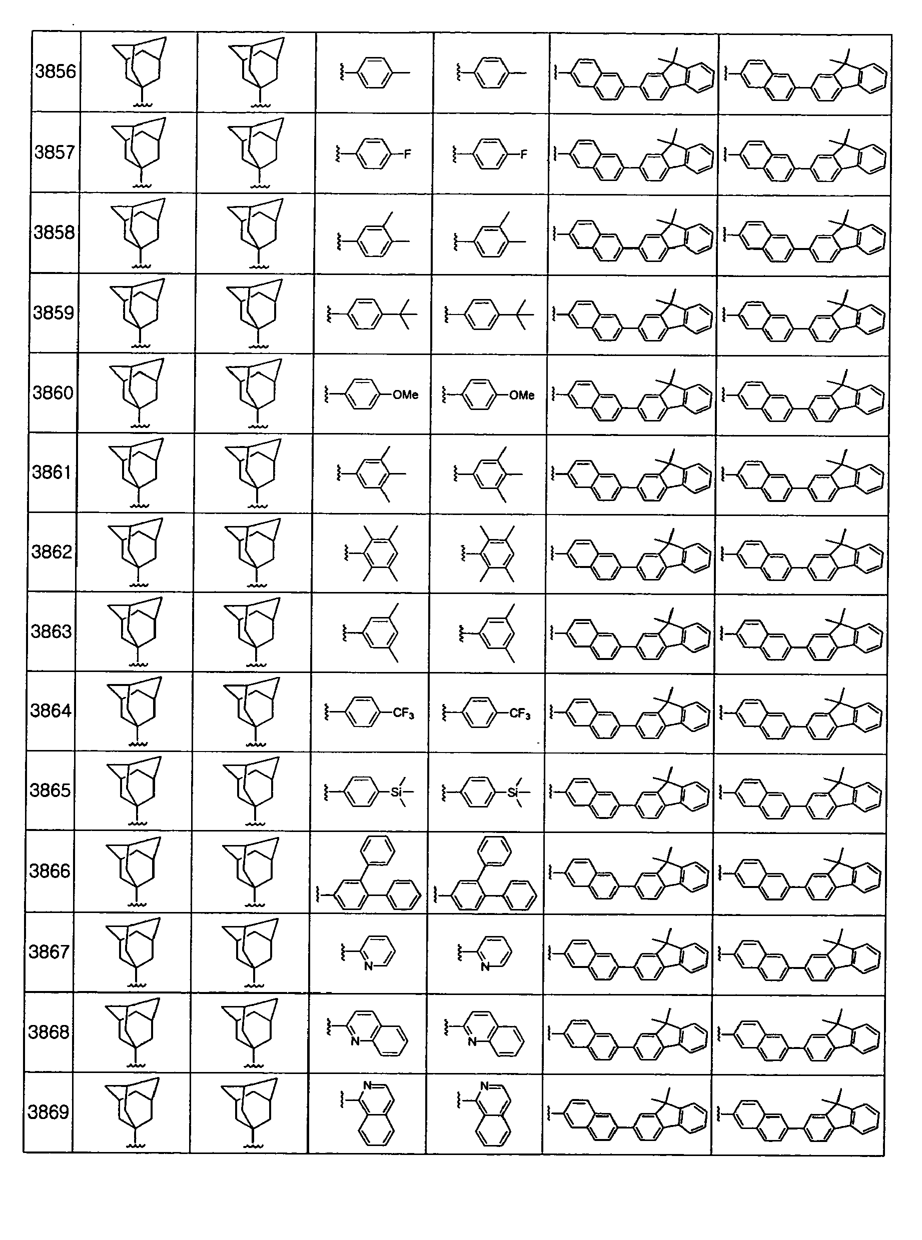

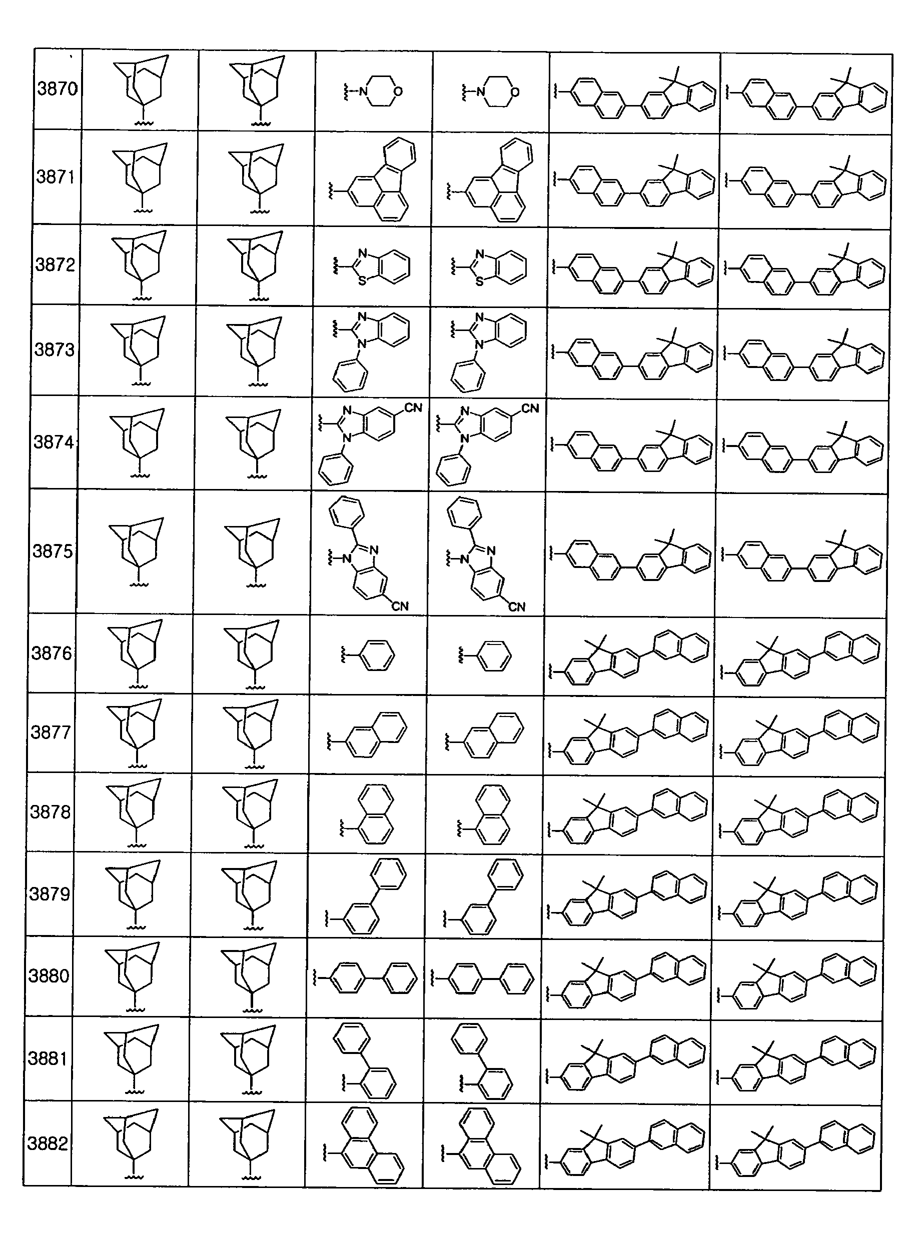

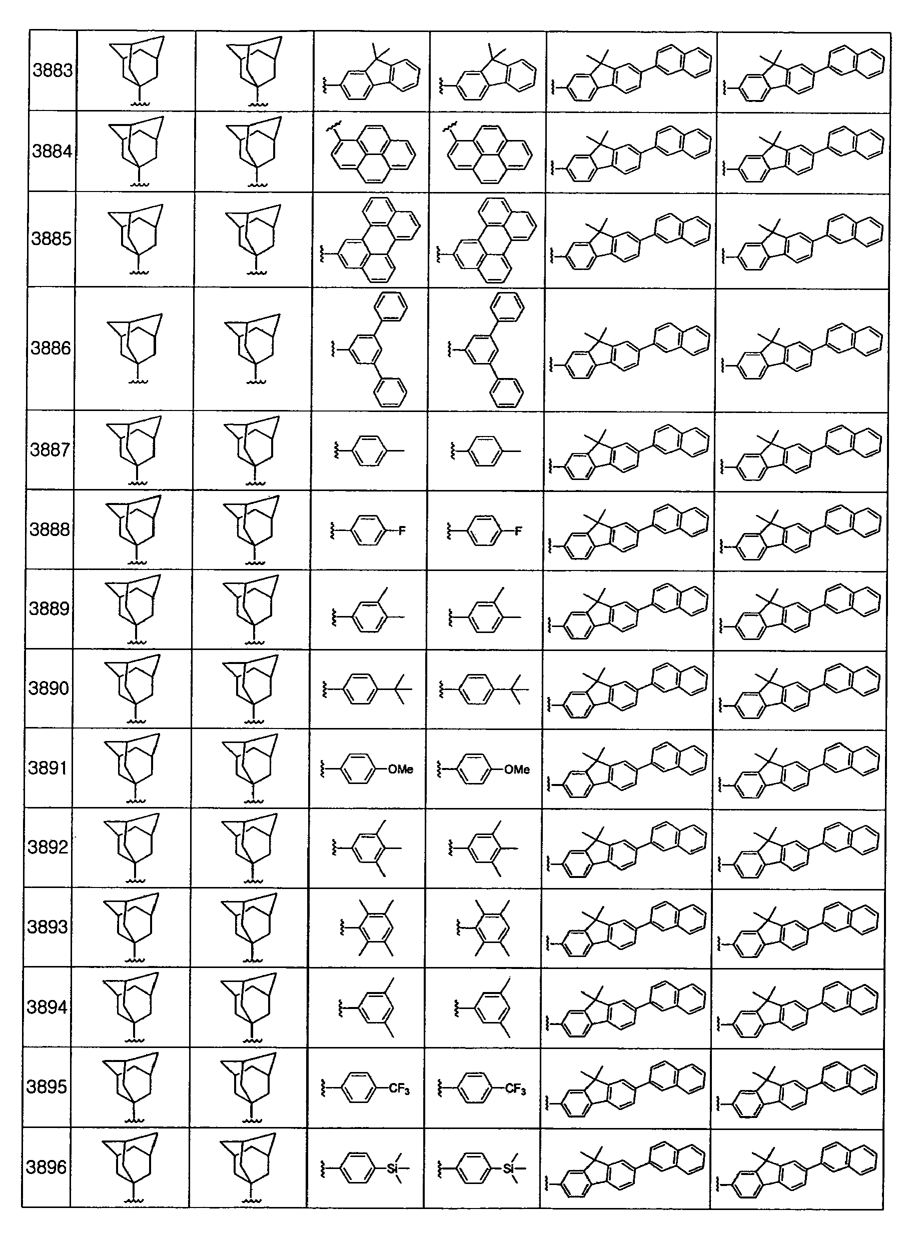

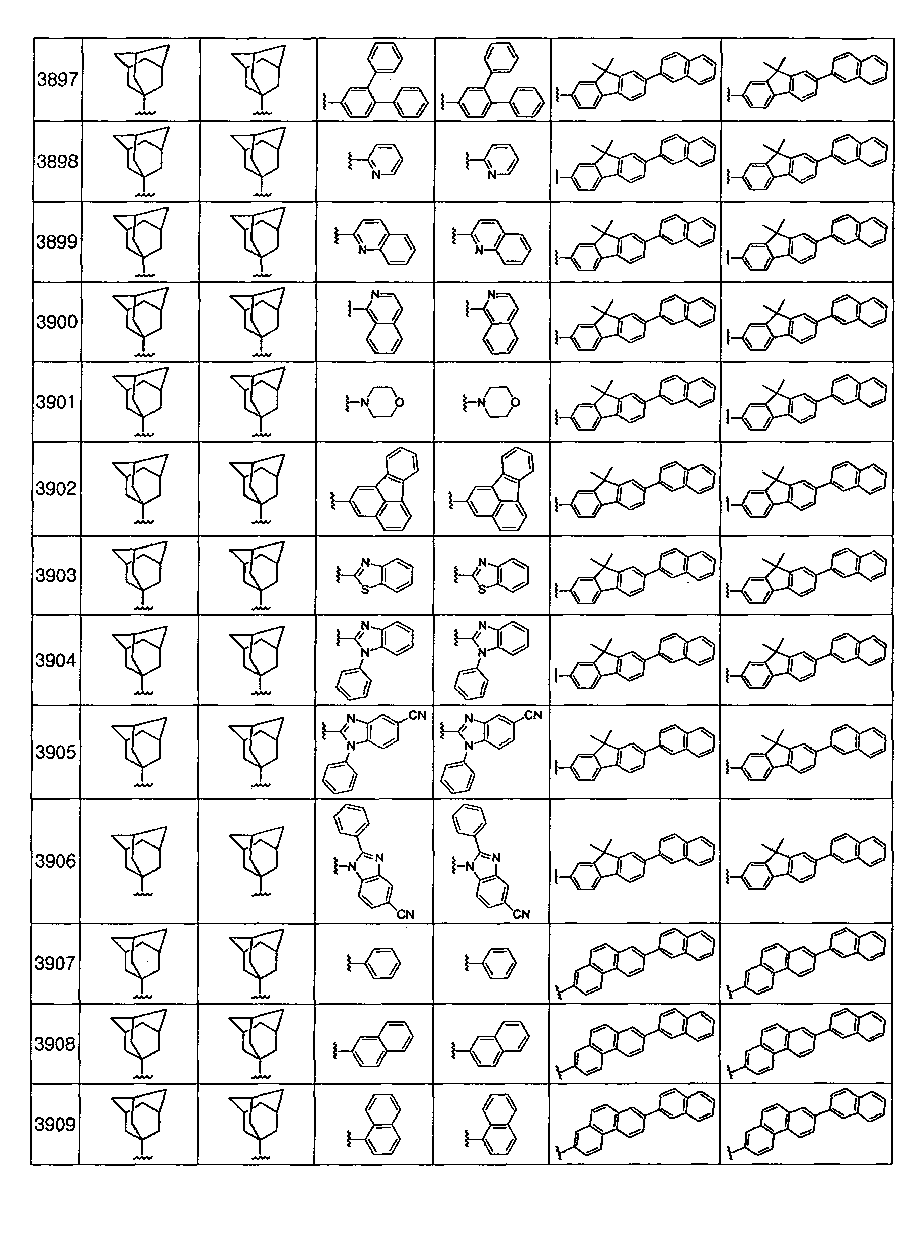

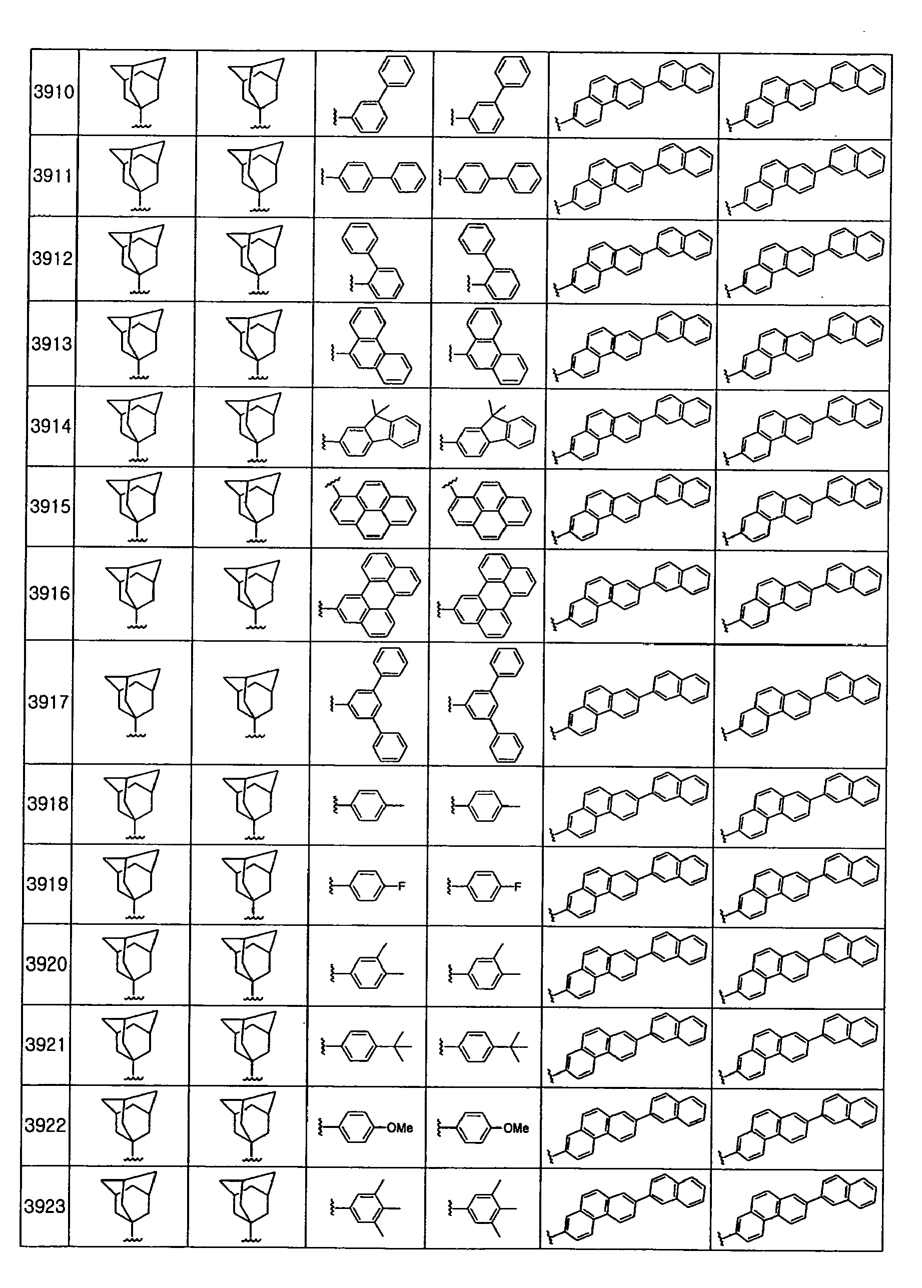

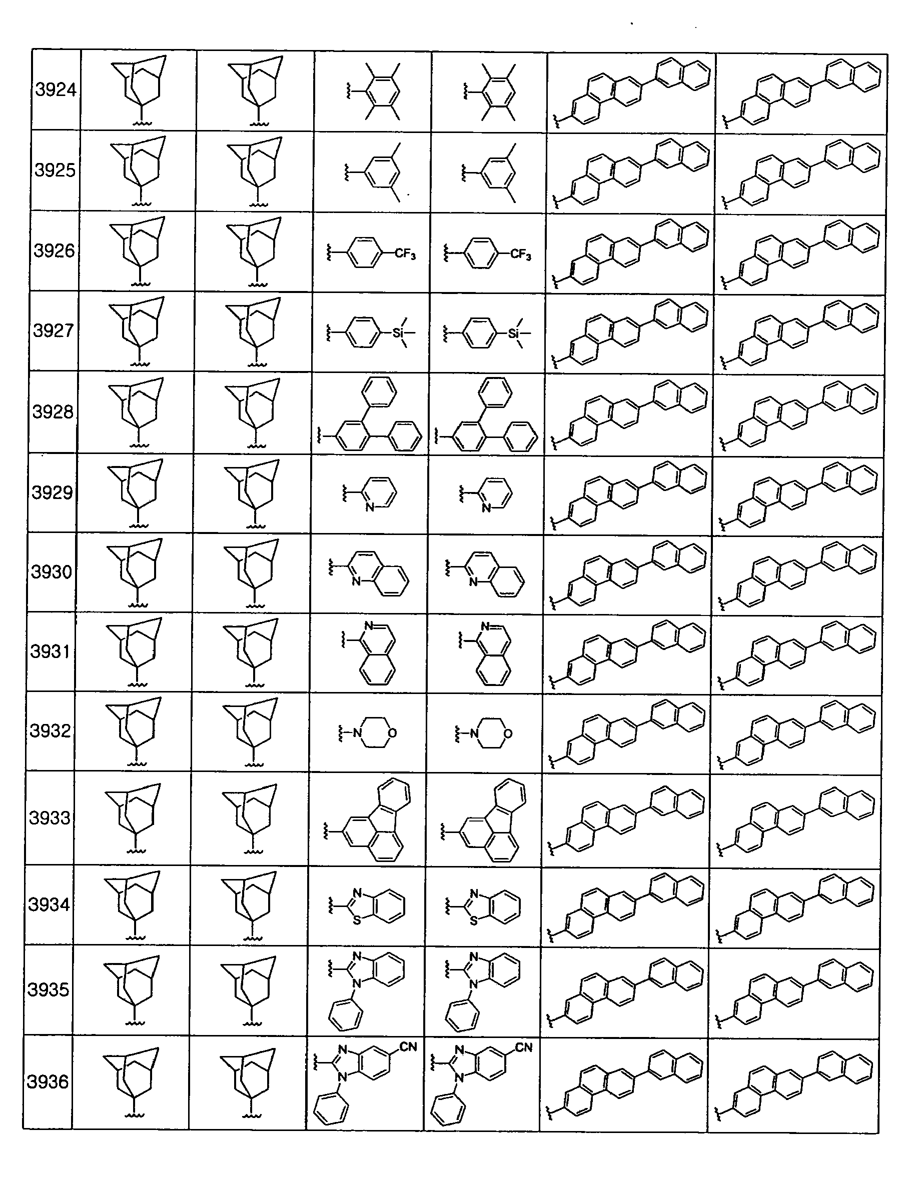

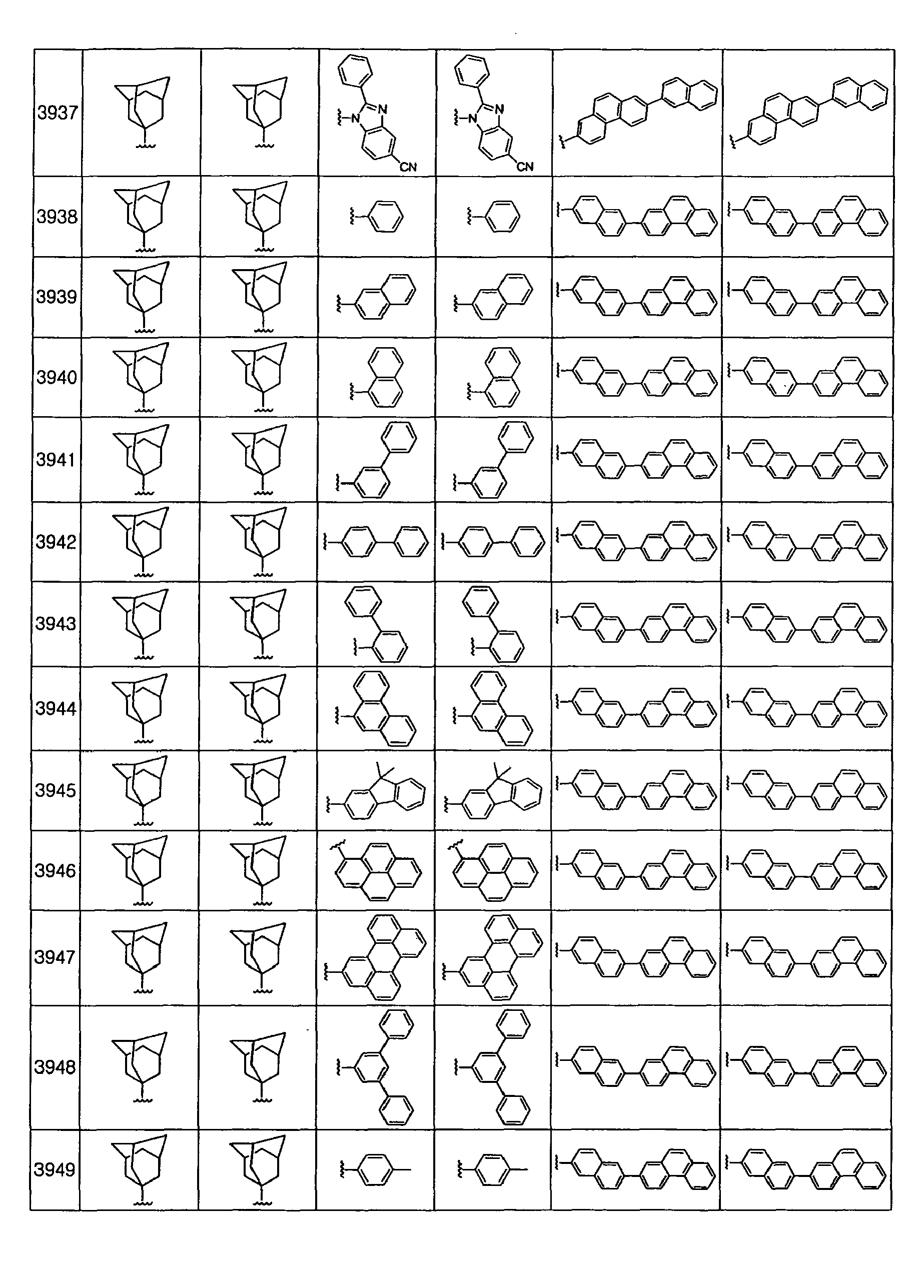

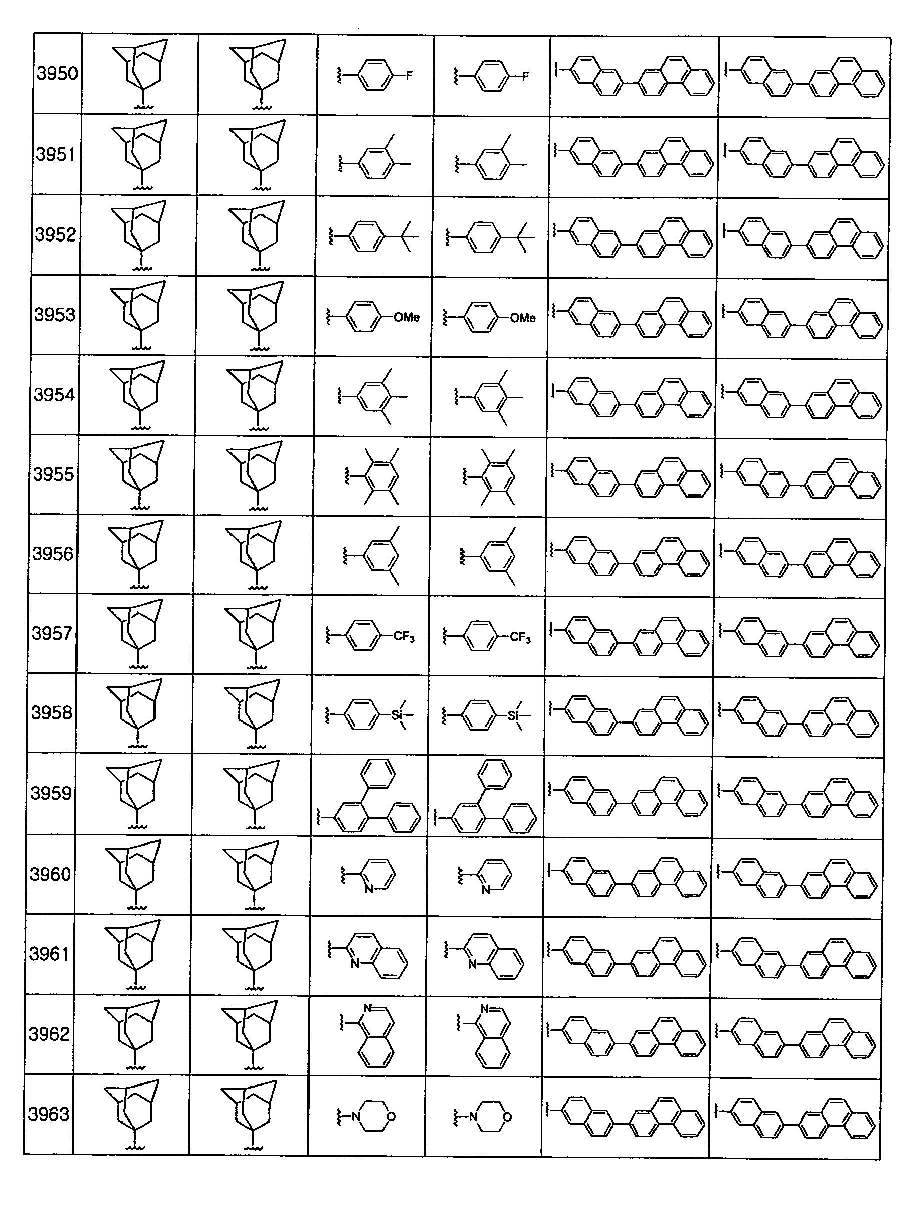

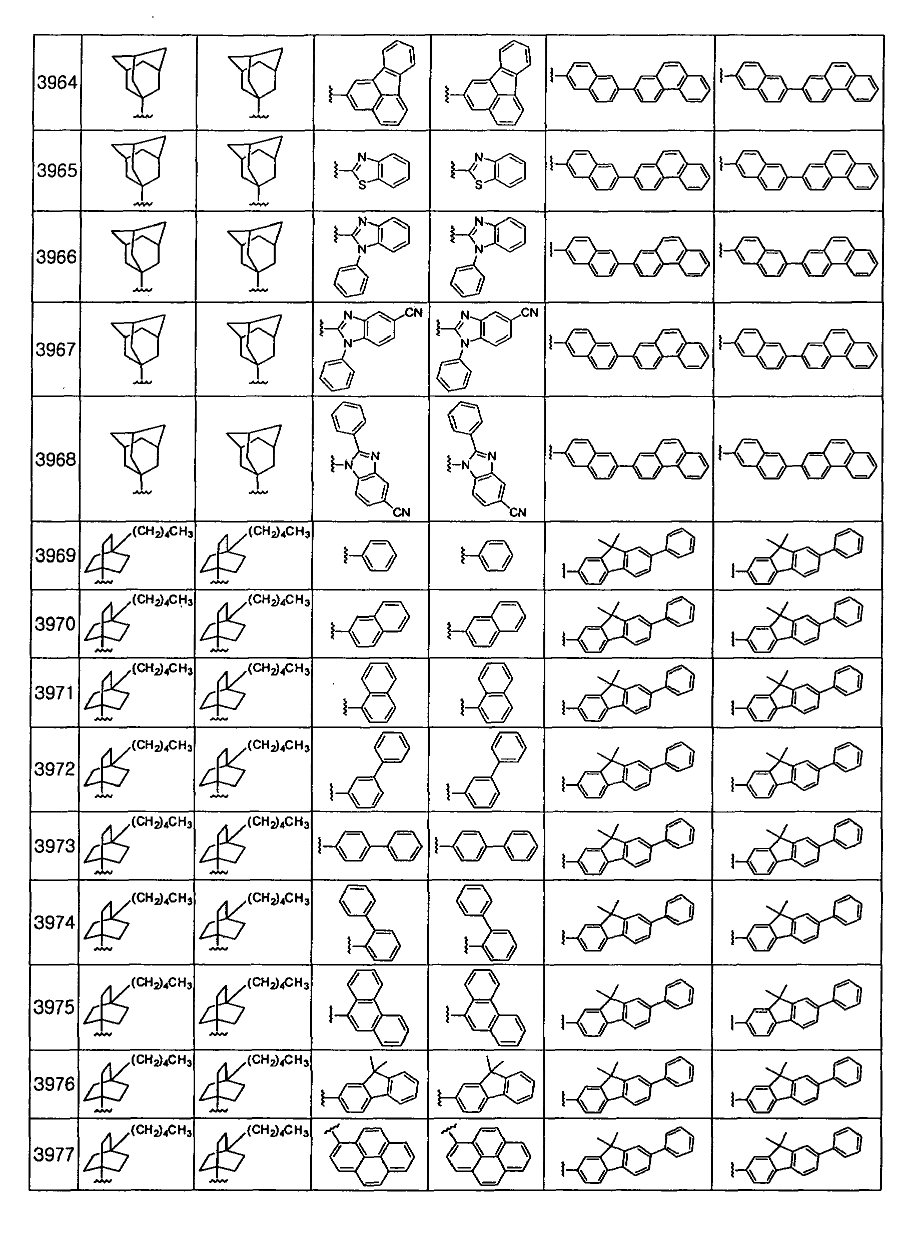

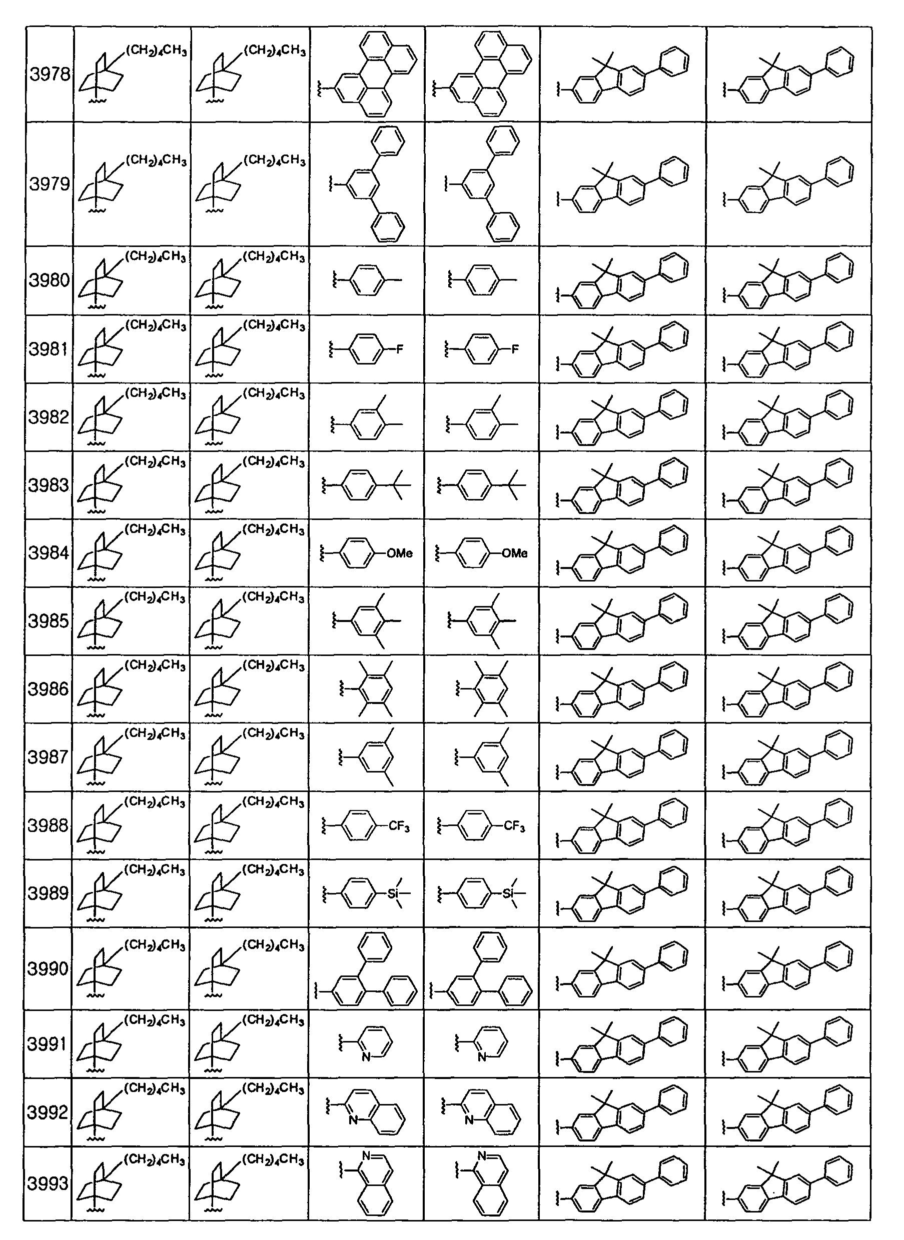

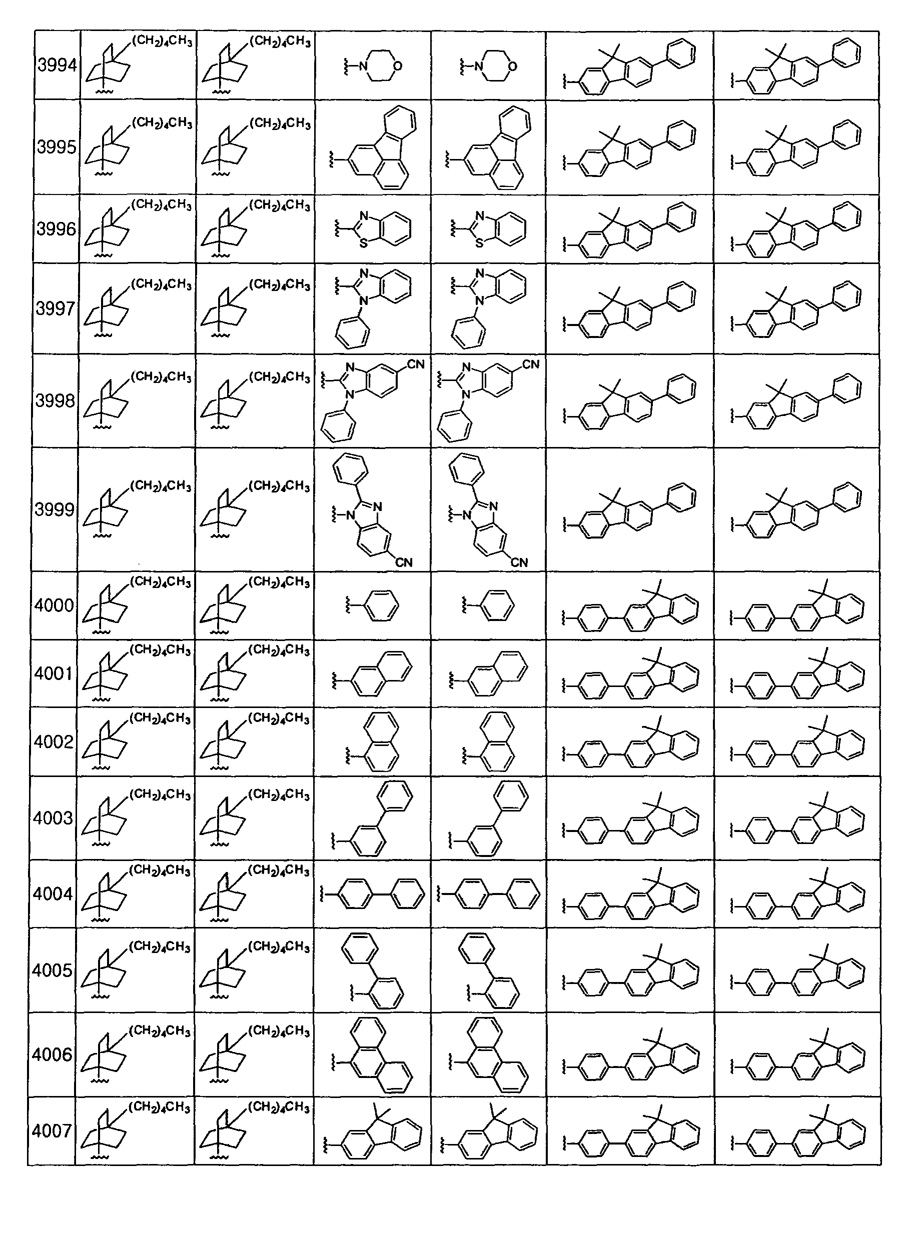

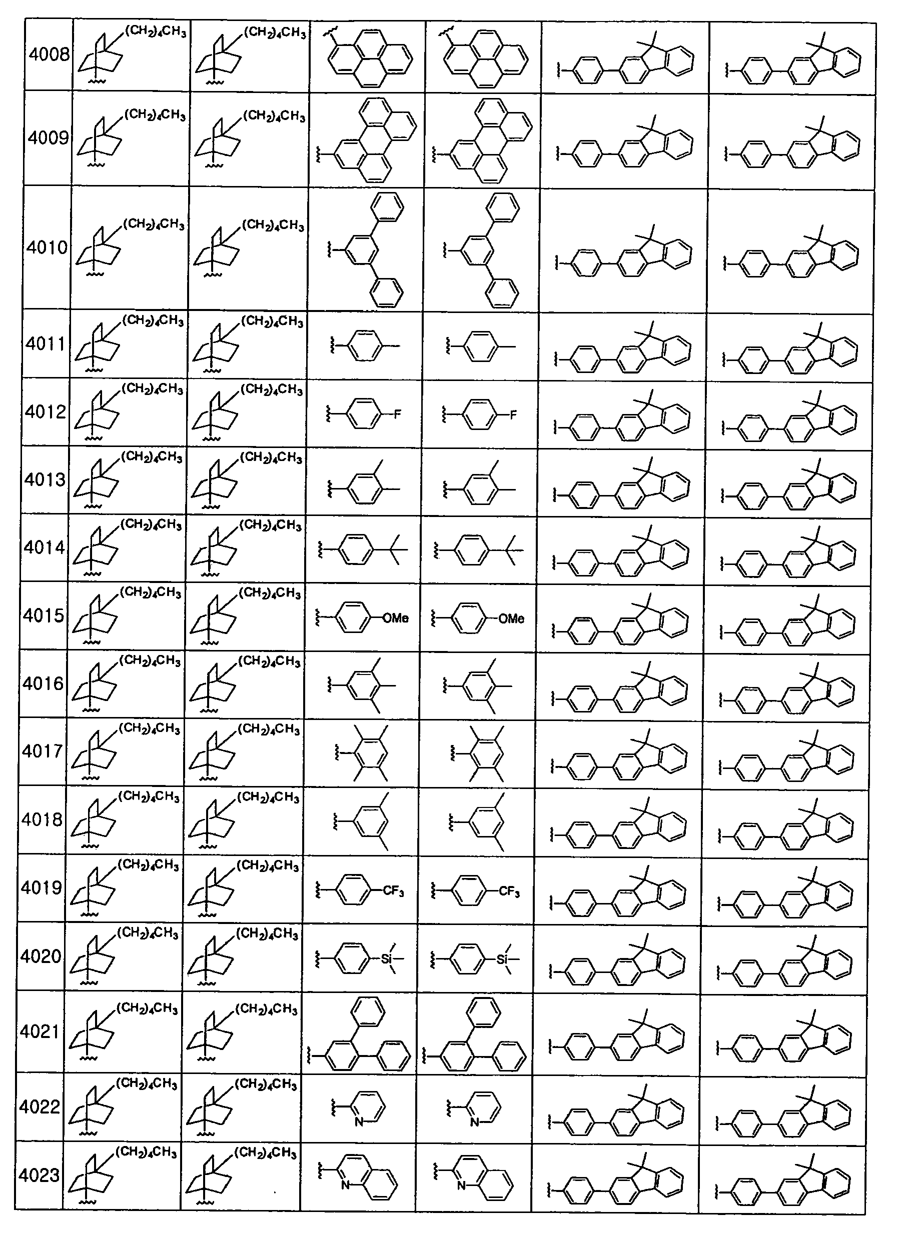

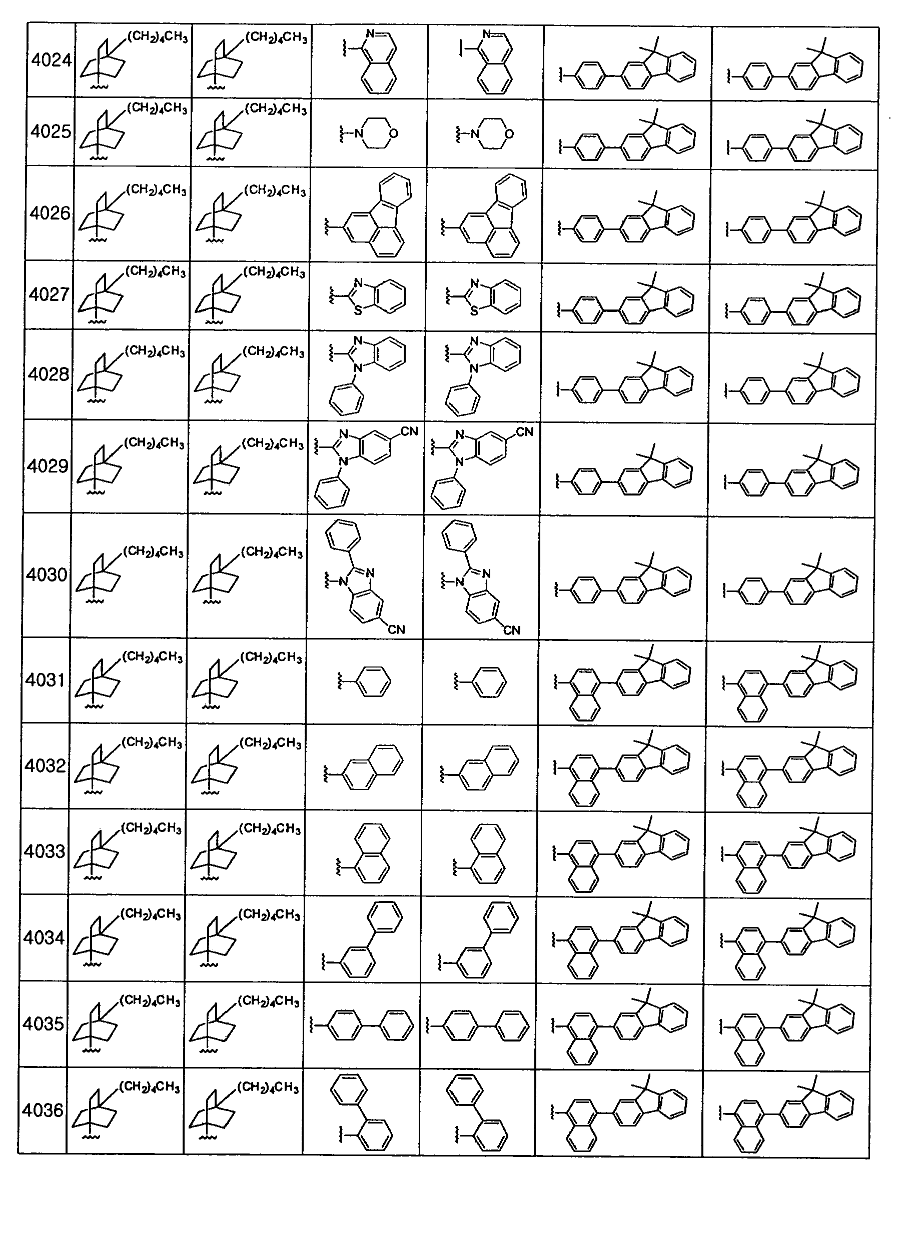

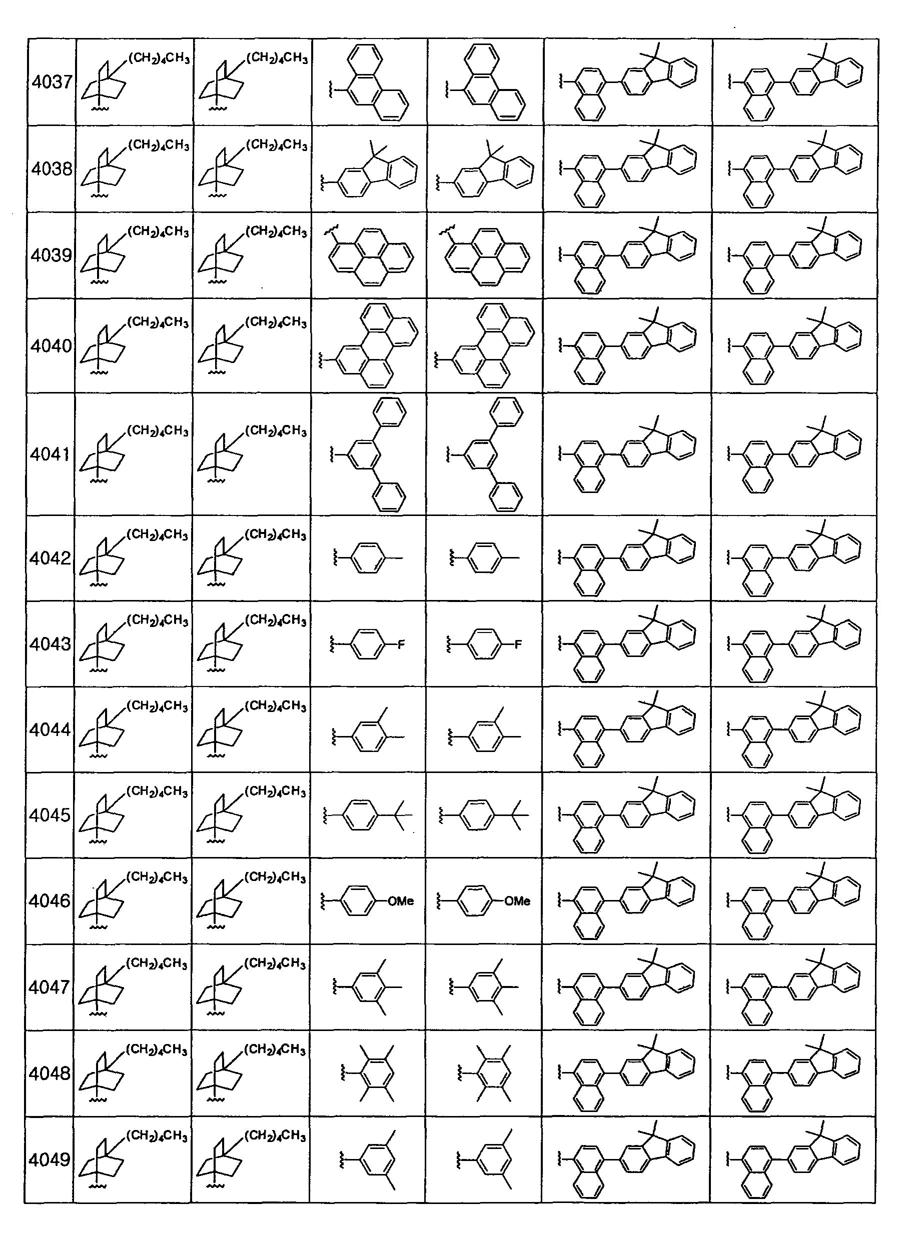

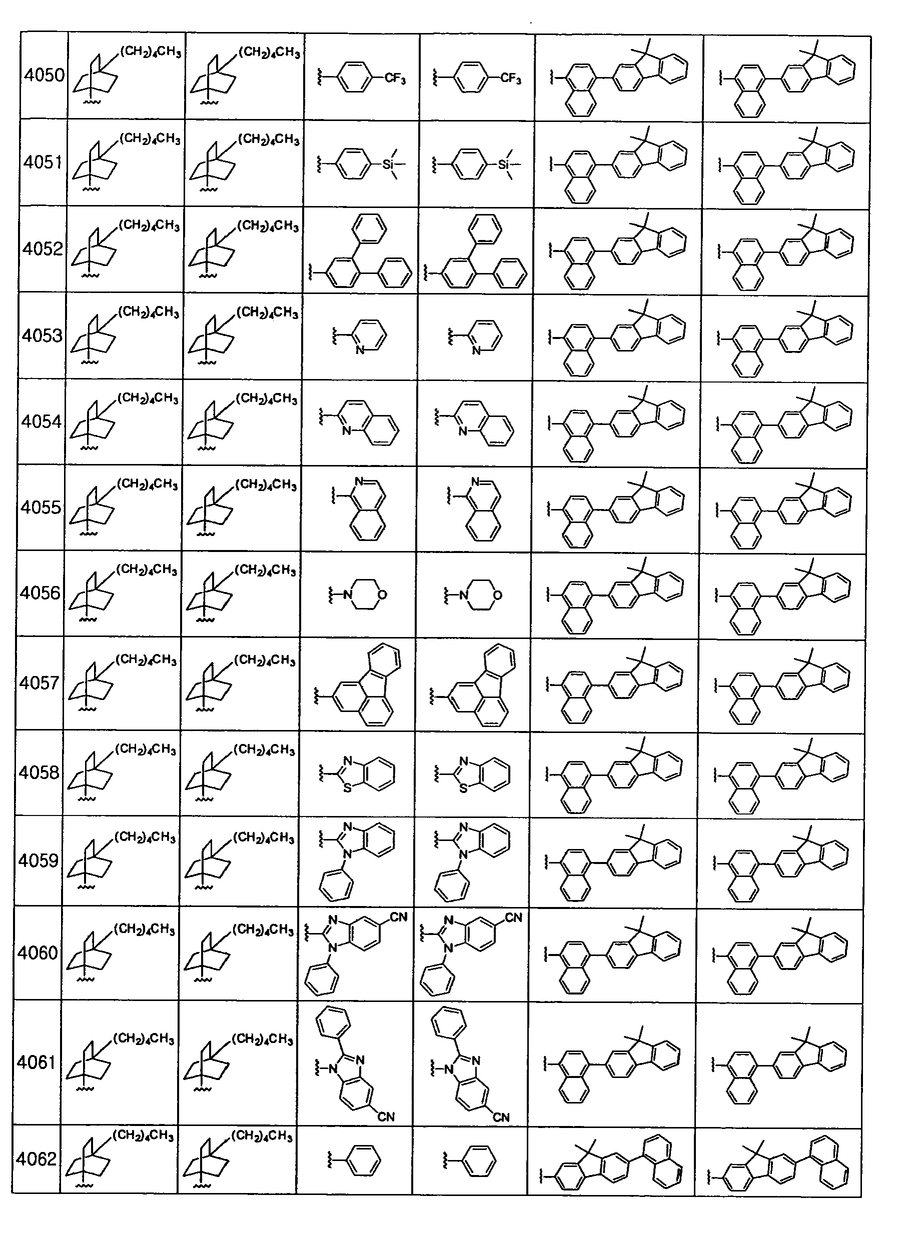

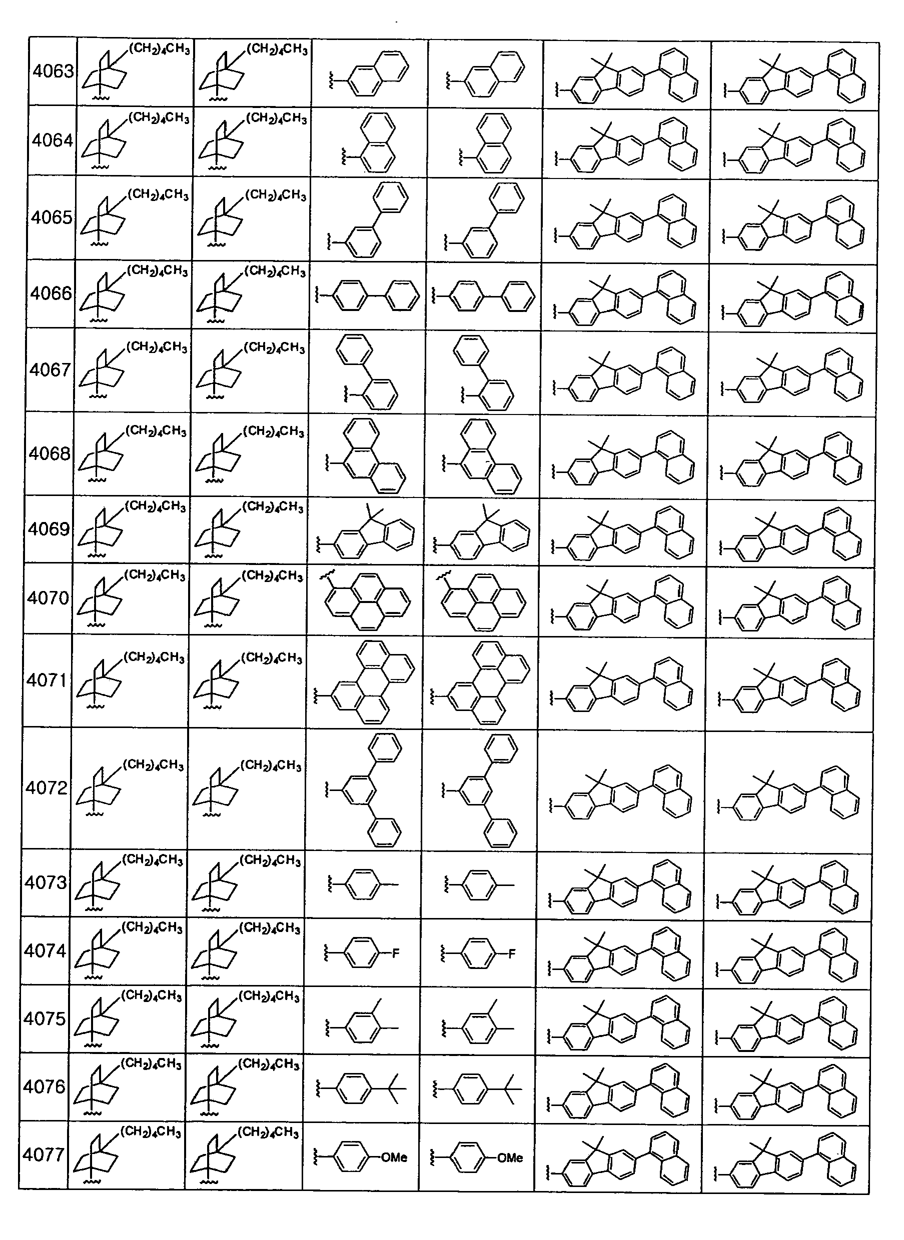

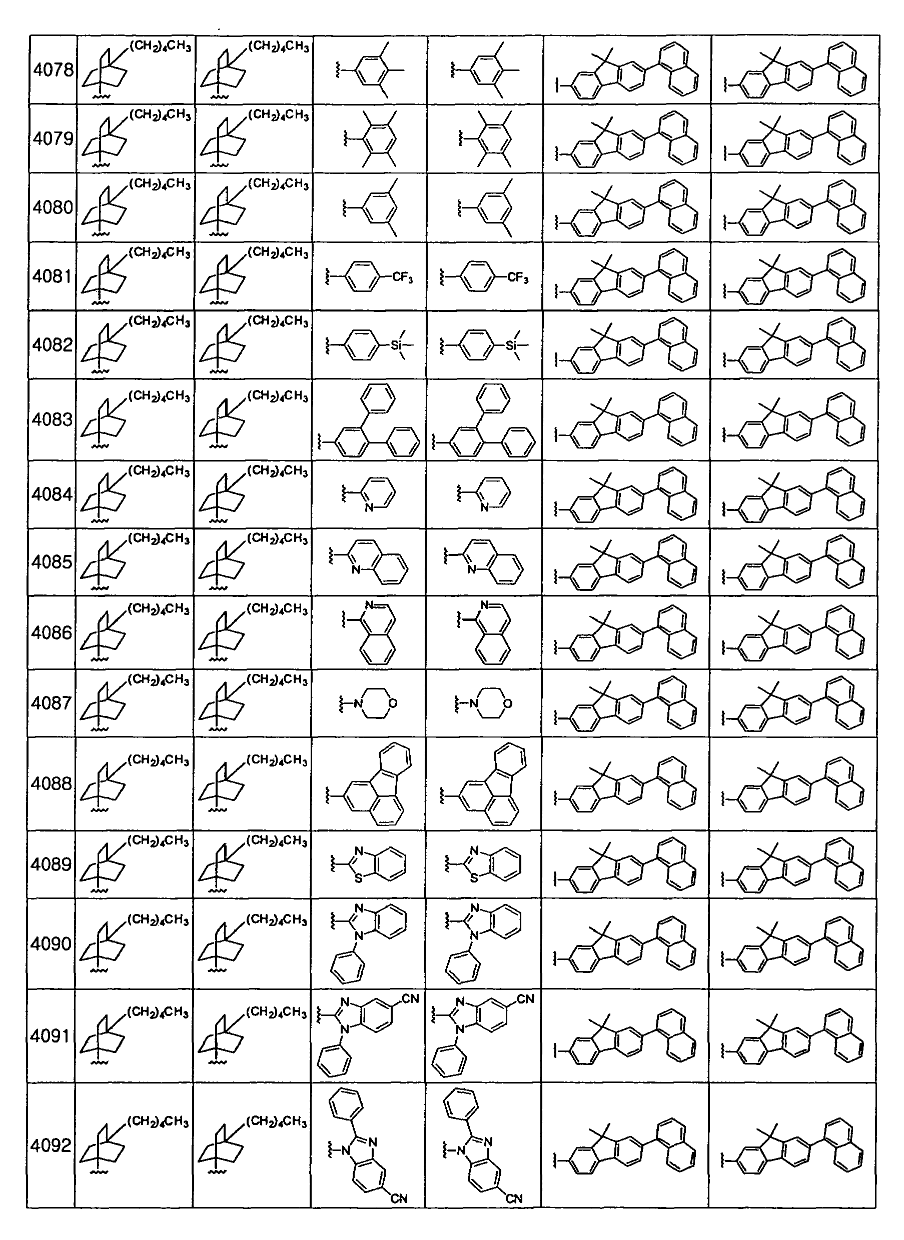

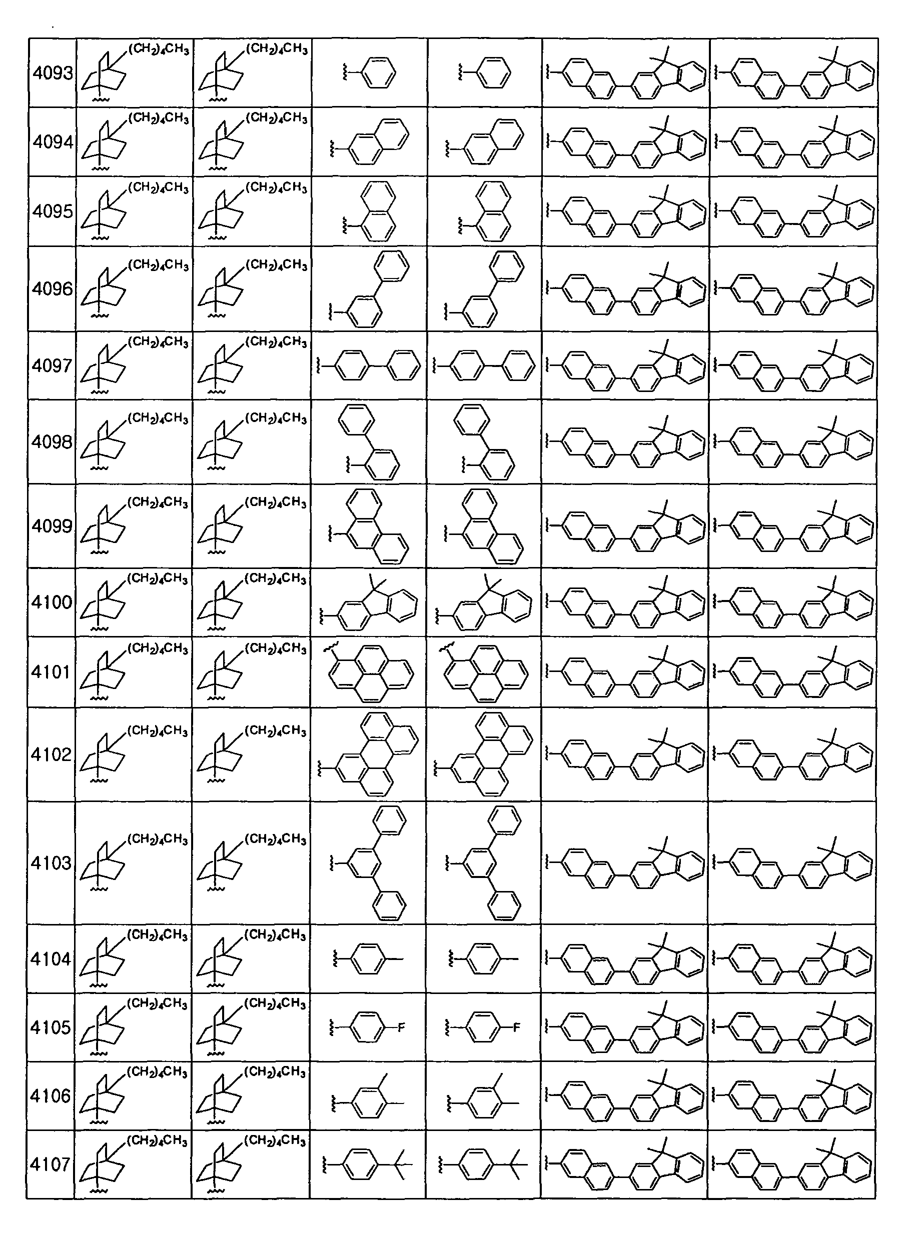

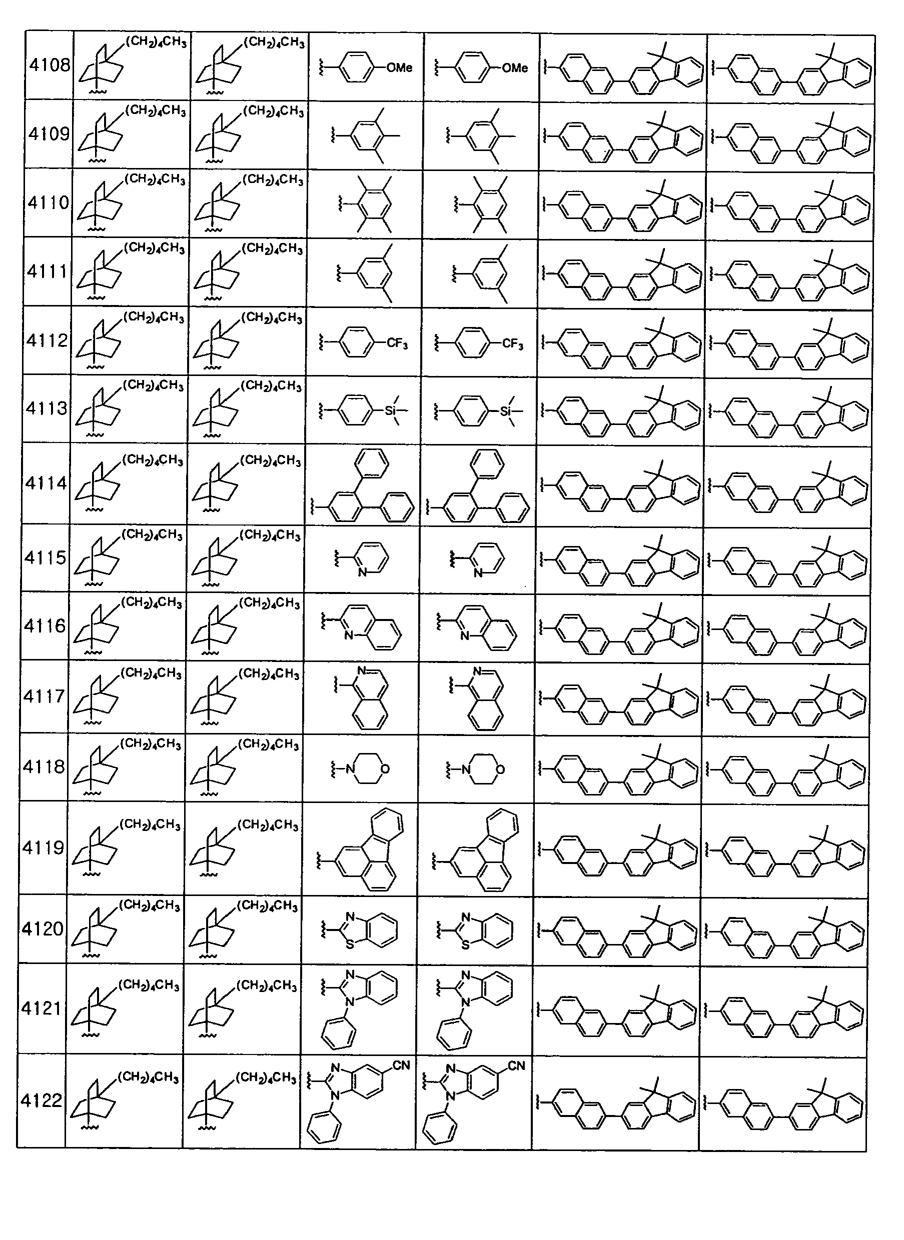

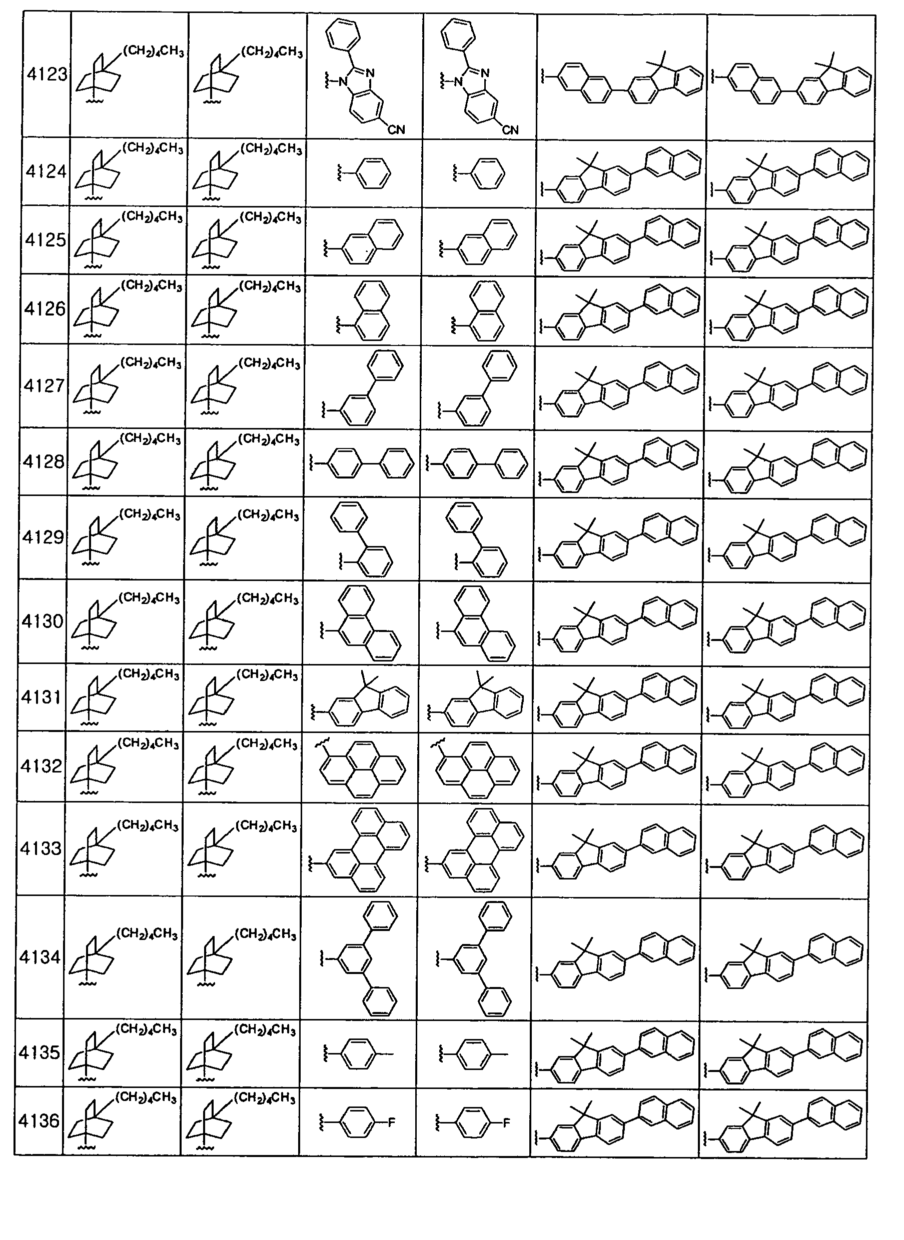

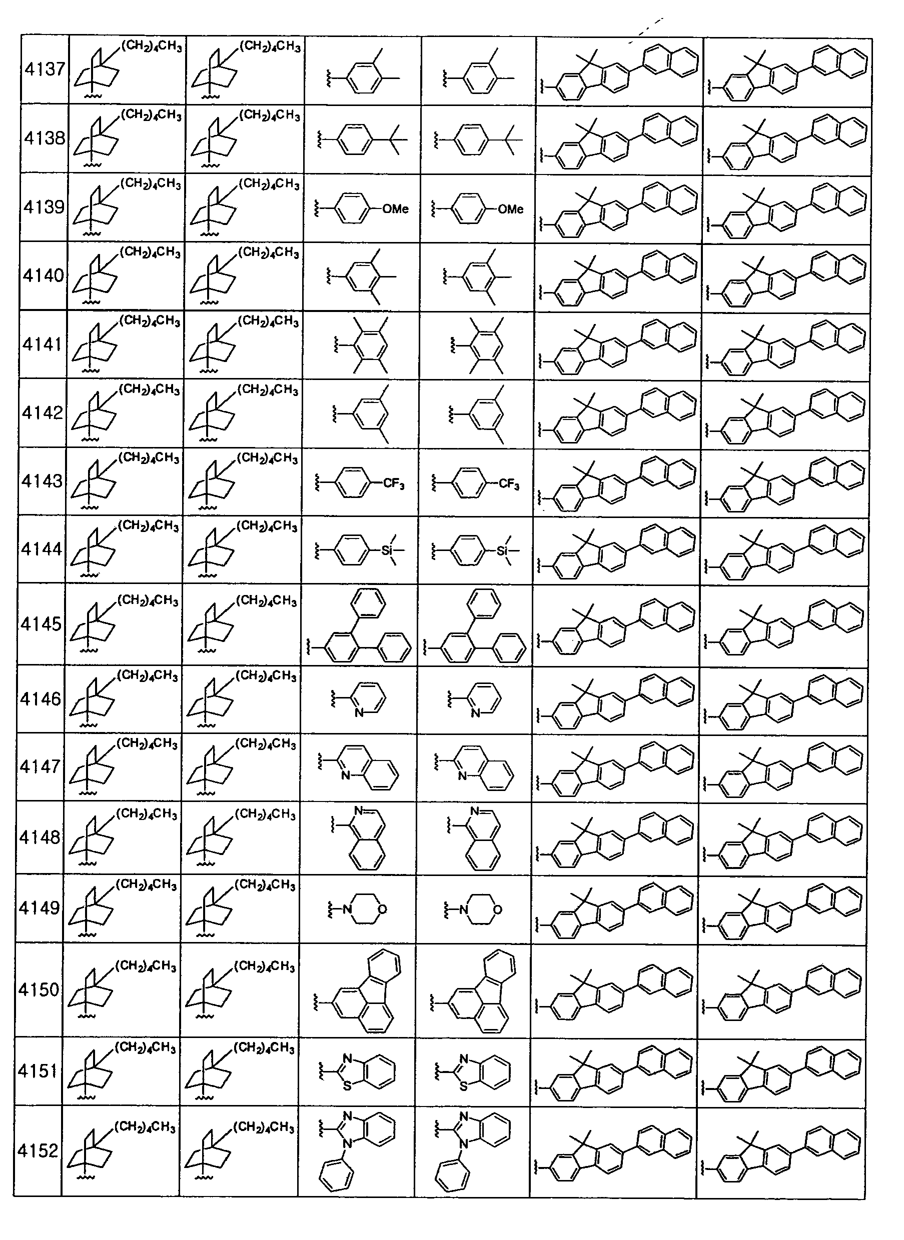

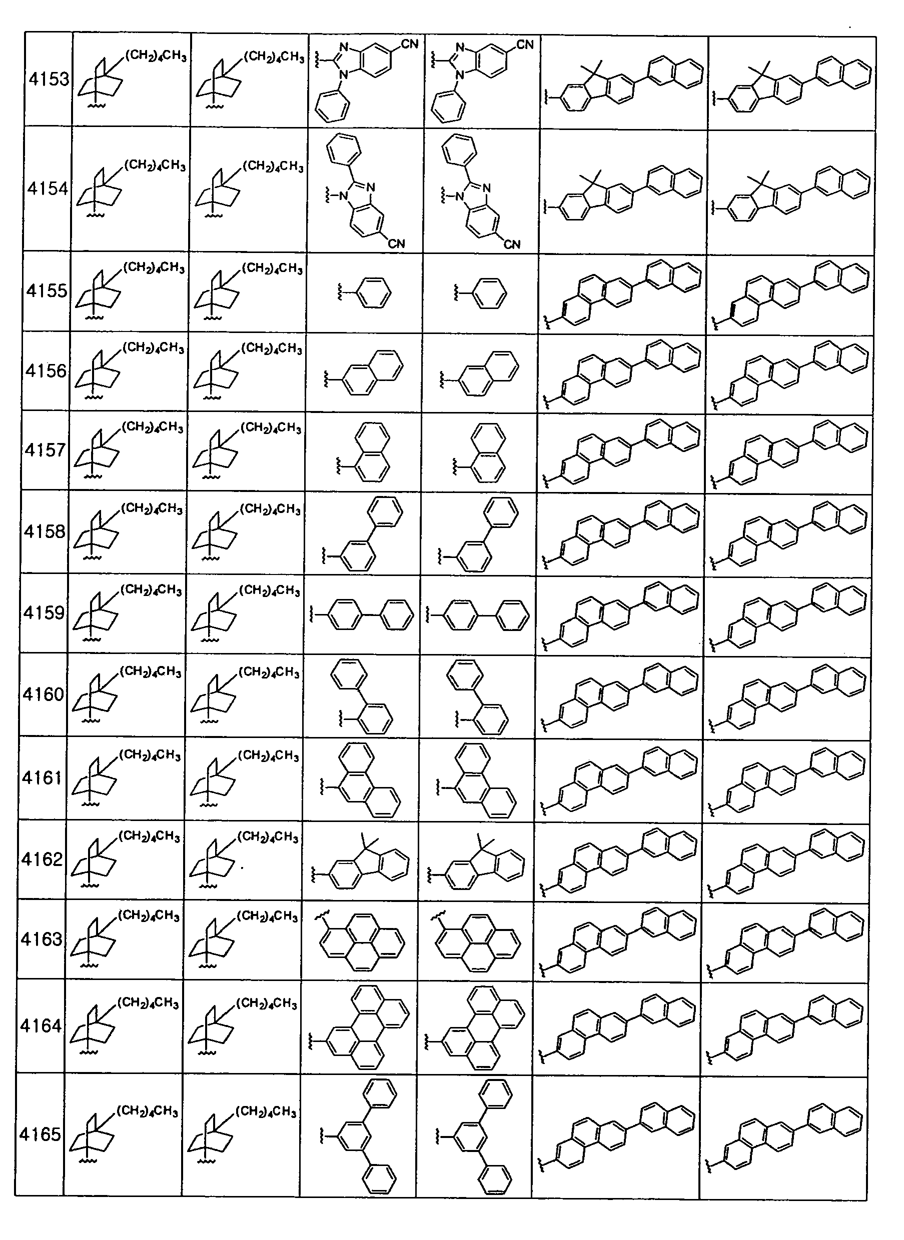

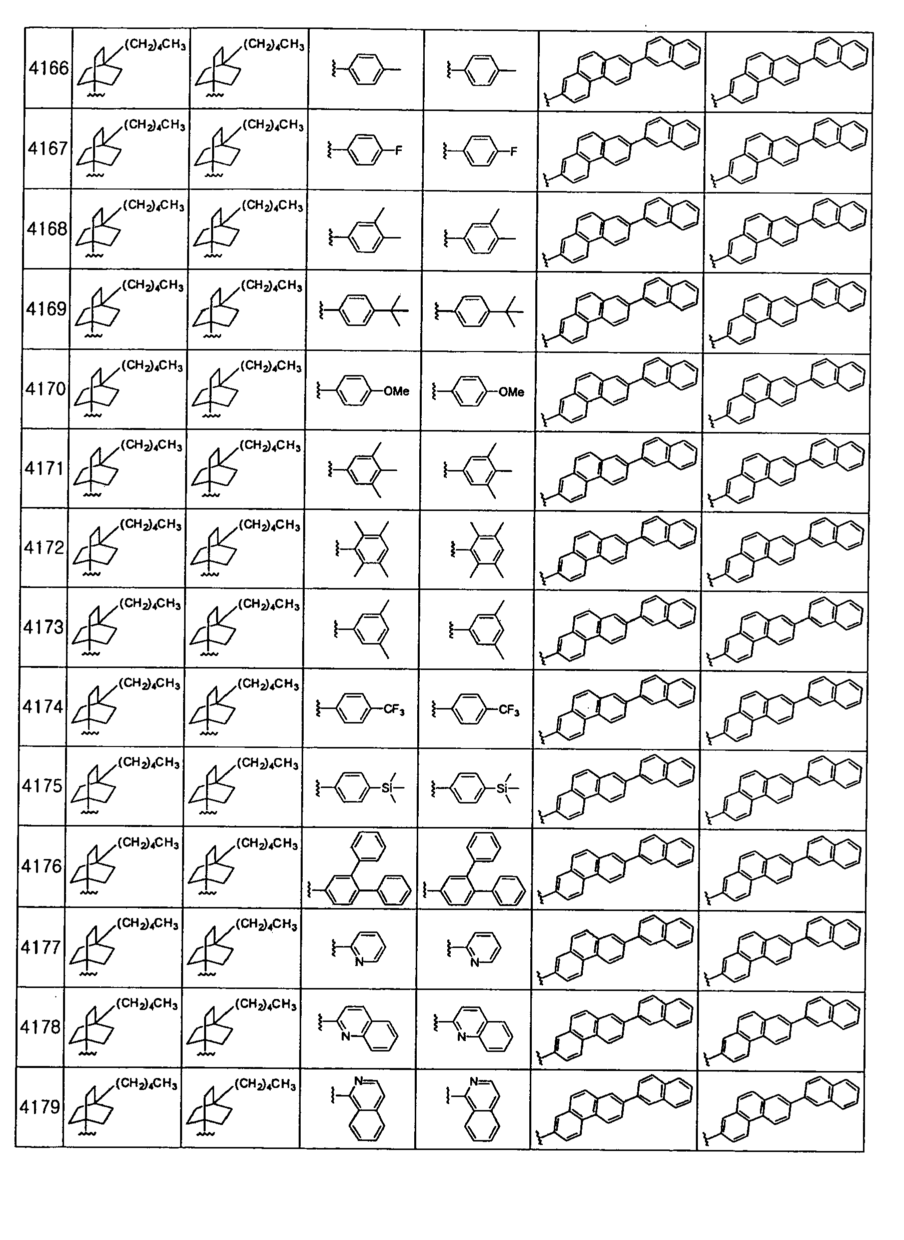

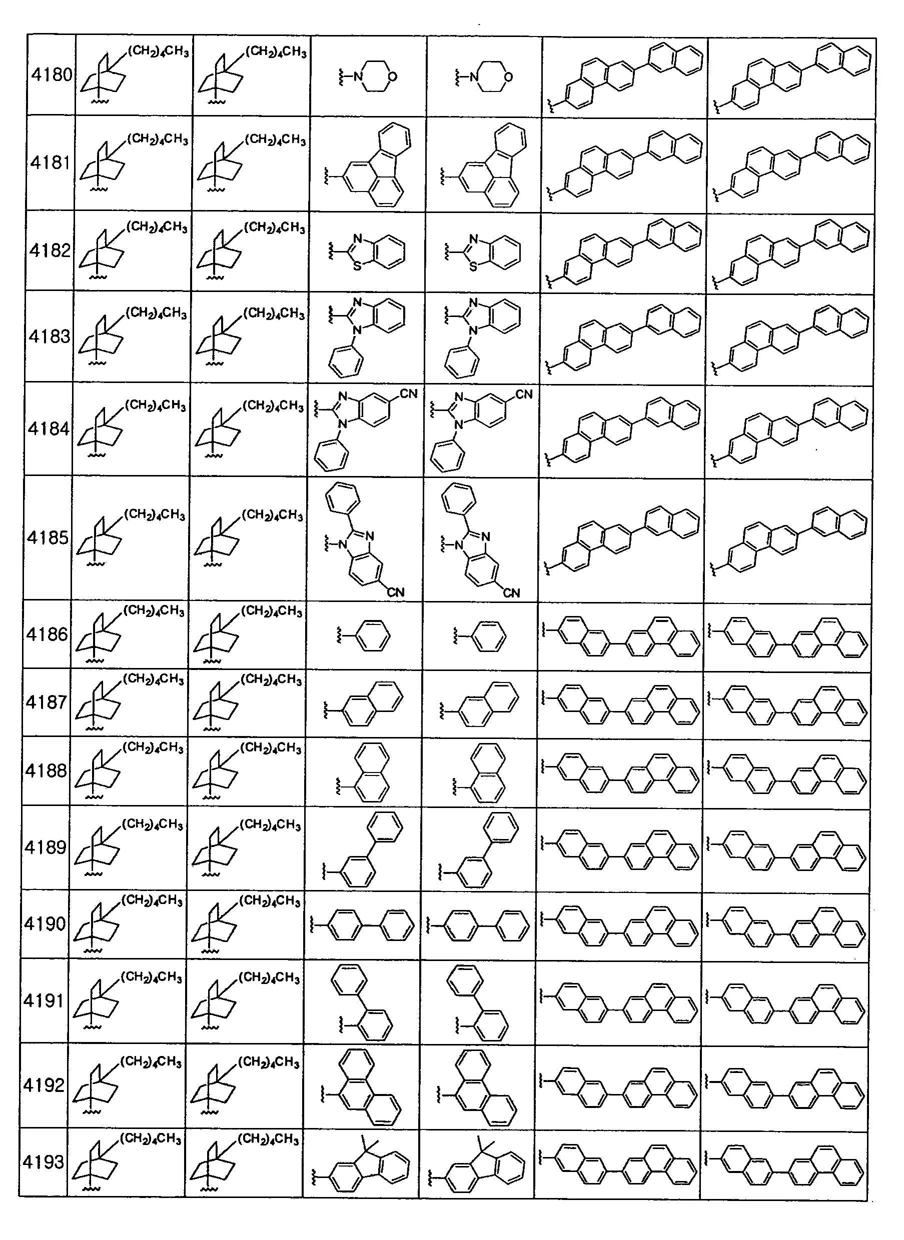

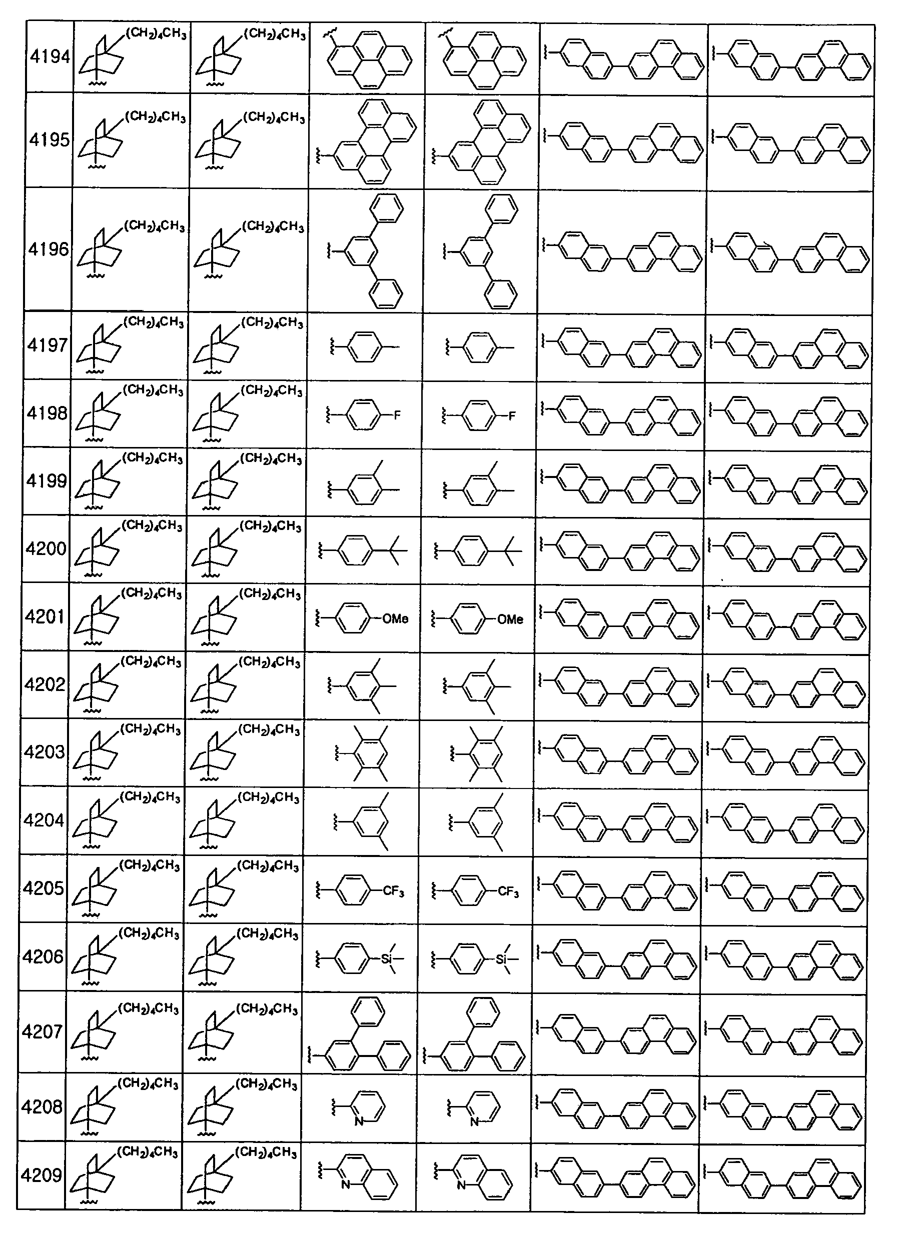

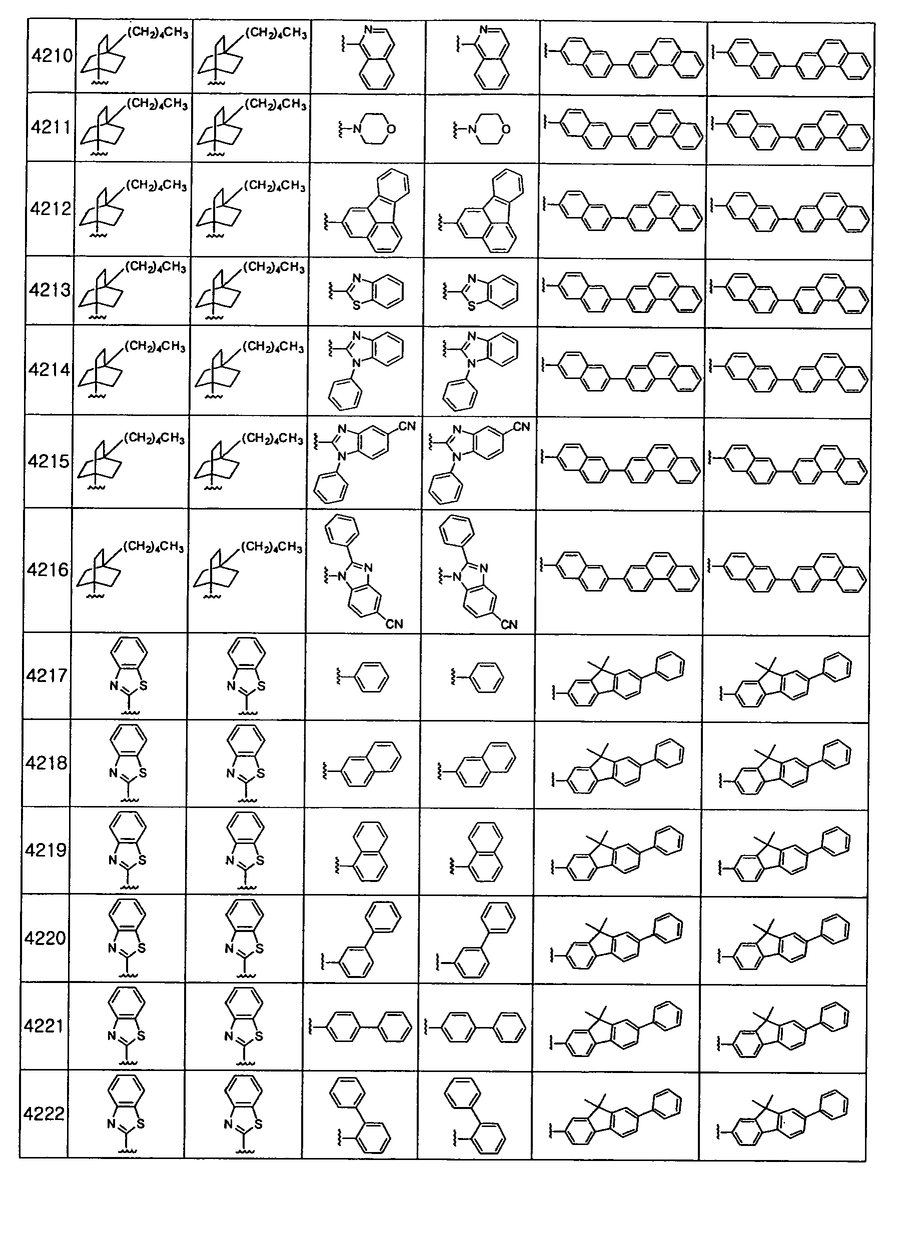

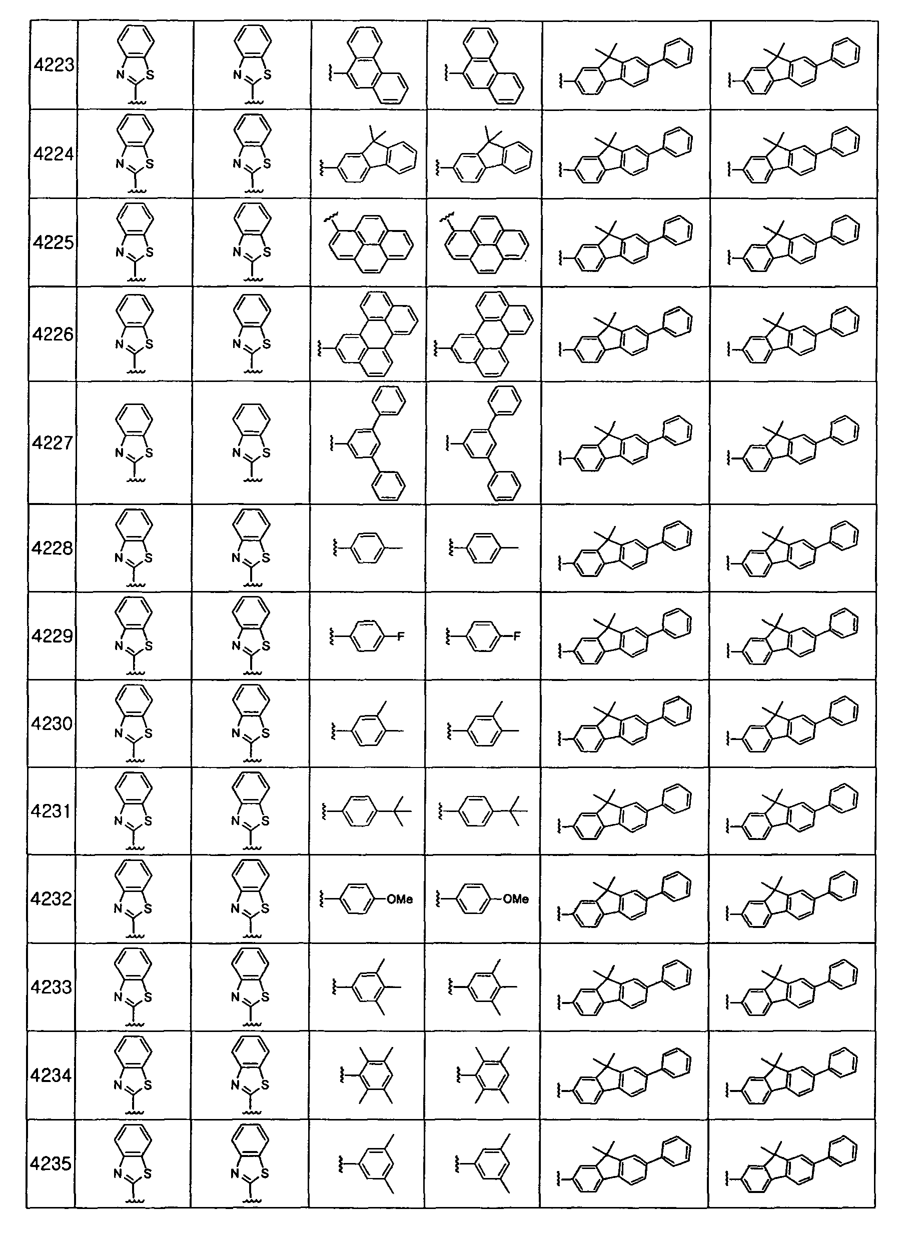

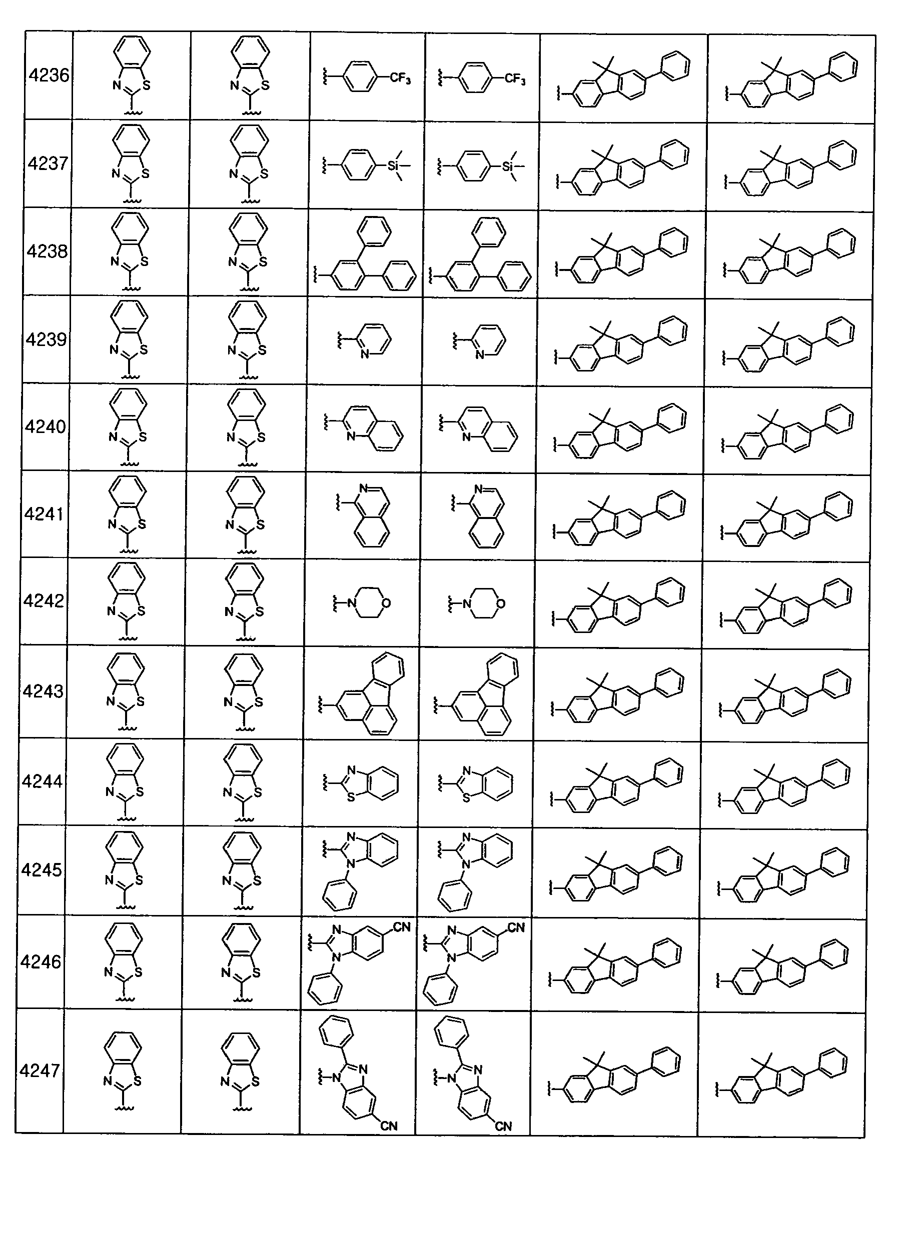

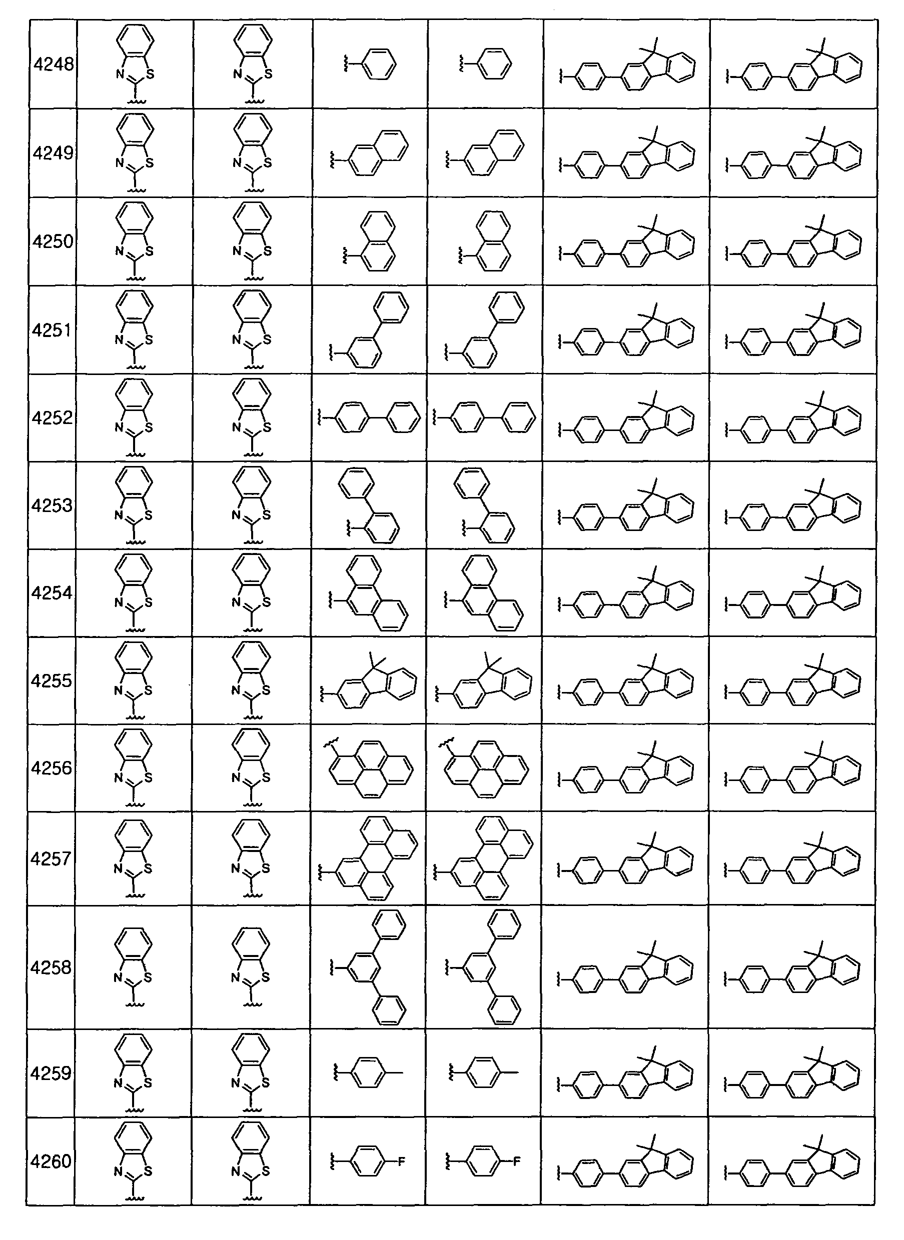

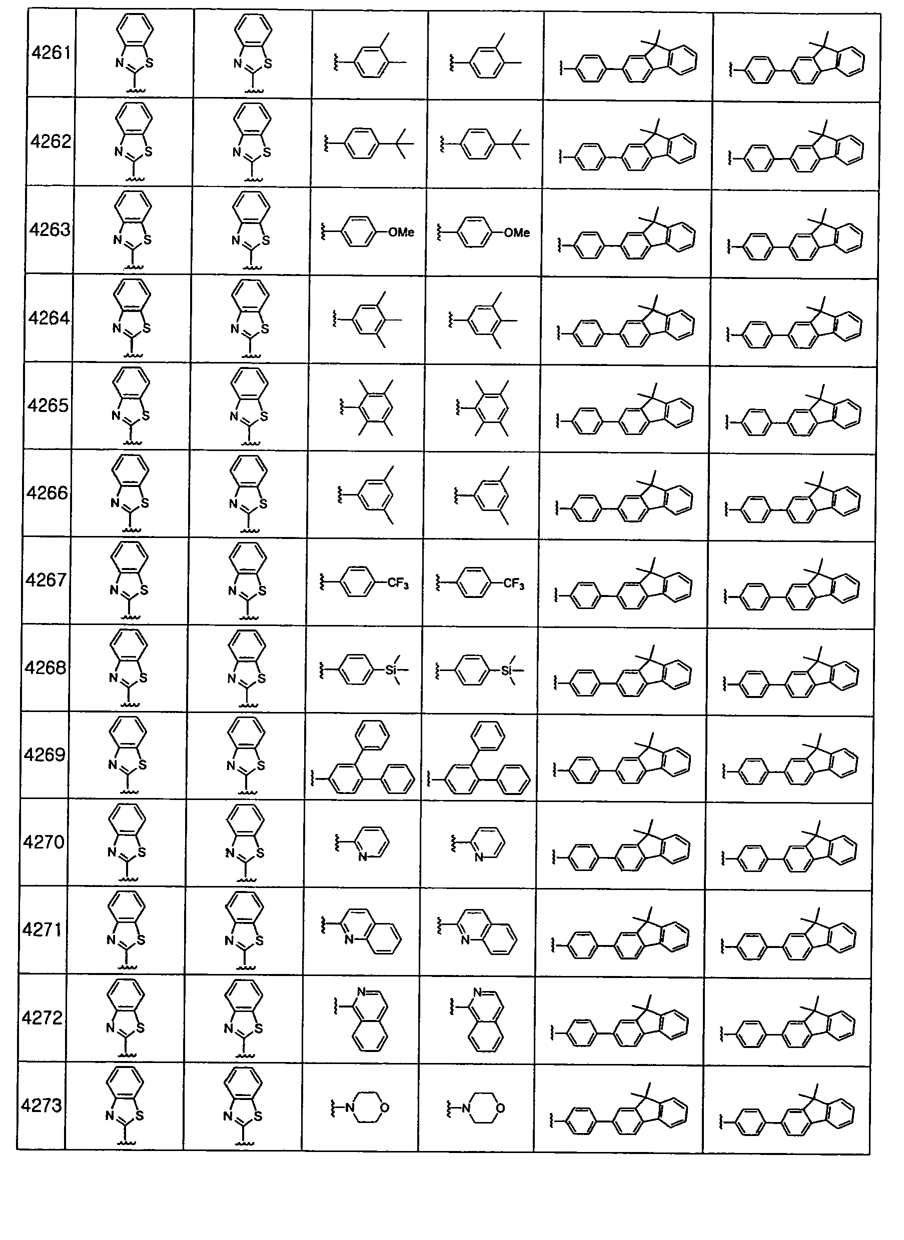

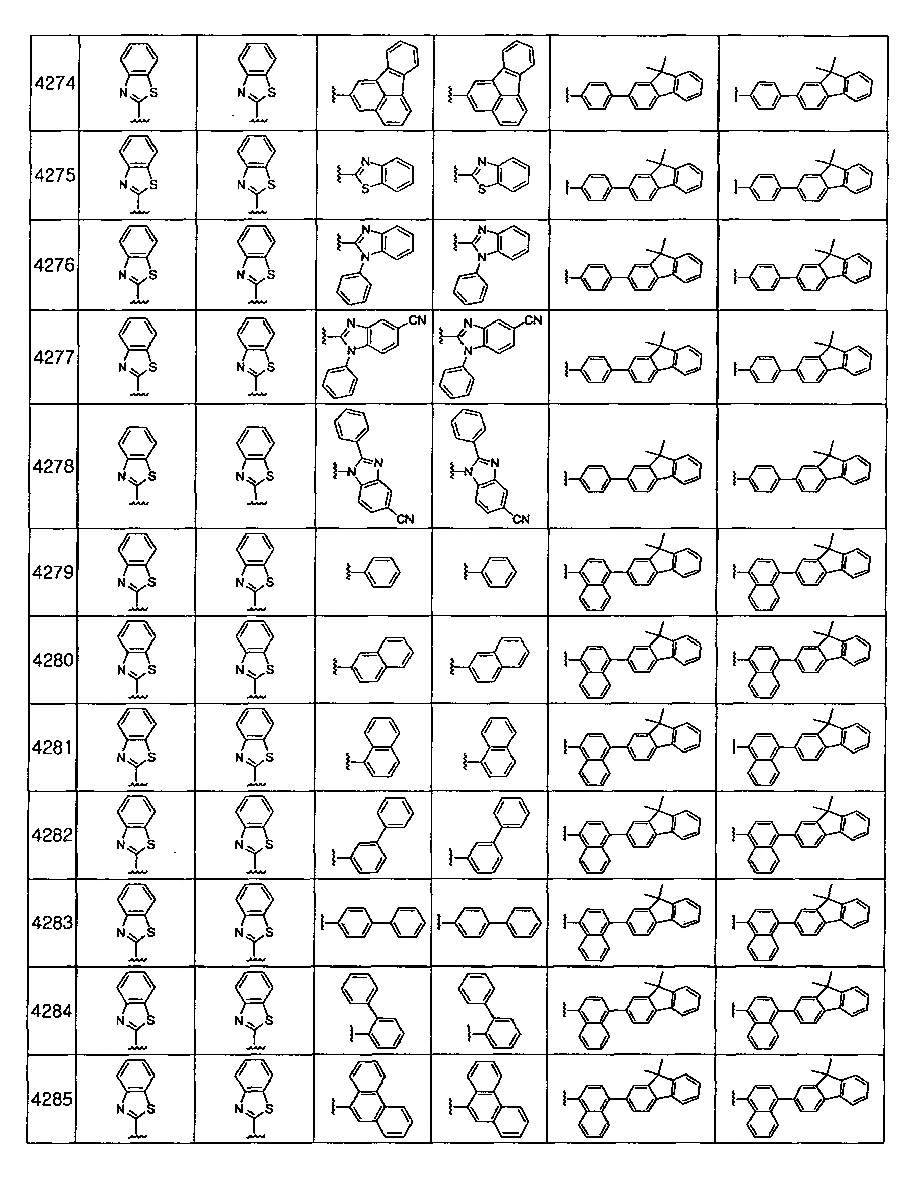

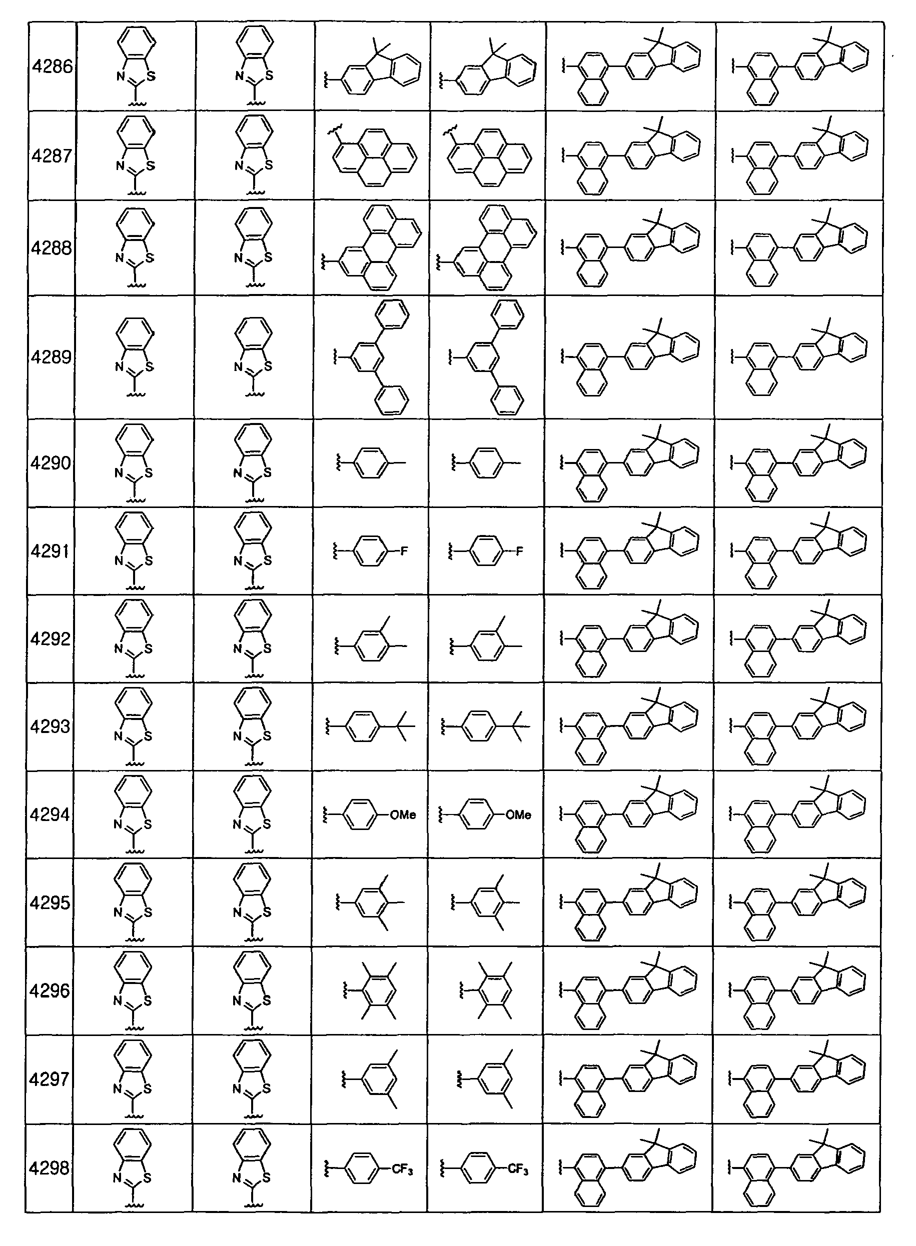

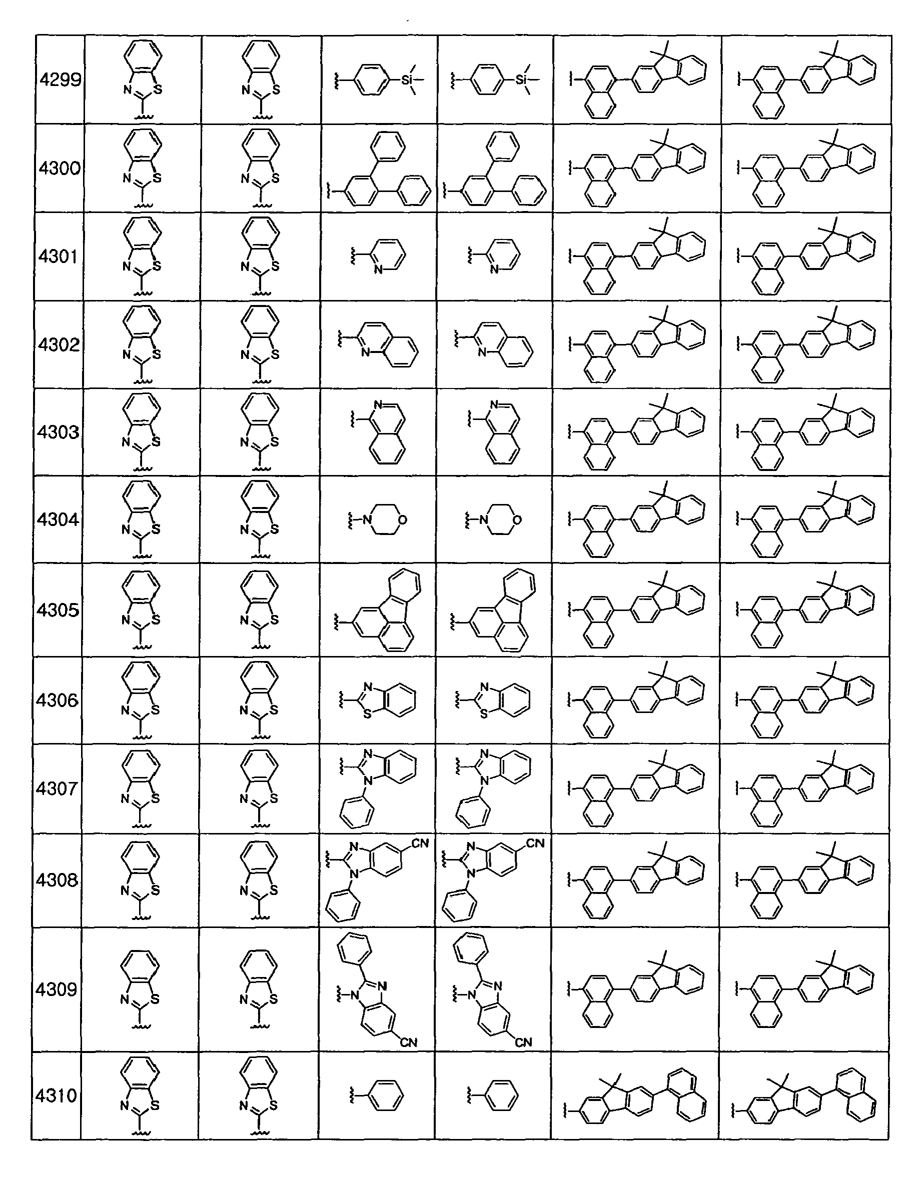

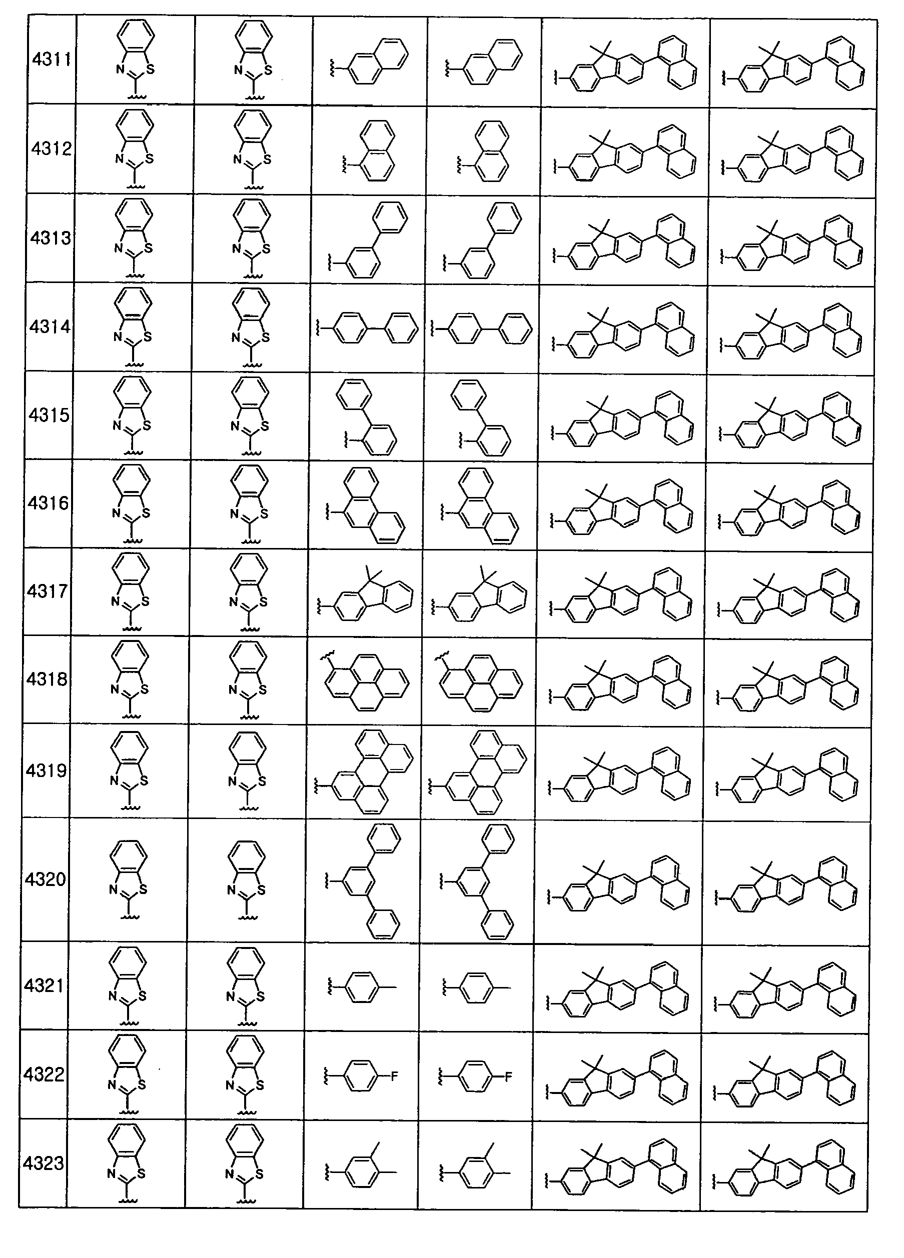

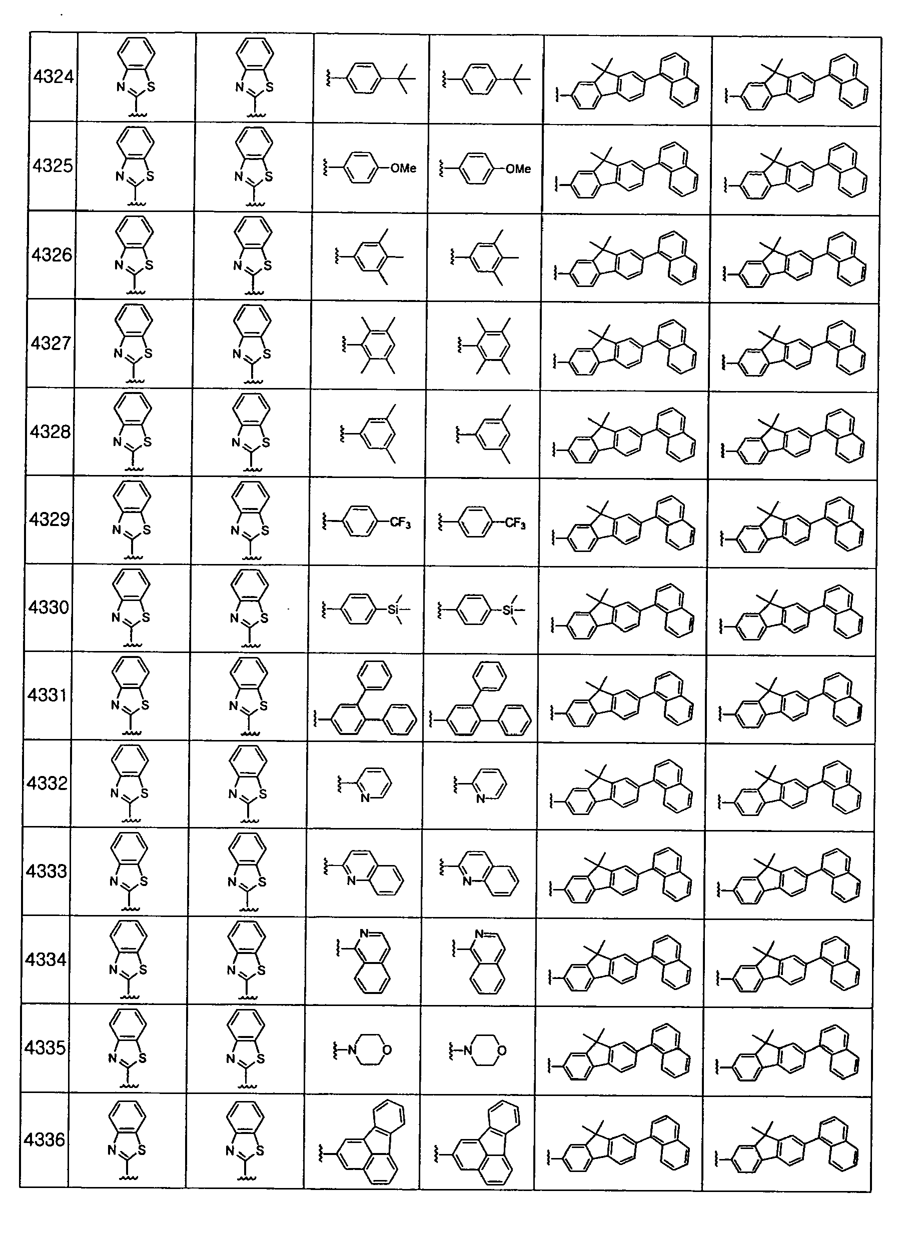

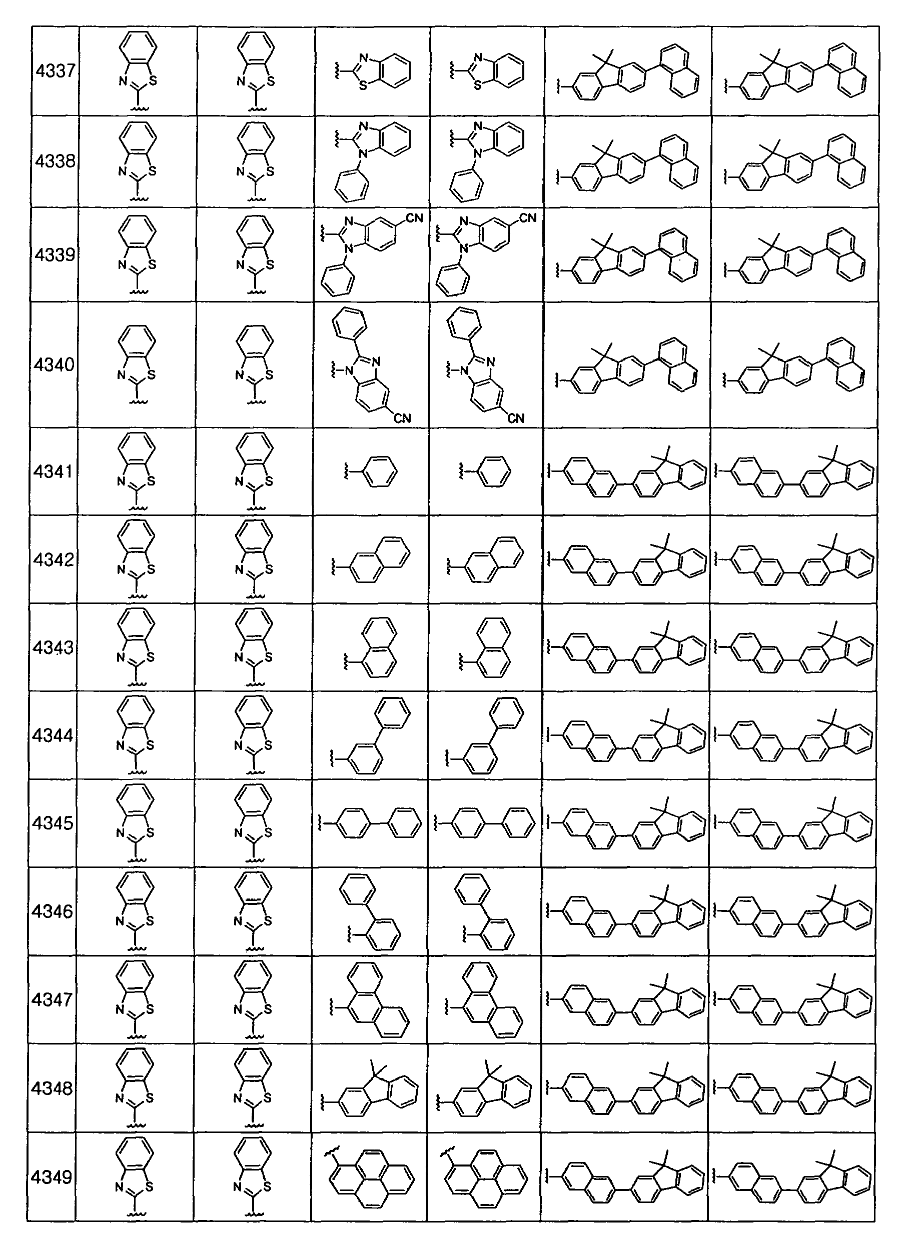

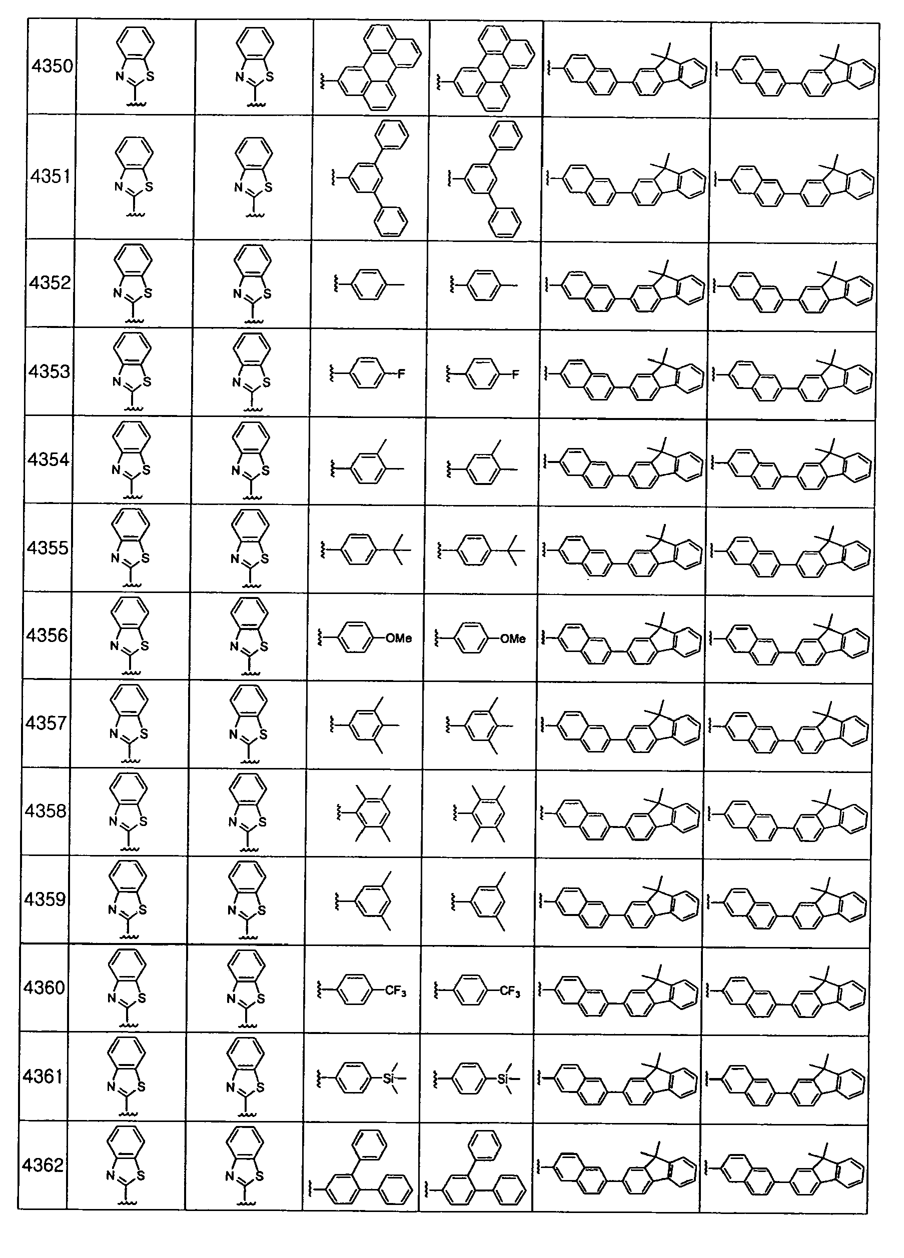

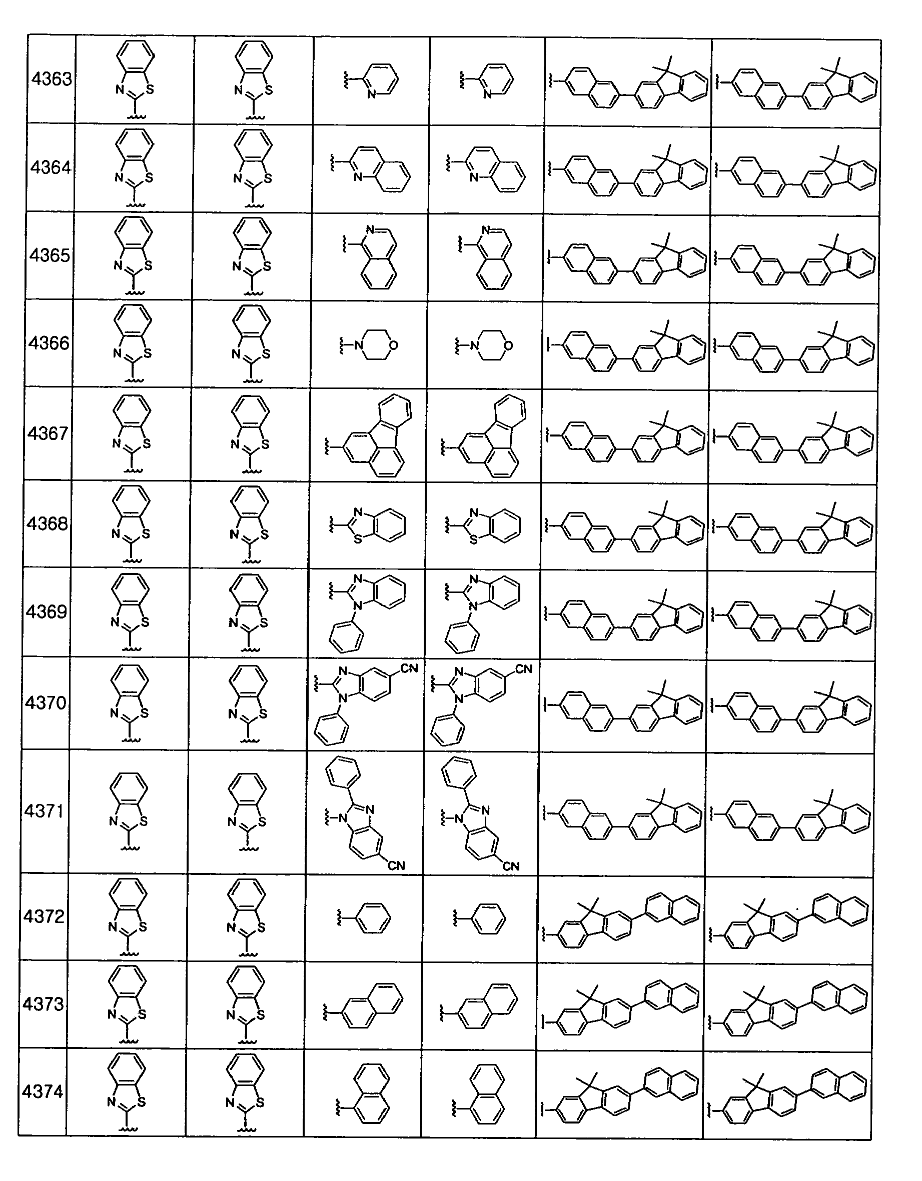

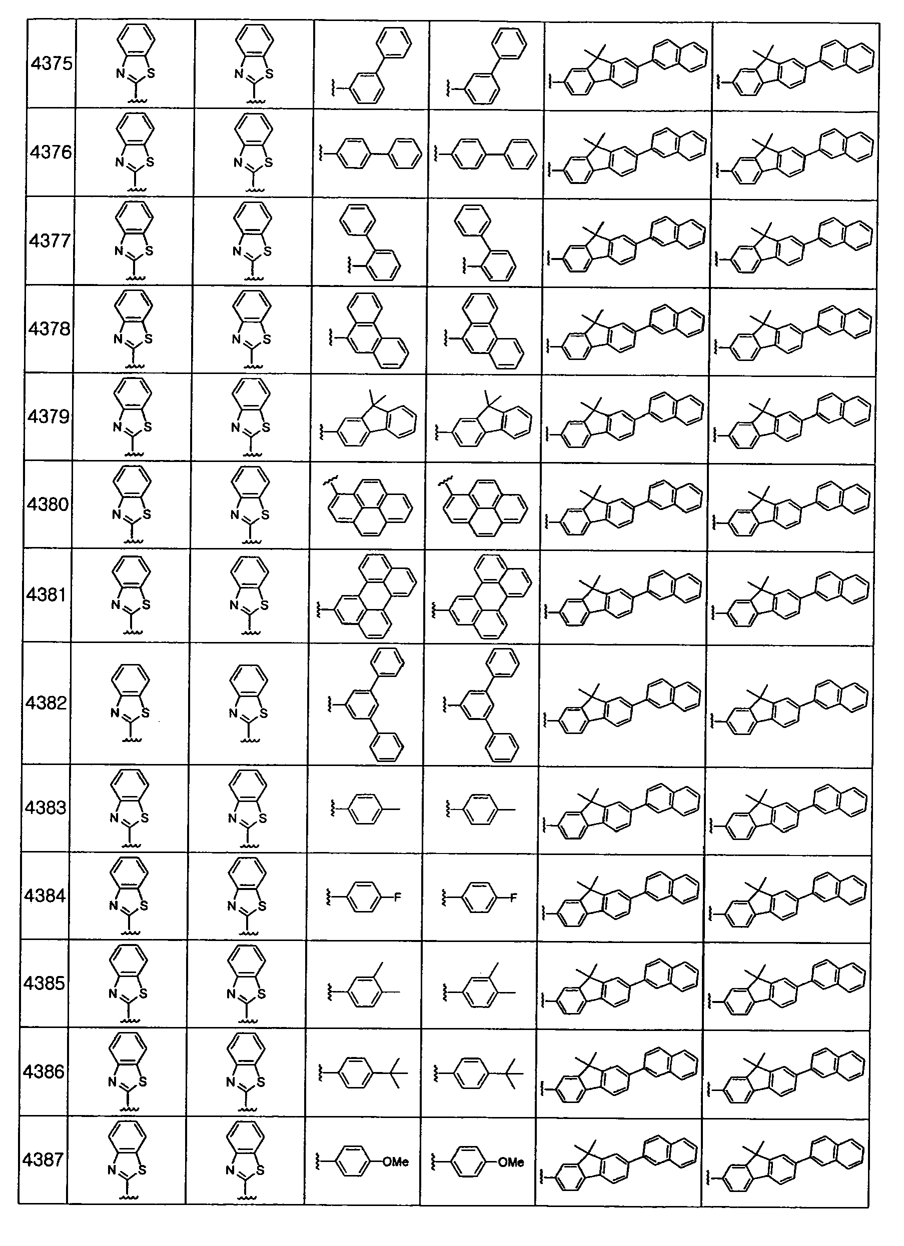

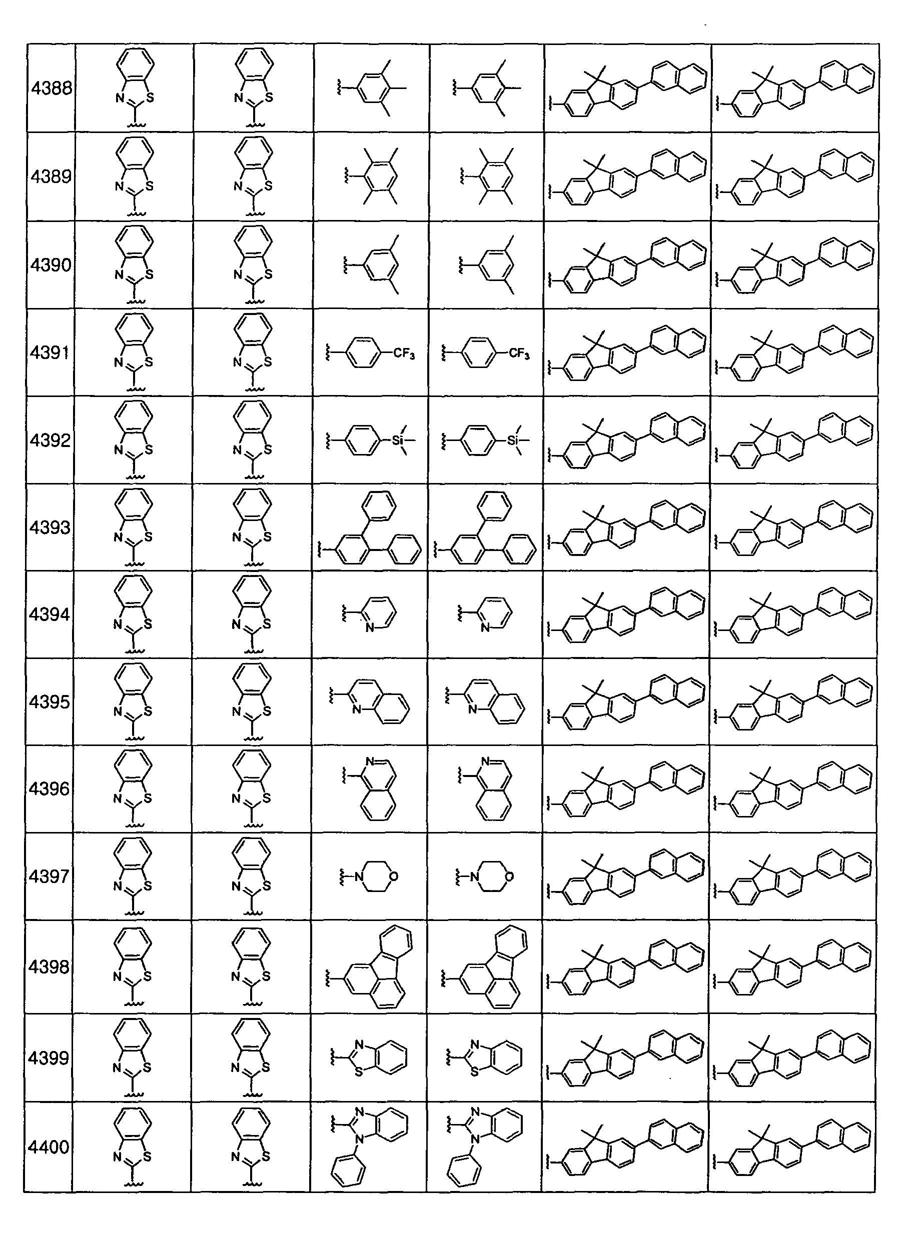

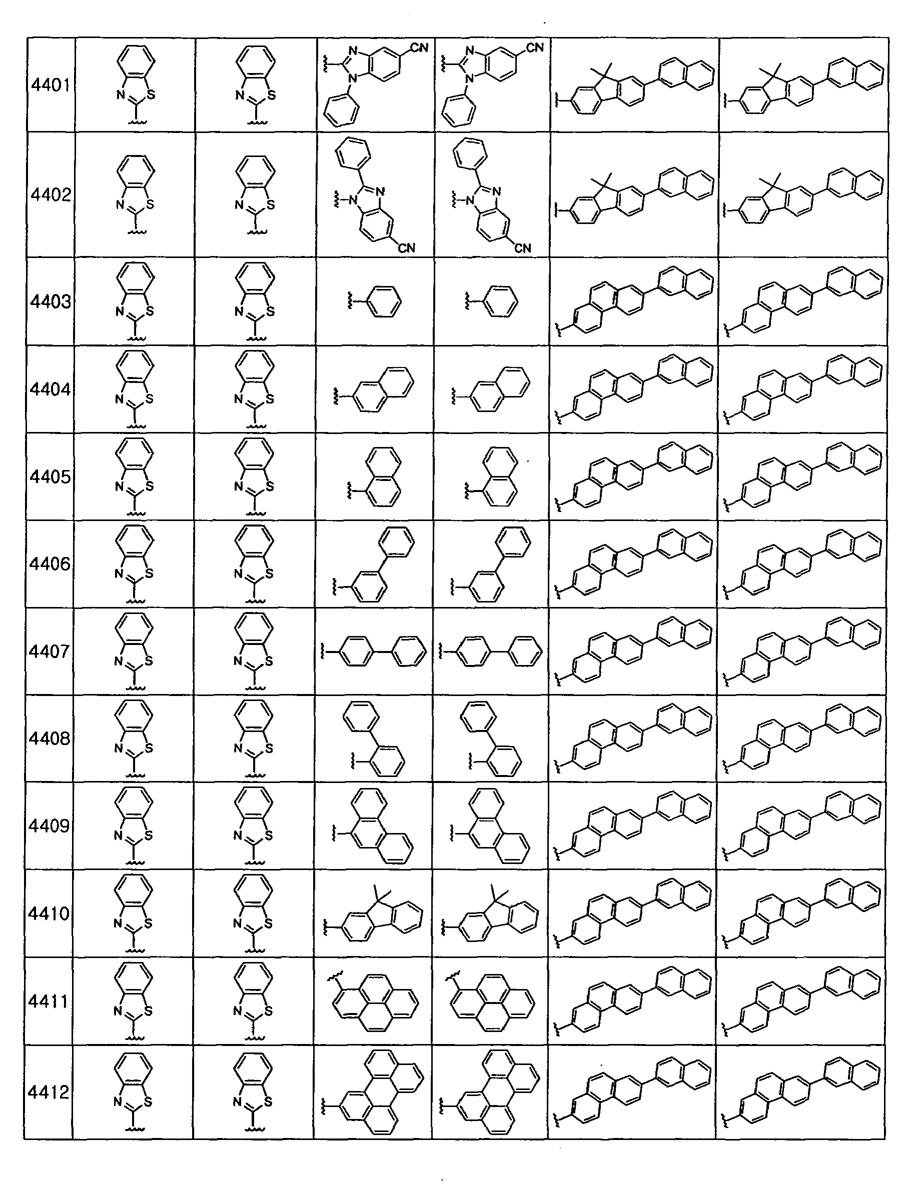

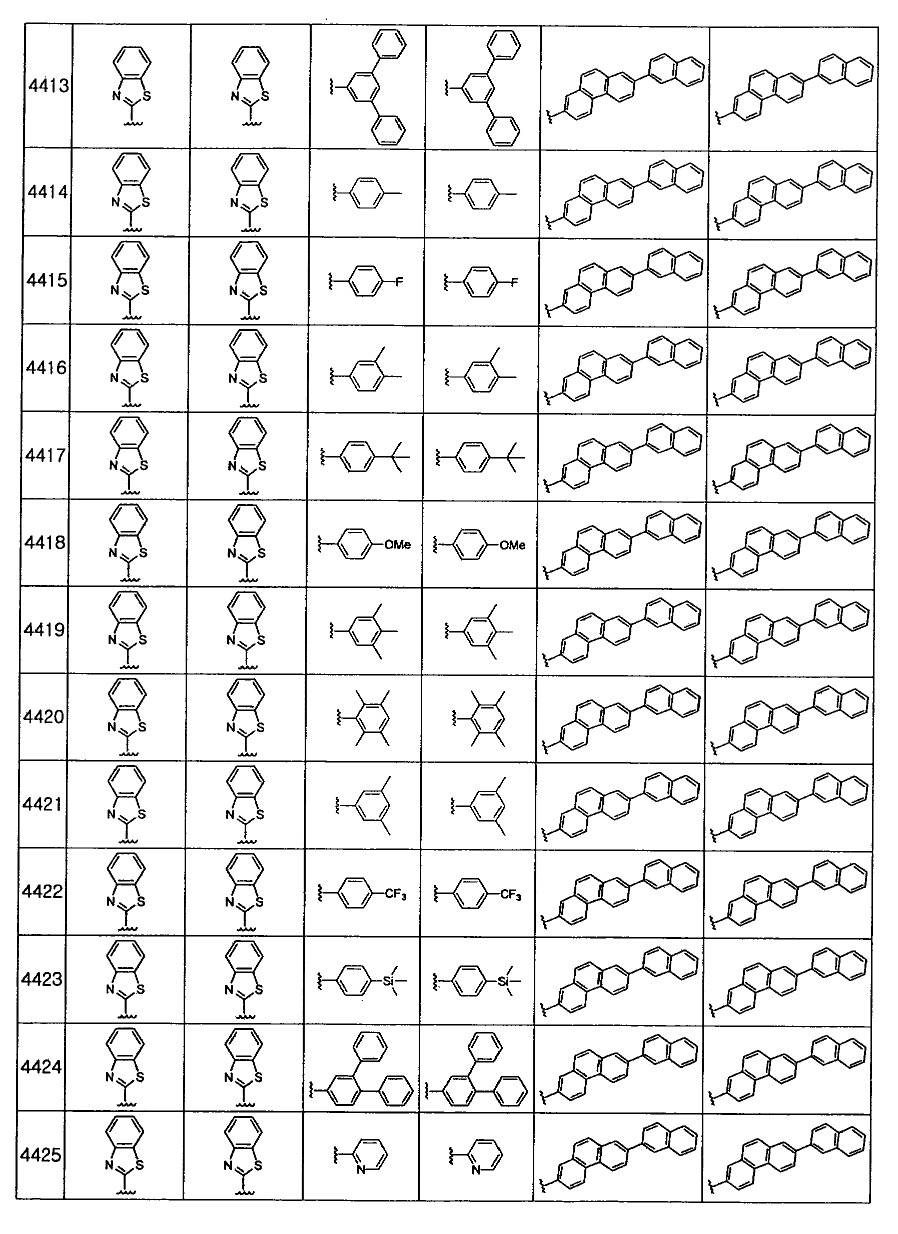

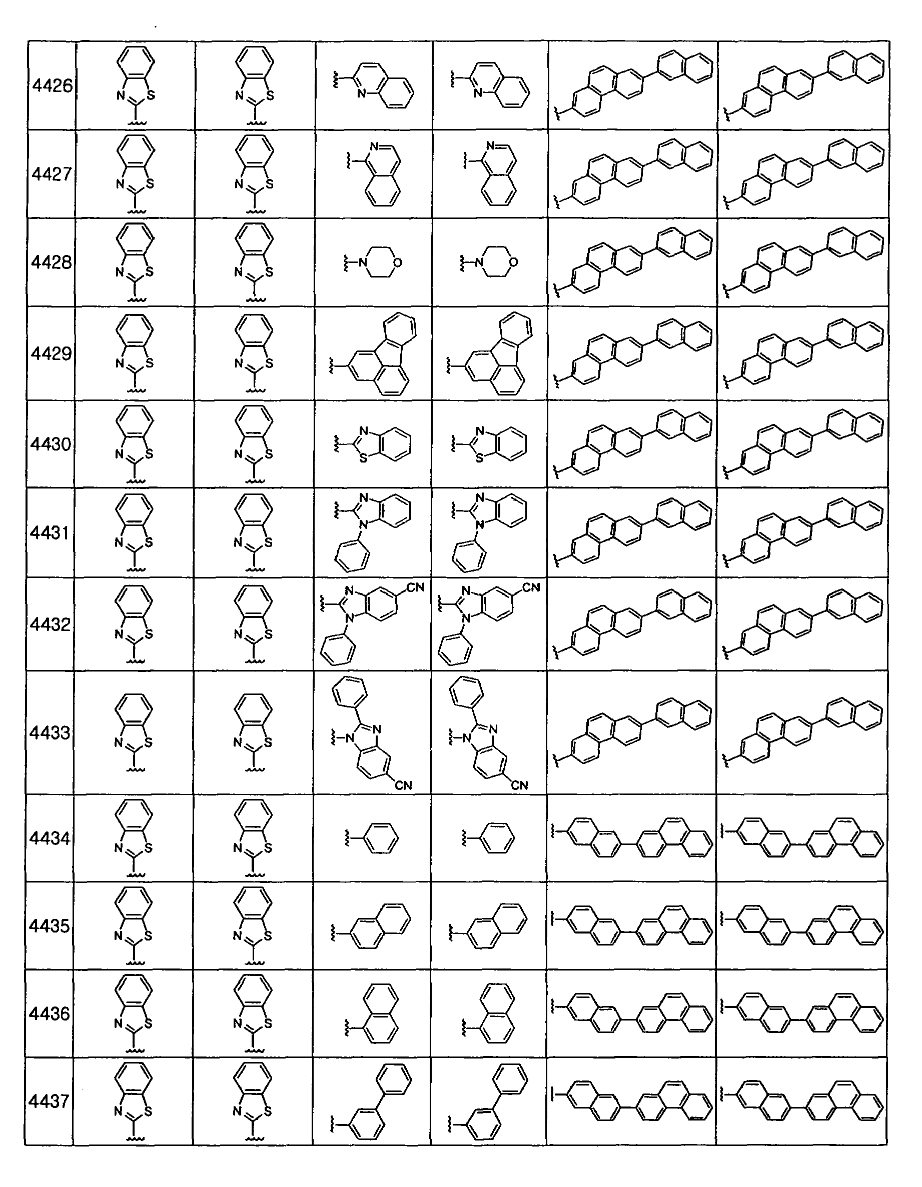

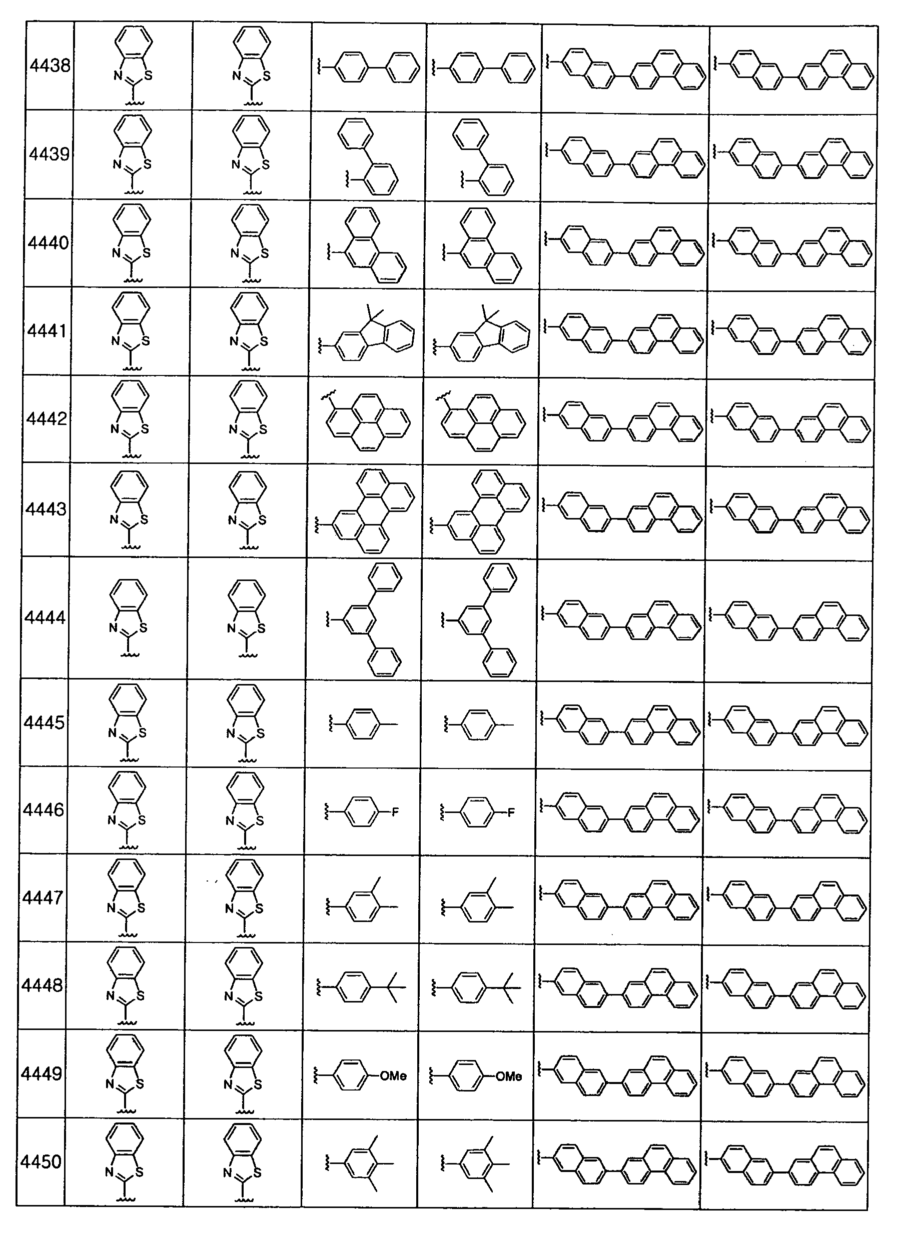

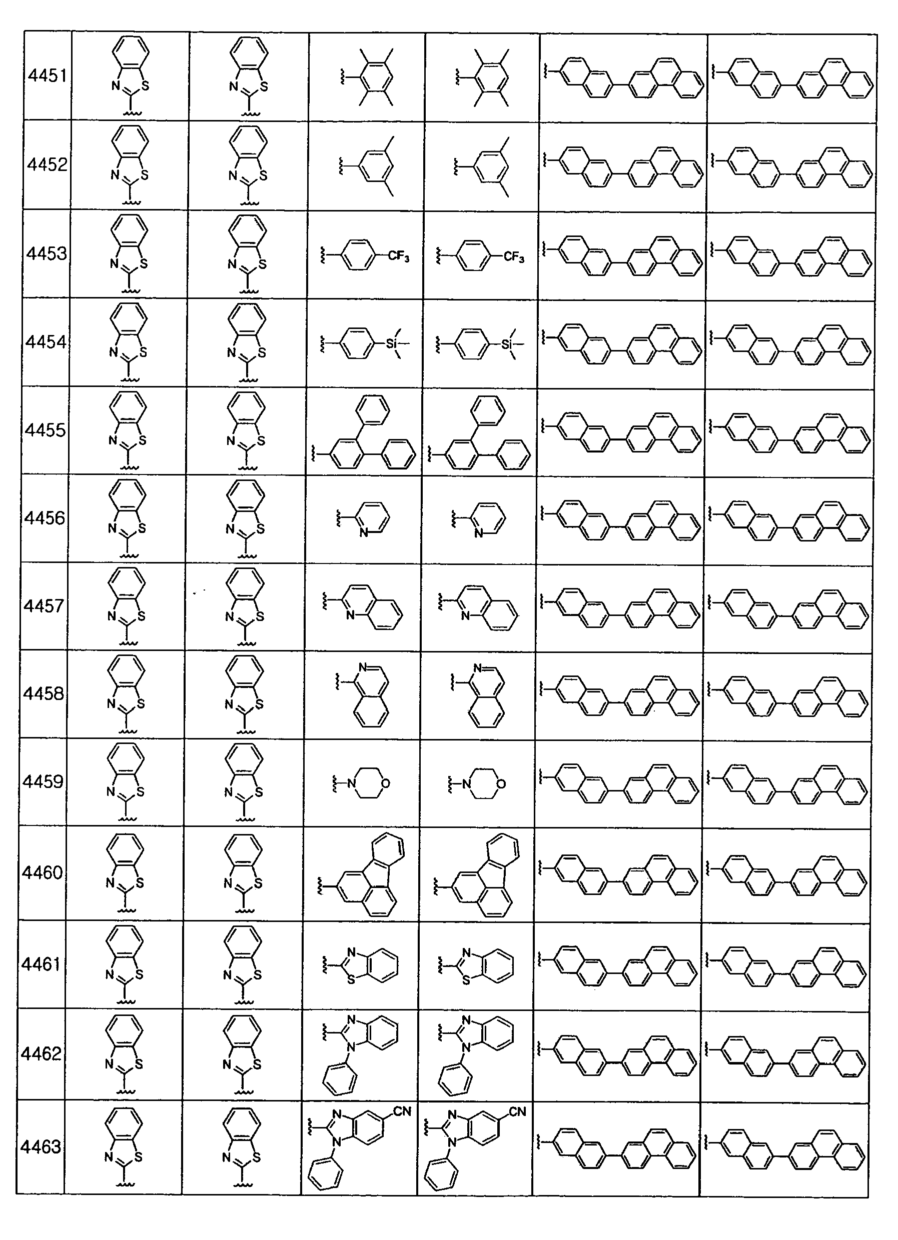

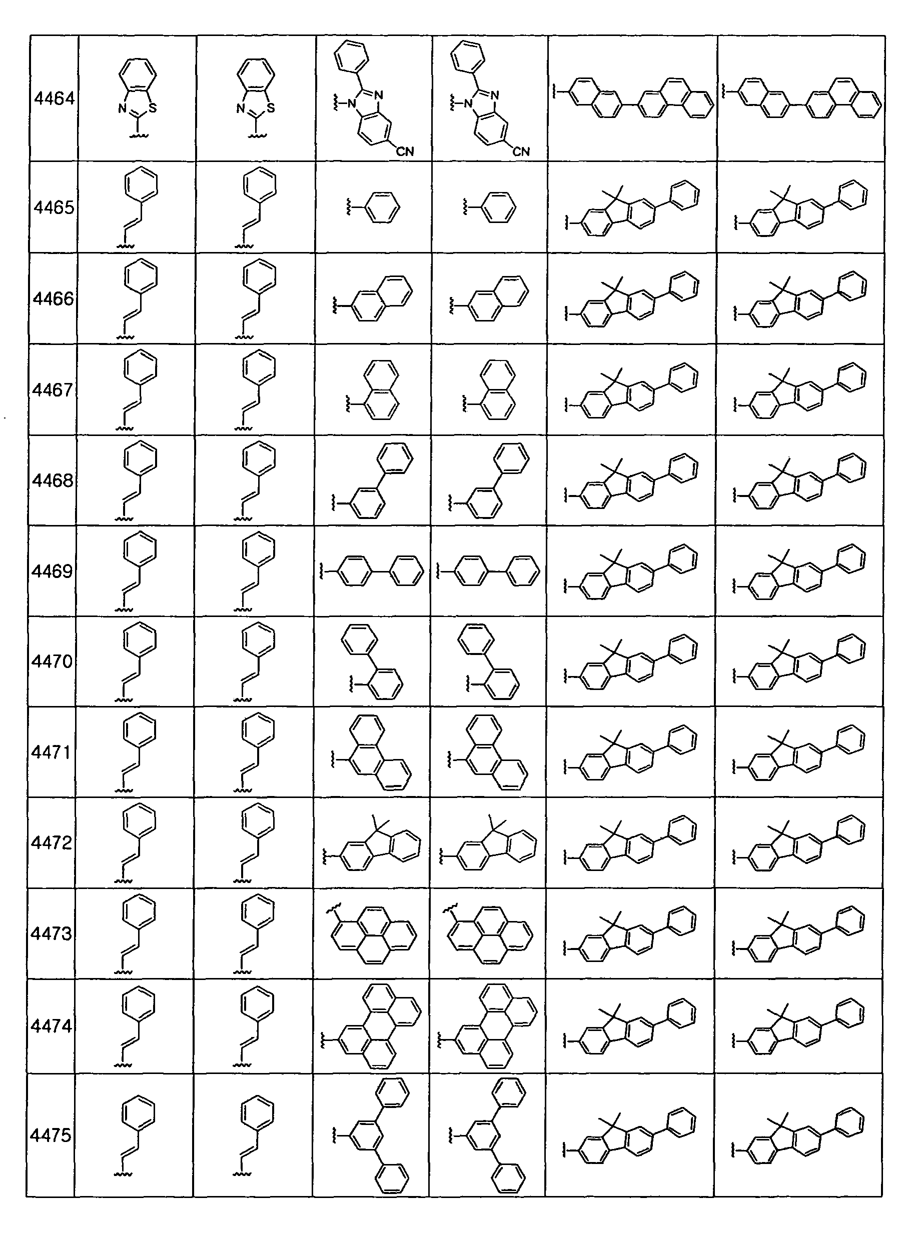

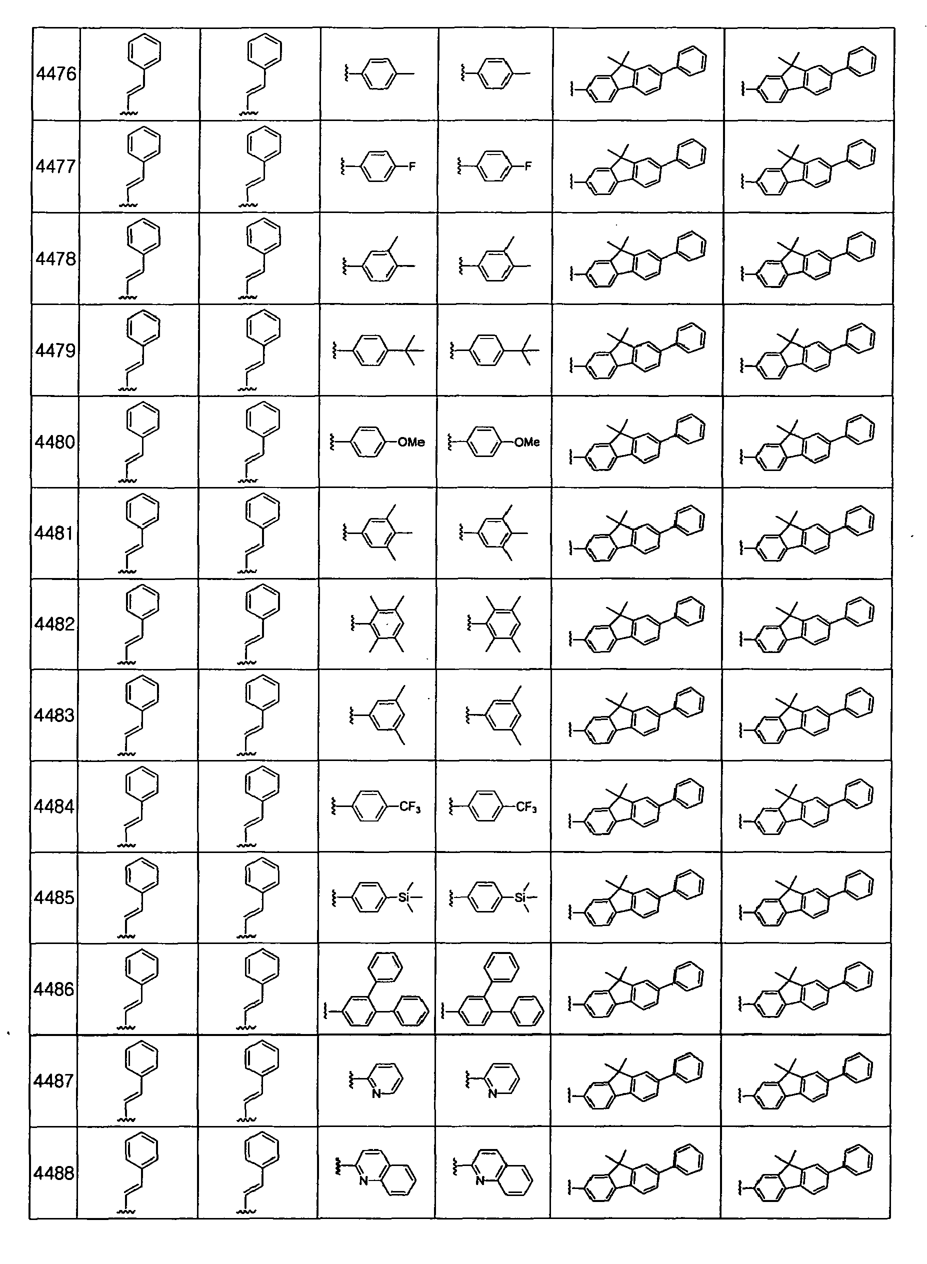

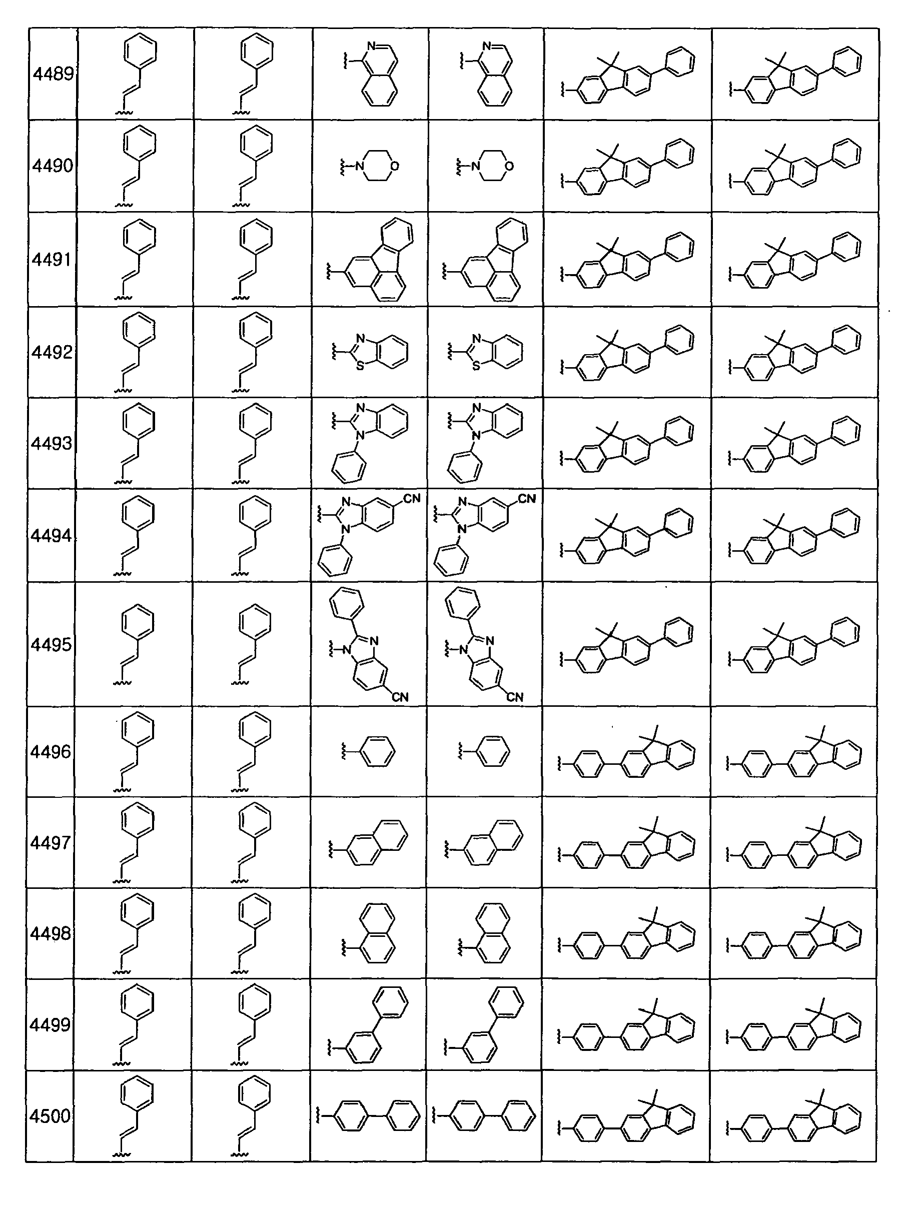

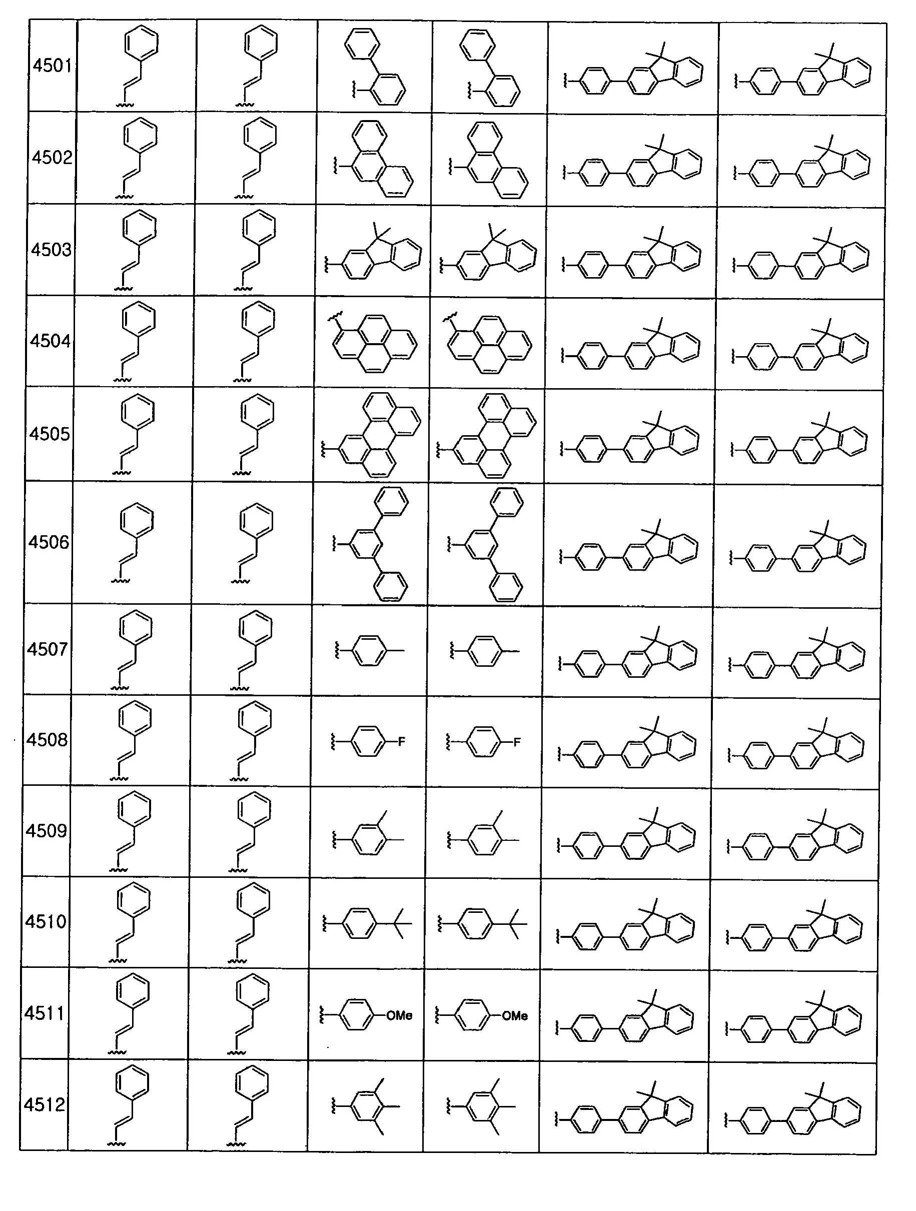

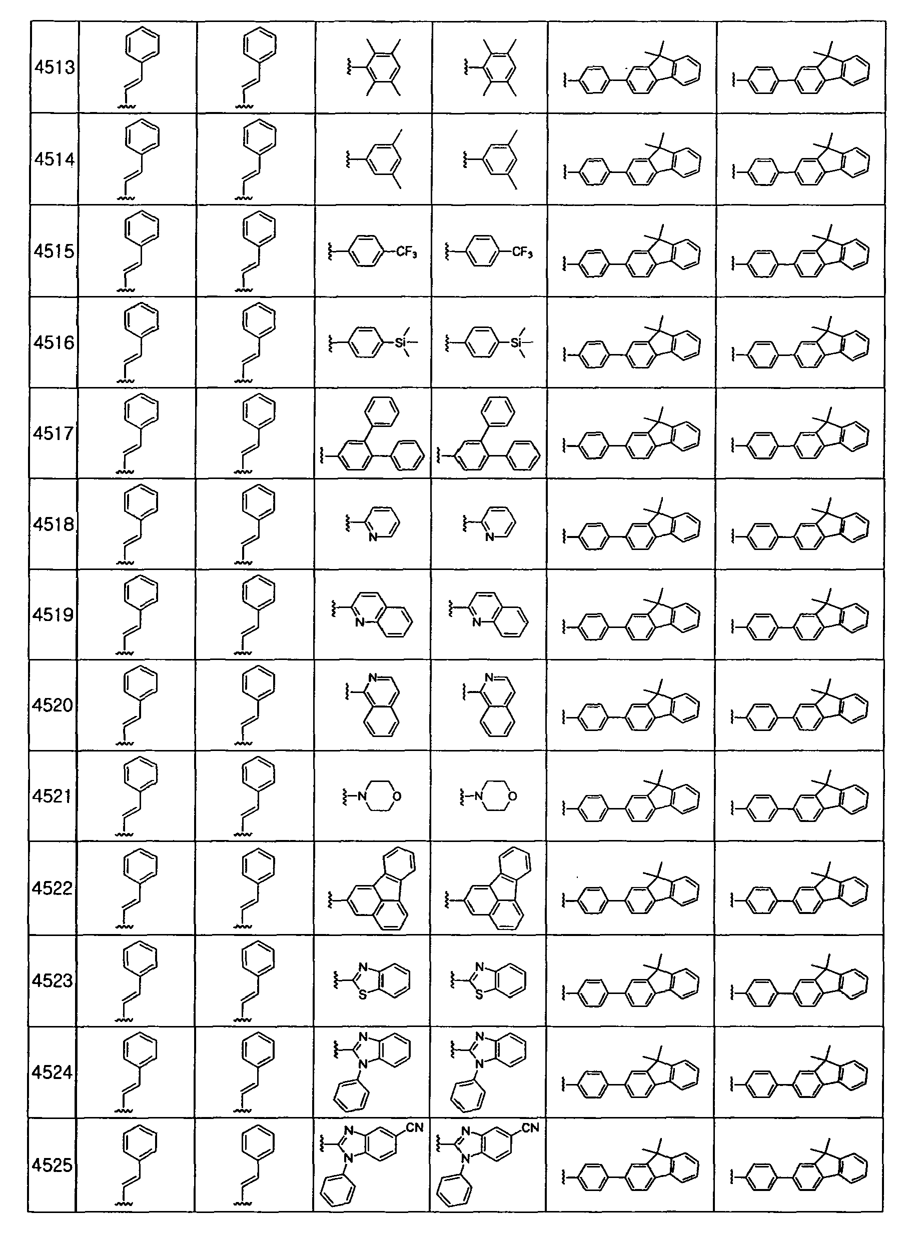

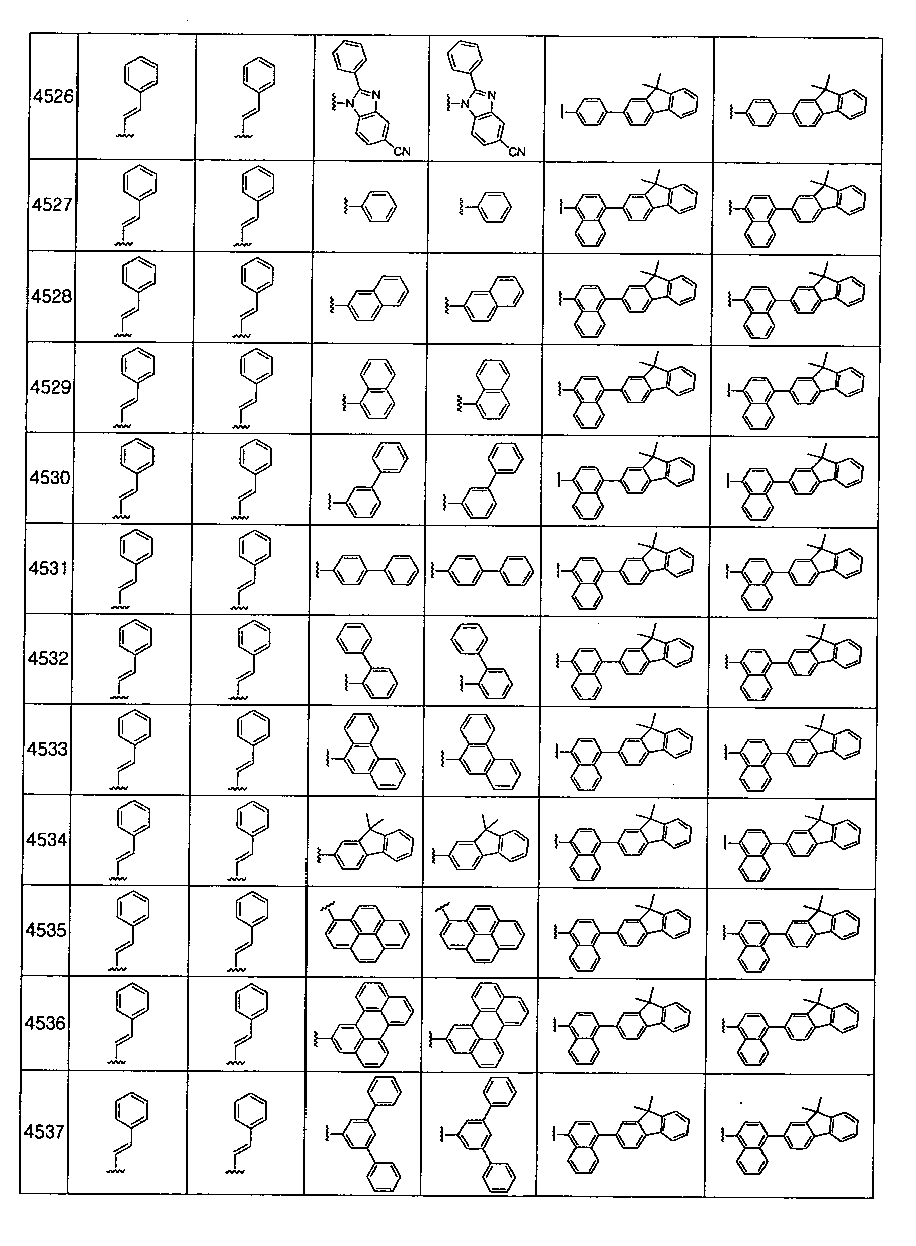

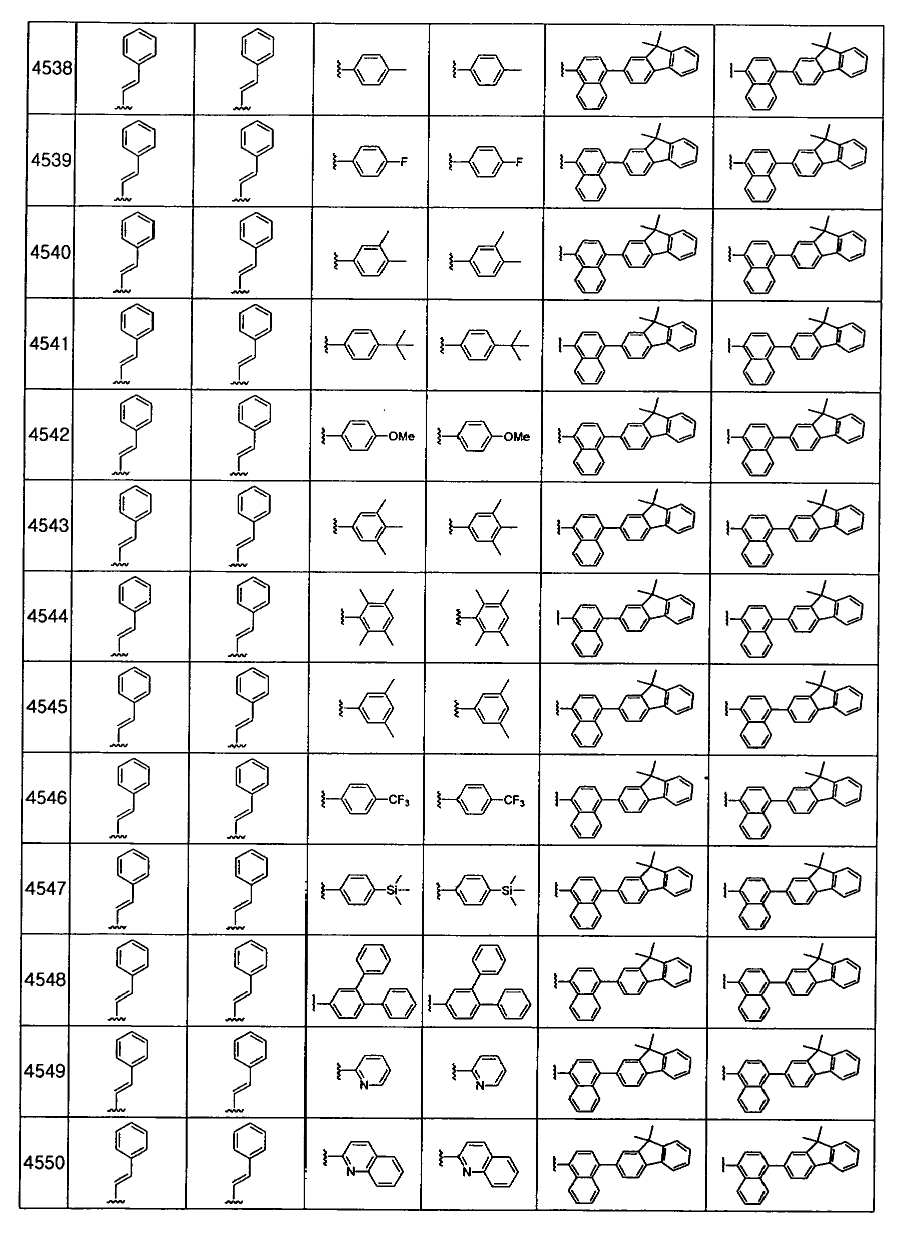

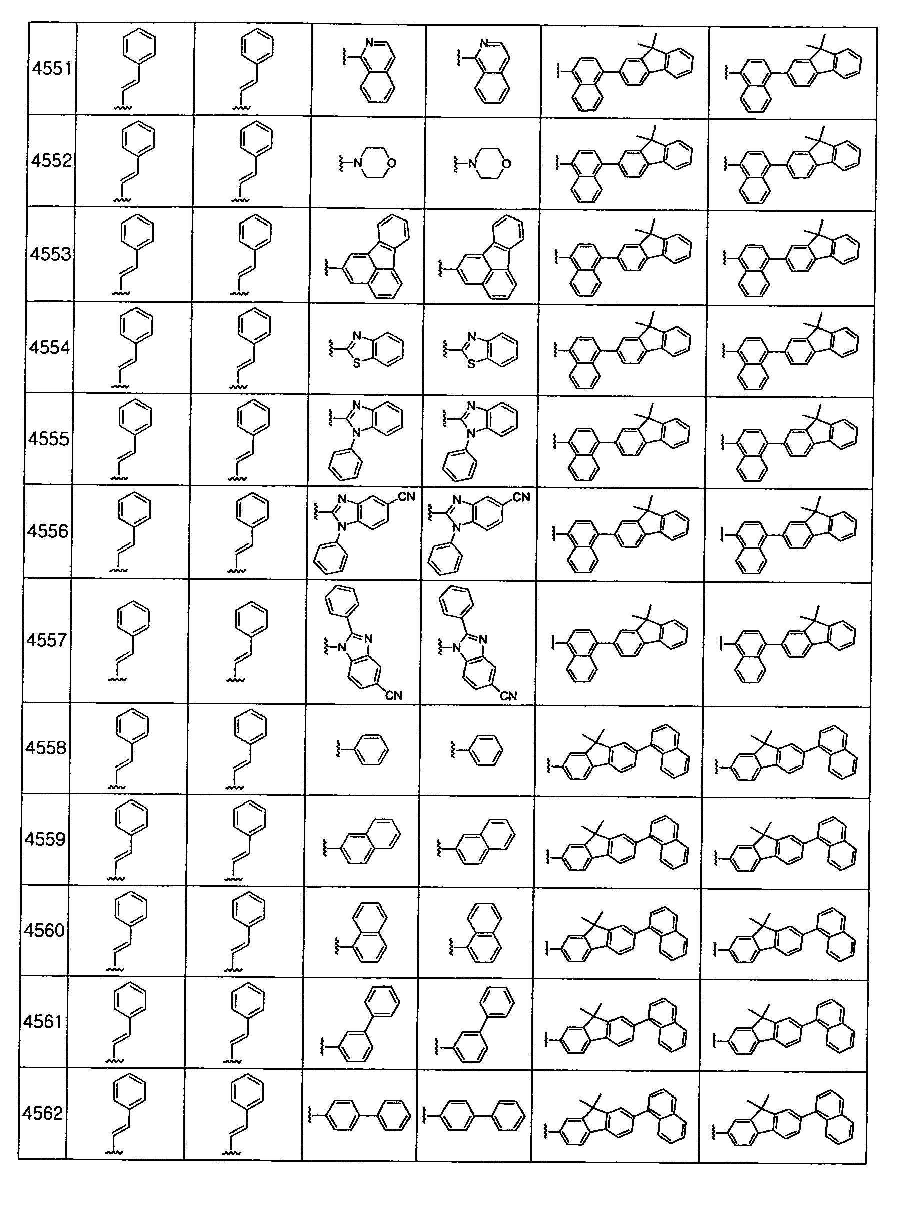

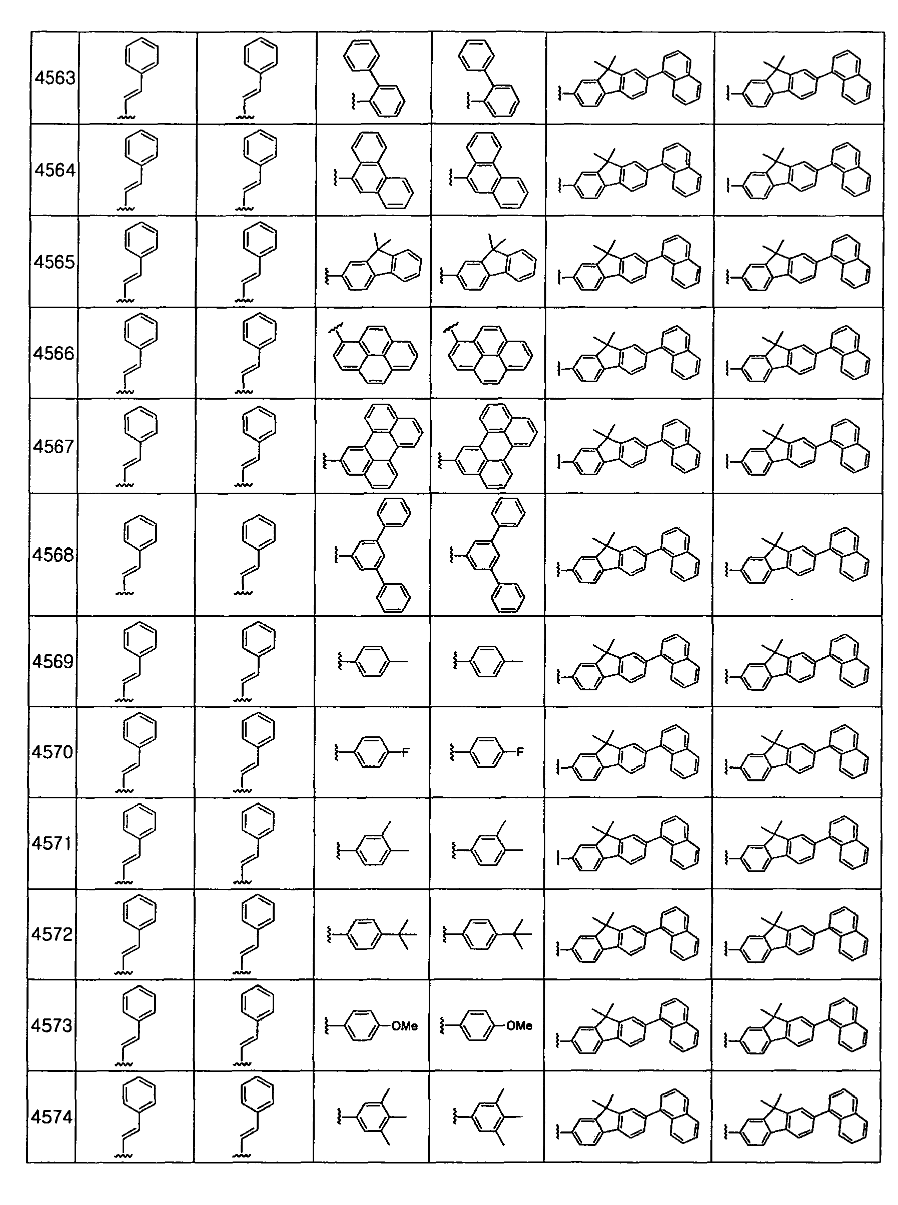

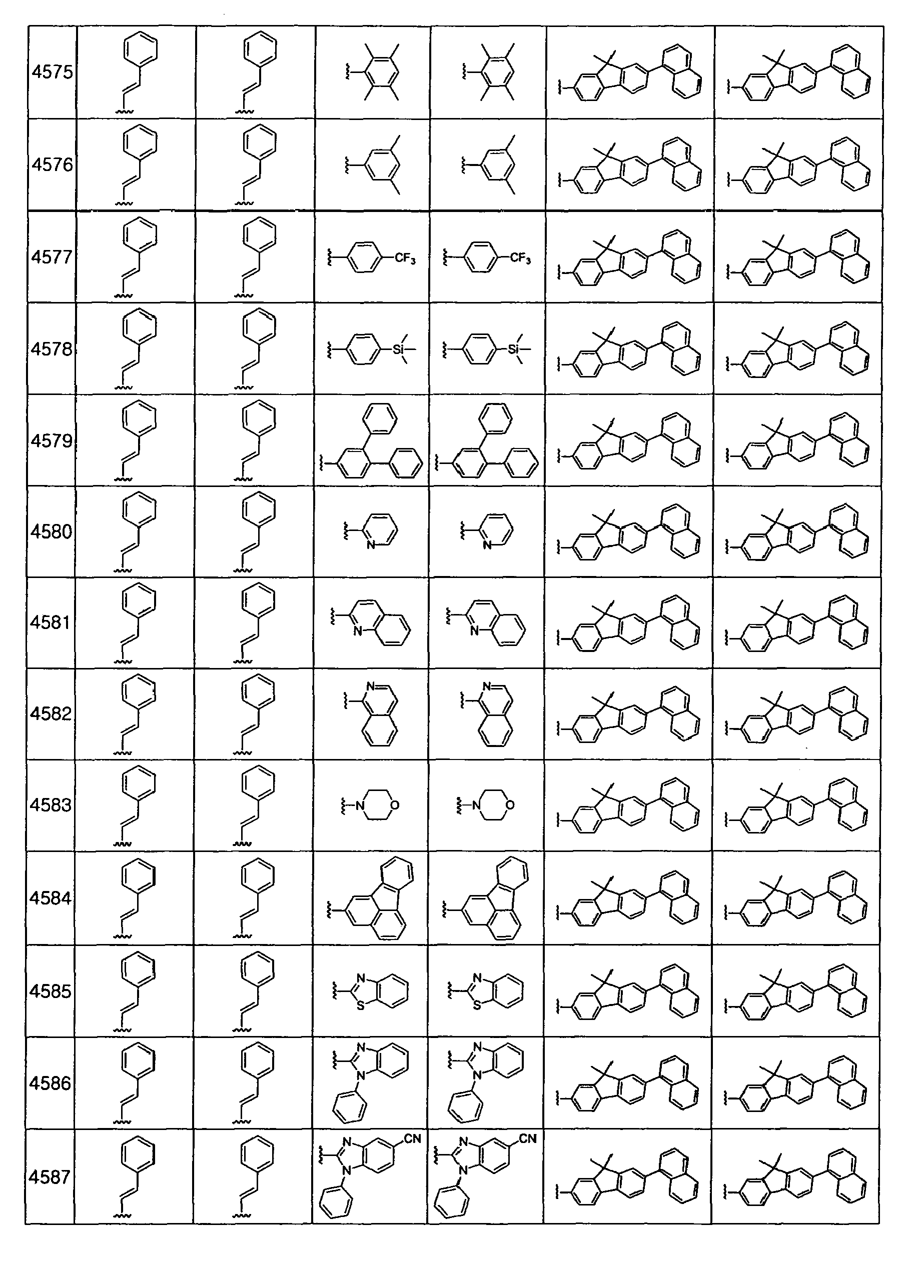

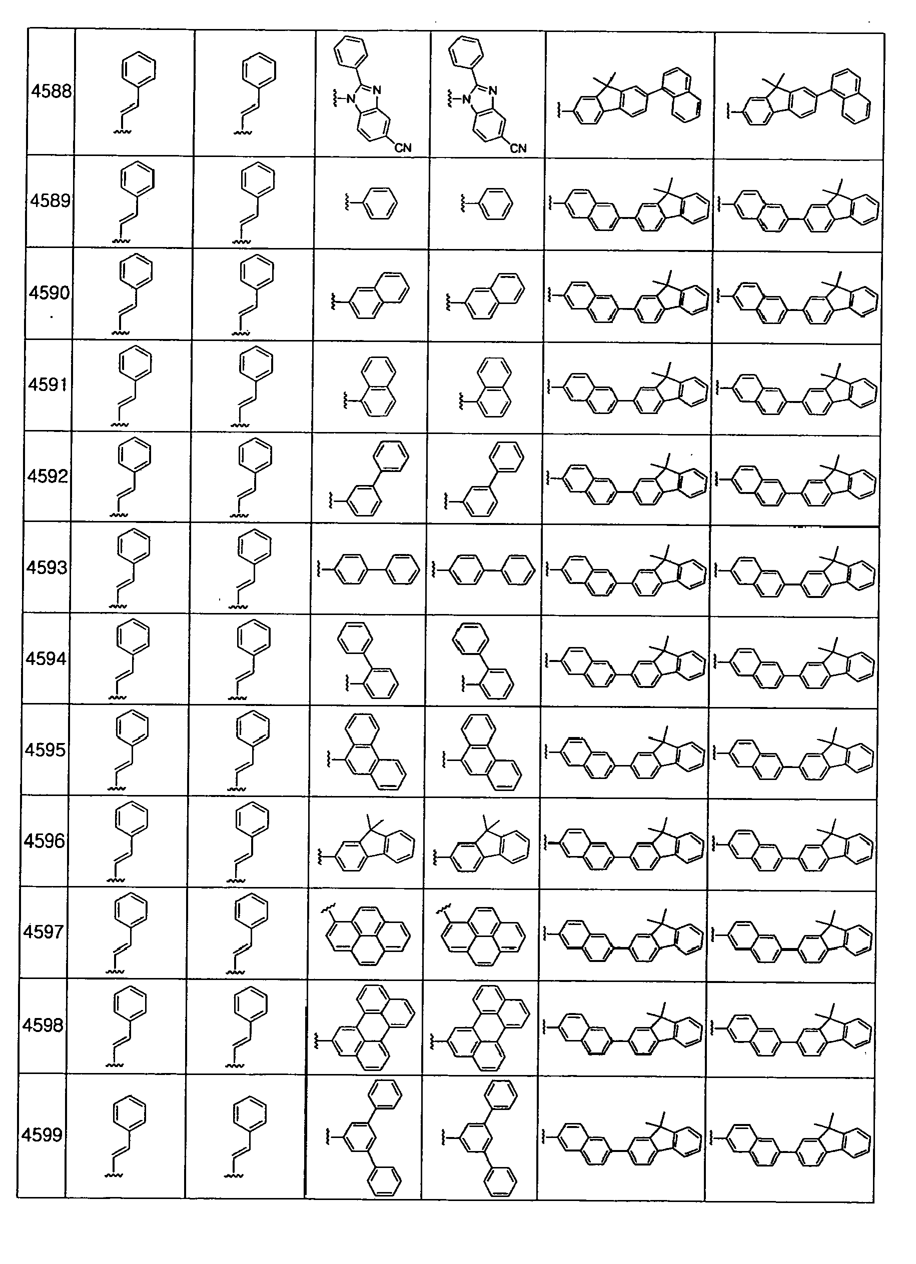

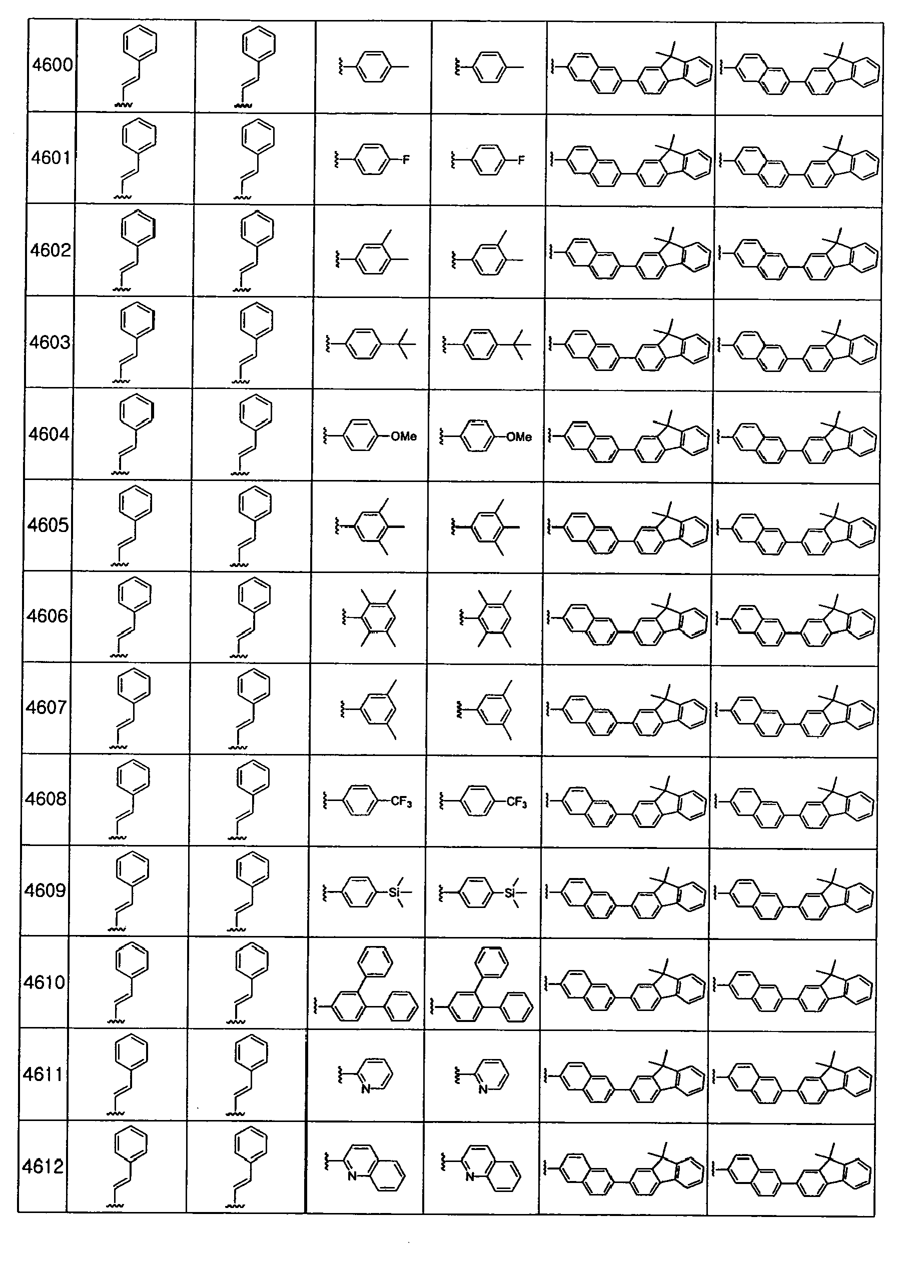

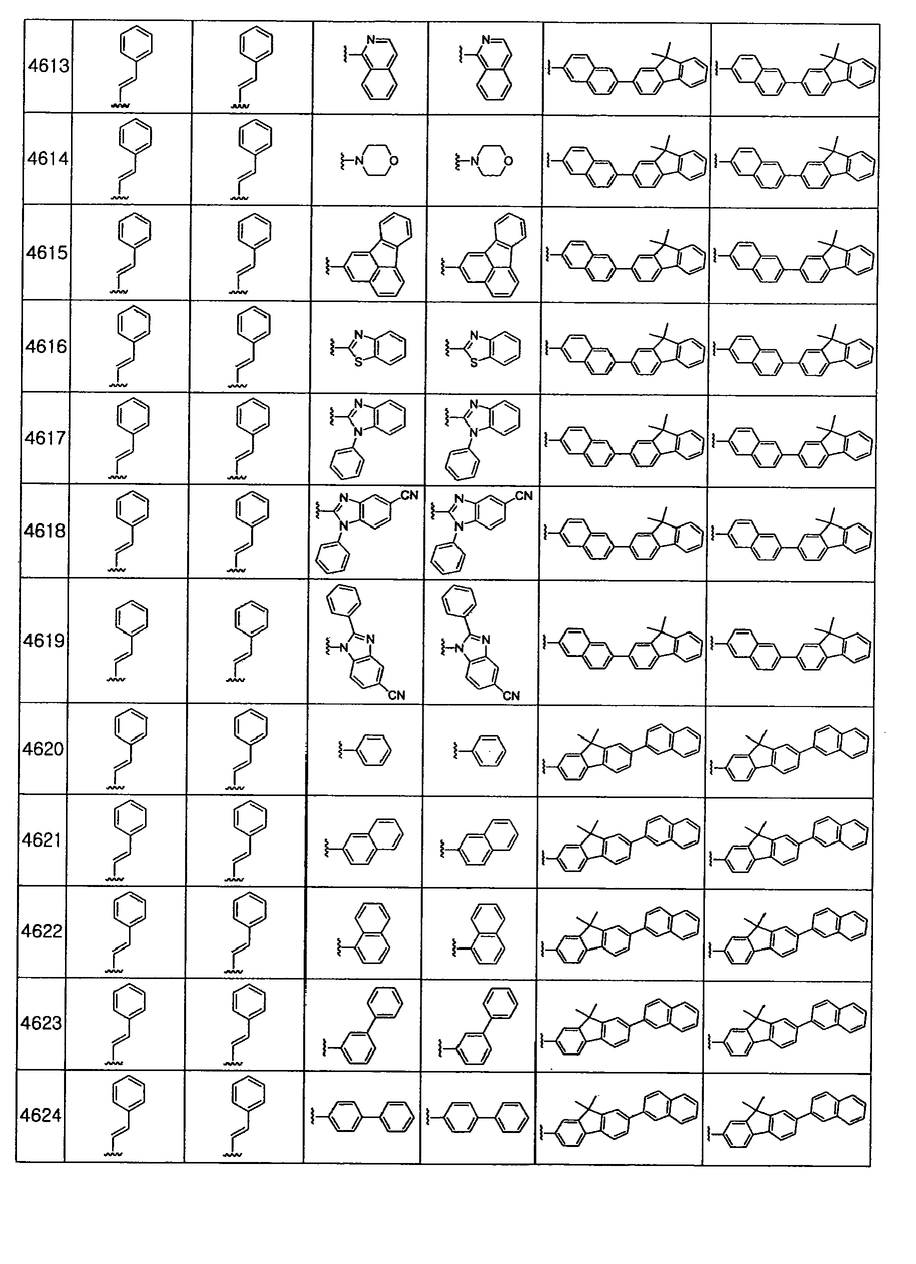

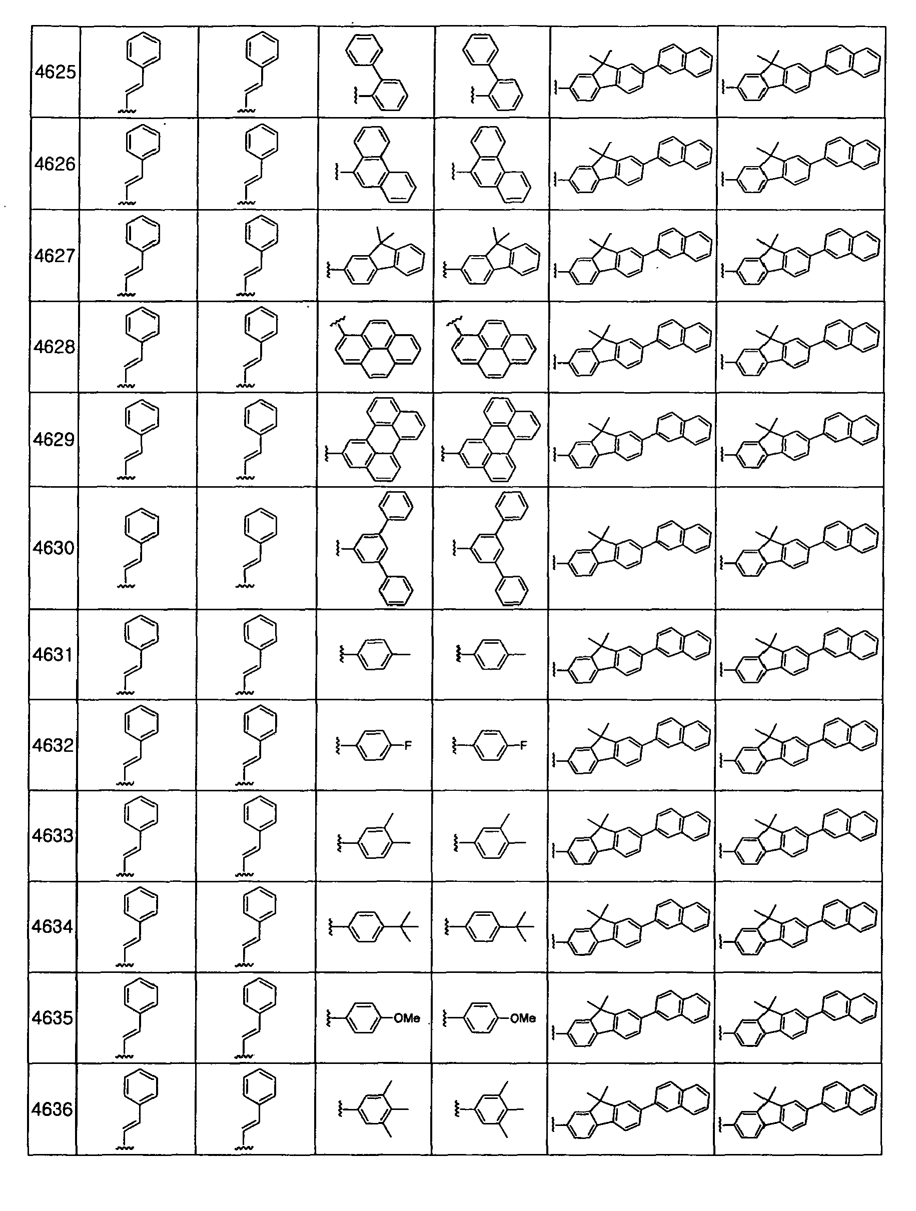

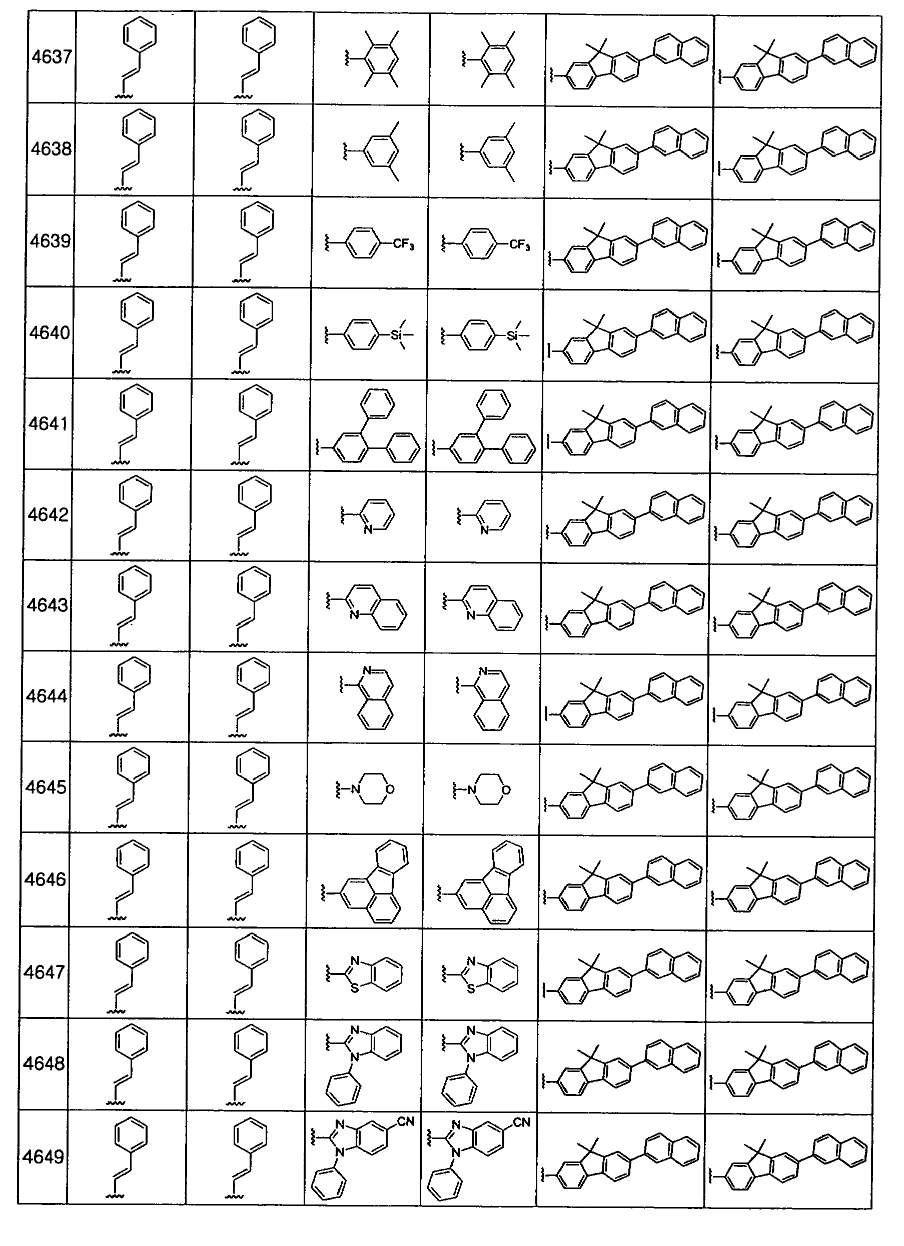

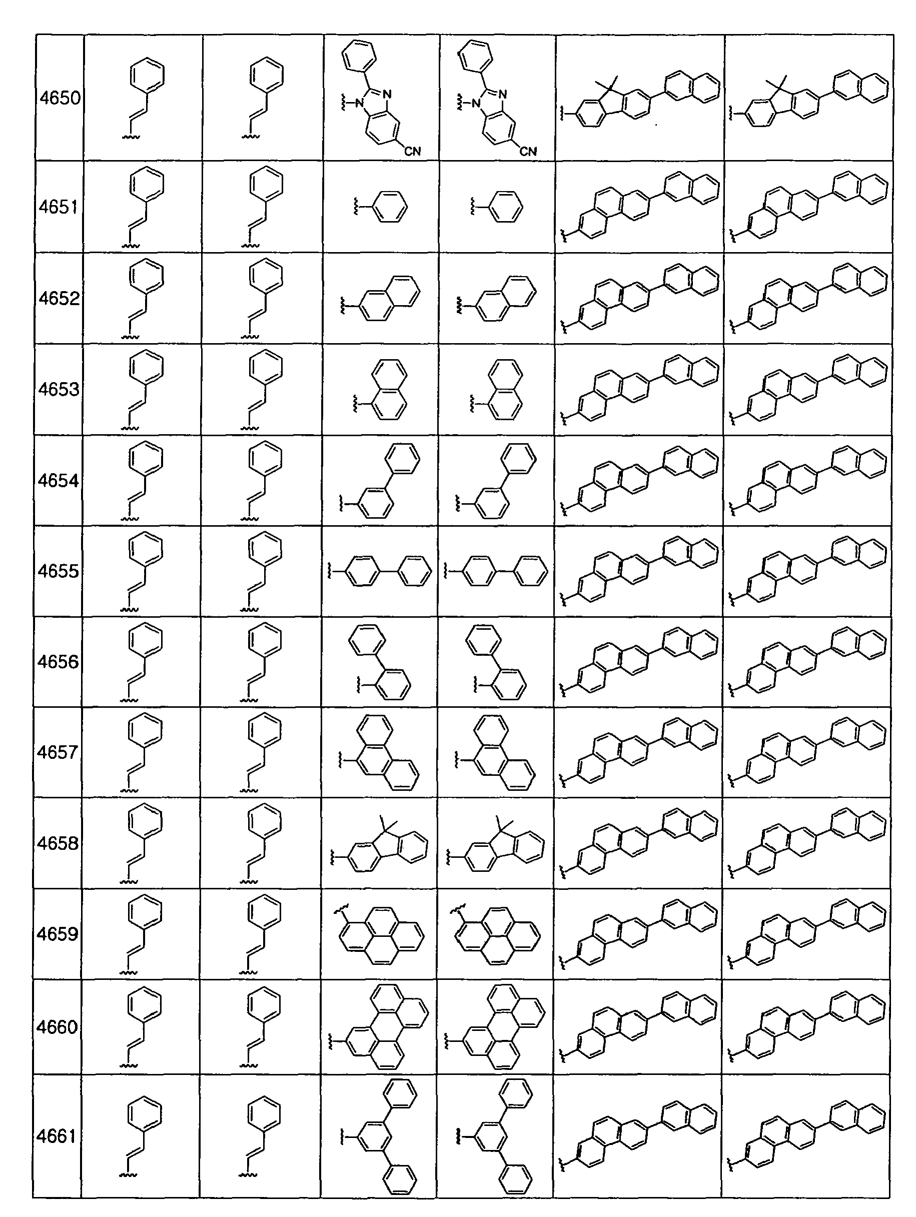

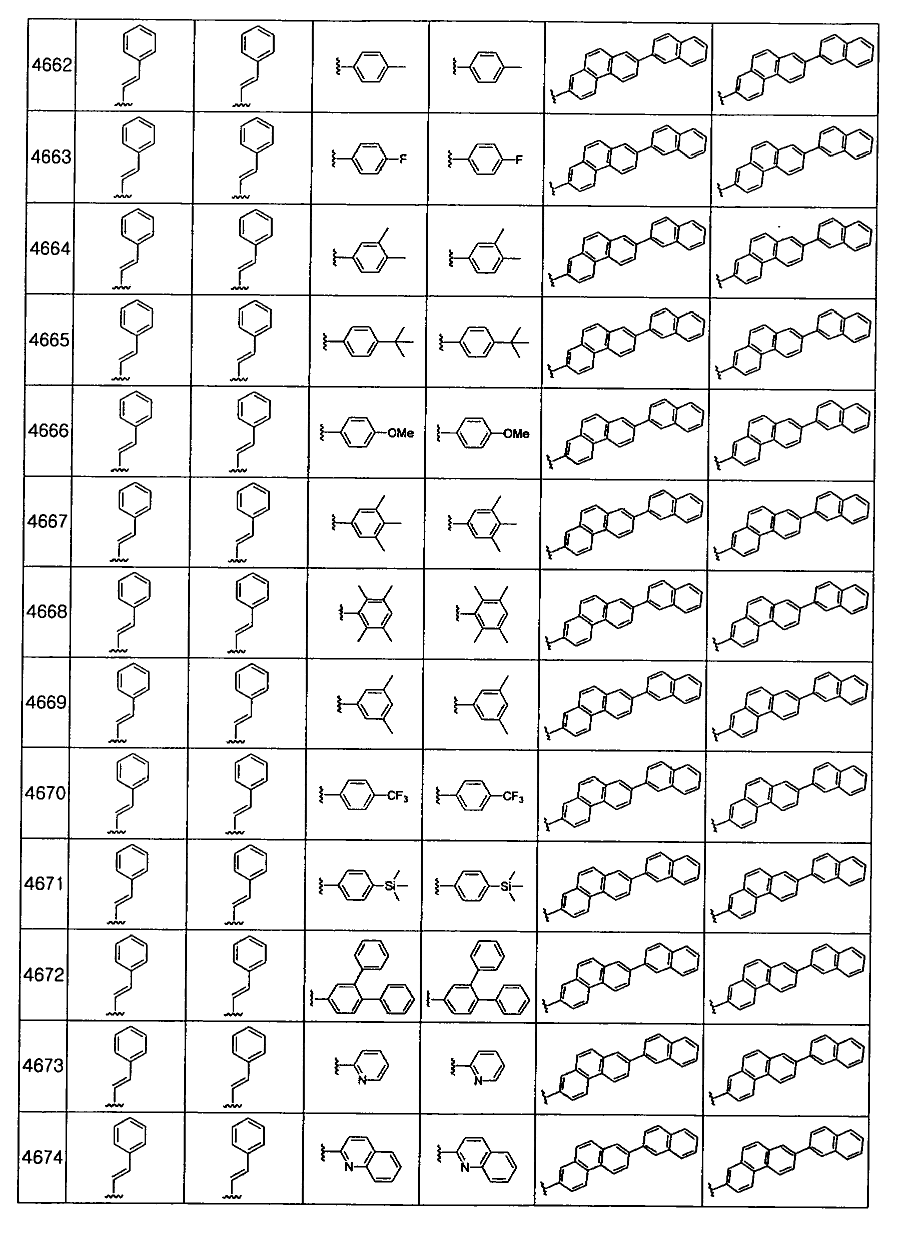

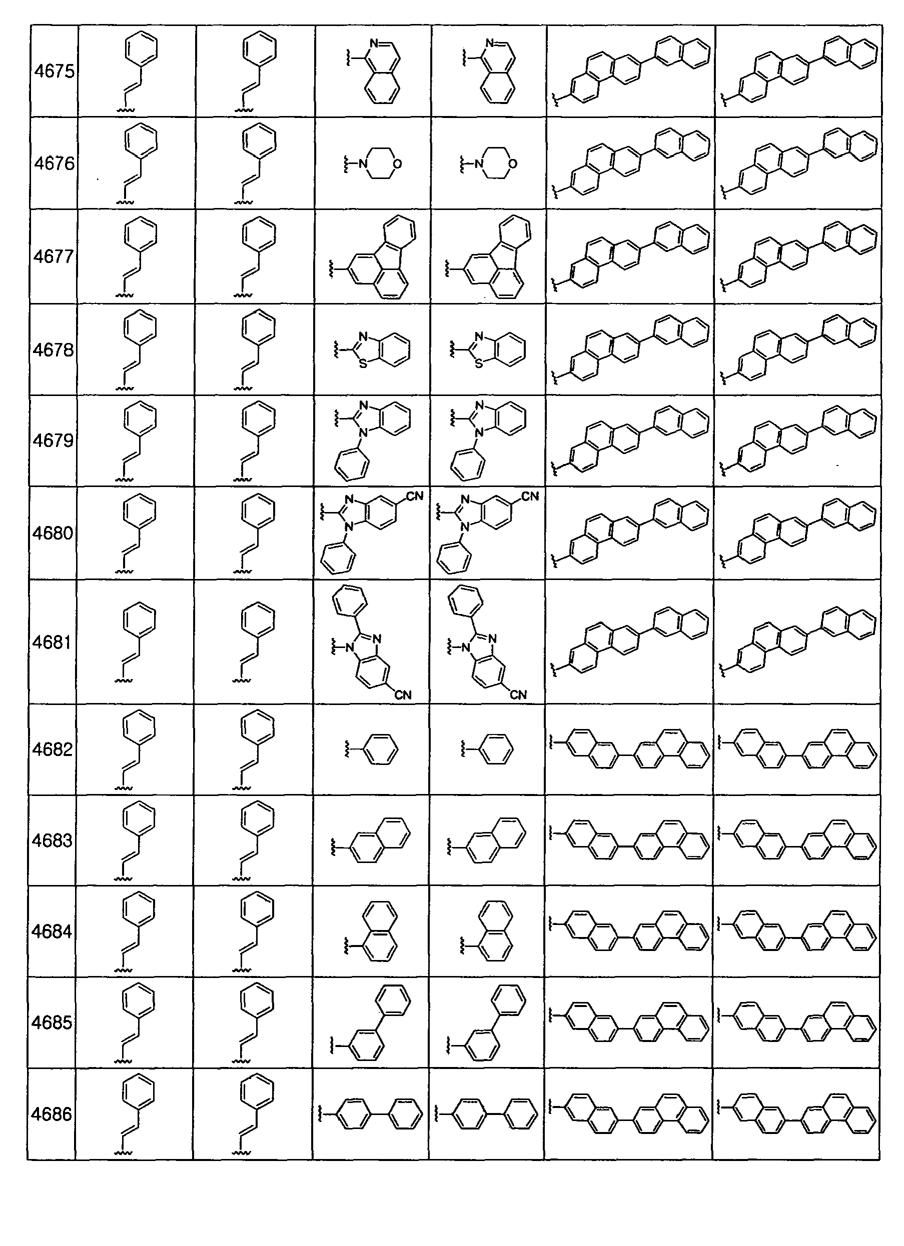

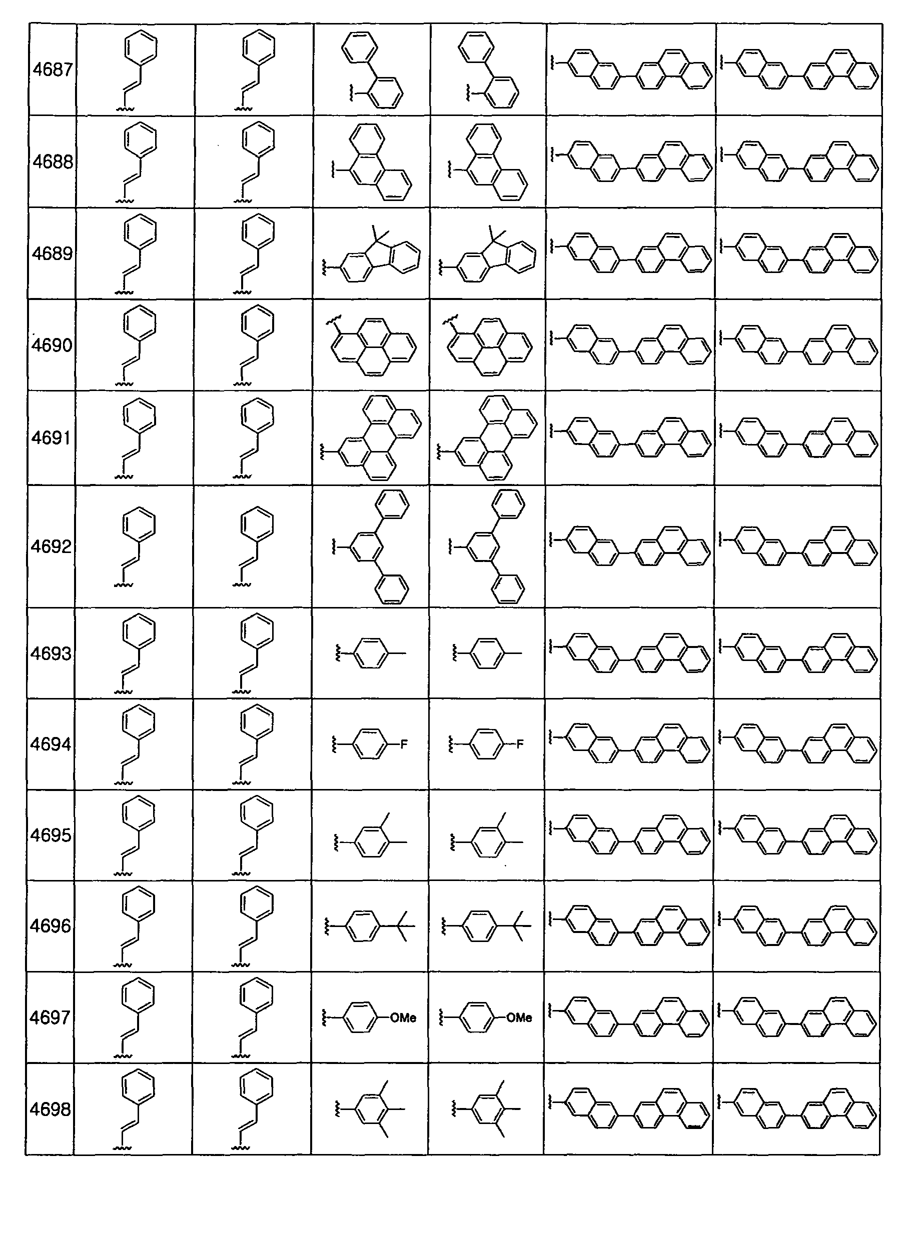

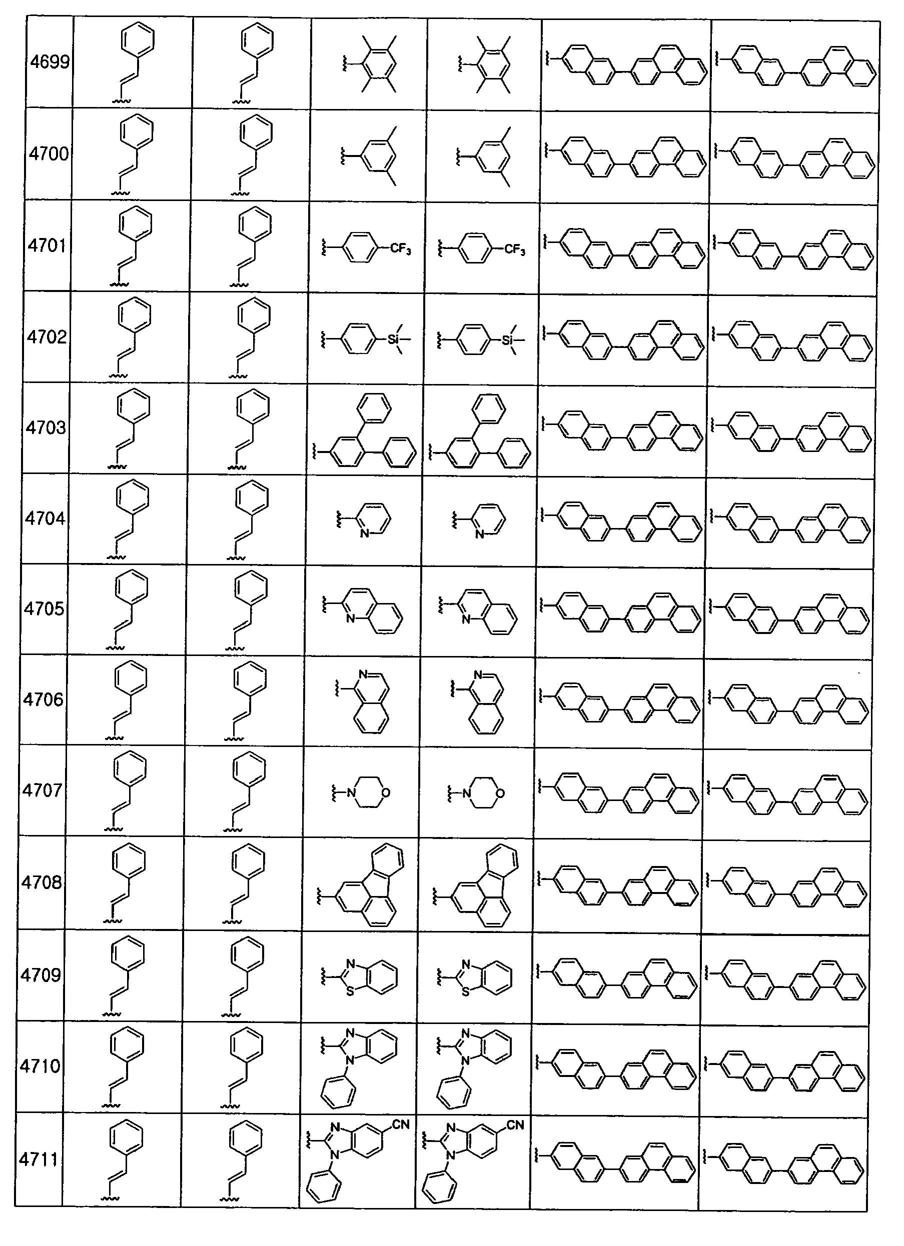

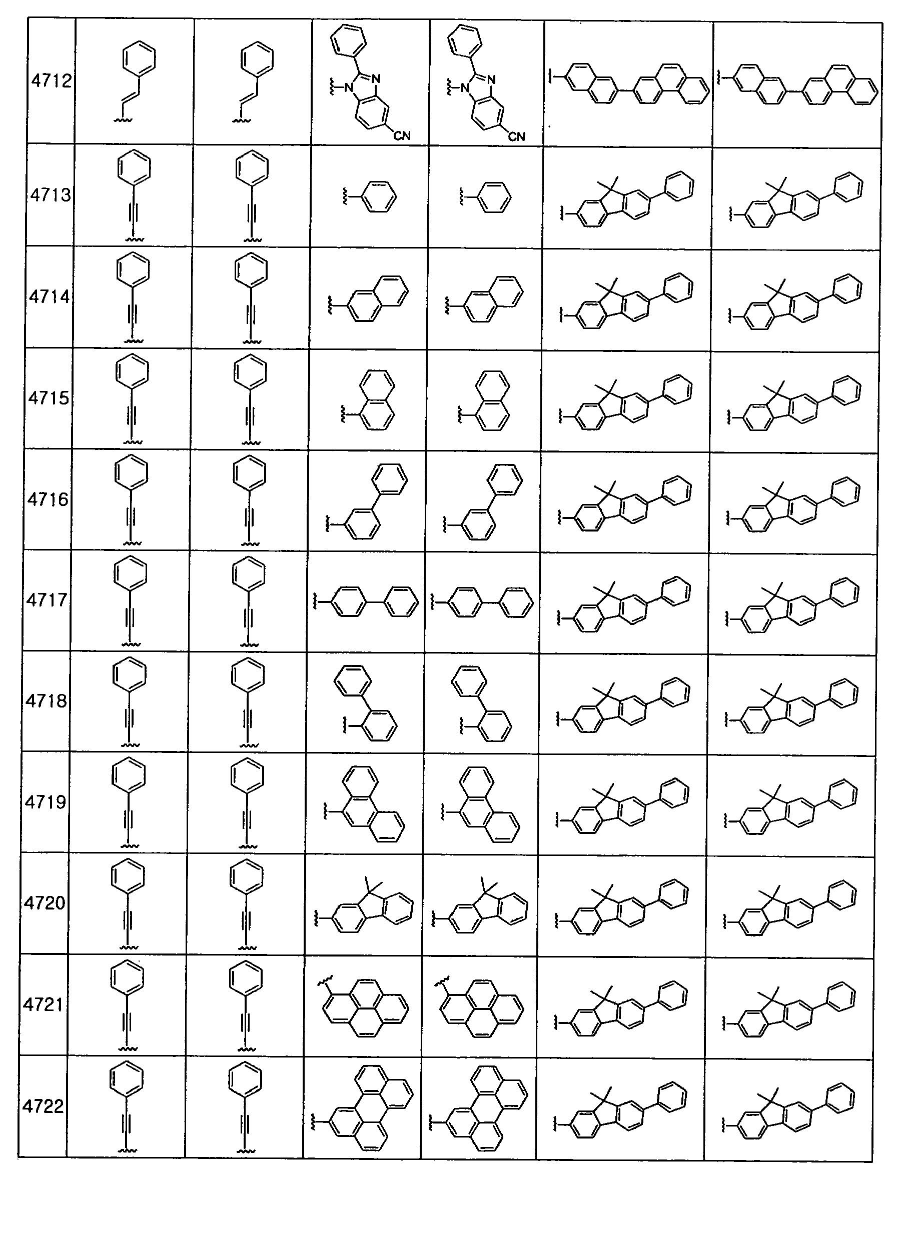

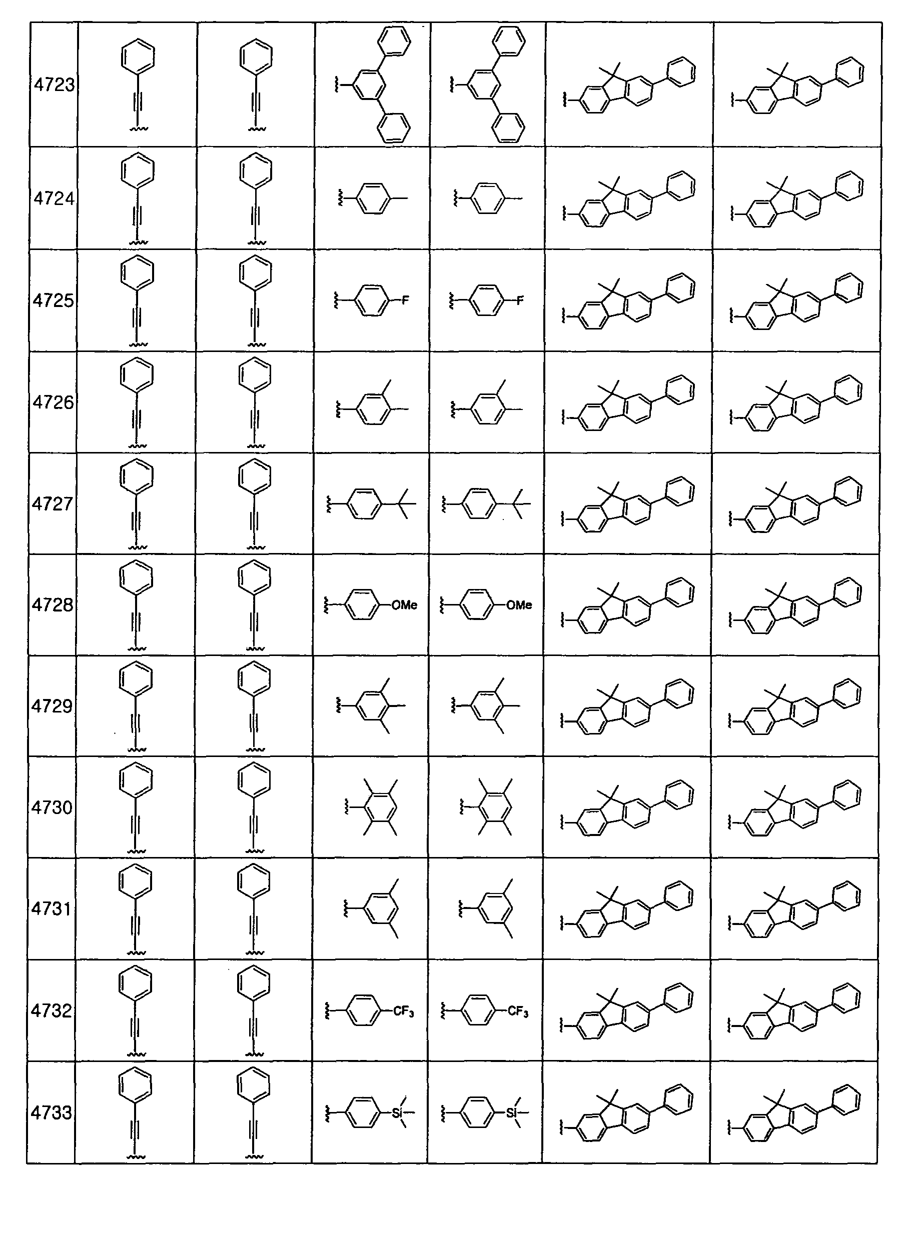

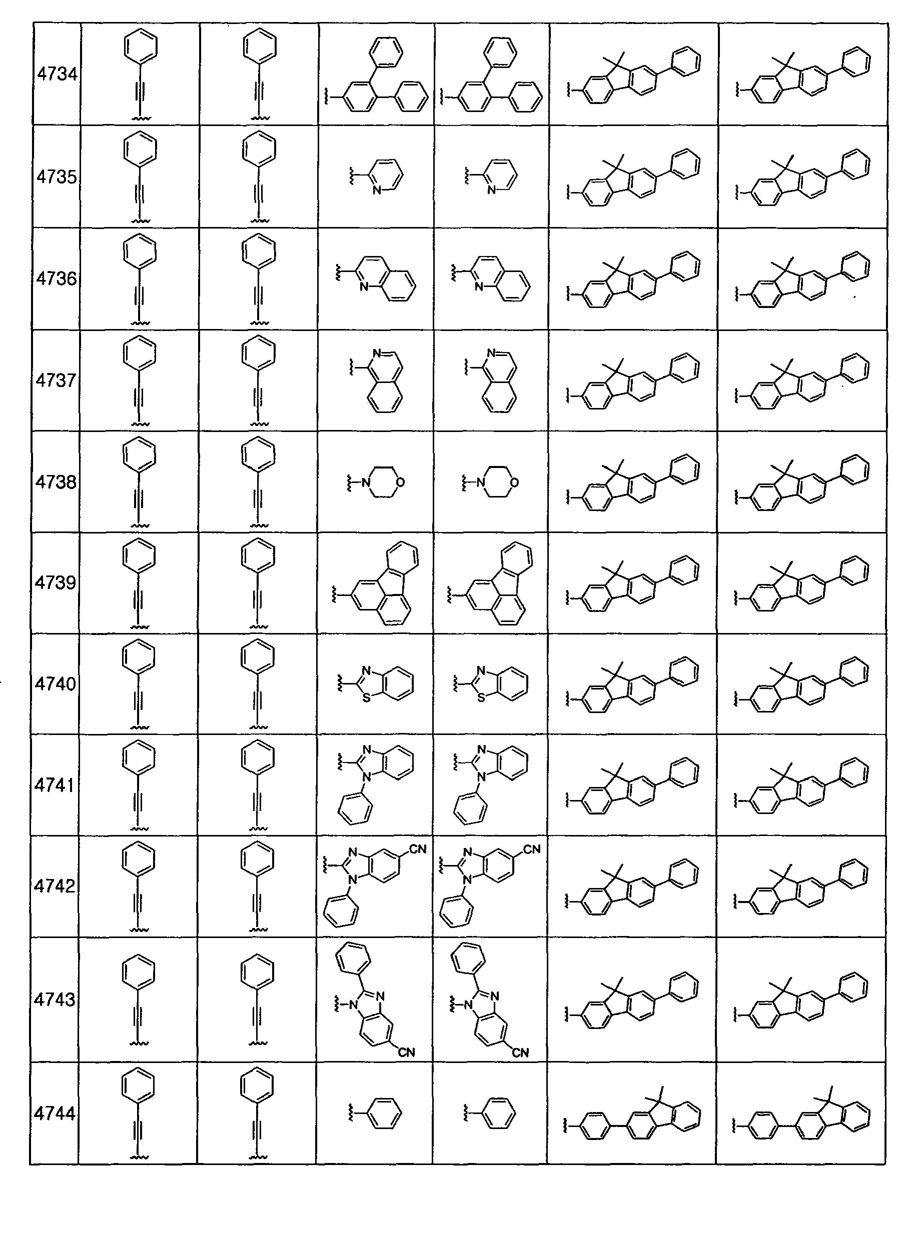

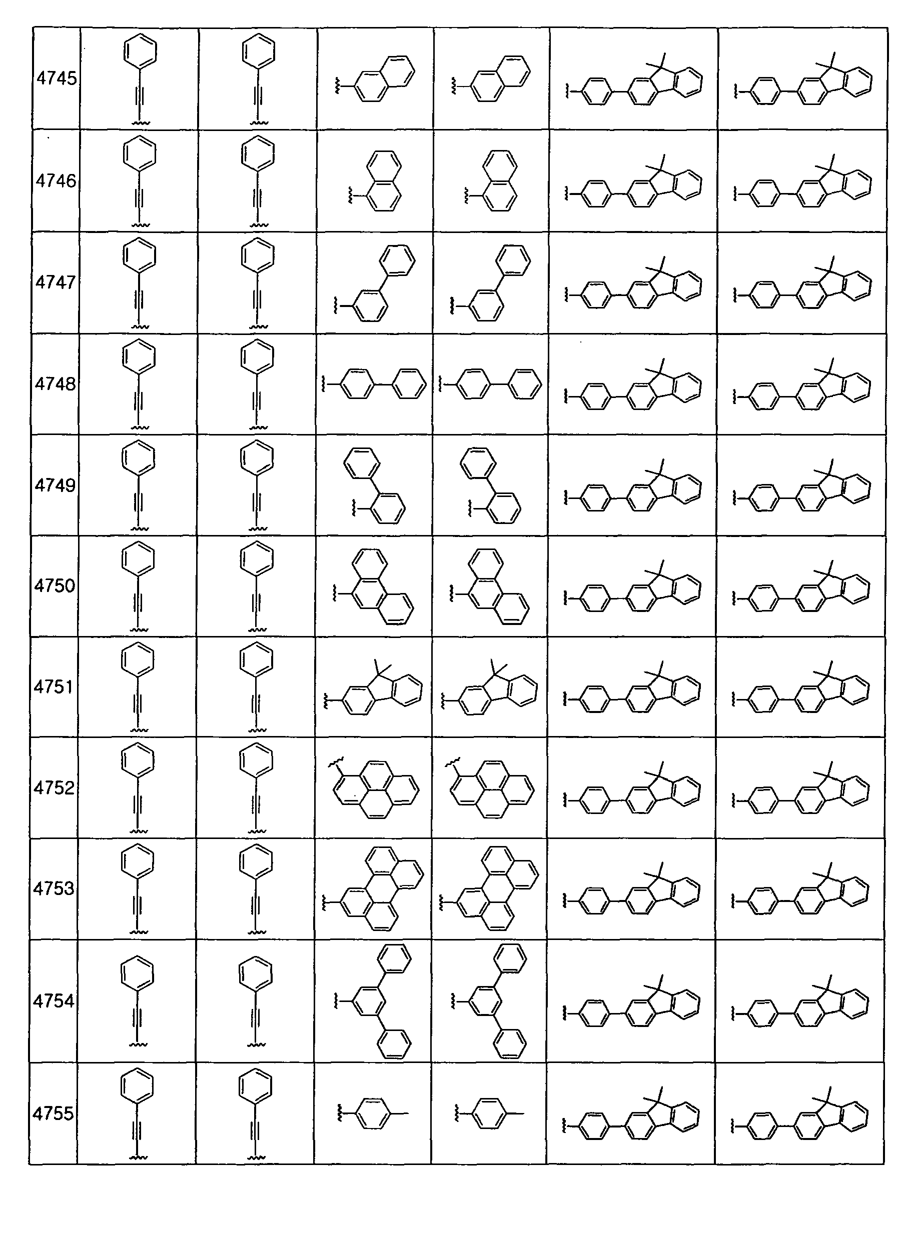

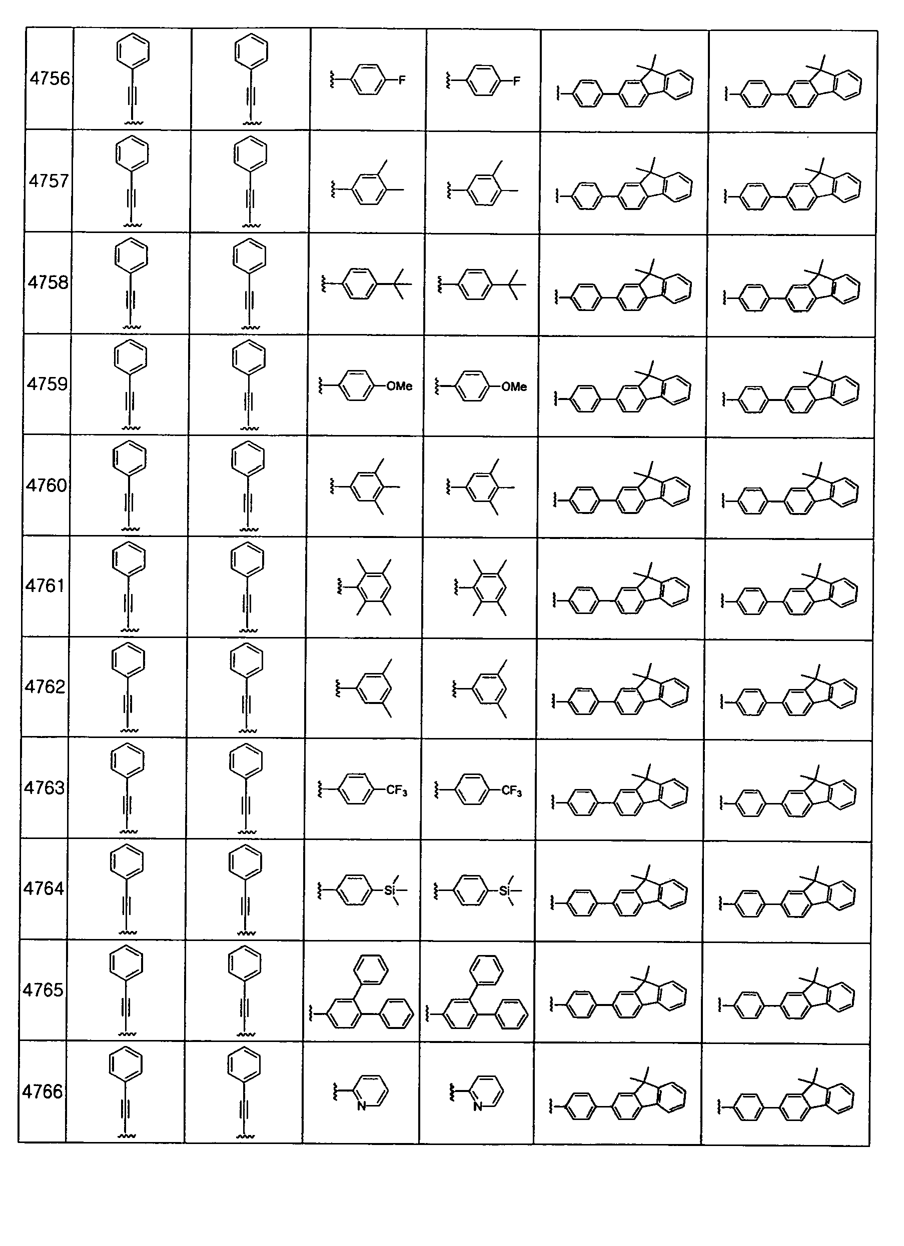

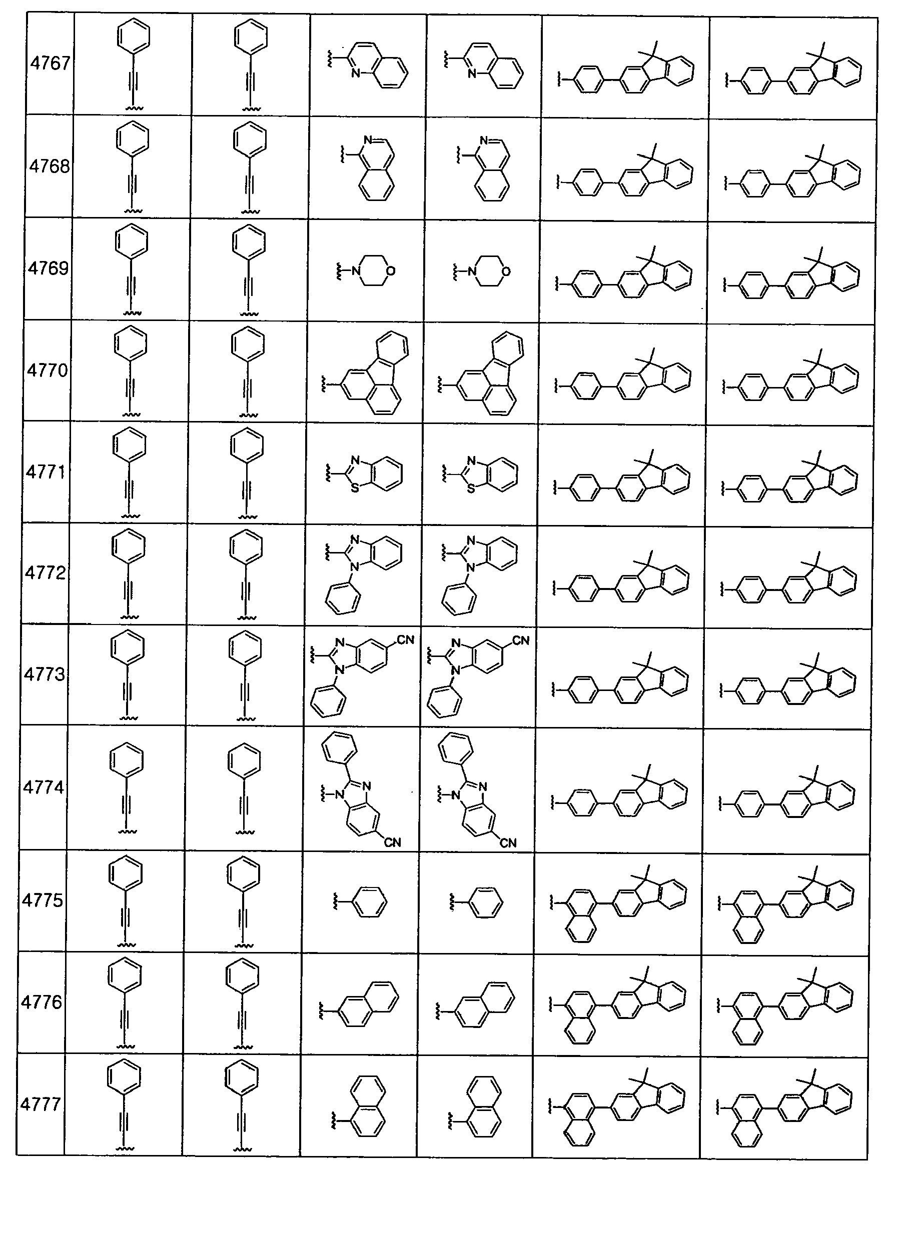

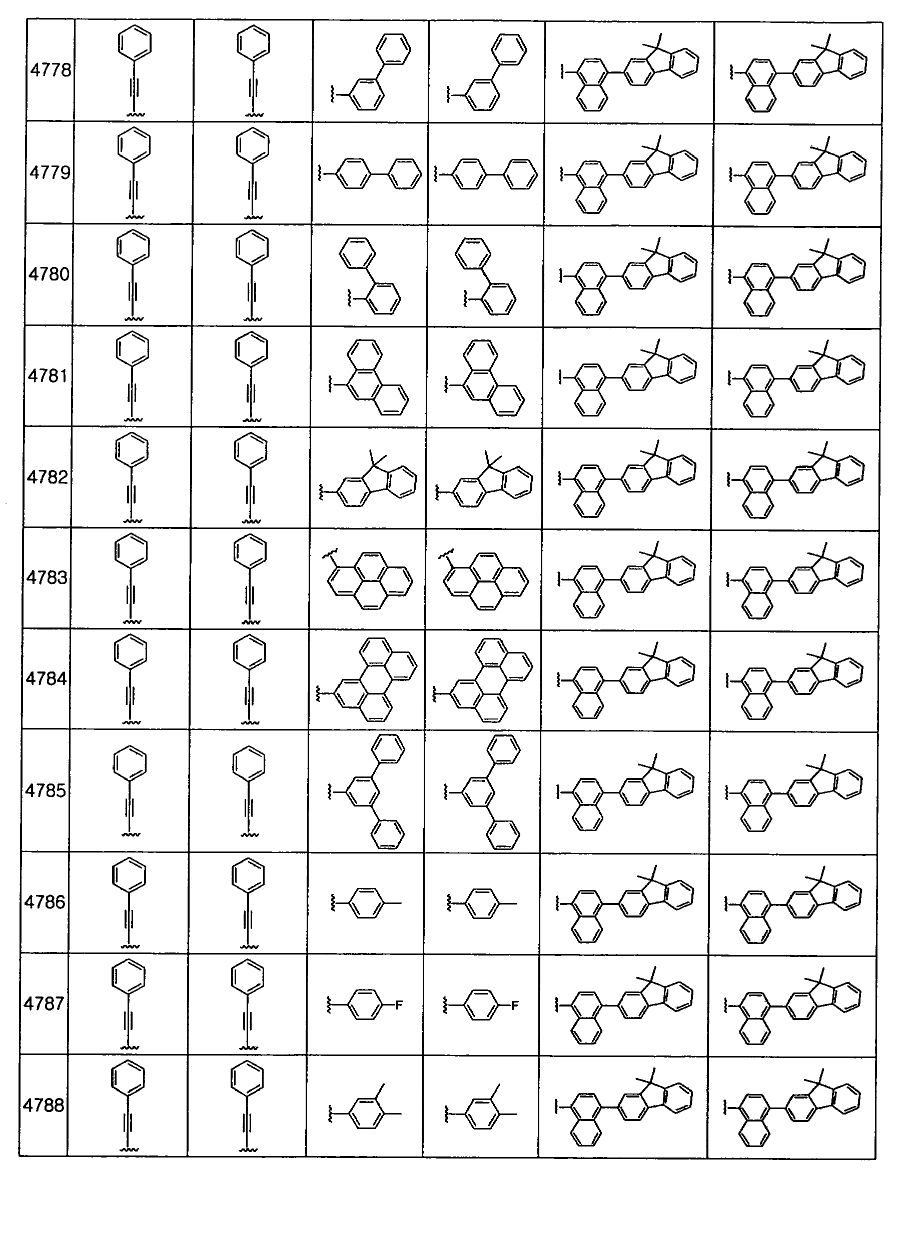

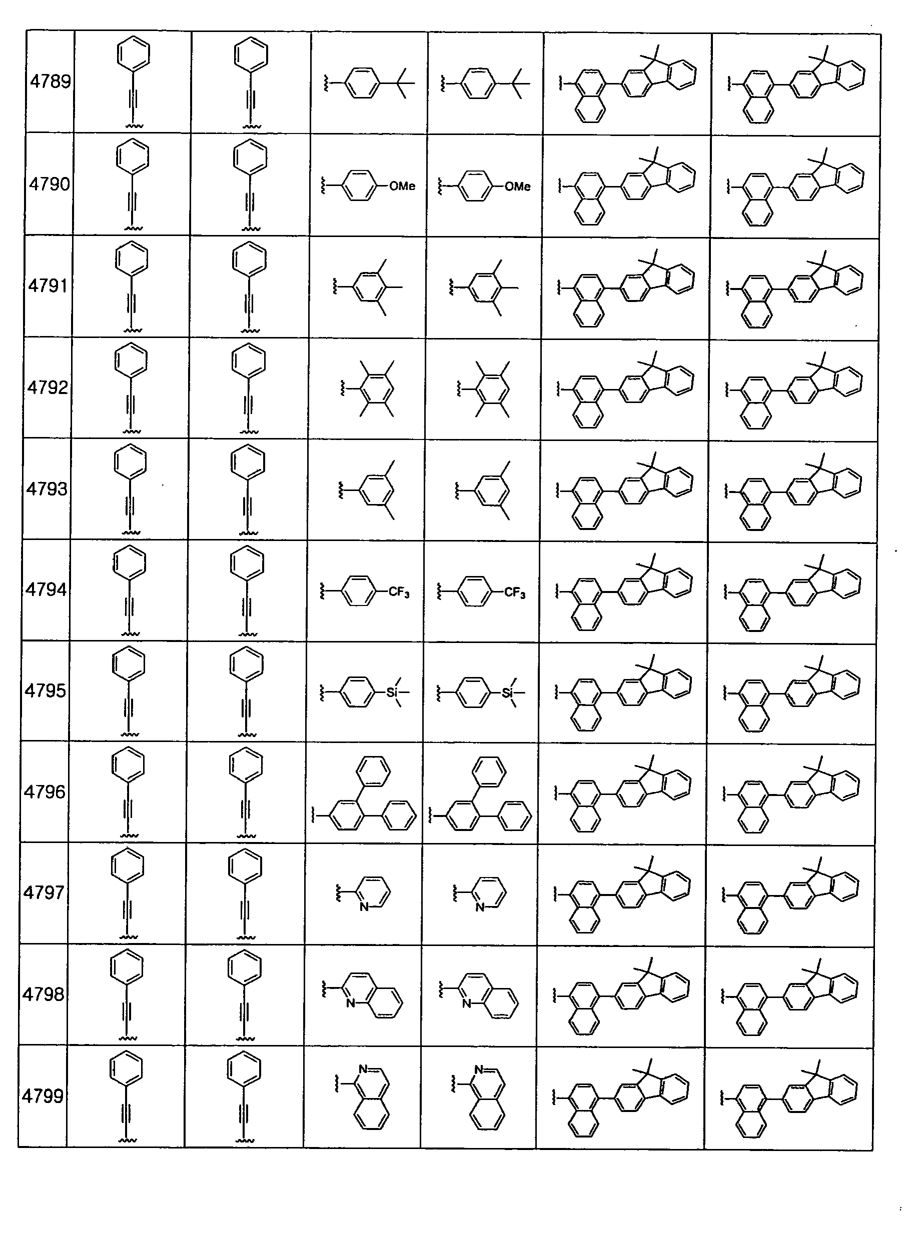

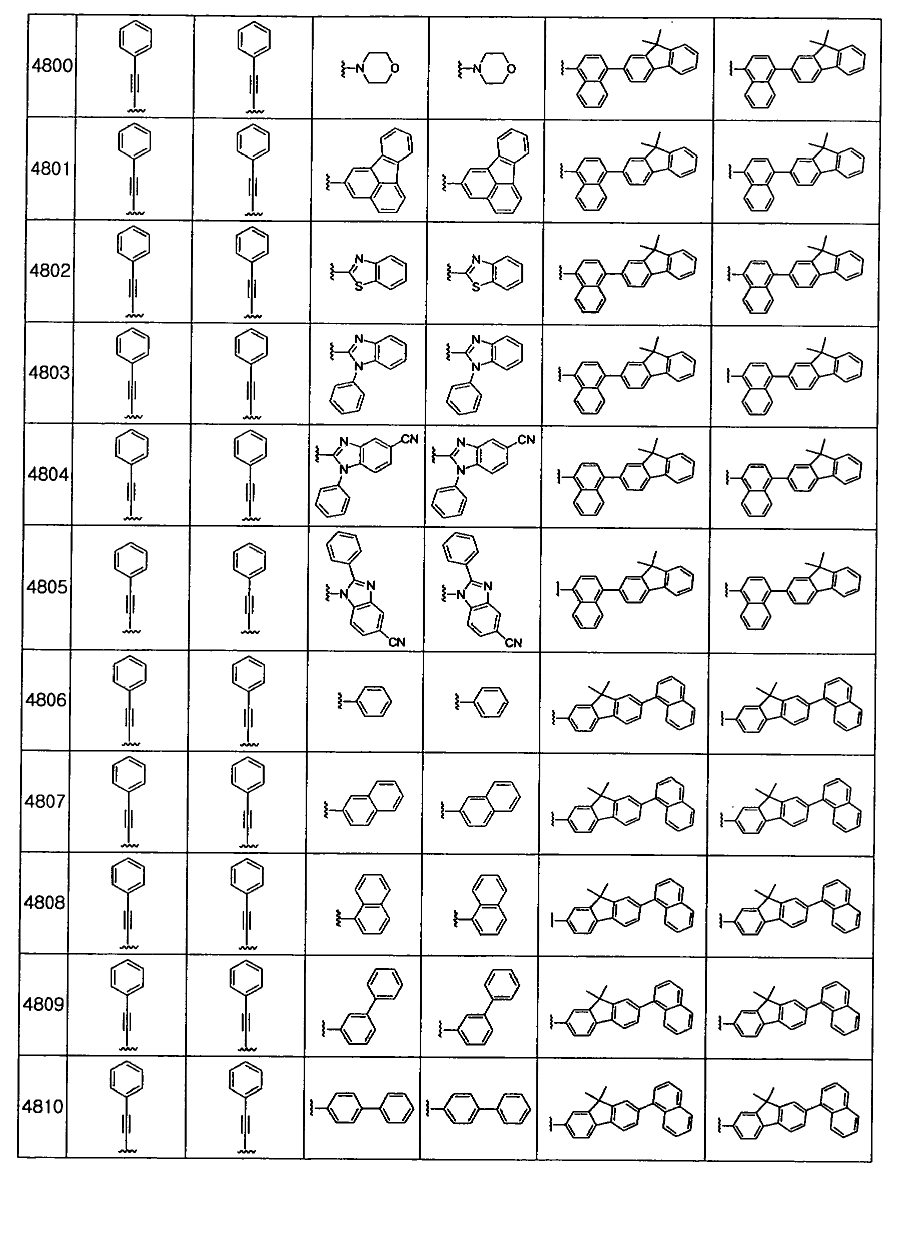

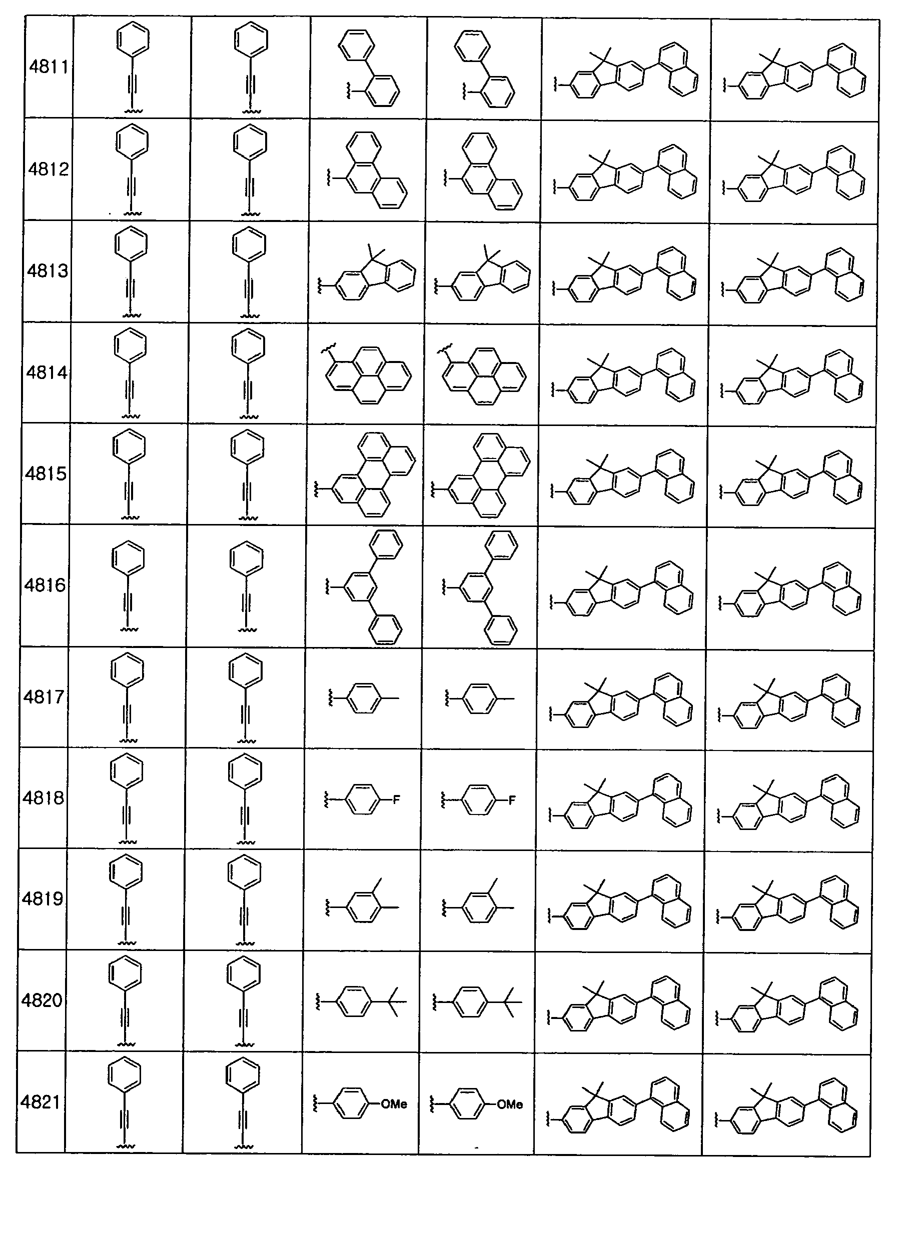

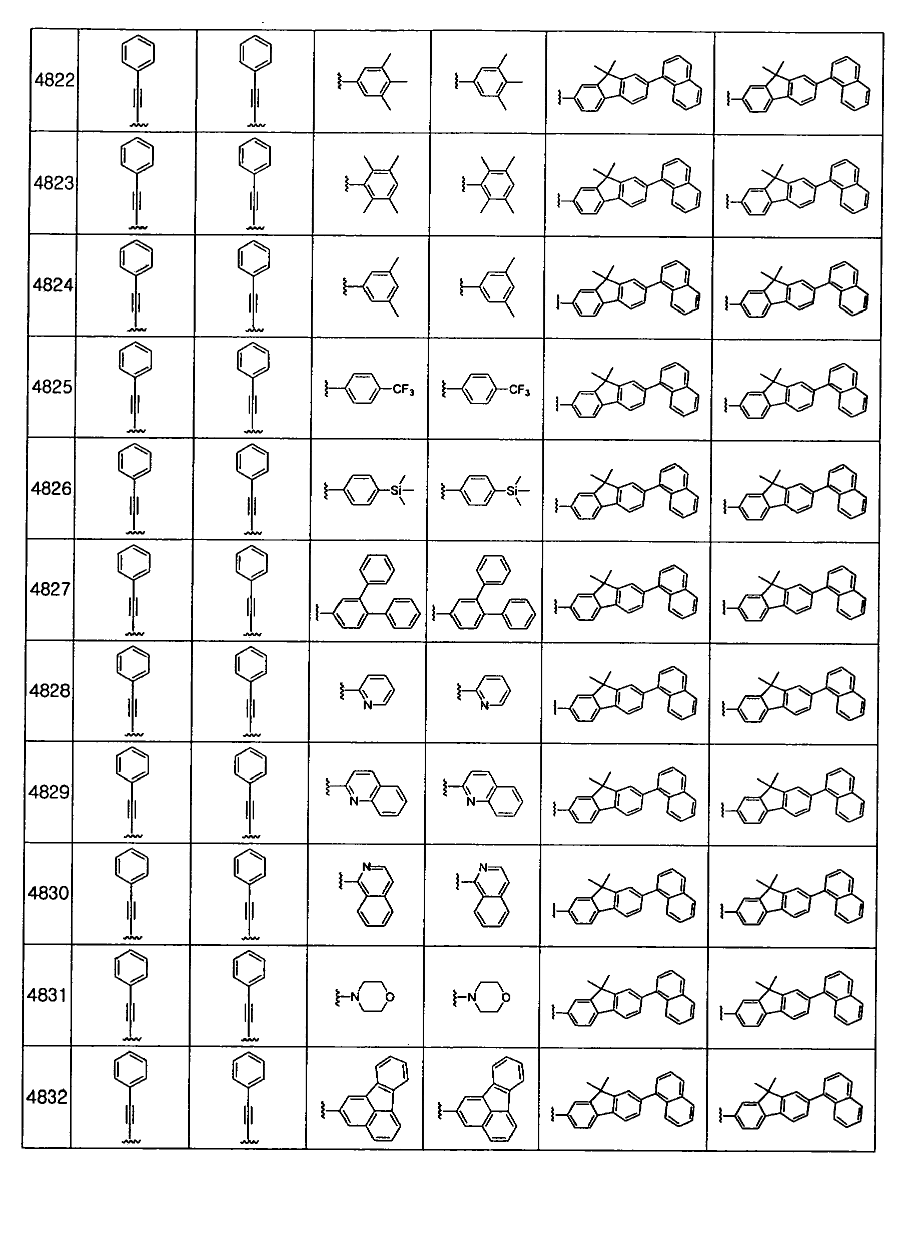

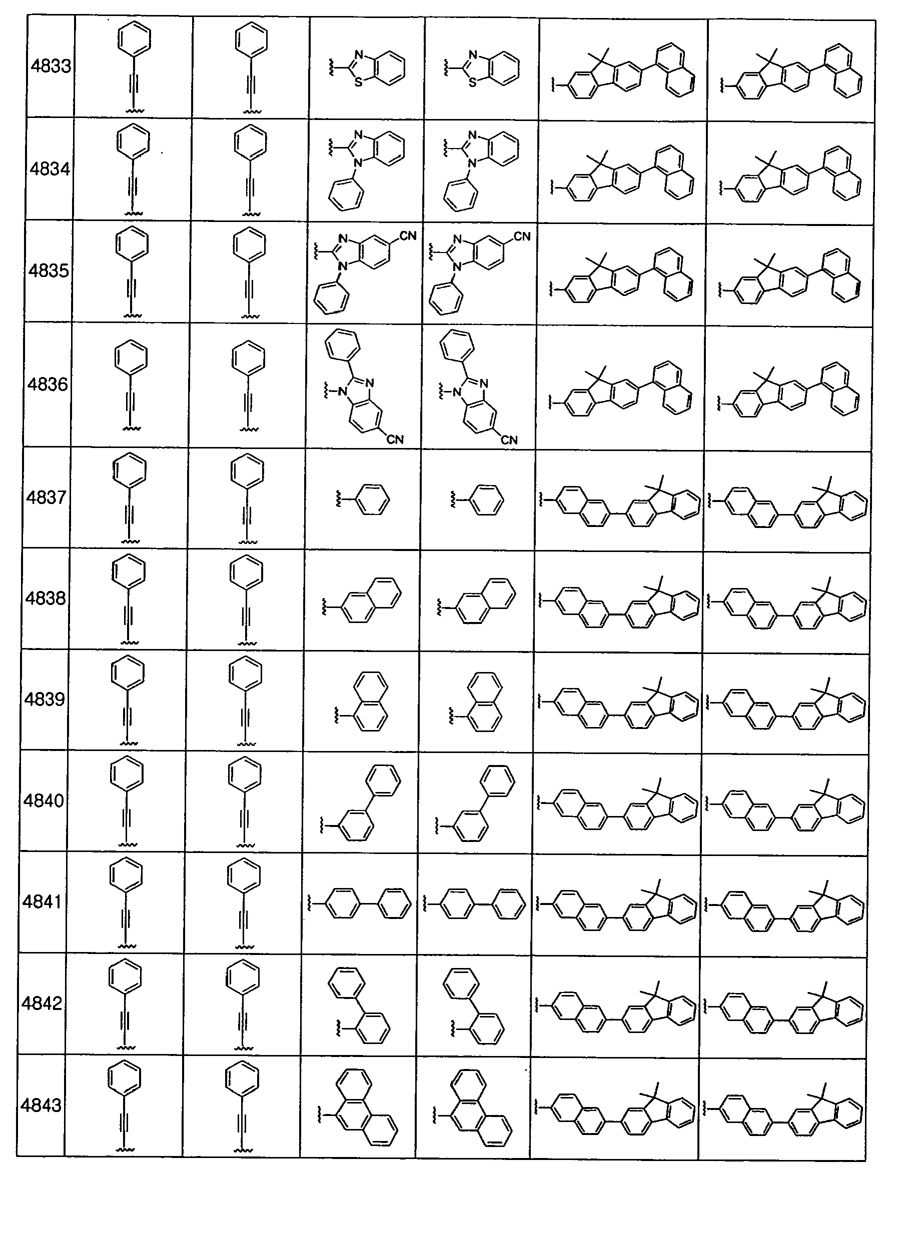

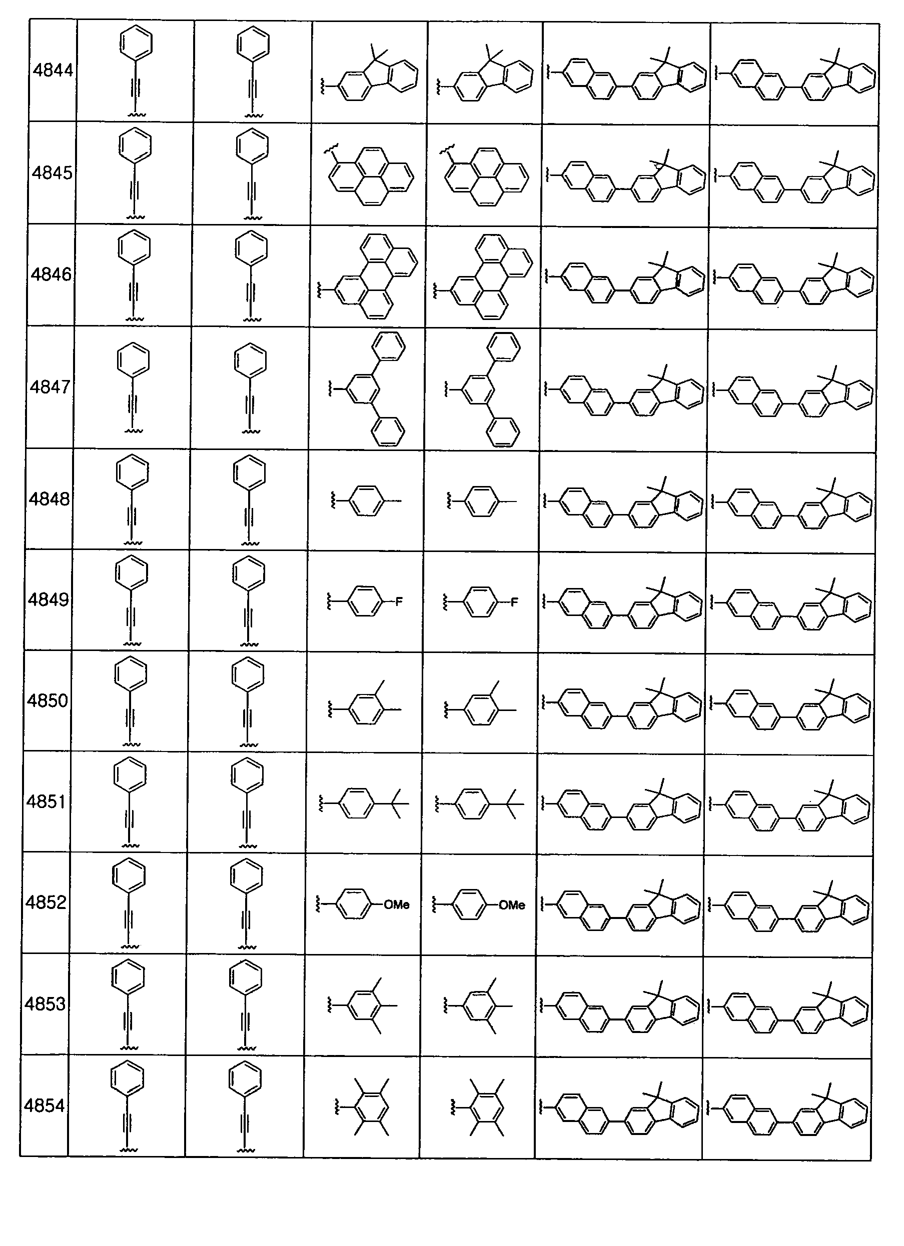

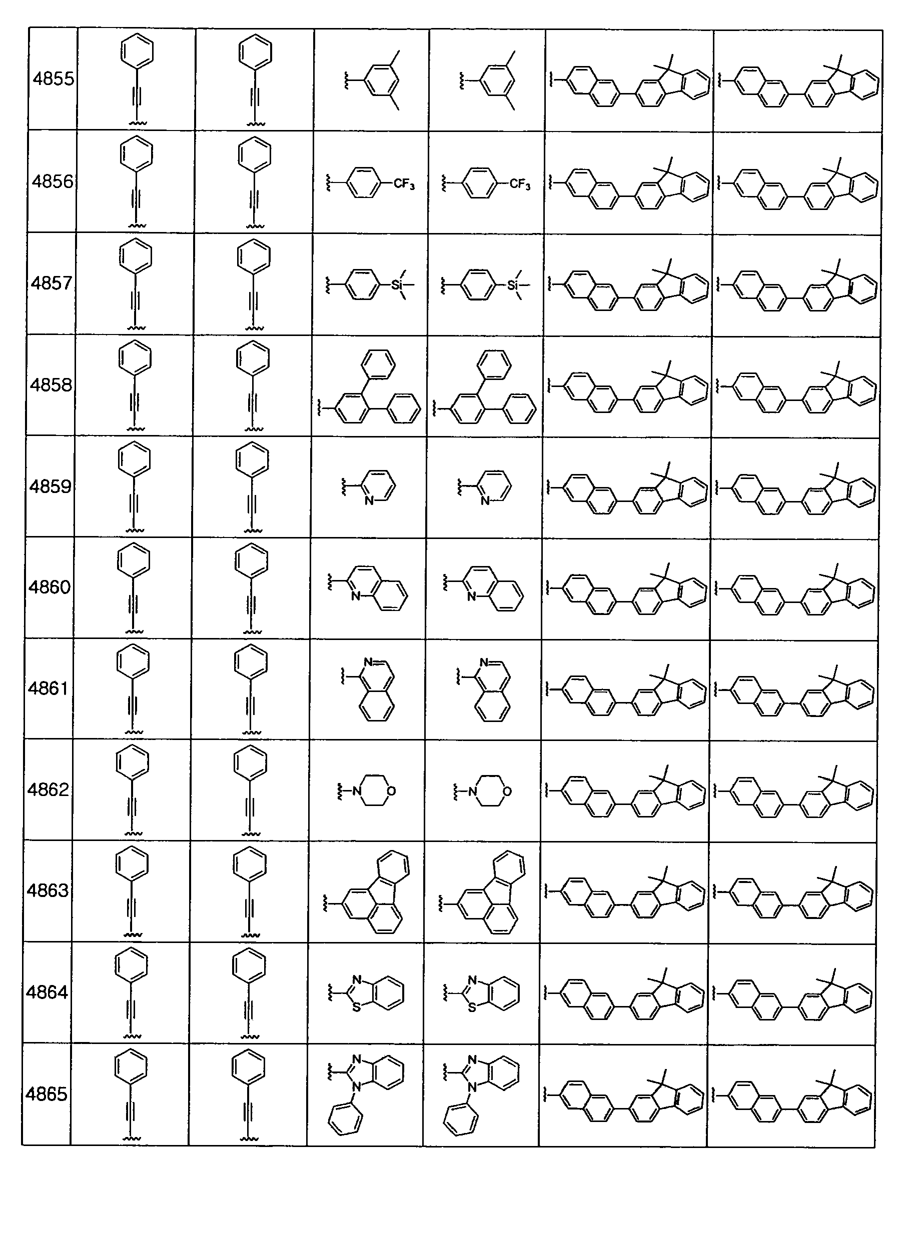

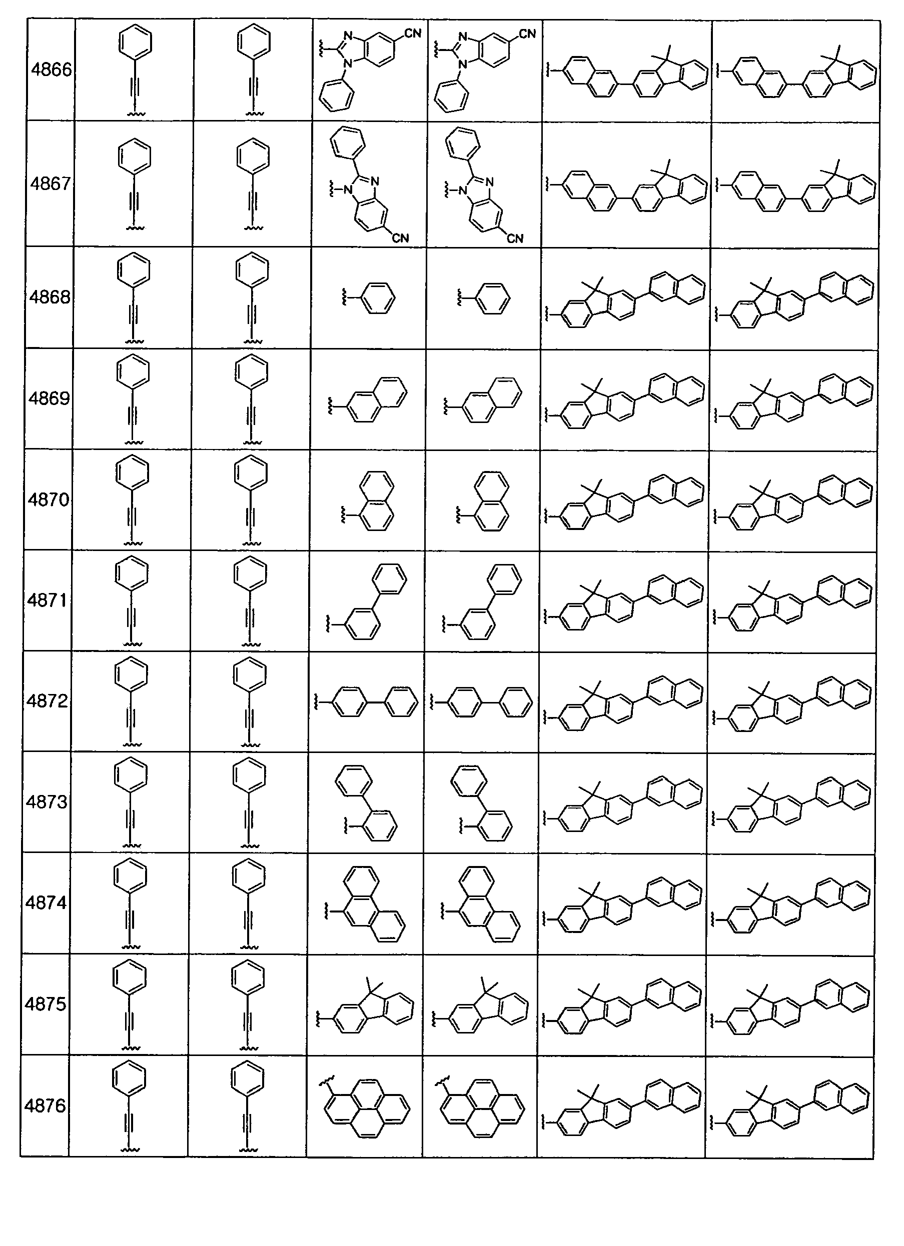

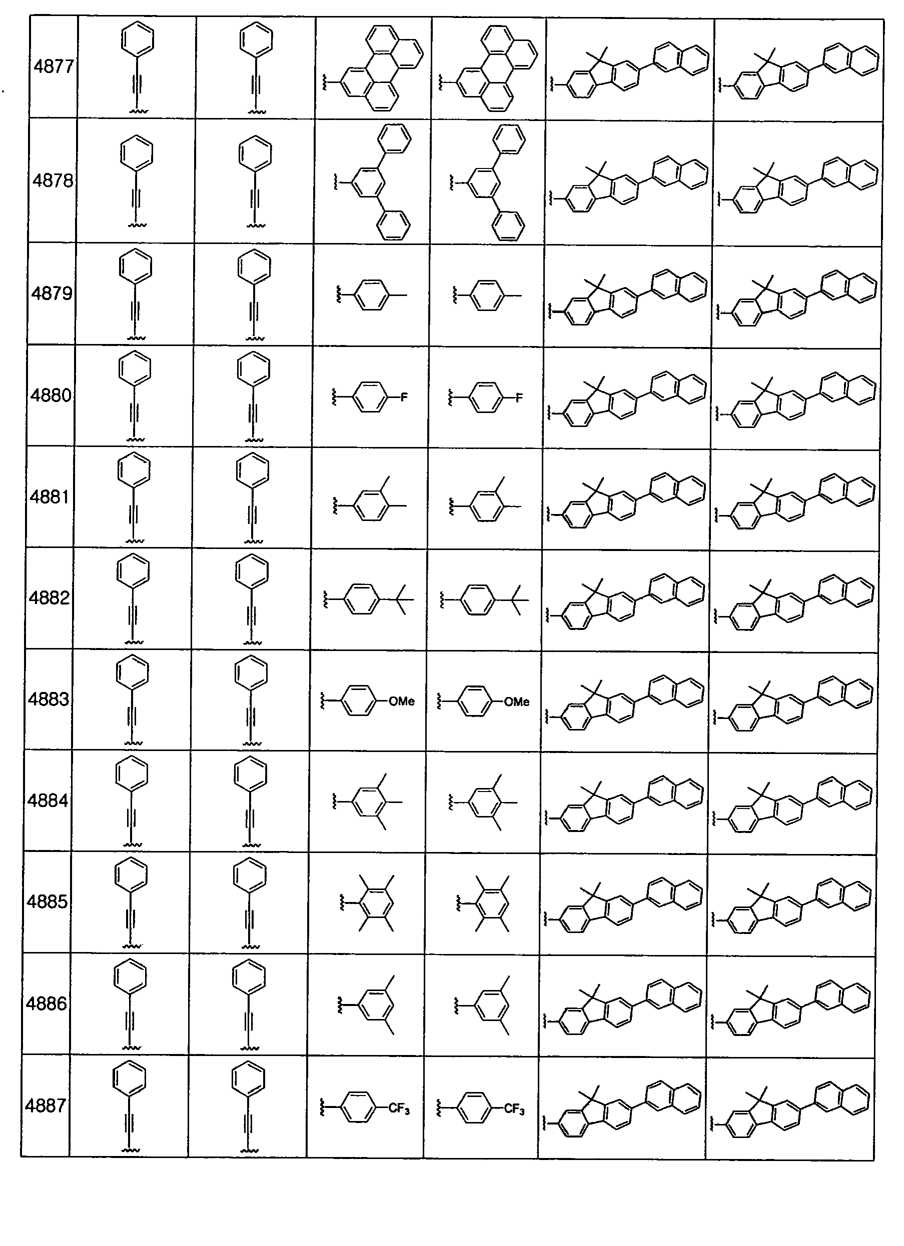

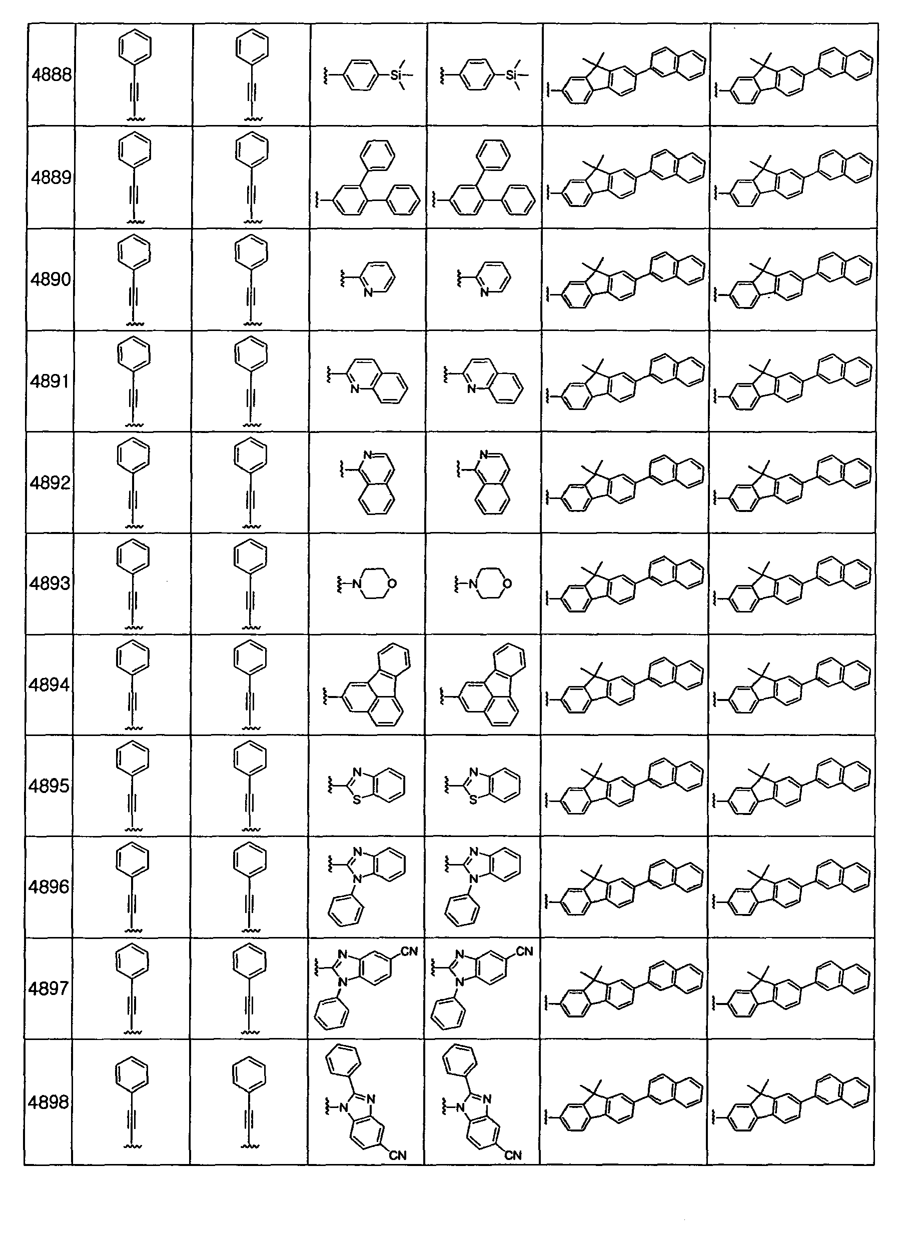

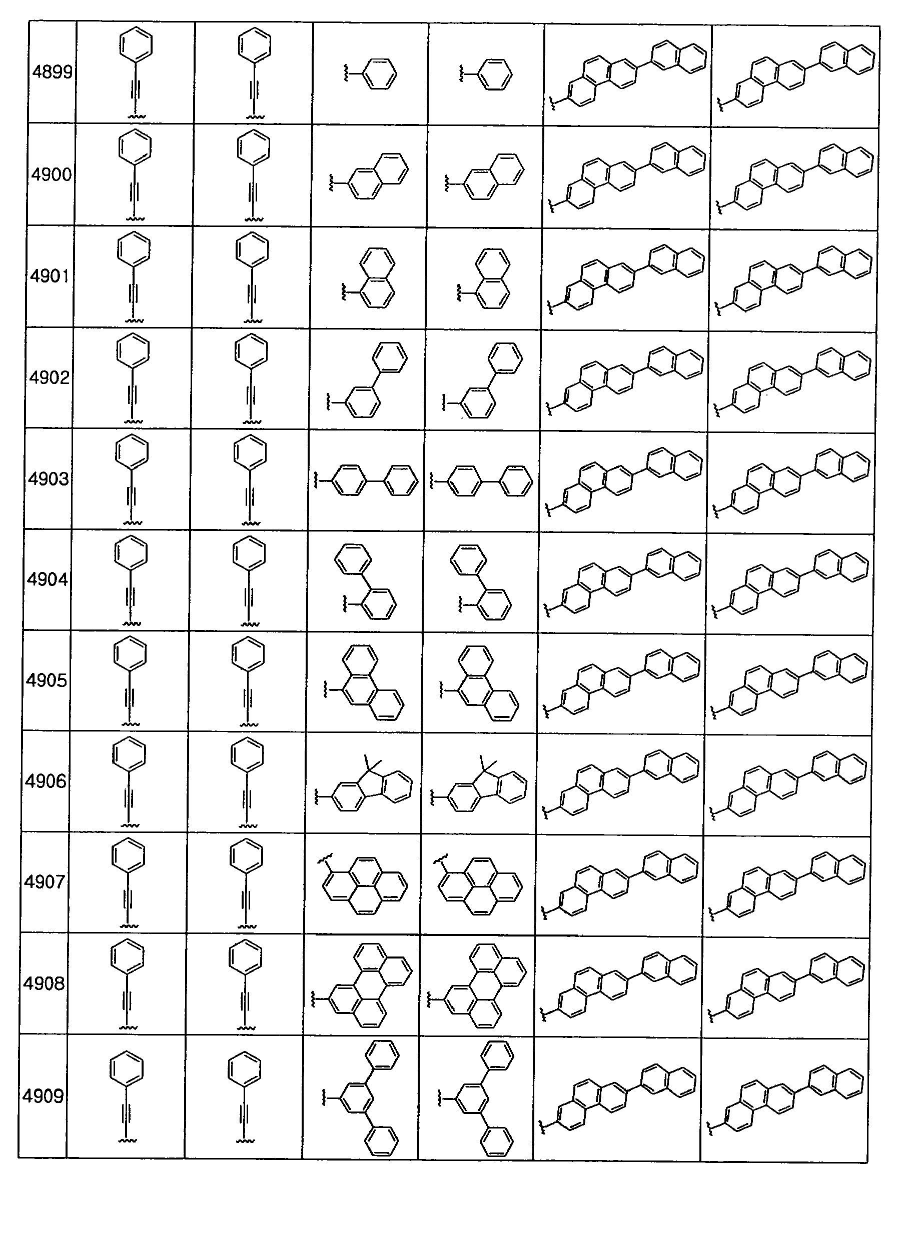

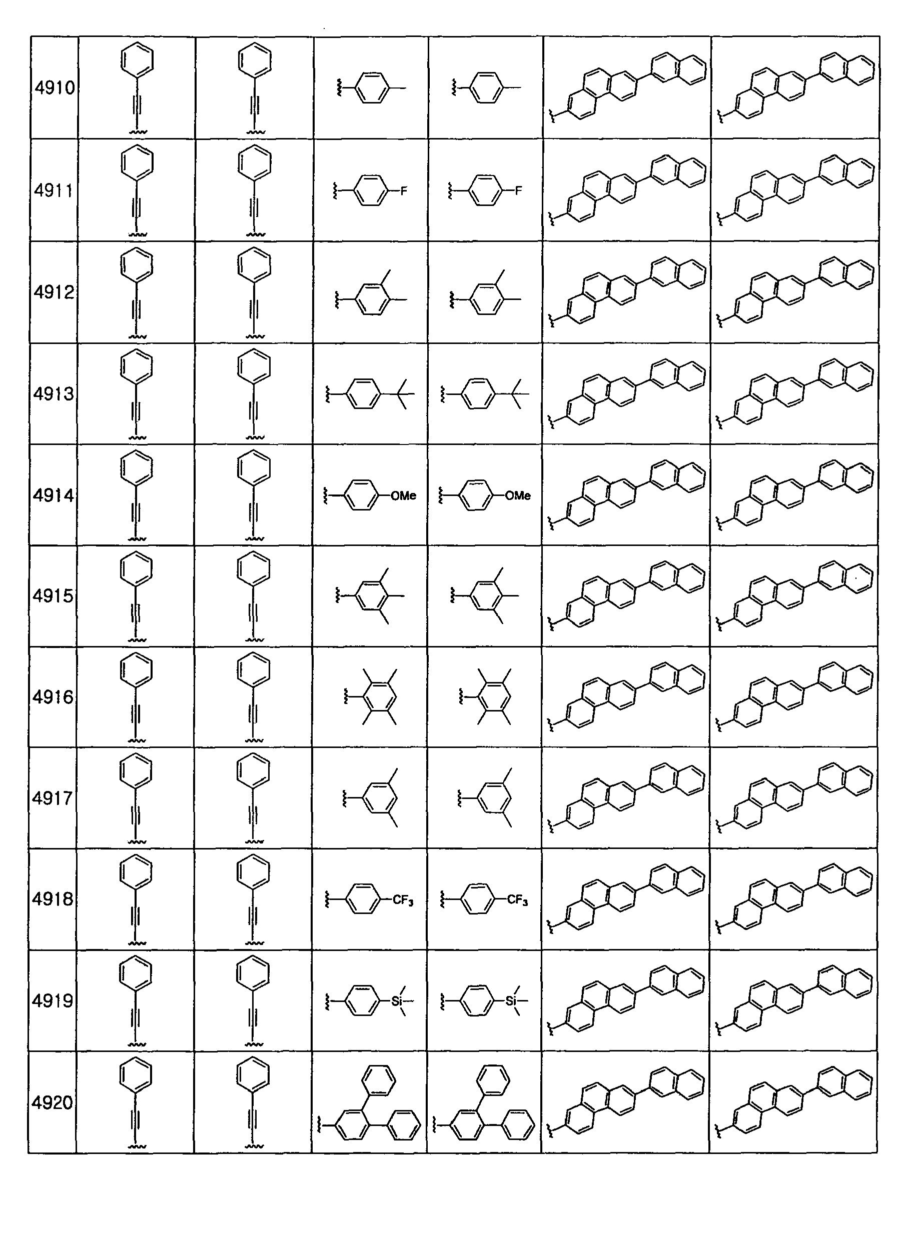

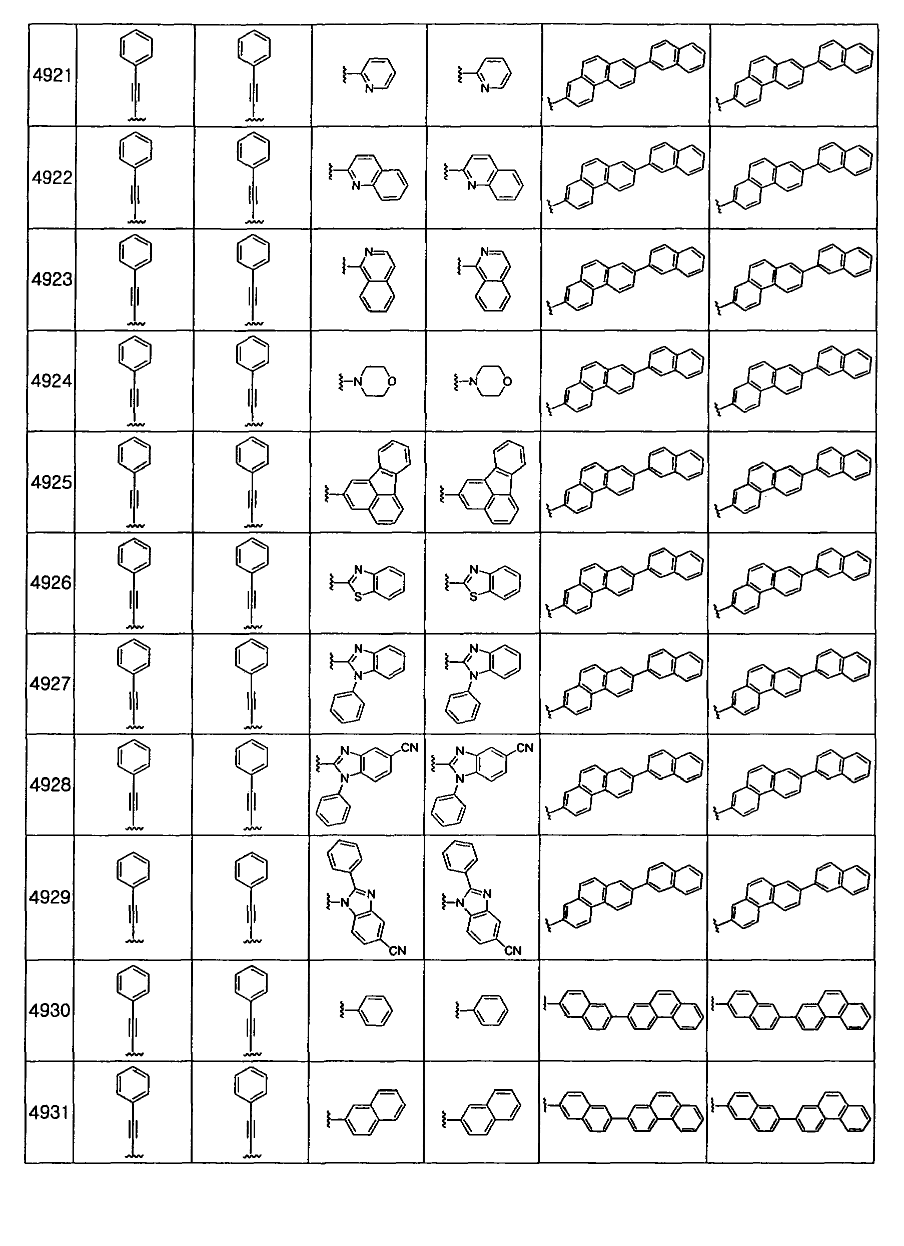

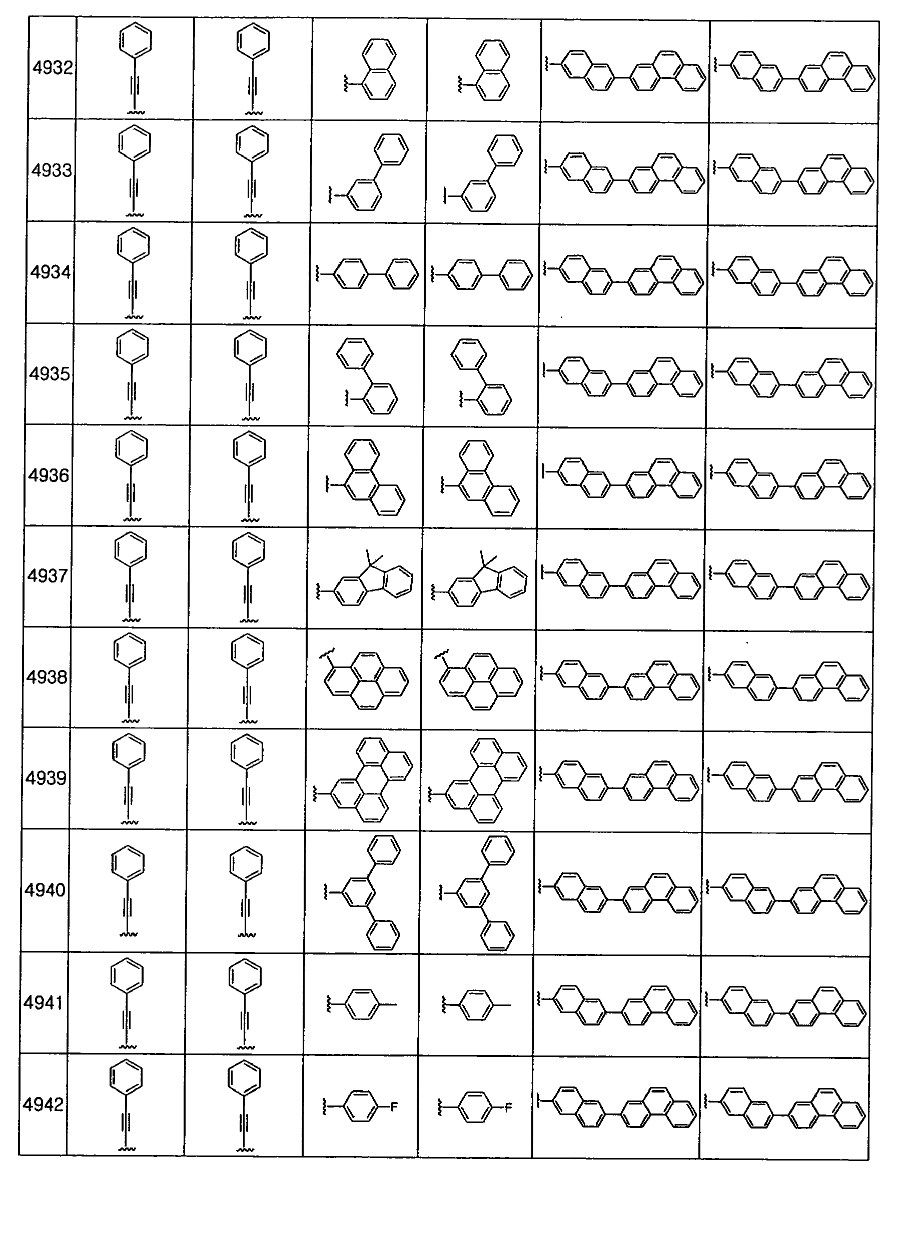

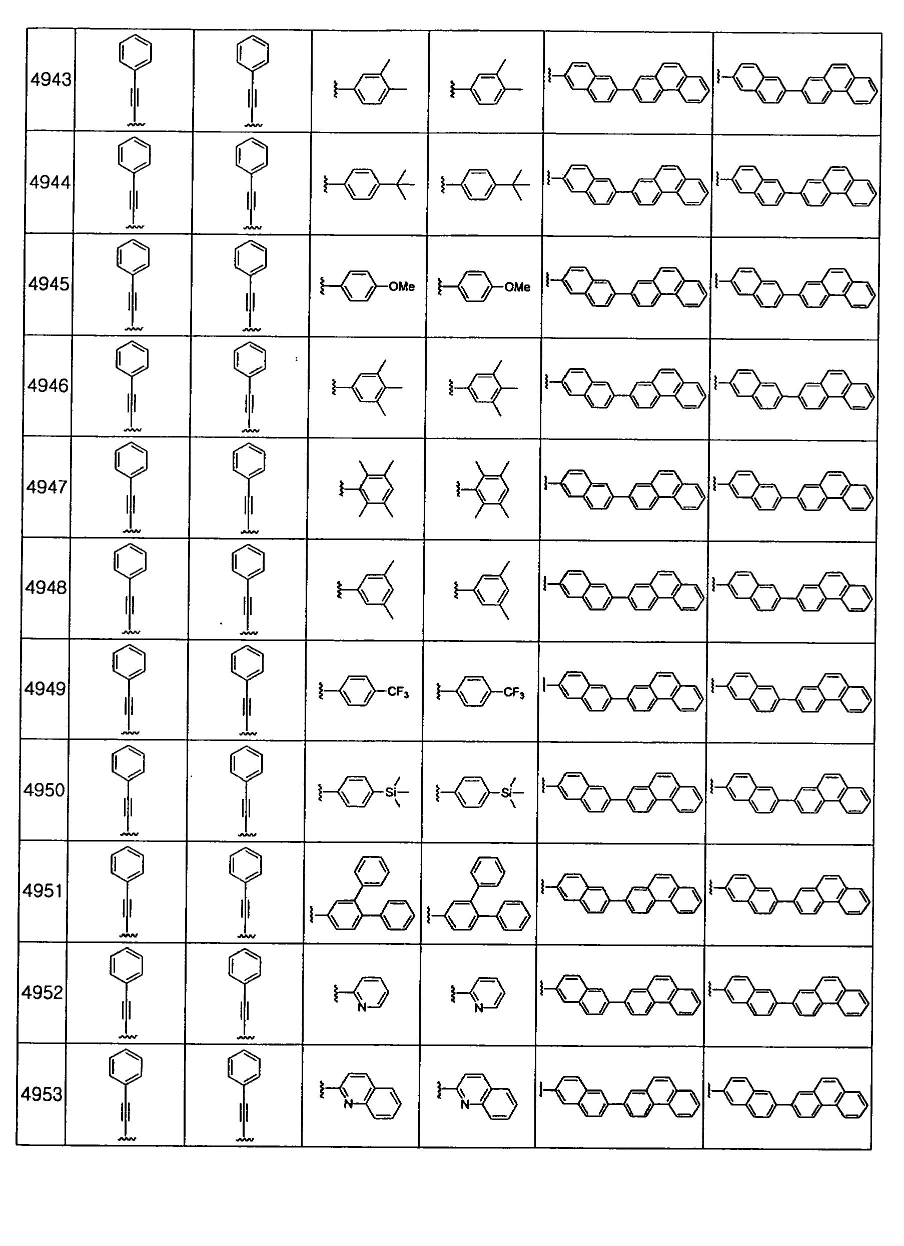

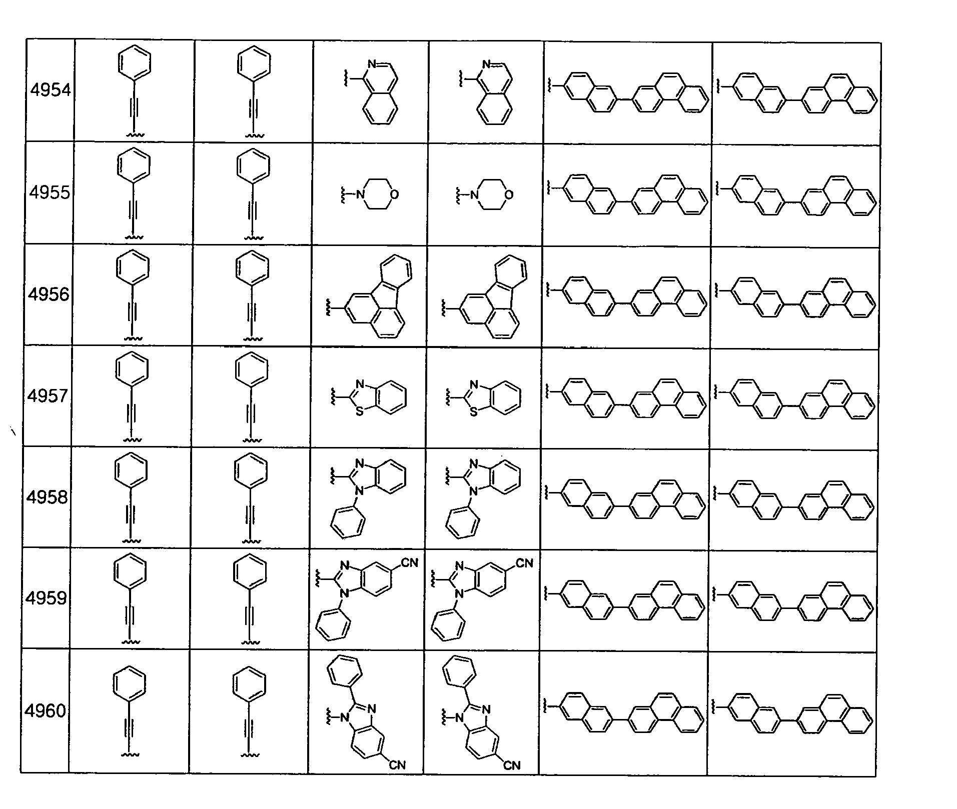

**Table 2**

| Comp. | ¹H NMR(CDCl₃, 200 MHz) | MS/FAB | |
|---|---|---|---|
| | | found | calculated |
| 1 | δ = 7.93(m, 2H), 7.78-7.77(m, 4H), 7.63-7.62(m, 4H), 7.52-7.51(m, 8H), 7.41(m, 2H), 7.20(m, 4H), 7.06(m, 2H), 6.81-8.75(m, 6H), 6.63-6.58(m, 6H), 1.72(s, 12H), 1.48(s, 18H) | 1008 | 1009.37 |
| 2 | δ = 7.93-7.77(m. 14H), 7.63-7.62(m, 4H), 7.52-7.36(m, 16H), 7.06(m, 2H). 6.82(m, 2H). 6.75(m, 2H), 6.58(m, 2H), 1.72(s, 12H), 1.48(s, 18H) | 1108 | 1109.48 |
| 3 | δ = 8.07-7.02(m, 4H), 7.93(m, 2H), 7.78-7.77(m, 4H), 7.63-7.51 (m, 18H), 7.41-7.38(m. 4H), 7.06(m, 2H). 6.96(m. 2H). 6.81(m, 2H), 6.75(m, 2H). 6.58(m, 2H), 1.72(s, 12H), 1.48 (s, 18H) | 1108 | 1109.48 |
| 5 | δ = 7.93(m, 2H), 7.78-7.77(m, 4H), 7.63-7.62(m, 4H), 7.54-7.51 (m, 20H), 7.41(m, 4H), 7.06(m, 2H), 6.81 (m, 2H). 6.75(m, 2H), 6.69(m, 4H), 6.58(m, 2H), 1.72(s, 12H), 1.48 (s, 18H) | 1161 | 1161.56 |
| 8 | δ = 7.93-7.87(m, 4H), 7.78-7.77(m, 4H). 7.63-7.62(m, 6H), 7.55-7.51 (m, 10H), 7.41-7.38(m, 4H), 7.28(m, 2H), 7.06(m. 2H), 6.82(m, 2H), 6.75(m, 4H), 6.58(m, 4H), 1.72(s, 24H), 1.48 (s,18H) | 1241 | 1241.69 |
| 12 | δ = 7.93(m, 2H), 7.78-7.77(m, 4H), 7.63-7.62(m, 4H), 7_{.}52-7.51 (m, 8H), 7.41 (m, 2H), 7.06(m, 2H), 6.98(m, 4H), 6.81(m, 2H), 6.75(m, 2H), 6.58(m, 2H). 6.51(m,4H), 2.34(s,6H), 1.73(s, 12H), 1.48(s, 18H) | 1037 | 1037.42 |
| 15 | δ = 7.93(m, 2H), 7.78-7.77(m, 4H), 7.63-7.62(m, 4H), 7.52-7.51(m. 8H), 7.41 (m, 2H), 7.01(m, 6H), 6.81 (m, 2H), 6.75(m, 2H), 6.58-6.55(m, 6H), 1.72(s, 12H), 1.48(s,18H), 1:35(s. 18H) | 1121 | 1121.58 |
| 19 | δ = 7.93(m, 2H), 7.78-7.77(m, 4H), 7.63-7.62(m, 4H), 752-751(m 8H), 7.41(m. 2H). 7.06(m, 2H), 6.81(m, .2H), 6.75-7.71(m, 4H), 6.58(m, 2H), 6.36(m, 4H), 2.34(s, 12H), 1.72(s, 12H), 1.48(s, 18H) | 1065 | 1065.47 |
| 23 | δ = 8.07(m, 2H), 7.93(m, 2H), 7.78-7.77(m, 4H), 7.63-7.51 (m, 14H), 7.41(m, 2H), 7.06(m, 2H), 6.81 (m, 2H), 6.75-6.70(m, 4H), 6.62-6.58(m, 4H), 1.72(s, 12H), 1.48(s, 18H) | 1011 | 1011.34 |
| 32 | δ = 7.93-7.87(m, 4H), 7.78-7.77(m, 4H), 7.63(m, 2H). 7.55-7.54(m, 6H), 7.38(m, 2H), 7.28-7.20(m, 6H), 7.06(s, 2H), 6.81 (m, 4H), 6.69-6.63(m, 8H), 1.72(s, 12H), 1.48(s, 18H) | 1008 | 1009.37 |
| 33 | δ = 7.93-7.77(m, 16H), 7.63(m, 2H), 7.55-7.49(m, 10H), 7.38-7.28(m, 6H), 7.06(s, 2H), 6.81 (m, 2H), 6.69(m, 4H), 1.72(s, 12H), 1.48(s, 18H) | 1109 | 1 1109.48 |
| 34 | δ = 8.07-8.02(m, 4H). 7.93-7.87(m, 4H), 7.78-7.77(m, 4H), 7.63-7.54(m, 14H), 7.38(m, 4H), 7.28(m, 2H), 7.06(m, 2H), 6.98(m, 2H), 6.81(m, 2H), 6.69(m, 4H), 1.72(s, 12H), 1.48(s, 18H) | 1109 | 1109.48 |
| 35 | δ = 7.93-7.87(m, 4H), 7.78-7.77(m, 4H), 7.63(m, 2H), 7.55-7.51(m, 14H), 7.44-7.38(m, 6H), 7.28(m, 2H), 7.06(m, 2H), 6.89-6.88(m, 4H), 6.81(m, 2H), 6.69(m, 4H), 6.59(m ,2H), 1.72(s, 12H), 1.48(s, 18H) | 1161 | 1161.56 |
| 39 | δ = 7.93-7.87(m, 6H), 7.78-7.77(m, 4H), 7.63-7.62(m, 4H), 7.55-7.54(m, 8H), 7.38(m. 4H), 7.28(m, 4H), 7.06(m, 2H), 6.81(m, 2H), 6.75(m, 2H), 6.69(m, 4H), 6.58(m, 2H), 1.72(s, 12H), 1.48(s, 18H) | 1241 | 1241.69 |
| 43 | δ = 7.93-7.87(m, 4H), 7.78-7.77(m, 4H), 7.63(m, 2H), 7.55-7.54(m, 6H), 7.38(m, 2H), 7.28(m, 2H), 7.06(m, 2H), 6.98(m, 4H), 6.81(m, 2H), 6.69(m, 4H), 6.51(m, 4H), 2.34(s, 6H), 1.72(s, 12H), 1.48(s, 18H) | 1037 | 1037.42 |
| 74 | δ = 8.49(m, 2H), 8.07(m, 2H), 7.93-7.87(m, 4H), 7.78-7.77(m, 6H), 7.63(m, 2H), 7.55-7.53(m, 6H), 7.38(m, 2H), 7.28(m, 2H), 7.06-6.98(m, 8H), 6.81(m, 2H), 6.51(m, 4H), 2.34(s, 6H), 1.72(s, 12H), 1.48(s, 18H) | 1137 | 1137.54 |
| 94 | δ = 8.55(m, 2H), 8.42(m, 2H), 8.08-8.04(m. 4H), 7.93(m, 2H), 7.78-7.77(m, 4H), 7.63-7.55(10H). 7.20(m, 4H), 7.06(m, 2H), 6.81-6.75(m, 6H), 6.63-6.58(m, 6H), 1.72(s, 12H), 1.48(s, 18H) | 1109 | 1109.48 |
| 117 | δ = 8.75(m, 2H), 8.55(m, 2H), 8.42(m, 2H), 8.08-8.04(m, 4H), 7.93(m, 2H), 7.80-7.77(m, 6H), 7.63-7.55(m, 10H), 7.40(m, 4H), 7.19(m. 2H), 7.06(m, 2H). 6.81(m, 2H), 6.75(m, 2H), 6.62-6.58(m, 4H), 1.72(s,12H), 1.48(s, 18H) | 1211 | 1211.58 |
| 126 | δ = 7.93-7.74(m, 26M, 7.55-7.46(m, 10H), 7.38-7.36(m, 4H). 7.28(m, 2H), 7.06(m, 2H), 6.81(m, 2H), 1.72(s, 12H), 1.48(s, 18H) | 1209 | 1209.60 |
| 158 | δ = 8.02-7.92(m, 12H), 7.78-7.73(m, 6H), 7.63-7.53(m, 16H), 7.38(m, 2H), 7.06(m, 2H), 6.98(m. 2H), 6.81(m, 2H), 6.75(m, 2H), 6.58(m, 2H), 1.72(s, 12H), 1.48(s, 18H) | 1209 | 1209.60 |
| 198 | δ = 8.99(m, 2H), 8.68(m, 2H), 8.34(m, 2H), 8.10(m, 2H), 8.00(m, 4H), 7.92(m, 2H), 7.78-7.71(m, 8H), 7.59-7.58(m, 6H), 7.32(m, 2H), 7.08-7.06(m, 4H), 6.98(m, 4H), 6.81(m, 2H), 6.51(m, 4H), 2.34(s, 6H), 1.48(s, 18H) | 1105 | 1105.45 |
| 223 | δ = 8.99-8.93(m, 4H), 8.34(m 2H), 8.12-8.10(m, 4H), 7.82-7.64(m, 18H), 7.54-7.41(m 12H), 7.16-7.06(m, 8H), 6.87-6.81(m, 4H), 6.69(m, 2H), 1.48(s, 18H) | 1229 | 1229.59 |
| 249 | δ = 7.96-7.93(m, 4H). 7.77-7.75(m, 4H), 7.63-7.62(m, 4H). 7.52-7.51(m, 8H), 7.41(m 2H), 7.20(m, 4H), 7.03(m, 2H), 6.83-6.75(m, 6H), 6.63-6.58(m, 6H), 1.72(s. 12H) | 897 | 897.15 |
| 286 | δ = 8.93(m, 4H), 8.12(m, 4H), 7.93-7.75(m 18H), 7.63(m 2H), 7.55-7.54(m, 6H), 7.38(m, 2H), 7.28(m, 2H), 7.03(m, 2H), 6.91(m, 2H), 6.83(m, 2H), 6.69(m, 4H), 1.72(s, 12H) | 1097 | 1097.39 |
| 353 | δ = 8.55(m, 2H), 8.42(m, 2H), 7.08-8.04(m, 4H), 7.96-7.93(m, 4H), 7.77-7.75(m ,4H), 7.63-7.55(m, 10H), 7.03-6.98(m, 6H), 6.83(m. 2H), 6.75(m, 2H), 6.58-6.51(m, 6H), 2.34(s, 6H), 1.72(s, 12H) | 1025 | 1025.32 |
| 422 | δ = 8.00-7.92(m, 10H), 7.77-7.73(m, 6H), 7.63-7.58(m, 10H), 7.03(m, 2H). 6.83(m, 2H), 6.75-6.71(m ,4H), 6.58(m ,2H), 6.36(m. 4H), 2.34(s, 12H), 1.72(s, 12H) | 1053 | 1053.38 |
| 528 | δ = 7.93-7.87(m, 4H), 7.78-7.77(m, 4H), 7.63(m,2H), 7.55-7.54(m, 6H), 7.38(2H), 7.28-7.20(m, 6H), 7.06(s. 2H), 6.81 (m, 4H), 6.69-6.63(m, 8H), 2.649s, 6H), 1.72(s, 12H) | 925 | 925.21 |
| 602 | δ = 8.55(m, 2H), 8.42(m, 2H), 8.08-8.04(m ,4H), 7.93(m ,2H). 7.78-7.77(m, 4H), 7.63-7.55(m ,10H), 7.06-6.99(m, 6H), 6.81-6.75(m, 4H), 6.61-6.58(m, 6H), 2.64(s, 6H), 1.72(s, 12H) | 1061 | 1061.31 |
| 648 | δ = 8.18(m. 2H), 8.01 (m ,2H), 7.93-7.63(m, 18H), 7.55-7.46(m, 10H), 7.38(m, 2H), 7.28(m, 2H), 7.06(m, 2H), 6.81 (m ,2H), 2.64(s, 6H), 1.72(s, 12H) | 1139 | 1139.47 |
| 745 | δ = 7.93(m, 2H), 7.77-7.75(m ,4H), 7.63-7.62(m, 4H), 7.52-7.51(m, 16H), 7.41(m, 4H), 7.20(m, 4H), 7.03(m, 2H), 6.83-6.75(m, 6H), 6.63-6.58(m, 6H), 1.72(s, 12H) | 1049 | 1049.35 |
| 777 | δ = 7.93-7.75(m, 16H), 7.63(m, 2H), 7.55-7.50(m, 16H), 7.49-7.41(m, 4H), 7.38-7.36(m, 4H), 7.28(m, 2H), 7.03(m, 2H), 6.83(m, 2H), 6.69(m, 4H), 1.72(s, 12H) | 1149 | 1149.46 |
| 852 | δ = 8.55(m ,2H), 8.42(m, 2H), 8.08-8.04(m, 4H), 7.93(m. 2H), 7.77-7.75(m, 4H), 7.63-7.51(m, 18H), 7.41 (m, 2H). 7.03-7.01(m, 6H), 6.83(m, 2H), 6.75(m, 2H), 6.55(m, 6H), 1.72(s; 12H), 1.35(s, 18H) | 1261 | 1261.68 |
| 913 | δ = 8.00-7.92(m, 8H), 7.77-7.73(m, 6H), 7.63-7.58(m, 10H), 7.52-7.51(m, 8H). 7.41 (m, 2H). 7.03(m, 2H). 6.86-6.83(m, 4H), 6.75(m, 2H). 7.58(m, 2H), 6.43(m ,2H), 6.32(m, 2H), 2.34(s, 12H), 1.72(s, 12H) | 1205 | 1205.57 |
| 982 | δ = 8.99-8.93(m, 4H), 8.34(m, 2H), 8.12-8.10(m, 4H), 7.88-7.64(m, 18H), 7.52-7.41 (m. 14H), 7.15(m, 4H), 7.03(m ,2H), 6.83(m ,2H), 6.61(m, 4H), 0.25(s, 18H) | 1261 | 1261.74 |
| 993 | δ = 7.93(m, 2H), 7.78-7.77(m, 4H), 7.63-7.62(m, 4H), 7.52-7.51 (m, 8H), 7.41 (m, 2H), 7.20(m, 4H), 7.06(m, 2H), 6.81-6.75(m, 6H), 6.63-6.58(m, 6H), 2.72(m, 2H), 1.88(m, 4H), 1.72(s. 12H), 1.63(m, 4H), 1.53(m, 4H), 1.49-1.43(m, 8H) | 1061 | 1061.44 |
| 1024 | δ = 7.93-7.87(m, 4H), 7.78-7.77(m, 4H), 7.63(m, 2H), 7.55-7.54(m. 6H), 7.38(m, 2H), 7.28-7.20(m. 6H), 7.06(m ,2H), 6.81(m, 4H), 6.69-6.63(m, 8H), 2.72(m, 2H), 1.88(m, 4H), 1.72(s, 12H), 1.63(m, 4H), 1.53(m, 4H), 1.49-1.43(m. 8H) | 1061 | 1061.44 |
| 1068 | δ = 8.49(m, 2H), 8.07(m ,2H), 7.93-7.87(m, 4H). 7.78-7.77(m, 6H), 7.63(m ,2H), 7.55-7.53(m, 6H), 7.38(m, 2H), 7.28(m, 2H), 7.06-7.04(m, 4H), 6.86-6.81(m, 4H), 6.43(m, 2H), 6.32(m ,2H), 2.72(m ,2H), 2.34(s, 12H), 1.88(m, 4H), 1.72(s, 12H), 1.63(m ,4H), 1.53(m, 4H), 1.49-1.43(m, 8H) | 1217 | 1217.66 |
| 1133 | δ = 7.93-7.64(m, 18H), 7.55-7.46(m, 6H). 7.38(m. 2H), 7.28(m, 2H), 7.06(m. 2H), 6.81(m, 2H), 6.24(s, 4H), 2.72(m, 2H), 2.34(s, 12H), 2.18(s, 6H), 1.88(m, 4H), 1.72(s, 12H), 1.63(m, 4H), 1.53(m, 4H), 1.49-1.43(m, 8H) | 1245 | 1245.72 |
| 1205 | δ = 8.99(m, 2H), 8.68(m, 2H), 8.42(m, 4H), 8.34(m, 2H), 8.10(m, 6H), 8.00-7.92(m, 6H), 7.71-7.49(m, 20H), 7.34-7.32(m, 4H), 7.06(m, 4H), 6.91(m, 2H), 6.81(m, 2H), 2.72(m ,2H), 1.88(m, 4H), 1.63-1.43(m, 16H) | 1377 | 1377.75 |
| 1272 | δ = 8.55(m ,2H), 8.42(m, 2H), 8.08-8.04(m, 4H), 7.93-7.87(m, 4H), 7.77-7.75(m, 4H), 7.55-7.54(m, 14H), 7.38(m, 2H), 7.28-7.20(m, 6H), 7.03(m ,2H), 6.83-6.81(m, 4H), 6.69-6.63(m, 8H) | 1149 | 1149.46 |
| 1314 | δ = 8.55-8.42(m, 6H), 8.08-8.04(m, 6H), 7.93-7.87(m, 4H), 7.78-7.75(m, 6H), 7.63-7.53(m ,14H), 7.38(m, 2H), 7.28(m, 2H), 7.04-6.98(m, 8H), 6.83(m. 2H), 6.51(m, 4H), 2.34(s, 6H), 1.72(s, 12H) | 1277 | 1277.63 |
| 1387 | δ = 8.55(m, 2H), 8.42(m, 2H), 8.08-8.04(m, 6H), 7.93-7.84(m. 6H), 7.77-7.46(m, 26H), 7.38(m, 2H), 7.28(m, 2H), 7.03(m, 2H), 6.83(m, 2H), 6.70-6.62(m, 4H), 1.72(s, 12H) | 1251 | 1251.56 |
| 1462 | δ = 8.99-8.93(m, 4H), 8.55(m, 2H), 8.42(m, 2H), 8.34(m, 2H), 8.12-8.04(m, 8H), 7.88-7.4Hm, 42H), 7.03(m, 2H), 6.83(m, 2H), 6.69(m, 4H) | 1369 | 1369.69 |
| 1492 | δ = 7.93(m, 2H), 7.77-7.75(m, 4H), 7.63-7.62(m, 4H), 7.52-7.41(m, 22H), 7.03(m, 2H), 6.89-6.83(m, 6H), 6.75(m, 2H), 6.59-6.58(m, 4H), 1.72(s, 12H), 0.25(s, 18H) | 1193 | 1193.71 |
| 1561 | δ = 8.49(m, 2H), 8.07(m,2H), 7.93-7.87(m, 4H), 7.78-7.75(m, 6H), 7.63(m, 2H), 7.55-7.41 (m ,26H), 7.38(m ,2H), 7.28(m, 2H), 7.06-7.03(m, 6H), 6.85-6.83(m. 6H), 1.72(s, 12H), 0.25(s, 18H) | 1446 | 1446.02 |
| 1627 | δ = 7.93-7.84(m, 6H), 7.77-7.63(m, 12H), 7.55-7.46(m, 6H), 7.38(m, 2H), 7.28(m, 2H), 7.03-7.01(m, 6H), 6.83(m, 2H), 6.55(m, 4H), 1.72(s, 12H), 1.35(s, 18H), 0.25(s, 18H) | 1253 | 1253.85 |
| 1737 | δ = 8.00-7.92(m, 8H), 7.77-7.73(m, 6H), 7.63-7.51 (m. 18H), 7.41(m, 2H), 7.20(m, 4H), 7.03(m, 2H), 6.81-6.75(m, 6H), 6.63-6.58(m, 6H), 1.72(s, 12H) | 1149 | 1149.46 |
| 1770 | δ = 8.07-7.87(m, 14H), 7.77-7.73(m, 6H). 7.63-7.53(m, 20H), 7.38(m, 4H), 7.28(m ,2H), 7.03-6.98(m, 4H), 6.83(m ,2H), 6.69(m, 4H), 1.72(s. 12H) | 1249 | 1249.58 |
| 1849 | δ = 8.55(m, 2H), 8.42(m, 2H), 8.08-7.93(m ,12H), 7.77-7.73(m, 6H), 7.63-7.55(m, 16H), 7.37(m, 4H), 7.03(m, 2H), 6.83(m, 2H), 6.75(m, 2H), 6.58-6.56(m, 6H), 1.72(s, 12H) | 1385 | 1385.58 |
| 1875 | δ = 8.00-7.84(m, 12H), 7.77-7.46(m, 26H), 7.38(m, 2H), 7.28(m, 2H), 7.03-7.01 (m, 6H), 6.83(m, 2H), 6.55(m, 4H), 1.72(s, 12H), 1.35(s. 18H) | 1361 | 1361.79 |
| 1971 | δ = 8.99-8.93(m, 4H), 8.34(m, 2H), 8.12-8.10(m, 4H), 8.00-7.59(m, 32H), 7.46(m, 4H), 7.03(m, 2H). 6.83(m ,2H), 6.22(m, 2H), 2.34(s, 12H), 2.12(s, 12H) | 1329 | 1329.71 |
| 2016 | δ = 7.93-7.87(m, 4H), 7.77-7.75(m, 4H), 7.63(m, 2H), 7.55-7.51(m, 14H), 7.41-7.38(m, 4H), 7.28-7.20(m, 14H), 7.03(m, 2H), 6.83-6.81(m, 4H), 6.69-6.63(m, 8H), 1.72(s, 12H) | 1201 | 1201.54 |
| 2086 | δ = 8.55(m, 2H), 8.42(m, 2H), 8.12-8.04(m, 6H), 7.93-7.71 (m, 20H), 7.63-7.51 (m, 18H), 7.41 (m ,2H), 7.25(m, 8H), 7.03-7.02(m, 4H), 6.83(m, 2H), 6.75(m, 2H), 6.58(m, 2H), 1.72(s, 12H) | 1551 | 1551.95 |
| 2227 | δ = 8.99(m, 4H), 8.34(m ,2H), 8.12-8.10(m ,4H), 7.88-7.64(m, 18H). 7.52-7.46(m, 14H), 7.25(m, 8H), 7.03(m ,2H), 6.83(m ,2H), 3.65(m, 8H), 3.1Hm, 8H) | 1287 | 1287.59 |
| 2240 | δ = 7.93-7.87(m, 4H), 7.77-7.75(m, 4H), 7.63-7.28(m, 52H), 7.03(m, 2H), 6.83(m, 2H), 6.75(m, 4H), 6.58(m. 4H), 1.72(s, 24H) | 1646 | 1646.25 |
| 2400 | δ = 8.00-7.92(m. 8H), 7.77-7.73(m, 6H), 7.63-7.37(m, 40H), 7.03-6.99(m, 6H), 6.83(m, 2H), 6.75(m, 2H), 6.61-6.58(m, 6H), 1.72(s, 12H) | 1550 | 1550.03 |
| 2512 | δ = 7.93-7.87(m, 8H), 7.77-7.75(m, 6H), 7.63(m, 4H). 7.55-7.54(m, 8H), 7.38(m, 4H), 7.28-7.20(m, 8H), 7.03(m, 2H), 6.83-6.81(m, 4H), 6.69-6.63(m, 8H), 1.72(s, 24H) | 1281 | 1281.67 |
| 2523 | δ = 7.93-7.87(m, 8H), 7.77-7.75(m, 6H), 7.63(m, 4H), 7.55-7.54(m, 8H), 7.38(m, 4H), 7.28(m, 4H), 7.03-6.98(m, 6H), 6.83(m, 2H), 6.69(m, 4H), 6.51 (m. 4H), 2.34(s, 6H), 1.72(s, 24H) | 1309 | 1309.72 |
| 2728 | δ = 7.93(m, 2H), 7.77-7.75(m, 4H), 7.66-7.62(m, 10H), 7.52-7.51(m, 24H), 7.41(m, 6H), 7.20(m, 4H), 7.03(m. 2H). 6.83-6.75(m, 6H), 6.63-6.58(m, 6H), 1.72(s, 12H) | 1353 | 1353.73 |
| 2775 | δ = 7.93-7.87(m, 4H), 7.77-7.75(m, 4H), 7.66-7.63(m, 8H), 7.55-7.38(m, 28H), 7.28(m ,2H), 7.03(m, 2H), 6.83(m, 2H), 6.74-6.69(m, 8H), 6.52(m, 4H), 3.83(s, 6H), 1.72(s, 12H) | 1413 | 1413.78 |
| 2978 | δ = 7.93-7.74(m, 16H), 7.63-7.49(m, 16H), 7.38-7.28(m, 8H), 7.03(s, 2H), 6.83(m, 2H), 6.69(m, 4H), 2.34(s, 12H), 1.72(s, 12H) | 1205 | 1205.57 |
| 3222 | δ = 8.56(m ,2H), 7.93-7.87(m, 4H), 7.77-7.75(m, 4H), 7.63-7.22(m, 34H), 7.03(m ,2H), 6.83(m ,2H), 6.69(m, 4H), 2.34(s, 12H), 1.72(s, 12H) | 1337 | 1337.62 |
| 3258 | δ = 8.07-7.02(m, 4H), 7.93-7.87(m, 4H), 7.77-7.75(m, 4H), 7.63-7.53(m, 14H), 7.38-7.37(m, 12H), 7.28(m, 2H), 7.03-6.98(m, 4H), 6.83(m, 2H), 6.69(m, 4H). 1.72(s, 12H), 1.35(s, 18H) | 1261 | 1261.68 |
| 3394 | δ = 8.00(m, 4H), 7.93-7.92(m, 4H). 7.77-7.73(m. 6H), 7.63-7.59(m, 10H), 7.38-7.37(m, 8H), 7.03-7.01 (m, 6H), 6.83(m, 2H), 6.75(m, 2H), 5.68-6.55(m, 6H), 1.72(s, 12H), 1.35(s. 36H) | 1373 | 1373.89 |
| 3465 | δ = 8.99-8.93(m, 4H), 8.75(m, 2H), 8.34(m, 2H), 8.12-8.10(m, 4H), 7.88-7.64(m, 20H), 7.46-7.37(m, 16H), 7.19(m, 2H), 7.03(m, 2H). 6.83(m, 2H), 6.62(m, 2H), 1.35(s, 18H) | 1331 | 1331.68 |
| 3484 | δ = 8.93(m, 4H), 8.12-8.10(m, 4H), 7.93-7.75(m, 16H), 7.63-7.62(m, 4H), 7.52-7.41(m, 10H), 7.03-6.98(m, 6H), 6.83(m, 2H), 6.75(m, 2H), 6.58-6.51 (m, 6H), 2.34(s, 6H), 1.72(s, 12H) | 1277 | 1277.63 |
| 3507 | δ = 8.93(m, 4H), 8.12(m, 4H), 7.93-7.75(m, 18H), 7.63(m, 2H), 7.55-7.38(m,20H), 7.28(m, 2H), 7.02(m ,2H), 6.89-6.83(m, 6H), 6.69(m. 4H), 6.59(m, 2H), 1.72(s, 12H) | 1401 | 1401.77 |
| 3616 | δ = 8.93(m, 4H), 8.12(m, 4H), 7.93-7.64(m, 28H), 7.55-7.46(m, 6H), 7.38-7.37(m, 6H), 7.28(m ,2H), 7.03(m, 2H), 6.83(m, 2H), 6.56(m, 4H), 1.72(s, 12H) | 1485 | 1485.69 |
| 3670 | δ = 8.99-8.93(m, 6H), 8.68(m ,2H), 8.34(m ,2H), 8.12-8.10(m, 6H), 8.00-7.71 (m ,24H), 7.59-7.58(m, 6H), 7.32(m, 2H), 7.08-6.98(m, 8H), 6.83(m, 2H), 6.51(m, 4H), 2.34(s, 6H) | 1345 | 1345.67 |
| 3752 | δ = 7.93-7.87(m, 4H), 7.78-7.77(m, 4H), 7.63(m, 2H), 7.55-7.54(m, 6H), 7.38(m, 2H), 7.28-7.20(m. 6H), 7.06(m, 2H), 6.81 (m. 4H), 6.69-6.63(m, 8H), 2.02-2.01 (m, 18H), 1.72-1.71 (m, 24H) | 1165 | 1165.59 |
| 3763 | δ = 7.93-7.87(m, 4H), 7.78-7.77(m, 4H), 7.63(m, 2H), 7.55-7.54(m, 6H), 7.38(m, 2H), 7.28(m, 2H), 7.06(m, 2H), 6.98(m, 4H), 6.81(m, 2H), 6.69(m, 4H), 6.51(m, 4H), 2.34(s, 6H), 2.02-2.01(m, 18H), 1.72-1.71(m, 24H) | 1193 | 1193.64 |
| 4001 | δ = 7.93-7.74(m, 16H), 7.63(m, 2H), 7.55-7.49(m, 10H), 7.38-7.28(m, 6H), 7.06(m, 2H), 6.81(m, 2H), 6.69(m, 4H), 1.91(m, 6H). 1.72(s, 12H), 1.66-1.56(m. 12H), 1.31-1.22(m, 22H), 0.88(m, 6H) | 1353 | 1353.90 |
| 4053 | δ = 8.49(m, 2H), 8.07(m, 4H), 7.93-7.87(m, 4H), 7.78-7.77(m, 6H), 7.63-7.54(m, 10H), 7.38(m, 2H), 7.28(m, 2H), 7.04(m, 4H, 6.81(m, 2H), 6.70-6.62(m, 4H), 1.91(m, 6H), 1.72(s, 12H), 1.66-1.56(m, 12H), 1.31-1.22(m, 22H), 0.88(m, 6H) | 1355 | 1355.87 |
| 4217 | δ = 8.18(m, 2H), 8.01(m, 2H), 7.93(m, 2H), 7.77-7.75(m, 4H), 7.63-7.62(m, 4H), 7.53-7.41(m, 14H), 7.20(m, 4H), 7.02(m, 2H), 6.83-6.75(m, 6H), 6.63-6.58(m, 6H), 1.72(s, 12H) | 1163 | 1163.49 |
| 4249 | δ = 8.18(m, 2H), 8.01(m, 2H), 7.93-7.75(m, 16H), 7.63(m, 2H), 7.55-7.50(m, 14H), 7.38-7.28(m, 6H), 7.03(m ,2H), 6.83(m, 2H), 6.69(m. 4H), 1.72(s, 12H) | 1263 | 1263.61 |
| 4281 | δ = 8.49(m, 2H), 8.18(m ,2H), 8.02-8.01(m, 8H), 7.93-7.87(m, 4H), 7.78-7.77(m, 6H), 7.63-7.53(m, 18H), 7.38(m, 4H), 7.28(m, 2H), 7.04-6.98(m, 6H), 6.83(m ,2H), 1.72(s, 12H) | 1363 | 1363.73 |
| 4314 | δ = 8.55(m ,2H), 8.42(m, 2H), 8.18(m, 2H), 8.08-8.01(m, 6H), 7.93(m ,2H), 7.77-7.75(m ,4H), 7.63-7.41(m, ,28H), 7.03(m ,2H), 6.83(m, 2H), 6.69(m, 6H), 6.58(m ,2H), 1.72(s, 12H) | 1415 | 1415.80 |
| 4348 | δ = 8.18(m, 2H), 8.01(m, 2H), 7.93-7.84(m, 8H), 7.77-7.62(m, 14H), 7.55-7.46(m, 12H), 7.38(m ,4H), 7.28(m, 4H), 7.03(m, 2H), 6.83(m, 2H), 6.75(m, 2H), 6.58(m, 2H), 1.72(s, 24H) | 1495 | 1495.93 |
| 4383 | δ = 8.18(m, 2H), 8.01-7.93(m, 10H), 7.77-7.73(m, 6H), 7.63-7.53(m, 14H), 7.03-6.98(m, 6H), 6.83(m ,2H), 6.75(m, 2H), 6.58-6.51(m, 6H), 2.34(s, 6H), 1.72(s, 12H) | 1291 | 1291.67 |
| 4417 | δ = 8.99(m, 2H), 8.68(m, 2H), 8.34(m, 2H), 8.18(m, 2H), 8.10(m, 2H), 8.01-8.00(m, 6H), 7.92(m, 2H), 7.75-7.71 (m, 8H), 7.59-7.53(m, 10H), 7.32(m, 2H), 7.08-7.01(m, 8H), 6.83(m, 2H), 6.55(m, 4H), 1.35(s, 18H) | 1343 | 1343.74 |
| 4452 | δ = 8.93-8.90(m, 4H), 8.34(m ,2H), 8.18-8.10(m, 6H), 8.01(m ,2H), 7.88-7.64(m, 18H), 7.53-7.46(m, 8H), 7.03(m ,2H), 6.83(m ,2H), 6.71 (m ,2H), 6.36(m, 4H), 2.34(s, 12H) | 1287 | 1287.63 |
| 4465 | δ = 7.93(m, 2H), 7.77-7.72(m, 8H), 7.63-7.62(m. 4H), 7.52-7.41(m, 14H), 7.30(m ,2H), 7.20(m, 4H), 7.03(m, 2H), 6.95(s, 4H). 6.83-6.75(m, 6H), 6.63-6.58(m, 6H), 1.72(s, 12H) | 1101 | 1101.42 |
| 4497 | δ = 7.93-7.72(m, 20H), 7.63(m, 2H), 7.55-7.28(m, 22H), 7.03(m ,2H), 6.95(s, 4H), 6.83(m, 2H). 6.69(m ,4H), 1.72(s, 12H) | 1201 | 1201.54 |
| 4529 | δ = 8.49(m ,2H), 8.07-8.02(m, 6H), 7.93-7.87(m, 4H), 7.78-7.72(m, 10H), 7.63-7.28(m ,26H), 7.04-6.98(m, 6H), 6.95(s, 4H), 6.83(m, 2H), 1.72(s, 12H) | 1301 | 1301.66 |
| 4561 | δ = 8.55(m, 2H), 8.42(m, 2H), 8.08-8.04(m, 4H), 7.93(m, 2H), 7.77-7.72(m, 8H), 7.63-7.41(m, 26H), 7.30(m, 2H), 7.03(m ,2H), 6.95(s, 4H), 6.89-6.83(m, 6H), 6.75(m ,2H), 6.59-6.58(m, 4H), 1.729s, 12H) | 1353 | 1353.73 |
| 4596 | δ = 7.93-7.84(m, 8H), 7.77-7.63(m, 18H), 7.55-7.28(m. 22H), 7.03(m, 2H), 6.95(s, 4H), 6.83(m ,2H), 6.75(m ,2H), 6.58(m ,2H), 1.72(s, 24H) | 1433 | 1433.86 |
| 4631 | δ = 8.00-7.92(m, 8H). 7.77-7.72(m, 10H), 7.63-7.58(m, 10H), 7.45(m, 4H), 7.30(m ,2H), 7.03-6.98(m, 6H), 6.95(s, 4H), 6.83(m, 2H), 6.75(m ,2H), 6.58-6.51(m, 6H), 2.34(s, 6H), 1.72(s, 12H) | 1229 | 1229.59 |
| 4665 | δ = 8.99(m, 2H), 8.68(m, 2H), 8.34(m, 2H). 8.10(m, 2H), 8.00-7.92(m, 6H), 7.75-7.71 (m, 12H), 7.59-7.58(m, 6H), 7.45(m , 4H), 7.32-7.30(m. 4H), 7.08-7.03(m, 4H), 6.95(s, 4H), 6.83(m ,2H), 6.71 (m, 2H), 6.36(m, 4H), 2.34(s, 12H) | 1225 | 1225.56 |
| 4703 | δ = 8.99-8.93(m, 4H), 8.34(m ,2H), 8.12-8.10(m, 4H), 7.88-7.41(m, 52H), 7.30(m, 2H), 7.03(m .2H). 6.98(m, 2H), 6.95(s, 4H), 6.83(m, 2H), 6.65(m, 2H) | 1473 | 1473.84 |
| 4713 | δ = 7.93(m, 2H), 7.77-7.75(m, 4H), 7.63-7.41(m, 24H), 7.20(m, 4H), 7.03(m ,2H), 6.83-6.75(m, 6H), 6.63-6.58(m. 6H). 1.72(s, 12H) | 1097 | 1097.39 |

### [Example 1] Manufacture of an OLED by using an organic EL compound according to the invention

An OLED device was manufactured by using EL material according to the invention.

First, a transparent electrode ITO thin film (15 Ω/□) (2) prepared from glass for OLED (produced by Samsung Corning) (1) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injection layer (3) having 60 nm of thickness on the ITO substrate.

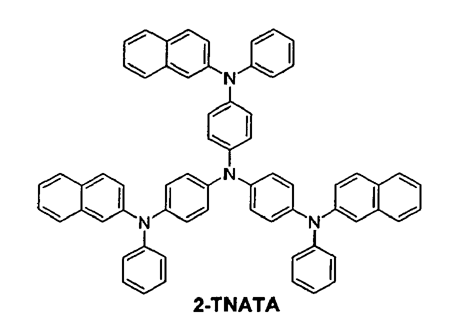

Then, to another cell of the vacuum vapor-deposit device, charged was N,N'-bis(α-naphthyl)-N,N'-diphenyl-4,4'-diamine (NPB), and electric current was applied to the cell to evaporate NPB, thereby providing vapor-deposit of a hole transport layer (4) of 20 nm of thickness on the hole injection layer.

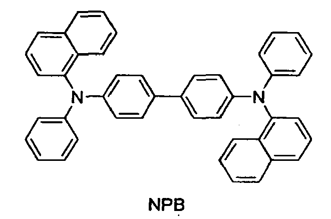

After forming the hole injection layer and hole transport layer, an EL layer was vapor-deposited thereon as follows. To one cell of said vacuum vapor-deposit device, charged was the host (H-6) (having the chemical structure shown below), and Compound (1025) according to the present invention was charged as a dopant to another cell. The two materials were evaporated at different rates to carry out doping at a concentration of 2 to 5 mol% on the basis of the host, to vapor-deposit an electroluminescent layer (5) having 30 nm of thickness on the hole transport layer.

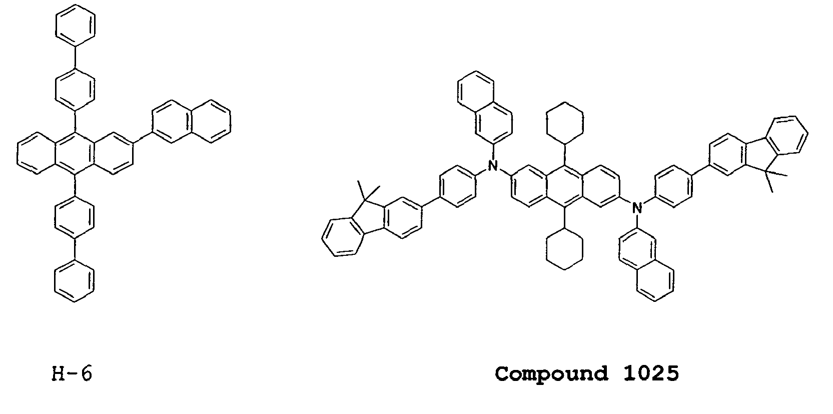

Then, tris(8-hydroxyquinoline)aluminum (III) (Alq) (of which the structure is shown below) was vapor-deposited as an electron transport layer (6) with a thickness of 20 nm, and lithium quinolate (Liq) (of which the structure is shown below) was vapor-deposited as an electron injection layer (7) with a thickness of 1 to 2 nm. Thereafter, an Al cathode (8) was vapor-deposited with a thickness of 150 nm by using another vacuum vapor-deposit device to manufacture an OLED.

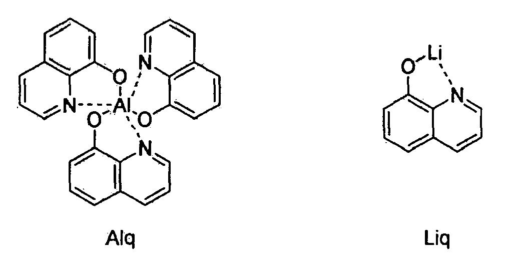

Each compound was used as an EL material for an OLED, after purifying via vacuum sublimation under 10⁻⁶ torr.

### [Comparative Example 1] Manufacture of an OLED by using a conventional EL material

After formation of a hole injection layer and a hole transport layer as described in Example 1, another cell of the vacuum deposition device was charged with tris(8-hydroxyquinoline)aluminum (III) (Alq) as an electroluminescent host material, and still another cell was charged with Coumarin 545T (C545T) having the structure shown below. Two substances were doped by evaporation at different rates to vapor-deposit an electroluminescent layer with a thickness of 30 nm on the hole transport layer. Preferable doping concentration is from 1 to 3 mol% on the basis of Alq.

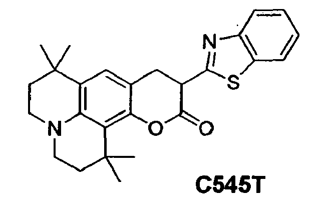

Then, an electron transport layer and an electron injection layer were vapor-deposited according to the same procedure as described in Example 1, and an Al cathode was vapor-deposited by using another vacuum vapor-deposit device with a thickness of 150 nm, to manufacture an OLED.

### [Comparative Example 2] Manufacture of an OLED by using a conventional EL material

After formation of a hole injection layer and a hole transport layer as described in Example 1, another cell of the vacuum deposition device was charged with H-5 as an electroluminescent host material, and still another cell was charged with Compound D-1. Two substances were doped by evaporating at different rates (with a doping concentration of 2 to 5% on the basis of H-5) to vapor-deposit an electroluminescent layer with a thickness of 30 nm on the hole transport layer.

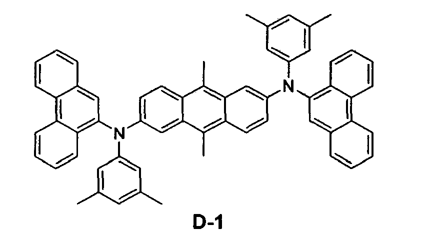

Then, an electron transport layer and an electron injection layer were vapor-deposited according to the same procedure as described in Example 1, and an Al cathode was vapor-deposited by using another vacuum vapor-deposit device with a thickness of 150 nm, to manufacture an OLED.

### [Example 2] EL properties of OLED's manufactured

The luminous efficiencies of OLED's manufactured from Example 1 and Comparative Examples 1-2, comprising an organic EL compound according to the invention and conventional electroluminescent compounds, respectively, were measured at 5,000 cd/m² and 20,000 cd/m², individually, of which the results are shown in Table 3. Since the electroluminescent properties in high luminance region are very important, particularly in case of green electroluminescent materials, the data at high luminance (about 20,000 cd/m²) are attached in order to reflect the properties.

**Table 3**

| No. | Host | Dopant | Doping Conc. (mol%) | Efficiency(cd/A) | | Color |
|---|---|---|---|---|---|---|
| | | | | @5,000 cd/m² | @20,000 cd/m² | |
| 1 | H-6 | Compound 1 | 3.0 | 17.7 | 17.1 | Green |
| 2 | H-6 | Compound 2 | 3.0 | 20.1 | 19.8 | Green |
| 3 | H-6 | Compound 3 | 3.0 | 18.2 | 17.8 | Green |
| 4 | H-6 | Compound 8 | 3.0 | 18.7 | 18.0 | Green |
| 5 | H-6 | Compound 12 | 3.0 | 17.8 | 17.5 | Green |
| 6 | H-6 | Compound 15 | 3.0 | 16.9 | 16.4 | Green |
| 7 | H-6 | Compound 19 | 3.0 | 17.2 | 16.8 | Green |
| 8 | H-6 | Compound 23 | 3.0 | 18.9 | 18.4 | Green |
| 9 | H-6 | Compound 32 | 3.0 | 19.4 | 18.9 | Green |
| 10 | H-6 | Compound 34 | 3.0 | 19.3 | 18.8 | Green |
| 11 | H-6 | Compound 35 | 3.0 | 19.5 | 19.0 | Green |
| 12 | H-6 | Compound 39 | 3.0 | 17.1 | 16.7 | Green |
| 13 | H-6 | Compound 43 | 3.0 | 16.7 | 16.2 | Green |
| 14 | H-6 | Compound 50 | 3.0 | 18.3 | 17.6 | Green |
| 15 | H-6 | Compound 59 | 3.0 | 19.5 | 19.1 | Green |
| 16 | H-6 | Compound 74 | 3.0 | 19.2 | 18.7 | Green |
| 17 | H-6 | Compound 94 | 3.0 | 19.8 | 19.3 | Green |
| 18 | H-6 | Compound 117 | 3.0 | 20.3 | 19.7 | Green |
| 19 | H-6 | Compound 158 | 3.0 | 17.8 | 17.2 | Green |
| 20 | H-6 | Compound 198 | 3.0 | 17.9 | 17.2 | Green |
| 21 | H-6 | Compound 223 | 3.0 | 18.5 | 18.0 | Green |
| 22 | H-6 | Compound 249 | 3.0 | 16.4 | 15.8 | Green |
| 23 | H-6 | Compound 286 | 3.0 | 16.8 | 16.2 | Green |
| 24 | H-6 | Compound 353 | 3.0 | 17.9 | 17.4 | Green |
| 25 | H-6 | Compound 422 | 3.0 | 20.5 | 20.1 | Green |
| 26 | H-6 | Compound 528 | 3.0 | 20.4 | 20.1 | Green |
| 27 | H-6 | Compound 602 | 3.0 | 21.1 | 20.7 | Green |
| 28 | H-6 | Compound 648 | 3.0 | 19.7 | 19.5 | Green |
| 29 | H-6 | Compound 745 | 3.0 | 18.7 | 18.0 | Green |
| 30 | H-6 | Compound 777 | 3.0 | 18.4 | 17.8 | Green |
| 31 | H-6 | Compound 852 | 3.0 | 19.6 | 19.2 | Green |
| 32 | H-6 | Compound 913 | 3.0 | 16.7 | 16.2 | Green |
| 33 | H-6 | Compound 982 | 3.0 | 16.9 | 16.4 | Green |
| 34 | H-6 | Compound 993 | 3.0 | 16.4 | 15.8 | Green |
| 35 | H-6 | Compound 1025 | 3.0 | 22.5 | 21.8 | Green |
| 36 | H-6 | Compound 1068 | 3.0 | 20.2 | 19.7 | Green |
| 37 | H-6 | Compound 1133 | 3.0 | 19.2 | 18.5 | Green |
| 38 | H-6 | Compound 1205 | 3.0 | 17.8 | 17.6 | Green |
| 39 | H-6 | Compound 1244 | 3.0 | 17.5 | 16.8 | Green |
| 40 | H-6 | Compound 1273 | 3.0 | 20.8 | 20.4 | Green |
| 41 | H-6 | Compound 1314 | 3.0 | 18.2 | 17.7 | Green |
| 42 | H-6 | Compound 1387 | 3.0 | 18.1 | 17.7 | Green |
| 43 | H-6 | Compound 1392 | 3.0 | 19.2 | 18.5 | Green |
| 44 | H-6 | Compound 1462 | 3.0 | 17.5 | 16.9 | Green |
| 45 | H-6 | Compound 1561 | 3.0 | 18.1 | 17.4 | Green |
| 46 | H-6 | Compound 1627 | 3.0 | 16.8 | 16.4 | Green |
| 47 | H-6 | Compound 1737 | 3.0 | 16.4 | 15.8 | Green |
| 48 | H-6 | Compound 1770 | 3.0 | 20.9 | 19.8 | Green |
| 49 | H-6 | Compound 1849 | 3.0 | 17.2 | 16.7 | Green |
| 50 | H-6 | Compound 1875 | 3.0 | 19.7 | 18.9 | Green |
| 51 | H-6 | Compound 1971 | 3.0 | 17.8 | 15.6 | Green |
| 52 | H-6 | Compound 2016 | 3.0 | 18.5 | 17.8 | Green |
| 53 | H-6 | Compound 2086 | 3.0 | 21.4 | 19.4 | Green |
| 54 | H-6 | Compound 2227 | 3.0 | 18.7 | 17.7 | Green |
| 55 | H-6 | Compound 2240 | 3.0 | 18.1 | 17.7 | Green |
| 56 | H-6 | Compound 2400 | 3.0 | 19.9 | 19.2 | Green |
| 57 | H-6 | Compound 2512 | 3.0 | 17.5 | 16.9 | Green |
| 58 | H-6 | Compound 2523 | 3.0 | 19.1 | 17.8 | Green |
| 59 | H-6 | Compound 2728 | 3.0 | 18.1 | 17.8 | Green |
| 60 | H-6 | Compound 2775 | 3.0 | 20.2 | 19.6 | Green |
| 61 | H-6 | Compound 2803 | 3.0 | 19.0 | 18.5 | Green |
| 62 | H-6 | Compound 2867 | 3.0 | 18.8 | 16.6 | Green |
| 63 | H-6 | Compound 2925 | 3.0 | 17.5 | 16.8 | Green |
| 64 | H-6 | Compound 2978 | 3.0 | 19.8 | 19.0 | Green |
| 65 | H-6 | Compound 3021 | 3.0 | 18.4 | 17.7 | Green |
| 66 | H-6 | Compound 3222 | 3.0 | 18.2 | 17.8 | Green |
| 67 | H-6 | Compound 3258 | 3.0 | 19.2 | 18.5 | Green |
| 68 | H-6 | Compound 3394 | 3.0 | 18.5 | 16.9 | Green |
| 69 | H-6 | Compound 3413 | 3.0 | 22.2 | 21.5 | Green |
| 70 | H-6 | Compound 3455 | 3.0 | 20.1 | 19.7 | Green |
| 71 | H-6 | Compound 3468 | 3.0 | 19.2 | 18.5 | Green |
| 72 | H-6 | Compound 3471 | 3.0 | 17.7 | 17.6 | Green |
| 73 | H-6 | Compound 3503 | 3.0 | 17.5 | 16.8 | Green |
| 74 | H-6 | Compound 3529 | 3.0 | 17.8 | 17.4 | Green |
| 75 | H-6 | Compound 3677 | 3.0 | 18.2 | 18.0 | Green |
| 76 | H-6 | Compound 3689 | 3.0 | 18.1 | 17.5 | Green |
| 77 | H-6 | Compound 3898 | 3.0 | 19.2 | 18.5 | Green |
| 78 | H-6 | Compound 4164 | 3.0 | 17.5 | 16.9 | Green |
| 79 | H-6 | Compound 4274 | 3.0 | 18.8 | 17.0 | Green |
| 80 | H-6 | Compound 4578 | 3.0 | 16.8 | 16.0 | Green |
| 81 | H-13 | Compound 2 | 3.0 | 18.7 | 18.1 | Green |
| 82 | H-13 | Compound 3 | 3.0 | 20.7 | 19.9 | Green |
| 83 | H-13 | Compound 8 | 3.0 | 19.3 | 18.5 | Green |
| 84 | H-13 | Compound 12 | 3.0 | 18.6 | 18.1 | Green |
| 85 | H-13 | Compound 15 | 3.0 | 18.8 | 18.5 | Green |
| 86 | H-13 | Compound 23 | 3.0 | 17.8 | 17.3 | Green |
| 87 | H-13 | Compound 24 | 3.0 | 17.6 | 16.9 | Green |
| 88 | H-13 | Compound 39 | 3.0 | 19.9 | 19.3 | Green |
| 89 | H-13 | Compound 59 | 3.0 | 19.1 | 18.5 | Green |
| 90 | H-13 | Compound 74 | 3.0 | 19.8 | 19.6 | Green |
| 91 | H-13 | Compound 94 | 3.0 | 19.6 | 19.1 | Green |
| 92 | H-13 | Compound 117 | 3.0 | 18.2 | 17.5 | Green |
| 93 | H-13 | Compound 158 | 3.0 | 17.3 | 16.1 | Green |
| 94 | H-13 | Compound 286 | 3.0 | 19.3 | 18.6 | Green |
| 95 | H-13 | Compound 353 | 3.0 | 20.5 | 20.0 | Green |
| 96 | H-13 | Compound 528 | 3.0 | 19.4 | 18.6 | Green |
| 97 | H-13 | Compound 648 | 3.0 | 20.2 | 19.5 | Green |
| 98 | H-13 | Compound 745 | 3.0 | 17.3 | 16.7 | Green |
| 99 | H-13 | Compound 982 | 3.0 | 18.8 | 18.2 | Green |
| 100 | H-13 | Compound 1025 | 3.0 | 21.9 | 21.2 | Green |
| 101 | H-13 | Compound 1133 | 3.0 | 18.3 | 17.3 | Green |
| 102 | H-13 | Compound 1273 | 3.0 | 16.6 | 15.9 | Green |
| 103 | H-13 | Compound 1314 | 3.0 | 19.7 | 19.1 | Green |
| 104 | H-13 | Compound 1392 | 3.0 | 18.9 | 18.1 | Green |
| 105 | H-13 | Compound 1561 | 3.0 | 19.0 | 18.4 | Green |
| 106 | H-13 | Compound 1627 | 3.0 | 19.9 | 18.4 | Green |
| 107 | H-13 | Compound 1737 | 3.0 | 21.7 | 20.8 | Green |
| 108 | H-13 | Compound 1875 | 3.0 | 20.4 | 19.5 | Green |
| 109 | H-13 | Compound 1971 | 3.0 | 16.7 | 16.0 | Green |
| 110 | H-13 | Compound 2086 | 3.0 | 16.4 | 15.8 | Green |
| 111 | H-13 | Compound 2240 | 3.0 | 19.8 | 19.1 | Green |
| 112 | H-13 | Compound 2512 | 3.0 | 15.7 | 15.2 | Green |
| 113 | H-13 | Compound 2523 | 3.0 | 16.6 | 16.2 | Green |
| 114 | H-13 | Compound 2728 | 3.0 | 16.4 | 15.8 | Green |
| 115 | H-13 | Compound 2775 | 3.0 | 19.1 | 18.8 | Green |
| 116 | H-13 | Compound 2978 | 3.0 | 20.7 | 18.7 | Green |
| 117 | H-13 | Compound 3258 | 3.0 | 21.2 | 20.5 | Green |
| 118 | H-13 | Compound 3394 | 3.0 | 18.8 | 17.4 | Green |
| 119 | H-13 | Compound 3503 | 3.0 | 17.5 | 16.8 | Green |
| 120 | H-13 | Compound 3529 | 3.0 | 17.8 | 17.4 | Green |
| 121 | H-13 | Compound 3677 | 3.0 | 18.2 | 17.7 | Green |
| 122 | H-13 | Compound 3689 | 3.0 | 18.7 | 16.8 | Green |
| 123 | H-13 | Compound 3898 | 3.0 | 19.6 | 18.2 | Green |
| 124 | H-13 | Compound 4164 | 3.0 | 17.5 | 15.9 | Green |
| 125 | H-13 | Compound 4274 | 3.0 | 19.1 | 18.4 | Green |
| 126 | H-13 | Compound 4578 | 3.0 | 21.8 | 20.7 | Green |
| 127 | H-24 | Compound 2 | 3.0 | 18.2 | 17.5 | Green |
| 128 | H-24 | Compound 3 | 3.0 | 19.9 | 19.0 | Green |
| 129 | H-24 | Compound 8 | 3.0 | 19.1 | 18.6 | Green |
| 130 | H-24 | Compound 12 | 3.0 | 18.0 | 17.4 | Green |
| 131 | H-24 | Compound 15 | 3.0 | 18.4 | 17.9 | Green |
| 132 | H-24 | Compound 23 | 3.0 | 17.5 | 17.0 | Green |
| 133 | H-24 | Compound 24 | 3.0 | 17.8 | 17.0 | Green |
| 134 | H-24 | Compound 39 | 3.0 | 19.2 | 18.5 | Green |
| 135 | H-24 | Compound 59 | 3.0 | 18.8 | 18.2 | Green |
| 136 | H-24 | Compound 74 | 3.0 | 19.4 | 19.0 | Green |
| 137 | H-24 | Compound 94 | 3.0 | 20.6 | 20.0 | Green |
| 138 | H-24 | Compound 117 | 3.0 | 19.5 | 18.5 | Green |
| 139 | H-24 | Compound 158 | 3.0 | 18.2 | 17.3 | Green |
| 140 | H-24 | Compound 286 | 3.0 | 19.8 | 18.9 | Green |
| 141 | H-24 | Compound 353 | 3.0 | 20.6 | 19.2 | Green |
| 142 | H-24 | Compound 528 | 3.0 | 19.9 | 19.1 | Green |
| 143 | H-24 | Compound 648 | 3.0 | 17.6 | 16.0 | Green |
| 144 | H-24 | Compound 745 | 3.0 | 17.5 | 16.8 | Green |
| 145 | H-24 | Compound 982 | 3.0 | 19.4 | 19.0 | Green |
| 146 | H-24 | Compound 1025 | 3.0 | 21.8 | 20.9 | Green |
| 147 | H-24 | Compound 1133 | 3.0 | 18.8 | 18.1 | Green |
| 148 | H-24 | Compound 1273 | 3.0 | 16.6 | 15.9 | Green |
| 149 | H-24 | Compound 1314 | 3.0 | 19.7 | 19.1 | Green |
| 150 | H-24 | Compound 1392 | 3.0 | 17.9 | 17.0 | Green |
| 151 | H-24 | Compound 1561 | 3.0 | 19.2 | 18.0 | Green |
| 152 | H-24 | Compound 1627 | 3.0 | 20.9 | 19.8 | Green |
| 153 | H-24 | Compound 1737 | 3.0 | 21.6 | 20.8 | Green |
| 154 | H-24 | Compound 1875 | 3.0 | 20.5 | 19.5 | Green |
| 155 | H-24 | Compound 1971 | 3.0 | 18.7 | 16.0 | Green |
| 156 | H-24 | Compound 2086 | 3.0 | 19.4 | 17.8 | Green |
| 157 | H-119 | Compound 2 | 3.0 | 19.8 | 19.5 | Green |
| 158 | H-119 | Compound 3 | 3.0 | 20.0 | 19.5 | Green |
| 159 | H-119 | Compound 8 | 3.0 | 19.7 | 19.6 | Green |
| 160 | H-119 | Compound 12 | 3.0 | 18.7 | 18.4 | Green |
| 161 | H-119 | Compound 15 | 3.0 | 18.8 | 18.6 | Green |
| 162 | H-119 | Compound 23 | 3.0 | 18.5 | 18.1 | Green |
| 163 | H-119 | Compound 24 | 3.0 | 18.5 | 18.4 | Green |
| 164 | H-119 | Compound 39 | 3.0 | 19.8 | 19.5 | Green |
| 165 | H-119 | Compound 59 | 3.0 | 19.9 | 19.6 | Green |
| 166 | H-119 | Compound 74 | 3.0 | 20.7 | 20.5 | Green |
| 167 | H-119 | Compound 94 | 3.0 | 21.4 | 21.2 | Green |
| 168 | H-119 | Compound 117 | 3.0 | 20.1 | 20.0 | Green |
| 169 | H-119 | Compound 158 | 3.0 | 19.2 | 19.1 | Green |
| 170 | H-119 | Compound 286 | 3.0 | 19.7 | 19.6 | Green |
| 171 | H-119 | Compound 353 | 3.0 | 20.5 | 20.2 | Green |
| 172 | H-119 | Compound 528 | 3.0 | 18.9 | 18.6 | Green |
| 173 | H-119 | Compound 648 | 3.0 | 18.6 | 18.0 | Green |
| 174 | H-119 | Compound 745 | 3.0 | 17.7 | 17.5 | Green |
| 175 | H-119 | Compound 982 | 3.0 | 19.8 | 19.7 | Green |
| 176 | H-119 | Compound 1025 | 3.0 | 21.7 | 21.5 | Green |
| 177 | H-119 | Compound 1133 | 3.0 | 18.7 | 18.6 | Green |
| 178 | H-119 | Compound 1273 | 3.0 | 17.6 | 17.4 | Green |
| 179 | H-119 | Compound 1314 | 3.0 | 19.6 | 19.4 | Green |
| 180 | H-119 | Compound 1392 | 3.0 | 18.7 | 18.6 | Green |
| 181 | H-119 | Compound 1561 | 3.0 | 19.2 | 19.0 | Green |
| 182 | H-119 | Compound 1627 | 3.0 | 21.3 | 21.2 | Green |
| 183 | H-119 | Compound 1737 | 3.0 | 18.4 | 18.2 | Green |
| 184 | H-119 | Compound 1875 | 3.0 | 18.5 | 18.3 | Green |
| 185 | H-119 | Compound 1971 | 3.0 | 19.7 | 19.5 | Green |
| 186 | H-119 | Compound 2086 | 3.0 | 18.4 | 18.3 | Green |
| 187 | H-119 | Compound 2240 | 3.0 | 17.6 | 17.4 | Green |
| 188 | H-119 | Compound 2512 | 3.0 | 18.7 | 18.6 | Green |
| 189 | H-119 | Compound 2523 | 3.0 | 18.5 | 18.3 | Green |
| 190 | H-119 | Compound 2728 | 3.0 | 19.6 | 19.4 | Green |
| 191 | H-119 | Compound 2775 | 3.0 | 17.3 | 17.1 | Green |
| 192 | H-119 | Compound 2978 | 3.0 | 19.2 | 19.0 | Green |
| 193 | H-119 | Compound 3258 | 3.0 | 20.8 | 20.4 | Green |
| 194 | H-119 | Compound 3394 | 3.0 | 19.7 | 19.5 | Green |
| 195 | H-119 | Compound 4578 | 3.0 | 19.9 | 19.5 | Green |
| Comp. Ex.1 | Alq | C545T | 1.0 | 10.3 | 9.1 | Green |
| Comp. Ex.2 | H-6 | D-1 | 3.0 | 16.3 | 14.1 | Green |

As can be seen from Table 3, when the organic EL compound according to the present invention was employed in a green electroluminescent device, the device exhibited significantly improved luminous efficiency, while maintaining at least comparable color purity as compared to Comparative Example 1.

Particularly, the fact that decrease in the efficiency of the electroluminescent materials of high performance according to the present invention is within the range from 1 to 2 cd/A, even at a high luminance (20,000 cd/m²), implies excellent property as an EL material maintained even at a high luminance, not only at a low luminance. This demonstrates that the compounds can be advantageously employed in both passive and active organic OLED's.

This feature is contrary to that of conventional EL material having excellent electron conductivity, and the result shows the most noticeable advantage of the EL material according to the present invention.

## Claims

1. An organic electroluminescent compound represented by Chemical Formula (1): 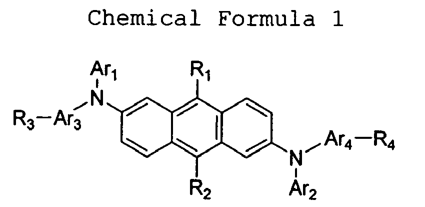 wherein, R₁ and R₂ independently represent hydrogen, deuterium, (C1-C60)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, (C7-C15)tricycloalkyl, (C4-C15)bicycloalkyl, (C6-C60)aryl or (C3-C60)heteroaryl, and the alkyl, alkenyl, alkynyl, cycloalkyl, trialkylsilyl, dialkylarylsilyl, triarylsilyl, tricycloalkyl, bicycloalkyl, aryl or heteroaryl of R₁ and R₂ may be further substituted by one or more substituent(s) selected from deuterium, (C1-C60)alkyl, (C1-C20)alkenyl, (C1-C20)alkynyl, halogen, cyano, phenyl, biphenyl, fluorenyl, naphthyl and anthryl; and
Ar₁ and Ar₂ independently represent (C6-C60)aryl, (C3-C60)heteroaryl, morpholino or thiomorpholino, and the aryl or heteroaryl of Ar₁ and Ar₂ may be further substituted by one or more substituent(s) selected from a group consisting of deuterium, (C1-C60)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl, tri(C6-C20)arylsilyl, phenyl, biphenyl, fluorenyl, naphthyl and anthryl;
Ar₃ and Ar₄ independently represent (C6-C20)arylene with or without (C1-C20)alkyl substituent;
R₃ and R₄ independently represent (C1-C20)alkyl or (C6-C20)aryl, and the aryl of R₃ and R₄ may be further substituted by deuterium or (C1-C20)alkyl;
provided that total number of carbons in 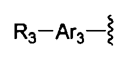 and is from 21 to 60.

2. The organic electroluminescent compound according to claim 1, wherein 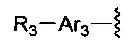 and 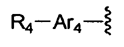 are independently selected from the following structures: 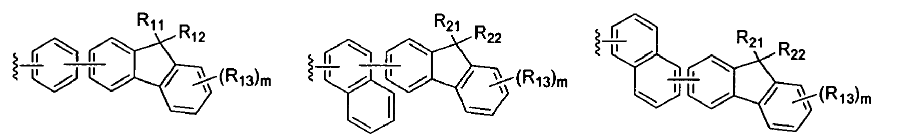 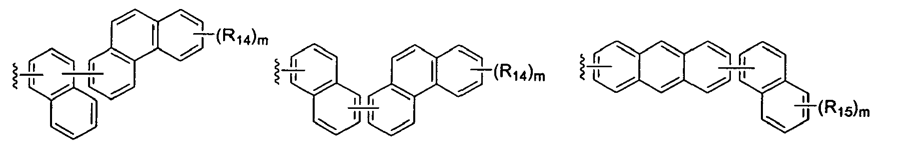 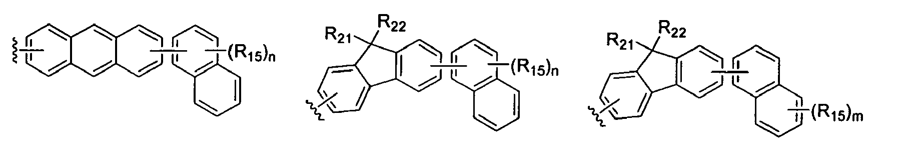 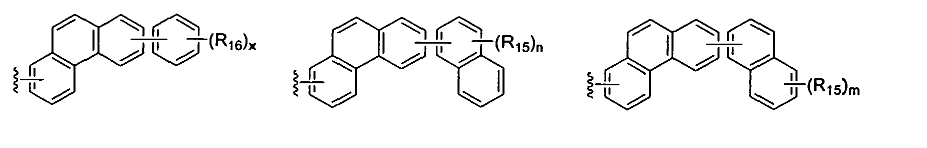 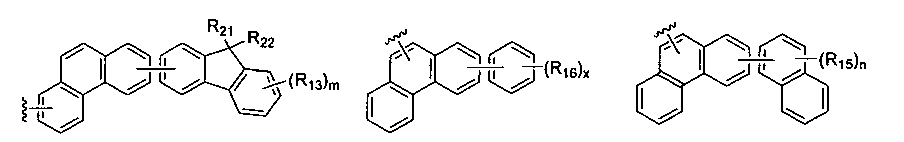 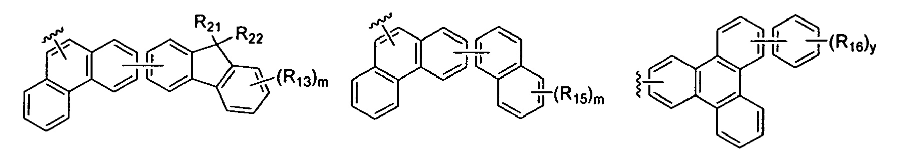 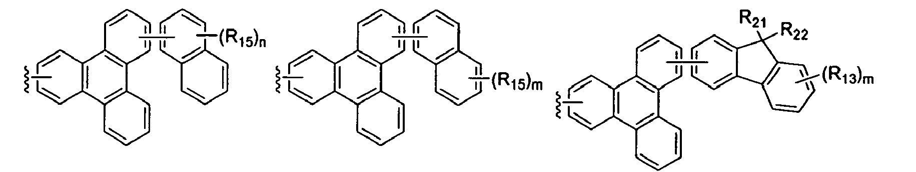 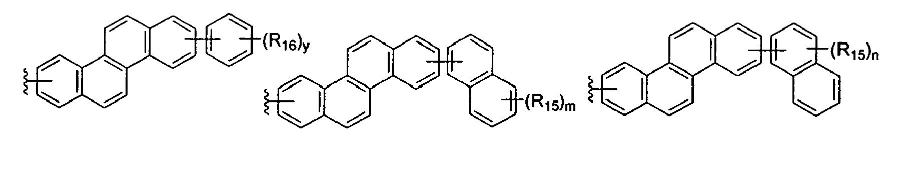 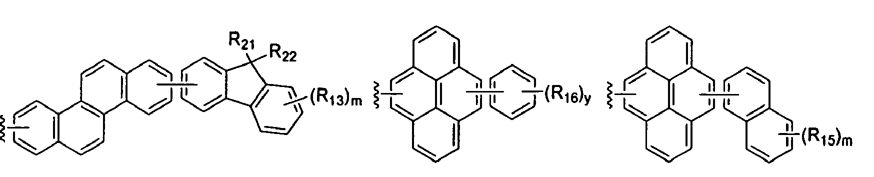 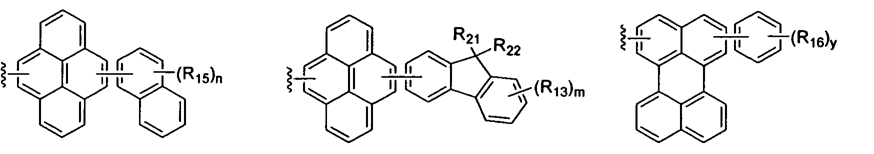 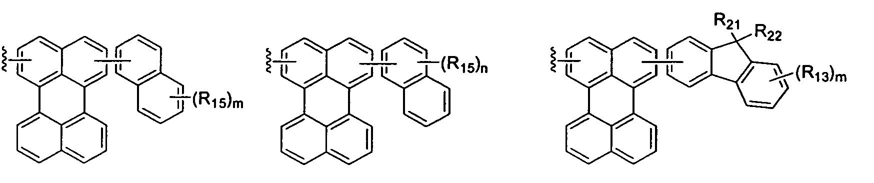 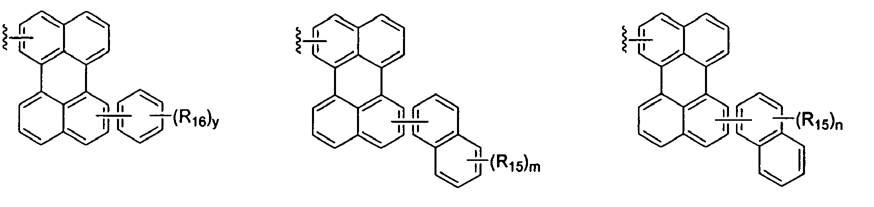 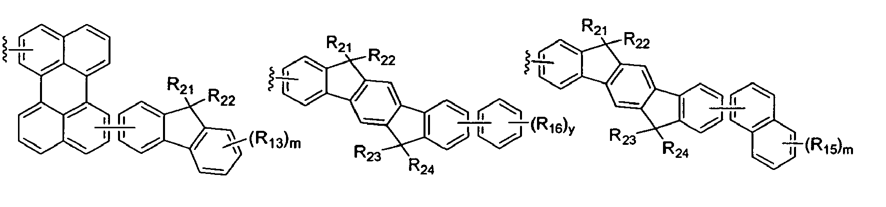 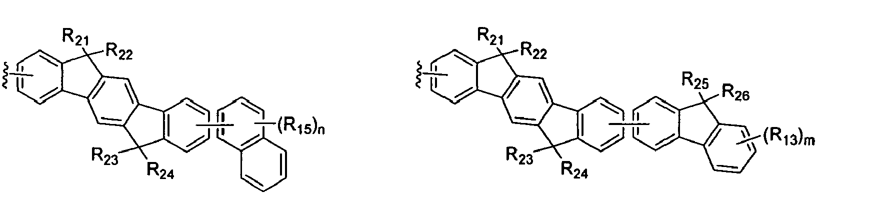 wherein, R₁₁ through R₁₆ independently represent (C1-C20)alkyl or (C6-C20)aryl;
R₂₁ through R₂₆ independently represent hydrogen, (C1-C20)alkyl or (C6-C20)aryl; the aryl of R₁₁ through R₁₆ and R₂₁ through R₂₆ may be further substituted by deuterium or (C1-C20)alkyl;
m is an integer from 0 to 4;
n is an integer from 0 to 3;
x is an integer from 1 to 5; and
y is an integer from 0 to 5.

3. An organic electroluminescent device comprising an organic electroluminescent compound represented by Chemical Formula (1): 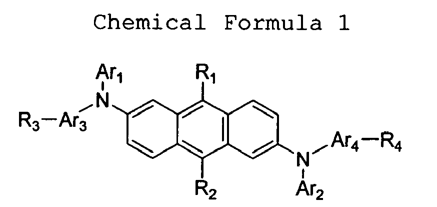 wherein, R₁ and R₂ independently represent hydrogen, deuterium, (C1-C60)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, (C7-C15)tricycloalkyl, (C4-C15)bicycloalkyl, (C6-C60)aryl or (C3-C60)heteroaryl, and the alkyl, alkenyl, alkynyl, cycloalkyl, trialkylsilyl, dialkylarylsilyl, triarylsilyl, tricycloalkyl, bicycloalkyl, aryl or heteroaryl of R₁ and R₂ may be further substituted by one or more substituent(s) selected from deuterium, (C1-C60)alkyl, (C1-C20)alkenyl, (C1-C20)alkynyl, halogen, cyano, phenyl, biphenyl, fluorenyl, naphthyl and anthryl; and
Ar₁ and Ar₂ independently represent (C6-C60)aryl, (C3-C60)heteroaryl, morpholino or thiomorpholino, and the aryl or heteroaryl of Ar₁ and Ar₂ may be further substituted by one or more substituent(s) selected from a group consisting of deuterium, (C1-C60)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl, tri(C6-C20)arylsilyl, phenyl, biphenyl, fluorenyl, naphthyl and anthryl;
Ar₃ and Ar₄ independently represent (C6-C20)arylene with or without (C1-C20)alkyl substituent;
R₃ and R₄ independently represent (C1-C20)alkyl or (C6-C20)aryl, and the aryl of R₃ and R₄ may be further substituted by deuterium or (C1-C20)alkyl;
provided that total number of carbons in 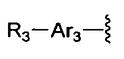 and 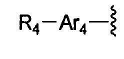 is from 21 to 60 wherein the organic electroluminescent compound is employed as a dopant material of an electroluminescent layer.

4. An organic electroluminescent device which is comprised of a first electrode; a second electrode; and at least one organic layer(s) interposed between the first electrode and the second electrode; wherein the organic layer comprises an organic electroluminescent compound represented by Chemical Formula (1): 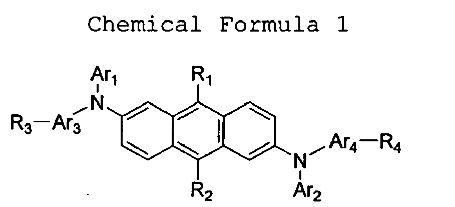 wherein, R₁ and R₂ independently represent hydrogen, deuterium, (C1-C60)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, (C7-C15)tricycloalkyl, (C4-C15)bicycloalkyl, (C6-C60)aryl or (C3-C60)heteroaryl, and the alkyl, alkenyl, alkynyl, cycloalkyl, trialkylsilyl, dialkylarylsilyl, triarylsilyl, tricycloalkyl, bicycloalkyl, aryl or heteroaryl of R₁ and R₂ may be further substituted by one or more substituent(s) selected from deuterium, (C1-C60)alkyl, (C1-C20)alkenyl, (C1-C20)alkynyl, halogen, cyano, phenyl, biphenyl, fluorenyl, naphthyl and anthryl; and
Ar₁ and Ar₂ independently represent (C6-C60)aryl, (C3-C60)heteroaryl, morpholino or thiomorpholino, and the aryl or heteroaryl of Ar₁ and Ar₂ may be further substituted by one or more substituent(s) selected from a group consisting of deuterium, (C1-C60)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl, tri(C6-C20)arylsilyl, phenyl, biphenyl, fluorenyl, naphthyl and anthryl;
Ar₃ and Ar₄ independently represent (C6-C20)arylene with or without (C1-C20)alkyl substituent;
R₃ and R₄ independently represent (C1-C20)alkyl or (C6-C20)aryl, and the aryl of R₃ and R₄ may be further substituted by deuterium or (C1-C20)alkyl;
provided that total number of carbons in 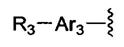 and 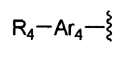 is from 21 to 60 and one or more host(s) selected from the compounds represented by Chemical Formula (2) or (3):
Chemical Formula 2 (Ar₁₁)_{b}-L₁-(Ar₁₂)_{c}
Chemical Formula 3 (Ar₁₃)_{d}-L₂-(Ar₁₄)ₑ
wherein, L₁ represents (C6-C60)arylene or (C4-C60)heteroarylene;
L₂ represents anthracenylene;
Ar₁₁ through Ar₁₄ are independently selected from hydrogen, (C1-C60)alkyl, (C1-C60)alkoxy, halogen, (C4-C60)heteroaryl, (C5-C60)cycloalkyl and (C6-C60)aryl; and the cycloalkyl, aryl or heteroaryl of Ar₁₁ through Ar₁₄ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
b, c, d and e independently represent an integer from 0 to 4.

5. The organic electroluminescent device according to claim 4, wherein the organic layer comprises one or more compound(s) selected from a group consisting of arylamine compounds and styrylarylamine compounds.

6. The organic electroluminescent device according to claim 4, wherein the organic layer comprises one or more metal(s) selected from a group consisting of organic metals of Group 1, Group 2, 4^{th} period and 5^{th} period transition metals, lanthanide metals and d-transition elements.

7. The organic electroluminescent device according to claim 4, which is an organic display comprising an organic electroluminescent compound having the electroluminescent peak with wavelength of not more than 500 nm, and an organic electroluminescent compound having the electroluminescent peak with wavelength of not less than 560 nm, at the same time.

8. The organic electroluminescent device according to claim 4, wherein the organic layer comprises an electroluminescent layer and a charge generating layer.

9. The organic electroluminescent device according to claim 4, wherein a mixed region of reductive dopant and organic substance, or a mixed region of oxidative dopant and organic substance is placed on the inner surface of one or both electrode(s) among the pair of electrodes.

10. An organic solar cell which comprises an organic electroluminescent compound represented by an organic electroluminescent compound represented by Chemical Formula (1): 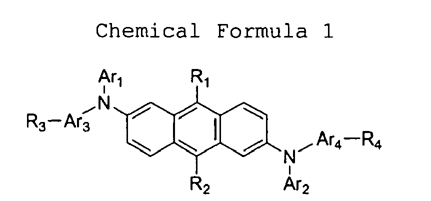 wherein, R₁ and R₂ independently represent hydrogen, deuterium, (C1-C60)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, (C7-C15)tricycloalkyl, (C4-C15)bicycloalkyl, (C6-C60)aryl or (C3-C60)heteroaryl, and the alkyl, alkenyl, alkynyl, cycloalkyl, trialkylsilyl, dialkylarylsilyl, triarylsilyl, tricycloalkyl, bicycloalkyl, aryl or heteroaryl of R₁ and R₂ may be further substituted by one or more substituent(s) selected from deuterium, (C1-C60)alkyl, (C1-C20)alkenyl, (C1-C20)alkynyl, halogen, cyano, phenyl, biphenyl, fluorenyl, naphthyl and anthryl; and
Ar₁ and Ar₂ independently represent (C6-C60)aryl, (C3-C60)heteroaryl, morpholino or thiomorpholino, and the aryl or heteroaryl of Ar₁ and Ar₂ may be further substituted by one or more substituent(s) selected from a group consisting of deuterium, (C1-C60)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl, tri(C6-C20)arylsilyl, phenyl, biphenyl, fluorenyl, naphthyl and anthryl;
Ar₃ and Ar₄ independently represent (C6-C20)arylene with or without (C1-C20)alkyl substituent;
R₃ and R₄ independently represent (C1-C20)alkyl or (C6-C20)aryl, and the aryl of R₃ and R₄ may be further substituted by deuterium or (C1-C20)alkyl;
provided that total number of carbons in 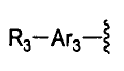 and 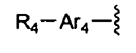 is from 21 to 60.
